# EUROPEAN PATENT APPLICATION

(11) **EP 1 279 744 A2**
(43) Date of publication of application: **29.01.2003**
(21) Application number: 02255249.1
(22) Date of filing: 26.07.2002
(51) Int. Cl.: C12Q 1/68, C07K 14/47, A61P 5/48, C12N 5/10, C12N 15/85

(54) **Identification and use of molecules implicated in pain**

(30) Priority: 27.07.2001 GB 0118354; 07.02.2002 GB 0202910
(71) Applicant: WARNER-LAMBERT COMPANY, Morris Plains New Jersey 07950 (US)
(72) Inventor: Brooksbank, Robert A., c/o Pfizer Ltd UK Pat. Dept, Sandwich, Kent CT13 9NJ (GB); Dixon, Alistair K., Cambridgeshire CBI 2LL (GB); Lee, Kevin, c/o Pfizer Ltd UK Pat. Dept, Sandwich, Kent CT13 9NJ (GB); Pinnock, Robert D., c/o Pfizer Global Res. & Dev., Ann Arbor, MI 48105 (US)
(74) Representative: Hayles, James Richard

(57) **Abstract**

The invention relates to the use of:
(a) an isolated gene sequence that is up regulated in the spinal cord in response to streptozocin-induced diabetes;
(b) an isolated gene sequence comprising a nucleic acid sequence of Tables I to X.
(c) an isolated gene sequence having at least 80% sequence identity with a nucleic acid sequence of Tables I to X;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell containing the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus; or
(i) an isolated polypeptide encoded by a nucleotide sequence according to any one of (a) to (d); or
(k) an isolated antibody that binds specifically to a polypeptide according to (h) or (i);
in the screening of compounds for the treatment of pain, or for the diagnosis of pain.

The invention also relates to the use of naturally occurring compounds such as peptide ligands of the expression products of the above gene sequences and their associated signal transduction pathways for use in the treatment of pain.

## Description

### FIELD OF THE INVENTION

The present invention relates to nucleic acids, their expression products and pathways involved in pain, and their use in screening for molecules that can alleviate pain. The invention further relates to methods for the assay and diagnosis of pain in patients.

### BACKGROUND TO THE INVENTION

Pain is currently classified into four general types. Post-operative acute pain can be successfully treated with existing pain medications of e.g. the opioid and non-steroidal anti-inflammatory (NSAID) types, and is usually short-term and self-limiting. A second type of pain, present e.g. in cancer and arthritis, is also responsive to medication initially with a NSAID and in its later stages with opioids. Neuropathic pain arises from damage to the central or peripheral nerve systems, and is more effectively treated with antidepressants or anticonvulsants. A fourth type of pain called central sensitisation results from changes in the central nervous system as a result of chronic pain, these changes often being irreversible and difficult to treat. Nerve pain from shingles or diabetes falls into this and the neuropathic category. Changes occur where pain is at first poorly controlled and gradually progress to the point where a person is sensitive to stimuli which would not normally cause pain, for example a light touch. People with pain of this kind often describe a widening of the pain area to include areas which had originally not been injured or which were thought not to be involved in pain. This classification is, however based on clinical symptoms rather than on the underlying pain mechanisms.

Opiates such as morphine belong to a traditional class of pain-relieving compounds that are now recognized as binding to opiate receptors. Naturally occurring polypeptides have also been found to have opiate-like effects on the central nervous system, and these include β-endorphin, met-enkephalin and leu-enkephalin.

Salicin was isolated at the beginning of the 19th century, and from that discovery a number of NSAIDs such as aspirin, paracetamol, ibuprofen, flurbiprofen and naproxen were developed. NSAIDs are by far the most widely used pain-relieving compounds, but can exhibit side effects, in particular irritation of the GI tract that can lead to the formation of ulcers, gastrointestinal bleeding and anaemia.

Interest in the neurobiology of pain is developing: see a colloquium sponsored by the US National Academy of Sciences in December 1998 concerning the neurobiology of pain and reviewed in *The Scientist* **13[1]**, 12, 1999. Many pain mechanisms were discussed including the role of the capsaicin receptors in pain, (M.J. Caterina *et al*., *Nature,* **389**, 816-824, 1997). Large dosages of capsaicin were reported to disable that receptor, (W.R. Robbins *et al*.*, Anesthesia and Analgesia,* **86**, 579-583, 1998). Additionally, a c-resistant sodium channel found in small diameter pain-sensing neurons (PN3) was discussed (A.N. Akopian *et al*., *Nature,* **379,** 257-262, 1986) and L. Sangameswaran *et al*., *Journal of Biological Chemistry,* **271**, 953-956, 1996). Its involvement in transmission and sensitization to pain signals has been reported, (S.D. Novakovic *et al*., *Journal of Neuroscience,* **18**, 2174-2187, 1998). A further tetrodotoxinresistant sodium channel has been reported (S. Tate *et al.*, *Nature Neuroscience,* **1**, 653-655 1998).

Second messenger systems have also been shown to be important since knockoutmice lacking protein kinase C (PKC) γ were reported to respond to acute pain e.g. from a hot surface, but not to respond to neuropathic pain when their spinal nerves are injured (Malmberg *et al*., Science, **278,** 279-283 (1997).

Present methods for identifying novel compounds that relieve pain of one or more of the types indicated above suffer from the defect that they are dependent either on the relatively limited number of receptors known to be involved in pain or on the empirical identification of new receptors which is an uncertain process. In relation to known receptors, for example the opioid receptor, research directed to improved compounds offers the possibility of screening compounds that have a better therapeutic ratio and fewer side effects. It does not lead naturally to compounds for different pain receptors that have new modes of action and new and qualitatively different benefits. Even when newly identified additional receptors are taken into account, known receptors revolve around tens of gene products. However, there are between 30,000 and 40,000 genes in the genome of an animal and more of them are concerned with nervous system function than with peripheral function. We therefore concluded that a large number of receptors and pathways are important to the transduction of pain, but up to now have remained unknown.

### SUMMARY OF THE INVENTION

It is an object of the invention to provide sequences of genetic material for which no role in pain has previously been disclosed, and which are useful, for example, in:
- identifying metabolic pathways for the transduction of pain
- identifying from said metabolic pathways compounds having utility in the diagnosis or treatment of pain
- producing proteins and polypeptides with a role in the transduction of pain;
- producing genetically modified non-human animals that are useful in the screening of compounds having utility in the treatment or diagnosis of pain.
- Identifying ligand molecules for said receptors involved in metabolic pathways and having utility in the treatment of pain.

It is yet a further object of the invention to provide molecules, non-human animals and microorganisms that can be used in screening compounds for pharmacological activity, especially pain-reducing activity.

In one aspect, the invention relates to the use in the screening of compounds that are effective in the treatment of pain, or in the diagnosis of pain, of:
(a) an isolated gene sequence that is up-regulated in the spinal cord of a mammal in response to streptozocin-induced diabetes;
(b) an isolated gene sequence having at least 80% sequence identity with any of the nucleic acid sequences of Tables I-X, preferably 85% sequence identity, more preferably 90%, increasingly preferably 95%, most preferably 99%.
(c) an isolated nucleic acid sequence comprising a sequence that is hybridizable to any of the gene sequences according to (a) and (b) under stringent hybridisation conditions;
(d) a recombinant vector comprising any one of the gene sequences according to (a), (b) or (c);
(e) a host cell containing the vector according to (d);
(f) a non-human animal, for use in the screening of compounds that are effective in the treatment of pain, or in the diagnosis of pain, having in its genome an introduced gene sequence or a removed or down-regulated nucleotide sequence that is an isolated gene sequence that is up-regulated in the spinal cord of a mammal in response to streptozocin-induced diabetes;
(g) an isolated polypeptide containing an amino acid sequence at least 90% identical to an amino acid sequence encoded by a nucleic acid or gene sequence according to any one of (a) to (d), or a variant polypeptide thereof with sequential amino acid deletions from the C terminus and/or the N-terminus; or
(h) an isolated antibody that binds specifically to the isolated polypeptide according to (g).

The invention also relates to the use of naturally occurring compounds such as peptide ligands of the expression products of the above gene sequences and their associated signal transduction pathways for the treatment of pain.

### DESCRIPTION OF PREFERRED EMBODIMENTS

### DEFINITIONS

Within the context of the present invention:
- "Comprising" means consisting of or including. Thus nucleic acid comprising a defined sequence includes nucleic acid that may contain a full-length gene or full-length cDNA. The gene may include any of the naturally occurring regulatory sequence(s), such as a transcription and translation start site, a promoter, a TATA box in the case of eukaryotes, and transcriptional and translational stop sites. Further, a nucleic acid sequence comprising a cDNA or gene may include any appropriate regulatory sequences for the efficient expression thereof *in vitro.*
- "Isolated" requires that the material be removed from its original environment (e.g. the natural environment if it is naturally occurring). For example, a naturally occurring polynucleotide or a peptide present in a living animal is not isolated, but the same polynucleotide or peptide, separated from some or all of the coexisting materials in the natural system, is isolated. Such polynucleotide can be part of a vector and/or such polynucleotide or peptide can be part of a composition, and still be isolated in that the vector or composition is not a part of its natural environment.
- "Purified" does not require absolute purity; instead it is intended as a relative definition. Purification of starting materials or natural materials from their native environment to at least one order of magnitude, preferably two or three orders, and more preferably four or five orders of magnitude is expressly contemplated.
   - "Nucleic acid sequence" or "gene sequence" means a sequence of nucleotides or any variant or homologue thereof, or truncated or extended sequence thereof, and is preferably indicated by a Genebank accession number. Also within the scope of the present invention are up-regulated nucleic acid sequences which encode expression products which are components of signaling pathways. This invention also includes any variant or homologue or truncated or extended sequence of the up-regulated nucleotide sequence. Also within the scope of the present invention, the term "nucleic acid(s) product", or "expression product" or "gene product" or a combination of these terms refers without being biased, to any, protein(s), polypeptide(s), peptide(s) or fragment(s) encoded by the up-regulated nucleotide sequence.

   - " Operably linked" refers to a linkage of polynucleotide elements in a functional relationship. For instance, a promoter or an enhancer is operably linked to a coding sequence if it regulates the transcription of the coding sequence. In particular, two DNA molecules (such as a polynucleotide containing a promoter region and a polynucleotide encoding a desired polypeptide) are said to be "operably linked" if the nature of the linkage between the two polynucleotides does not (1) result in the introduction of a frame-shift mutation and (2) interfere with the ability of the polynucleotide containing the promoter to direct the transcription of the coding polynucleotide.
   - "Pain" includes chronic pain and in particular diabetic pain.
   - "Stringent hybridization conditions" is a recognized term in the art and for a given nucleic acid sequence refers to those conditions which permit hybridization of that sequence to its complementary sequence and closely homologous sequences. Conditions of high stringency may be illustrated in relation to filter-bound DNA as for example. 2X SCC, 65°C (where SSC = 0.15M sodium chloride, 0.015M sodium citrate, pH 7.2), or as 0.5M NaHPO₄, 7% sodium dodecyl sulfate (SDS), 1mM EDTA, at 65°C, and washing in 0.1xSCC/0.1% SDS at 68°C (Ausubel F.M. *et al*., eds, 1989, *Current Protocols in Molecular Biology,* Vol. I, Green Publishing Associates, Inc., and John Wiley & Sons Inc., New York, at p. 2. 10.3). Hybridization conditions can be rendered highly stringent by raising the temperature and/or by the addition of increasing amounts of formamide, to destabilize the hybrid duplex of nonhomologous nucleic acid sequence relative to homologous and closely homologous nucleic acid sequences. Thus, particular hybridisation conditions can be readily manipulated, and will generally be chosen depending on the desired results.
   - "Variants or homologues" include (a) sequence variations of naturally existing gene(s) resulting from polymorphism(s), mutation(s), or other alteration(s) as compared to the above identified sequences, and which do not deprive the encoded protein of function (b) recombinant DNA molecules, such as cDNA molecules encoding genes indicated by the relevant Genebank accession numbers and (c) any sequence that hybridizes with the above nucleic acids under stringent conditions and encodes a functional protein or fragment thereof.

"Gene product" refers to polypeptide - which is interchangeable with the term protein - which is encoded by a nucleotide sequence and includes single-chain polypeptide molecules as well as multiple-polypeptide complexes where individual constituent polypeptides are linked by covalent or non-covalent means.
Polypeptides of the present invention may be produced by synthetic means (e.g. as described by Geysen *et al*., 1996) or by recombinant means.

The terms "variant", "homologue", "fragment", "analogue" or "derivative" in relation to the amino acid sequence for the polypeptide of the present invention includes any substitution of, variation of, modification of, replacement of, deletion of or addition of one (or more) amino acid from or to the sequence providing the resultant polypeptide has the native gene product activity. In particular, the term "homologue" covers homology with respect to structure and/or function. With respect to sequence homology, there is at least 90%, more preferably at least 95% homology to an amino acid sequence encoded by the relevant nucleotide sequence shown in Tables I - X, preferably there is at least 98% homology.

Typically, for the variant, homologue or fragment of the present invention, the types of amino acid substitutions that could be made should maintain the hydrophobicity/hydrophilicity of the amino acid sequence. Amino acid substitutions may include the use of non-naturally occurring amino acid analogues.

In addition, or in the alternative, the protein itself could be produced using chemical methods to synthesize a polypeptide, in whole or in part. For example, peptides can be synthesized by solid phase techniques, cleaved from the resin, and purified by preparative high performance liquid chromatography (e.g. Creighton (1983) Proteins Structures and Molecular Principles, WH Freeman and Co., New York, NY, USA). The composition of the synthetic peptides may be confirmed by amino acid analysis or sequencing (e.g. the Edman degradation procedure).

Direct peptide synthesis can be performed using various solid-phase techniques (Roberge JY *et al* Science Vol 269 1995 202-204) and automated synthesis may be achieved, for example, using the ABI 431 A Peptide Synthesizer (Perkin Elmer) in accordance with the instructions provided by the manufacturer. Additionally, the amino acid sequence of a gene product, or any part thereof, may be altered during direct synthesis and/or combined using chemical methods with a sequence from other subunits, or any part thereof, to produce a variant polypeptide.

In another embodiment of the invention, a gene product natural, modified or recombinant amino acid sequence may be ligated to a heterologous sequence to encode a fusion protein. For example, for screening of libraries for compounds and peptide agonists and antagonists of gene product activity, it may be useful to encode a chimeric gene product expressing a heterologous epitope that is recognised by a commercially available antibody. A fusion protein may also be engineered to contain a cleavage site located between a gene product sequence and the heterologous protein sequence, so that the gene product may be cleaved and purified away from the heterologous moiety.

The gene product may also be expressed as a recombinant protein with one or more additional polypeptide domains added to facilitate protein purification. Such purification facilitating domains include, but are not limited to, metal chelating peptides such as histidine-tryptophan modules that allow purification on immobilised metals (Porath J, Protein Expr Purif Vol 3 1992 p263-281), protein A domains that allow purification on immobilised immunoglobulin, and the domain utilised in the FLAGS extension/affinity purification system (Immunex Corp, Seattle, WA, USA). The inclusion of a cleavable linker sequence such as Factor XA or enterokinase (Invitrogen, San Diego, CA, USA) between the purification domain and the gene product is useful to facilitate purification.

### IDENTIFIED SEQUENCES

The inventors have identified nucleotide sequences that give rise to expression products listed in Tables I - X, that become differentially expressed in the spinal cord in response to streptozocin-induced diabetes and that are believed to be involved in the transduction of pain. In the Tables below, * denotes more preferred nucleic acid sequences and ** denotes most preferred nucleic acid sequences. These nucleic acid sequences have not previously been implicated in the transduction of pain.

The validity of the present experimental procedure was confirmed by the fact that nucleotide sequences were obtained as a result of the investigation whose function in the transduction of pain has been previously confirmed and established. These nucleic acid sequences are not part of this invention.. Any of the nucleic acid sequences and expression products can be used to develop screening technologies for the identification of novel molecules for the prevention or treatment of pain. These screening technologies could also be used to ascribe new pain therapeutic indications to molecules, which have not previously been ascribed for the prevention or treatment of pain. Furthermore, the said nucleic acid sequences can be used as diagnostic tools and for the development of diagnostic tools.

The sequences of the invention have been identified as up-regulated in the spinal cord in response to streptozocin-induced diabetes. They have not been identified as up-regulated in an experiment involving chronic constrictive injury of the sciatic nerve.

**Table I -**

| **Sequences whose expression products are kinases** | | | | | |
|---|---|---|---|---|---|
| **Expression product or name (Reference)** | **Rat Accession Number** | **Mouse accession number** | **Human Accession Number** | **Reaction** | **Assay** |
| Janus protein tyrosine kinase 1 (JAK1) (Ref: N/A) | AJ000556 (SEQ ID No's 1 - 2) | 563728 | | Non-receptor Tyr kinase | kinase |
| Phosphatidylinositol 4-kinase Ref : 1) | D84667 (SEQ ID No's 3-4) | | | Cell signalling | Phospholipid kinase |
| Rho kinase Alpha (Ref: 2) | U38481 SEQ ID NO's 5-6) | U58513 | D87931 | Cell signalling | Ser/Thr kinase; reorganization of the cytoskeleton. |
| Casein kinase II beta subunit * (Ref: 3) | L15619 (SEQ ID No's 7-8) | | | Cell signalling | Ser/Thr kinase; cell growth and proliferation |
| ERK3, protein serine/threonine kinase, extracellular * (Ref: 4) | M64301 (SEQ ID NO's 9 10) | | X80692 | MAP kinase pathway | Ser/Thr kinase; mediation of response to extracellular signals |
| Neural receptor protein-tyrosine kinase (trkB) * (Ref: 5) | M55291 (SEQ ID NO's 11 12) | X17647 | | Tyrosine kinase receptor | kinase |

**Table II -**

| **Sequences whose expression products are phosphatases** | | | | | |
|---|---|---|---|---|---|
| **Expression product or name (Reference)** | **Rat accession number** | **Mouse accession number** | **Human Accession Number** | **Reaction** | **Assay** |
| Protein tyrosine phosphatase * (Ref: 6) | AJ007016 (SEQ ID No's 13-14) | AF035644 | U48297 | N/A | Phosphatase |
| Putative receptor tyrosine phosphatase * (REF : N/A) | U55057 (SEQ ID NO's 15-16) | | | N/A | Receptor Tyr |
| Calcineurin, A subunit, beta isoform * (Ref: 7) | M31809 (SEQ ID NO's 17-18) | | M29550 | N/A | N/A |

**Table III -**

| **Sequences whose expression products are phosphodiesterases** | | | | |
|---|---|---|---|---|
| **Expression product or name (Reference)** | **RAT ACCESSION NUMBER** | **MOUSE ACCESSION NUMBER** | **HUMAN ACCESSION NUMBER** | **Reaction** |
| Phosphodiesterase, cAMP-specific (PDE4D) ** (Ref: 8) | L27058 (SEQ ID No's 19-20) | | L20966 | Cell signalling; homolog of the dnc learning and memory gene |

**Table IV -**

| **Sequences whose expression products are receptors** | | | | | |
|---|---|---|---|---|---|
| **Expression Product or Name (Ref)** | **Rat Accession Number** | **Mouse accession Number** | **Human Accession Number** | **Reaction** | **Assay** |
| Neurotensin | X97121 | | | Recognises | N/A |
| receptor type 2 (NTR2) (Ref: 9) | (SEQ ID No's 21 22) | | | neurotensin and levocabastine | |
| Putative interleukin 1 receptor accessory protein ** (Ref : 10) | | X85999 (SEQ ID No's 23-24) | | N/A | IL-1 |

**Table V -**

| **Sequences whose expression products are transporters** | | | | | |
|---|---|---|---|---|---|
| **Expression Product or Name (Ref)** | **Rat Accession Number** | **Mouse Accession Number** | **Human Accession Number** | **Reaction** | **Assay** |
| Amino acid system A transporter (Ref: 11) | AF249673 (SEQ ID No's 25-26) | | | Amino acid uptake | Transporter |
| Digoxin carrier protein (Ref: 12) | U88036 (SEQ ID No's 27-28) | | | Organic anion transporter | Transporter; brain ; digoxin and cardiac glycosides |
| Synaptic vesicle transmembrane transporter (Ref: 13) | L01788 (SEQ ID No 29) | | | Transmembrane transport | Transporter |
| Glycine transporter ** (Ref: 14) | L13600 (SEQ ID No's 30-31) | | | Neurotransmitter transport | Transporter assay (glycine) |
| Putative SEDL spondyloepiphesial dysplasia tarda gene (Ref: 15) | AF157065 | | AF157065 (SEQ ID No's 32-33) | Vesicle traficking | transporter |
| Delta aminolevulinate synthase, erythroid-specific ** (Ref : 16) | D86297 (SEQ ID No's 34-35) | | | | |
| Polypeptide GalNAc transferase T1 ** (Ref: 17) | U35890 (SEQ ID No's 36-37) | | | | |
| Putative NAD+ ADP-ribosyltransferase. **(Ref : 18) | | AJ007780 (SEQ ID No's 38-39) | | | |

**Table VI -**

| **Sequences whose expression products are G-protein coupled receptor proteins** | | | | | |
|---|---|---|---|---|---|
| **Expression product or Name (Ref)** | **Rat Accession Number** | **Mouse Accession Number** | **Human Accession Number** | **Reaction** | **Assay** |
| Putative K-ras (Ref: 19) | | | M54968 (SEQ ID No's 40-41) | Cell signalling | N/A |
| ARF-like protein 5 ARL5 * (Ref: 20) | RAS-related | | X78604 (SEQ ID No's 42-43) | Membrane traficking | N/A |

**Table VII -**

| **Sequences whose expression products are DNA-binding proteins** | | | | | |
|---|---|---|---|---|---|
| **Expression Product or Name (Ref)** | **Rat Accession Number** | **Mouse Accession Number** | **Human Accession Number** | **Reaction** | **Assay** |
| TBP-binding protein TIP 120 (Ref: 21) | D87671 (SEQ ID No's 44-45) | D87671 | | DNA replication | N/A |
| Putative chromodomain helicase-DNA binding protein (Ref: 22) | AF054177 (SEQ ID No's 46-47) | | | Chromatin structural protein | Helicase |
| Putative origin recognition complex subunit 4 : Orc 4 gene (Ref: 23) | | AJ003140 (SEQ ID No's 48-49) | | DNA replication | N/A |

**Table VIII -**

| **Sequences whose expression products are proteases** | | | | | |
|---|---|---|---|---|---|
| **Expression Product or Name (Ref)** | **Rat Accession Number** | **Mouse Accession Number** | **Human Accession Number** | **Reaction** | **Assay** |
| Carboxypeptidase D precursor (Ref: 24) | U62897 (SEQ ID No's 50-51) | D85391 | U65090 | Pre-hormone metallocarboxy peptidase | Protease |
| Caspase 2 (Ich-1) (Ref: 25) | U77933 (SEQ ID No's 52-53) | | | Apoptotic protein | protease |
| Cathepsin B (Ref: 26) | X82396 (SEQ UD No's 54-55) | M14222 | | Thiol protease | protease |
| Cathepsin L (EC 34.22.15) (Ref: 27) | Y00697 (SEQ ID No's 56-57) | M20495 | | Cysteine protease | protease |
| Dipeptidyl-peptidase (dpp6) | M76427 (SEQ ID | AF092507 | M96850 | Inactive aminopeptidase | N/A |
| (Ref: 28) | No's 58-59) | | | family member | |
| Proteasome subunit C8 (Ref: 29) | M58593 (SEQ ID No's 60-61) | AF055983 | D00762 | Protein turnover | Endoprotease |
| Putative deubiquitinating enzyme 8 (Ref: 30) | D29956 | AF057146 (Seq ID No's 62-63) | D29956 | Protein turnover | N/A |

**Table IX -**

| **Sequences whose expression products are other enzymes** | | | | | |
|---|---|---|---|---|---|
| **Expression product or Name (Ref)** | **Rat Accession Number** | **Mouse Accession Number** | **Human Accession Number** | **Reaction** | **Assay** |
| Putative ubiquitin-conjugating enzyme (EC 6.3.2.-) E2 (Ref: 31) | | | E12457 (SEQ ID No 64) | Ubiquination | Ligase |
| Putative tryptophanyl-tRNA synthetase (Ref: 32) | | X69656 (SEQ ID No's 65-66) | | Protein systhesis | Ligase |
| Aldolase A (Ref 33) | M12919 (SEQ ID No's 67-68) | Y00516 | M11560 | Glycolysis | Lyase |
| 3-Hydroxy-3-methylglutaryl coenzyme A reductase (Ref : 34) | M29249 (SEQ ID NO's 69-70) | | M11058 | N/A | Oxidoreductase |
| NADH subunit 5, mitochondrial (Ref: N/A) | X14848 (SEQ ID No 71) | | | Energy metabolism | Oxidoreductase |
| Electron transfer flavoprotein (ETF), alpha subunit (Ref: 35) | M22030 (SEQ ID No's 72-73) | | | Energy metabolism | Oxidoreductase |
| Delta-amino levulinate synthase, erythroid-specific ** (Ref: 36) | D86297 (SEQ ID No's 74-75) | | | Porphyrin biosysthesis | Transferase |
| Polypeptide GalNAc transferase T1 * (Ref: 37) | U35890 (SEQ ID No's 76-77) | | | Protein glycosylation | Transferase |
| Arginase II (Ref: 38) | U90887 SEQ ID NO's 78-79) | U90886 | | NO synthesis | Hydrolase |
| ATP synthase gamma chain, mitochondrial (REF: 39) | L19927 (SEQ ID NO 80-81) | | D16563 | Energy metabolism | Hydrolase |
| N-G,N-G-dimethylarginine dimethylamino-hydrolase (Ref 40) | D86041 (SEQ ID No's 82-83) | | | NO metabolism | Hydrolase |
| Putative gluco-cerebrosidase (Ref: 41) | | M24119 (SEQ ID NO's 84-85) | | N/A | Hydrolase |
| Putative N-acetyl-glucosamine-6-sulfatase (Ref: 42) | | | Z12173 (SEQ ID NO's 86-87) | N/A | Hydrolase |

**Table X -**

| **Other sequences** | | | | |
|---|---|---|---|---|
| **Expression Product or Name (Ref)** | **Rat Accession Number** | **Mouse Accession Number** | **Human Accession Number** | **Reaction** |
| 12S rRNA (Ref: 43) | J01438 (SEQ ID No 88) | S63167 | | Protein synthesis |
| Ischaemia responsive 94kD protein (Ref: 44) | AF077354 (Seq ID No's 89-90) | AF077354 | | Stress response |
| Putative cops2 signalosome component (Ref: 45) | AF071312 (SEQ ID No's 91-92) | | | Protein turnover |
| rS-Rex-s, human NSP C homolog (Ref: 46) | U17603 (SEQ ID No's 93-94) | | | Unknown |
| Rat haemoglobin beta-chain (Ref: 47) | M27169 (SEQ ID No 95) | | | Haemoglobin |
| PMSG-induced ovarian mRNA (Ref: N/A) | D84485 (SEQ ID No 96) | | | Unknown |
| TBP binding protein TIP 120 (Ref: 48) | D87671 (SEQ ID No's 97-98) | D87671 | | DNA replication |
| Chromosone region maintenance 1 protein ; CRM1 (Ref: 49) | AJ238278 (SEQ ID No's 99-100) | | | Promotes nuclear export of proteins |
| SGC10 (Ref 50) | | L20263 (Seq. ID No 101) | | Unknown |
| Myosin heavy chain (Ref: 51) | S68736 (Seq ID No's 102-103) | | | Cytoskeleton |
| Putative KIAA1157 (Ref: 52) | AB032983 (SEQ ID NO's 104-105) | | AB032983 | Unknown |
| Putative Shyc (Ref: 53) | | AF072697 (SEQ ID No's 106-107) | | Unknown |
| Microtubule associated protein MAP2 (Ref : 54) | U30938 (Seq ID No 108) | | | Cytoskeleton/ cellular transport |
| Putative chromatin structure regulator, related to human SUPT6H (U46691) (Ref: 55) | U40375 (SEQ ID NO's 109-110) | | | Structural protein |
| Putative ZPR1 zinc finger protein (Ref: 56) | | U41287 (SEQ ID No's 111-112) | | Unknown |
| Putative KIAA0627 (Ref: 57) | AB014527 (SEQ ID No's 113-114) | | | Unknown |
| Statin-related protein (REF NO : 58) | M62751 (SEQ ID NO's 115-116) | M62751 | X70940 | Protein synthesis |
| Dyactin subunit p62 (Ref: 59) | AF190798 (SEQ ID NO's 117-118) | AF190798 | | Cytoskeleton |
| Phosphacan (Ref: 60) | U04998 (SEQ ID No's 119-120) | | | Extracellular interacting protein |
| Putative endomembrane protein emp70 isolog (Ref: N/A) | AF160213 | | AF160213 (SEQ ID No's 121-122) | Unknowm |
| Putative iswi homologue Ref: 61 | L27127 (Drosophilia) (SEQ ID NO's 123-124) | | | Chromatin structure |
| Putative sorting nexin 6 (Ref: 62) | AF121856 | | AF121856 (SEQ ID No's 125-126) | Protein turnover |
| Putative profilin II (REF: 63) | L10678 | | L10678 (SEQ ID NO's 127-128) | Cytoskeleton protein |
| Putative testican (Ref: 64) | | MMTCAN X92864 (SEQ ID NO's 129-130) | | Extracellular interactions |
| Putative p53 responsive EI24 (Ref: 65) | | U41751 (SEQ ID NO's 131-132) | | p53 induced apoptosis |
| Matrin 3 (Ref: 66) | M63485 (SEQ ID NO's 133-134) | M63485 | | Nuclear matrix |
| Putative KIAA0287 (Ref: N/A) | AB006625 | | AB006625 (SEQ ID NO's 135-136) | Unknown |
| Putative Rho GDP dissociation inhibitor GDI (Ref : 67) | | L42463 (SEQ ID No's 137-138) | | Cell signalling |
| Putative gene for motor protein (Ref: N/A) | | | D21094 (SEQ ID No's 139-140) | Unknown |
| Putative phosohatidyl | | U96725 | | IP3 second |
| inositol transfer protein alpha (Ref: 68) | | (SEQ ID No's 141-142) | | messenger production |
| Putative centrin (Cetn2) (Ref: 69) | | AF080592 (SEQ ID No's 143-144) | | Mitosis-centrosome association |
| Beta-tubulin (Ref: 70) | X03369 (SEQ ID No's 145-146) | | | Cytoskeleton |
| Putative formin binding protein (Ref: 71) | | U40751 (SEQ ID NO's 147-148) | | Unknown |
| Putative NY-REN-58 antigen (Ref: 72) | | | AF155115 (SEQ ID No's 149-150) | Unknown |
| Putative radixin (Ref: N/A) | | X60672 (SEQ ID No's 151-152) | | Cytoskeleton protein |
| Splicing fctor 1 homolog (Ref: N/A) | AF079873 (SEQ ID No's 153-154) | AF079873 | | |
| SH3-containing protein p4015 (Ref: 73) | AF026505 (SEQ ID No's 155-156) | AF026505 | | Unknown |
| Putative sperm specific protein (Ref: 74) | | | X91879 (SEQ ID No's 157-158) | Unknown |
| Guanine-nucleotide-releasing protein (mss4) (Ref: N/A) | L10336 (SEQ IS No's 159-160) | | | |
| Intracellular calcium-binding protein MRP14 (Ref: 75) | L18948 (SEQ ID No's 161-162) | L18948 | | Has been implicated in susceptibility to induced chronic disease (arthritis) |
| PTB-like protein (Ref: N/A) | AJ010585 (SEQ ID NO's 163-164) | AJ010585 | AF176085 | Cell signalling |
| Putative 26S proteasome subunit CADp44 (Ref: 76) | U36395* (SEQ ID No's 165-166) | | | Protein turnover |
| Putative BM-020 (Ref: 77) | AF208862 | | AF208862 (SEQ ID No's 167-168) | Unknown |
| Putative bullous pemphigoid antigen 1 (Ref: 78) | L11690 | | L11690 (SEQ ID No's 169-170) | Cell adhesion |
| Putative coatomer protein γ-2-COP (Ref: 79) | AF205065 (Seq ID No's 171-172) | | AF157833 | Vesicle coat protein |
| Putative F-box protein Fbx21 (Ref: 80) | AF174601 | | AF174601 (SEQ ID No's 173-174) | Unknown |
| pUTATIVE kiaa0235 (Ref: 81) | D87078 | | D87078 (SEQ ID No's 175-176) | Unknown |
| Putative KIAA 1212 (Ref: 82) | | | AB033308 (SEQ ID No's 177-178) | Unknown |
| Putative osteoglycin (Ref: 83) | | D31951 (SEQ ID No's 179-180) | | Bone formation |
| Putative RNA binding protein related to Sam68 (Ref: N/A) | | | AF051321 (SEQ ID NO's 181-182) | Cell signalling |
| Ribosomal protein 11 (Ref: 84) | X62146 (SEQ ID No's 183-184) | | | Protein synthesis |
| Tspan-2 (Ref: 85) | AJ271442 (SEQ ID NO's 185-186) | | | Plasma membrane protein; signalling in oligodendrocytes |
| AMP deaminase isoform C (Ref: 86) | U90888 (SEQ ID No's 187-188) | | | Energy metabolism |
| Globin, α, major (Ref: 87) | M17083 (SEQ ID No's 189-190) | | | haemoglobin |
| Cytochrome b, mitochrondrial (Ref: 88) | X14848 Y17319 (SEQ ID NO 191) | | | Energy metabolism |
| Putative BB 1 progression enhanced gene (Ref: 89) | S82470 (SEQ ID NO's 192-193) | | | Unknown |
| Putative CGI-21 (Ref: 90) | AF132955 (SEQ ID No's 194-195) | | | Unknown |
| Myelin proteolipid brain PLP (Ref: 91) | M11185 (SEQ ID NO's 196-197) | | | Brain proteolipid protein |

### PRODUCTION OF POLYPEPTIDES AND NUCLEIC ACIDS

### Vectors

Recombinant expression vectors comprising a nucleic acid can be employed to express any of the nucleic acid sequences of the invention. The expression products derived from such vector constructs can be used to develop screening technologies for the identification of molecules that can be used to prevent or treat pain, and in the development of diagnostic tools for the identification and characterization of pain. The expression vectors may also be used for constructing transgenic non-human animals.

Gene expression requires that appropriate signals be provided in the vectors, said signals including various regulatory elements such as enhancers/promoters from viral and/or mammalian sources that drive expression of the genes of interest in host cells. The regulatory sequences of the expression vectors used in the invention are operably linked to the nucleic acid sequences encoding the pain-associated proteins of interest or a peptide fragment thereof.

Generally, recombinant expression vectors include origins of replication, selectable markers, and a promoter derived from a highly expressed gene to direct transcription of a downstream nucleotide sequence. The heterologous nucleotide sequence is assembled in an appropriate frame with the translation, initiation and termination sequences, and if applicable a leader sequence direct the expression product into the periplasmic space, the extra-cellular medium or cell membrane.

In a specific embodiment wherein the vector is adapted for expressing desired sequences in mammalian host cells, preferred vectors will comprise an origin of replication from the desired host, a suitable promoter and an enhancer, and also any necessary ribosome binding sites, polyadenylation site, transcriptional termination sequences, and optionally 5'-flanking non-transcribed sequences. DNA sequences derived from the SV40 or CMV viral genomes, for example SV 40 or CMV origin, early promoters, enhancers, and polyadenylation sites may be used to provide the required non-transcribed genetic elements.

A recombinant expression vector used in the invention advantageously also comprises an untranscribed polynucleotide region located at the 3'end of the coding sequence (ORF), this 3'-UTR (untranslated region) polynucleotide being useful for stabilizing the corresponding mRNA or for increasing the expression rate of the vector insert if this 3'-UTR harbours regulation signal elements such as enhancer sequences.

Suitable promoter regions used in the expression vectors are chosen taking into account the host cell in which the nucleic acid sequence is to be expressed. A suitable promoter may be heterologous with respect to the nucleic acid sequence for which it controls the expression, or alternatively can be endogenous to the native polynucleotide containing the coding sequence to be expressed. Additionally, the promoter is generally heterologous with respect to the recombinant vector sequences within which the construct promoter/coding sequence has been inserted. Preferred promoters are the LacI, LacZ, T3 or T7 bacteriophage RNA polymerase promoters, the lambda PR, PL and Trp promoters (EP-0 036 776), the polyhedrin promoter, or the p10 protein promoter from *baculovirus* (kit Novagen; Smith *et al*., (1983); O'Reilly *et al.* (1992).

Preferred selectable marker genes contained in the expression recombinant vectors used in the invention for selection of transformed host cells are preferably dehydrofolate reductase or neomycin resistance for eukaryotic cell culture, TRP1 for *S. cerevisiae* or tetracycline, rifampicin or ampicillin resistance in *E. coli,* or Levamsaccharase for *Mycobacteria*, this latter marker being a negative selection marker.

Preferred bacterial vectors are listed hereafter as illustrative but not limitative examples: pQE70, pQE60, pQE-9 (Quiagen), pD10, phagescript, psiX174, p.Bluescript SK, pNH8A, pNH16A, pNH18A, pNH46A (Stratagene); pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIA express).

Preferred bacteriophage recombinant vectors of the invention are P1 bacteriophage vectors such as described by Sternberg N.L. (1992;1994).

A suitable vector for the expression of any of the pain associated polypeptides used in the invention or fragments thereof, is a baculovirus vector that can be propagated in insect cells and in insect cell-lines. A specific suitable host vector system is the pVL 1392/1393 *baculovirus* transfer vector (Pharmingen) that is used to transfect the SF9 cell line (ATCC N°CRL 1711) that is derived from *Spodoptera frugiperda.*

The recombinant expression vectors of the invention may also be derived from an adenovirus. Suitable adenoviruses are described by Feldman and Steig (1996) or Ohno *et al.* (1994). Another preferred recombinant adenovirus is the human adenovirus type two or five (Ad 2 or Ad 5) or an adenovirus of animal origin (Patent Application WO 94/26914)

Particularly preferred retrovirus for the preparation or construction of retroviral *in vitro* or *in vivo* gene delivery vehicles include retroviruses selected from the group consisting of Mink-Cell Focus Inducing Virus, Murine Sarcoma Virus, and Ross Sarcoma Virus. Other preferred retroviral vectors are those described in Roth *et al.* (1996), in PCT Application WO 93/25234, in PCT Application WO 94/06920, and also in Roux *et al.* (1989), Julan *et al.*(1992) and Nada *et al.* (1991).

Yet, another viral vector system that is contemplated is the Adeno Associated Viruses (AAV) such as those described by Flotte *et al.* (1992), Samulski *et al.* (1989) and McLaughlin *et al.* (1996).

### Host cells expressing pain associated polypeptides

Host cells that endogenously express pain associated polypeptides or have been transformed or transfected with one of the nucleic acid sequences described herein, or with one of the recombinant vector described above, particularly a recombinant expression vector, can be used in the present invention. Also included are host cells that are transformed (prokaryotic cells) or are transfected (eukaryotic cells) with a recombinant vector such as one of those described above.

Preferred host cells used as recipients for the expression vectors used in the invention are the following:
(a) prokaryotic host cells: *Escherichia coli,* strains. (i.e. DH5-α, strain) *Bacillus subtilis*, *Salmonella typhimurium* and strains from species like *Pseudomonas*, *Streptomyces* and *Staphylococcus* for the expression of up and down-regulated nucleic acid sequences modulated by pain, characterized by having at least 80% sequence identity with any of the nucleic acid sequences of Tables I - X. Plasmid propagation in these host cells can provide plasmids for transfecting other cells.
(b) eukaryotic host cells: HeLa cells (ATCC N°CCL2; N°CCL2.1; N°CCL2.2), Cv 1 cells (ATCC N°CCL70), COS cells (ATCC N°CRL 1650; N°CRL 1651), Sf-9 cells (ATCC N°CRL 1711), C127 cells (ATCC N°CRL-1804), 3T3 cells (ATCC N°CRL-6361), CHO cells (ATCC N°CCL-61), human kidney 293 cells (ATCC N° 45504; N°CRL-1573), BHK (ECACC N°84100 501; N°84111301), PC12 (ATCC N° CRL-1721), NT2, SHSY5Y (ATCC N° CRL-2266), NG108 (ECACC N°88112302) and F11, SK-N-SH (ATCC N° CRL-HTB-11), SK-N-BE(2) (ATCC N° CRL-2271), IMR-32 (ATCC N° CCL-127). A preferred system to which the nucleic acids of the invention can be expressed are neuronal cell lines such as PC12, NT2, SHSY5Y, NG108 and F11, SK-N-SH, SK-N-BE(2), IMR-32 cell lines, COS cells, 3T3 cells, HeLa cells, 292 cells and CHO cells. The above cell lines could be used for the expression of any of the nucleic acid sequences of Tables I - X.

When a nucleic acid sequence of any of Tables I - X is expressed using a neuronal cell line, the sequence can be expressed through an endogenous promoter or native neuronal promoter, or an exogenous promoter. Suitable exogenous promoters include SV40 and CMV and eukaryotic promoters such as the tetracycline promoter. The preferred promoter when pain associated molecules are endogenously expressed is an endogenous promoter. A preferred promoter in a recombinant cell line is the CMV promoter.

In a specific embodiment of the host cells described above, these host cells have also been transfected or transformed with a polynucleotide or a recombinant vector for the expression of a natural ligand of any of the nucleic acid sequences of Tables I - X or a modulator of these expression products.

### PROTEINS, POLYPEPTIDES AND FRAGMENTS

The expression products of the nucleic acid sequences of Tables I - X or fragment(s) thereof can be prepared using recombinant technology, cell lines or chemical synthesis. Recombinant methods, chemical methods or chemical synthetic methods can be used to modify a gene in order to introduce into the gene product, or a fragment of the gene product, features such as recognition tags, cleavage sites or other modifications. For efficient polypeptide production, the endogenous expression system or recombinant expression system should allow the expression products to be expressed in a manner that will allow the production of a functional protein or fragment thereof which can be purified. Preferred cell lines are those that allow high levels of expression of polypeptide or fragments thereof. For efficient polypeptide production, the endogenous expression system or recombinant expression system should allow the expression products to be expressed in a manner that will allows the production of a functional protein or fragment thereof which can be purified. Preferred cell lines are those that allow high levels of expression of polypeptide or fragments thereof. Such cell lines include cell lines which naturally express any of the nucleic acid sequences of Tables I, II, or III or common mammalian cell lines such as CHO cells or COS cells, etc, or more specific neuronal cell lines such as PC12. However, other cell types that are commonly used for recombinant protein production such as insect cells, amphibian cells such as oocytes, yeast and prokaryotic cell lines such as *E. coli* can also be used.

The expression products of Tables I - X or fragments thereof can be utilized in screens to identify potential therapeutic ligands, either as a purified protein, as a protein chimera such as those produced by phage display, as a cell membrane (lipid or detergent) preparation, or in intact cells.

The invention also relates generally to the use of proteins, peptides and peptide fragments for the development of screening technologies for the identification of molecules for the prevention or treatment of pain, and the development of diagnostic tools for the identification and characterization of pain. These peptides include expression products of the nucleic acid sequences of Tables I - X and purified or isolated polypeptides or fragments thereof having at least 90%, preferably 95%, more preferably 98% and most preferably 99% sequence identity with the any of the expression products of nucleic acid sequences of Tables I - X. Expressed peptides and fragments of any of these nucleic acid sequences can be used to develop screening technologies for the identification of novel molecules for the prevention or treatment of pain. These screening technologies could also be used to ascribe new pain therapeutic indications to molecules, which have not previously been ascribed for the prevention or treatment of pain. Furthermore the said expressed peptides and fragments can be used as diagnostic tools and for the development of diagnostic tools.

### SCREENING METHODS

As discussed above, we have identified nucleic acid sequences whose expression is regulated by pain, particularly chronic pain and more particularly diabetic pain. The expression products of these nucleic acids can be used for screening ligand molecules for their ability to prevent or treat pain, and particularly, but not exclusively, chronic pain. The main types of screens that can be used are described below. The test compound can be a peptide, protein or chemical entity, either alone or in combination(s), or in a mixture with any substance. The test compound may even represent a library of compounds.

The expression products of any of the nucleic acid sequences of Tables I - X or fragments thereof can be utilized in a ligand binding screen format, a functional screen format or in vivo format. Examples of screening formats are provided.

### A) Ligand binding screen

In ligand binding screening a test compound is contacted with an expression product of one of the sequences of Tables I - X, and the ability of said test compound to interact with said expression product is determined, e.g. the ability of the test compound(s) to bind to the expression product is determined. The expression product can be a part of an intact cell, lipidic preparation or a purified polypeptide(s), optionally attached to a support, such as beads, a column or a plate etc.

Binding of the test compound is preferably performed in the presence of a ligand to allow an assessment of the binding activity of each test compound. The ligand may be contacted with the expression product either before, simultaneously or after the test compound. The ligand should be detectable and/or quantifiable. To achieve this, the ligand can be labelled in a number of ways, for example with a chromophore, radioactive, fluorescent, phosphorescent, enzymatic or antibody label. Methods of labelling are known to those in the art. If the ligand is not directly detectable it should be amenable to detection and quantification by secondary detection, which may employ the above technologies. Alternatively the expression product or fragment thereof can be detectable or quantifiable. This can be achieved in a similar manner to that described above.

Binding of the test compound modifies the interaction of the ligand with its binding site and changes the affinity or binding of the ligand for/to its binding site. The difference between the observed amount of ligand bound relative to the theoretical maximum amount of ligand bound (or to the ligand bound in the absence of a test compound under the same conditions) is a reflection of the binding ability (and optionally the amount and/or affinity) of a test compound to bind the expression product.

Alternatively, the amount of test compound bound to the expression product can be determined by a combination of chromatography and spectroscopy. This can be achieved with technologies such as Biacore (Amersham Pharmacia). The amount of test compound bound to the expression product can also be determined by direct measurement of the change in mass upon compound or ligand binding to the expression product. Alternatively, the expression product, compound or ligand can be fluorescently labelled and the association of expression product with the test compound can be followed by changes in Fluorescence Energy Transfer (FRET).

The invention therefore includes a method of screening for pain alleviating compounds, comprising:
a) contacting a test compound or test compounds in the presence of a ligand with an expression product of any of the above nucleic acid sequences of Tables I - X or with a cell expressing at least one copy of the expression product or with a lipidic matrix containing at least one copy of the expression product;
b) determining the binding of the test compound to the expression product, and
c) selecting test compounds on the basis of their binding abilities.

In the above method, the ligand may be added prior to, simultaneously with or after contact of the test compound with the expression product. Non limiting examples and methodology can be gained from the teachings of the *Molecular Probes handbook* and references therein (Molecular Probes, Inc., 4849 Pitchford Ave, Eugene, USA), *Methods in neurotransmitter receptor analysis* (Yamamura HI., Enna, SJ., and Kuhar, MJ., Raven Press New York, the *Glaxo Pocket Guide to Pharmacology,* Dr. Michael Sheehan, Glaxo Group Research Ltd, Ware, Herts SG12 0DP), *Bylund DB and Murrin LC* (2000, Life Sciences, 67 (24) 2897-911), *Owicki JC* (*2000*, *J. biomol Screen (5) 297-306), Alberts et al (1994*, *Molecular Biology of the Cell*, *3*^{*rd*} *Edn*, *Garland Publication Inc)*, *Butler JE*, *(2000 Methods 22(1):4-23*, *Sanberg SA* (2000, Curr Opin Biotechnol 11(1) 47-53), and *Christopoulos A* (1999, biochem Pharmacol 58(5) 735-48).

### B) Functional screening

### (a) Kinase assays

The expression products of any of the nucleic acid sequences of Tables I - X which encodes a kinase, and in particular the nucleic acid sequences listed in Table I, are amenable to screening using kinase assay technology.

Kinases have the ability to add phosphate molecules to specific residues in ligands such as binding peptides in the presence of a substrate such as adenosine triphosphate (ATP). Formation of a complex between the kinase, the ligand and substrate results in the transfer of a phosphate group from the substrate to the ligand. Compounds that modulate the activity of the kinase can be determined with a kinase functional screen. Functional screening for modulators of kinase activity therefore involves contacting one or more test compounds with an expression product of one of the nucleic acid sequences of Tables I-X which encodes a kinase, and determining the ability of said test compound to modulate the transfer of a phosphate group from the substrate to the ligand.

The expression product can be part of an intact cell or of a lipidic preparation or it can be a purified polypeptide(s), optionally attached to a support, for example beads, a column, or a plate. Binding is preferably performed in the presence of ligand and substrate to allow an assessment of the binding activity of each test compound.

The ligand should contain a specific kinase recognition sequence and it should not be phosphorylated at its phosphoryation site. The ligand and/or substrate may be contacted with the kinase either before, simultaneously or after the test compound. Optionally the substrate may be labelled with a kinase transferable labelled phosphate. The assay is monitored by the phosphorylation state of the substrate and/or the ligand. The ligand should be such that its phosphorylation state can be determined. An alternative method to do this is to label the ligand with a phosphorylation-state-sensitive molecule. To achieve this, the ligand can be labelled in a number of ways, for example with a chromophore, radioactive, fluorescent, phosphorescent, enzymatic or antibody label. If the ligand is not directly detectable it should be amenable to detection and quantification by secondary detection, which may employ the above technologies. Such technologies are known to those in the art.

Binding of the test compound to the kinase modifies its ability to transfer a phosphate group from the substrate to the ligand. The difference between the observed amount of phosphate transfer relative to the theoretical maximum amount of phosphate transfer is a reflection of the modulatory effect of the test compound. Alternatively, the degree of phosphate transfer can be determined by a combination of chromatography and spectroscopy. The extent of phosphorylation of the ligand peptide or dephosphorylation of the substrate can also be determined by direct measurement. This can be achieved with technologies such as Biacore (Amersham Pharmacia).

The invention also provides a method for screening compounds for the ability to relieve pain, which comprises:
(a) contacting one or more test compounds in the presence of ligand and substrate with an expression product of any of the nucleotide sequences set out in Tables I - X which is a kinase or with a cell containing at least 1 copy of the expression product or with a lipidic matrix containing at least 1 copy of an expression product;
(b) determining the amount of phosphate transfer from the substrate to the ligand; and
(c) selecting test compounds on the basis of their capacity to modulate phosphate transfer.

Optionally ligand, substrate and/or other essential molecules may be added prior to contacting the test compound with expression product of step (a) or after step (a). Non limiting examples and methodology can be gained from the teachings of the *Molecular Probes handbook* and references therein (Molecular Probes, Inc., 4849 Pitchford Ave, Eugene, USA), *Methods in Molecular Biology 2000*; *99:* 191-201, *Oncogene* 2000 20; 19(49): 5690-701, and *FASAB Journal,* (10, 6, P55, P1458, 1996, Pocius D Amrein K *et al*).

### b) Phosphatase assays

The expression products of any of the nucleic acid sequences of Tables I - X which encodes a phosphatase, and in particular the nucleic acid sequences listed in Table II, are amenable to screening using phosphatase assay technology.

Phosphatase enzymes have the ability to remove phosphate molecules from specific residues in ligands such as peptides. This reaction takes place in the presence of a substrate such as Adenosine Diphosphate (ADP). The complexing of the phosphatase polypeptide, ligand and substrate results in the transfer of a phosphate group from the ligand to substrate. Compounds that modulate the activity of the Phosphatase can be determined with a Phosphatase functional screen. This screen detects the reverse of the Kinase functional screen outlined above.

The invention also provides a method for screening compounds for the ability to relieve pain, which comprises:
(a) contacting one or more test compounds in the presence of ligand and substrate with an expression product of any of the nucleotide sequences of Tables I - X which is a phosphatase or with a cell containing at least 1 copy of the expression product or with a lipidic matrix containing at least 1 copy of an expression product;
(b) determining the amount of phosphate transfer from the ligand to the substrate; and
(c) selecting test compounds on the basis of their capacity to modulate phosphate transfer.

Optionally ligand, substrate and/or other essential molecules may be added prior to contacting the test compound with expression product of step (a) or after step (a). Non-limiting examples and methodology can be gained from the teachings of the *Molecular Probes handbook* and references therein (Molecular Probes, Inc., 4849 Pitchford Ave, Eugene, USA), and *FASAB Journal,* (10, 6, P55, P1458, 1996, Pocius D Amrein K *et al*).

### c) Phosphodiesterase assays

An expression product of any of the nucleic acid sequences of Tables I - X which encodes a phosphodiesterase, and in particular any of the nucleic acid sequences listed in Table III, is amenable to screening using phosphodiesterase assay technology.

Phosphodiesterases have the ability to cleave cyclic nucleotides cAMP (cyclic adenosine monophosphate) and/or cGMP (cyclic guanosine monophosphate) (substrate) at their 3'phosphatase bond to form 5'AMP and 5'GMP. Functional screening for modulators of phosphodiesterase polypeptide comprises contacting one or more test compounds with an expression product as set out above which is a phosphodiesterase and determining the ability of said test compound(s) to modulate the cleavage of cyclic nucleotides cAMP and/or cGMP at their 3'phosphatase bond to form 5'AMP and 5'GMP. The expression product can be part of an intact cell or lipidic preparation or a purified polypeptide(s), optionally attached to a support, for example beads, a column, or a plate. Binding is preferably performed in the presence of cAMP or cGMP to allow an assessment of the binding activity of each test compound. The cAMP or cGMP and other essential molecules may be contacted with the phosphodiesterase peptide either before, simultaneously or after the test compound(s).

A characteristic of the cAMP or cGMP is that it can readily be radiolabelled (Thompson *et al*, *Advances in cyclic nucleotide research,* **10,** 69-92 (1974)). The conversion of cAMP or cGMP to 5'AMP or 5'GMP can be detected with the use of chromatography and separation technologies. Such technology is known to those in the art. Binding of the test compound to the phosphodiesterase polypeptide modifies its ability to convert cAMP or cGMP to 5'AMP or 5'GMP. The difference between the observed amount of conversion relative to the theoretical maximum amount of conversion is a reflection of the modulatory effect of the test compound(s).

A non-limiting general understanding of how to assay for the activity of Phosphodiesterases can be gained from *Horton*, *JK. And Baxendale*, *PM* (Methods in molecular Biology, 1995, 41, p 91-105, Eds. Kendall, DA. and Hill, SJ, Humana Press, Towota, NJ) and *Molecular Probes handbook* and references therein (Molecular Probes, Inc., 4849 Pitchford Ave, Eugene, USA).

The invention also provides a method of screening for pain alleviating compounds, comprising;
a) contacting one or more test compounds in the presence of cAMP and/or cGMP with an expression product of any of the nucleotide sequences of Tables I - X which is a phosphodiesterase or with a cell expressing at least 1 copy of an expression product or with a lipidic matrix containing at least 1 copy of an expression product;
b) determining the amount of cAMP and/or cyclic Guanosine Mono Phosphate converted to 5'AMP and/or 5'GMP, and
a) selecting test compounds on the basis of their ability to modulate said conversion.

### d) Receptor assays

An expression product of any of the nucleic acid sequences of Tables I - X which encodes a receptor, and in particular any of the nucleic acid sequences listed in Table IV, is amenable to screening using receptor assay technology.

Receptors are membrane associated proteins that initiate intracellular signalling upon ligand binding. Therefore, the identification of molecules for the prevention and treatment of pain can be achieved with the use of a ligand-binding assay, as outlined above. Such an assay would utilize an endogenous or non-endogenous ligand as a component of the ligand-binding assay. The binding of this ligand to the receptor in the presence of one or more test compounds would be measured as described above. Such is the nature of receptors that the assay is usually, but not exclusively performed with a receptor as an intact cell or membranous preparation.

The invention therefore includes a method of screening for pain alleviating compounds, comprising:
a) contacting a test compound or test compounds in the presence of a ligand with cells expressing at least one copy of the expression product of any of the nucleotide sequences as set out in Tables I - X which is a receptor or with a lipidic matrix containing at least one copy of the expression product;
b) determining the binding of the test compound to the expression product, and
c) selecting test compounds on the basis of their binding abilities.

### e) Transporter protein assays

An expression product of any of the nucleic acid sequences of Tables I - X which encodes a transporter protein, and in particular any of the nucleic acid sequences listed in Table V, is amenable to screening using transporter protein assay technology. Non limiting examples of technologies and methodologies are given by *Carroll FI*, *et al* (1995, Medical Research Review, Sep15 (5) p419-444), *Veldhuis JD and Johnson Ml* (1994, Neurosci. Biobehav Rep., winter 18(4) 605-12), *Hediger MA and Nussberger S* (1995, Expt Nephrol, July-Aug 3(4) p211-218, *Endou H and Kanai Y,* (1999, Nippon Yakurigaku Zasshi, Oct. 114 Suppl 1:1p-16p), *Olivier B et al* (2000, Prog. Drug Res., 54, 59-119), *Braun A et al* (2000, Eur J Pharm Sci, Oct 11, Supply 2 S51-60) and *Molecular Probes handbook and references therein* (Molecular Probes, Inc., 4849 Pitchfork Ave, Eugene, USA).

The main function of transporter proteins is to facilitate the movement of molecules across a cellular membrane. Compounds that modulate the activity of transporter proteins can be determined with a transporter protein functional screen. Functional screening for modulators of transporter proteins comprises contacting at least one test compound with an expression product as aforesaid which is a transporter protein and determining the ability of said test compound to modulate the activity of said transporter protein. The expression product can be part of an intact cell, or lipidic preparation, optionally attached to a support, for example beads, a column or a plate. Binding is preferably performed in the presence of the molecule to be transported, which should only able to pass through a cell membrane or lipidic matrix with the aid of the transporter protein. The molecule to be transported should be able to be followed when it moves into a cell or through a lipidic matrix. Preferably the molecule to be transported is labelled to aid in characterization, e.g. with a chromophore, radioactive, fluorescent, phosphorescent, enzymatic or antibody label. If the molecule to be transported is not directly detectable it should be amenable to detection and quantification by secondary detection, which may employ the above technologies. The molecule to be transported may be contacted with the transporter protein before, simultaneously with or after the test compound. If binding of the test compound to the transporter protein modifies its ability to transport molecules through a membranous or lipidic matrix then the difference between the observed amount of transported molecule in a cell/or through a lipidic matrix relative to the theoretical maximum amount is a reflection of the modulatory effect of the test compound.

The invention further provides a method for screening compounds for their ability to relieve pain, comprising
a) contacting at least one test compound in the presence of transporter molecules with a cell containing at least one copy of an expression product of any of the nucleotide sequences of Tables I - X which is a transporter protein or with a lipidic matrix containing at least one copy of the expression product;
c) measuring the movement of transported molecules into or from the cell, or across the lipidic matrix; and
d) selecting test compounds on the basis of their ability to modulate the movement of transported molecules.

### f) G-protein coupled receptor protein assays

An expression product of any of the nucleic acid sequences of Tables I - X that encodes a G-protein coupled receptor protein, and in particular any of the nucleic acid sequences listed in Table VI, is amenable to screening using G-protein coupled receptor protein assay technology.

G-protein coupled receptor proteins (GPCRs) are membrane associated proteins whose main function is to transduce a signal through a cellular membrane. Upon ligand binding, GPCRs undergo a conformational change that allows complexing of the GPCRs with a G-protein. G-proteins possess a GTP/GDP binding site. The formation of the G-protein/ligand complex allows exchange of GTP for GDP, resulting in a conformational change of the G-protein. This conformational change initiates signal transduction.

Functional screening for modulators of GPCRs comprises contacting at least one test compound with an expression product as aforesaid which is a G-protein coupled receptor protein, and assessing the ability of the test compound(s) to modulate the exchange of GTP for GDP or the modulation of the GPCR signal transduction pathway. The expression product can be part of an intact cell or lipidic preparation, optionally attached to a support, for example beads, a column or a plate. Binding is preferably performed in the presence of a ligand, G-protein and GTP/GDP to allow an assessment of the binding activity of each test compound. Alternatively components of the signalling pathway are also included. In particular, in a preferred embodiment, a labelled GTP is used and the ability of the test compound(s) to modulate the exchange of GTP to GDP is determined. A further optional characteristic of the assay can be the inclusion of a reporter molecule that enables monitoring the regulation of the signalling pathway. The ligand may be contacted with the GPCR before, simultaneously with or after the test compound. Binding of the test compound to the GPCR modifies its ability to modulate the exchange of GTP for GDP and hence the modulation of signal transduction. The difference between the observed amount of GTP exchanged for GDP relative to the theoretical maximum amount of GTP is a reflection of the modulatory effect of the test compound. Likewise the relative activities of signal transduction reporter molecules are also a reflection of the modulatory effect of the test compound. Non limiting examples of technologies and methodologies can be found in *Molecular Probes handbook* and references therein (Molecular Probes, Inc., 4849 Pitchford Ave, Eugene, USA), *Glaxo Pocket Guide to Pharmacology*, (Michael Sheehan, Pharmacology Division staff, Glaxo Group Research Ltd., Ware, Herts SC12 0DP) and *Xing et al* (2000, J. Recept. Signal. Transduct. Res. 20(40 189-210).

The invention provides a method of screening compounds for their ability to relieve pain, comprising:
a) contacting at least one test compound in the presence of a ligand, GTP/GDP, G-protein with a cell containing at least one copy of an expression product of any of the nucleotide sequences of Tables I - X which is aG-protein coupled receptor protein or with a lipidic matrix containing at least one copy of the expression product;
b) measuring the exchange of GTP for GDP, and
c) selecting test compounds on the basis of their ability to modulate said exchange.

In the above method, the ligand, GTP/GDP, G-protein and other essential molecules can be added before, simultaneously with or after the contacting of the test compound(s) with the cell line or lipidic matrix in step a).

### g) DNA-binding protein assays

An expression product of any of the nucleic acid sequences of Tables I - X that encodes a DNA-binding protein, and in particular any of the nucleic acid sequences listed in Table VII, is amenable to screening using DNA-binding protein assay technology.

DNA binding proteins are proteins that are able to complex with DNA. The complexing of the DNA binding protein with the DNA in some instances requires a specific nucleic acid sequence. Screens can be developed in a similar manner to ligand binding screens as previously indicated and will utilise DNA as the ligand. DNA-binding protein assays can be carried using similar principles described in ligand binding assays as described above. Non limiting examples of methodology and technology can be found in the teachings of *Haukanes BI and Kvam* C (Biotechnology, 1993 Jan 11 60-63), *Alberts B et al* (Molecular Biology of the Cell, 1994, 3^{rd} Edn., Garland Publications Inc, *Kirigiti P and Machida CA* (2000 Methods Mol Biol, 126, 431-51) and *Molecular Probes handbook* and references therein (Molecular Probes, Inc., 4849 Pitchford Ave., Eugene, USA).

The invention therefore includes a method of screening for pain alleviating compounds, comprising:
a) contacting a test compound or test compounds in the presence of a plurality of nucleic acid sequences with an expression product of any of the nucleotide sequences as set out in Tables I - X which is a DNA binding protein or with cell expressing at least one copy of the expression product or with a lipidic matrix containing at least one copy of the expression product;
b) determining the binding of the test compound to the expression product, and
c) selecting test compounds on the basis of their binding abilities.

In the above method, the plurality of nucleic acid sequence may be added prior to, simultaneously with or after contact of the test compound with the expression product.

### h) Protease assays

An expression product of any of the nucleic acid sequencesof Tables I - X that encodes a protease protein, and in particular any of the nucleic acid sequences listed in Table VIII, is amenable to screening using protease protein assay technology. Non-limiting examples of such technologies are illustrated in *Molecular Probes handbook* and references therein (Molecular Probes, Inc., 4849 Pitchford Ave, Eugene, USA), *Cook et al* (1992, Structure and function of the aspartic proteinases, Ed. Dunn BM, Plenum Press, New York, 525-528) and *Peranteau AG et al (1995,* Anal Biochem, 1, 227(1) 242-5).

Protease proteins have the property of being able to cleave peptide sequences. In brief, one characteristic of the protease-screening assay is the peptide ligand, which may contains at least one copy of a protease protein recognition sequence. The cleavage of this peptide by the protease results in the fragmentation of the peptide ligand. The propensity of the protease to fragment the peptide ligand in the presence of a candidate compound(s) is a measure of the inhibitory qualities of the candidate compound(s).

The protease-screening assay may be performed in the presence of at least a plurality of ligand binding peptide and a protease. These screening assays can be performed in the presence of isolated expression product, expression product in cells or expression product in a lipidic matrix. The invention therefore includes a method of screening for pain alleviating compounds, comprising:
(a) contacting one or more test compounds in the presence of peptide ligand with an expression product of any of the nucleotide sequences as set out in Tables I - X which is a protease or with a lipidic matrix containing at least one copy of the expression product;
(b) determining the amount of cleavage of peptide ligand; and
(c) selecting test compounds on the basis of their ability to modulate the amount of ligand cleavage.

### j) Assays using other enzymes

Expression products of any of the nucleic acid sequences of Tables I - X that encode other enzymes, e.g. ligases, lyases, oxidoreductases, transferases and hydrolases, and in particular any of the nucleic acid sequences listed in Table IX, is amenable to screening using appropriate assay technology.

Each class of enzyme has a defined function. Ligases have the property of being able to splice molecules together. This is achieved with the conversion of ATP substrate to AMP. Therefore, the activity of a ligase can be followed by monitoring the conversion of ATP to AMP. Such technologies are known to those in the art. Non-limiting examples and methodologies can are illustrated by Ghee. *T. Tan et al* (1996,Biochem J. 314, 993-1000, *Yang SW et al* (1992, 15: 89(6) 2227-31 and references therein, and in *Molecular Probes handbook* and references therein (Molecular Probes, Inc., 4849 Pitchford Ave, Eugene, USA).

The invention also provides methods of screening for pain alleviating compounds, comprising;
a) contacting one or more test compounds in the presence of ATP with an expression product of any of the nucleotide sequences as set out in Tables I - X which is a ligase or with a cell expressing at least 1 copy of an expression product or with a lipidic matrix containing at least 1 copy of an expression product;
b) determining the amount of ATP converted to AMP, and
c) selecting test compounds on the basis of their ability to modulate said conversion.

Lyases are enzymes that catalyze cleavage by reactions other than hydrolysis. These enzymes can be grouped into seven groups according to type of bond cleaved. These groups are carbon-carbon lyases (E.C. No 4.1), carbon-oxygen lyases (E.C. No 4.2), carbon-nitrogen lyases (E.C. No 4.3), carbon-sulphur lyase (E.C. No 4.4) carbonhalide lyases (E.C. No 4.5), phosphorous-oxygen lyases (E.C. No 4.6) and other lyases (E.C. No 4.99) (Analytical Biochemistry 3^{rd} Edn, David J. Holme and Hazel Peck, Longman press). The enzyme commission number (E.C.) of the International Union of Biochemistry relates to the type of reaction catalyzed by the enzyme. Further *teachings on how to develop assays and screens for lyases can be obtained from Methods in Enzymology* (Academic Press).

Oxidoreductases are enzymes that catalyze the transfer of hydrogen or oxygen atoms or electrons. These enzymes can by sub-grouped into twenty categories according to their specific mode of action. These groups are oxidoreductases acting on the CH-OH group of donors (E.C. No1.1), oxidoreductases acting on the aldehyde or oxo group of donors (E.C. No 1.2), oxidoreductases acting on the CH-CH group of the donor (E.C. No 1.3), oxidoreductases acting on the CH-NH2 group of donors (E.C. 1.4), oxidoreductases acting on the CH-NH group of donor (E.C. 1.5), oxidoreductases acting on the NADH or NADPH (E.C. No 1.6), oxidoreductases acting on other nitrogen compounds as donors (E.C. No 1.7), oxidoreductases acting on a sulphur group of donors (E.C. No 1.8), oxidoreductases acting on a haem group of donors (E.C. No1.9), oxidoreductases acting on diphenols and related substances as donors (E.C. 1.10), oxidoreductases acting on hydrogen peroxide as acceptor (E.C. No 1.11), oxidoreductases acting on hydrogen as donor (E.C. No 1.12), oxidoreductases acting on single donors with incorporation of molecular oxygen (E.C. No 1.13), oxidoreductases acting on paired donors with incorporation of molecular oxygen (E.C. No 1.14), oxidoreductases acting on superoxide radicals as acceptors (E.C. 1.15), oxidoreductases oxidizing metal ions (E.C. No 1.16), oxidoreductases acting on -CH2 groups (E.C. No 1.17), oxidoreductases acting on reduced ferredoxin as donor (E.C. No 1.18), oxidoreductases acting on reduced flavodoxin as donor (E.C. No 1.19) and other oxidoreductases (E.C. No 1.97) (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for oxidoreductases can be obtained from *Methods in Enzymology* (Academic Press) with special reference to volume 249.

Transferases are enzymes that catalyze the transfer of specific groups. They are classified into eight sub groups according to function, transferring one-carbon group (E.C. No 2.1), Transferring aldehyde or ketonic residues (E.C. No 2.2), Acetyltransferases (E.C. 2.3), glycosyltransferases (E.C. No 2.4), transferring alkyl or aryl groups other than methyl groups (E.C. No 2.5), transferring nitrogenous groups (E.C. No 2.6), transferring phosphorous-containing groups (E.C. No 2.7) and transferring sulphur-containing groups (E.C. No 2.8) (Analytical Biochemistry 3^{rd} Edn, David J. Holme and Hazel Peck, Longman Press). The enzyme commission number (E.C.) of the International Union of Biochemistry relates to the type of reaction catalyzed by the enzyme. Further teachings on how to develop assays and screens for transferases can be obtained from Methods in Enzymology (Academic Press).

Hydrolases are enzymes that catalyze hydrolytic reactions and are sub-grouped into eleven classes according to the type of reaction they carry out. Hydrolases acting on ester bonds (E.C. No 3.1), hydrolases acting on glycosyl compounds (E.C. No 3.2), hydrolases acting on ether bonds (E.C. No 3.3), hydrolases acting on peptide bonds (E.C. No 3.4), hydrolases acting on carbon-nitrogen bonds, other than peptide bonds (E.C. No 3.5), hydrolases acting on acid anhydrides (E.C. No 3.6), hydrolases acting on acid anhydrides (E.C. No 3.6), hydrolases acting on carbon-carbon bonds (E.C. No 3.7), hydrolases acting on halide bonds (E.C. No 3.8), hydrolases acting on phosphorous-nitrogen bonds (E.C. No 3.9), hydrolases acting on sulphur-nitrogen bonds (E.C. No 3.10) and hydrolases acting on carbon-phosphorous bonds (Analytical Biochemistry 3^{rd} Edn, David J. Holme and Hazel Peck, Longman Press). The enzyme commission number (E.C.) of the International Union of Biochemistry relates to the type of reaction catalyzed by the enzyme. Further teachings on how to develop assays and screens for hydrolases can be obtained from Methods in Enzymology (Academic Press) with special reference to volume 249.

### C) In vivo functional screen

Any of the nucleic acid sequences described in Tables I - X or homologues thereof may be inserted by means of an appropriate vector into the genome of a lower vertebrate or of an invertebrate animal or may be inactivated or down regulated in the genome of said animal. The resulting genetically modified animal may be used for screening compounds for effectiveness in the regulation of pain. The invertebrate may, for example, be a nematode e.g. *Caenorhabditis elegans*, which is a favourable organism for the study of response to noxious stimuli. Its genome sequence has been determined, see *Science,* **282,** 2012 (1998), it can be bred and handled with the speed of a micro-organism (it is a self-fertilizing hermaphrodite) and can therefore be used in a high throughput screening format (WO 00/63424, WO 00/63425, WO 00/63426 and WO 00/63427), and it offers a full set of organ systems, including a simple nervous system and contains many similarly functioning genes and signaling pathways to mammals. A thermal avoidance model based on a reflexive withdrawal reaction to an acute heat stimulus has been described by Wittenburg *et al*, *Proc. Natl*. *Acad*. *Sci*. *USA*, **96,** 10477-10482 (1999), and allows the screening of compounds for the treatment of pain with the modulation of pain sensation as an endpoint.

The genome of *C. elegans* can be manipulated using homologous recombination technology which allows direct replacement of nucleic acids encoding *C. elegans* with their identified mammalian counterpart. Replacement of these nucleic acids with those nucleic acids outlined above would allow for the direct screening of test compound(s) with their expression products. Any of the pain-related genes described above may be ligated into a plasmid and introduced into oocytes of the worm by microinjection to produce germline transformants. Successful plasmid injection into *C. elegans* and expression of inserted sequences has been reported by Devgen B.V., Ghent, Belgium. It is also possible to produce by routine methods worms in which the target sequences are down-regulated or not expressed (knock-out worms). Further non limiting examples of methodology and technology can be found in the teachings of *Hazendonk et al* (1997, Nat genet. 17(1) 119-21), *Alberts et al*, (1994, Molecular Biology of the Cell 3^{rd} Ed. Garland Publishing Inc, *Caenorhabditis elegans* is anatomically and genetically simple), *Broverman S et al*, (1993, PNAS USA 15; 90(10) 4359-63) and Mello et al (1991, 10(12) 3959-70).

A further method for screening compounds for ability to modify response to pain, e.g. relieve pain, comprises:
(a) contacting one or more test compounds with at least one *C elegans* containing at least one copy of a sequence as set out above;
(b) subjecting the C. elegans to a nociceptive stimulus;
(c) observing the response of the *C.elegans* to said stimulus; and
d) selecting test compounds on the basis of their ability to modify the response of *C. elegans* to said stimulus.

### DIAGNOSTIC TOOLS AND KITS

### Affinity peptides, ligands and substrates

Pain associated polypeptides and fragments thereof can be detected at the tissue and cellular levels with the use of affinity peptides, ligands and substrates, which will enable a skilled person to define more precisely a patient's ailment and help in the prescription of a medicament. Such affinity peptides are characterized in that firstly they are able to bind specifically to a pain associated polypeptide, and secondly that they are capable of being detected. Such peptides can take the form of a peptide or polypeptide for example an antibody domain or fragment, or a peptide/polypeptide ligand or substrate, or a polypeptide complex such as an antibody.

The preparation of such peptides and polypeptides are known to those in the art. Antibodies, these may be polyclonal or monoclonal, and include antibodies derived from immunized animals or from hybridomas, or derivatives thereof such as humanized antibodies, Fab or F(ab')2 antibody fragments or any other antibody fragment retaining the antigen binding specificity.

Antibodies directed against pain-associated gene product molecules may be produced according to conventional techniques, including the immunization of a suitable mammal with the peptides or polypeptides or fragment thereof. Polyclonal antibodies can be obtained directly from the serum of immunized animals. Monoclonal antibodies are usually produced from hybridomas, resulting from a fusion between splenocytes of immunized animals and an immortalized cell line (such as a myeloma). Fragments of said antibodies can be produced by protease cleavage, according to known techniques. Single chain antibodies can be prepared according to the techniques described in US 4,946,778. Detection of these affinity peptides could be achieved by labelling. Technologies which allow detection of peptides, such as enzymatic labelling, fluorescence labelling or radio-labelling are well known to those in the art. Optionally these affinity peptides, ligands and substrates could themselves be detected with the use of a molecule that has specific affinity to the peptides, ligands and substrates and is itself labelled.

The invention further provides a kit, which comprises;
(a) affinity peptide and/or ligand and/or substrate for an expression product of a gene sequence that is up-regulated in the spinal cord of a mammal in response to streptozocin-induced diabetes; and
(b) a defined quantity of an expression product of a gene sequence that is up-regulated in the spinal cord of a mammal both in response to streptozocin-induced diabetes, for simultaneous, separate or sequential use in detecting and/or quantifying an expression product of a gene sequence that is up-regulated in the spinal cord of a mammal in response to streptozocin-induced diabetes

### Complimentary nucleic acids

Pain associated nucleic acid sequences can be characterized at the tissue and cellular levels with the use of complimentary nucleic acid sequences. Detection of the level of expression of pain associated nucleic acid sequences can help in the prognosis of a pain condition and the prescription of a medicament. These complimentary nucleic acids are characterized in that they can hybridize to a pain associated nucleic acid sequence and their presence can be detected through various techniques. Such techniques are known to those in the art and may include detection by polymerase chain reaction or detection by labelling of complimentary nucleic acid sequences by enzymatic labelling, affinity labelling fluorescent labelling or radio-labelling. Complimentary strand nucleic acid sequences of this invention are 10 to 50 bases long, more preferably 15 to 50 bases long and most preferably 15 to 30 bases long, and hybridize to the coding sequence of the nucleic acid sequence.

A further aspect of this invention is a kit that comprises:
(a) nucleic acid sequences capable of hybridization to a nucleic acid sequence that is up-regulated in the spinal cord of a mammal in response streptozocin-induced diabetes and
(b) a defined quantity of one or more nucleic acid sequences capable of hybridization to a nucleic acid sequence that is up-regulated in the spinal cord of a mammal in response to streptozocin-induced diabetes, for simultaneous, separate or sequential use in detecting and/or quantifying a gene sequence that is up-regulated in the spinal cord of a mammal in response to streptozocin-induced diabetes.

### IDENTIFICATION AND VALIDATION

Subtractive hybridization enables the identification of nucleic acid sequences whose expression profiles are modified by a stimulus. Upon stimulation of a system (in the case of this invention a nociceptive stimulus on an animal model) all observed changes in the level of nucleic acid sequence expression are due to the reaction of the system to the stimulus. Characterization of these changes in expression by way of identification of nucleic acid sequence and level of expression is both identification and validation.

The inventors have developed a four step process which allows for the simultaneous identification and validation of nucleic acid sequences whose expression are regulated by a pain stimulus, preferably a chronic pain stimulus, and more preferably a diabetic pain stimulus. This process may comprise the following steps:
(a) induction of a nociceptive stimulus in test animals;
(b) extraction of nucleic acids from specific neuronal tissue of test and control animals;
(c) selective amplification of differentially expressed nucleic acid sequences; and
(d) identification and characterization of differentially expressed gene products that are modulated by a nociceptive stimulus.

The above process is described in more detail below.

### (a) Induction of nociceptive stimulus

The effect of the selected nociceptive stimulus on the test animal needs to be confirmable. The test subjects are therefore a species that has a "developed" nervous system, preferably similar to that of humans, most preferably rats or mice. Advantageously, the nociceptive stimulus is analogous to known pain paradigms in humans. One such paradigm of pain which is used herein is the pain associated with diabetes, which can be induced in rodents with the use of streptozotocin (STZ). However, other pain paradigms could be used.

Streptozotocin (STZ) induces hyperglycemia and Type 1 diabetes mellitus in rats. In particular, STZ contains a glucose analogue that allows it to be taken up by the glucose transporter 2 present on the surface of pancreatic β cells, the site of insulin synthesis. Once inside the cell, STZ causes a reduction in the level of nicotinamide adenine dinucleotide (NAD⁺). The decrease in NAD⁺ levels eventually leads to necrosis of the pancreatic β cell, causing a reduction in insulin levels and then diabetes, leading to neuropathy (diabetic) and neuropathic pain (R. B. Weiss, *Cancer Treat. Rep.,* **66**, 427-438 1982, Guy *et al*, *Diabetologica*, **28**, 131-137 1985; Ziegler *et al*, *Pain,* **34**, 1-10 1988; Archer *et al*, *J. Neural*. *Neurosurgeon. Psychiatry*, **46**, 491-499 1983). The diabetic rat model has been shown to be a reliable model of hyperalgesia. We have used an STZ-induced diabetic rat model to create a state of hyperlagesia that can be compared with control animals (Courteix *et al*, *Pain,* **53**, 81-88 1993).

### (b) Extraction of nucleic acids from neuronal tissue of test and control animals

The inventors have determined that RNA extraction of whole spinal cord nervous tissue provides a way of identifying nucleic acid sequences whose expression is modulated by streptozotocin induced pain. Test (subjected to the nociceptive stimulus) and control animals were sacrificed, and the tissue to be studied e.g. neural tissue separated. Techniques for so doing vary widely from animal to animal and will be familiar to skilled persons.

A cDNA library can be prepared from total RNA extracted from neural tissue of the test and control animals. Where possible, however, it is preferred to isolate the mRNA from the total RNA of the test and control animals, by affinity chromatography on oligo (dT)-cellulose, and then reverse transcribe the mRNA from the test and control animals to give test and control cDNA. Converting mRNA from the test and control animals to corresponding cDNA may be carried out by any suitable reverse transcription method, e.g. a method described by Gubler & Hoffman, *Gene,* 25, 263-269 (1983). If desired a proprietary kit may be used e.g. the CapFinder PCR cDNA Library Construction Kit (Life Technologies) which is based on long-distance PCR and permits the construction of cDNA libraries from nanograms of total RNA.

### (c) Selective amplification of differentially expressed nucleic acids

The reverse transcribed cDNA of the test and control animals is subjected to subtractive hybridisation and amplification so that differentially expressed sequences become selectively amplified and commonly expressed sequences become suppressed, so as to over-produce DNA associated with said nociceptic stimulus. A wide range of subtractive hybridisation methods can be used, but the preferred method is so-called suppression subtractive hybridisation, see US-A-5565340 and Diatchenko *et al*, *Proc. Nat. Acad. Sci.* USA, 93, 6025-6030 (1996), the disclosures of which are herein incorporated by reference. Kits for carrying out this method are available from CLONTECH Laboratories, Inc.

### (d) Cloning and Sequencing the differentially expressed cDNA

The differentially expressed cDNA is ligated into a cloning vector, after which cells of *E. coli* are transformed with the vector and cultured. Positive clones are selected and burst to release plasmids containing the cDNA insert. The plasmids are primed using forward and reverse primers to either side of the cloning site and the cDNA insert is sequenced. Vector and adaptor sequences are then removed from the output data from the sequencer, leaving only the nucleotide sequence of the differentially expressed gene. The sequence is then checked against data held in a database for homology to known nucleotide sequences and genes and including expressed sequence tags (ESTs) and coding sequences for proteins.

### (e) Validation of the above method

The importance of the sequences that we have identified in pain is confirmed by the fact that genes that represent nucleic acid sequences which have previously been implicated in pain, including Calmodulin (pRCM1, Genebank X13933), Enkephalin (Genebank Y07503) and Neurotensin receptor type 2 (Genebank X97121) have also been identified using this method.

The inventors have identified nucleic acid sequences of the MAP kinase pathway, a previously non pain-associated biological pathway. The inventors have subsequently shown that intra-spinal injection of a MEK inhibitor (MEK is part of the MAP kinase pathway) produces a powerful inhibition of pain (Patent application No US 60/144292). Subsequently, it has been shown that the MAP kinase is also implicated in acute inflammatory pain (Woolf *et al*, Nature Neuroscience 1999).

The invention will now be further described in the following Example.

### EXAMPLE

### Induction of diabetes

Diabetes was induced in adult (150-200g) male Sprague-Dawley rats (n=6) as described by Courteix *et al (supra).* Animals were injected intraperitoneally with streptozotocin (STZ)(50 mg/kg) dissolved in distilled water. Control or sham animals (age-matched animals, n=6) were injected with distilled water only.

### Nociceptive testing

Static allodynia (a form of hyperlagesia) was measured using a method described by Chaplan *let al,* "Quantitative assessment of tactile allodinya in the rat paw", *J*. *Neurosci. Methods,* **53**, 55-63 (1994). A series of von Frey filaments of different buckling weight (i.e. the load required for the filament to bend) were applied to the plantar surface of the right hand paw. The starting filament had a buckling weight of 20g. Lifting of the paw was taken to be a positive result, in which case a filament with the next lowest buckling weight was used for the next measurement. The test was continued until a filament was found for which there was an absence of response for longer than 5 seconds whereas a re-test with the next heaviest filament gave a positive response. Animals were considered hyperalgesic if their thresholds were found to be <4g of those of comparable untreated rats, see Calcutt & Chaplan, "Spinal pharmacology of tactile allodynia in diabetic rats", *British J. Pharmacol*, **122,** 1478*-*1482 (1997).

### Tissue Extraction

STZ-treated and control animals were anaesthetized with 4% halothane and perfused with ice-cold 0.9% saline containing 1% citric acid (pH adjusted to 7.4 with NaOH). The animals were decapitated and the lumbar spinal cord exposed. A 2-centimetre length of spinal cord ending at L6 (lumbar-6 forward) was removed from the spinal column. Attached dorsal root ganglia and contaminating spinal connective tissues were removed. The spinal cord tissue was snap frozen in dry ice and isopentane.

### Total RNA Extraction

Total RNA was extracted from the above pooled male rat tissues using the TRIZOL Reagent Kit (Life Technologies). Briefly, tissue samples were homogenised fully using a Polytron homogenizer in 1ml of TRIZOL reagent per 50-100mg of tissue. Homogenized samples were then incubated at room temperature for 5 minutes and phase separated using 0.2ml chloroform per 1ml TRIZOL reagent followed by centrifugation at 3,000g. The aqueous phase was transferred to a fresh tube and the RNA precipitated with an equal volume of isopropyl alcohol and followed by centrifugation at 10,000g. The RNA pellet was washed in 75% ethanol and re-centrifuged. The pellet was then air dried and re-suspended in water.

### mRNA Extraction

In contrast to ribosomal RNA and transfer RNA, the vast majority of mRNAs of mammalian cells carry tracts of poly(A⁺) at their 3' termini. mRNAs can therefore be separated from the bulk of cellular RNA by affinity chromatography on oligo (dT)-cellulose. mRNA was extracted from Total RNA using the MESSAGEMAKER Kit (Life Technologies) in which mRNA (previously heated to 65°C in order to disrupt secondary structures and so expose the poly (A⁺) tails) was bound to oligo(dT) cellulose under high salt concentrations (0.5M NaCl) in a filter syringe. Unbound RNA was then washed away and the poly(A⁺) mRNA eluted in distilled water. A tenth of the volume of 7.5 M Ammonium Acetate, 50µg of glycogen/ml mRNA sample and 2 volumes of absolute alcohol were then added to the samples which are then placed at -20°C overnight. Following precipitation, the mRNA was spun down at 12,000g for 30 minutes at 4°C. RNAase free water was used to re-suspend the pellets, which were then and stored at - 80°C.

### PCR SELECT

The technique used was based on that of the CLONTECH PCR Select Subtraction Kit. The following protocol was performed using STZ-treated lumbar spinal cord Poly A ⁺ RNA as the 'Tester' and Sham lumbar spinal cord poly A⁺ RNA as the 'Driver' (Forward Subtraction). A second subtraction experiment using the Sham lumbar spinal cord mRNA as the 'Tester' and STZ treated lumbar spinal cord mRNA as the 'Driver' (Reverse Subtraction) was performed in parallel using the same reagents and protocol. As a control for both experiments, the subtraction was also carried out using human skeletal muscle mRNA that had been provided by the kit manufacturer.

### First-Strand cDNA Synthesis

2 µg of PolyA⁺ RNA and 1 µl of cDNA synthesis primer (10 µM) were combined in a 0.5ml Eppendorf tube and sterile H₂O was added where necessary to achieve a final volume of 5 µl. The contents were mixed gently and incubated in a thermal cycler at 70°C for 2 min. The tubes were then cooled on ice for two minutes, after which 2 µl of 5X First-strand buffer, 1 µl of dNTP mix (10 mM each), sterile H₂O and 1 µl of AMV reverse transcriptase (20 units/µl) was also added. The tubes were then placed at 42°C for 1.5 hr in an air incubator. First-strand cDNA synthesis was terminated by placing the tubes on ice. (the human skeletal muscle cDNA produced by this step was used as the 'control driver' in later steps).

### Second-Strand cDNA Synthesis

48.4 µl of Sterile H₂O, 16.0 µl of 5X Second-strand buffer, 1.6 µl of dNTP mix (10 mM) and 4.0 µl of 20X second-strand enzyme cocktail were added to each of the first-strand synthesis reaction tubes. The contents were then mixed and incubated at 16°C in a thermal cycler for 2 hr. 6 units (2µl) of T4 DNA polymerase was then introduced and the tubes were incubated for a further 30 min at 16°C. In order to terminate second-strand synthesis, 4µl of 20X EDTA/glycogen mix was added. A phenol:chloroform extraction was then carried out using the following protocol:

100 µl of phenol:chloroform:isoamyl alcohol (25:24:1) was added to the tubes which were then vortexed thoroughly and centrifuged at 14,000 rpm for 10 min at room temperature. The top aqueous layer was removed and placed in a fresh tube. 100 µl of chloroform:isoamyl alcohol (24:1) was then added to the aqueous layer and the tubes were again vortexed and centrifuged at 14,000 rpm for 10 min. 40 µl of 4 M NH₄OAc and 300 µl of 95% ethanol were then added and the tubes centrifuged at 14,000 rpm for 20 min. The supernatant was removed carefully, then 500 µl of 80% ethanol was added to the pellet. The tubes were centrifuged at 14,000 rpm for 10 min and the supernatant was removed so that the pellet could be air-dried. The precipitate was then dissolved in 50 µl of H₂O. 6 µl was transferred to a fresh microcentrifuge tube. The remainder of the sample was stored at -20°C until needed.

### Rsa I Digestion

All products of the above procedures were subjected to a restriction digest, using the restriction endonuclease *Rsa* I, in order to generate ds cDNA fragments that are short and thus are optimal for subtraction hybridisation due to the standard kinetics of the hybridisation. Also, as *Rsa* I makes a double stranded cut in the middle of a recognition sequence, 'blunt ends' of a known nucleotide sequence are produced allowing ligation of adaptors onto these ends in a later step. The following reagents were added to the 6 µl product of the second hybridisation (see above): 43.5 µl of ds cDNA, 5.0 µl 10X *Rsa* I restriction buffer and 1.5 µl of *Rsa* I (10 units/ µl). The reaction mixture was incubated at 37°C for 1.5 hr. 2.5 µl of 20X EDTA/glycogen mix was used to terminate the reaction. A phenol:chloroform extraction was then performed as above (second-strand cDNA synthesis section). The pellet produced was then dissolved in 5.5 µl of H₂O and stored at -20°C until needed. The preparation of the experimental 'Driver' cDNAs and the control skeletal muscle cDNA was thus completed.

### Adaptor Ligation

The adaptors were not ligated to the driver cDNA.

1 µl of each *Rsa* I-digested experimental cDNA (from the *Rsa* I Digestion above) was diluted with 5 µl of sterile water. Preparation of the control skeletal muscle tester cDNA was then undertaken by briefly centrifuging the tube containing control DNA (*Hae* III-digest of φX174 DNA [3 ng/ µl]) and diluting 2 µl of the DNA with 38 µl of sterile water (to 150 ng/ml). 1 µl of control skeletal muscle cDNA (from the *Rsa* I Digestion) was then mixed with 5 µl of the diluted φX174/ *Hae* III DNA (150 ng/ml) in order to produce the control skeletal muscle tester cDNA.

### Preparation of the adaptor-ligated tester cDNA

A ligation master mix was prepared by combining 3 µl of sterile water, 2 µl of 5X ligation buffer and 1 µl T4 DNA ligase (400 units/µl) per reaction. 2 µl of adaptor 1 (10 µM) was then added to 2 µl of the diluted tester cDNA. To this, 6 µl of the ligation master mix was also added. The tube was therefore labelled Tester 1-1. In a separate tube, 2 µl of the adaptor 2R (10 µM) was mixed with 2 µl of the diluted tester cDNA and 6 µl of the master mix. This tube was named Tester 1-2.

2 µl of Tester 1-1 and 2 µl of Tester 1-2 were then placed into fresh tubes. These would later be used as the unsubtracted tester control. The remainder of the contents of Tester 1-1 and Tester 1-2 tubes were then centrifuged briefly and incubated at 16°C overnight. The ligation reaction was stopped by adding 1 µl of EDTA/glycogen mix and the samples were heated at 72°C for 5 min in order to inactivate the ligase. In doing so, preparation of the experimental and control skeletal muscle adaptor-ligated tester cDNAs was complete.

1 µl from each unsubtracted tester control was then removed and diluted into 1 ml of water. These samples were set aside as they were to be used later for PCR (see below). All of the samples were stored at -20°C

### Analysis of Ligation efficiency

1 µl of each ligated cDNA was diluted into 200 µl of water and the following reagents were then combined in four separate tubes:

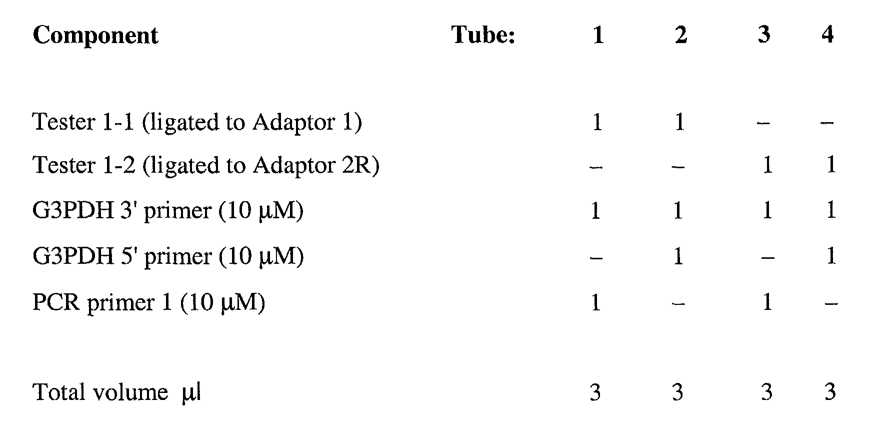

A master mix for all of the reaction tubes plus one additional tube was made up by adding 18.5 µl of sterile H₂O, 2.5 µl of 10X PCR reaction buffer, 0.5 µl of dNTP mix (10 mM), and 0.5 µl of 50X Advantage cDNA Polymerase Mix, per reaction, into a fresh tube. 22 µl of this master mix was then aliquotted into each of the 4 reaction tubes prepared above. The contents of the tubes were overlaid with 50 µl of mineral oil. The reaction mix was incubated in a thermal cycler at 75°C for 5 min in order to extend the adaptors. The following protocol was then carried out immediately in a thermal cycler (Perkin-Elmer GeneAmp PCR Systems 2400): 94°C for 30 sec (1 cycle), 94°C 10 sec, 65°C 30 sec and then 68°C 2.5 min (25 cycles)

### First Hybridisation

1.5 µl of the Adaptor 1-ligated Tester 1-1 was combined with 1.5 µl of the *Rsa* I-digested driver cDNA, prepared earlier and 1 µl of 4X Hybridisation buffer. This process was then repeated combining the Adaptor 2R-ligated Tester 1-2 with the *Rsa* I-digested driver cDNA and 4X Hybridisation buffer. The samples were incubated in a thermal cycler at 98°C for 1.5 min followed by incubation at 68°C for 8 hr.

### Second Hybridisation

1 µl of Driver cDNA (i.e. the *Rsa* I-digested cDNA (see above)), 1 µl 4X Hybridisation buffer and 2 µl Sterile H₂O were all combined in a fresh tube. 1 µl of this mix was then removed and placed in a new tube, overlaid with 1 drop of mineral oil and incubated at 98°C for 1.5 min in order to denature the driver. The following procedure was used to simultaneously mix the driver with hybridisation samples 1 and 2 (prepared in the first hybridisation), thus ensuring that the two hybridisation samples were mixed together only in the presence of freshly denatured driver: A micropipetter was set at 15 µl. The pipette tip was then touched onto the mineral oil/sample interface of the tube containing hybridisation sample 2. The entire sample was drawn partway into the tip before it was removed from the tube in order to draw a small amount of air into the tip. The pipette tip was then touched onto the interface of the tube containing the freshly denatured driver (i.e. the tip contained both samples separated by a small pocket of air) before the entire mixture was transferred to the tube containing hybridisation sample 1. The reaction was then incubated at 68°C overnight. 200 µl of dilution buffer was added to the tube, which was then heated in a thermal cycler at 68°C for 7 min. The product of this second hybridisation was stored at -20°C.

### PCR Amplification

Seven PCR reactions were set up: (1) The forward-subtracted experimental cDNA, (2) the unsubtracted tester control (see preparation of the adaptor ligated tester cDNA), (3) the reverse-subtracted experimental cDNA, (4) the unsubtracted tester control for the reverse subtraction, (5) the subtracted control skeletal muscle cDNA, (6) the unsubtracted tester control for the control subtraction, and (7) the PCR control subtracted cDNA (provided in the kit). The PCR control subtracted cDNA was required to provide a positive PCR control as it contained a successfully subtracted mixture of *Hae* III-digested φX174 DNA.

The PCR templates were prepared by aliquotting 1 µl of each diluted cDNA (i.e., each subtracted sample from the second hybridisation and the corresponding diluted unsubtracted tester control produced by the adaptor ligation see above) into an appropriately labelled tube. 1 µl of the PCR control subtracted cDNA was placed into a fresh tube. A master mix for all of the primary PCR tubes, plus one additional reaction, was then prepared by combining 19.5 µl of sterile water, 2.5 µl of 10X PCR reaction buffer, 0.5 µl of dNTP Mix (10 mM), 1.0 µl of PCR primer 1 (10 µM) and 0.5 µl of 50X Advantage cDNA Polymerase Mix. 24 µl of Master Mix was then aliquotted into each of the 7 reaction tubes prepared above and the mixture was overlaid with 50 µl of mineral oil, before being incubated in a thermal cycler at 75°C for 5 min in order to extend the adaptors. Thermal cycling was then immediately started using the following protocol: 94°C 25 sec (1 cycle), 94°C 10 sec, 66°C 30 sec and 72°C 1.5 min (32 cycles).

3 µl of each primary PCR mixture was then diluted in 27 µl of H₂O, 1 µl of each of these dilutions was then placed into a fresh tube.

A master mix for the secondary PCRs, (plus an additional reaction) was set up by combining 18.5 µl of sterile water, 2.5 µl of 10X PCR reaction buffer, 1.0 µl of Nested PCR primer 1 (10 µM), 1.0 µl of Nested PCR primer 2R (10 µM), 0.5 µl of dNTP Mix (10 mM) and 0.5 µl of 50X Advantage cDNA Polymerase Mix per reaction. 24 µl of this Master Mix was then added into each reaction tube containing the 1 µl diluted primary PCR mixture. The following PCR protocol was then carried out: 94°C 10 sec, 68°C 30 sec and 72°C 1.5 min (12 cycles). The reaction products were then stored at -20°C.

### Ligation into a Vector/ Transformation & PCR

The products of the PCR amplification (enriched for differentially expressed cDNAs) were ligated into the pCR2.1-TOPO vector using a T/A cloning kit (Invitrogen), transformed into TOPO One Shot competent cells according to the manufacturers protocol and grown up on LB (Luria-Bertani) Agar plates overnight at 37°C. 1,000 colonies were then individually picked (using fresh sterile tips) and dipped into 5 µl of sterile water which had been aliquotted previously into 96 well PCR plates. The water/colonies were heated in a thermal cycler at 100°C for 10 minutes in order to burst the cells, thus releasing the plasmids containing a differentially expressed cDNA insert. The 5 µl of water/plasmid was then used as a template in a PCR reaction (see below) using M13 Forward and Reverse primers (10 ng/µl), complementary to the M13 site present on either side of the cloning site on the vector. 5 µl of the PCR product was then run on a 2% agarose gel and stained by ethidium bromide. PCR products of an amplified insert were identified and 5 µl of the remainder of the PCR product (i.e. from the 15 µl that had not been run on the gel) was diluted 1/10 with water. 5 µl of the diluted PCR product was then used as a template in a sequencing reaction.

### Sequencing

A sequencing reaction containing M13 primer (3.2 pmol/µl), 'BigDye' reaction mix (i.e. AmpliTaq® DNA polymerase, MgCl_{2,} buffer and fluorescent dNTPs [each of the four deoxynucleoside triphosphates is linked to a specific fluorescent donor dye which in turn is attached to a specific acceptor dye]) and cDNA template (diluted PCR product) was set up. The reaction was carried out on a thermal cycler for 25 cycles of 10 seconds at 96°C, 20 seconds at 50°C and 4 minutes at 60°C. Each reaction product was then purified through a hydrated Centri-Sep column, and lyophilised. The pellets were resuspended in Template Suppression Reagent and sequenced on an ABI Prism 310 Genetic Analyser. The analyser uses an ion laser to excite the specific donor dye that transfers its energy to the acceptor dye, which emits a specific energy spectrum that can be read by the sequencer.

The first 150 potentially upregulated genes were sequenced at Parke-Davis, Cambridge. The remaining 877 from both the forward and back subtractions were sequenced at the applicant's core sequencing facility in Ann Arbor, MI, USA.

### Bioinformatics

The sequencing results were analysed using the computer program CHROMAS in which the vector and adaptor sequences were clipped off, leaving only the nucleotide sequence of the differentially expressed gene. Each sequence was then checked for homology to known genes, Expressed Sequence Tags (ESTs) and Proteins using various Basic Local Alignment Search Tool (BLAST) searches against the Genbank sequence database at the National Centre for Biotechnology Information, Bethesda, Maryland, USA (NCBI).

Two lists were derived called STZup and STZdown that contain the nucleic acid sequences from the forward and back subtracted libraries respectively. In each list there are given accession numbers and descriptions for the known rat genes identified, and where available corresponding mouse or human genes. Sequences that are considered to be of interest are listed in Tables I - IX above.
References have been given where available for the sequences that have been found, and sequence listings have been given in the form in which they currently appear in publicly searchable databases e.g. the NCBI databaase (National Center for Biotechnology Information, National Library of Medicine, National Institutes of Health, Bethesda, Maryland, MD 20894, USA, www.ncbi.nlm.nih.gov). These sequence listings are given for the purposes of identification only. The invention includes the use of subsequently revised versions of the above sequences (which may incorporate small differences to the version set out herein) and homologous sequences or similar proteins in other species as determined by a high percentage identity (e.g. above 50%, preferably above 90%), length of alignment and functional equivalence.

### References (Tables I - X)

1. Nakagawa, T., Goto, K. and Kondo, H., Cloning and characterization of a 92 kDa soluble phosphatidylinositol 4-kinase, Biochem. J,. 320 (Pt 2), 643-649 (1996).
2. Leung, T., Manser, E., Tan, L. and Lim, L., A novel serine/threonine kinase binding the Ras-related RhoA GTPase which translocates the kinase to peripheral membranes, J. Biol. Chem. 270 (49), 29051-29054 (1995); Leung, T., Chen, X.Q., Manser, E. and Lim, L., The p160 RhoA-binding kinase ROK alpha is a member of a kinase family and is involved in the reorganization of the cytoskeleton, Mol. Cell. Biol. 16 (10), 5313-5327 (1996).
3. Ahmed, K., Davis, A., Hanten, J., Lambert, D., McIvor, R.S. and Goueli, S.A., Cloning of cDNAs encoding the alpha and beta subunits of rat casein kinase 2 (CK-2): Investigation of molecular regulation of CK-2 by androgens in rat ventral prostate, Cell. Mol. Biol. Res. 39, 451-462 (1993).
4. Boulton, T.G., Nye, S.H., Robbins,D.J., Ip, N.Y., Radziejewska, E., Morgenbesser, S.D., DePinho, R.A., Panayotatos, N., Cobb, M.H. and Yancopoulos, G.D., ERKs: A family of protein-serine/threonine kinases that are activated and tyrosine phosphorylated in response to insulin and NGF, Cell 65, 663-675 (1991).
5. Middlemas, D.S., Lindberg, R.A. and Hunter, T., trkB, a neural receptor protein-tyrosine kinase: evidence for a full-length and two truncated receptors, Mol. Cell. Biol. 11, 143-153 (1991).
6. Carter, D.A., Expression of a novel rat protein tyrosine phosphatase gene, Biochim. Biophys. Acta 1442 (2-3), 405-408 (1998).
7. Kuno, T., Takeda, T., Hirai, M., Ito, A., Mukai, H. and Tanaka, C., Evidence for a second isoform of the catalytic subunit of calmodulin-dependent protein phosphatase (calcineurin A), Biochem. Biophys. Res. Commun. 165, 1352-1358 (1989).
8. Bolger, G.B., Rodgers, L. and Riggs, M., Differential CNS expression of alternative mRNA isoforms of the mammalian genes encoding cAMP-specific phosphodiesterases, Gene 149 (2), 237-244 (1994).
9. Chalon, P., Vita, N., Kaghad, M., Guillemot, M., Bonnin,J., Delpech, B., Le Fur, G., Ferrara, P. and Caput, D., Molecular cloning of a levocabastine-sensitive neurotensin binding site, FEBS Lett., 386 (2-3), 91-94 (1996).
10. Greenfeder S.A., Nunes, P., Kwee, L., Labow, M., Chizzonite, R.A. and Ju, G., Molecular cloning and characterization of a second subunit of the interleukin 1 receptor complex, J. Biol. Chem. 270 (23), 13757-13765 (1995).
11. Sugawara, M., Nakanishi, T., Fei, Y.J., Huang, W., Ganapathy, M.E., Leibach, F.H. and Ganapathy, V., Cloning of an amino acid transporter with functional characteristics and tissue expression pattern identical to that of system A, J. Biol. Chem. 275 (22), 16473-16477 (2000).
12. Noe, B., Hagenbuch, B., Stieger, B. and Meier, P.J., Isolation of a multispecific organic anion and cardiac glycoside transporter from rat brain, Proc. Natl. Acad. Sci. U.S.A. 94 (19), 10346-10350 (1997).
13. Feany, M.B., Lee, S., Edwards, R.H. and Buckley, K.M., The synaptic vesicle protein SV2 is a novel type of transmembrane transporter, Cell 70, 861-867 (1992).
14. Borowsky, B., Mezey, E. and Hoffman, B.J., Two glycine transporter variants with distinct localization in the CNS and peripheral tissues are encoded by a common gene, Neuron 10 (5), 851-863 (1993).
15. Gedeon, A.K., Colley, A., Jamieson, R., Thompson, E.M., Rogers, J., Sillence, D., Tiller, G.E., Mulley, J.C. and Gecz, J., Identification of the gene (SEDL) causing X-linked spondyloepiphyseal dysplasia tarda, Nat. Genet. 22 (4), 400-404 (1999).
16. Munakata, H., Yamagami, T., Nagai, T., Yamamoto, M. and Hayashi, N., Purification and structure of rat erythroid-specific delta-aminolevulinate synthase, J. Biochem. 114 (1), 103-111 (1993).
17. Hagen, F.K., Gregoire, C.A. and Tabak, L.A., Cloning and sequence homology of a rat UDP-GalNAc:polypeptide N-acetylgalactosaminyltransferase, Glycoconj. J. 12 (6), 901-909 (1995).
18. Ame, J.C., Rolli, V., Schreiber, V., Niedergang, C., Apiou, F., Decker, P., Muller, S., Hoger, T., Menissier-de Murcia, J. and de Murcia, G., PARP-2, A novel mammalian DNA damage-dependent poly(ADP-ribose) polymerase, J. Biol. Chem. 274 (25), 17860-17868 (1999).
19. Kahn, S., Yamamoto, F., Almoguera, C., Winter, E., Forrester, K., Jordano, J. and Perucho, M., The c-K-ras gene and human cancer (review), Anticancer Res. 7 (4A), 639-652 (1987).
20. Breiner, M., Schurmann, A., Becker, W. and Joost, H.G., Cloning of a novel member (ARL5) of the ARF-family of Ras-related GTPases, Biochim. Biophys. Acta 1308 (1), 1-6 (1996).
21. Yogosawa, S., Makino, Y., Yoshida, T., Kishimoto, T., Muramatsu, M. and Tamura, T., Molecular cloning of a novel 120-kDa TBP-interacting protein, Biochem. Biophys. Res. Commun. 229 (2), 612-617 (1996).
22. Mao, M., Fu, G., Wu, J.-S., Zhang, Q.-H., Zhou, J., Kan, L.-X., Huang, Q.-H., He, K.-L., Gu, B.-W., Han, Z.-G., Shen, Y., Gu, J., Yu, Y.-P., Xu, S.-H., Wang, Y., Chen, S.-J. and Chen, Z., Identification of genes expressed in human CD34(+) hematopoietic stem/progenitor cells by expressed sequence tags and efficient full-length cDNA cloning, Proc. Natl. Acad. Sci. U.S.A. 95 (14), 8175-8180 (1998); Zhang, Q.H., Ye, M., Wu, X.Y., Ren, S.X., Zhao, M., Zhao, C.J., Fu ,G., Shen,Y., Fan, H.Y., Lu, G., Zhong, M., Xu, X.R., Han, Z.G., Zhang, J.W., Tao, J., Huang, Q.H., Zhou, J., Hu, G.X., Gu, J., Chen, S.J. and Chen, Z., Cloning and functional analysis of cDNAs with open reading frames for 300 previously undefined genes expressed in CD34+ hematopoietic stem/progenitor cells, Genome Res. 10 (10), 1546-1560 (2000).
23. Springer, J., Kneissl, M., Putter, V. and Grummt, F., Identification and characterization of MmORC4 and MmORC5, two subunits of the mouse origin of replication recognition complex, Chromosoma 108 (4), 243-249 (1999).
24. Xin, X., Varlamov, O., Day, R., Dong, W., Bridgett, M.M., Leiter, E.H. and Fricker, L D., Cloning and sequence analysis of cDNA encoding rat carboxypeptidase D, DNA Cell Biol. 16 (7), 897-909 (1997).
25. Sato, N., Milligan, C.E., Uchiyama, Y. and Oppenheim, R.W., Cloning and expression of the cDNA encoding rat caspase-2, Gene 202 (1-2), 127-132 (1997).
26. Guenette, R.S., Mooibroek, M., Wong, K., Wong, P. and Tenniswood, M., Cathepsin B, a cysteine protease implicated in metastatic progression, is also expressed during regression of the rat prostate and mammary glands, Eur. J. Biochem. 226 (2), 311-321 (1994).
27. Ishidoh, K., Towatari, T., Imajoh, S., Kawasaki, H., Kominami, E., Katunuma, N. and Suzuki, K., Molecular cloning and sequencing of cDNA for rat cathepsin L, FEBS Lett. 223 (1), 69-73 (1987).
28. Wada, K., Yokotani, N., Hunter, C., Doi, K., Wenthold, R.J. and Shimasaki, S., Differential expression of two distinct forms of mRNA encoding members of a dipeptidyl aminopeptidase family, Proc. Natl. Acad. Sci. U.S.A. 89 (1), 197-201(1992).
29. Tanaka, K., Kanayama, H., Tamura, T., Lee, D.H., Kumatori, A., Fujiwara, T., Ichihara, A., Tokunaga, F., Aruga, R. and Iwanaga, S., cDNA cloning and sequencing of component C8 of proteasomes from rat hepatoma cells, Biochem. Biophys. Res. Commun. 171, 676-683, (1990).
30. Gnesutta, N., Ceriani, M., Innocenti, M., Mauri, I., Zippel, R., Sturani, E., Borgonovo, B., Berruti, G. and Martegani, E., Cloning and Characterization of Mouse UBPy, a Deubiquitinating Enzyme That Interacts with the Ras Guanine Nucleotide Exchange Factor CDC25Mm/Ras-GRF1, J. Biol. Chem. 276 (42), 39448-39454 (2001).
31. Kato, M., Yamaguchi, T., Kin, N. and Sekine, S., HUMAN UBIQUITIN-BOUND ENZYME E2 AND CDNA CODING THE SAME, Patent: JP 1997000262-A 1, 07-JAN-1997.
32. Pajot, B., Sarger, C., Bonnet, J. and Garret, M., An alternative splicing modifies the C-terminal end of tryptophanyl-tRNA synthetase in murine embryonic stem cells, J. Mol. Biol. 242 (4), 599-603 (1994).
33. Mukai, T., Joh, K., Arai, Y., Yatsuki, H. and Hori, K., Tissue-specific expression of rat aldolase A mRNAs: Three molecular species differing only in the 5'-terminal sequences, J. Biol. Chem. 261, 3347-3354 (1986).
34 Hangjiong, C., Williams, D. and Shapiro, D., Cloning and sequencing of rat 3-hydroxy-3-methylglutaryl coenzyme a reductase, J. Lipid Res. (1989).
35. Shinzawa, K., Inagaki, T., Ohishi, N., Ichihara, C., Tsukagoshi, N., Udaka, S. and Yagi, K., Molecular cloning of a cDNA for alpha-subunit of rat liver electron transfer flavoprotein, Biochem. Biophys. Res. Commun. 155, 300-304 (1988).
36. Munakata, H., Yamagami, T., Nagai, T., Yamamoto, M. and Hayashi, N., Purification and structure of rat erythroid-specific delta-aminolevulinate synthase, J. Biochem. 114(1), 103-111 (1993).
37. Hagen, F.K., Gregoire, C.A. and Tabak, L.A., Cloning and sequence homology of a rat UDP-GalNAc:polypeptide N-acetylgalactosaminyltransferase, Glycoconj. J. 12 (6), 901-909 (1995).
38. Iyer, R.K., Bando, J.M., Jenkinson, C.P., Vockley, J.G., Kim, P.S., Kern, R.M., Cederbaum, S.D. and Grody, W.W., Cloning and characterization of the mouse and rat type II arginase genes, Mol. Genet. Metab. 63 (3), 168-175 (1998).
39. Jabs, E.W., Thomas, P.J., Bernstein, M., Coss, C., Ferreira, G.C. and Pedersen, P.L. Chromosomal localization of genes required for the terminal steps of oxidative metabolism: alpha and gamma subunits of ATP synthase and the phosphate carrier, Hum. Genet. 93 (5), 600-602 (1994).
40. Kimoto, M., Sasakawa, T., Tsuji, H., Miyatake, S., Oka, T., Nio, N. and Ogawa, T., Cloning and sequencing of cDNA encoding NG,NG-dimethylarginine dimethylaminohydrolase from rat kidney, Biochim. Biophys. Acta 1337 (1), 6-10 (1997).
41. O'Neill, R.R., Tokoru, T., Kozak, C.A. and Brady, R.O., Comparison of the chromosomal localization of murine and human glucocerebrosidase genes and of the deduced amino acid sequences, Proc. Natl. Acad. Sci. U.S.A. 86, 5049-5053 (1989).
42. Robertson, D.A., Freeman, C., Morris, C.P. and Hopwood, J.J., A cDNA clone for human glucosamine-6-sulphatase reveals differences between arylsulphatases and non-arylsulphatases, Biochem. J. 288 (Pt 2), 539-544 (1992).
43. Kobayashi M., Seki T., Yaginuma K., Koike K. "Nucleotide sequences of small ribosomal RNA and adjacent transfer RNA, genes in rat mitochondrial DNA", Gene 16(1-3):297-307(1981); Saccone C., Cantatore P., Gadaleta G., Gallerani R., Lanave C., Pepe G., Kroon A.M., "The nucleotide sequence of the large ribosomal RNA gene and the adjacent tRNA genes from rat mitochondria", Nucleic Acids Res. 9(16):4139-4148(1981).
44. Yagita Y., Kitagawa K., Taguchi A., Ohtsuki T., Kuwabara K., Mabuchi T., RA Matsumoto M., Yanagihara T., Hori M., "Molecular cloning of a novel member of the HSP110 family of genes, ischemia-responsive protein 94 kDa (irp94), expressed in rat brain after transient forebrain ischemia", J. Neurochem. 72(4):1544-1551(1999).
45 Wei N., Tsuge T., Serino G., Dohmae N., Takio K., Matsui M., Deng X.W., "The COP9 complex is conserved between plants and mammals and is related to the 26S proteasome regulatory complex", Curr. Biol. 8(16):919-922(1998).
46. Baka I.D., Ninkina N.N., Pinon L.G., Adu J., Davies A.M., Georgiev G.P., RA Buchman V.L., "Intracellular compartmentalization of two differentially spliced s-rex/NSP mRNAs in neurons", Mol. Cell. Neurosci. 7(4):289-303(1996).
47. Stevanovic M., Paunesku T., Radosavljevic D., Drmanac R., Crkvenjakov R., "variant chromosomal arrangement of adult beta-globin genes in rat", Gene 79:139-150(1989).
48. Yogosawa, S., Makino, Y., Yoshida, T., Kishimoto, T., Muramatsu, M. and Tamura, T., Molecular cloning of a novel 120-kDa TBP-interacting protein, Biochem. Biophys. Res. Commun. 229 (2), 612-617 (1996).
49. Bodendorf, U., Cziepluch, C., Jauniaux, J.C., Rommelaere, J. and Salom, N., Nuclear export factor CRM1 interacts with nonstructural proteins NS2 from parvovirus minute virus of mice, J. Virol. 73 (9), 7769-7779 (1999).
50 Okazaki, T., Yoshida, B.N., Avraham, K.B., Wang, H., Wuenschell, C.W., Jenkins, N.A., Copeland, N.G., Anderson, D.J. and Mori, N., Molecular diversity of the SCG10/stathmin gene family in the mouse, Genomics 18 (2), 360-373 (1993).
51. Ogata, I., Saez, C.G., Greenwel, P., Ponce M de, L., Geerts, A.,, Leinwand, L.A. and Rojkind, M., Rat liver fat-storing cell lines express sarcomeric myosin heavy chain mRNA and protein, Cell Motil. Cytoskeleton 26 (2), 125-132 (1993).
52. Hirosawa, M., Nagase, T., Ishikawa, K., Kikuno, R., Nomura, N. and Ohara, O., Characterization of cDNA clones selected by the GeneMark analysis from size-fractionated cDNA libraries from human brain, DNA Res. 6 (5), 329-336 (1999).
53. Koster, F., Schinke, B., Niemann, S. and Hermans-Borgmeyer, I., Identification of shyc, a novel gene expressed in the murine developing and adult nervous system, Neurosci. Lett. 252 (1), 69-71 (1998).
54. Kindler, S., Muller, R., Chung, W.J. and Garner, C.C., Molecular characterization of dendritically localized transcripts encoding MAP2, Brain Res. Mol. Brain Res. 36 (1), 63-69 (1996).
55. Chiang, P.W., Wang, S., Smithivas, P., Song, W.J., Ramamoorthy, S., Hillman, J., Puett, S., Van Keuren, M.L., Crombez, E., Kumar, A., Glover, T.W., Miller, D.E., Tsai, C.H., Blackburn, C.C., Chen, X.N., Sun, Z., Cheng, J.F., Korenberg, J.R. and Kurnit, D.M., Identification and analysis of the human and murine putative chromatin structure regulator SUPT6H and Supt6h, Genomics 34 (3), 328-333 (1996).
56. Galcheva-Gargova, Z., Konstantinov, K.N., Wu, I.H., Klier, F.G., Barrett, T. and Davis, R.J., Binding of zinc finger protein ZPR1 to the epidermal growth factor receptor, Science 272 (5269), 1797-1802 (1996).
57. Ishikawa, K., Nagase, T., Suyama, M., Miyajima N., Tanaka, A., Kotani, H., Nomura, N. and Ohara, O., Prediction of the coding sequences of unidentified human genes. X. The complete sequences of 100 new cDNA clones from brain which can code for large proteins in vitro, DNA Res. 5 (3), 169-176 (1998).
58. Ann, D.K., Moutsatsos, I.K., Nakamura, T., Lin, H.H., Mao, P.-L., Lee, M.-J., Chin, S., Liem, R.K.H. and Wang, E., Isolation and characterization of the rat chromosomal gene for a polypeptide (pS1) antigenically related to statin, J. Biol. Chem. 266, 10429-10437 (1991).
59. Eckley, D.M., Gill, S.R., Melkonian, K.A., Bingham, J.B., Goodson, H.V., Heuser, J.E. and Schroer, T.A., Analysis of Dynactin Subcomplexes Reveals a Novel Actin-related Protein Associated with the Arp1 Minifilament Pointed End, J. Cell Biol. 147 (2), 307-320 (1999).
60. Maurel, P., Rauch, U., Flad, M., Margolis, R.K. and Margolis, R.U., Phosphacan, a chondroitin sulfate proteoglycan of brain that interacts with neurons and neural cell-adhesion molecules, is an extracellular variant of a receptor-type protein tyrosine phosphatase, Proc. Natl. Acad. Sci. U.S.A. 91 (7), 2512-2516 (1994).
61. Elfring L.K., Deuring, R., McCallum, C.M., Peterson, C.L. and Tamkun, J.W., Identification and characterization of Drosophila relatives of the yeast transcriptional activator SNF2/SWI2, Mol. Cell. Biol. 14 (4), 2225-2234 (1994).
62. Teasdale, R.D., Loci, D., Houghton, F., Karlsson, L. and Gleeson, P.A., A large family of endosome-localized proteins related to sorting nexin 1, Biochem. J. 358 (Pt 1), 7-16 (2001).
63. Honore, B., Madsen, P.S., Andersen, A.H. and Leffers, H., Cloning and expression of a novel human profilin variant, profilin II, FEBS Lett. 330, 151-155 (1993).
64. Bonnet, F., Perin, J.P., Charbonnier, F., Camuzat, A., Roussel, G., Nussbaum, J.L. and Alliel, P.M., Structure and cellular distribution of mouse brain testican. Association with the postsynaptic area of hippocampus pyramidal cells, J. Biol. Chem. 271 (8), 4373-4380 (1996).
65. Lehar, S.M., Nacht, M., Jacks, T., Vater, C.A., Chittenden, T. and Guild, B.C., Identification and cloning of EI24, a gene induced by p53 in etoposide-treated cells, Oncogene 12 (6), 1181-1187 (1996).
66. Belgrader, P., Dey, R. and Berezney, R., Molecular cloning of matrin 3. A 125-kilodalton protein of the nuclear matrix contains an extensive acidic domain, J. Biol. Chem. 266, 9893-9899 (1991).
67. Zalcman, G., Closson, V., Camonis, J., Honore, N., Rousseau-Merck, M.F., Tavitian, A. and Olofsson, B., RhoGDI-3 is a new GDP dissociation inhibitor (GDI). Identification of a non-cytosolic GDI protein interacting with the small GTP-binding proteins RhoB and RhoG, J. Biol. Chem. 271 (48), 30366-30374 (1996).
68. Hamilton, B.A., Smith, D.J., Mueller, K.L., Kerrebrock, A.W., Bronson, R.T., van Berkel, V., Daly, M.J., Kruglyak, L., Reeve, M.P., Nemhauser, J.L., Hawkins, T.L., Rubin, E.M. and Lander, E.S., The vibrator mutation causes neurodegeneration via reduced expression of PITP alpha: positional complementation cloning and extragenic suppression, Neuron 18 (5), 711-722 (1997).
69. Hart P.E., Poynter, G.M., Whitehead, C.M., Orth, J.D., Glantz, J.N., Busby, R.C., Barrett, S.L. and Salisbury, J.L., Characterization of the X-linked murine centrin Cetn2 gene, Gene 264 (2), 205-213 (2001).
70. Ginzburg, I., Teichman, A., Dodemont, H.J., Behar, L. and Littauer, U.Z., Regulation of three beta-tubulin mRNAs during rat brain development, EMBO J. 4 (13B), 3667-3673 (1985).
71. Chan, D.C., Bedford, M.T. and Leder, P., Formin binding proteins bear WWP/WW domains that bind proline-rich peptides and functionally resemble SH3 domains, EMBO J. 15 (5), 1045-1054 (1996).
72. Scanlan, M.J., Gordan, J.D., Williamson, B., Stockert, E., Bander, N.H., Jongeneel, V., Gure, A.O., Jager, D., Jager, E., Knuth, A., Chen, Y.-T. and Old, L.J., Antigens recognized by autologous antibody in patients with renal-cell carcinoma, Int. J. Cancer 83 (4), 456-464 (1999).
73. Kawabe, H., Hata, Y., Takeuchi, M., Ide, N., Mizoguchi, A. and Takai, Y., nArgBP2, a novel neural member of ponsin/ArgBP2/vinexin family that interacts with synapse-associated protein 90/postsynaptic density-95-associated protein (SAPAP), J. Biol. Chem. 274 (43), 30914-30918 (1999).
74. Shankar, S., Mohapatra, B. and Suri, A., Cloning of a novel human testis mRNA specifically expressed in testicular haploid germ cells, having unique palindromic sequences and encoding a leucine zipper dimerization motif, Biochem. Biophys. Res. Commun. 243 (2), 561-565 (1998).
75. Imamichi, T., Uchida, I., Wahl, S.M. and McCartney-Francis, N., Expression and cloning of migration inhibitory factor-related protein (MRP)8 and MRP14 in arthritis-susceptible rats, Biochem. Biophys. Res. Commun. 194, 819-825 (1993).
76. Bauer, V.W., Swaffield, J.C., Johnston, S.A. and Andrews, M.T., CADp44: a novel regulatory subunit of the 26S proteasome and the mammalian homolog of yeast Sug2p, Gene 181 (1-2), 63-69 (1996).
77. Zhang, Q.H., Ye, M., Wu, X.Y., Ren, S.X., Zhao, M., Zhao, C.J., Fu, G., Shen, Y., Fan, H.Y., Lu, G., Zhong, M., Xu, X.R., Han,Z.G., Zhang,J.W., Tao, J., Huang, Q.H., Zhou, J., Hu, G.X., Gu, J., Chen, S.J. and Chen, Z., Cloning and functional analysis of cDNAs with open reading frames for 300 previously undefined genes expressed in CD34+ hematopoietic stem/progenitor cells, Genome Res. 10 (10), 1546-1560 (2000).
78. Elgart, G.W. and Stanley, J.R., Cloning of the 5' mRNA for the 230-kD bullous pemphigoid antigen by rapid amplification of cDNA ends, J. Invest. Dermatol. 101 (2), 244-246 (1993).
79. Lee, Y.J., Park, C.W., Hahn, Y., Park, J., Lee, J., Yun, J.H., Hyun, B., and Chung,J.H., Mitl/Lb9 and Copg2, new members of mouse imprinted genes closely linked to Pegl/Mest(1), FEBS Lett. 472 (2-3), 230-234 (2000).
80. Cenciarelli, C., Chiaur, D.S., Guardavaccaro, D., Parks, W., Vidal, M and Pagano, M., Identification of a family of human F-box proteins, Curr. Biol. 9 (20), 1177-1179 (1999).
81. Nagase, T., Seki, N., Ishikawa, K., Ohira, M., Kawarabayasi, Y., Ohara, O., Tanaka, A., Kotani, H., Miyajima, N. and Nomura, N., Prediction of the coding sequences of unidentified human genes. VI. The coding sequences of 80 new genes (KIAA0201-KIAA0280) deduced by analysis of cDNA clones from cell line KG-1 and brain, DNA Res. 3 (5), 321-329 (1996).
82. Nagase, T., Ishikawa, K., Kikuno, R., Hirosawa, M., Nomura, N. and Ohara, O., Prediction of the coding sequences of unidentified human genes. XV. The complete sequences of 100 new cDNA clones from brain which code for large proteins in vitro, DNA Res. 6 (5), 337-345 (1999).
83. Ujita, M., Shinomura, T. and Kimata, K., Molecular cloning of the mouse osteoglycin-encoding gene, Gene 158 (2), 237-240 (1995).
84. Chan, Y.L., Olvera, J., Paz, V. and Wool, I.G., The primary structure of rat ribosomal protein L11, Biochem. Biophys. Res. Commun. 185 (1), 356-362 (1992).
85. Birling, M.C., Tait, S., Hardy, R.J. and Brophy, P.J., A novel rat tetraspan protein in cells of the oligodendrocyte lineage, J. Neurochem. 73 (6), 2600-2608 (1999).
86. Mahnke-Zizelman, D.K., D'cunha, J., Wojnar, J.M., Brogley, M.A. and Sabina, R.L., Regulation of rat AMP deaminase 3 (isoform C) by development and skeletal muscle fibre type, Biochem. J. 326 (Pt 2), 521-529 (1997).
87. Satoh, H., Fujii, H. and Okazaki, T., Molecular cloning and sequence analysis of two rat major globin cDNAs, Biochem. Biophys. Res. Commun. 146, 618-624 (1987).
88. Gadaleta, G., Pepe, G., De Candia, G., Quagliariello, C., Sbisa, E. and Saccone, C. The complete nucleotide sequence of the Rattus norvegicus mitochondrial genome: cryptic signals revealed by comparative analysis between vertebrates, Journal of molecular evolution. 28 (6), 497-516 (1989).
89. Fukunaga-Johnson, N., Lee, S.W., Liebert, M. and Grossman, H.B., Molecular analysis of a gene, BB1, overexpressed in bladder and breast carcinoma, Anticancer Res. 16 (3A), 1085-1090 (1996).
90. Lai, C.H., Chou, C.Y., Ch'ang, L.Y., Liu, C.S. and Lin, Wc., Identification of novel human genes evolutionarily conserved in Caenorhabditis elegans by comparative proteomics, Genome Res. 10 (5), 703-713 (2000).
91. Milner, R.J., Lai, C., Nave, K.-A., Lenoir, D., Ogata, J. and Sutcliffe, J.G., Nucleotide sequences of two mRNAs for rat brain myelin proteolipid protein, Cell 42, 931-939 (1985).

## Claims

1. Use of:
(a) an isolated gene sequence that is up regulated in the spinal cord in response to streptozocin-induced diabetes;
(b) an isolated gene sequence comprising a nucleic acid sequence of Tables I to X.
(c) an isolated gene sequence having at least 80% sequence identity with a nucleic acid sequence of Tables I to X;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell containing the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus; or
(i) an isolated polypeptide encoded by a nucleotide sequence according to any one of (a) to (d); or
(j) an isolated antibody that binds specifically to a polypeptide according to (h) or (i);
in the screening of compounds for the treatment of pain, or for the diagnosis of pain.

2. Use according to claim 1 wherein the isolated gene sequence encodes a kinase.

3. Use according to claim 1 or 2, wherein the isolated gene sequence encodes an expression product or fragment thereof of Janus protein tyrosine kinase 1 (JAK1), (AJ000556, S63728), phosphatidylinositol 4-kinase, (D84667) or Rho kinase alpha (U38481, U58513, D87931).

4. Use according to claim 1 or 2, wherein the isolated gene sequence encodes an expression product or fragment thereof of casein kinase II beta subunit (L15619), ERK3, protein serine/threonine kinase, extracellular (M64301, X80692), or neural receptor protein-tyrosine kinase (trkB), (M55291, X17647).

5. Use according to claim 1 wherein the isolated gene sequence encodes a phosphatase.

6. Use according to claim 1 or 5, wherein the isolated gene sequence encodes an expression product or fragment thereof of protein tyrosine phosphatase (AJ007016, AF035644, U48297), putative receptor tyrosine phosphatase (U55057) or calcineurin, A subunit, beta isoform (M31809, M29550).

7. Use according to claim 1 wherein the isolated gene sequence encodes a phosphodiesterase.

8. Use according to claim 1 or 7, wherein the isolated gene sequence encodes an expression product or fragment thereof of phosphodiesterase, cAMP-specific (PDE4D) (L27058, L20966).

9. Use according to claim 1 wherein the isolated gene sequence encodes a receptor protein.

10. Use according to claim 1 or 9, wherein the isolated gene sequence encodes an expression product or fragment thereof of neurotensin receptor type 2 (NTR2) (X97121) or putative interleukin 1 receptor accessory protein (X85999).

11. Use according to claim 1 wherein the isolated gene sequence encodes a transporter protein.

12. Use according to claim 1 or 11, wherein the isolated gene sequence encodes an expression product or fragment thereof of amino acid system A transporter (AF249673), digoxin carrier protein (U88036), synaptic vesicle transmembrane transporter (L01788), glycine transporter (L13600), putative SEDL spondylo-epiphesial dysplasia tarda gene (AF157065), delta-aminolevulinate synthase, erythroid-specific (D86297), polypeptide GalNAc transferase T1 (U35890) or putative NAD⁺ ADP-ribosyltransferase (AJ007780).

13. Use according to claim 1 wherein the isolated gene sequence encodes a G-protein coupled receptor protein.

14. Use according to claim 1 or 13, wherein the isolated gene sequence encodes an expression product or fragment thereof of putative K-ras (M54968) or ARF-like protein 5 ARL5 (X78604).

15. Use according to claim 1 wherein the isolated gene sequence encodes a DNA binding protein.

16. Use according to claim 1 or 15, wherein the isolated gene sequence encodes an expression product or fragment thereof of TBP-binding protein TIP 120 (D87671), putative chromodomain-helicase-DNA-binding protein (AF054177) or putative origin recognition complex subunit 4 : Orc 4 gene (AJ003140).

17. Use according to claim 1 wherein the isolated gene sequence encodes a protease protein.

18. Use according to claim 1 or 17, wherein the isolated gene sequence encodes an expression product or fragment thereof of carboxypeptidase D precursor (U62897, D85391, U65090), caspase 2 (Ich-1) (U77933), cathepsin B (X82396, M14222), cathepsin L (EC 34.22.15) (Y00697, M20495), dipeptidylpeptidase (dpp6) (M76427, AF092507, M96859), proteasome subunit C8 (M58593, AF055983, D00762) or putative deubiquitinating enzyme 8 (AF057146 D29956).

19. Use according to claim 1 wherein the isolated gene sequence encodes a G-protein coupled receptor protein.

20. Use according to claim 1 or 19, wherein the isolated gene sequence encodes an expression product or fragment thereof of putative K ras (M54968) or ARF-like protein 5 ARL 5 (X78604).

21. Use according to claim 1 wherein the isolated gene sequence encodes a ligase, lyase, oxidoreductase, transferase or hydrolase.

22. Use according to claim 1 or 21, wherein the isolated gene sequence encodes an expression product or fragment thereof of putative ubiquitin-conjugating enzyme (EC 6.3.2.-) E2 (E12457), putative tryptophanyl-tRNA synthetase (X69656), aldolase A (M12919, Y00516, M11560), 3-hydroxy-3-methylglutaryl coenzyme A reductase (M29249, M11058), NADH subunit 5, mitochondrial (X14848), electron transfer flavoprotein (ETF), alpha subunit (M22030), delta-amino-levulinate synthase, erythroid-specific (D86297), polypeptide GalNAc transferase T1 (35890), putative NAD+ ADP-ribosyl-transferase (AJ007780), arginase II (U90887, U90886), ATP synthase gamma chain, mitochondrial (L1992, D16563), N-G,N-G-dimethylarginine dimethylaminohydrolase (D86041), putative gluco-cerebrosidase (M24119), putative N-acetyl-glucosamine-6-sulfatase (Z12173), putitive ubiquitin conjugating enzyme (Y17267), or NADH subunit 5, mitochondrial (14848).

23. A non-human animal having in its genome an introduced gene sequence or a removed or down-regulated gene sequence , said gene sequence being up-regulated in the spinal cord of a mammal in response to streptozocin-induced diabetes.

24. A non-human animal according to claim 23, wherein the introduced gene sequence is according to any of claims 1 to 22

25. A non-human animal according to claim 23 or 24 which is *C. elegans.*

26. A kit comprising;
(a) affinity peptide and/or ligand and/or substrate for an expression product of a gene sequence that is up-regulated in the spinal cord of a mammal in response to streptozocin-induced diabetes; and
(b) a defined quantity of an expression product of a gene sequence that is up-regulated in the spinal cord of a mammal in response to streptozocin-induced diabetes,
for simultaneous, separate or sequential use in detecting and/or quantifying up-regulation of a gene sequence in the spinal cord of a mammal in response to streptozocin-induced diabetes.

27. A kit according to claim 26, wherein the gene sequence is defined in any of claims 1 to 22.

28. A kit comprising:
(a) nucleic acid sequences capable of hybridization to a nucleic acid sequence that is up-regulated in the spinal cord of a mammal in response to streptozocin-induced diabetes; and
(b) a defined quantity of one or more nucleic acid sequences capable of hybridization to a nucleic acid sequence that is up-regulated in the spinal cord of a mammal in response to streptozocin-induced diabetes,
for simultaneous, separate or sequential use in detecting and/or quantifying up-regulation of a gene sequence in the spinal cord of a mammal in response to streptozocin-induced diabetes.

29. The kit of claim 28, wherein the gene sequence is according to any of claims 1 to 22.

30. A compound that modulates the action of an expression product of a gene sequence that is up-regulated in the spinal cord of a mammal in response to streptozocin-induced diabetes.

31. A compound according to claim 30 wherein the gene sequence is listed in Tables I to X.

32. A compound according to claim 30 or 31 wherein the nucleotide sequence is according to any one of claims 1 to 22.

33. A compound according to any one of claims 30 to 32 for use as a medicament.

34. A compound according to any one of claims 30 to 33 for the treatment or diagnosis of pain.

35. A pharmaceutical composition comprising a compound according to any one of claims 30 to 34 and a pharmaceutically acceptable carrier or diluent.

36. Use of a compound according to any one of claims 30 to 34 in the manufacture of a medicament for the treatment or diagnosis of pain.

37. Use of a compound according to any one of claims 30 to 34 in the manufacture of a medicament for the treatment or diagnosis of chronic pain.

38. A method of treatment of pain, which comprises administering to a patient an effective amount of a compound according to any one of claims 30 to 34.


SEQUENCE LISTING

<110> Warner-Lambert Company
      Lee, Kevin
      Dixon, Alistair
      Brooksbank, Robert
      Pinnock, Robert

<120> Identification and Use of Molecules Implicated in Pain

<130> WL-A-018200

<150> GB 0118354.0
<151> 2001-07-27

<160> 197

<170> PatentIn Ver. 2.1

<210> 1
<211> 866
<212> PRT
<213> Rattus norvegicus

<220>
<223> Janus protein tyrosine kinase 1

<400> 1
His Ser Pro Lys Lys Gln Lys Asn Gly Tyr Glu Lys Lys Arg Val Pro
  1               5                  10                  15

Glu Ala Thr Pro Leu Leu Asp Ala Ser Ser Leu Glu Tyr Leu Phe Ala
             20                  25                  30

Gln Gly Gln Tyr Asp Leu Ile Lys Cys Leu Ala Pro Ile Arg Asp Pro
         35                  40                  45

Lys Thr Glu Gln Asp Gly His Asp Ile Glu Asn Glu Cys Leu Gly Met
     50                  55                  60

Ala Val Leu Ala Ile Ser His Tyr Ala Met Met Lys Lys Met Gln Leu
 65                  70                  75                  80

Pro Glu Leu Pro Lys Asp Ile Ser Tyr Lys Arg Tyr Ile Pro Glu Thr
                 85                  90                  95

Leu Asn Lys Ser Ile Arg Gln Arg Asn Leu Leu Thr Arg Met Arg Ile
            100                 105                 110

Asn Asn Val Phe Lys Asp Phe Leu Lys Glu Phe Asn Asn Lys Thr Ile
        115                 120                 125

Cys Asp Ser Ser Val Ser Thr His Asp Leu Lys Val Lys Tyr Leu Ala
    130                 135                 140

Thr Leu Glu Thr Leu Thr Lys His Tyr Gly Ala Glu Ile Phe Glu Thr
145                 150                 155                 160

Ser Met Leu Leu Ile Ser Ser Glu Asn Glu Pro Ser Arg Cys His Ser
                165                 170                 175

Asn Asp Ser Gly Asn Val Leu Tyr Glu Val Met Val Thr Gly Asn Leu
            180                 185                 190

Gly Ile Gln Trp Arg Gln Lys Pro Asn Val Leu Pro Val Glu Lys Glu
        195                 200                 205

Lys Asn Lys Leu Lys Arg Lys Lys Leu Asp Tyr Ile Lys His Lys Lys
    210                 215                 220

Glu Asp Ala Arg Asn Lys Leu Arg Glu Glu Trp Asn Ser Phe Ser Tyr
225                 230                 235                 240

Phe Pro Glu Ile Thr His Ile Val Ile Lys Glu Ser Val Val Ser Ile
                245                 250                 255

Asn Lys Gln Asp Asn Lys Asn Met Glu Leu Lys Leu Ser Ser His Glu
            260                 265                 270

Gly Ala Leu Ser Phe Val Ser Leu Val Asp Gly Tyr Phe Arg Leu Thr
        275                 280                 285

Ala Asp Ala His His Tyr Leu Cys Thr Asp Val Ala Pro Pro Leu Ile
    290                 295                 300

Val His Asn Ile Gln Asn Gly Cys His Gly Pro Ile Cys Thr Asp Ile
305                 310                 315                 320

Ala Phe Asn Lys Leu Arg Gln Glu Gly Ser Glu Glu Gly Met Tyr Val
                325                 330                 335

Leu Arg Trp Ser Cys Thr Asp Phe Asp Asn Ile Leu Ile Ala Val Ser
            340                 345                 350

Cys Phe Glu Lys Ser Glu Val Leu Gly Asn Gln Lys Gln Phe Lys Asn
        355                 360                 365

Phe Gln Ile Glu Val Gln Lys Gly Arg Tyr Ser Leu His Gly Ser Val
    370                 375                 380

Asp His Phe Pro Ser Leu Arg Asp Leu Met Asn His Leu Lys Lys Gln
385                 390                 395                 400

Ile Leu Arg Thr Asp Asn Ile Ser Phe Val Leu Lys Arg Cys Cys Gln
                405                 410                 415

Ser Lys Pro Arg Glu Ile Ser Asn Leu Leu Val Ala Thr Lys Lys Ala
            420                 425                 430

Gln Glu Trp Gln Pro Val Tyr Ser Met Ser Gln Leu Ser Phe Asp Arg
        435                 440                 445

Ile Leu Lys Lys Asp Ile Ile Gln Gly Glu His Leu Gly Arg Gly Thr
    450                 455                 460

Arg Thr His Ile Tyr Ser Gly Thr Leu Leu Asp Tyr Lys Asp Asp Glu
465                 470                 475                 480

Gly Ile Ala Glu Glu Lys Lys Ile Lys Val Ile Leu Lys Val Leu Asp
                485                 490                 495

Pro Ser His Arg Asp Ile Ser Leu Ala Phe Phe Glu Ala Ala Ser Met
            500                 505                 510

Met Arg Gln Val Ser His Lys His Ile Val Tyr Leu Tyr Gly Val Cys
        515                 520                 525

Val Arg Asp Val Glu Asn Ile Met Val Glu Glu Phe Val Glu Gly Gly
    530                 535                 540

Pro Leu Asp Leu Phe Met His Arg Lys Ser Asp Ala Leu Thr Thr Pro
545                 550                 555                 560

Trp Lys Phe Lys Val Ala Lys Gln Leu Ala Ser Ala Leu Ser Tyr Leu
                565                 570                 575

Glu Asp Lys Asp Leu Val His Gly Asn Val Cys Thr Lys Asn Leu Leu
            580                 585                 590

Leu Ala Arg Glu Gly Ile Asp Ser Asp Ile Gly Pro Phe Ile Lys Leu
        595                 600                 605

Ser Asp Leu Gly Ile Pro Val Ser Val Leu Thr Arg Gln Glu Cys Ile
    610                 615                 620

Glu Arg Asn Pro Trp Ile Ala Pro Glu Cys Val Glu Asp Ser Lys Asn
625                 630                 635                 640

Leu Ser Val Ala Ala Asp Lys Trp Ser Phe Gly Thr Thr Leu Trp Glu
                645                 650                 655

Ile Arg Tyr Asp Gly Glu Ile Pro Leu Lys Asp Lys Thr Leu Ile Glu
            660                 665                 670

Lys Glu Arg Phe Tyr Glu Ser Arg Cys Arg Pro Val Thr Pro Ser Cys
        675                 680                 685

Lys Glu Leu Ala Asp Leu Met Thr Arg Cys Met Asn Tyr Asp Pro Asn
    690                 695                 700

Gln Arg Pro Phe Phe Arg Ala Ile Met Arg Asp Ile Asn Lys Leu Glu
705                 710                 715                 720

Glu Gln Asn Pro Asp Ile Val Ser Glu Lys His Pro Ile Thr Glu Val
                725                 730                 735

Asp Pro Thr His Phe Glu Lys Arg Phe Leu Lys Arg Ile Arg Asp Leu
            740                 745                 750

Gly Glu Gly His Phe Gly Lys Val Glu Leu Cys Arg Tyr Asp Pro Glu
        755                 760                 765

Gly Asp Asn Thr Gly Glu Gln Val Ala Val Lys Ser Leu Lys Pro Glu
    770                 775                 780

Ser Gly Gly Asn His Ile Ala Asp Leu Lys Lys Glu Ile Glu Ile Leu
785                 790                 795                 800

Arg Asn Leu Tyr His Glu Asn Ile Val Lys Tyr Lys Gly Ile Cys Met
                805                 810                 815

Glu Asp Gly Gly Asn Gly Ile Lys Leu Ile Met Glu Phe Leu Pro Ser

            820                 825                 830

Gly Ser Leu Lys Glu Tyr Leu Pro Lys Asn Lys Asn Lys Ile Asn Leu
        835                 840                 845

Lys Gln Gln Leu Lys Tyr Ala Ile Gln Ile Cys Lys Gly Met Asp Tyr
    850                 855                 860

Leu Gly
865

<210> 2
<211> 2597
<212> DNA
<213> Rattus norvegicus

<220>
<223> Janus protein tyrosine kinase 1

<400> 2
cattctccaa agaagcagaa aaatggctat gagaagaaaa gggttccaga agcaacccca 60
ctccttgatg ccagttcact ggagtatctg tttgcgcagg gacagtatga tttgatcaaa 120
tgcctggctc ccattcggga ccccaagaca gagcaagatg gacatgatat tgagaatgag 180
tgcctgggca tggctgtcct ggccatctca cactatgcca tgatgaagaa gatgcagttg 240
ccagagcttc ccaaagacat cagctacaag cgatatattc cagaaacatt gaataaatcc 300
atcagacaga ggaatcttct taccaggatg cgaataaata atgttttcaa ggatttcttg 360
aaggaattta acaacaagac catctgtgac agcagtgtgt ctacacacga cctgaaggtg 420
aagtacctgg ctaccttgga aaccttaacc aagcattatg gagccgagat atttgagact 480
tctatgctcc tgatttcatc agaaaatgag ccgagtcggt gccattccaa cgacagtggc 540
aatgttctct atgaagtcat ggtaacagga aatctcggga tccagtggcg gcagaaacca 600
aatgttcttc ctgttgaaaa ggaaaaaaat aaactgaagc ggaaaaaact ggactatatt 660
aaacacaaga aggaggatgc cagaaacaaa ctccgggaag agtggaacag tttttcctat 720
ttccccgaga ttacccacat tgttatcaag gagtctgtgg tcagcattaa caaacaggac 780
aacaagaaca tggaactaaa gctctcttct catgagggag ccttgtcctt cgtgtccctg 840
gtagatggct acttccggct cactgcagat gctcaccatt acctctgtac tgatgtggct 900
cccccgctga ttgtccacaa catacagaat ggctgtcacg gtccaatctg cacagatatt 960
gcattcaata agctgcggca ggaggggagt gaggagggga tgtacgtgct gaggtggagc 1020
tgcaccgact ttgacaacat tcttattgct gtcagctgct tcgaaaagtc cgaggtattg 1080
ggtaatcaga agcagttcaa gaactttcag attgaggtgc agaagggccg ctacagcctg 1140
catggctctg ttgaccactt tcccagcctg agagacctca tgaaccacct caagaagcag 1200
atcctgcgca cggacaatat aagctttgtg ctgaaacgct gctgtcagtc taagcctcga 1260
gaaatctcca acctactagt agccactaag aaagcccagg agtggcagcc tgtctactct 1320
atgagccagc tgagtttcga tcggatcctt aagaaagata ttatccaagg tgagcacctt 1380
ggcagaggca caagaacaca tatctattct gggaccctgc tggattacaa ggacgatgaa 1440
ggaattgctg aagaaaagaa gataaaagtg attctcaaag tcctagaccc tagccaccgg 1500
gacatttcac tggccttctt tgaggcagcc agcatgatga gacaggtttc ccacaaacac 1560
atcgtgtacc tctacggcgt ctgtgtccga gatgtagaaa atatcatggt ggaagagttt 1620
gtggaggggg ggccattgga tctcttcatg caccggaaaa gtgatgcact tactacccct 1680
tggaagttta aagttgccaa acagctggcc agcgccctga gttacttgga agataaagac 1740
ctcgttcatg gaaatgtgtg cactaaaaac ctcctcctgg cccgtgaggg cattgacagt 1800
gacattggcc cgttcatcaa gcttagtgac cttggcatcc cagtctctgt gctgaccagg 1860
caagagtgca tagagcgaaa tccctggatc gctcctgagt gtgttgaaga ctccaagaac 1920
ctgagtgtgg ctgctgacaa gtggagcttt ggaaccacac tctgggaaat ccgctacgat 1980
ggcgagatcc cactcaagga caagaccctc attgagaaag agaggtttta tgaaagccgc 2040
tgcaggccag tgacaccatc ttgcaaggag ctagctgacc tcatgactcg ctgcatgaac 2100
tatgatccca accagagacc tttcttccga gccatcatga gggacattaa caagctggag 2160
gagcagaatc cagacattgt ttcagaaaag cacccaataa cagaggtgga tcccactcat 2220
tttgaaaagc gtttcctaaa gaggattcgt gacttgggag agggtcactt tgggaaggtt 2280
gagctctgca gatatgatcc tgagggagac aacacagggg aacaggtagc tgtcaagtcc 2340
ctgaagcctg agagtggagg taaccacata gctgatctga agaaggagat agagatctta 2400

cggaacctct accacgagaa cattgtgaag tataaaggaa tctgcatgga agacgggggg 2460
aatggtatca agctcatcat ggagtttctg ccttcgggaa gcctaaagga atatctgcca 2520
aagaataaga acaaaatcaa cctcaaacag cagctaaaat atgccatcca gatttgtaag 2580
gggatggact atctggg 2597

<210> 3
<211> 816
<212> PRT
<213> Rattus norvegicus

<220>
<223> Phosphatidylinisitol 4-kinase

<400> 3
Met Gly Asp Met Val Val Glu Pro Ala Thr Leu Lys Pro Thr Ser Glu
1               5                   10                  15

Pro Thr Pro Ser Pro Ser Gly Asn Asn Gly Gly Ser Leu Leu Ser Val
            20                  25                  30

Ile Thr Glu Gly Val Gly Glu Leu Ser Val Ile Asp Pro Glu Val Ala
        35                  40                  45

Gln Lys Ala Cys Gln Glu Val Leu Glu Lys Val Lys Leu Leu His Gly
    50                  55                  60

Gly Val Ala Ile Ser Ser Lys Gly Ser Pro Leu Glu Leu Val Asn Gly
65                  70                  75                  80

Asp Gly Val Asp Asn Glu Ile Arg Cys Leu Asp Asp Pro Pro Thr Glu
                85                  90                  95

Ile Arg Glu Glu Glu Asp Glu Met Glu Pro Gly Val Val Ser Gly Thr
            100                 105                 110

Ala Lys Gly Thr Arg Arg Arg Arg Gln Asn Asn Ser Ala Lys Gln Ser
        115                 120                 125

Trp Leu Leu Arg Leu Phe Glu Ser Lys Leu Phe Asp Ile Ser Met Ala
    130                 135                 140

Ile Ser Tyr Leu Tyr Asn Ser Lys Glu Pro Gly Val Gln Ala Tyr Ile
145                 150                 155                 160

Gly Asn Arg Leu Phe Cys Phe Arg Asn Glu Asp Val Asp Phe Tyr Leu
                165                 170                 175

Pro Gln Leu Leu Asn Met Tyr Ile His Met Asp Glu Asp Val Gly Asp
            180                 185                 190

Ala Ile Lys Pro Tyr Ile Val His Arg Cys Arg Gln Ser Ile Asn Phe
        195                 200                 205

Ser Leu Gln Cys Ala Leu Leu Leu Gly Ala Tyr Ser Ser Asp Met His
    210                 215                 220

Ile Ser Thr Gln Arg His Ser Arg Gly Thr Lys Leu Arg Lys Leu Ile
225                 230                 235                 240

Leu Ser Asp Glu Leu Lys Pro Ala His Arg Lys Arg Glu Leu Pro Thr

245                 250                 255

Leu Ser Pro Ala Pro Asp Thr Gly Leu Ser Pro Ser Lys Arg Thr His
            260                 265                 270

Gln Arg Ser Lys Ser Asp Ala Thr Ala Ser Ile Ser Leu Ser Ser Asn
        275                 280                 285

Leu Lys Arg Thr Ala Ser Asn Pro Lys Val Glu Asn Glu Asp Glu Glu
    290                 295                 300

Leu Ser Ser Ser Thr Glu Ser Ile Asp Asn Ser Phe Ser Ser Pro Val
305                 310                 315                 320

Arg Leu Ala Pro Glu Arg Glu Phe Ile Lys Ser Leu Met Ala Ile Gly
                325                 330                 335

Lys Arg Leu Ala Thr Leu Pro Thr Lys Glu Gln Lys Thr Gln Arg Leu
            340                 345                 350

Ile Ser Glu Leu Ser Leu Leu Asn His Lys Leu Pro Ala Arg Val Trp
        355                 360                 365

Leu Pro Thr Ala Gly Phe Asp His His Val Val Arg Val Pro His Thr
    370                 375                 380

Gln Ala Val Val Leu Asn Ser Lys Asp Lys Ala Pro Tyr Leu Ile Tyr
385                 390                 395                 400

Val Glu Val Leu Glu Cys Glu Asn Phe Asp Thr Thr Asn Val Pro Ala
                405                 410                 415

Arg Ile Pro Glu Asn Arg Ile Arg Ser Thr Arg Ser Val Glu Asn Leu
            420                 425                 430

Pro Glu Cys Gly Ile Thr His Glu Gln Arg Ala Gly Ser Phe Ser Thr
        435                 440                 445

Val Pro Asn Tyr Asp Asn Asp Asp Glu Ala Trp Ser Val Asp Asp Ile
    450                 455                 460

Gly Glu Leu Gln Val Glu Leu Pro Glu Val His Thr Asn Ser Cys Asp
465                 470                 475                 480

Asn Ile Ser Gln Phe Ser Val Asp Ser Ile Thr Ser Gln Glu Ser Lys
                485                 490                 495

Glu Pro Val Phe Ile Ala Ala Gly Asp Ile Arg Arg Arg Leu Ser Glu
            500                 505                 510

Gln Leu Ala His Thr Pro Thr Ala Phe Lys Arg Asp Pro Glu Asp Pro
        515                 520                 525

Ser Ala Val Ala Leu Lys Glu Pro Trp Gln Glu Lys Val Arg Arg Ile
    530                 535                 540

Arg Glu Gly Ser Pro Tyr Gly His Leu Pro Asn Trp Arg Leu Leu Ser
545                 550                 555                 560

Val Ile Val Lys Cys Gly Asp Asp Leu Arg Gln Glu Leu Leu Ala Phe
                565                 570                 575

Gln Val Leu Lys Gln Leu Gln Ser Ile Trp Glu Gln Glu Arg Val Pro
            580                 585                 590

Leu Trp Ile Lys Pro Tyr Lys Ile Leu Val Ile Ser Ala Asp Ser Gly
        595                 600                 605

Met Ile Glu Pro Val Val Asn Ala Val Ser Ile His Gln Val Lys Lys
    610                 615                 620

Gln Ser Gln Leu Ser Leu Leu Asp Tyr Phe Leu Gln Glu His Gly Ser
625                 630                 635                 640

Tyr Thr Thr Glu Ala Phe Leu Ser Ala Gln Arg Asn Phe Val Gln Ser
                645                 650                 655

Cys Ala Gly Tyr Cys Leu Val Cys Tyr Leu Leu Gln Val Lys Asp Arg
            660                 665                 670

His Asn Gly Asn Ile Leu Leu Asp Ala Glu Gly His Ile Ile His Ile
        675                 680                 685

Asp Phe Gly Phe Ile Leu Ser Ser Ser Pro Arg Asn Leu Gly Phe Glu
    690                 695                 700

Thr Ser Ala Phe Lys Leu Thr Thr Glu Phe Val Asp Val Met Gly Gly
705                 710                 715                 720

Leu Asn Gly Asp Met Phe Asn Tyr Tyr Lys Met Leu Met Leu Gln Gly
                725                 730                 735

Leu Ile Ala Ala Arg Lys His Met Asp Lys Val Val Gln Ile Val Glu
            740                 745                 750

Ile Met Gln Gln Gly Ser Gln Leu Pro Cys Phe His Gly Ser Ser Thr
        755                 760                 765

Ile Arg Asn Leu Lys Glu Arg Phe His Met Ser Met Thr Glu Glu Gln
    770                 775                 780

Leu Gln Leu Leu Val Glu Gln Met Val Asp Gly Ser Met Arg Ser Ile
785                 790                 795                 800

Thr Thr Lys Leu Tyr Asp Gly Phe Gln Tyr Leu Thr Asn Gly Ile Met
                805                 810                 815

<210> 4
<211> 3205
<212> DNA
<213> Rattus norvegicus

<220>
<223> Phosphatidylinisitol 4-kinase

<400> 4
   ttactatggc atcggtagtg tcctgacagt tcggaaagca ggaaagcaag cgccgagaag 60
   aaaggctcgg gcccagcgga ctcttacatc gcaacccatc aaggaattgt tattttgggt 120
   cctgcaagtg gggtccggtc caacttgagg tagcgtgaag gccccttcag gcggcgccgc 180
   gggcagcccc gcagccgggg cctggtgcag ccgccgcggc cgctgtcagg gaagcgcagg 240
   cggccaatgg aacccgggag cggccgctgc tgctgaggcg gcggtgtcgg cagtccgacc 300
   ccgaccgcct gcaccccctc cgcgagggtc ccccggagct tggaagctcg aagtctggct 360
   gtagccatgg gagacatggt agtggagcct gccaccctga agccaacttc tgagcctact 420
   cctagcccat cagggaataa cgggggctcc ctactaagcg tcatcacgga gggggtcggg 480
   gaactgtcag tgattgaccc tgaggtggcc cagaaggcct gccaggaggt actggagaaa 540
   gtcaagcttt tgcatggagg tgtagccatc tctagcaaag gcagcccact ggagttggtt 600
   aatggggatg gtgtggacaa tgagatccgt tgcctagatg atccacctac cgagatcagg 660
   gaggaagaag atgagatgga gcctggtgtg gtctctggca cagccaaagg aacaagaaga 720
   agacgacaga acaactcagc caaacagtct tggctcctga ggctgtttga atcaaaacta 780
   tttgacatct ctatggccat ttcatacttg tataactcca aggagccggg agtgcaagcc 840
   tacattggca accggctctt ctgctttcgc aatgaggatg tggacttcta tttgccccag 900
   ttgcttaaca tgtatatcca tatggatgag gatgtgggtg atgccattaa accctacata 960
   gtccaccgtt gtcgccagag catcaacttt tccctccagt gtgccctgtt gcttggggcc 1020
   tactcttcag acatgcacat ttccactcaa cgacactccc gagggaccaa gttacggaag 1080
   ctaatcctct cagatgagct gaagccagct caccgaaaga gggagctgcc aactttaagc 1140
   ccagcccctg acacaggact gtctccctct aaaaggactc accagcgctc taagtcagat 1200
   gccacggcca gcataagtct cagcagcaac ctgaaacgaa cagccagcaa ccctaaagtg 1260
   gagaatgagg atgaggagct ctcctccagc accgagagta ttgataattc attcagttcc 1320
   cctgtccggc tggcccctga gcgagaattc atcaagtcct tgatggcaat cggcaaacgg 1380
   ctggccacgc tccccactaa agagcaaaaa acacaaaggc tgatctcaga actttccctg 1440
   ctcaaccata agctccctgc ccgagtctgg ctgcccacag ctggctttga ccaccacgtg 1500
   gtccgtgtgc cccacacaca agctgttgtc ctcaattcca aggataaggc tccctacctg 1560
   atctatgtgg aagttcttga atgtgaaaac ttcgacacaa ctaatgttcc tgcccggatt 1620
   cctgaaaacc gaattcggag tacgaggtct gtggagaacc tgccggaatg tggtatcact 1680
   cacgagcagc gggctggcag cttcagcact gtgcccaatt atgacaatga cgatgaagcc 1740
   tggtcggtgg atgacatagg cgagctgcag gtggagctcc cagaagtgca caccaacagc 1800
   tgtgacaaca tctctcagtt ctcggtggac agcatcacca gccaggagag caaggagcct 1860
   gtgttcattg cggcagggga catcagacgg cgcctttcag aacagctggc tcacactccc 1920
   acagccttca aacgagaccc tgaagaccct tctgcagtcg ccctcaaaga gccctggcag 1980
   gagaaagtgc ggaggatcag agaaggttcc ccgtatggcc atcttcccaa ttggcgactc 2040
   ctttcagtca ttgtcaagtg tggagatgac cttcgccagg agctgctggc tttccaggtg 2100
   ttgaaacaac tgcagtccat ttgggaacag gagcgagtgc ctctttggat caagccatat 2160
   aagattcttg tgatttcagc tgacagtggc atgattgaac cagtagtcaa cgctgtgtcc 2220
   atccaccagg tgaagaaaca gtcacagctc tccttgctcg attacttcct gcaggagcat 2280
   ggcagttaca ccactgaggc attcctcagt gcccagcgca attttgtgca aagttgtgct 2340
   ggctactgct tggtctgcta cctattacaa gtcaaggaca ggcacaatgg gaacatcctt 2400
   ctggacgcag aaggtcacat catccacatc gactttggct tcatcctttc cagctcaccc 2460
   cgaaacctgg gcttcgagac gtcagccttt aagctgacca cagaatttgt ggatgtaatg 2520
   ggcggcctga acggtgatat gttcaactac tataagatgc tcatgctgca agggctgatc 2580
   gctgctcgca aacacatgga caaggtggta cagattgtgg agatcatgca gcaaggttct 2640
   cagcttcctt gcttccatgg ctccagcacc attcgcaacc tcaaagagag gttccacatg 2700
   agcatgactg aggagcagct gcagctgctg gtggagcaga tggtggacgg cagcatgagg 2760
   tccatcacca ccaaactcta cgatggcttc cagtacctca ccaatggcat catgtgacac 2820
   cactttcagg cccagaagtg gtgggattga aggcaccctc cttaggagga cccttgccta 2880
   agaaaaccta aatcacaaaa tcccacctac ctaaccacct acccaaggga aatggaaggc 2940
   aaaaaacaca aaggatcatg tggtaaccat gagagctggc caaagggtag gagagccaac 3000
   catggggtcc agacttgctg gggcttcccc acctcttgct gtgtcagtat tgccacctga 3060
   cagatcccag gactcactgc cctccagaga acagagatga tgaacatgag gaggactgga 3120
   accttcttcc cctcggggat ctcatctttt actatccctg ggtcccagca agatgggagg 3180
   agtgggttct tggtacttag gactt 3205

<210> 5
<211> 1379
<212> PRT
<213> Rattus norvegicus

<220>
<223> ROK-alpha

<400> 5
Met Pro Gly Ala Pro Glu Ala Ala Ala Gly Asp Gly Ala Gly Ala Gly
1               5                   10                  15

Arg Gln Arg Lys Leu Glu Ala Leu Ile Arg Asp Pro Arg Ser Pro Ile
            20                  25                  30

Asn Val Glu Ser Leu Leu Asp Gly Leu Asn Ser Leu Val Leu Asp Leu
        35                  40                  45

Asp Phe Pro Ala Leu Arg Lys Asn Lys Asn Ile Asp Asn Phe Leu Asn
    50                  55                  60

Arg Tyr Glu Lys Ile Val Lys Lys Ile Arg Gly Leu Gln Met Lys Ala
65                  70                  75                  80

Glu Asp Tyr Asp Val Val Lys Val Ile Gly Arg Gly Ala Phe Gly Glu
                85                  90                  95

Val Gln Leu Val Arg His Lys Ala Ser Gln Lys Val Tyr Ala Met Lys
            100                 105                 110

Leu Leu Ser Lys Phe Glu Met Ile Lys Arg Ser Asp Ser Ala Phe Phe
        115                 120                 125

Trp Glu Glu Arg Asp Ile Met Ala Phe Ala Asn Ser Pro Trp Val Val
    130                 135                 140

Gln Leu Phe Cys Ala Phe Gln Asp Asp Arg Tyr Leu Tyr Met Val Met
145                 150                 155                 160

Glu Tyr Met Pro Gly Gly Asp Leu Val Asn Leu Met Ser Asn Tyr Asp
                165                 170                 175

Val Pro Glu Lys Trp Ala Lys Phe Tyr Thr Ala Glu Val Val Leu Ala
            180                 185                 190

Leu Asp Ala Ile His Ser Met Gly Leu Ile His Arg Asp Val Lys Pro
        195                 200                 205

Asp Asn Met Leu Leu Asp Lys His Gly His Leu Lys Leu Ala Asp Phe
    210                 215                 220

Gly Thr Cys Met Lys Met Asp Glu Thr Gly Met Val His Cys Asp Thr
225                 230                 235                 240

Ala Val Gly Thr Pro Asp Tyr Ile Ser Pro Glu Val Leu Lys Ser Gln
                245                 250                 255

Gly Gly Asp Gly Tyr Tyr Gly Arg Glu Cys Asp Trp Trp Ser Val Gly
            260                 265                 270

Val Phe Leu Phe Glu Met Leu Val Gly Asp Thr Pro Phe Tyr Ala Asp
        275                 280                 285

Ser Leu Val Gly Thr Tyr Ser Lys Ile Met Asp His Lys Asn Ser Leu
    290                 295                 300

Cys Phe Pro Glu Asp Thr Glu Ile Ser Lys His Ala Lys Asn Leu Ile
305                 310                 315                 320

Cys Ala Phe Leu Thr Asp Arg Glu Val Arg Leu Gly Arg Asn Gly Val
                325                 330                 335

Glu Glu Ile Lys Ser Ala Ser Phe Phe Lys Asn Asp Gln Trp Asn Trp
            340                 345                 350

Asp Asn Ile Arg Glu Thr Ala Ala Pro Val Val Pro Glu Leu Ser Ser
        355                 360                 365

Asp Ile Asp Ser Ser Asn Phe Asp Asp Ile Glu Asp Asp Lys Gly Asp
    370                 375                 380

Val Glu Thr Phe Pro Ile Pro Lys Ala Phe Val Gly Asn Gln Leu Pro
385                 390                 395                 400

Phe Ile Gly Phe Thr Tyr Phe Arg Glu Asn Leu Leu Leu Ser Asp Ser
                405                 410                 415

Pro Pro Cys Arg Glu Asn Asp Ala Ile Gln Thr Arg Lys Ser Glu Glu
            420                 425                 430

Ser Gln Glu Ile Gln Lys Lys Leu Tyr Ala Leu Glu Glu His Leu Ser
        435                 440                 445

Ser Glu Val Gln Ala Lys Glu Glu Leu Glu Gln Lys Cys Lys Ser Ile
    450                 455                 460

Asn Thr Arg Leu Glu Lys Thr Ala Lys Glu Leu Glu Glu Glu Ile Thr
465                 470                 475                 480

Phe Arg Lys Asn Val Glu Ser Thr Leu Arg Gln Leu Glu Arg Glu Lys
                485                 490                 495

Ala Leu Leu Gln His Lys Asn Ala Glu Tyr Gln Arg Lys Ala Asp His
            500                 505                 510

Glu Ala Asp Lys Lys Arg Asn Leu Glu Asn Asp Val Asn Ser Leu Lys
        515                 520                 525

Asp Gln Leu Glu Asp Leu Lys Lys Arg Asn Gln Ser Ser Gln Ile Ser
    530                 535                 540

Thr Glu Lys Val Asn Gln Leu Gln Lys Gln Leu Asp Glu Ala Asn Ala
545                 550                 555                 560

Leu Leu Arg Thr Glu Ser Asp Thr Ala Ala Arg Leu Arg Lys Thr Gln
                565                 570                 575

Ala Glu Ser Ser Lys Gln Ile Gln Gln Leu Glu Ser Asn Asn Arg Asp
            580                 585                 590

Leu Gln Asp Lys Asn Cys Leu Leu Glu Thr Ala Lys Leu Lys Leu Glu
        595                 600                 605

Lys Glu Phe Ile Asn Leu Gln Ser Ala Leu Glu Ser Glu Arg Arg Asp
    610                 615                 620

Arg Thr His Gly Ser Glu Ile Ile Asn Asp Leu Gln Gly Arg Ile Ser
625                 630                 635                 640

Gly Leu Glu Glu Asp Leu Lys Thr Gly Lys Thr Leu Leu Ala Lys Val
                645                 650                 655

Glu Leu Glu Lys Arg Gln Leu Gln Glu Lys Leu Thr Asp Leu Glu Lys
            660                 665                 670

Glu Lys Ser Asn Met Glu Ile Asp Met Thr Tyr Gln Leu Lys Val Ile
        675                 680                 685

Gln Gln Ser Leu Glu Gln Glu Glu Ala Glu His Lys Thr Thr Lys Ala
    690                 695                 700

Arg Leu Ala Asp Lys Asn Lys Ile Tyr Glu Ser Ile Glu Glu Ala Lys
705                 710                 715                 720

Ser Glu Ala Met Lys Glu Met Glu Lys Lys Leu Leu Glu Glu Arg Ser
                725                 730                 735

Leu Lys Gln Lys Val Glu Asn Leu Leu Leu Glu Ala Glu Lys Arg Cys
            740                 745                 750

Ser Ile Leu Asp Cys Asp Leu Lys Gln Ser Gln Gln Lys Leu Asn Glu
        755                 760                 765

Leu Leu Lys Gln Lys Asp Val Leu Asn Glu Asp Val Arg Asn Leu Thr
    770                 775                 780

Leu Lys Ile Glu Gln Glu Thr Gln Lys Arg Cys Leu Met Gln Asn Asp
785                 790                 795                 800

Leu Lys Met Gln Thr Gln Gln Val Asn Thr Leu Lys Met Ser Glu Lys
                805                 810                 815

Gln Ile Lys Gln Glu Asn Asn His Leu Met Glu Met Lys Met Asn Leu
            820                 825                 830

Glu Lys Gln Asn Ala Glu Leu Arg Lys Glu Arg Gln Asp Ala Asp Gly
        835                 840                 845

Gln Met Lys Glu Leu Gln Asp Gln Leu Glu Ala Glu Gln Tyr Phe Ser
    850                 855                 860

Thr Leu Tyr Lys Thr Gln Val Arg Glu Leu Lys Glu Glu Asn Glu Glu
865                 870                 875                 880

Lys Thr Lys Leu Cys Lys Glu Leu Gln Gln Lys Lys Gln Asp Leu Gln
                885                 890                 895

Asp Glu Arg Asp Ser Leu Ala Ala Gln Leu Glu Ile Thr Leu Thr Lys
            900                 905                 910

Ala Asp Ser Glu Gln Leu Ala Arg Ser Ile Ala Glu Glu Gln Tyr Ser
        915                 920                 925

Asp Leu Glu Lys Glu Lys Ile Met Lys Glu Leu Glu Ile Lys Glu Met
    930                 935                 940

Met Ala Arg His Lys Gln Glu Leu Thr Glu Lys Asp Ala Thr Ile Ala
945                 950                 955                 960

Ser Leu Glu Glu Thr Asn Arg Thr Leu Thr Ser Asp Val Ala Asn Leu

                965                 970                 975

Ala Asn Glu Lys Glu Glu Leu Asn Asn Lys Leu Lys Asp Thr Gln Glu
            980                 985                 990

Gln Leu Ser Lys Leu Lys Asp Glu Glu Ile Ser Ala Ala Ala Ile Lys
        995                 1000                1005

Ala Gln Phe Glu Lys Gln Leu Leu Thr Glu Arg Thr Leu Lys Thr Gln
    1010                1015                1020

Ala Val Asn Lys Leu Ala Glu Ile Met Asn Arg Lys Glu Pro Val Lys
1025                1030                1035                1040

Arg Gly Ser Asp Thr Asp Val Arg Arg Lys Glu Lys Glu Asn Arg Lys
                1045                1050                1055

Leu His Met Glu Leu Lys Ser Glu Arg Glu Lys Leu Thr Gln Gln Met
            1060                1065                1070

Ile Lys Tyr Gln Lys Glu Leu Asn Glu Met Gln Ala Gln Ile Ala Glu
        1075                1080                1085

Glu Ser Gln Ile Arg Ile Glu Leu Gln Met Thr Leu Asp Ser Lys Asp
    1090                1095                1100

Ser Asp Ile Glu Gln Leu Arg Ser Gln Leu Gln Ala Leu His Ile Gly
1105                1110                1115                1120

Met Asp Ser Ser Ser Ile Gly Ser Gly Pro Gly Asp Ala Glu Pro Asp
                1125                1130                1135

Asp Gly Phe Pro Glu Ser Arg Leu Glu Gly Trp Leu Ser Leu Pro Val
            1140                1145                1150

Arg Asn Asn Thr Lys Lys Phe Gly Trp Val Lys Lys Tyr Val Ile Val
        1155                1160                1165

Ser Ser Lys Lys Ile Leu Phe Tyr Asp Ser Glu Gln Asp Lys Glu Gln
    1170                1175                1180

Ser Asn Pro Tyr Met Val Leu Asp Ile Asp Lys Leu Phe His Val Arg
1185                1190                1195                1200

Pro Val Thr Gln Thr Asp Val Tyr Arg Ala Asp Ala Lys Glu Ile Pro
                1205                1210                1215

Arg Ile Phe Gln Ile Leu Tyr Ala Asn Glu Gly Glu Ser Lys Lys Glu
            1220                1225                1230

Pro Glu Phe Pro Val Glu Pro Val Gly Glu Lys Ser Asn Tyr Ile Cys
        1235                1240                1245

His Lys Gly His Glu Phe Ile Pro Thr Leu Tyr His Phe Pro Thr Asn
    1250                1255                1260

Cys Glu Ala Cys Met Lys Pro Leu Trp His Met Phe Lys Pro Pro Pro
1265                1270                1275                1280

Ala Leu Glu Cys Ser Arg Cys His Ile Lys Cys His Lys Asp His Met
                1285                1290                1295

Asp Lys Lys Glu Glu Ile Ile Ala Pro Cys Lys Val Tyr Tyr Asp Ile
            1300                1305                1310

Ser Ser Ala Lys Asn Leu Leu Leu Leu Ala Asn Ser Thr Glu Glu Gln
        1315                1320                1325

Gln Lys Trp Val Ser Arg Leu Val Lys Lys Ile Pro Lys Lys Pro Pro
    1330                1335                1340

Ala Pro Asp Pro Phe Ala Arg Ser Ser Pro Arg Thr Ser Met Lys Ile
1345                1350                1355                1360

Gln Gln Asn Gln Ser Ile Arg Arg Pro Ser Arg Gln Leu Ala Pro Asn
                1365                1370                1375

Lys Pro Ser

<210> 6
<211> 4470
<212> DNA
<213> Rattus norvegicus

<220>
<223> ROK-alpha

<400> 6
gggccgggcg gaggttcgcg ggcggcgcta ggccgggcga agcggcatga gccggccccc 60
gccgacgggg aaaatgcccg gcgcccccga ggccgcggcc ggggacgggg cgggcgcggg 120
tcgtcagagg aagctggagg cgctcatccg agaccctcgc tcgcccatca acgtggaaag 180
cctgctggat ggcttaaatt ccttggtcct tgatttggat tttcctgctt tgaggaaaaa 240
taaaaatata gataatttcc taaatagata tgagaaaatt gtgaaaaaaa tcagaggttt 300
acagatgaaa gcagaagact atgatgttgt aaaagttatt ggaagaggtg cttttggtga 360
agttcagttg gttcgtcata aggcatcaca gaaggtttat gcaatgaagc ttcttagtaa 420
atttgaaatg ataaaaagat cagattctgc ttttttctgg gaagaaagag atattatggc 480
ttttgctaac agtccgtggg tggttcagct cttttgtgcc tttcaagatg acaggtatct 540
gtacatggtg atggagtaca tgccaggtgg agaccttgtt aacctgatga gcaattatga 600
tgtgcctgag aagtgggcca agttttatac tgctgaagtc gtgcttgcac tggatgcaat 660
acactccatg ggcttaatac acagagatgt gaagcccgat aacatgctat tggataaaca 720
cggacatcta aaattagcag attttggcac atgtatgaaa atggatgaaa caggcatggt 780
gcattgtgat acagcagttg gaactccaga ctacatatca cctgaggttc tgaaatcaca 840
aggaggtgat ggttactatg gacgagaatg tgattggtgg tctgtaggtg ttttcctttt 900
tgagatgtta gtaggggaca ctccatttta tgcagattca cttgtaggaa cctacagcaa 960
aattatggat cataaaaatt cactgtgttt tcctgaagat acagaaattt ctaaacatgc 1020
gaagaatctc atatgtgcct tcttaacaga cagggaggta cgacttggaa gaaatggggt 1080
agaagaaatc aaatcagcat ccttctttaa gaatgatcag tggaattggg ataacataag 1140
agagacggca gctccagtgg tgcctgagct cagcagtgac atagacagca gcaactttga 1200
tgacattgag gatgacaaag gcgatgtaga gaccttcccg attcctaaag cttttgtggg 1260
aaatcagctg ccttttatag gatttaccta ctttagagaa aatttgttac taagtgattc 1320
tccaccttgt agagaaaatg atgcaataca aacaaggaaa agtgaagaaa gtcaggagat 1380
tcagaaaaaa ttatatgcac tagaagaaca ccttagcagt gaggtacaag ccaaggagga 1440
actggaacag aagtgcaagt ctattaatac tcgtctagag aaaacagcaa aggaactaga 1500
agaagagatt acctttagga aaaatgtgga atcaacatta agacagctag aaagagaaaa 1560
ggcccttctt cagcacaaaa atgcagaata tcaacggaaa gctgatcatg aagctgacaa 1620
gaaacggaat ttggaaaatg atgttaacag cttaaaagat caacttgaag atttgaagaa 1680
aagaaaccag agctctcaga tatccactga gaaggtcaat cagctccaga aacaactgga 1740
tgaagctaat gctttgctaa gaacagagtc tgatactgca gcacggttaa gaaaaaccca 1800
ggcagaaagt tccaaacaga ttcagcagct ggaatctaac aatagagatc tacaagataa 1860
aaactgcctg cttgaaactg ccaagttaaa acttgagaag gaatttatca atcttcagtc 1920

agctctagaa tctgaaagaa gggaccggac ccatggatca gagataatta atgatttaca 1980
aggtagaata tctgggttgg aagaagattt gaagactggc aaaaccttac tagcaaaagt 2040
agagctggag aagagacagc tgcaggagaa gctcactgac ttagagaagg aaaagagcaa 2100
catggaaata gatatgacat atcaactgaa agttatacag cagagtttag aacaagaaga 2160
agctgaacac aagaccacaa aagcacgact agcggataaa aataagatct atgaatccat 2220
tgaagaagct aaatcagaag ccatgaaaga aatggagaag aagctgttgg aagagaggtc 2280
tttaaagcag aaagtggaga acctactcct ggaagccgag aagagatgtt ctatattgga 2340
ttgtgacctc aaacagtctc agcagaaact gaatgagctg ctgaagcaga aagatgtgct 2400
gaatgaggat gttagaaatt tgacattaaa aatagagcag gaaactcaga agcgttgtct 2460
tatgcaaaat gacctgaaga tgcagaccca gcaagttaac accctaaaaa tgtcagaaaa 2520
gcaaataaaa caagaaaata atcatcttat ggaaatgaaa atgaacttgg aaaagcaaaa 2580
tgctgaactt cgaaaagagc ggcaggatgc agatgggcag atgaaggaac tgcaagacca 2640
acttgaagca gagcagtatt tctcaaccct ctataaaaca caagttagag aacttaaaga 2700
agaaaatgaa gagaaaacta aactttgtaa agagttgcag cagaaaaagc aggatttaca 2760
ggatgaaaga gactccttgg ctgcccagct ggagatcacc ctcaccaaag cagactctga 2820
gcagctggct cgctccattg ctgaggagca atactctgac ttggaaaaag agaagatcat 2880
gaaagagctg gaaatcaaag agatgatggc tagacacaaa caagagctta ctgaaaagga 2940
cgctacaatt gcatcccttg aagaaacaaa tagaacacta actagtgatg ttgcaaatct 3000
tgcaaacgag aaagaagaac taaacaacaa gttgaaagat acccaagaac aactgtcaaa 3060
gttgaaagat gaagagatca gtgcagcggc tattaaagca cagtttgaga agcagctgct 3120
gactgagcga acactcaaga ctcaagctgt gaataagttg gcagagatca tgaatcgaaa 3180
agaacctgtc aagcgtggta gtgacacaga tgtgcgaaga aaagaaaagg aaaacagaaa 3240
attacatatg gagcttaagt ctgaacgtga aaaattgaca caacagatga tcaaatatca 3300
gaaagaactg aatgaaatgc aggctcaaat agctgaagag agccaaattc gaatagaact 3360
gcagatgacc ctggacagta aagacagtga cattgaacag cttcggtcgc agctccaggc 3420
cttgcatatt ggtatggaca gttccagtat aggcagtgga ccaggggatg ctgagcctga 3480
tgacggattt ccagaatcac gattagaagg atggctgtcg ttgcctgtgc ggaacaacac 3540
taaaaagttt ggatgggtta agaagtatgt gattgtaagc agcaagaaga ttcttttcta 3600
tgatagtgaa caagataaag agcagtctaa cccttacatg gtgttagaca tagacaagct 3660
atttcatgtc cgacctgtta ctcagacaga tgtatacaga gcagatgcta aagaaattcc 3720
aaggatattt cagatcctgt atgccaatga aggagaaagt aaaaaggaac cagaatttcc 3780
agtggaacca gtgggagaaa aatctaatta tatttgccac aaaggacatg agtttattcc 3840
tactctttat cacttcccaa ccaactgtga ggcctgtatg aagccactgt ggcacatgtt 3900
taaacctcct cctgctctag agtgcagtag atgccacatt aaatgtcata aagatcacat 3960
ggacaaaaag gaagagatca tagccccctg caaagtttat tatgatatat catcggcaaa 4020
gaatctgttg ttattagcaa attcaacgga agaacagcag aaatgggtta gtcggttggt 4080
gaaaaagata cctaaaaagc ctccagctcc agaccctttt gcccgatcat cccctagaac 4140
ctcaatgaaa atacaacaaa accagtccat tcggcggccc agtcgacagc ttgctccaaa 4200
caaaccaagc taacttgcct tctatgaaag cagttattga aggtgatcat attcttccat 4260
ccacaagact gaaatatggt ttctcaaaac ttaactaaaa agctatattt ttactgagac 4320
acatacatac accacatgtc gctcgttgcg tgcatgcttg cagtcccttc ccccctgaaa 4380
taattacaaa tcatggagag gtttgtctgg gctactttga acagttgaac aaacaatggt 4440
tggtgaggta aaaaaagtgt cctacaattc 4470

<210> 7
<211> 215
<212> PRT
<213> Rattus norvegicus

<220>
<223> Casein kinase II beta subunit

<400> 7
Met Ser Ser Ser Glu Glu Val Ser Trp Ile Ser Trp Phe Cys Gly Leu
1               5                   10                  15

Arg Gly Asn Glu Phe Phe Cys Glu Val Asp Glu Asp Tyr Ile Gln Asp
            20                  25                  30

Lys Phe Asn Leu Thr Gly Leu Asn Glu Gln Val Pro His Tyr Arg Gln

        35                  40                  45

Ala Leu Asp Met Ile Leu Asp Leu Glu Pro Asp Glu Glu Leu Glu Asp
    50                  55                  60

Asn Pro Asn Gln Ser Asp Leu Ile Glu Gln Ala Ala Glu Met Leu Tyr
65                  70                  75                  80

Gly Leu Ile His Ala Arg Tyr Ile Leu Thr Asn Arg Gly Ile Ala Gln
                85                  90                  95

Met Leu Glu Lys Tyr Gln Gln Gly Asp Phe Gly Tyr Cys Pro Arg Val
            100                 105                 110

Tyr Cys Glu Asn Gln Pro Met Leu Pro Ile Gly Leu Ser Asp Ile Pro
        115                 120                 125

Gly Glu Ala Met Val Lys Leu Tyr Cys Pro Lys Cys Met Asp Val Tyr
    130                 135                 140

Thr Pro Lys Ser Ser Arg His His His Thr Asp Gly Ala Tyr Phe Gly
145                 150                 155                 160

Thr Gly Phe Pro His Met Leu Phe Met Val His Pro Glu Tyr Arg Pro
                165                 170                 175

Lys Arg Pro Ala Asn Gln Phe Val Pro Arg Leu Tyr Gly Phe Lys Ile
            180                 185                 190

His Pro Met Ala Tyr Gln Leu Gln Leu Gln Ala Ala Ser Asn Phe Lys
        195                 200                 205

Ser Pro Val Lys Thr Ile Arg
    210                 215

<210> 8
<211> 1964
<212> DNA
<213> Rattus norvegicus

<220>
<223> Casein kinase II beta subunit

<400> 8
ggctgccgcg gcccggtcgg ctttctgcgc tgtagcggtc tctgccgttc cttggaagca 60
cagctcccct tccccgcccc agtcccagtc cccgtccggc cgcggacata aagatgagta 120
gctctgagga ggtgtcctgg atttcctggt tctgtgggct ccgtggtaat gaattcttct 180
gtgaggtgga tgaagactac atccaggaca aatttaatct tactggactc aatgagcagg 240
tgcctcacta tcgacaagcc ctagacatga tcttggacct ggaacctgat gaagagctgg 300
aagacaaccc caaccagagt gacttgattg agcaggcggc cgagatgctc tatgggttga 360
tccacgcccg ctacatcctc accaaccggg gcattgcaca aatgttggaa aagtaccagc 420
aaggagactt tggctactgt cctcgagtat actgtgagaa ccagccgatg cttcccatcg 480
gcctttcgga catcccagga gaggccatgg tgaagctcta ctgccccaag tgcatggacg 540
tgtacacacc caagtcctct aggcaccacc acacggatgg cgcatacttc ggcactggtt 600
tccctcacat gctcttcatg gtgcatcccg agtaccggcc caagcggccg gccaaccagt 660
ttgtgcccag gctctacggt ttcaagatcc atccaatggc ctaccagctg cagctccaag 720
ccgccagcaa cttcaagagc ccagtcaaga cgattcgctg agtgccctcc cacctcctct 780
gcctgtgaca ccaccgtccc tccgctgcca ccctttcagg aagtctatgg tttttagttt 840
aaattaaagg aattgttact gtggtgggga tatgaaataa aggaagagaa ggctaaaaaa 900
aaaaaaaaaa accgaattcg gcatctcacc taggtgtata cagctgcaca ttaccaatgg 960

ctgcagaaca cccaatcatg acctctagtg ctttatagca aaagtgtaaa gtggtacctg 1020
ggtgaccagc attgccatgg ttacatccat ttgcgtaatc aagcagctgt gcaactgctc 1080
ttctaacaga tgaacagctg catgcatctc cagcaggcat ggattgtctc ttgtctatga 1140
tcctcagtgc gttcacttag agtatttcta gagtttgagt ccttgccgtg atagagccat 1200
gtagggaatg cactgattgc atgttacccc aagcgtcatg aaaccttccg acacggtgac 1260
ctatttaatg gtcttgtttg ttgacatgac aaattaacat tcttagagtt acatctcgaa 1320
aaaagcattt gtgatagata agccctttga gccttgtggc taaatttttg tggctttgtt 1380
taactttcaa aggttatata tgcactaacc ttttttgatg gctaagtagg ctttaaatta 1440
cagtaaattt tttcaaataa aactggctgt aaaatatatt ttgaattaga gttgttcact 1500
ttttcatagc tacttatgtt tttttcccaa taatttattt cacatctcta ccagtgaacg 1560
cagcccatca ctagaaaatg accttgtcct gatttcagtt tcaactatta gttttaaagc 1620
tcactgttga ataagaggaa gtggtggtgc attttaaatt gactttcatg tgcttttaaa 1680
atatgacaga tctccttgat aatgtacttt tatttgatct caagttgtat aaaaccaata 1740
aatttgtgtt actttgattg cagtagtatc ttatgcatag tgattccatg ttatatgcag 1800
actagttagg caactgtttt cttagttaca agcttcactt ttgtgcagtt aaaaaaacaa 1860
aagtaggcta cagtctgtgc catgttgatg tacagtttct gaaattgttt tacaagactt 1920
tgataataaa acccttaaac tcacaaaaaa aaaaaaaaaa aacc 1964

<210> 9
<211> 543
<212> PRT
<213> Rattus norvegicus

<220>
<223> ERK3

<400> 9
Met Ala Glu Lys Phe Glu Ser Leu Met Asn Ile His Gly Phe Asp Leu
1               5                   10                  15

Gly Ser Arg Tyr Met Asp Leu Lys Pro Leu Gly Cys Gly Gly Asn Gly
            20                  25                  30

Leu Val Phe Ser Ala Val Asp Asn Asp Cys Asp Lys Arg Val Ala Ile
        35                  40                  45

Lys Lys Ile Val Leu Thr Asp Pro Gln Ser Val Lys His Ala Leu Arg
    50                  55                  60

Glu Ile Lys Ile Ile Arg Arg Leu Asp His Asp Asn Ile Val Lys Val
65                  70                  75                  80

Phe Glu Ile Leu Gly Pro Ser Gly Ser Gln Leu Thr Asp Asp Val Gly
                85                  90                  95

Ser Leu Thr Glu Leu Asn Ser Val Tyr Ile Val Gln Glu Tyr Met Glu
            100                 105                 110

Thr Asp Leu Ala Asn Val Leu Glu Gln Gly Pro Leu Leu Glu Glu His
        115                 120                 125

Ala Arg Leu Phe Met Tyr Gln Leu Leu Arg Gly Leu Lys Tyr Ile His
    130                 135                 140

Ser Ala Asn Val Leu His Arg Asp Leu Lys Pro Ala Asn Leu Phe Ile
145                 150                 155                 160

Asn Thr Glu Asp Leu Val Leu Lys Ile Gly Asp Phe Gly Leu Ala Arg
                165                 170                 175

Ile Met Asp Pro His Tyr Ser His Lys Gly His Leu Ser Glu Gly Leu
            180                 185                 190

Val Thr Lys Trp Tyr Arg Ser Pro Arg Leu Leu Leu Ser Pro Asn Asn
        195                 200                 205

Tyr Thr Lys Ala Ile Asp Met Trp Ala Ala Gly Cys Ile Phe Ala Glu
    210                 215                 220

Met Leu Thr Gly Lys Thr Leu Phe Ala Gly Ala His Glu Leu Glu Gln
225                 230                 235                 240

Met Gln Leu Ile Leu Glu Ser Ile Pro Val Val His Glu Glu Asp Arg
                245                 250                 255

Gln Glu Leu Leu Ser Val Ile Pro Val Tyr Ile Arg Asn Asp Met Thr
            260                 265                 270

Glu Pro His Lys Pro Leu Thr Gln Leu Leu Pro Gly Ile Ser Arg Glu
        275                 280                 285

Ala Leu Asp Phe Leu Glu Gln Ile Leu Thr Phe Ser Pro Met Asp Arg
    290                 295                 300

Leu Thr Ala Glu Glu Ala Leu Ser His Pro Tyr Met Ser Ile Tyr Ser
305                 310                 315                 320

Phe Pro Thr Asp Glu Pro Ile Ser Ser His Pro Phe His Ile Glu Asp
                325                 330                 335

Glu Val Asp Asp Ile Leu Leu Met Asp Glu Thr His Ser His Ile Tyr
            340                 345                 350

Asn Trp Glu Arg Tyr His Asp Cys Gln Phe Ser Glu His Asp Trp Pro
        355                 360                 365

Ile His Asn Asn Phe Asp Ile Asp Glu Val Gln Leu Asp Pro Arg Ala
    370                 375                 380

Leu Ser Asp Val Thr Asp Glu Glu Glu Val Gln Val Asp Pro Arg Lys
385                 390                 395                 400

Tyr Leu Asp Gly Asp Arg Glu Lys Tyr Leu Glu Asp Pro Ala Phe Asp
                405                 410                 415

Thr Ser Tyr Ser Ala Glu Pro Cys Trp Gln Tyr Pro Asp His His Glu
            420                 425                 430

Asn Lys Tyr Cys Asp Leu Glu Cys Ser His Thr Cys Asn Tyr Lys Thr
        435                 440                 445

Arg Ser Pro Ser Tyr Leu Asp Asn Leu Val Trp Arg Glu Ser Glu Val
    450                 455                 460

Asn His Tyr Tyr Glu Pro Lys Leu Ile Ile Asp Leu Ser Asn Trp Lys
465                 470                 475                 480

Glu Gln Ser Lys Asp Lys Ser Asp Lys Arg Gly Lys Ser Lys Cys Glu
                485                 490                 495

Arg Asn Gly Leu Val Lys Arg Arg Leu Arg Leu Arg Lys Arg Pro Ser

                500                 505                 510

Ser Trp Leu Arg Gly Arg Gly Ala Lys Ala Leu Thr Leu Met Pro Ser
        515                 520                 525

Ser Gln Ala Pro Phe Ser Ser Val Pro Ser Val Ser Leu Leu Thr
    530                 535                 540


<210> 10
<211> 3662
<212> DNA
<213> Rattus norvegicus

<220>
<223> ERK3

<400> 10
agacctgccg ggcgcatatt tattcacagt tttgtcccat gttaagtcgg ttagcatagt 60
gaatctgagt gcatagtatg tcatttcatt ccgttgagtt tctcgagtgt tttctttaaa 120
tgtctgcaga gtcgctaccc ttccttgaac tatgaagcac tgcaatcttc ttaattctca 180
gtatgaagag agatttttga gctttaagtc tgaggggaac tcagcaggcc tggttggcgt 240
ctgcaatgaa catcaagaaa ccatcgtgct gtgggaatgt gatcgttttt ctcccttttt 300
gagagatctt tccttttgat gccagttttc ttccttgttt acacaagttc aacaatttga 360
aaggaaaagg caattgtaag ggttttaaaa tggcagagaa atttgaaagt ctcatgaaca 420
ttcatggctt tgatctgggt tccaggtaca tggacttaaa accattgggc tgtggaggca 480
atggcttggt tttttctgct gtagacaatg actgtgacaa aagagtagcc atcaagaaaa 540
ttgtcctcac cgatccccag agtgtcaaac atgccctccg tgaaatcaaa attattagaa 600
gacttgacca cgataacatt gtgaaagtgt ttgaaattct tggtcccagt ggaagccagc 660
tgacagacga tgtgggctct ctaacagagc tgaatagcgt ctacattgtt caggagtaca 720
tggagacaga cttggcgaac gtgctggagc agggcccttt actggaggag catgccaggc 780
tcttcatgta ccagctgctg cgtgggctca agtacatcca ctctgcaaac gtgctgcaca 840
gggatctcaa gccggccaac cttttcatta acactgaaga cttggtgctg aagattggtg 900
actttggcct ggcccggatc atggatcctc attattccca taagggtcat ctttctgaag 960
gattggttac caaatggtac agatctccac ggcttttact ttctcctaat aactatacta 1020
aagccattga catgtgggct gcaggctgca tctttgctga aatgctgact ggtaaaaccc 1080
tctttgcagg tgcacatgaa cttgaacaga tgcagctgat cttggagtct atccctgttg 1140
tgcacgagga agatcggcag gagcttctca gcgtgattcc agtttacatt agaaacgaca 1200
tgactgagcc acacaaaccg ctgactcagc tgcttccggg gattagtcgg gaagcactgg 1260
atttcctgga acagattctg acgttcagtc ccatggaccg gctgacagcc gaggaagcac 1320
tttcccatcc ttacatgagc atctactctt tcccaacgga cgagcctatt tccagccatc 1380
ctttccacat agaagacgaa gtggacgaca ttttgctaat ggatgaaaca cacagtcaca 1440
tttataactg ggaaaggtac cacgattgtc agttctcgga gcatgactgg cctattcata 1500
acaactttga tatcgatgag gttcagcttg acccgagagc tctgtctgat gtcaccgatg 1560
aagaagaagt tcaagttgat cctcgaaagt acttggatgg agaccgagag aagtatctgg 1620
aggatcccgc cttcgacacc agctactctg ctgagccttg ctggcagtac ccagatcacc 1680
acgagaacaa gtactgtgat ctggagtgta gccacacctg taactacaaa acaaggtcgc 1740
catcatactt agataacctg gtgtggaggg agagcgaggt taaccattac tatgagccca 1800
agcttattat agatctttcc aactggaaag agcaaagtaa ggacaaatcc gacaagagag 1860
gcaagtccaa gtgtgagagg aacgggttgg tcaagcgcag attgcgcttg aggaagcgtc 1920
ccagcagctg gctgagaggg agaggggcca aggctttgac tttgatgcct tcatcgcagg 1980
caccgttcag ctcagtgccc agcgtgagtc tgctgacgta gttgacaagt taaacgactt 2040
gaatagctca gtgtcccagc tagaaatgaa aagcctgata tccaagtcag tcagccgaga 2100
aaagcaagaa aagggaaggg ctaacctggc ccagctggga gccttgtacc agccctcctg 2160
ggagagccag tttgtgagtg gcggggagga gtgcttcctt atcagtcagt tttgttgtga 2220
ggtcaggaag gacgaacacg tggagaagga gaacacttac accagctatt tggacaagtt 2280
ttttagcagg aaggaggatt ctgaaatgct agaaactgag ccagtggaag aagggaagcg 2340
tggggagaga ggccgtgagg cagggcttct gagcagcggt ggggagtttc tcctgagcag 2400
gcagctagag tccataggca ccccgcagtt ccacagtcca gggggatccc cactcaagtc 2460
catccaggcc acgttaacac cttccgctat gaaatcttcc cctcaaatcc ctcacaagac 2520
atacagcaac attctgaaac atctgaacta aacactcagc agacacttct tttgttcttc 2580

atgaaatgtg ttgtgtcttt tttatcacta atgttttaag tcattttttt tttacttgaa 2640
tcagaaggtg tcattaattt gcaaggattt ttcttggttc tcagtttgta aaacacagag 2700
ttttttctac atgtgagtta gttttcattt gaactggcat gtcgtttgca cacacacaaa 2760
gaatagagca aaacaatgca gtgcaggagg agacaagatg cgctaggatg gacagacatt 2820
ctcacagacc agtgacctgc ttacaggaaa caaaaccttg ccttgaaact tacacagtga 2880
gactgtacat aattgcatga aaagatctat ttttttcctg aaacattttt cattcattag 2940
tattttcaag tttttcatac tgtacacatt tcttaagaca catgatacca gcagcaactg 3000
aaaacgaatg ccgaatttgg tacacatgtg ttatctacct caaggtaaca aaagtatgcg 3060
ggcgaaacct aacccaccca tagtcgtccg cggcatatgc acttgtatct agccagcgtt 3120
ggccgcagta accaatgaga ctcgtccgcc atttatcaat gtcctggtgt tcatccttta 3180
cagtgaagtg ttagatacat cacatcttat ttatttttag caaatcagta tattttctgt 3240
atttaattat aaaaggtaac ttagtttaag tttatttgca actgcccttc ttcccgtttg 3300
gcactatggt ttgttgcctg ccgagctgat ctgagaagtc agcttgtccc gaggctgtcc 3360
atgtacgtta agtaaagtgc tcactgtgta taggaatctg tattttggag gtgcttgatc 3420
tatctacaaa gaaaaaaatt aggaatttat tataaaatgc tcctagaagt cttaatggtg 3480
tttatttttt aaaaccttgt aatgttagac ttgtgtgcat ggaagtgatt aaggtacatc 3540
attattgtag tttgaacatt gtacatgata agccttcccc cacccccgtt tttactgtat 3600
gtttttattg aatgatctat tccccatccc taggcaagca tgaataaaat taggttaaat 3660
gt 3662

<210> 11
<211> 821
<212> PRT
<213> Rattus norvegicus

<220>
<223> Neural receptor protein-tyrosine kinase

<400> 11
Met Ser Pro Trp Pro Arg Trp His Gly Pro Ala Met Ala Arg Leu Trp
1               5                   10                  15

Gly Leu Cys Leu Leu Val Leu Gly Phe Trp Arg Ala Ser Leu Ala Cys
            20                  25                  30

Pro Met Ser Cys Lys Cys Ser Thr Thr Arg Ile Trp Cys Thr Glu Pro
        35                  40                  45

Ser Pro Gly Ile Val Ala Phe Pro Arg Leu Glu Pro Asn Ser Ile Asp
    50                  55                  60

Pro Glu Asn Ile Thr Glu Ile Leu Ile Ala Asn Gln Lys Arg Leu Glu
65                  70                  75                  80

Ile Ile Asn Glu Asp Asp Val Glu Ala Tyr Val Gly Leu Lys Asn Leu
                85                  90                  95

Thr Ile Val Asp Ser Gly Leu Lys Phe Val Ala Tyr Lys Ala Phe Leu
            100                 105                 110

Lys Asn Gly Asn Leu Arg His Ile Asn Phe Thr Arg Asn Lys Leu Thr
        115                 120                 125

Ser Leu Ser Arg Arg His Phe Arg His Leu Asp Leu Ser Asp Leu Ile
    130                 135                 140

Leu Thr Gly Asn Pro Phe Thr Cys Ser Cys Asp Ile Met Trp Leu Lys
145                 150                 155                 160

Thr Leu Gln Glu Thr Lys Ser Ser Pro Asp Thr Gln Asp Leu Tyr Cys

                165                 170                 175

Leu Asn Glu Ser Ser Lys Asn Thr Pro Leu Ala Asn Leu Gln Ile Pro
            180                 185                 190

Asn Cys Gly Leu Pro Ser Ala Arg Leu Ala Ala Pro Asn Leu Thr Val
        195                 200                 205

Glu Glu Gly Lys Ser Val Thr Ile Ser Cys Ser Val Gly Gly Asp Pro
    210                 215                 220

Leu Pro Thr Leu Tyr Trp Asp Val Gly Asn Leu Val Ser Lys His Met
225                 230                 235                 240

Asn Glu Thr Ser His Thr Gln Gly Ser Leu Arg Ile Thr Asn Ile Ser
                245                 250                 255

Ser Asp Asp Ser Gly Lys Gln Ile Ser Cys Val Ala Glu Asn Leu Val
            260                 265                 270

Gly Glu Asp Gln Asp Ser Val Asn Leu Thr Val His Phe Ala Pro Thr
        275                 280                 285

Ile Thr Phe Leu Glu Ser Pro Thr Ser Asp His His Trp Cys Ile Pro
    290                 295                 300

Phe Thr Val Arg Gly Asn Pro Lys Pro Ala Leu Gln Trp Phe Tyr Asn
305                 310                 315                 320

Gly Ala Ile Leu Asn Glu Ser Lys Tyr Ile Cys Thr Lys Ile His Val
                325                 330                 335

Thr Asn His Thr Glu Tyr His Gly Cys Leu Gln Leu Asp Asn Pro Thr
            340                 345                 350

His Met Asn Asn Gly Asp Tyr Thr Leu Met Ala Lys Asn Glu Tyr Gly
        355                 360                 365

Lys Asp Glu Arg Gln Ile Ser Ala His Phe Met Gly Arg Pro Gly Val
    370                 375                 380

Asp Tyr Glu Thr Asn Pro Asn Tyr Pro Glu Val Leu Tyr Glu Asp Trp
385                 390                 395                 400

Thr Thr Pro Thr Asp Ile Gly Asp Thr Thr Asn Lys Ser Asn Glu Ile
                405                 410                 415

Pro Ser Thr Asp Val Ala Asp Gln Thr Asn Arg Glu His Leu Ser Val
            420                 425                 430

Tyr Ala Val Val Val Ile Ala Ser Val Val Gly Phe Cys Leu Leu Val
        435                 440                 445

Met Leu Leu Leu Leu Lys Leu Ala Arg His Ser Lys Phe Gly Met Lys
    450                 455                 460

Gly Pro Ala Ser Val Ile Ser Asn Asp Asp Asp Ser Ala Ser Pro Leu
465                 470                 475                 480

His His Ile Ser Asn Gly Ser Asn Thr Pro Ser Ser Ser Glu Gly Gly
                485                 490                 495

Pro Asp Ala Val Ile Ile Gly Met Thr Lys Ile Pro Val Ile Glu Asn
            500                 505                 510

Pro Gln Tyr Phe Gly Ile Thr Asn Ser Gln Leu Lys Pro Asp Thr Phe
        515                 520                 525

Val Gln His Ile Lys Arg His Asn Ile Val Leu Lys Arg Glu Leu Gly
    530                 535                 540

Glu Gly Ala Phe Gly Lys Val Phe Leu Ala Glu Cys Tyr Asn Leu Cys
545                 550                 555                 560

Pro Glu Gln Asp Lys Ile Leu Val Ala Val Lys Thr Leu Lys Asp Ala
                565                 570                 575

Ser Asp Asn Ala Arg Lys Asp Phe His Arg Glu Ala Glu Leu Leu Thr
            580                 585                 590

Asn Leu Gln His Glu His Ile Val Lys Phe Tyr Gly Val Cys Val Glu
        595                 600                 605

Gly Asp Pro Leu Ile Met Val Phe Glu Tyr Met Lys His Gly Asp Leu
    610                 615                 620

Asn Lys Phe Leu Arg Ala His Gly Pro Asp Ala Val Leu Met Ala Glu
625                 630                 635                 640

Gly Asn Pro Pro Thr Glu Leu Thr Gln Ser Gln Met Leu His Ile Ala
                645                 650                 655

Gln Gln Ile Ala Ala Gly Met Val Tyr Leu Ala Ser Gln His Phe Val
            660                 665                 670

His Arg Asp Leu Ala Thr Arg Asn Cys Leu Val Gly Glu Asn Leu Leu
        675                 680                 685

Val Lys Ile Gly Asp Phe Gly Met Ser Arg Asp Val Tyr Ser Thr Asp
    690                 695                 700

Tyr Tyr Arg Val Gly Gly His Thr Met Leu Pro Ile Arg Trp Met Pro
705                 710                 715                 720

Pro Glu Ser Ile Met Tyr Arg Lys Phe Thr Thr Glu Ser Asp Val Trp
                725                 730                 735

Ser Leu Gly Val Val Leu Trp Glu Ile Phe Thr Tyr Gly Lys Gln Pro
            740                 745                 750

Trp Tyr Gln Leu Ser Asn Asn Glu Val Ile Glu Cys Ile Thr Gln Gly
        755                 760                 765

Arg Val Leu Gln Arg Pro Arg Thr Cys Pro Gln Glu Val Tyr Glu Leu
    770                 775                 780

Met Leu Gly Cys Trp Gln Arg Glu Pro His Thr Arg Lys Asn Ile Lys
785                 790                 795                 800

Asn Ile His Thr Leu Leu Gln Asn Leu Ala Lys Ala Ser Pro Val Tyr
                805                 810                 815

Leu Asp Ile Leu Gly
            820

<210> 12
<211> 4757
<212> DNA
<213> Rattus norvegicus

<220>
<223> Neural receptor protein-tyrosine kinase

<400> 12
atctgtgtgc gagtgcgtgt gcgtgcgtgt gtgtgtgtgt gtgtgtgtgt gtgtgtgtgt 60
gtgtgagcgt gtgtgttttt ggatttcata ctaattttct ggagtttctg cccctgctct 120
gcgtcagccc tcacgtcact tcgccagcag tagcagaggc ggcggcggcc gccggttaga 180
gcccagtcgc tgcttcagct gctgttgctg cttctgcggc gctctgctcc ctgcgctggc 240
tacgggaggc cgggggagcc gcgccgacag tcctctgtgg ccagggccgg cactgtcctg 300
ctaccgcagt tgctccccag ccctgaggtg cgcaccgata tcgatatccg tgccggttta 360
gcggttctgc gacccaaaga gtccagggag agccaccgag tggcgcctgg cgtataggac 420
catgcagccg ccttgtggct tggagcagcg gcccgtgatg ttccagccac tgtgaaccat 480
ttggtcagcg ccaacctgct cagccccagc accgacaggc tcagcctctg gtacgctcct 540
ctcggcggga ggccatcagc accaagcagc aagagggctc agggaaggcc tcccccctcc 600
ggcgggggac gcctggctca gcgtagggac acgcactctg actgactggc actggcagct 660
cgggatgtcg ccctggccga ggtggcatgg acccgccatg gcgcggctct ggggcttatg 720
cttgctggtc ttgggcttct ggagggcttc tcttgcctgc cccatgtcct gcaaatgcag 780
caccactagg atttggtgta ccgagccttc tcctggcatc gtggcatttc cgaggttgga 840
acctaacagc attgacccag agaacatcac cgaaattctc attgcaaacc agaaaaggtt 900
agaaatcatc aatgaagatg atgtcgaagc ttacgtgggg ctgaaaaacc ttacaattgt 960
ggattccggc ttaaagtttg tggcttacaa ggcgtttctg aagaacggca acctgcggca 1020
catcaatttc actcgaaaca agctgacgag tttgtccagg agacatttcc gccaccttga 1080
cttgtctgac ctgatcctga cgggtaatcc gttcacgtgt tcctgtgaca tcatgtggct 1140
caagactctc caggagacga aatccagccc cgacactcag gatttgtatt gcctcaatga 1200
gagcagcaag aatacccctc tggcgaacct gcagattccc aattgtggtc tgccgtctgc 1260
acgtctggcc gctcctaacc tcacggtgga ggaagggaag tctgtgacca tttcctgcag 1320
cgtcgggggt gacccgctcc ccaccttgta ctgggacgtt gggaatttgg tttccaaaca 1380
catgaatgaa acaagccaca cacagggctc cttaaggata acaaacattt catcggatga 1440
cagtgggaaa caaatctctt gtgtggcaga aaacctcgtc ggagaagatc aagactctgt 1500
gaacctcact gtgcattttg caccaaccat cacatttctc gaatctccaa cctcagacca 1560
ccactggtgc atcccattca ctgtgagagg caaccccaag ccagcacttc agtggttcta 1620
caacggagcc atactgaatg aatccaagta catctgtacc aaaatacacg tcaccaatca 1680
cacggagtac cacggctgcc tccagctgga taaccccact catatgaata atggagacta 1740
caccctaatg gccaagaatg aatatgggaa ggacgagaga cagatttctg ctcacttcat 1800
gggccggcct ggagttgact atgagacaaa cccaaattac cctgaagtcc tctatgaaga 1860
ctggaccacg ccaactgaca tcggggatac tacaaacaaa agtaatgaga tcccctccac 1920
ggatgttgct gaccaaacca atcgggagca tctctcggtc tatgctgtgg tggtgattgc 1980
ctctgtggta ggattctgcc tgctggtgat gctgcttctg ctcaagttgg cgagacattc 2040
caagtttggc atgaaaggcc cagcttccgt catcagcaac gacgatgact ctgccagccc 2100
tctccaccac atctccaacg ggagcaacac tccgtcttct tcggagggcg ggcccgatgc 2160
tgtcatcatt gggatgacca agatccctgt cattgaaaac ccccagtact tcggtatcac 2220
caacagccag ctcaagccgg acacatttgt tcagcacatc aagagacaca acatcgttct 2280
gaagagggag cttggagaag gagcctttgg gaaagttttc ctagcggagt gctataacct 2340
ctgccccgag caggataaga tcctggtggc cgtgaagacg ctgaaggacg ccagcgacaa 2400
tgctcgcaag gactttcatc gcgaagccga gctgctgacc aacctccagc acgagcacat 2460
tgtcaagttc tacggtgtct gtgtggaggg cgacccactc atcatggtct ttgagtacat 2520
gaagcacggg gacctcaaca agttccttag ggcacacggg ccagatgcag tgctgatggc 2580
agagggtaac ccgcccaccg agctgacgca gtcgcagatg ctgcacatcg ctcagcaaat 2640
cgcagcaggc atggtctacc tggcatccca acacttcgtg caccgagacc tggccacccg 2700
gaactgcttg gtaggagaga acctgctggt gaaaattggg gacttcggga tgtcccggga 2760
tgtatacagc accgactact accgggttgg tggccacaca atgttgccca tccgatggat 2820
gcctccagag agcatcatgt acaggaaatt caccaccgag agtgacgtct ggagcctggg 2880

agttgtgttg tgggagatct tcacctacgg caagcagccc tggtatcagc tatcaaacaa 2940
cgaggtgata gaatgcatca cccagggcag agtccttcag cggcctcgca cgtgtcccca 3000
ggaggtgtac gagctgatgc tgggatgctg gcagcgggaa ccacacacaa ggaagaacat 3060
caagaacatc cacacactcc ttcagaactt ggcgaaggcg tcgcccgtct acctggacat 3120
cctaggctag actccctctt ctcccagacg gcccttccca aggcacccct cagacctctt 3180
aactgccgct gatgtcacca ccttgctgtc cttcgctctg acagtgttaa caagacaagg 3240
agcggctctc cggggtgagg cagtgcgcac ttccccatcc acagacagta tcgactcgct 3300
tctggctttg tcgctttctc tccctttggt ttgtttcttt cttttgccca ttctccattt 3360
atttatttat ttatttattt atttatttat ttatttattt atttatctat ctatctatct 3420
atttatttat ttatttattg gtcttcactg cttcatggtc ctcggcctct ctccttgacc 3480
gatctggctt ctgtactcct attcactgta catagacaaa ggccttaaca aacctgattt 3540
gttatatcag cagacactcc agtttgccca ccacaactaa caatgccttg ttgtattcct 3600
gcctttgatg tggatgaaaa aaagggaaaa aaaataatca aacatctgac ttaaaccgtc 3660
acttccgatg tacagacacg gggcgtttct atggattcac ttctatctat ctatttattt 3720
atttatctat ttatttattt ctcttctttg ttgttttccg gtggttttag cctgtgtatg 3780
agaagggaaa gtcatgtaca gtctgggaaa actttatctg tgggaaatgg aaaccagaag 3840
gggaaagaag ctttaccata aagcacagca ggagtgagac acagaaaagc cattggatca 3900
gccagagtcc gtcctgcata ggaaaaccca gcagccatca ggctggagga tcatgttcgg 3960
cactgacccc cgaggacctt tctgaggagg acacagaatg ttaaactctg catcatggac 4020
acagtttccg atcacagata ctggccttca atggaaaaaa aaaaaaaaaa aacccagata 4080
gttcttgtga gacctggaca gcacgtccaa catccagaca ttgtggtcgg gcacagtgac 4140
agagttgatg catttctcac gggttattct acagagcttt tgtcaagtcc aatggaagga 4200
ggtagattct tgttcagata tgatttcggg aaaaaccgag tccttgacaa agacaggaga 4260
caccctcagt tgggaggcaa gtttctctta ccttggactt tctcacacag caattctcac 4320
ccccaccccc tccactctca cctgtcttgt aactgtgcaa acaaaagtgt gcatggtctt 4380
tgtcagttga tacctttgtg cacctctgtg cagaaactgc tgtctgtccc ggctgtggta 4440
cccgatcagt ggggtagatc cacgaaaggt ctcattttag gccgctttgg gaaggtaacc 4500
agatcggtag ctggaagcac tctccagtag gtggcgaagg gtgagtgggt ctgctgaagc 4560
ctgcatatct tcacccacct caaacccacc gggctgcaca ggggacaggc acaggccacc 4620
cctgagggac agggaagctc tcttgggata ccacctgagt ttacattcag tgtgctcagg 4680
tcaagtctct cgctcggggc tctgtttcgg ggagaatggt ttcattccaa cgcactcatt 4740
atcaggattc tgttttc 4757

<210> 13
<211> 167
<212> PRT
<213> Rattus norvegicus

<220>
<223> Protein tyrosine phosphatase

<400> 13
Met Asn Arg Pro Ala Pro Val Glu Ile Ser Tyr Glu Asn Met Arg Phe
1               5                   10                  15

Leu Ile Thr His Asn Pro Ala Asn Ala Thr Leu Asn Lys Phe Thr Glu
            20                  25                  30

Glu Leu Lys Lys Tyr Gly Val Thr Thr Leu Val Arg Val Cys Asp Ala
        35                  40                  45

Thr Tyr Asp Lys Ala Pro Val Glu Lys Glu Gly Ile His Val Leu Asp
    50                  55                  60

Trp Pro Phe Asp Asp Gly Ala Pro Pro Pro Asn Gln Ile Val Asp Asp
65                  70                  75                  80

Trp Leu Asn Leu Leu Lys Thr Lys Phe Arg Glu Glu Pro Gly Cys Cys
                85                  90                  95

Val Ala Val His Cys Val Ala Gly Leu Gly Arg Ala Pro Val Leu Val
            100                 105                 110

Ala Leu Ala Leu Ile Glu Cys Gly Met Lys Tyr Glu Asp Ala Val Gln
        115                 120                 125

Phe Ile Arg Gln Lys Arg Arg Gly Ala Phe Asn Ser Lys Gln Leu Leu
    130                 135                 140

Tyr Leu Glu Lys Tyr Arg Pro Lys Met Arg Leu Arg Ser Arg Asp Thr
145                 150                 155                 160

Asn Gly His Cys Cys Val Gln
                165


<210> 14
<211> 1095
<212> DNA
<213> Rattus norvegicus

<220>
<223> Protein tyrosine phosphatase

<400> 14
aaattgcaag cgtattcttt taatgactcc agtaaaatta agcatcaagt aaacaaaagt 60
ggaaaagaac ctacactttt aacttgtctc actagtgcct aaatgtagtt taaaggctgc 120
ttaaattttg tgtgtagttg gattttttgg aagctgaagg tatccatctg cagacattga 180
ggcccaagtt gaatttggat tcgagtggat tcttaacact tctgcctgtg ctgaagagaa 240
gcttcataag gaacaagcaa gttgaataga gaagatagtg atcaataaga ggcatttagt 300
ggtcttttta atgttttctg ctgcgaaaca tttcaagatt tattgatttt tttttttcat 360
tttccccacc acactcacac acgcacgctc acacttttta tttgccataa tgaaccgtcc 420
agcccctgtg gagatctcgt acgagaacat gcgttttctg ataactcaca acccagccaa 480
tgcgactctc aacaagttca ccgaggaact caagaagtac ggagtgacaa ctttggtccg 540
agtttgtgat gctacctatg acaaagctcc agttgaaaaa gaagggatcc acgttctaga 600
ctggccattt gatgacggag ctccgccccc taatcagata gtagatgatt ggctaaacct 660
gttaaagacc aaattccggg aagaaccagg ctgctgtgtc gcagtgcatt gtgttgcagg 720
attgggaagg gctcctgtgc tagttgcact tgcactgatt gaatgcggaa tgaagtatga 780
agatgctgtt cagtttataa gacaaaaaag aagaggagca ttcaattcca aacagctgct 840
ttacttggag aaataccgac ctaagatgcg attacgctcc agagatacca acgggcactg 900
ctgtgttcag tagaagcaga ggaaggccgg ctggatcgtg gcattagagg gaactctggg 960
tacctggaaa tgtgaatctg gattcttacc tgtgtcatca aagtagtgat ggattccgta 1020
ctcctcgact cctcatgatt gagaagaagg caaacgataa agaaatccct ctataacacg 1080
aataaaatgt ttaag                                                  1095


<210> 15
<211> 1436
<212> PRT
<213> Rattus norvegicus

<220>
<223> Receptor protein tyrosine phosphatase-lambda

<400> 15
Met Ala Arg Ala Gln Ala Leu Val Leu Ala Leu Thr Phe Gln Phe Cys
  1               5                  10                  15

Ala Pro Glu Thr Glu Thr Pro Ala Ala Gly Cys Thr Phe Glu Glu Ala
             20                  25                  30

Ser Asp Pro Val Val Pro Cys Glu Phe Ser Gln Ala Gln Tyr Asp Asp
        35                  40                  45

Phe Gln Trp Glu Gln Val Arg Ile His Pro Gly Thr Arg Thr Pro Glu
    50                  55                  60

Asp Leu Pro His Gly Ala Tyr Leu Met Val Asn Ala Ser Gln His Thr
65                  70                  75                  80

Pro Gly Gln Arg Ala His Ile Ile Phe Gln Thr Leu Ser Glu Asn Asp
                85                  90                  95

Thr His Cys Val Gln Phe Ser Tyr Phe Leu Tyr Ser Arg Asp Gly His
            100                 105                 110

Ser Pro Gly Thr Leu Gly Val Tyr Val Arg Val Asn Gly Gly Pro Leu
        115                 120                 125

Gly Ser Ala Val Trp Asn Met Thr Gly Ser His Gly Arg Gln Trp His
    130                 135                 140

Gln Ala Glu Leu Ala Val Ser Thr Phe Trp Pro Asn Glu Phe Gln Val
145                 150                 155                 160

Leu Phe Glu Ala Leu Ile Ser Pro Asp His Lys Gly Tyr Ile Gly Leu
                165                 170                 175

Asp Asp Ile Leu Leu Phe Ser Tyr Pro Cys Ala Lys Ala Pro His Phe
            180                 185                 190

Ser Arg Leu Gly Asp Val Glu Val Asn Ala Gly Gln Asn Ala Ser Phe
        195                 200                 205

Gln Cys Met Ala Ala Gly Arg Ala Ala Glu Ala Glu His Phe Phe Leu
    210                 215                 220

Gln Arg Gln Ser Gly Val Leu Val Pro Ala Ala Gly Val Arg His Ile
225                 230                 235                 240

Ser His Arg Arg Phe Leu Ala Thr Phe Pro Leu Ala Ser Val Gly Arg
                245                 250                 255

Ser Glu Gln Asp Leu Tyr Arg Cys Val Ser Gln Ala Pro Arg Gly Ala
            260                 265                 270

Gly Val Ser Asn Phe Ala Glu Leu Ile Val Lys Glu Pro Pro Thr Pro
        275                 280                 285

Ile Ala Pro Pro Gln Leu Leu Arg Ala Gly Pro Thr Tyr Leu Ile Ile
    290                 295                 300

Gln Leu Asn Thr Asn Ser Ile Ile Gly Asp Gly Pro Ile Val Arg Lys
305                 310                 315                 320

Glu Ile Glu Tyr Arg Met Ala Arg Gly Pro Trp Ala Glu Val His Ala
                325                 330                 335

Val Asn Leu Gln Thr Tyr Lys Leu Trp His Leu Asp Pro Asp Thr Glu
            340                 345                 350

Tyr Glu Ile Ser Val Leu Leu Thr Arg Pro Gly Asp Gly Gly Thr Gly

        355                 360                 365

Arg Pro Gly Pro Pro Leu Ile Ser Arg Thr Lys Cys Ala Glu Pro Thr
    370                 375                 380

Arg Ala Pro Lys Gly Leu Ala Phe Ala Glu Ile Gln Ala Arg Gln Leu
385                 390                 395                 400

Thr Leu Gln Trp Glu Pro Leu Gly Tyr Asn Val Thr Arg Cys His Thr
                405                 410                 415

Tyr Ala Val Ser Leu Cys Tyr Arg Tyr Thr Leu Gly Gly Ser His Asn
            420                 425                 430

Gln Thr Ile Arg Glu Cys Val Lys Met Glu Arg Gly Ala Ser Arg Tyr
        435                 440                 445

Thr Ile Lys Asn Leu Leu Pro Phe Arg Asn Ile His Val Arg Leu Ile
    450                 455                 460

Leu Thr Asn Pro Glu Gly Arg Lys Glu Gly Lys Glu Val Thr Phe Gln
465                 470                 475                 480

Thr Asp Glu Asp Val Pro Gly Gly Ile Ala Ala Glu Ser Leu Thr Phe
                485                 490                 495

Thr Pro Leu Glu Asp Met Ile Phe Leu Lys Trp Glu Glu Pro Gln Glu
            500                 505                 510

Pro Asn Gly Leu Ile Thr Gln Tyr Glu Ile Ser Tyr Gln Ser Ile Glu
        515                 520                 525

Ser Ser Asp Pro Ala Val Asn Val Pro Gly Pro Arg Arg Thr Ile Ser
    530                 535                 540

Lys Leu Arg Asn Glu Thr Tyr His Val Phe Ser Asn Leu His Pro Gly
545                 550                 555                 560

Thr Thr Tyr Leu Phe Ser Val Arg Ala Arg Thr Ser Lys Gly Phe Gly
                565                 570                 575

Gln Ala Ala Leu Thr Glu Ile Thr Thr Asn Ile Ser Ala Pro Ser Phe
            580                 585                 590

Asp Tyr Ala Asp Met Pro Ser Pro Leu Gly Glu Ser Glu Asn Thr Ile
        595                 600                 605

Thr Val Leu Leu Arg Pro Ala Gln Gly Arg Gly Ala Pro Ile Ser Val
    610                 615                 620

Tyr Gln Val Val Val Glu Glu Glu Arg Pro Arg Arg Leu Arg Arg Glu
625                 630                 635                 640

Pro Gly Ala Gln Asp Cys Phe Ser Val Pro Leu Thr Phe Glu Thr Ala
                645                 650                 655

Leu Ala Arg Gly Leu Val His Tyr Phe Gly Ala Glu Leu Ala Ala Ser
            660                 665                 670

Ser Leu Leu Glu Ala Met Pro Phe Thr Val Gly Asp Asn Gln Thr Tyr
        675                 680                 685

Arg Gly Phe Trp Asn Pro Pro Leu Glu Pro Arg Lys Ala Tyr Leu Ile
    690                 695                 700

Tyr Phe Gln Ala Ala Ser His Leu Lys Gly Glu Thr Arg Leu Asn Cys
705                 710                 715                 720

Ile Arg Ile Ala Arg Lys Ala Ala Cys Lys Glu Ser Lys Arg Pro Leu
                725                 730                 735

Glu Val Ser Gln Arg Ser Glu Glu Met Gly Leu Ile Leu Gly Ile Cys
            740                 745                 750

Ala Gly Gly Leu Ala Val Leu Ile Leu Leu Leu Gly Ala Ile Ile Val
        755                 760                 765

Ile Ile Arg Lys Gly Lys Pro Val Asn Met Thr Lys Ala Thr Val Asn
    770                 775                 780

Tyr Arg Gln Glu Lys Thr His Met Met Ser Ala Val Asp Arg Ser Phe
785                 790                 795                 800

Thr Asp Gln Ser Thr Leu Gln Glu Asp Glu Arg Leu Gly Leu Ser Phe
                805                 810                 815

Met Asp Ala Pro Gly Tyr Ser Pro Arg Gly Asp Gln Arg Ser Gly Gly
            820                 825                 830

Val Thr Glu Ala Ser Ser Leu Leu Gly Gly Ser Pro Arg Arg Pro Cys
        835                 840                 845

Gly Arg Lys Gly Ser Pro Tyr His Thr Gly Gln Leu His Pro Ala Val
    850                 855                 860

Arg Val Ala Asp Leu Leu Gln His Ile Asn Gln Met Lys Thr Ala Glu
865                 870                 875                 880

Gly Tyr Gly Phe Lys Gln Glu Tyr Glu Ser Phe Phe Glu Gly Trp Asp
                885                 890                 895

Ala Thr Lys Lys Lys Asp Lys Leu Lys Gly Gly Arg Gln Glu Pro Val
            900                 905                 910

Ser Ala Tyr Asp Arg His His Val Lys Leu His Pro Met Leu Ala Asp
        915                 920                 925

Pro Asp Ala Asp Tyr Ile Ser Ala Asn Tyr Ile Asp Gly Tyr His Arg
    930                 935                 940

Ser Asn His Phe Ile Ala Thr Gln Gly Pro Lys Pro Glu Met Ile Tyr
945                 950                 955                 960

Asp Phe Trp Arg Met Val Trp Gln Glu Gln Cys Ala Ser Ile Val Met
                965                 970                 975

Ile Thr Lys Leu Val Glu Val Gly Arg Val Lys Cys Ser Arg Tyr Trp
            980                 985                 990

Pro Glu Asp Ser Asp Met Tyr Gly Asp Ile Lys Ile Thr Leu Val Lys
        995                 1000                1005

Thr Glu Thr Leu Ala Glu Tyr Val Val Arg Thr Phe Ala Leu Glu Arg
1010                1015                1020

Arg Gly Tyr Ser Ala Arg His Glu Val Arg Gln Phe His Phe Thr Ala
1025                1030                1035                1040

Trp Pro Glu His Gly Val Pro Tyr His Ala Thr Gly Leu Leu Ala Phe
                1045                1050                1055

Ile Arg Arg Val Lys Ala Ser Thr Pro Pro Asp Ala Gly Pro Ile Val
            1060                1065                1070

Ile His Cys Ser Ala Gly Thr Gly Arg Thr Gly Cys Tyr Ile Val Leu
        1075                1080                1085

Asp Val Met Leu Asp Met Ala Glu Cys Glu Gly Val Val Asp Ile Tyr
    1090                1095                1100

Asn Cys Val Lys Thr Leu Cys Ser Arg Arg Val Asn Met Ile Gln Thr
1105                1110                1115                1120

Glu Glu Gln Tyr Ile Phe Ile His Asp Ala Ile Leu Glu Ala Cys Leu
                1125                1130                1135

Cys Gly Glu Thr Thr Ile Pro Val Asn Glu Phe Arg Ala Thr Tyr Arg
            1140                1145                1150

Glu Met Ile Arg Ile Asp Pro Gln Ser Asn Ser Ser Gln Leu Arg Glu
        1155                1160                1165

Glu Phe Gln Thr Leu Asn Ser Val Thr Pro Pro Leu Asp Val Glu Glu
    1170                1175                1180

Cys Ser Ile Ala Leu Leu Pro Arg Asn Arg Asp Lys Asn Arg Ser Met
1185                1190                1195                1200

Asp Val Leu Pro Pro Asp Arg Cys Leu Pro Phe Leu Ile Ser Ser Asp
                1205                1210                1215

Gly Asp Pro Asn Asn Tyr Ile Asn Ala Ala Leu Thr Asp Ser Tyr Thr
            1220                1225                1230

Arg Ser Ala Ala Phe Ile Val Thr Leu His Pro Leu Gln Ser Thr Thr
        1235                1240                1245

Pro Asp Phe Trp Arg Leu Val Tyr Asp Tyr Gly Cys Thr Ser Ile Val
    1250                1255                1260

Met Leu Asn Gln Leu Asn Gln Ser Asn Ser Ala Trp Pro Cys Leu Gln
1265                1270                1275                1280

Tyr Trp Pro Glu Pro Gly Arg Gln Gln Tyr Gly Leu Met Glu Val Glu
                1285                1290                1295

Phe Val Ser Gly Thr Ala Asn Glu Asp Leu Val Ser Arg Val Phe Arg
            1300                1305                1310

Val Gln Asn Ser Ser Arg Leu Gln Glu Gly His Leu Leu Val Arg His
        1315                1320                1325

Phe Gln Phe Leu Arg Trp Ser Ala Tyr Arg Asp Thr Pro Asp Ser Arg

1330                1335                1340

Lys Ala Phe Leu His Leu Leu Ala Glu Val Asp Lys Trp Gln Ala Glu
1345                1350                1355                1360

Ser Gly Asp Gly Arg Thr Val Val His Cys Leu Asn Gly Gly Gly Arg
                1365                1370                1375

Ser Gly Thr Phe Cys Ala Cys Ala Thr Val Leu Glu Met Ile Arg Cys
            1380                1385                1390

His Ser Leu Val Asp Val Phe Phe Ala Ala Lys Thr Leu Arg Asn Tyr
        1395                1400                1405

Lys Pro Asn Met Val Glu Thr Met Asp Gln Tyr His Phe Cys Tyr Asp
    1410                1415                1420

Val Ala Leu Glu Tyr Leu Glu Ala Leu Glu Leu Arg
1425                1430                1435

<210> 16
<211> 5732
<212> DNA
<213> Rattus norvegicus

<220>
<223> Receptor protein tyrosine phosphatase-lambda

<400> 16
gttgactact cagctgccag aacatccaat ctggctcctg caactttaga ccaacatatt 60
gtgtttgatc ttctcctgaa caacttggga gatacgtctg atcttcagct tggtacatac 120
agttgcgcag tgaatggcac ttacgtgttc attgtgcaca tgctaaagct ggcatgatta 180
atgttcgact gctatgtcaa cctgattaac aatgaggatg tcttggtgtc agctatgcca 240
acgatggtgc tccagaccgg cgccagtccc gctccgcgcg gcactgtcca ctacggctcc 300
cgctcgcctt gggctcccgg tcgggctccg gaggcgtcgc ctccccagct gcgggtctcc 360
aggacctagg cggcggccat ggcccgggct caggctctgg tcctggcgct caccttccag 420
ttctgcgcgc ctgagaccga gactcccgca gctggctgca ccttcgagga ggcgagtgac 480
ccggtcgtgc cctgcgagtt cagccaggct cagtatgacg acttccaatg ggagcaagtg 540
cggatccacc ccggcacccg gacccctgaa gacctgcccc atggtgccta cttgatggtc 600
aatgcttctc agcatacccc aggtcagagg gcccacatca tcttccagac cctgagcgag 660
aacgacaccc attgtgtgca gttcagctac ttcctgtaca gcagggatgg gcacagccca 720
ggcaccctgg gggtctacgt gcgcgtgaat gggggccctc tgggcagtgc cgtgtggaat 780
atgaccggat cccacggccg tcagtggcac caggctgagc tggctgtcag caccttctgg 840
cccaatgagt ttcaggtgct gtttgaggcc ctcatctccc cagaccacaa gggctacata 900
ggcttagacg acatcttgct cttcagctat ccctgcgcaa aggcccctca cttctcccgc 960
cttggggacg tggaggtcaa tgcaggccag aacgcatcct tccaatgcat ggcagcaggc 1020
agagccgcag aggcagaaca cttcttcctg cagcgtcaga gtggagtgct ggtgcctgcg 1080
gccggggtgc ggcacatcag tcaccgtcgc ttcctggcca cttttccgct ggcctcggta 1140
ggccgctcag agcaggatct gtaccgttgc gtgtcccagg ccccgcgtgg tgctggcgtc 1200
tccaactttg cagagctcat cgtcaaagag cctcccaccc ccatcgcgcc cccacagctg 1260
ctgcgtgcag gccccaccta cctcattatc cagctcaaca ccaactccat cattggcgac 1320
gggccgatcg tgcgcaagga gatcgagtac cgcatggcac ggggcccgtg ggccgaggtg 1380
cacgctgtca acctgcagac ctacaagctg tggcatctgg acccagacac tgagtatgaa 1440
atcagcgtgc tgctcacacg cccgggagat ggaggcacag gccgccctgg gccaccactg 1500
atcagccgga ccaagtgcgc agagcccacg agggccccca aaggtctggc ttttgctgag 1560
atccaggctc gccagctgac cctgcagtgg gagcccctgg gctataatgt cacacgttgt 1620
catacctacg ctgtgtccct ttgctatcgc tacaccctgg gcggcagcca caaccagacc 1680
atccgggagt gtgtgaagat ggagcggggt gccagccgct acaccatcaa gaatctgctg 1740
ccattcagaa acatccacgt gcgtctgatt ctcacaaacc ctgaggggcg caaggagggc 1800
aaggaggtca ccttccagac agatgaagat gtgcctggtg ggattgcagc tgagtcccta 1860

accttcactc cactggagga catgatcttt ctcaagtggg aggagcccca ggagcccaat 1920
ggcctcatca ctcagtatga gatcagctac caaagcattg agtcctcaga cccagcagtg 1980
aacgtgcccg gcccgagacg caccatctcc aaactccgga atgagactta ccacgtcttc 2040
tccaacctgc atcccggcac cacgtatctg ttctccgtgc gtgctcggac gagcaagggc 2100
ttcggccagg cggctctcac tgagataacc accaacatct cagctcccag ctttgattat 2160
gccgacatgc cgtcacccct gggcgagtcc gagaacacca tcactgtgct gttgaggccg 2220
gcccagggcc gaggagcccc catcagcgtc taccaggtgg ttgtggagga agagcggcca 2280
cggcgcttgc ggcgggagcc cggagctcag gactgcttct cggtacctct gacctttgag 2340
acggccctgg ctcgcggcct ggtgcactac tttggggctg aactggctgc cagcagcctg 2400
cttgaggcca tgcccttcac cgtgggtgac aaccagacct atcgtggctt ctggaaccca 2460
ccgcttgagc ccagaaaggc ctatctcatc tatttccagg cagcaagcca cctgaaaggg 2520
gaaacccgac tgaactgcat ccgaattgcc aggaaagctg cgtgcaagga gagcaagcga 2580
cccctcgaag tgtcccagag atcggaggag atggggctca tcctgggcat ctgtgcaggt 2640
ggtcttgccg tcctcattct cctcctgggg gccatcattg tcatcatccg caaagggaag 2700
ccagtgaaca tgacgaaagc cacggtcaac taccgccagg agaagactca catgatgagt 2760
gccgtggacc gcagcttcac agatcagagt actctgcagg aggatgagcg gttgggtctg 2820
tcctttatgg atgctcctgg ctatagtcct cgtggagacc agcgaagcgg tggtgtcacc 2880
gaggccagca gcctcctggg gggttctcca aggcgcccat gcggccggaa gggttctccg 2940
tatcataccg ggcagctcca ccctgcagtc cgagtggctg accttctaca gcacatcaac 3000
cagatgaaga cagccgaggg ctacggcttc aagcaggagt acgagagttt ctttgagggc 3060
tgggacgcca ccaagaagaa agacaagctc aagggcggcc gacaggagcc agtgtctgcc 3120
tatgatcgac accatgtgaa actacacccg atgctggcag accctgatgc cgactacatc 3180
tctgccaact acatagacgg ctaccacagg tcaaaccact tcatagccac tcaagggcca 3240
aagcctgaga tgatctacga tttctggcgc atggtgtggc aggaacagtg tgcgagcatc 3300
gtcatgatca ccaagctggt agaggtgggc agggtgaagt gttctcgcta ctggcctgag 3360
gactcagaca tgtatgggga catcaagatc acgctggtaa agacagagac actggctgag 3420
tatgtggtgc gcacctttgc cctggagcgg agaggttact cagcccggca tgaggtccgc 3480
cagttccatt tcacagcgtg gccagagcat ggtgtcccct accacgccac ggggctgctg 3540
gccttcatcc ggcgtgtgaa ggcttccact ccacctgatg ccgggcccat tgtcattcac 3600
tgcagtgcag gaactggccg cacaggctgc tacatcgtcc tggatgtgat gctggacatg 3660
gctgaatgtg agggggtcgt ggacatttac aactgtgtga agaccctctg ttcccgacgg 3720
gtcaacatga tccagacgga ggaacaatat atcttcatcc acgatgcaat cttggaggcc 3780
tgcctgtgtg gggagaccac catccctgtc aacgagttca gggccaccta cagggagatg 3840
atccgcattg accctcagag caattcctcc cagcttcggg aagagttcca gacgctgaac 3900
tcggtcacgc cgccgctgga tgtggaggag tgtagcattg ccctgctgcc ccggaatcga 3960
gacaagaacc gtagcatgga tgtgctgcca ccagaccgct gcctgccctt cctcatctcc 4020
agtgatgggg accccaataa ctacatcaat gcagcactga ctgacagcta cacacggagc 4080
gccgccttca tcgtgaccct gcacccgctg cagagtacca cgcccgactt ctggcggctg 4140
gtctacgact acgggtgcac ctccatcgtc atgctgaacc aacttaacca gtccaactcc 4200
gcctggccct gcttgcagta ctggccggag ccaggccgac agcagtatgg gctcatggag 4260
gtggagtttg tgtctggcac agcaaacgag gatttggtgt cccgagtgtt ccgggtgcag 4320
aactcttctc ggctgcagga gggtcacctg ctggtacggc acttccagtt tctgcgttgg 4380
tctgcttatc gggacacgcc tgactccagg aaggcctttc tgcacctgtt ggctgaggtg 4440
gacaagtggc aggcagagag tggggatggg cgcaccgtgg tgcattgtct caacgggggt 4500
ggccgcagtg gcaccttctg cgcctgtgcc acggtcttgg agatgatccg ctgtcacagc 4560
ctggtggatg ttttctttgc tgccaaaaca cttcggaact acaagcccaa tatggtggag 4620
accatggatc agtatcattt ctgctacgac gtggccctgg agtacctgga ggctctggag 4680
ttgagatagc aggcgcctga cctggggcac ccagtgaaca cccagggcat ggcccatcat 4740
cccagatgag gagggcctgt ggccccaact ttgctcagcc ataattccac agggacaaca 4800
ctggaacgga cggacactgc accatcttgg tgacccccac gggaaggctg caggccaagg 4860
agaagctttg caagactgta tcagccccac ctctagaggg ccctgcagac ctgtgcagag 4920
aagctcgcct ggaccaaaat agctagtgct ggagagcaca ggccaggccc ctctgctcca 4980
tcacagtcct tggccagaaa tgaatgagtg tctgcagaga gcacccatgg tttgcaccca 5040
gtatggtcct ttctgcacgt ggtggaggct cactgggact tggcaggggc tgagtccccg 5100
agagtcctga agctgggact cttccccgtc tcgccggtgg gacccgctga gcatcctgca 5160
gctccattct ccatccccac tgcccctaca gacctggggt gctttgctcg ctttcctcct 5220
gcttctgagc ttttcctgca acaggacccg tgcctccttc ctgggctcca tccctgcctg 5280
gcccagtata tgcagaatga tatacttcag ctccttcttc cctggccttt gggtctccca 5340
tggttcagtc ctgctcagct tgggcctgtg acaatccaca aggctgaatc acagcccctg 5400
gggttgaggt ccctgtggct cttggtgagg ctgccactgg atcggggcag gctagaacag 5460
ggctggtgtc agctcctaga gtacagagga agaagggata ctttggaatg gaggaccagt 5520

gctttttttg ttgttgttat tttgttattt ttttgatggg agggtgggaa gttctcttta 5580
taatggggta ggccacaccc ccatttcgtg cctcaatttc cccatctgta aactgtagat 5640
atgactactg acctacctca cagggggctg tggggaggtg taaggtaatg tttgtaaagc 5700
gctttgtaaa taaatgtgct ctctgaatgc ca 5732

<210> 17
<211> 525
<212> PRT
<213> Rattus norvegicus

<220>
<223> Calcineurin A-beta

<400> 17
Met Ala Ala Pro Glu Pro Ala Arg Ala Ala Pro Pro Pro Pro Pro Pro
1               5                   10                  15

Pro Pro Pro Pro Leu Gly Ala Asp Arg Val Val Lys Ala Val Pro Phe
            20                  25                  30

Pro Pro Thr His Arg Leu Thr Ser Glu Glu Val Phe Asp Met Asp Gly
        35                  40                  45

Ile Pro Arg Val Asp Val Leu Lys Asn His Leu Val Lys Glu Gly Arg
    50                  55                  60

Val Asp Glu Glu Ile Ala Leu Arg Ile Ile Asn Glu Gly Ala Ala Ile
65                  70                  75                  80

Leu Arg Arg Glu Lys Thr Met Ile Glu Val Glu Ala Pro Ile Thr Val
                85                  90                  95

Cys Gly Asp Ile His Gly Gln Phe Phe Asp Leu Met Lys Leu Phe Glu
            100                 105                 110

Val Gly Gly Ser Pro Ala Asn Thr Arg Tyr Leu Phe Leu Gly Asp Tyr
        115                 120                 125

Val Asp Arg Gly Tyr Phe Ser Ile Glu Cys Val Leu Tyr Leu Trp Val
    130                 135                 140

Leu Lys Ile Leu Tyr Pro Ser Thr Leu Phe Leu Leu Arg Gly Asn His
145                 150                 155                 160

Glu Cys Arg His Leu Thr Glu Tyr Phe Thr Phe Lys Gln Glu Cys Lys
                165                 170                 175

Ile Lys Tyr Ser Glu Arg Val Tyr Glu Ala Cys Met Glu Ala Phe Asp
            180                 185                 190

Ser Leu Pro Leu Ala Ala Leu Leu Asn Gln Gln Phe Leu Cys Val His
        195                 200                 205

Gly Gly Leu Ser Pro Glu Ile His Thr Leu Asp Asp Ile Arg Arg Leu
    210                 215                 220

Asp Arg Phe Lys Glu Pro Pro Ala Phe Gly Pro Met Cys Asp Leu Leu
225                 230                 235                 240

Trp Ser Asp Pro Ser Glu Asp Phe Gly Asn Glu Lys Ser Gln Glu His

245                 250                 255

Phe Ser His Asn Thr Val Arg Gly Cys Ser Tyr Phe Tyr Asn Tyr Pro
            260                 265                 270

Ala Val Cys Glu Phe Leu Gln Asn Asn Asn Leu Leu Ser Ile Ile Arg
        275                 280                 285

Ala His Glu Ala Gln Asp Ala Gly Tyr Arg Met Tyr Arg Lys Ser Gln
    290                 295                 300

Thr Thr Gly Phe Pro Ser Leu Ile Thr Ile Phe Ser Ala Pro Asn Tyr
305                 310                 315                 320

Leu Asp Val Tyr Asn Asn Lys Ala Ala Val Leu Lys Tyr Glu Asn Asn
                325                 330                 335

Val Met Asn Ile Arg Gln Phe Asn Cys Ser Pro His Pro Tyr Trp Leu
            340                 345                 350

Pro Asn Phe Met Asp Val Phe Thr Trp Ser Leu Pro Phe Val Gly Glu
        355                 360                 365

Lys Val Thr Glu Met Leu Val Asn Val Leu Ser Ile Cys Ser Asp Asp
    370                 375                 380

Glu Leu Met Thr Glu Gly Glu Asp Gln Phe Asp Val Gly Ser Ala Ala
385                 390                 395                 400

Ala Arg Lys Glu Ile Ile Arg Asn Lys Ile Arg Ala Ile Gly Lys Met
                405                 410                 415

Ala Arg Val Phe Ser Val Leu Arg Glu Glu Ser Glu Ser Val Leu Thr
            420                 425                 430

Leu Lys Gly Leu Thr Pro Thr Gly Met Leu Pro Ser Gly Val Leu Ala
        435                 440                 445

Gly Gly Arg Gln Thr Leu Gln Ser Ala Thr Val Glu Ala Ile Glu Ala
    450                 455                 460

Glu Lys Ala Ile Arg Gly Ser Ser Pro Pro His Arg Ile Cys Ser Phe
465                 470                 475                 480

Glu Glu Ala Lys Gly Leu Asp Arg Ile Asn Glu Arg Met Pro Pro Arg
                485                 490                 495

Lys Asp Ala Val Gln Gln Asp Gly Phe Asn Ser Leu Asn Thr Ala His
            500                 505                 510

Thr Thr Glu Asn His Gly Thr Gly Asn His Ser Ala Gln
        515                 520                 525


<210> 18
<211> 2266
<212> DNA
<213> Rattus norvegicus

<220>
<223> Calcineurin A-beta

<400> 18
cccgcccggc ccggcaccta gccgagcccc gggcccagca tggccgcccc ggagccggcc 60
cgggccgcgc cgccccctcc cccgcccccg ccgccccccc tcggggccga ccgcgtcgtc 120
aaagccgttc cttttccccc aactcaccgg ttgacatctg aagaagtgtt tgatatggat 180
gggataccca gggttgatgt tctgaagaac catctggtaa aagaaggtcg ggtagatgaa 240
gaaattgcac taagaattat caatgagggt gctgccatac ttaggcggga gaaaaccatg 300
atagaagtag aagctccaat tacagtgtgt ggtgacatcc atggccaatt ttttgatctg 360
atgaaacttt ttgaagtagg aggatcacct gctaatacac gatacctttt tcttggtgat 420
tatgtggaca gaggttattt tagtatagag tgtgtcttgt atttatgggt cttgaagatt 480
ctatacccaa gcacattatt ccttctgaga ggcaaccatg aatgcagaca cctcactgag 540
tattttacct ttaagcagga atgtaaaatt aagtattcag aaagggtcta tgaagcttgt 600
atggaggctt ttgacagctt gccccttgct gcacttctaa accaacaatt tctttgtgtt 660
catggtggac tttcaccaga gatacacaca ctggatgata ttaggagatt agatagattt 720
aaagagccac ctgcatttgg accaatgtgt gacttgctgt ggtctgatcc ttctgaagac 780
tttggaaatg aaaaatcaca agaacatttt agtcataata cggttcgagg atgttcttat 840
ttttataact atccagcagt gtgtgaattt ttgcaaaaca ataatttgtt atcgattatt 900
agagctcatg aagctcaaga tgcaggctat agaatgtaca gaaaaagtca aactacaggg 960
tttccttcat taataacaat tttttcggca cctaattact tagatgtcta caataataaa 1020
gctgctgtac taaagtatga gaacaatgtg atgaacatcc gccagtttaa ctgttctcca 1080
catccttact ggttgcccaa ttttatggat gtctttacat ggtctttacc atttgttgga 1140
gaaaaagtca cagagatgtt ggtaaatgtt ctcagtattt gctctgatga tgaactaatg 1200
acagaagggg aagaccagtt tgatgtaggt tcagctgcag cccggaaaga aatcataaga 1260
aacaagatcc gagcaattgg caagatggca agagtcttct ctgttctcag ggaggagagt 1320
gaaagcgtgc tgacactcaa gggcctgact cccacaggga tgttgcctag tggagtgttg 1380
gctggaggac ggcagacctt gcaaagtgcc acagttgagg ctattgaggc tgaaaaagca 1440
atacgaggat cctctccacc acatagaatc tgcagttttg aagaggcaaa gggtttggat 1500
aggatcaatg agagaatgcc accccggaaa gatgctgtgc agcaagatgg tttcaattcc 1560
ctgaacaccg cacataccac tgagaaccac gggactggca accatagtgc ccagtgacca 1620
gcggcttccc agggatggac agatcactaa ggagccggag gggtcggccg agctgatgat 1680
aaatgtcata atctctctga agaaacaaac cattgtgctt tttgagaccc ttgccccctt 1740
cccggatgga ggcttgaggg ccttgggact tgtgctgtcc ataagattgg ggaatcgctg 1800
ccacagcgga gagcagtgag caaggggctt ggggcaaatt ccagtggagg aagcccaaac 1860
ctccatttat gcttgtggtt cacacattta cgtttacaaa tgagatttcc tttgttttct 1920
ccctcagtag aattagattt ttttcaacca tgactttaaa tgcaatcttt agagttaatg 1980
tggaacctcc ctcaccccca ccccatgaaa tgtctttaag aggatggatt agcatggtct 2040
taaaatatat ttctgaggtt actagatgta ttttgaattg tagacaaaat ctgagaaacc 2100
cagttggtgt ttatataaaa acgctgacct caggtcatag ttcttaaatg tggctaattc 2160
tgtaacatag tcttggtatt tttcaattat gaatgcataa actatttcta agaagactct 2220
tacttgaaca agatccaaaa aaccaattta gatccttttt gccccc 2266

<210> 19
<211> 564
<212> PRT
<213> Rattus norvegicus

<220>
<223> Phosphodiesterase, cAMP-specific

<400> 19
Met Lys Glu Gln Gly Gly Thr Val Ser Gly Ala Gly Ser Ser Arg Gly
1               5                   10                  15

Gly Gly Asp Ser Ala Met Ala Ser Leu Gln Pro Leu Gln Pro Asn Tyr
            20                  25                  30

Leu Ser Val Cys Leu Phe Ala Glu Glu Ser Tyr Gln Lys Leu Ala Met
        35                  40                  45

Glu Thr Leu Glu Glu Leu Asp Trp Cys Leu Asp Gln Leu Glu Thr Ile

50                  55                  60

Gln Thr Tyr Arg Ser Val Ser Glu Met Ala Ser Asn Lys Phe Lys Arg
65                  70                  75                  80

Met Leu Asn Arg Glu Leu Thr His Leu Ser Glu Met Ser Arg Ser Gly
                85                  90                  95

Asn Gln Val Ser Glu Tyr Ile Ser Asn Thr Phe Leu Asp Lys Gln Asn
            100                 105                 110

Asp Val Glu Ile Pro Ser Pro Thr Gln Lys Asp Arg Glu Lys Lys Lys
        115                 120                 125

Lys Gln Gln Leu Met Thr Gln Ile Ser Gly Val Lys Lys Leu Met His
    130                 135                 140

Ser Ser Ser Leu Asn Asn Thr Ser Ile Ser Arg Phe Gly Val Asn Thr
145                 150                 155                 160

Glu Asn Glu Asp His Leu Ala Lys Glu Leu Glu Asp Leu Asn Lys Trp
                165                 170                 175

Gly Leu Asn Ile Phe Asn Val Ala Gly Tyr Ser His Asn Arg Pro Leu
            180                 185                 190

Thr Cys Ile Met Tyr Ala Ile Phe Gln Glu Arg Asp Leu Leu Lys Thr
        195                 200                 205

Phe Lys Ile Ser Ser Asp Thr Phe Val Thr Tyr Met Met Thr Leu Glu
    210                 215                 220

Asp His Tyr His Ser Asp Val Ala Tyr His Asn Ser Leu His Ala Ala
225                 230                 235                 240

Asp Val Ala Gln Ser Thr His Val Leu Leu Ser Thr Pro Ala Leu Asp
                245                 250                 255

Ala Val Phe Thr Asp Leu Glu Ile Leu Ala Ala Ile Phe Ala Ala Ala
            260                 265                 270

Ile His Asp Val Asp His Pro Gly Val Ser Asn Gln Phe Leu Ile Asn
        275                 280                 285

Thr Asn Ser Glu Leu Ala Leu Met Tyr Asn Asp Glu Ser Val Leu Glu
    290                 295                 300

Asn His His Leu Ala Val Gly Phe Lys Leu Leu Gln Glu Glu His Cys
305                 310                 315                 320

Asp Ile Phe Gln Asn Leu Thr Lys Lys Gln Arg Gln Thr Leu Arg Lys
                325                 330                 335

Met Val Ile Asp Met Val Leu Ala Thr Asp Met Ser Lys His Met Ser
            340                 345                 350

Leu Leu Ala Asp Leu Lys Thr Met Val Glu Thr Lys Lys Val Thr Ser
        355                 360                 365

Ser Gly Val Leu Leu Leu Asp Asn Tyr Thr Asp Arg Ile Gln Val Leu
    370                 375                 380

Arg Asn Met Val His Cys Ala Asp Leu Ser Asn Pro Thr Lys Ser Leu
385                 390                 395                 400

Glu Leu Tyr Arg Gln Trp Thr Asp Arg Ile Met Glu Glu Phe Phe Gln
                405                 410                 415

Gln Gly Asp Lys Glu Arg Glu Arg Gly Met Glu Ile Ser Pro Met Cys
            420                 425                 430

Asp Lys His Thr Ala Ser Val Glu Lys Ser Gln Val Gly Phe Ile Asp
        435                 440                 445

Tyr Ile Val His Pro Leu Trp Glu Thr Trp Ala Asp Leu Val Gln Pro
    450                 455                 460

Asp Ala Gln Asp Ile Leu Asp Thr Leu Glu Asp Asn Arg Asn Trp Tyr
465                 470                 475                 480

Gln Ser Met Ile Pro Gln Ser Pro Ser Pro Pro Leu Asp Glu Arg Ser
                485                 490                 495

Arg Asp Cys Gln Gly Leu Met Glu Lys Phe Gln Phe Glu Leu Thr Leu
            500                 505                 510

Glu Glu Glu Asp Ser Glu Gly Pro Glu Lys Glu Gly Glu Gly Pro Asn
        515                 520                 525

Tyr Phe Ser Ser Thr Lys Thr Leu Cys Val Ile Asp Pro Glu Asn Arg
    530                 535                 540

Asp Ser Leu Glu Glu Thr Asp Ile Asp Ile Ala Thr Glu Asp Lys Ser
545                 550                 555                 560

Leu Ile Asp Thr

<210> 20
<211> 2647
<212> DNA
<213> Rattus norvegicus

<220>
<223> Phosphodiesterase, cAMP specific

<400> 20
   gtcttgtcat caggagacct cattttacct ctaggttaag ggagagaatc tatgaagaga 60
   aaggaatagt ctgtgtctcg gtcttgtccg ggtcagtgtt tctgagagct cacagtggcc 120
   acctgaagca tttttcccca gaatgaatga ctgccctgcc tgagaacaga agagccaaac 180
   agttcccccc acatggccat agggagctgg tttcatttag aagaaaagca aagagagggg 240
   aaagcctccc tcatttctcc tccggacggc aaacattcag aaatgacatc acacacccca 300
   cagccccggg atgactaagg cagaagtagc ctgagaaaac tctgctctgc cctgagtttt 360
   agggcacagt tatgcagatg agcgtctggg cgcaggttcc cgccttcttc ctctgaggaa 420
   gtttcttggt agatcactga cacctcatcc cggcgagggg gtgaaaactt ggcaccagcc 480
   actccccctc ccgggcagag caccagaaag agcttggaag caaggagtcg gcaagcaaac 540
   aatgaaggag caagggggca ccgtcagtgg cgccgggagc agccgaggcg gaggagactc 600
   ggctatggcc agcctgcagc cgctgcagcc taactacctg tctgtgtgtt tgtttgcaga 660
   agaatcatat cagaaactag caatggagac gctggaggaa ctagactggt gcctagacca 720
   gctagagacc atccagacct accgctctgt cagcgagatg gcttcaaaca agttcaaaag 780
   gatgctgaac cgggagctga cacacctctc agagatgagc agatcaggga accaagtgtc 840
   tgaatacatt tcgaacacgt tcttagacaa gcagaacgat gtggaaatcc catctcccac 900
   ccagaaggac agggagaaga agaagaagca gcagctcatg acccagataa gtggagtgaa 960
   gaaactgatg cacagctcaa gcctgaacaa cacaagcatc tcacgctttg gagtcaacac 1020
   ggaaaatgag gatcatctag ccaaggagct ggaagacctg aacaaatggg gccttaacat 1080
   cttcaacgtg gctgggtact cccataatcg gcccctcaca tgcatcatgt acgccatttt 1140
   ccaggaaaga gaccttctaa agacgtttaa aatctcctcc gacaccttcg taacctacat 1200
   gatgacttta gaagaccatt accattctga tgtggcgtat cacaacagcc tgcacgctgc 1260
   tgacgtggcc cagtcaacgc acgttctcct ctctacgcca gcactggatg ctgtcttcac 1320
   agacctggaa atcctggctg ccatttttgc agctgccatc catgatgttg atcatcctgg 1380
   agtctccaat cagtttctca tcaatacaaa ttccgaactt gctttgatgt ataatgacga 1440
   atctgtgctg gaaaaccatc acctcgctgt gggattcaag ctccttcaag aggaacattg 1500
   cgacatcttt cagaatctta ccaagaagca acgccagaca ctcaggaaaa tggtgattga 1560
   catggtgtta gcaactgata tgtccaagca catgagcctc ctggctgacc ttaaaacgat 1620
   ggtagaaacc aaaaaggtga cgagctccgg tgttctcctc ctggacaact atactgaccg 1680
   gatacaggtt cttcgcaaca tggtacattg tgcagacctg agcaacccta ccaagtcctt 1740
   ggagttgtat cggcaatgga ctgatcgcat catggaggag tttttccaac agggagacaa 1800
   agaacgggag aggggaatgg agattagccc aatgtgtgat aaacacacag cttctgtgga 1860
   aaagtcccag gttggtttca ttgactacat tgtccatcca ttgtgggaga cctgggcaga 1920
   cctggttcag cctgatgctc aagacatttt ggacacacta gaagataaca ggaactggta 1980
   ccagagtatg attccccaga gcccctctcc accactggac gagaggagca gggactgcca 2040
   aggccttatg gagaagtttc agttcgaact gacccttgaa gaagaggatt ctgaaggacc 2100
   ggaaaaggag ggagaaggcc ccaactattt cagcagcaca aagacacttt gtgtgatcga 2160
   tccagagaac agggattctc tggaagagac tgacatagac attgccacag aagacaagtc 2220
   tctgatcgac acataatctc cctctgtgtg gaggtgaaca ttctatcctt gacgagcatg 2280
   ccagctgagt ggtagggccc acctaccaga gccaaggcct gcacaaaaca aaggccacct 2340
   ggctttgcag ttacttgagt ttggagccag aatgcaaggc cgtgaagcaa atagcagttc 2400
   cgtgctgcct tgccttgccg gcgagcttgg cgagacccgc agctgtagta gaagccagtt 2460
   cccagcacag ctaaatggct tgaaaacaga ggacagaaag ctgagagatt gctctgcaat 2520
   aggtgttgag ggctgtcccc gacaggtgac tgaactcact aacaacttca tctataaatc 2580
   tcacccatcc tgttgtctgc caacctgtgt gccttttttg taaaatgttt tcgtgtcttt 2640
   gaaatgc 2647

<210> 21
<211> 416
<212> PRT
<213> Rattus norvegicus

<220>
<223> Neurotensin receptor type 2

<400> 21
Met Glu Thr Ser Ser Pro Trp Pro Pro Arg Pro Ser Pro Ser Ala Gly
1               5                   10                  15

Leu Ser Leu Glu Ala Arg Leu Gly Val Asp Thr Arg Leu Trp Ala Lys
            20                  25                  30

Val Leu Phe Thr Ala Leu Tyr Ser Leu Ile Phe Ala Phe Gly Thr Ala
        35                  40                  45

Gly Asn Ala Leu Ser Val His Val Val Leu Lys Ala Arg Ala Gly Arg
    50                  55                  60

Pro Gly Arg Leu Arg Tyr His Val Leu Ser Leu Ala Leu Ser Ala Leu
65                  70                  75                  80

Leu Leu Leu Leu Val Ser Met Pro Met Glu Leu Tyr Asn Phe Val Trp
                85                  90                  95

Ser His Tyr Pro Trp Val Phe Gly Asp Leu Gly Cys Arg Gly Tyr Tyr

            100                 105                 110

Phe Val Arg Glu Leu Cys Ala Tyr Ala Thr Val Leu Ser Val Ala Ser
        115                 120                 125

Leu Ser Ala Glu Arg Cys Leu Ala Val Cys Gln Pro Leu Arg Ala Arg
    130                 135                 140

Arg Leu Leu Thr Pro Arg Arg Thr Arg Arg Leu Leu Ser Leu Val Trp
145                 150                 155                 160

Val Ala Ser Leu Gly Leu Ala Leu Pro Met Ala Val Ile Met Gly Gln
                165                 170                 175

Lys His Glu Val Glu Ser Ala Asp Gly Glu Pro Glu Pro Ala Ser Arg
            180                 185                 190

Val Cys Thr Val Leu Val Ser Arg Ala Thr Leu Gln Val Phe Ile Gln
        195                 200                 205

Val Asn Val Leu Val Ser Phe Ala Leu Pro Leu Ala Leu Thr Ala Phe
    210                 215                 220

Leu Asn Gly Ile Thr Val Asn His Leu Met Ala Leu Tyr Ser Gln Val
225                 230                 235                 240

Pro Ser Ala Ser Ala Gln Val Ser Ser Ile Pro Ser Arg Leu Glu Leu
                245                 250                 255

Leu Ser Glu Glu Gly Leu Leu Gly Phe Ile Thr Trp Arg Lys Thr Leu
            260                 265                 270

Ser Leu Gly Val Gln Ala Ser Leu Val Arg His Lys Asp Ala Ser Gln
        275                 280                 285

Ile Arg Ser Leu Gln His Ser Ala Gln Val Leu Arg Ala Ile Val Ala
    290                 295                 300

Val Tyr Val Ile Cys Trp Leu Pro Tyr His Ala Arg Arg Leu Met Tyr
305                 310                 315                 320

Cys Tyr Ile Pro Asp Asp Gly Trp Thr Asn Glu Leu Tyr Asp Phe Tyr
                325                 330                 335

His Tyr Phe Tyr Met Val Thr Asn Thr Leu Phe Tyr Val Ser Ser Ala
            340                 345                 350

Val Thr Pro Ile Leu Tyr Asn Ala Val Ser Ser Ser Phe Arg Lys Leu
        355                 360                 365

Phe Leu Glu Ser Leu Gly Ser Leu Cys Gly Glu Gln His Ser Leu Val
    370                 375                 380

Pro Leu Pro Gln Glu Ala Pro Glu Ser Thr Thr Ser Thr Tyr Ser Phe
385                 390                 395                 400

Arg Leu Trp Gly Ser Pro Arg Asn Pro Ser Leu Gly Glu Ile Gln Val
                405                 410                 415

<210> 22
<211> 1529
<212> DNA
<213> Rattus norvegicus

<220>
<223> Neurotensin receptor type 2

<400> 22
tgagtgagag ggagggagct caggccacca cgagacagag atggagacca gcagtccgtg 60
gcctccgagg cccagcccca gcgcagggct gagcctggag gcgcggctgg gcgtggacac 120
tcgcctctgg gccaaggtgc tgttcaccgc gctctactcg ctcatcttcg catttggcac 180
agcgggcaat gcgctgtccg tgcacgtggt gctgaaggcg cgggccggtc gccccgggcg 240
cctgcgctac cacgtgctca gcctggcgct ctcagccctg ctgctactgc tggtcagcat 300
gcccatggag ctctacaact tcgtgtggtc ccactaccca tgggtcttcg gcgatctggg 360
ctgccgtggc tattacttcg tgcgcgagct gtgcgcctac gccacagtgc tgagcgttgc 420
cagcctaagc gcagagcgct gcctggccgt gtgccagccg ctgcgcgccc gccgccttct 480
caccccgcgc cgcacccgcc gcctgctgtc actggtctgg gtcgcctctc tgggccttgc 540
cctgcccatg gcggttatca tgggacagaa gcacgaagtg gaaagcgcgg acggggagcc 600
tgagcctgcc tcgcgtgtgt gcacggtgct ggtgagccgc gccacacttc aggtcttcat 660
ccaggtgaat gtgctggtgt ccttcgctct ccccttggca ctcactgctt tcctgaatgg 720
catcactgtc aaccacctga tggccctcta ctcccaggta ccatcagctt ctgcccaagt 780
cagctccatc cccagccgcc tggagctcct gagtgaggaa ggcctcctgg gcttcatcac 840
gtggagaaag actctctccc tgggggtcca agccagcctg gtgagacaca aggatgccag 900
ccagatccgc agcctccagc acagcgccca ggttctcaga gccatcgtgg ctgtgtatgt 960
catctgctgg ctgccgtacc atgcccgccg actcatgtac tgctacatcc ccgatgatgg 1020
atggactaat gagctctatg atttctatca ctatttctac atggtgacca acacgctctt 1080
ctatgtcagc tcagcagtga ccccaatcct ctacaacgcc gtgtcttcct ccttcagaaa 1140
gctcttcctg gaatccctcg gctccctgtg tggtgaacag cactccttgg tgcccttacc 1200
ccaagaagcc ccagagtcaa ccactagtac gtacagtttc cggctttggg gatccccaag 1260
aaaccccagc ctgggagaaa tacaagtatg aagagaacaa acaatggctg cttgggacat 1320
gcccgtcaga caagccatgc catcactaac agtccaggtg gacctactga cccagtgcat 1380
actgcaggca aaccacataa cacctactgc cctcagcttc ccacagagaa caacagagtt 1440
gaagaatagg aaccgtggcc tagtgatgaa ggtgcccagt gccaggcctg gtacatagtc 1500
actactcaat aaattttaac ccggtgctg 1529

<210> 23
<211> 570
<212> PRT
<213> Mus musculus

<220>
<223> Second subunit of the interleukin 1 receptor complex

<400> 23
Met Gly Leu Leu Trp Tyr Leu Met Ser Leu Ser Phe Tyr Gly Ile Leu
1               5                   10                  15

Gln Ser His Ala Ser Glu Arg Cys Asp Asp Trp Gly Leu Asp Thr Met
            20                  25                  30

Arg Gln Ile Gln Val Phe Glu Asp Glu Pro Ala Arg Ile Lys Cys Pro
        35                  40                  45

Leu Phe Glu His Phe Leu Lys Tyr Asn Tyr Ser Thr Ala His Ser Ser
    50                  55                  60

Gly Leu Thr Leu Ile Trp Tyr Trp Thr Arg Gln Asp Arg Asp Leu Glu

65                  70                  75                  80

Glu Pro Ile Asn Phe Arg Leu Pro Glu Asn Arg Ile Ser Lys Glu Lys
                85                  90                  95

Asp Val Leu Trp Phe Arg Pro Thr Leu Leu Asn Asp Thr Gly Asn Tyr
            100                 105                 110

Thr Cys Met Leu Arg Asn Thr Thr Tyr Cys Ser Lys Val Ala Phe Pro
        115                 120                 125

Leu Glu Val Val Gln Lys Asp Ser Cys Phe Asn Ser Ala Met Arg Phe
    130                 135                 140

Pro Val His Lys Met Tyr Ile Glu His Gly Ile His Lys Ile Thr Cys
145                 150                 155                 160

Pro Asn Val Asp Gly Tyr Phe Pro Ser Ser Val Lys Pro Ser Val Thr
                165                 170                 175

Trp Tyr Lys Gly Cys Thr Glu Ile Val Asp Phe His Asn Val Leu Pro
            180                 185                 190

Glu Gly Met Asn Leu Ser Phe Phe Ile Pro Leu Val Ser Asn Asn Gly
        195                 200                 205

Asn Tyr Thr Cys Val Val Thr Tyr Pro Glu Asn Gly Arg Leu Phe His
    210                 215                 220

Leu Thr Arg Thr Val Thr Val Lys Val Val Gly Ser Pro Lys Asp Ala
225                 230                 235                 240

Leu Pro Pro Gln Ile Tyr Ser Pro Asn Asp Arg Val Val Tyr Glu Lys
                245                 250                 255

Glu Pro Gly Glu Glu Leu Val Ile Pro Cys Lys Val Tyr Phe Ser Phe
            260                 265                 270

Ile Met Asp Ser His Asn Glu Val Trp Trp Thr Ile Asp Gly Lys Lys
        275                 280                 285

Pro Asp Asp Val Thr Val Asp Ile Thr Ile Asn Glu Ser Val Ser Tyr
    290                 295                 300

Ser Ser Thr Glu Asp Glu Thr Arg Thr Gln Ile Leu Ser Ile Lys Lys
305                 310                 315                 320

Val Thr Pro Glu Asp Leu Arg Arg Asn Tyr Val Cys His Ala Arg Asn
                325                 330                 335

Thr Lys Gly Glu Ala Glu Gln Ala Ala Lys Val Lys Gln Lys Val Ile
            340                 345                 350

Pro Pro Arg Tyr Thr Val Glu Leu Ala Cys Gly Phe Gly Ala Thr Val
        355                 360                 365

Phe Leu Val Val Val Leu Ile Val Val Tyr His Val Tyr Trp Leu Glu
    370                 375                 380

Met Val Leu Phe Tyr Arg Ala His Phe Gly Thr Asp Glu Thr Ile Leu
385                 390                 395                 400

Asp Gly Lys Glu Tyr Asp Ile Tyr Val Ser Tyr Ala Arg Asn Val Glu
                405                 410                 415

Glu Glu Glu Phe Val Leu Leu Thr Leu Arg Gly Val Leu Glu Asn Glu
            420                 425                 430

Phe Gly Tyr Lys Leu Cys Ile Phe Asp Arg Asp Ser Leu Pro Gly Gly
        435                 440                 445

Ile Val Thr Asp Glu Thr Leu Ser Phe Ile Gln Lys Ser Arg Arg Leu
    450                 455                 460

Leu Val Val Leu Ser Pro Asn Tyr Val Leu Gln Gly Thr Gln Ala Leu
465                 470                 475                 480

Leu Glu Leu Lys Ala Gly Leu Glu Asn Met Ala Ser Arg Gly Asn Ile
                485                 490                 495

Asn Val Ile Leu Val Gln Tyr Lys Ala Val Lys Asp Met Lys Val Lys
            500                 505                 510

Glu Leu Lys Arg Ala Lys Thr Val Leu Thr Val Ile Lys Trp Lys Gly
        515                 520                 525

Glu Lys Ser Lys Tyr Pro Gln Gly Arg Phe Trp Lys Gln Leu Gln Val
    530                 535                 540

Ala Met Pro Val Lys Lys Ser Pro Arg Trp Ser Ser Asn Asp Lys Gln
545                 550                 555                 560

Gly Leu Ser Tyr Ser Ser Leu Lys Asn Val
                565                 570


<210> 24
<211> 3355
<212> DNA
<213> Mus musculus

<220>
<223> Second subunit of the interleukin 1 receptor complex

<400> 24
cattgtgctc taaagctgcc agcatctggc tttcctaggt tcggcttgga ccattgtgct 60
gaaagaggca gtggtcggcc accctgcatc catctggtcg ctgtcgccga gcctgctggg 120
ctggcccgat aaggatggga cttctgtggt atttgatgag tctgtccttc tatgggatcc 180
tgcagagtca tgcttcggag cgctgtgatg actggggact agataccatg cgacaaatcc 240
aagtgtttga agatgagccg gctcgaatca agtgccccct ctttgaacac ttcctgaagt 300
acaactacag cactgcccat tcctctggcc ttaccctgat ctggtactgg accaggcaag 360
accgggacct ggaggagccc attaacttcc gcctcccaga gaatcgcatc agtaaggaga 420
aagatgtgct ctggttccgg cccaccctcc tcaatgacac gggcaattac acctgcatgt 480
tgaggaacac aacttactgc agcaaagttg catttcccct ggaagttgtt cagaaggaca 540
gctgtttcaa ttctgccatg agattcccag tgcacaagat gtatattgaa catggcattc 600
ataagatcac atgtccaaat gtagacggat actttccttc cagtgtcaaa ccatcggtca 660
cttggtataa gggttgtact gaaatagtgg actttcataa tgtactaccc gagggcatga 720
acttgagctt tttcatcccc ttggtttcaa ataacggaaa ttacacatgt gtggttacat 780
atcctgaaaa cggacgtctc tttcacctca ccaggactgt gactgtaaag gtggtgggct 840
caccaaagga tgcattgcca ccccagatct attctccaaa tgaccgtgtt gtctatgaga 900
aagaaccagg agaggaactg gttattccct gcaaagtcta tttcagtttc attatggact 960
cccacaatga ggtctggtgg accattgatg gaaagaagcc tgatgacgtc acagtcgaca 1020

tcactattaa tgaaagtgta agttattctt caacggaaga tgaaacaagg actcagattt 1080
tgagcatcaa gaaagtcacc ccggaggatc tcaggcgcaa ctatgtctgt catgctcgaa 1140
ataccaaagg ggaagctgag caggctgcca aggtgaaaca gaaagtcata ccaccaaggt 1200
acacagtaga actcgcctgt ggttttggag ccacggtctt tctggtagtg gttctcattg 1260
tggtttacca tgtttactgg ctggagatgg tcctctttta ccgagctcac tttggaacag 1320
atgaaacaat tcttgatgga aaggagtatg atatttatgt ttcctatgca agaaatgtgg 1380
aagaagagga atttgtgctg ctgacgctgc gtggagtttt ggagaatgag tttggataca 1440
agctgtgcat cttcgacaga gacagcctgc ctgggggaat tgtcacagat gagaccctga 1500
gcttcattca gaaaagcaga cgactcctgg ttgtcctaag tcccaactac gtgctccagg 1560
gaacacaagc cctcctggag ctcaaggctg gcctagaaaa tatggcctcc cggggcaaca 1620
tcaacgtcat tttagtgcag tacaaagctg tgaaggacat gaaggtgaaa gagctgaagc 1680
gggctaagac ggtgctcacg gtcattaaat ggaaaggaga gaaatccaag tatcctcagg 1740
gcaggttctg gaagcagttg caggtggcca tgccagtgaa gaagagtccc aggtggtcta 1800
gcaatgacaa gcagggtctc tcctactcat ccctgaaaaa cgtatgaaag gagaagtgag 1860
ggggtacaaa gaacaaggcg gttcatggga ggaagggccc cctctttcct tctaggctgt 1920
ggcttcatag acagaaaaag agtcctgtct aagccccctg ggaggaaaat attccgggac 1980
tgtacgcctg gcagctctgc ctctcagcaa tactaagcgt tacagtgatg gactccacag 2040
tccgccacca gttctaaaag tcattttgat ctttacaagt aaagatgtgt ctgatgcacc 2100
cccacctctg ttcttccctc ttttctaatc tcttcgtaaa aatctcacct ctatggaact 2160
gctttgccca gtgactcaaa actctgttta aagatacttt acctgctgac aggagtatcg 2220
cctgtttctc agtaggacac tcctttaatt ttctcttctc ttccgtcatc cattaagcat 2280
gtaatcaggg actaaatctg tgtgagctga ggctatttca ctcctttgct tctgtgtctg 2340
ttgaatctac cacctaagca agactaacag tcaaactgca gctgttcctc gtgactgatt 2400
aagcgacata ctgccttcct gctactaaac cccattcgct ctcatctcag gtaatggaga 2460
tcatatttat aaacttgaaa aagccatggc ttttgtttta tcatagtaat ttctcatctg 2520
tctacagata gacaggtcgg tacttgttcc ttcctttccc tacctattgg ttctgtcact 2580
catatctcat aggcagcatc acgcttgctg cggtctgcgg ggaagcttta atggtgctaa 2640
tggagagtga gaaaatccaa aagcgctctc agggcatttt tcacatggga agagagcaca 2700
gaagagaatg cttggcggtt tgtttgttaa ttctgaccct agaggggaaa accaggaaaa 2760
gtggttaatc ctcactcctt cacacacctc ttcattatga agtggaggtg agaggtactg 2820
agtttccttt tggcattact attccaaaag gaatgattat tcatggtaat taattgtgca 2880
gtgcctgtag taccttgctc caaatggatc accctgcaaa tcattaataa tgaagtggtt 2940
ttattagttt caagcagaat ttcactattt ctcagacttc caaaagtgaa aggcaagtat 3000
aacattcaca tttagagagg ccaatttcta tttcatatct aacatgtata tatatgtata 3060
tacatatata catatatata tgatctctgt acacataaat tttatatgtc tatgtatata 3120
gaagtatcca caaatgacat gtgtgtgtgt atatccacct gcctataaat tatctctgtg 3180
tattattttg ggctatacat aatcacacac atacatcttt cagtagtcat tggcaaaata 3240
ttgaaagcca gtagaactag tgttcaatct ttaaattatg ttaatagtgt tatggttttg 3300
tgttgatatt ttcatttcat ttcatttctg ttccatttgt gaattccctt tagag 3355

<210> 25
<211> 504
<212> PRT
<213> Rattus norvegicus

<220>
<223> Amino acid transporter system A

<400> 25
Met Lys Lys Thr Glu Met Gly Arg Phe Asn Ile Ser Pro Asp Glu Asp
1               5                   10                  15

Ser Ser Ser Tyr Ser Ser Asn Gly Asp Phe Asn Tyr Ser Tyr Pro Thr
            20                  25                  30

Lys Gln Ala Ala Leu Lys Ser His Tyr Val Asp Val Asp Pro Glu Asn
        35                  40                  45

Gln Asn Phe Leu Leu Glu Ser Asn Leu Gly Lys Lys Lys Tyr Glu Thr
    50                  55                  60

Asp Phe His Pro Ser Thr Thr Ser Phe Gly Met Ser Val Phe Asn Leu
65                  70                  75                  80

Thr Asn Ala Ile Val Gly Asn Gly Ile Leu Gly Leu Ser Tyr Ala Met
                85                  90                  95

Ala Asn Thr Gly Ile Ala Leu Phe Ile Ile Leu Leu Thr Phe Val Ser
            100                 105                 110

Ile Phe Ser Leu Tyr Ser Val His Leu Leu Leu Lys Thr Ala Asn Glu
        115                 120                 125

Gly Gly Ser Leu Leu Tyr Glu Gln Leu Gly His Lys Ala Tyr Gly Leu
    130                 135                 140

Ala Gly Lys Leu Ala Ala Ser Gly Ser Ile Thr Met Gln Asn Ile Gly
145                 150                 155                 160

Ala Met Ser Ser Tyr Leu Phe Ile Val Lys Tyr Glu Leu Pro Leu Val
                165                 170                 175

Ile Lys Ala Leu Met Asn Ile Glu Asp Thr Asn Gly Leu Trp Tyr Leu
            180                 185                 190

Asn Gly Asp Tyr Leu Val Leu Leu Val Ser Phe Val Leu Ile Leu Pro
        195                 200                 205

Leu Ser Leu Leu Arg Asn Leu Gly Tyr Leu Gly Tyr Thr Ser Gly Leu
    210                 215                 220

Ser Leu Leu Cys Met Ile Phe Phe Leu Ile Val Val Ile Cys Lys Lys
225                 230                 235                 240

Phe Gln Ile Pro Cys Pro Val Glu Val Ala Leu Met Ala Asn Glu Thr
                245                 250                 255

Val Asn Gly Thr Phe Thr Gln Val Ala Leu Ala Ala Leu Ala Ser Asn
            260                 265                 270

Ser Thr Ala Ala Asp Thr Cys Arg Pro Arg Tyr Phe Ile Phe Asn Ser
        275                 280                 285

Gln Thr Val Tyr Ala Val Pro Ile Leu Thr Phe Ser Phe Val Cys His
    290                 295                 300

Pro Ala Val Leu Pro Ile Tyr Glu Glu Leu Lys Ser Arg Ser Arg Arg
305                 310                 315                 320

Arg Met Met Asn Val Ser Lys Ile Ser Phe Phe Ala Met Phe Leu Met
                325                 330                 335

Tyr Leu Leu Ala Ala Leu Phe Gly Tyr Leu Thr Phe Tyr Glu His Val
            340                 345                 350

Glu Ser Glu Leu Leu His Thr Tyr Ser Ala Ile Val Gly Thr Asp Ile
        355                 360                 365

Leu Leu Leu Val Val Arg Leu Ala Val Leu Val Ala Val Thr Leu Thr
    370                 375                 380

Val Pro Val Val Ile Phe Pro Ile Arg Ser Ser Val Thr His Leu Leu
385                 390                 395                 400

Cys Pro Thr Lys Glu Phe Ser Trp Phe Arg His Ser Val Ile Thr Val
                405                 410                 415

Thr Ile Leu Ala Phe Thr Asn Leu Leu Val Ile Phe Val Pro Thr Ile
            420                 425                 430

Arg Asp Ile Phe Gly Phe Ile Gly Ala Ser Ala Ala Ala Met Leu Ile
        435                 440                 445

Phe Ile Leu Pro Ser Ala Phe Tyr Ile Lys Leu Val Lys Lys Glu Pro
    450                 455                 460

Met Arg Ser Val Gln Lys Ile Gly Ala Leu Cys Phe Leu Leu Ser Gly
465                 470                 475                 480

Val Val Val Met Ile Gly Ser Met Gly Leu Ile Val Leu Asp Trp Val
                485                 490                 495

His Asp Ala Ser Ala Gly Gly His
            500


<210> 26
<211> 4632
<212> DNA
<213> Rattus norvegicus

<220>
<223> Amino acid transporter system A

<400> 26
ccacgcgtcc gcgccgtcgc ccgcgccacc accccttagt ctctgctgga ggatcgtgac 60
cgccattccc cgcttttttt tttcttttta taatctcttg aataattaaa tctaagaaag 120
aaaaaaaatc catctcttgt gcctgttgga ttgcggggat tttctttttt ttttaattta 180
aaaaagaaag gaaaaaaagg ggggggggac tccgaccaca cttatttttg cctcgtttta 240
caattctggt gtgaccgaga cagcccgcgg gagacgctgc cgtgaggtgg gccagctcgc 300
aggaagagca ccgaggcagc ctcgagggcc agtgcgccgc caaggacaaa aggaagctcg 360
cgccgcgcgc tgaacaccgt tcctccggat cagcatgaag aagaccgaaa tgggaaggtt 420
caacatctcc ccggatgagg acagcagcag ttacagctcc aacggtgact tcaactactc 480
ataccccacg aagcaggctg ctctaaaaag ccattatgtg gacgtggacc ccgaaaacca 540
gaacttttta cttgaatcga atctggggaa gaagaagtac gaaacagact ttcatccaag 600
tactacttcc tttggaatgt cagtatttaa tctgaccaat gcgattgtgg gcaatggaat 660
ccttgggctt tcttatgcca tggctaatac tggaattgct ctttttataa ttctcttgac 720
gttcgtgtcg atttttctcc tgtactccgt tcacctcctc ctcaagactg ccaacgaagg 780
agggtcttta ttatacgaac agttgggaca taaggcatac ggtctggctg ggaagctggc 840
agcctccgga tcgattacaa tgcagaacat cggagctatg tcaagctacc tcttcatagt 900
gaaatatgag ttacctttgg tgatcaaggc gttaatgaac attgaagata cgaatgggct 960
gtggtatctg aacggcgact atctggtcct tctggtgtcc ttcgtgctca ttctcccatt 1020
gtcactgctg agaaatctag gatacttggg gtataccagc ggcctttctc tgctctgtat 1080
gatattcttt ctgattgtgg tgatttgcaa gaagtttcag attccttgcc ctgtggaagt 1140
ggctttgatg gctaacgaaa cggtgaatgg cacctttacc caggtggctt tggcagcgct 1200
ggcgtccaac agcaccgcag ccgatacctg caggccccgt tactttatct tcaactcaca 1260
gactgtgtat gctgtgccca tcctgacgtt ttcctttgtc tgccatcccg ctgtccttcc 1320
catctatgaa gagctgaaga gccgcagccg gagaaggatg atgaacgtgt ccaagatttc 1380
tttcttcgcc atgtttctga tgtatttgct tgccgccctc ttcggatacc tgacatttta 1440
cgagcacgtg gagtcagaac tgctacatac ctactctgcg atcgtgggaa ctgacattct 1500
cctcctcgtt gtccgcctgg ctgtgctggt ggctgtcacc ctcactgtcc ccgtcgtcat 1560
tttcccaatc cggagttcgg tcactcactt actgtgccct acaaaagagt tcagttggtt 1620

ccggcacagt gtcattactg tgacgatcct ggccttcacc aatttgctcg tcatctttgt 1680
ccccactatc agggacatct tcgggttcat tggtgcatcg gccgctgcaa tgctgatctt 1740
catccttccg tctgctttct acatcaagtt agtgaagaaa gaacccatga gatccgtgca 1800
aaagattggg gctctgtgtt ttctcctgag tggcgtagtc gtgatgattg gaagcatggg 1860
cttgattgtc ctggactggg tccacgatgc ctctgcaggc ggacattaac cgccccactc 1920
aaactgaacg gtccatctgc ctgccgcgtt gggtccactc aactcgctca gacttcaccc 1980
gatgctttcg ggtcacttcc ttctgaagtg cagcctcctc gctggttctt ctggatgcag 2040
aataagtgaa cttttctttc tctttggttt tttttttaaa agaagcttat ctgtgtgtta 2100
gggaatggat attaacacca aaaaccacga gccttgggtt aagggaagtg acagtttatt 2160
ccattccaga gaatggacgg acttttatca aagccacatg tctggctgtc tcattgttta 2220
acttggatat tttatgattt tacttgaatg tgcctaacgg aaccattcga tgtgagaaac 2280
acttcttaat ttacagcaaa gtattgaaat aagcattgag aaaaacactt attttttgta 2340
ccaaaaaaat actttaaaag acctcagaag cactatttta ctttgaagaa attgcatttt 2400
ttgaacttta tccagaacca gttgtcaata aactccgtaa aacaatttca ttgatattta 2460
aaaatgaata ttagtattat gaaattattt gcctttttta taggcattat aagccaaata 2520
cttttttacc ccaaaatcat tttcgggaaa atgttacgca aagttgcacc atgagttagc 2580
atggttttgt tttgctcatt tcagattcat ttgaaggata attggtaatt gctgcaccaa 2640
atccggtgag cctccctccg ttcatcgctg tctttgcctt cagataattg gttcattaaa 2700
actaaaatgt ttgtgtcttc aattaggctg ggaaacactg taggaaatga aataactgat 2760
tttgaggtac caaatagtgc aattgggtaa aacagggttt attcagttgc gtctgtcccc 2820
agagttctac tgtcaactct gggtcgtgct tttgggccca tcctttaaaa catcgcttcc 2880
aaaagtgaaa atgggcgttt gatggcccgc aggccaaagc tgtccaatag aaagcagtag 2940
aactttcaaa cgctcgccta ctggaagcgt gatttacttg acaacacgcg gctcagttgt 3000
gttcctgctg tgtctacagt agaggtctag acggcaggct gcaccgtgct gtacactgca 3060
agctctcttc agaaaggtca ctgggtttct catgcagtaa gctttctaag aactcgcact 3120
ggatcgtcat ctcattgtac gatgcagaat tgcttgttta catggaacga gtgatcagtt 3180
agggaagcag tacgagcgat tgtacccagt tctggtccag ctgcatgctt agctttccac 3240
ttcagctcca gttacaagtg gaagcatttc tttgaggctt ggctttaaga gcacatttac 3300
tgtatgttaa cggtgtatgg tgaatatatt attaaataat gtggtacttg cccatcaggc 3360
ataatgtcta agatctaata tacataattc cattaagcgg tggagggaag caagtggaat 3420
cgtcatctgg ctcctgaggt ttttctcttg aactaaactt ttttttttgg ttcgaggcaa 3480
gggccagaac aaatgtgaca cacatggttt ttgttacagt cttgtattgt atatgtgact 3540
aaattcatag gcaagatgtg tctgctacta aagacccttg cagacgggaa gacgtctggt 3600
agtgctacgt tgggtacaac caatggtcag ttctgtcccg aagatgaaat tgctggggat 3660
catgtagacg caaaggcagt gagaagaaga cactccaccc tgctgctttt cagtccttca 3720
agtccagaac ctcaccttta gtcaaaagac ttggcacttc tgctcgaagt cctaaaaaga 3780
gtcgccgcta aggtctgcca ggggagctga ggaacaggca tccaagaaaa ggtgccatgg 3840
caaccaggat cctcctcaca gccgatcagt gtggagagag cacaactgaa ggttcacaat 3900
gaatttcagg ccaaattcca acgcttgggg ctctgtcagc cttgaggttc ctaaaccgag 3960
ttctcagtct tgagcagtct tcagtattaa aaccactgtc ccgtcacctc ggtttgcatt 4020
actgtcttcc acggaacgtt tcaataactg tactgttatt tatagagcag cattaatcca 4080
ttaaagctaa cctatttttc aatatttatg gtaaactgta catacattgt ccatatgtat 4140
ttgtaaataa gctgtatata ctgttggaat tgggtcttgg gttcaagtgt atatattccc 4200
cctaaatttt cttaactgca ttttgatgaa ttcacattat atataggtcc ccggattact 4260
tgtacagagg attcaatatt gacattgtgt ataatactaa aattgaaaat aacttgttta 4320
aattgtattt gcaataaact ggttggtcat caacacaagt tcagatatca aactaaaagc 4380
tttaaatgag gtgtctaaat tttcaaaatg cagtaggtac aacacggcta tctggtaact 4440
tgtcatgtga tttcaatgga agtcacgtgg actccttgta tgtatatagt gtaaatgccc 4500
ccgctgtact gtagaggctg acaggccact gtggcttgtt ggcagggagt gctatgccag 4560
tttcaatgaa acaatgtatg tttgttttaa ctgaactaaa ataaacactt aatcctgaaa 4620
aaaaaaaaaa aa 4632

<210> 27
<211> 661
<212> PRT
<213> Rattus norvegicus

<220>
<223> Brain digoxin carrier protein

<400> 27
Met Gly Lys Ser Glu Lys Arg Val Ala Thr His Gly Val Arg Cys Phe
1               5                   10                  15

Ala Lys Ile Lys Met Phe Leu Leu Ala Leu Thr Cys Ala Tyr Val Ser
            20                  25                  30

Lys Ser Leu Ser Gly Thr Tyr Met Asn Ser Met Leu Thr Gln Ile Glu
        35                  40                  45

Arg Gln Phe Gly Ile Pro Thr Ser Ile Val Gly Leu Ile Asn Gly Ser
    50                  55                  60

Phe Glu Ile Gly Asn Leu Leu Leu Ile Ile Phe Val Ser Tyr Phe Gly
65                  70                  75                  80

Thr Lys Leu His Arg Pro Ile Met Ile Gly Val Gly Cys Ala Val Met
                85                  90                  95

Gly Leu Gly Cys Phe Leu Ile Ser Leu Pro His Phe Leu Met Gly Gln
            100                 105                 110

Tyr Glu Tyr Glu Thr Ile Leu Pro Thr Ser Asn Val Ser Ser Asn Ser
        115                 120                 125

Phe Phe Cys Val Glu Asn Arg Ser Gln Thr Leu Asn Pro Thr Gln Asp
    130                 135                 140

Pro Ser Glu Cys Val Lys Glu Met Lys Ser Leu Met Trp Ile Tyr Val
145                 150                 155                 160

Leu Val Gly Asn Ile Ile Arg Gly Ile Gly Glu Thr Pro Ile Met Pro
                165                 170                 175

Leu Gly Ile Ser Tyr Ile Glu Asp Phe Ala Lys Ser Glu Asn Ser Pro
            180                 185                 190

Leu Tyr Ile Gly Ile Leu Glu Thr Gly Met Thr Ile Gly Pro Leu Ile
        195                 200                 205

Gly Leu Leu Leu Ala Ser Ser Cys Ala Asn Ile Tyr Val Asp Ile Glu
    210                 215                 220

Ser Val Asn Thr Asp Asp Leu Thr Ile Thr Pro Thr Asp Thr Arg Trp
225                 230                 235                 240

Val Gly Ala Trp Trp Ile Gly Phe Leu Val Cys Ala Gly Val Asn Ile
                245                 250                 255

Leu Thr Ser Phe Pro Phe Phe Phe Phe Pro Lys Thr Leu Pro Lys Glu
            260                 265                 270

Gly Leu Gln Glu Asn Val Asp Gly Thr Glu Asn Ala Lys Glu Lys Lys
        275                 280                 285

His Arg Lys Lys Ala Lys Glu Glu Lys Arg Gly Ile Thr Lys Asp Phe
    290                 295                 300

Phe Val Phe Met Lys Ser Leu Ser Cys Asn Pro Ile Tyr Met Leu Phe
305                 310                 315                 320

Ile Leu Ile Ser Val Leu Gln Phe Asn Ala Phe Ile Asn Ser Phe Thr
                325                 330                 335

Phe Met Pro Lys Tyr Leu Glu Gln Gln Tyr Gly Lys Ser Thr Ala Glu
            340                 345                 350

Val Val Phe Leu Met Gly Leu Tyr Met Leu Pro Pro Ile Cys Leu Gly
        355                 360                 365

Tyr Leu Ile Gly Gly Leu Ile Met Lys Lys Phe Lys Val Thr Val Lys
    370                 375                 380

Lys Ala Ala His Leu Ala Phe Trp Leu Cys Leu Ser Glu Tyr Leu Leu
385                 390                 395                 400

Ser Phe Leu Ser Tyr Val Met Thr Cys Asp Asn Phe Pro Val Ala Gly
                405                 410                 415

Leu Thr Thr Ser Tyr Glu Gly Val Gln His Gln Leu Tyr Val Glu Asn
            420                 425                 430

Lys Val Leu Ala Asp Cys Asn Thr Arg Cys Asn Cys Ser Thr Asn Thr
        435                 440                 445

Trp Asp Pro Val Cys Gly Asp Asn Gly Leu Ala Tyr Met Ser Ala Cys
    450                 455                 460

Leu Ala Gly Cys Glu Lys Ser Val Gly Thr Gly Thr Asn Met Val Phe
465                 470                 475                 480

Gln Asn Cys Ser Cys Ile Gln Ser Ser Gly Asn Ser Ser Ala Val Leu
                485                 490                 495

Gly Leu Cys Asn Lys Gly Pro Asp Cys Ala Asn Lys Leu Gln Tyr Phe
            500                 505                 510

Leu Ile Ile Ala Ile Phe Gly Cys Phe Ile Tyr Ser Leu Ala Gly Ile
        515                 520                 525

Pro Gly Tyr Met Val Leu Leu Arg Cys Ile Lys Ser Glu Glu Lys Ser
    530                 535                 540

Leu Gly Val Gly Leu His Ala Phe Cys Ile Arg Ile Leu Ala Gly Ile
545                 550                 555                 560

Pro Ala Pro Ile Tyr Phe Gly Ala Leu Ile Asp Arg Thr Cys Leu His
                565                 570                 575

Trp Gly Thr Leu Lys Cys Gly Glu Pro Gly Ala Cys Arg Met Tyr Asp
            580                 585                 590

Ile Asn Ser Phe Arg Arg Leu Tyr Leu Gly Leu Pro Ala Ala Leu Arg
        595                 600                 605

Gly Ala Ser Phe Val Pro Ala Phe Phe Ile Leu Arg Leu Thr Arg Thr
    610                 615                 620

Phe Gln Phe Pro Gly Asp Ile Glu Ser Ser Lys Thr Asp His Ala Glu
625                 630                 635                 640

Met Lys Leu Thr Leu Lys Glu Ser Glu Cys Thr Glu Val Leu Arg Ser

                645                 650                 655

Lys Val Thr Glu Asp
            660

<210> 28
<211> 3622
<212> DNA
<213> Rattus norvegicus

<220>
<223> Brain digoxin carrier protein

<400> 28
gctgctctga ctttctttta gtctcagcat ggagaggacc gtcttctaaa gcttcttcat 60
aaaaacagca gtaagattat ttaaagaata gatatctgga aacaatcaga agaacaacat 120
gggaaaatct gagaaaaggg ttgcaaccca tggggtcaga tgttttgcca agatcaagat 180
gtttctgttg gcattaacat gtgcatatgt atccaaatca ttatcaggaa cttatatgaa 240
ttccatgctc acacaaatag agagacaatt cggtatcccc acatctatag ttgggcttat 300
caatgggagc tttgaaatag gaaacctttt gttgattata tttgtgagtt attttggaac 360
aaaacttcac agacctatca tgattggtgt tggatgtgca gttatgggcc tggggtgttt 420
cttaatctcg ctaccccatt tcctcatggg ccaatatgaa tatgaaacga ttttacctac 480
aagcaacgtg tcctcaaaca gcttcttctg tgtggaaaac agatcccaga ccttaaatcc 540
aacacaagac ccctcagagt gtgtgaaaga aatgaaatca ttaatgtgga tatatgtact 600
ggtaggaaac ataatacgtg gaattggtga aactcccatc atgcccttgg gtatttccta 660
cattgaagac tttgccaaat ctgaaaactc tcctttatac attgggattt tagaaacagg 720
aatgaccatt ggccctttga ttggacttct gttggcttct tcctgtgcaa acatttatgt 780
agacattgag tctgtgaata cagatgacct gaccataact cccacagata cacgctgggt 840
cggagcttgg tggatcggct ttttggtctg tgcaggagtg aatatcctga ccagctttcc 900
ctttttcttt tttcccaaaa cacttccaaa ggaaggatta caggagaatg tggatggaac 960
tgaaaatgcc aaagagaaga aacacagaaa aaaggccaag gaagaaaaac gaggaatcac 1020
taaagatttc tttgtgttca tgaagagcct ctcctgcaat ccaatttaca tgcttttcat 1080
ccttataagt gttctccagt tcaatgcatt tatcaattca tttaccttca tgcctaagta 1140
tctggaacag caatatggaa aatccactgc tgaggtagtc ttccttatgg gtctttatat 1200
gttacctcca atatgcctcg gatatttaat tggtggtttg attatgaaga agttcaaggt 1260
tactgtcaag aaagctgcac acttagcatt ctggctctgc ctgtctgagt accttctgtc 1320
tttccttagc tatgtgatga cctgtgataa ttttccagtg gcaggcttaa caacctctta 1380
tgaaggggtt cagcaccaac tatatgtgga gaacaaggtc cttgctgact gtaacacaag 1440
gtgtaactgc tcaacgaaca catgggatcc agtgtgtgga gacaatggcc tggcatacat 1500
gtcagcctgc cttgcaggct gtgagaagtc tgttggaaca ggaaccaaca tggtgtttca 1560
gaattgcagc tgcattcagt catcgggaaa ctcatctgca gtcctgggcc tgtgtaacaa 1620
aggccctgac tgtgccaaca agctgcagta cttcttaatc atagcaatat ttggctgttt 1680
catatactcg ctggcaggca ttccagggta tatggttctt ctgaggtgta tcaagtctga 1740
agagaagtca cttggagttg ggttacatgc attttgcata agaatattag ctggcattcc 1800
tgcacccatt tactttggag ctttgataga cagaacctgt ttacattggg gaaccctgaa 1860
atgtggtgag cccggggcat gcaggatgta tgacataaac agcttcagac gtctttacct 1920
tggattgccg gctgcactaa gaggagcaag ctttgtcccc gccttcttca ttctaagact 1980
tacgaggaca ttccagttcc ctggggacat tgagtcttca aaaactgatc atgcggagat 2040
gaagctcacc ttgaaggaaa gtgagtgcac agaagtccta aggtcgaaag tgacggagga 2100
ctgaaaacga agctgtaatg agttttctac tgccctatgc aaggccatga agagaatgta 2160
cacttcacta gttttgaatc atgagagata caattggaac tcttaggtta tccataaggc 2220
cgtcaaagtt acttcattca tgataaaatt atttactgat agcattttca gaaggctgac 2280
atagtactca agattttccc agggaaaact tctatagtgg ccttcaccct taaccttaaa 2340
gctgccttca ttttcaacca gcatgttctc ttttaactca atcaagggaa gtggatgttt 2400
cccacacatt ctcaaatatc tttgaaactt tcctattgca gaaatatcat ttagatgttt 2460
ttaatttata tactgatgct ggagatcaaa atatacatct tggttaagcc agattgcgtt 2520
agtttgtttt gatttatcct ctgcatgtgc aaaacttctg catctgtctt gtgtacttag 2580
gagtggtaac tctcttttac ttctaagatt agactcttca gagtgtgcca tctcctgttt 2640
tcagtccctt ctatcattac ttctgtcaca cagttgatca tttcacatac atcactgaaa 2700
actttaatca ggttgttaac cagtcatgta gcaaagatga ttgggactct ttttctctaa 2760

caattcaaag ctggtcatga aactcttttt taaaaatcaa gagtagggga aaactagtcc 2820
tttcaaaggc tccttgtaga gatgggctgt atctcagtgg aatagttatt acctaatgta 2880
tgtgaggccc caggttcaac cacaacgtag ggtaaaccaa taaagtaata aaaataacgt 2940
aagtccagat gcatcatcag atattctaaa aggctattct catattcagg gggcttcaat 3000
ggcttagtgt tcattctatt caagggccat ggagcacata gttattaaca ttcataataa 3060
acttagagta aaacctttaa agagggacca gatagaaagt tcgatagaaa gaactgtttg 3120
ccaccgaacc tgaaaaggtt gttgtgatcc ttgggaccaa cgtgaaggag agaacaaact 3180
ctcacaagtt gtgctatatc tctttttaat tgtgcatgcc ccattgcaaa tcaattaata 3240
aaaaaagcat taaaaggttt aagaccgaca tttgctgtaa aattatagct cataaacgtg 3300
aaagtacaca tcaaaaataa aatcaagttg tggttgtttt aatgagaaat atccctccta 3360
ggcataggca tttggatatt tggtttctaa tgaatgactc tgcttaggga agattggatg 3420
tgcatcccta agacaaaagg tgaatcactg agatgggttg aaagttaaaa gcctcaccta 3480
cttccagtac actctctgct ttgtgctttg gttgatgata tgaaatcatg gtttcctgct 3540
ccagccacca tgcctggtgc ttgccttcat gaacttccat ccctggagtc atgcgttaaa 3600
ataaactcct ttttttaatg tg 3622

<210> 29
<211> 3663
<212> DNA
<213> Rattus norvegicus

<220>
<223> Synaptic vesicle transmembrane transporter (SV2)

<400> 29
ttgtgctcta aagcaactca ccctactgaa ctgggtgcag agcaaagccc ttttcgccct 60
tttccccatc tggacttctc tggccagttc ctcttagtcc gatcccaaag acactggaac 120
acatttctaa agggtcttct tgatccctcc aattcattga gcaaagggct gaaaaagaag 180
cagagagtaa ggtagagcca gtgactcgcc cccaagcccc atcatggaag aaggctttcg 240
agaccgagca gcgttcatcc gtggggccaa agacattgcc aaggaagtta agaagcacgc 300
ggccaagaag gtggtgaagg gtctcgacag agtccaggat gagtattccc gaaggtccta 360
ctcccgcttt gaggaggagg aggatgatga tgacttccct gcccctgctg acggctatta 420
ccgcggagaa ggggcccagg atgaggagga aggtggcgct tccagtgatg ccactgaggg 480
ccacgatgag gatgatgaga tctacgaggg agaatatcag ggcatccccc gggcagagtc 540
tgggggcaaa ggcgaacgga tggcagatgg ggcacccctg gctggagtga gaggggcctt 600
aagtgatggg gagggtcccc ctgggggtcg cggggaggcg cagcggcgta aagatcggga 660
agaattggct cagcagtatg agaccatcct ccgggagtgc ggccatggtc gcttccagtg 720
gacactctac ttcgtgctgg gtctggcgct gatggccgat ggtgtagagg tctttgtggt 780
gggctttgtg ctgcccagtg ctgagaaaga tatgtgcctg tcggactcca acaaaggcat 840
gctaggcctc attgtgtacc tgggcatgat ggtgggggcc ttcctctggg gaggcctggc 900
tgatcggctg ggtcggagac agtgtctgct catctcgctc tcagtcaaca gcgtcttcgc 960
tttcttctca tccttcgtcc agggttatgg caccttcctt ttctgccgcc tcctttctgg 1020
ggttgggatt ggtggttcca tccccattgt cttctcctat ttttcggagt ttctggccca 1080
ggagaaacgt ggggagcatt tgagctggct ctgtatgttc tggatgattg gtggcgtgta 1140
tgcagctgca atggcctggg ccatcatccc ccactatggg tggagtttcc agatgggctc 1200
tgcttaccag ttccacagct ggagggtctt tgtcctcgtc tgtgcctttc cctctgtgtt 1260
tgccatcggg gctctgacta cgcagccgga gagtccccgc ttcttcttag agaatgggaa 1320
gcacgatgag gcctggatgg tgctgaagca ggttcatgac accaacatgc gagccaaggg 1380
ccatcctgag cgagtcttct cagtaaccca cattaaaacg attcatcagg aggatgaatt 1440
gattgagatc cagtcagaca caggaacctg gtaccagcgc tggggagtgc gggctttgag 1500
cctggggggt caggtttggg ggaacttcct ctcctgcttc agtccagagt accggcgcat 1560
cactctgatg atgatggggg tatggttcac catgtccttc agctactacg gtttgactgt 1620
ctggtttccc gacatgatcc gccatctcca ggctgtggac tatgcagccc gaaccaaagt 1680
gttcccaggg gagcgcgtgg agcacgtgac atttaacttc acactggaga atcagatcca 1740
ccgaggggga cagtacttca atgacaagtt catcgggctg cgtctgaagt cagtgtcctt 1800
tgaggattcc ctgtttgagg aatgttactt tgaagatgtc acatccagca acacattctt 1860
ccgcaactgc acattcatca acaccgtgtt ctacaacacg gacctgtttg agtacaagtt 1920
cgtgaacagc cgcctggtga acagcacatt cctgcacaat aaggaaggtt gcccactaga 1980
tgtgacaggg acgggcgaag gtgcctacat ggtgtacttt gtcagcttct tggggacact 2040
ggctgtgctc cctggaaata ttgtgtctgc tctgctcatg gacaagattg gcaggctcag 2100

aatgcttgct ggttccagtg tgttgtcctg tgtttcctgc ttcttcctgt cttttgggaa 2160
cagtgagtca gccatgatcg ctctgctctg cctttttggg ggagtcagta ttgcatcctg 2220
gaacgcgctg gacgtgctga ctgttgaact ctacccttcc gacaagagga cgacggcctt 2280
cggcttcctg aatgccctgt gtaagctggc agctgtactg ggcatcagca tcttcacgtc 2340
ctttgtggga atcaccaagg ccgctcccat cctcttcgcc tcagctgcgc ttgcccttgg 2400
tagctctctg gctctgaagc tgcctgagac ccggggacag gtgctgcagt gagggatggg 2460
ggagtgtctc aggggcttta gggatggcag gcacactgtg accaataatt tcttttatcc 2520
ctaccctgcc ctgctgtcct ggtcctactc cgtgtttggt gtcttagctg tgtgcctgtg 2580
tgcatgtgtg tgaccctgac gggcaggggc tacggggagg gtccctttgt cccatgtttg 2640
ggaggaggga ctccccacct gctgccaccc tcaactttgc acaaggagaa ggctgagctg 2700
catccttctc tccctcagtg ttagcagggg tggggacgac tgtttctctg ctccaggtgt 2760
tccagaattt ctgcctttcc catcattccc tccgcctagg ccctggtgaa ccacaggtat 2820
ggagttatag tgggggctga ggcttggacc aaaagaactt cttgagtggg agcctcccaa 2880
ggatgctggg gagtagcaat aaaccttagc ctccgttttc acctcaattc aggctacaag 2940
tgtgaagcct ggattttatg gaattagttt tctgattctt atttatatgt aagttctgag 3000
gcagcttagc tggactgtgt gtggatgtat acatacactc atatgtgtgt gtgtgtgcgc 3060
gcgtgcgtgt gcgtgtgtgt atgtgtgcca tggggtaggg gtaccactat actgttcaat 3120
tataagccaa gagtagtagt ttcagtgagc acacacacaa cactgttttt ctatcgtaac 3180
tcccagaatc ttgtacctgt gttggggctg caggcagaag tccttggtca ggctggggtg 3240
agccctgcaa tcttggggga cctgagggca cctgacaagg actttctcct tcctctagag 3300
aggtcctacc cactagccac agccctccca tctgacctgt ccacacaggc agtgtatcag 3360
aggaaagaaa gggaaaataa ccaggcacac atggtcaaac cagcaggtct gaaagcacaa 3420
gaagctgggg caggggcagg aagcaggggt ccatccccta acccttctca aaaaggctgg 3480
gtcgtgaggg acccctaatg cagggacctg aagcctcagt ttccccatct tgcccttcca 3540
cagaacagcc tctgtaggta gagctgcccc ccgtcctacc ctactcttgt ggctgctttc 3600
tttggtactc ttcccactcc caccgtagct gtgacgtgtt gtagttttta gctgtttctt 3660
tag 3663

<210> 30
<211> 633
<212> PRT
<213> Rattus norvegicus

<220>
<223> Glycine transporter

<400> 30
Met Val Gly Lys Gly Ala Lys Gly Met Leu Asn Gly Ala Val Pro Ser
1               5                   10                  15

Glu Ala Thr Lys Lys Asp Gln Asn Leu Thr Arg Gly Asn Trp Gly Asn
            20                  25                  30

Gln Ile Glu Phe Val Leu Thr Ser Val Gly Tyr Ala Val Gly Leu Gly
        35                  40                  45

Asn Val Trp Arg Phe Pro Tyr Leu Cys Tyr Arg Asn Gly Gly Gly Ala
    50                  55                  60

Phe Met Phe Pro Tyr Phe Ile Met Leu Val Phe Cys Gly Ile Pro Leu
65                  70                  75                  80

Phe Phe Met Glu Leu Ser Phe Gly Gln Phe Ala Ser Gln Gly Cys Leu
                85                  90                  95

Gly Val Trp Arg Ile Ser Pro Met Phe Lys Gly Val Gly Tyr Gly Met
            100                 105                 110

Met Val Val Ser Thr Tyr Ile Gly Ile Tyr Tyr Asn Val Val Ile Cys
        115                 120                 125

Ile Ala Phe Tyr Tyr Phe Phe Ser Ser Met Thr His Val Leu Pro Trp
    130                 135                 140

Ala Tyr Cys Asn Asn Pro Trp Asn Thr Pro Asp Cys Ala Gly Val Leu
145                 150                 155                 160

Asp Ala Ser Asn Leu Thr Asn Gly Ser Arg Pro Thr Ala Leu Ser Gly
                165                 170                 175

Asn Leu Ser His Leu Phe Asn Tyr Thr Leu Gln Arg Thr Ser Pro Ser
            180                 185                 190

Glu Glu Tyr Trp Arg Leu Tyr Val Leu Lys Leu Ser Asp Asp Ile Gly
        195                 200                 205

Asp Phe Gly Glu Val Arg Leu Pro Leu Leu Gly Cys Leu Gly Val Ser
    210                 215                 220

Trp Val Val Val Phe Leu Cys Leu Ile Arg Gly Val Lys Ser Ser Gly
225                 230                 235                 240

Lys Val Val Tyr Phe Thr Ala Thr Phe Pro Tyr Val Val Leu Thr Ile
                245                 250                 255

Leu Phe Val Arg Gly Val Thr Leu Glu Gly Ala Phe Thr Gly Ile Met
            260                 265                 270

Tyr Tyr Leu Thr Pro Lys Trp Asp Lys Ile Leu Glu Ala Lys Val Trp
        275                 280                 285

Gly Asp Ala Ala Ser Gln Ile Phe Tyr Ser Leu Gly Cys Ala Trp Gly
    290                 295                 300

Gly Leu Ile Thr Met Ala Ser Tyr Asn Lys Phe His Asn Asn Cys Tyr
305                 310                 315                 320

Arg Asp Ser Val Ile Ile Ser Ile Thr Asn Cys Ala Thr Ser Val Tyr
                325                 330                 335

Ala Gly Phe Val Ile Phe Ser Ile Leu Gly Phe Met Ala Asn His Leu
            340                 345                 350

Gly Val Asp Val Ser Arg Val Ala Asp His Gly Pro Gly Leu Ala Phe
        355                 360                 365

Val Ala Tyr Pro Glu Ala Leu Thr Leu Leu Pro Ile Ser Pro Leu Trp
    370                 375                 380

Ser Leu Leu Phe Phe Phe Met Leu Ile Leu Leu Gly Leu Gly Thr Gln
385                 390                 395                 400

Phe Cys Leu Leu Glu Thr Leu Val Thr Ala Ile Val Asp Glu Val Gly
                405                 410                 415

Asn Glu Trp Ile Leu Gln Lys Lys Thr Tyr Val Thr Leu Gly Val Ala
            420                 425                 430

Val Ala Gly Phe Leu Leu Gly Ile Pro Leu Thr Ser Gln Ala Gly Ile
        435                 440                 445

Tyr Trp Leu Leu Leu Met Asp Asn Tyr Ala Ala Ser Phe Ser Leu Val
    450                 455                 460

Val Ile Ser Cys Ile Met Cys Val Ser Ile Met Tyr Ile Tyr Gly His
465                 470                 475                 480

Arg Asn Tyr Phe Gln Asp Ile Gln Met Met Leu Gly Phe Pro Pro Pro
                485                 490                 495

Leu Phe Phe Gln Ile Cys Trp Arg Phe Val Ser Pro Thr Ile Ile Phe
            500                 505                 510

Phe Ile Leu Ile Phe Thr Val Ile Gln Tyr Arg Pro Ile Thr Tyr Asn
        515                 520                 525

His Tyr Gln Tyr Pro Gly Trp Ala Val Ala Ile Gly Phe Leu Met Ala
    530                 535                 540

Leu Ser Ser Val Ile Cys Ile Pro Leu Tyr Ala Leu Phe Gln Leu Cys
545                 550                 555                 560

Arg Thr Asp Gly Asp Thr Leu Leu Gln Arg Leu Lys Asn Ala Thr Lys
                565                 570                 575

Pro Ser Arg Asp Trp Gly Pro Ala Leu Leu Glu His Arg Thr Gly Arg
            580                 585                 590

Tyr Ala Pro Thr Thr Thr Pro Ser Pro Glu Asp Gly Phe Glu Val Gln
        595                 600                 605

Pro Leu His Pro Asp Lys Ala Gln Ile Pro Ile Val Gly Ser Asn Gly
    610                 615                 620

Ser Ser Arg Leu Gln Asp Ser Arg Ile
625                 630


<210> 31
<211> 2558
<212> DNA
<213> Rattus norvegicus

<220>
<223> Glycine transporter

<400> 31
ctcttgaggc ctcttgtctg agaggcactg aacgcaggag tctgcaggtg tggtccagat 60
ctccagatcc cccaacccac tgccaccatg gtaggaaaag gtgccaaagg gatgctgaat 120
ggtgctgtgc ccagcgaggc caccaagaag gaccagaacc tcacacgggg caactggggc 180
aaccagatcg agtttgtact gacgagcgtg ggctatgccg tgggcctggg caatgtgtgg 240
cgtttcccat acctctgcta tcgcaacggg ggaggcgcct tcatgtttcc ctacttcatc 300
atgctggtct tctgcggcat tcctctcttc ttcatggagc tctccttcgg ccagtttgca 360
agccagggct gcctgggggt ctggaggatc agccccatgt tcaaaggcgt gggctatggt 420
atgatggtgg tgtccacgta catcggtatc tactacaacg tggtcatctg catcgccttc 480
tactacttct tctcgtccat gacgcatgtg ctgccctggg cttactgcaa taatccctgg 540
aacacacctg actgtgccgg tgtgctggat gcttccaatc tcaccaatgg ctcccggccc 600
actgccctgt ctggcaacct gtctcacctg ttcaactaca ccttgcaaag gaccagcccc 660
agtgaggagt actggaggct gtatgtgctg aagctgtcgg atgacattgg agattttgga 720
gaagtgcggc ttcctctcct aggctgcctt ggcgtctcct gggtggttgt cttcctctgc 780
ctcattcgag gagtcaagtc ttcagggaaa gtggtgtact tcacggccac atttccctat 840
gtggtgctga ccattctgtt tgttcgtgga gtgaccctgg aaggagcctt cacgggtatc 900

atgtactacc tgaccccaaa gtgggacaag atcctggagg ccaaggtgtg gggggatgca 960
gcctctcaga tcttctattc cctgggctgt gcatggggtg gcctcatcac catggcatcc 1020
tacaacaaat tccacaacaa ctgctaccgg gacagcgtca tcatcagcat caccaattgt 1080
gctaccagtg tctatgctgg cttcgtcatc ttctctatcc taggcttcat ggccaatcac 1140
ctgggtgtgg atgtgtctcg ggtggcagac cacgggcccg ggctagcttt cgtggcttac 1200
cccgaggctc tcacactgct tcccatctcc ccgctctggt ccttgctgtt tttcttcatg 1260
ctcatcctgc tgggactcgg tactcagttc tgcctcctgg agaccctagt cactgccatt 1320
gtggatgagg tggggaatga gtggattctg cagaagaaga cctacgtgac cttgggtgtg 1380
gctgtggctg gcttcttgct gggtatccct cttaccagcc aggcgggcat ctactggctg 1440
ctgttgatgg acaactacgc agccagcttc tccttggttg tcatctcctg catcatgtgc 1500
gtgtccatca tgtatatcta tgggcaccgg aactacttcc aggacattca gatgatgctg 1560
gggttcccac cgcctctctt cttccagatc tgttggcgtt ttgtctctcc cactatcatc 1620
tttttcattc tcatcttcac ggtgatccag taccggccaa tcacttacaa ccactaccag 1680
tacccaggct gggctgtggc catcggcttc ctcatggctt tgtcgtctgt catctgcatc 1740
ccattgtacg cattgttcca gctctgccgc acagatgggg acacacttct tcagcgtttg 1800
aaaaatgcca caaagccaag cagagactgg ggccctgccc tcctggagca ccggactggg 1860
cgctatgccc ccactacaac cccctctcct gaagatgggt ttgaggttca gccactgcac 1920
ccggacaagg cccagatccc catcgtgggc agtaacggct ccagccgcct ccaggactcc 1980
cggatatgag cacagttgtt gcaaggggag aagccccacc caacccttgc tcctaccaca 2040
gagactgagg aggtggtgga ccggtgtgac tgcctgcccc atcatgccct ggccagggtg 2100
gctgctgtca ccttggccac cactgctcat gtccccagtg tttacacatc ctttggatgc 2160
caagacagca gctgggggtg gaaaagtgat gggggtgggg gggaggtgcg tgggggtggg 2220
gcatggggcg gcagtgtcca aagcactttg gaggaggtct caggccatgt ccccagaggc 2280
ttccaggctt cattatccct catgctctgc cctgagctgt ttcactacgg gataagaggt 2340
tttgtttcca caccccacct gttcccctcc agcctcatga ccagtgttcc tgaccccaga 2400
acagacccta gcctcttgcc caggcaaatc tgtatcttcc tgctccggac aaggtgaggc 2460
gcccgggggc tgtacagtgt tacttgttgg actgcaccac tcagccctgt ttatttgtgt 2520
aattattttt ttaaaaaatt gtattacctg tggttgcc 2558

<210> 32
<211> 140
<212> PRT
<213> Homo sapiens

<220>
<223> SEDL

<400> 32

<210> 33
<211> 2319
<212> DNA
<213> Homo sapiens

<220>
<223> SEDL

<400> 33
   cagaaactta agatttgtca gcttttttgc ttcactaata ctttcttctt tcctagtttt 60
   caatgaatcc attttatgaa cccaattctc ctattcgatc aagtgcattt gacagaaaag 120
   ttcagtttct tgggaagaaa caccttttaa gctgaatgca gaaaattcca aaataaatga 180
   tgtcaccaca atggtgtata ctcaggaatg tgtacattgt aagttacttg attaaatagc 240
   ctggaaaact tttgtgtatt ctcagcttat ctaaacctaa tgaaattcct tttatattta 300
   aaaatagtac attctgtctc atgtcacgta tcaatagatc aattggtatt tccttgtgaa 360
   cagtgttatt tataaagagt tcattatcaa taatcatgtt tttttttttt ttttttttct 420
   gatacagagt ctcactctgt tgccaggctg gagtgcagtg gcgcaatctt ggctcactgc 480
   aacctccgcc tcccaggttc aggcgattct cctgcctcag cctccaagta gctgggacta 540
   caggcgcgtg ccaccacgcc cggctaattt ttgtattttt agtagagaca gggtttcacc 600
   atattggtca ggctggtctt gaactcctga cctcgtgatc tgcccgcctt ggcctcccaa 660
   agtactggga ttacaggcgt gagccaccgt gcccaaccat gaaatatttt tacttaaaaa 720
   ttgggaataa gctcgctttt tttttttttg agatggagtc ttgctcctgt tgtgcaggct 780
   gtagtgcagt ggcacgatct tggctcactg caacctccac ctcccgggtt caagcaattc 840
   tccttcttca gcctcccgag tagctgagat tataggcgtg caccaccaca cctggctaat 900
   ttttgtattt ttagtagaga cagggtttca ccatattggt caggctggtc ttgaactcct 960
   gacctcgtga tccacccacc tcaggaagtg ctgggattac aggcgtgtga gccaccacgc 1020
   ccggccatga aatattttta cttaaaaatt gggaataagc tttttggttt tttgtgggtt 1080
   tttgtttttg tttttgtttt tttgtttttt tgagatggag tcttgctcct gttgtgcagg 1140
   ctggagtgca gtggcacagt cttggctcac tgcaacctcc acctcctggg ttcaagcaat 1200
   tctccttctt cagcctcccg agtagctggg attacaggca tgcaccacca cacctggcta 1260
   atttttgtgt ttttagtaga gacggggttt cgctattttg gccgggctgg tttcaaactc 1320
   ctgacctcag ttgatccacc cgcctcagcc tcccaaagtg ctaggattac aggcatgaac 1380
   caccgtgccc ggccaggaat aagcttttga cttacccaat tcagataggt tgatctttgg 1440
   ggtttttttt ttttttttga gatggagttt cgctctgttg tccaggctgg agtgcaatgg 1500
   cagttgtttc accgtaacct ctgcctcctg acttcaagca attctcctgc ctcagcctcc 1560
   caaagtgctg ggattacagg tgtgagccac cgtgcccggc ccagataggt tgatcttaat 1620
   gtaacaaccc aaaaataaat gtcatagtca agatttggta gataaattta aaattaaaat 1680
   attctgcagt tgggagtgaa atgtgatagc acatacgttg acattatgca tttagagatg 1740
   ttataaaaat gtataggcag tatacacagc actactcaag aagccaaaga aacacttgtg 1800
   cagtgctaag tgtcacatgt ctgcttccac cagaggctag gaatagtgat cctgctataa 1860
   tgtgagaacc caaatcatgt ttataaaata ggacgctggg agcagttgca ggccacagtg 1920
   aagtgtgttg cttctgtcac tttatattct tatatttcct ttccctcaga cagtaaactg 1980
   ttgatactcg tacttgtaaa aaattgtaag gcaattttta gtattgttgt caaagaaaag 2040
   cttggattac attaacattt gtattcagtc ttttaggtga tacaccagag ggggctggga 2100
   attgtctctt tgttcctata agtagatctt aatgtaaaat agtaaatgtc cattgaaaag 2160
   caagtaatac agcctgtgct aattgggagc acttgaacaa ttttgttcca ttctgagtaa 2220
   cttttgagca agtaattcta agctttgtct tgtatccttg tgtgaaattg cataattttt 2280
   accctatttg atttcttaaa taaagatgtt tgctcaaga 2319

<210> 34
<211> 587
<212> PRT
<213> Rattus norvegicus

<220>
<223> Erythroid-specific delta-aminolevulinate synthase

<400> 34
Met Val Ala Ala Ala Met Leu Leu Arg Ser Cys Pro Val Leu Ser Lys
1               5                   10                  15

Gly Pro Thr Gly Leu Leu Gly Lys Val Ala Lys Thr Tyr Gln Phe Leu
            20                  25                  30

Phe Gly Ile Gly Arg Cys Pro Ile Leu Ala Thr Gln Gly Pro Thr Cys
        35                  40                  45

Ser Gln Ile His Leu Lys Ala Thr Lys Ala Gly Ala Asp Ser Pro Ser
    50                  55                  60

Trp Thr Lys Ser His Cys Pro Phe Met Leu Ser Glu Leu Gln Asp Arg
65                  70                  75                  80

Lys Ser Lys Ile Val Gln Arg Ala Ala Pro Glu Val Gln Glu Asp Val
                85                  90                  95

Lys Thr Phe Lys Thr Asp Leu Leu Ser Phe Met Glu Ser Thr Thr Arg
            100                 105                 110

Ser Gln Ser Val Pro Arg Phe Gln Asp Pro Glu Gln Thr Gly Gly Ala
        115                 120                 125

Pro Pro Leu Leu Ile Gln Asn Asn Met Thr Gly Ser Gln Ala Phe Gly
    130                 135                 140

Tyr Asp Gln Phe Phe Arg Asp Lys Ile Met Glu Lys Lys Gln Asp His
145                 150                 155                 160

Thr Tyr Arg Val Phe Lys Thr Val Asn Arg Trp Ala Asn Ala Tyr Pro
                165                 170                 175

Phe Ala Gln His Phe Ser Glu Ala Ser Met Asp Ser Lys Asp Val Ser
            180                 185                 190

Val Trp Cys Ser Asn Asp Tyr Leu Gly Ile Ser Arg His Pro Arg Val
        195                 200                 205

Leu Gln Ala Ile Glu Glu Thr Leu Lys Asn His Gly Ala Gly Ala Gly
    210                 215                 220

Gly Thr Arg Asn Ile Ser Gly Thr Ser Lys Phe His Val Glu Leu Glu
225                 230                 235                 240

Gln Glu Leu Ala Glu Leu His His Lys Asp Ser Ala Leu Leu Phe Ser
                245                 250                 255

Ser Cys Phe Val Ala Asn Asp Ser Thr Leu Phe Thr Leu Ala Lys Leu
            260                 265                 270

Leu Pro Gly Cys Glu Ile Tyr Ser Asp Ala Gly Asn His Ala Ser Met
        275                 280                 285

Ile Gln Gly Ile Arg Asn Ser Gly Ala Val Lys Phe Val Phe Arg His
    290                 295                 300

Asn Asp Pro Gly His Leu Lys Lys Leu Leu Glu Lys Ser Asp Pro Lys
305                 310                 315                 320

Thr Pro Lys Ile Val Ala Phe Glu Thr Val His Ser Met Asp Gly Ala
                325                 330                 335

Ile Cys Pro Leu Glu Glu Leu Cys Asp Val Ala His Gln Tyr Gly Ala
            340                 345                 350

Leu Thr Phe Val Asp Glu Val His Ala Val Gly Leu Tyr Gly Thr Arg
        355                 360                 365

Gly Ala Gly Ile Gly Glu Arg Asp Gly Ile Met His Lys Leu Asp Ile
    370                 375                 380

Ile Ser Gly Thr Leu Gly Lys Ala Phe Gly Cys Val Gly Gly Tyr Ile
385                 390                 395                 400

Ala Ser Thr Arg Asp Leu Val Asp Met Val Arg Ser Tyr Ala Ala Gly
                405                 410                 415

Phe Ile Phe Thr Thr Ser Leu Pro Pro Met Val Leu Ser Gly Ala Leu
            420                 425                 430

Glu Ser Val Arg Leu Leu Lys Gly Glu Glu Gly Gln Ala Leu Arg Arg
        435                 440                 445

Ala His Gln Arg Asn Val Lys His Met Arg Gln Leu Leu Met Asp Arg
    450                 455                 460

Gly Phe Pro Val Ile Pro Cys Pro Ser His Ile Ile Pro Ile Arg Val
465                 470                 475                 480

Gly Asn Ala Ala Leu Asn Ser Lys Ile Cys Asp Leu Leu Leu Ala Lys
                485                 490                 495

His Ser Ile Tyr Val Gln Ala Ile Asn Tyr Pro Thr Val Pro Arg Gly
            500                 505                 510

Glu Glu Leu Leu Arg Leu Ala Pro Ser Pro His His Ser Pro Gln Met
        515                 520                 525

Met Glu Asn Phe Val Glu Lys Leu Leu Leu Ala Trp Thr Glu Val Gly
    530                 535                 540

Leu Pro Leu Gln Asp Val Ser Val Ala Ala Cys Asn Phe Cys Arg Arg
545                 550                 555                 560

Pro Val His Phe Glu Leu Met Ser Glu Trp Glu Arg Ser Tyr Phe Gly
                565                 570                 575

Asn Met Gly Pro Gln Tyr Val Thr Thr Tyr Ala
            580                 585


<210> 35
<211> 1899
<212> DNA
<213> Rattus norvegicus

<220>

<223> Erythroid-specific delta-aminolevulinate synthase

<400> 35
gactttgggc tcaggatggt ggcagcagct atgttgctac ggtcctgtcc agtgctctct 60
aagggcccca caggcctcct aggcaaagtg gctaaaactt accagttcct atttggtatt 120
ggacgctgcc ccatcctggc cactcaagga ccaacctgtt ctcaaatcca tcttaaggca 180
accaaggctg gagcagactc tccgtcttgg actaagagcc attgtccttt catgctgtca 240
gaactccaag acaggaagag caagattgtg cagagggcag ctccagaagt ccaagaggat 300
gtcaagactt tcaagacaga cctgctgagc ttcatggagt caactacccg aagccaatca 360
gtgcctcgtt tccaggatcc agagcagact ggaggggcac ctcccctgct gattcagaat 420
aatatgactg gaagccaggc ttttggttat gaccaatttt ttagagacaa gatcatggaa 480
aagaagcagg accacacata ccgtgtgttc aagactgtga atcgctgggc taatgcctac 540
ccctttgccc aacacttctc cgaggcatct atggactcaa aggatgtttc tgtctggtgc 600
agtaatgact atttgggcat aagcaggcac cctcgtgtct tgcaggccat agaggagacc 660
ctgaagaatc atggagctgg agctgggggc actcgcaata tctcaggtac aagcaagttt 720
catgtggagc ttgaacagga gctggctgaa ctacatcata aggactcggc tctgctcttc 780
tcctcctgtt ttgtggccaa tgattctact ctctttacac tggccaagct tctgccaggg 840
tgtgagatct actcagacgc aggcaatcat gcctccatga tccaaggcat tcgtaacagt 900
ggtgcagtca agtttgtctt cagacacaat gacccaggcc acctgaagaa acttcttgag 960
aagtccgatc ccaagacacc aaaaattgtg gcttttgaga ctgttcactc catggatggt 1020
gccatctgtc ctctggagga attgtgtgat gtggcccacc agtacggggc cctgaccttc 1080
gtggatgaag tccatgccgt aggactatat ggaacccggg gtgcagggat cggggagcgt 1140
gatggaatta tgcacaagct tgacatcatc tctggaactc ttggcaaggc ctttggctgt 1200
gtcggtggct atatcgccag cactcgggac ttggtggaca tggtgcgctc ctacgctgca 1260
ggcttcatct ttaccacttc actgcctccc atggtgctct ctggagctct agagtctgtg 1320
cgtctactca agggagagga gggtcaagcc ctgaggcgtg cacaccagcg caacgtcaaa 1380
catatgcgcc agctactaat ggacaggggc tttcctgtta tcccctgtcc cagccacatc 1440
atccccataa gggtgggtaa tgcggcactc aacagcaaga tctgtgatct tctgctcgcc 1500
aagcacagca tctatgtgca ggccatcaac tacccaactg tgcctcgtgg tgaggagctg 1560
ctgcgcttgg ccccctcccc ccaccacagc cctcagatga tggaaaactt tgtggagaag 1620
ttgctgctgg cctggactga ggtggggttg cccctccaag atgtgtctgt ggctgcatgc 1680
aacttctgcc gtcgtcctgt gcactttgag cttatgagcg agtgggagcg atcctacttt 1740
gggaacatgg gaccccaata tgttactacc tatgcctaag gagccagctg ccttagatgc 1800
caaccccacc tgcactcccc ctggggatgg tcttcctcct actctctgct ttgctgtgtg 1860
cttctagctg acttgattct aaaaataaag agcaacctg 1899

<210> 36
<211> 559
<212> PRT
<213> Rattus norvegicus

<220>
<223> Polypeptide GalNac transferase

<400> 36
Met Arg Lys Phe Ala Tyr Cys Lys Val Val Leu Ala Thr Ser Leu Val
1               5                   10                  15

Trp Val Leu Leu Asp Met Phe Leu Leu Leu Tyr Phe Ser Glu Cys Asn
            20                  25                  30

Lys Cys Glu Glu Lys Lys Glu Arg Gly Leu Pro Ala Gly Asp Val Leu
        35                  40                  45

Glu Leu Val Gln Lys Pro His Glu Gly Pro Gly Glu Met Gly Lys Pro
    50                  55                  60

Val Val Ile Pro Lys Glu Asp Gln Glu Lys Met Lys Glu Met Phe Lys
65                  70                  75                  80

Ile Asn Gln Phe Asn Leu Met Ala Ser Glu Met Ile Ala Phe Asn Arg
                85                  90                  95

Ser Leu Pro Asp Val Arg Leu Glu Gly Cys Lys Thr Lys Val Tyr Pro
            100                 105                 110

Asp Ser Leu Pro Thr Thr Ser Val Val Ile Val Phe His Asn Glu Ala
        115                 120                 125

Trp Ser Thr Leu Leu Arg Thr Val His Ser Val Ile Asn Arg Ser Pro
    130                 135                 140

Arg His Met Ile Glu Glu Ile Val Leu Val Asp Asp Ala Ser Glu Arg
145                 150                 155                 160

Asp Phe Leu Lys Arg Pro Leu Glu Ser Tyr Val Lys Lys Leu Lys Val
                165                 170                 175

Pro Val His Val Ile Arg Met Glu Gln Arg Ser Gly Leu Ile Arg Ala
            180                 185                 190

Arg Leu Lys Gly Ala Ala Val Ser Lys Gly Gln Val Ile Thr Phe Leu
        195                 200                 205

Asp Ala His Cys Glu Cys Thr Val Gly Trp Leu Glu Pro Leu Leu Ala
    210                 215                 220

Arg Ile Lys His Asp Arg Arg Thr Val Val Cys Pro Ile Ile Asp Val
225                 230                 235                 240

Ile Ser Asp Asp Thr Phe Glu Tyr Met Ala Gly Ser Asp Met Thr Tyr
                245                 250                 255

Gly Gly Phe Asn Trp Lys Leu Asn Phe Arg Trp Tyr Pro Val Pro Gln
            260                 265                 270

Arg Glu Met Asp Arg Arg Lys Gly Asp Arg Thr Leu Pro Val Arg Thr
        275                 280                 285

Pro Thr Met Ala Gly Gly Leu Phe Ser Ile Asp Arg Asp Tyr Phe Gln
    290                 295                 300

Glu Ile Gly Thr Tyr Asp Ala Gly Met Asp Ile Trp Gly Gly Glu Asn
305                 310                 315                 320

Leu Glu Ile Ser Phe Arg Ile Trp Gln Cys Gly Gly Thr Leu Glu Ile
                325                 330                 335

Val Thr Cys Ser His Val Gly His Val Phe Arg Lys Ala Thr Pro Tyr
            340                 345                 350

Thr Phe Pro Gly Gly Thr Gly Gln Ile Ile Asn Lys Asn Asn Arg Arg
        355                 360                 365

Leu Ala Glu Val Trp Met Asp Glu Phe Lys Asn Phe Phe Tyr Ile Ile
    370                 375                 380

Ser Pro Gly Val Thr Lys Val Asp Tyr Gly Asp Ile Ser Ser Arg Val
385                 390                 395                 400

Gly Leu Arg His Lys Leu Gln Cys Lys Pro Phe Ser Trp Tyr Leu Glu

405                 410                 415

Asn Ile Tyr Pro Asp Ser Gln Ile Pro Arg His Tyr Phe Ser Leu Gly
            420                 425                 430

Glu Ile Arg Asn Val Glu Thr Asn Gln Cys Leu Asp Asn Met Ala Arg
        435                 440                 445

Lys Glu Asn Glu Lys Val Gly Ile Phe Asn Cys His Gly Met Gly Gly
    450                 455                 460

Asn Gln Val Phe Ser Tyr Thr Ala Asn Lys Glu Ile Arg Thr Asp Asp
465                 470                 475                 480

Leu Cys Leu Asp Val Ser Lys Leu Asn Gly Pro Val Thr Met Leu Lys
                485                 490                 495

Cys His His Leu Lys Gly Asn Gln Leu Trp Glu Tyr Asp Pro Val Lys
            500                 505                 510

Leu Thr Leu Gln His Val Asn Ser Asn Gln Cys Leu Asp Lys Ala Thr
        515                 520                 525

Glu Glu Asp Ser Gln Val Pro Ser Ile Arg Asp Cys Thr Gly Ser Arg
    530                 535                 540

Ser Gln Gln Trp Leu Leu Arg Asn Val Thr Leu Pro Glu Ile Phe
545                 550                 555

<210> 37
<211> 1838
<212> DNA
<213> Rattus norvegicus

<220>
<223> Polypeptide GalNac transferase

<400> 37
atatgactgt gatttctgat gaaaccagat tggaataatt ttcatsatat ttgtatattt 60
atatatatat attttaaaat tttacatttg acttaaagtg ccatgagaaa atttgcatat 120
tgcaaggtgg ttctagccac ctccttggtt tgggtactct tggatatgtt cctgctgctt 180
tacttcagtg aatgcaacaa atgtgaagag aagaaggagc gagggcttcc tgctggggat 240
gttctagagc tggtgcagaa gcctcacgaa ggtcctggag aaatgggaaa accagtcgtc 300
attcctaaag aggatcagga aaagatgaaa gagatgttca aaatcaacca gttcaaccta 360
atggcgagtg agatgattgc attcaacaga tccttaccag acgtgaggct agaggggtgt 420
aaaacaaaag tgtacccaga tagccttcct acgacaagcg tggtgattgt ctttcacaat 480
gaggcgtgga gcaccctcct gcgaacagtc cacagtgtca ttaaccgctc accaagacac 540
atgatagaag agattgttct tgtagacgat gccagcgaaa gagacttttt gaaaagacct 600
ctagagagtt atgtgaaaaa gcttaaagtg ccagttcatg taattcgaat ggaacaacgt 660
tctgggttga tcagagctag attaaaagga gctgctgtgt ccaaaggcca agtgatcacc 720
ttcttagatg ctcactgcga gtgcacagta gggtggctgg agcctctgtt agcccggatc 780
aaacatgaca ggaggacagt ggtgtgtcct atcatcgatg tgatcagtga tgacactttt 840
gagtacatgg cgggctctga tatgacctat ggtggattca actggaagct caatttccgt 900
tggtatcctg ttccccaacg agaaatggac agaaggaaag gtgaccggac tcttcctgtc 960
agaacaccta caatggctgg aggcctgttt tcaatagaca gagattactt tcaggaaatt 1020
ggaacatatg atgctggaat ggatatttgg ggaggagaaa acctagaaat ttcctttagg 1080
atttggcagt gtggagggac tctggaaata gtgacgtgct cccacgttgg ccatgtgttt 1140
cggaaagcta cgccgtacac gttcccgggt ggcacggggc agatcatcaa caagaataac 1200
agacggctcg cggaggtgtg gatggatgaa ttcaagaatt tcttctacat aatttctcca 1260
ggtgttacaa aggtagatta tggtgatata tcatcaagag tcggtctgag acataaacta 1320

cagtgcaagc ctttctcttg gtacctagaa aatatttatc ccgattctca aattcctcgt 1380
cactatttct cattgggaga gatacgaaat gtggaaacaa atcaatgtct agataacatg 1440
gctagaaaag agaatgaaaa ggttggaatt tttaattgtc atggtatggg aggcaatcag 1500
gttttctctt acactgccaa caaagaaatt agaacagatg acctttgttt ggacgtttcc 1560
aaacttaatg gcccagttac catgctcaaa tgccaccacc taaaaggcaa ccagctctgg 1620
gagtatgacc cagtgaaatt aaccctgcag catgtgaaca gtaaccagtg cctggataaa 1680
gccacagaag aggacagcca ggtgcccagc atcagagact gcactggaag ccggtcccag 1740
cagtggcttc ttcgaaatgt cacccttcca gaaatattct gagaccaaat tacaacaaag 1800
aaaggattga ctgggctacc tcagcataag ccgaattc 1838

<210> 38
<211> 559
<212> PRT
<213> Mus musculus

<220>
<223> PARP-2

<400> 38
Met Ala Pro Arg Arg Gln Arg Ser Gly Ser Gly Arg Arg Val Leu Asn
1               5                   10                  15

Glu Ala Lys Lys Val Asp Asn Gly Asn Lys Ala Thr Glu Asp Asp Ser
            20                  25                  30

Pro Pro Gly Lys Lys Met Arg Thr Cys Gln Arg Lys Gly Pro Met Ala
        35                  40                  45

Gly Gly Lys Asp Ala Asp Arg Thr Lys Asp Asn Arg Asp Ser Val Lys
    50                  55                  60

Thr Leu Leu Leu Lys Gly Lys Ala Pro Val Asp Pro Glu Cys Ala Ala
65                  70                  75                  80

Lys Leu Gly Lys Ala His Val Tyr Cys Glu Gly Asp Asp Val Tyr Asp
                85                  90                  95

Val Met Leu Asn Gln Thr Asn Leu Gln Phe Asn Asn Asn Lys Tyr Tyr
            100                 105                 110

Leu Ile Gln Leu Leu Glu Asp Asp Ala Gln Arg Asn Phe Ser Val Trp
        115                 120                 125

Met Arg Trp Gly Arg Val Gly Lys Thr Gly Gln His Ser Leu Val Thr
    130                 135                 140

Cys Ser Gly Asp Leu Asn Lys Ala Lys Glu Ile Phe Gln Lys Lys Phe
145                 150                 155                 160

Leu Asp Lys Thr Lys Asn Asn Trp Glu Asp Arg Glu Asn Phe Glu Lys
                165                 170                 175

Val Pro Gly Lys Tyr Asp Met Leu Gln Met Asp Tyr Ala Ala Ser Thr
            180                 185                 190

Gln Asp Glu Ser Lys Thr Lys Glu Glu Glu Thr Leu Lys Pro Glu Ser
        195                 200                 205

Gln Leu Asp Leu Arg Val Gln Glu Leu Leu Lys Leu Ile Cys Asn Val
    210                 215                 220

Gln Thr Met Glu Glu Met Met Ile Glu Met Lys Tyr Asp Thr Lys Arg
225                 230                 235                 240

Ala Pro Leu Gly Lys Leu Thr Val Ala Gln Ile Lys Ala Gly Tyr Gln
                245                 250                 255

Ser Leu Lys Lys Ile Glu Asp Cys Ile Arg Ala Gly Gln His Gly Arg
            260                 265                 270

Ala Leu Val Glu Ala Cys Asn Glu Phe Tyr Thr Arg Ile Pro His Asp
        275                 280                 285

Phe Gly Leu Ser Ile Pro Pro Val Ile Arg Thr Glu Lys Glu Leu Ser
    290                 295                 300

Asp Lys Val Lys Leu Leu Glu Ala Leu Gly Asp Ile Glu Ile Ala Leu
305                 310                 315                 320

Lys Leu Val Lys Ser Glu Arg Gln Gly Leu Glu His Pro Leu Asp Gln
                325                 330                 335

His Tyr Arg Asn Leu His Cys Ala Leu Arg Pro Leu Asp His Glu Ser
            340                 345                 350

Asn Glu Phe Lys Val Ile Ser Gln Tyr Leu Gln Ser Thr His Ala Pro
        355                 360                 365

Thr His Lys Asp Tyr Thr Met Thr Leu Leu Asp Val Phe Glu Val Glu
    370                 375                 380

Lys Glu Gly Glu Lys Glu Ala Phe Arg Glu Asp Leu Pro Asn Arg Met
385                 390                 395                 400

Leu Leu Trp His Gly Ser Arg Leu Ser Asn Trp Val Gly Ile Leu Ser
                405                 410                 415

His Gly Leu Arg Val Ala Pro Pro Glu Ala Pro Ile Thr Gly Tyr Met
            420                 425                 430

Phe Gly Lys Gly Ile Tyr Phe Ala Asp Met Ser Ser Lys Ser Ala Asn
        435                 440                 445

Tyr Cys Phe Ala Ser Arg Leu Lys Asn Thr Gly Leu Leu Leu Leu Ser
    450                 455                 460

Glu Val Ala Leu Gly Gln Cys Asn Glu Leu Leu Glu Ala Asn Pro Lys
465                 470                 475                 480

Ala Gln Gly Leu Leu Arg Gly Lys His Ser Thr Lys Gly Met Gly Lys
                485                 490                 495

Met Ala Pro Ser Pro Ala His Phe Ile Thr Leu Asn Gly Ser Thr Val
            500                 505                 510

Pro Leu Gly Pro Ala Ser Asp Thr Gly Ile Leu Asn Pro Glu Gly Tyr
        515                 520                 525

Thr Leu Asn Tyr Asn Glu Phe Ile Val Tyr Ser Pro Asn Gln Val Arg
    530                 535                 540

Met Arg Tyr Leu Leu Lys Ile Gln Phe Asn Phe Leu Gln Leu Trp
545                 550                 555

<210> 39
<211> 1707
<212> DNA
<213> Mus musculus

<220>
<223> PARP-2

<400> 39
ctcgagtcaa gagcgatggc gccgcggcgg cagagatcag gctctggaag gcgagtgcta 60
aatgaagcca agaaagttga taatggcaac aaagcaacag aagacgactc tcctcctggc 120
aagaagatgc gcacgtgcca gagaaaaggg cctatggctg gagggaagga cgcagacagg 180
acaaaagaca atcgagactc tgtgaagacc ttgctgttaa agggcaaagc ccctgtggac 240
ccagagtgtg cagccaagct gggaaaggct catgtgtatt gtgaaggaga tgatgtctat 300
gatgtcatgc taaatcaaac caatctccag ttcaacaaca acaagtacta ccttattcag 360
ctgttagaag atgatgccca gaggaacttc agtgtttgga tgaggtgggg ccgagttgga 420
aagacggggc agcacagctt ggtgacttgt tctggtgacc tcaacaaagc aaaagaaata 480
tttcagaaaa aattccttga caaaactaaa aacaattggg aggaccgtga gaactttgaa 540
aaagtacctg gaaaatacga catgttacag atggactatg ctgccagcac gcaggatgaa 600
agtaaaacaa aagaagagga aactttgaag cctgagtctc agctggatct tcgagtccag 660
gagctgctaa agttgatctg taacgtgcag accatggaag aaatgatgat tgagatgaag 720
tatgacacca agagagcccc gcttggaaag ctgacagtgg cgcaaatcaa ggccggttac 780
cagtctctca agaagattga ggactgcatc cgcgctggcc agcatggcgg agcgcttgtt 840
gaagcgtgca atgaattcta caccaggatc cctcatgact ttggactctc catccctcca 900
gtaatccgga cagagaagga actgtcagac aaagtaaaac tgctagaggc attgggagac 960
attgaaattg cccttaaact ggtgaagtca gagcgccaag gcctagaaca cccactggac 1020
caacactata gaaacctaca ctgtgctttg cgtcctctgg accatgaaag taatgagttt 1080
aaggtgattt ctcagtacct acagtctacg catgctccta cacacaagga ctatactatg 1140
accttgctgg atgttttcga agtagagaag gaaggggaga aagaggcctt cagggaggac 1200
cttcctaaca ggatgctgct ctggcatgga tccaggctga gtaactgggt ggggatcctg 1260
agccacgggc ttagagttgc cccacctgag gctcccatca caggttatat gtttggaaaa 1320
ggaatctact ttgctgacat gtcctccaag agtgccaatt actgctttgc ctctcgccta 1380
aagaatacag gattgcttct tctgtcagag gtagctctag gtcagtgtaa tgaactactg 1440
gaggccaatc ctaaagcaca aggattgctt cggggcaagc atagcaccaa ggggatggga 1500
aagatggctc ccagccctgc ccacttcatc accctgaatg ggagtacagt gcccttagga 1560
ccagcaagtg acacaggaat tctcaatcca gaggggtaca ccctcaacta caatgagttt 1620
attgtttata gccccaacca ggtccgtatg cgataccttc taaagattca atttaacttc 1680
ctgcagctat ggtgaatgtt gctcgag 1707

<210> 40
<211> 188
<212> PRT
<213> Homo sapiens

<220>
<223> K-ras

<400> 40
Met Thr Glu Tyr Lys Leu Val Val Val Gly Ala Cys Gly Val Gly Lys
1               5                   10                  15

Ser Ala Leu Thr Ile Gln Leu Ile Gln Asn His Phe Val Asp Glu Tyr
            20                  25                  30

Asp Pro Thr Ile Glu Asp Ser Tyr Arg Lys Gln Val Val Ile Asp Gly
        35                  40                  45

Glu Thr Cys Leu Leu Asp Ile Leu Asp Thr Ala Gly Gln Glu Glu Tyr
    50                  55                  60

Ser Ala Met Arg Asp Gln Tyr Met Arg Thr Gly Glu Gly Phe Leu Cys
65                  70                  75                  80

Val Phe Ala Ile Asn Asn Thr Lys Ser Phe Glu Asp Ile His His Tyr
                85                  90                  95

Arg Glu Gln Ile Lys Arg Val Lys Asp Ser Glu Asp Val Pro Met Val
            100                 105                 110

Leu Val Gly Asn Lys Cys Asp Leu Pro Ser Arg Thr Val Asp Thr Lys
        115                 120                 125

Gln Ala Gln Asp Leu Ala Arg Ser Tyr Gly Ile Pro Phe Ile Glu Thr
    130                 135                 140

Ser Ala Lys Thr Arg Gln Gly Val Asp Asp Ala Phe Tyr Thr Leu Val
145                 150                 155                 160

Arg Glu Ile Arg Lys His Lys Glu Lys Met Ser Lys Asp Gly Lys Lys
                165                 170                 175

Lys Lys Lys Lys Ser Lys Thr Lys Cys Val Ile Met
            180                 185

<210> 41
<211> 5775
<212> DNA
<213> Homo sapiens

<220>
<223> K-ras

<400> 41
tcctaggcgg cggccgcggc ggcggaggca gcagcggcgg cggcagtggc ggcggcgaag 60
gtggcggcgg ctcggccagt actcccggcc cccgccattt cggactggga gcgagcgcgg 120
cgcaggcact gaaggcggcg gcggggccag aggctcagcg gctcccaggt gcgggagaga 180
ggcctgctga aaatgactga atataaactt gtggtagttg gagcttgtgg cgtaggcaag 240
agtgccttga cgatacagct aattcagaat cattttgtgg acgaatatga tccaacaata 300
gaggattcct acaggaagca agtagtaatt gatggagaaa cctgtctctt ggatattctc 360
gacacagcag gtcaagagga gtacagtgca atgagggacc agtacatgag gactggggag 420
ggctttcttt gtgtatttgc cataaataat actaaatcat ttgaagatat tcaccattat 480
agagaacaaa ttaaaagagt taaggactct gaagatgtac ctatggtcct agtaggaaat 540
aaatgtgatt tgccttctag aacagtagac acaaaacagg ctcaggactt agcaagaagt 600
tatggaattc cttttattga aacatcagca aagacaagac agggtgttga tgatgccttc 660
tatacattag ttcgagaaat tcgaaaacat aaagaaaaga tgagcaaaga tggtaaaaag 720
aagaaaaaga agtcaaagac aaagtgtgta attatgtaaa tacaatttgt acttttttct 780
taaggcatac tagtacaagt ggtaattttt gtacattaca ctaaattatt agcatttgtt 840
ttagcattac ctaatttttt tcctgctcca tgcagactgt tagcttttac cttaaatgct 900
tattttaaaa tgacagtgga agtttttttt tcctcgaagt gccagtattc ccagagtttt 960
ggtttttgaa ctagcaatgc ctgtgaaaaa gaaactgaat acctaagatt tctgtcttgg 1020
ggtttttggt gcatgcagtt gattacttct tatttttctt accaagtgtg aatgttggtg 1080
tgaaacaaat taatgaagct tttgaatcat ccctattctg tgttttatct agtcacataa 1140
atggattaat tactaatttc agttgagacc ttctaattgg tttttactga aacattgagg 1200
gacacaaatt tatgggcttc ctgatgatga ttcttctagg catcatgtcc tatagtttgt 1260
catccctgat gaatgtaaag ttacactgtt cacaaaggtt ttgtctcctt tccactgcta 1320
ttagtcatgg tcactctccc caaaatatta tattttttct ataaaaagaa aaaaatggaa 1380

aaaaattaca aggcaatgga aactattata aggccatttc cttttcacat tagataaatt 1440
actataaaga ctcctaatag ctttttcctg ttaaggcaga cccagtatga atgggattat 1500
tatagcaacc attttggggc tatatttaca tgctactaaa tttttataat aattgaaaag 1560
attttaacaa gtataaaaaa attctcatag gaattaaatg tagtctccct gtgtcagact 1620
gctctttcat agtataactt taaatctttt cttcaacttg agtctttgaa gatagtttta 1680
attctgcttg tgacattaaa agattatttg ggccagttat agcttattag gtgttgaaga 1740
gaccaaggtt gcaagccagg ccctgtgtga accttgagct ttcatagaga gtttcacagc 1800
atggactgtg tgccccacgg tcatccgagt ggttgtacga tgcattggtt agtcaaaaat 1860
ggggagggac tagggcagtt tggatagctc aacaagatac aatctcactc tgtggtggtc 1920
ctgctgacaa atcaagagca ttgcttttgt ttcttaagaa aacaaactct tttttaaaaa 1980
ttacttttaa atattaactc aaaagttgag attttggggt ggtggtgtgc caagacatta 2040
attttttttt taaacaatga agtgaaaaag ttttacaatc tctaggtttg gctagttctc 2100
ttaacactgg ttaaattaac attgcataaa cacttttcaa gtctgatcca tatttaataa 2160
tgctttaaaa taaaaataaa aacaatcctt ttgataaatt taaaatgtta cttattttaa 2220
aataaatgaa gtgagatggc atggtgaggt gaaagtatca ctggactagg ttgttggtga 2280
cttaggttct agataggtgt cttttaggac tctgattttg aggacatcac ttactatcca 2340
tttcttcatg ttaaaagaag tcatctcaaa ctcttagttt ttttttttta cactatgtga 2400
tttatattcc atttacataa ggatacactt atttgtcaag ctcagcacaa tctgtaaatt 2460
tttaacctat gttacaccat cttcagtgcc agtcttgggc aaaattgtgc aagaggtgaa 2520
gtttatattt gaatatccat tctcgtttta ggactcttct tccatattag tgtcatcttg 2580
cctccctacc ttccacatgc cccatgactt gatgcagttt taatacttgt aattccccta 2640
accataagat ttactgctgc tgtggatatc tccatgaagt tttcccactg agtcacatca 2700
gaaatgccct acatcttatt ttcctcaggg ctcaagagaa tctgacagat accataaagg 2760
gatttgacct aatcactaat tttcaggtgg tggctgatgc tttgaacatc tctttgctgc 2820
ccaatccatt agcgacagta ggatttttca accctggtat gaatagacag aaccctatcc 2880
agtggaagga gaatttaata aagatagtgc agaaagaatt ccttaggtaa tctataacta 2940
ggactactcc tggtaacagt aatacattcc attgttttag taaccagaaa tcttcatgca 3000
atgaaaaata ctttaattca tgaagcttac tttttttttt ttggtgtcag agtctcgctc 3060
ttgtcaccca ggctggaatg cagtggcgcc atctcagctc actgcaacct tccatcttcc 3120
caggttcaag cgattctcgt gcctcggcct cctgagtagc tgggattaca ggcgtgtgca 3180
ctacactcaa ctaatttttg tatttttagg agagacgggg tttcacctgt tggccaggct 3240
ggtctcgaac tcctgacctc aagtgattca cccaccttgg cctcataaac ctgttttgca 3300
gaactcattt attcagcaaa tatttattga gtgcctacca gatgccagtc accgcacaag 3360
gcactgggta tatggtatcc ccaaacaaga gacataatcc cggtccttag gtactgctag 3420
tgtggtctgt aatatcttac taaggccttt ggtatacgac ccagagataa cacgatgcgt 3480
attttagttt tgcaaagaag gggtttggtc tctgtgccag ctctataatt gttttgctac 3540
gattccactg aaactcttcg atcaagctac tttatgtaaa tcacttcatt gttttaaagg 3600
aataaacttg attatattgt ttttttattt ggcataactg tgattctttt aggacaatta 3660
ctgtacacat taaggtgtat gtcagatatt catattgacc caaatgtgta atattccagt 3720
tttctctgca taagtaatta aaatatactt aaaaattaat agttttatct gggtacaaat 3780
aaacagtgcc tgaactagtt cacagacaag ggaaacttct atgtaaaaat cactatgatt 3840
tctgaattgc tatgtgaaac tacagatctt tggaacactg tttaggtagg gtgttaagac 3900
ttgacacagt acctcgtttc tacacagaga aagaaatggc catacttcag gaactgcagt 3960
gcttatgagg ggatatttag gcctcttgaa tttttgatgt agatgggcat ttttttaagg 4020
tagtggttaa ttacctttat gtgaactttg aatggtttaa caaaagattt gtttttgtag 4080
agattttaaa gggggagaat tctagaaata aatgttacct aattattaca gccttaaaga 4140
caaaaatcct tgttgaagtt tttttaaaaa aagactaaat tacatagact taggcattaa 4200
catgtttgtg gaagaatata gcagacgtat attgtatcat ttgagtgaat gttcccaagt 4260
aggcattcta ggctctattt aactgagtca cactgcatag gaatttagaa cctaactttt 4320
ataggttatc aaaactgttg tcaccattgc acaattttgt cctaatatat acatagaaac 4380
tttgtggggc atgttaagtt acagtttgca caagttcatc tcatttgtat tccattgatt 4440
tttttttttc ttctaaacat tttttcttca aaacagtata tataactttt tttaggggat 4500
tttttttaga cagcaaaaaa ctatctgaag atttccattt gtcaaaaagt aatgatttct 4560
tgataattgt gtagtgaatg ttttttagaa cccagcagtt accttgaaag ctgaatttat 4620
atttagtaac ttctgtgtta atactggata gcatgaattc tgcattgaga aactgaatag 4680
ctgtcataaa atgctttctt tcctaaagaa agatactcac atgagttctt gaagaatagt 4740
cataactaga ttaagatctg tgttttagtt taatagtttg aagtgcctgt ttgggataat 4800
gataggtaat ttagatgaat ttaggggaaa aaaaagttat ctgcagttat gttgagggcc 4860
catctctccc cccacacccc cacagagcta actgggttac agtgttttat ccgaaagttt 4920
ccaattccac tgtcttgtgt tttcatgttg aaaatacttt tgcatttttc ctttgagtgc 4980
caatttctta ctagtactat ttcttaatgt aacatgttta cctggcctgt cttttaacta 5040

tttttgtata gtgtaaactg aaacatgcac attttgtaca ttgtgctttc ttttgtgggt 5100
catatgcagt gtgatccagt tgttttccat catttggttg cgctgaccta ggaatgttgg 5160
tcatatcaaa cattaaaaat gaccactctt ttaatgaaat taacttttaa atgtttatag 5220
gagtatgtgc tgtgaagtga tctaaaattt gtaatatttt tgtcatgaac tgtactactc 5280
ctaattattg taatgtaata aaaatagtta cagtgactat gagtgtgtat ttattcatgc 5340
aaatttgaac tgtttgcccc gaaatggata tggatacttt ataagccata gacactatag 5400
tataccagtg aatcttttat gcagcttgtt agaagtatcc ttttattttc taaaaggtgc 5460
tgtggatatt atgtaaaggc gtgtttgctt aaacaatttt ccatatttag aagtagatgc 5520
aaaacaaatc tgcctttatg acaaaaaaat aggataacat tatttattta tttcctttta 5580
tcaataaggt aattgataca caacaggtga cttggtttta ggcccaaagg tagcagcagc 5640
aacattaata atggaaataa ttgaatagtt agttatgtat gttaatgcca gtcaccagca 5700
ggctatttca aggtcagaag taatgactcc atacatatta tttatttcta taactacatt 5760
taaatcatta ccagg 5775

<210> 42
<211> 179
<212> PRT
<213> Homo sapiens

<220>
<223> ARF-like protein

<400> 42
Met Gly Ile Leu Phe Thr Arg Ile Trp Arg Leu Phe Asn His Gln Glu
1               5                   10                  15

His Lys Val Ile Ile Val Gly Leu Asp Asn Ala Gly Lys Thr Thr Ile
            20                  25                  30

Leu Tyr Gln Phe Ser Met Asn Glu Val Val His Thr Ser Pro Thr Ile
        35                  40                  45

Gly Ser Asn Val Glu Glu Ile Val Val Asn Asn Thr Arg Phe Leu Met
    50                  55                  60

Trp Asp Ile Gly Gly Gln Glu Ser Leu Arg Ser Ser Trp Asn Thr Tyr
65                  70                  75                  80

Tyr Thr Asn Thr Glu Phe Val Ile Val Val Val Asp Ser Thr Asp Arg
                85                  90                  95

Glu Arg Ile Ser Val Thr Arg Glu Glu Leu Tyr Lys Met Leu Ala His
            100                 105                 110

Glu Asp Leu Arg Lys Ala Gly Leu Leu Ile Phe Ala Asn Lys Gln Asp
        115                 120                 125

Val Lys Glu Cys Met Thr Val Ala Glu Ile Ser Gln Phe Leu Lys Leu
    130                 135                 140

Thr Ser Ile Lys Asp His Gln Trp His Ile Gln Ala Cys Cys Ala Leu
145                 150                 155                 160

Thr Gly Glu Gly Leu Cys Gln Gly Leu Glu Trp Met Met Ser Arg Leu
                165                 170                 175

Lys Ile Arg

<210> 43
<211> 2717
<212> DNA
<213> Homo sapiens

<220>
<223> ARF-like protein

<400> 43
agcctgagcc cgggcggctg gagggctccg ctgcccgagg atgggaattc tcttcacccg 60
gatctggaga ctgttcaatc accaggagca caaagttatt attgttgggc tggacaatgc 120
cgggaagact accatccttt accagttttc catgaatgaa gttgtacata catctcctac 180
aataggaagc aatgtggaag agattgtggt taataataca cgtttcctga tgtgggatat 240
tggtggccaa gaatcccttc gttcttcctg gaacacttat tatactaaca cagagtttgt 300
aatagttgtg gtggacagca cagacagaga aagaatttct gtaactagag aagagctcta 360
caaaatgcta gcccacgagg acctaaggaa agctgggttg ctgatttttg ctaataaaca 420
agatgttaaa gaatgcatga cagtagcaga aatctcccag tttttgaagc tcacttctat 480
aaaagaccac caatggcata tccaagcatg ctgtgctctc actggcgagg ggttatgcca 540
aggacttgaa tggatgatgt cacggctgaa gatcagatga tctcttctga ccccttctca 600
tggactctgt ataaacaaag tgctggactt tatctgaaag ctgcaaaaaa tggtttagat 660
atatttataa taaactgact taagagtttt ctacaagaag aaaattaaga ccatttattt 720
gaaaacagat gaaatgtcac cacctcgttt gctctctcat tccttccaaa cctgtgactg 780
gtattttctc atgatgaatc ctctccggac atgggagagt tctaatgaat acaaggagga 840
ggaagacatt tgaacttgga gctttatttc ctcgacgagc ccaccctgcc ccaagtgaat 900
gttccctttt cattacattg aatcaaatac ggatcagcac ggatactgtt tccagttttt 960
aaaaatgtaa taatattatg gaaagtattt tacttaaagt tgaatatgta ttgtgtatat 1020
acctcaagtt caggttaatg gctttgattt atttgtgttc taaaggaaag caaataacac 1080
aagttaatga cgttattttc ctgatgtcac tgagtaccat cttggtgtta attgtcactg 1140
tttagtttac cctctgttct ctgtattgtt ctaacaagcc aaccccagcc cagaatcggt 1200
aagctgctcc ttaaaaatga aacgggagaa agcttgacat tgtgtaccta tttttgttgc 1260
tcagtcatac agatggaaat ccataacgca agtgggtaga gtctgctgga ctcctggcct 1320
ggtgtggccg ggcagaatta ctcgagaagc agttgtcaga ctttcccttc tgctggtcct 1380
aattcagcca attcagctac taatcggtca ttcataaatt gtgattaaaa tgatcaggta 1440
atttttaatt gaaaatcatg ttgtgaaata tatctaccca aagaggtaac atcctataaa 1500
tagtaataat taatttagct gcagcctttt caggtattaa attatatgac aaacagttaa 1560
aatgaattta agagtaaaca taaggcatag ttttcagtga acattaataa tttctcaatt 1620
tgcagattag catctgcaaa ggtatcttct tccattgcat aaaatgcctt gtttaatagt 1680
taactaatca aatattctga atgacttcaa aaatttgatt gaaaatttga gatatcacat 1740
tatttgtact aggatttgac ctaaaacacc ttttatattt gttaaccttt cttaaattta 1800
gttaaaattt gaagtatttc tgtcgtaatt ataaacaaaa ttattttgtg ttttaacata 1860
ttcttggtga ctctttatac tttttgtgcc ctgaaaattt gaaggttact ccaaactgtc 1920
aagatttagg atattcaaga aaagatttaa ctagttttag tgtcagactt taaaatttcc 1980
taaggaattt ggtaagaaat agaagtccac tctataagca ttgaggaaac tttgaaaaac 2040
ctttaagtaa atgtaagact cttaatgact aaacccatcg gagtggatga gtgttcccct 2100
ggaggcttgt gtttgagacc tgtgctgtaa gcctcccact tgaccttcag cgtaatgttt 2160
cccacctgct cagagtgtca gcactcgtca ctaggctttc cagggctaag tccttcctgt 2220
tcacatgttc tcacactttt tcaaatcatg aggctttctc ctgtatagta aaaatcccct 2280
taaagactca aatctacgtc gtagttttgt cagagaccaa tagtccatcg agcccccacc 2340
taccccagat ctgcagcttc ttagctttta tctgaatagt ccttgcacag ttgcttgcct 2400
ctctgtcaca tcagttcagc acatagcagc acatcctggt gttgccgagg tattggctag 2460
aatttattct taattagatg tttaaagcat ttactccact gttaaagttg tcttagtgta 2520
gtcaggtatt gacatgacat aagaaagcat gaaactacca catttacaaa atatgaggga 2580
tttggggttt ggtttttttt gtttgtttgg ttggtttttt tttttttttg gtttttaact 2640
attttagact caaataatat catacctgtg tacttgtaca taaaaacaaa attattactg 2700
agtaaaaaga ttaatcg 2717

<210> 44
<211> 1230
<212> PRT
<213> Rattus norvegicus

<220>
<223> TBP-binding protein

<400> 44
Met Ala Ser Ala Ser Tyr His Ile Ser Asn Leu Leu Glu Lys Met Thr
1               5                   10                  15

Ser Ser Asp Lys Asp Phe Arg Phe Met Ala Thr Asn Asp Leu Met Thr
            20                  25                  30

Glu Leu Gln Lys Asp Ser Ile Lys Leu Asp Asp Asp Ser Glu Arg Lys
        35                  40                  45

Val Val Lys Met Ile Leu Lys Leu Leu Glu Asp Lys Asn Gly Glu Val
    50                  55                  60

Gln Asn Leu Ala Val Lys Cys Leu Gly Pro Leu Val Ser Lys Val Lys
65                  70                  75                  80

Glu Tyr Gln Val Glu Thr Ile Val Asp Thr Leu Cys Thr Asn Met Leu
                85                  90                  95

Ser Asp Lys Glu Gln Leu Arg Asp Ile Ser Ser Ile Gly Leu Lys Thr
            100                 105                 110

Val Ile Gly Glu Leu Pro Pro Ala Ser Ser Gly Ser Ala Leu Ala Ala
        115                 120                 125

Asn Val Cys Lys Lys Ile Thr Gly Arg Leu Thr Ser Ala Ile Ala Lys
    130                 135                 140

Gln Glu Asp Val Ser Val Gln Leu Glu Ala Leu Asp Ile Met Ala Asp
145                 150                 155                 160

Met Leu Ser Arg Gln Gly Gly Leu Leu Val Asn Phe His Pro Ser Ile
                165                 170                 175

Leu Thr Cys Leu Leu Pro Gln Leu Thr Ser Pro Arg Leu Ala Val Arg
            180                 185                 190

Lys Arg Thr Ile Ile Ala Leu Gly His Leu Val Met Ser Cys Gly Asn
        195                 200                 205

Ile Val Phe Val Asp Leu Ile Glu His Leu Leu Ser Glu Leu Ser Lys
    210                 215                 220

Asn Asp Ser Met Ser Thr Thr Arg Thr Tyr Ile Gln Cys Ile Ala Ala
225                 230                 235                 240

Ile Ser Arg Gln Ala Gly His Arg Ile Gly Glu Tyr Leu Glu Lys Ile
                245                 250                 255

Ile Pro Leu Val Val Lys Phe Cys Asn Val Asp Asp Asp Glu Leu Arg
            260                 265                 270

Glu Tyr Cys Ile Gln Ala Phe Glu Ser Phe Val Arg Arg Cys Pro Lys
        275                 280                 285

Glu Val Tyr Pro His Val Ser Thr Ile Ile Asn Ile Cys Leu Lys Tyr
    290                 295                 300

Leu Thr Tyr Asp Pro Asn Tyr Asn Tyr Asp Asp Glu Asp Glu Asp Glu
305                 310                 315                 320

Asn Ala Met Asp Ala Asp Gly Gly Asp Asp Asp Asp Gln Gly Ser Asp
                325                 330                 335

Asp Glu Tyr Ser Asp Asp Asp Asp Met Ser Trp Lys Val Arg Arg Ala
            340                 345                 350

Ala Ala Lys Cys Leu Asp Ala Val Val Ser Thr Arg His Glu Met Leu
        355                 360                 365

Pro Glu Phe Tyr Lys Thr Val Ser Pro Ala Leu Ile Ser Arg Phe Lys
    370                 375                 380

Glu Arg Glu Glu Asn Val Lys Ala Asp Val Phe His Ala Tyr Leu Ser
385                 390                 395                 400

Leu Leu Lys Gln Thr Arg Pro Val Gln Ser Trp Leu Cys Asp Pro Asp
                405                 410                 415

Ala Met Glu Gln Gly Glu Thr Pro Leu Thr Met Leu Gln Ser Gln Val
            420                 425                 430

Pro Asn Ile Val Lys Ala Leu His Lys Gln Met Lys Glu Lys Ser Val
        435                 440                 445

Lys Thr Arg Gln Cys Cys Phe Asn Met Leu Thr Glu Leu Val Asn Val
    450                 455                 460

Leu Pro Gly Ala Leu Thr Gln His Ile Pro Val Leu Val Pro Gly Ile
465                 470                 475                 480

Ile Phe Ser Leu Asn Asp Lys Ser Ser Ser Ser Asn Leu Lys Ile Asp
                485                 490                 495

Ala Leu Ser Cys Leu Tyr Val Ile Leu Cys Asn His Ser Pro Gln Val
            500                 505                 510

Phe His Pro His Val Gln Ala Leu Val Pro Pro Val Val Ala Cys Val
        515                 520                 525

Gly Asp Pro Phe Tyr Lys Ile Thr Ser Glu Ala Leu Leu Val Thr Gln
    530                 535                 540

Gln Leu Val Lys Val Ile Arg Pro Leu Asp Gln Pro Ser Ser Phe Asp
545                 550                 555                 560

Ala Thr Pro Tyr Ile Lys Asp Leu Phe Thr Cys Thr Ile Lys Arg Leu
                565                 570                 575

Lys Ala Ala Asp Ile Asp Gln Glu Val Lys Glu Arg Ala Ile Ser Cys
            580                 585                 590

Met Gly Gln Ile Ile Cys Asn Leu Gly Asp Asn Leu Gly Pro Asp Leu
        595                 600                 605

Ser Asn Thr Leu Gln Ile Phe Leu Glu Arg Leu Lys Asn Glu Ile Thr
    610                 615                 620

Arg Leu Thr Thr Val Lys Ala Leu Thr Leu Ile Ala Gly Ser Pro Leu
625                 630                 635                 640

Lys Ile Asp Leu Arg Pro Val Leu Gly Glu Gly Val Pro Ile Leu Ala
                645                 650                 655

Ser Phe Leu Arg Lys Asn Gln Arg Ala Leu Lys Leu Gly Thr Leu Ser
            660                 665                 670

Ala Leu Asp Ile Leu Ile Lys Asn Tyr Ser Asp Ser Leu Thr Ala Ala
        675                 680                 685

Met Ile Asp Ala Val Leu Asp Glu Leu Pro Pro Leu Ile Ser Glu Ser
    690                 695                 700

Asp Met His Val Ser Gln Met Ala Ile Ser Phe Leu Thr Thr Leu Ala
705                 710                 715                 720

Lys Val Tyr Pro Ser Ser Leu Ser Lys Ile Ser Gly Ser Ile Leu Asn
                725                 730                 735

Glu Leu Ile Gly Leu Val Arg Ser Pro Leu Leu Gln Gly Gly Ala Leu
            740                 745                 750

Ser Ala Met Leu Asp Phe Phe Gln Ala Leu Val Val Thr Gly Thr Asn
        755                 760                 765

Asn Leu Gly Tyr Met Asp Leu Leu Arg Met Leu Thr Gly Pro Val Tyr
    770                 775                 780

Ser Gln Ser Thr Ala Leu Thr His Lys Gln Ser Tyr Tyr Ser Ile Ala
785                 790                 795                 800

Lys Cys Val Ala Ala Leu Thr Arg Ala Cys Pro Lys Glu Gly Pro Ala
                805                 810                 815

Val Val Gly Gln Phe Ile Gln Asp Val Lys Asn Ser Arg Ser Thr Asp
            820                 825                 830

Ser Ile Arg Leu Leu Ala Leu Leu Ser Leu Gly Glu Val Gly His His
        835                 840                 845

Ile Asp Leu Ser Gly Gln Leu Glu Leu Lys Ser Val Ile Leu Glu Ala
    850                 855                 860

Phe Ser Ser Pro Ser Glu Glu Val Lys Ser Ala Ala Ser Tyr Ala Leu
865                 870                 875                 880

Gly Ser Ile Ser Val Gly Asn Leu Pro Glu Tyr Leu Pro Phe Val Leu
                885                 890                 895

Gln Glu Ile Thr Ser Gln Pro Lys Arg Gln Tyr Leu Leu Leu His Ser
            900                 905                 910

Leu Lys Glu Ile Ile Ser Ser Ala Ser Val Val Gly Leu Lys Pro Tyr
        915                 920                 925

Val Glu Asn Ile Trp Ala Leu Leu Leu Lys His Cys Glu Cys Ala Glu
    930                 935                 940

Glu Gly Thr Arg Asn Val Val Ala Glu Cys Leu Gly Lys Leu Thr Leu

945                 950                 955                 960

Ile Asp Pro Glu Thr Leu Leu Pro Arg Leu Lys Gly Tyr Leu Ile Ser
                965                 970                 975

Gly Ser Ser Tyr Ala Arg Ser Ser Val Val Thr Ala Val Lys Phe Thr
            980                 985                 990

Ile Ser Asp His Pro Gln Pro Ile Asp Pro Leu Leu Lys Asn Cys Ile
        995                 1000                1005

Gly Asp Phe Leu Lys Thr Leu Glu Asp Pro Asp Leu Asn Val Arg Arg
    1010                1015                1020

Val Ala Leu Val Thr Phe Asn Ser Ala Ala His Asn Lys Pro Ser Leu
1025                1030                1035                1040

Ile Arg Asp Leu Leu Asp Ser Val Leu Pro His Leu Tyr Asn Glu Thr
                1045                1050                1055

Lys Val Arg Lys Glu Leu Ile Arg Glu Val Glu Met Gly Pro Phe Lys
            1060                1065                1070

His Thr Val Asp Asp Gly Leu Asp Ile Arg Lys Ala Ala Phe Glu Cys
        1075                1080                1085

Met Tyr Thr Leu Leu Asp Ser Cys Leu Asp Arg Leu Asp Ile Phe Glu
    1090                1095                1100

Phe Leu Asn His Val Glu Asp Gly Leu Lys Asp His Tyr Asp Ile Lys
1105                1110                1115                1120

Met Leu Thr Phe Leu Met Leu Val Arg Leu Ser Thr Leu Cys Pro Ser
                1125                1130                1135

Ala Val Leu Gln Arg Leu Asp Arg Leu Val Glu Pro Leu Arg Ala Thr
            1140                1145                1150

Cys Thr Thr Lys Val Lys Ala Asn Ser Val Lys Gln Glu Phe Glu Lys
        1155                1160                1165

Gln Asp Glu Leu Lys Arg Ser Ala Met Arg Ala Val Ala Ala Leu Leu
    1170                1175                1180

Thr Ile Pro Glu Ala Glu Lys Ser Pro Leu Met Ser Glu Phe Gln Ser
1185                1190                1195                1200

Gln Ile Ser Ser Asn Pro Glu Leu Ala Ala Ile Phe Glu Ser Ile Gln
                1205                1210                1215

Lys Asp Ser Ser Ser Thr Asn Leu Glu Ser Met Asp Thr Ser
            1220                1225                1230


<210> 45
<211> 4383
<212> DNA
<213> Rattus norvegicus

<220>
<223> TBP-binding protein

<400> 45
tctggcctct tgccctaaga agcgagtgcg gagggagtgg gagcgagact gcccgaacgc 60
aagtgtctgg cttcccggcg aggcccaggc gtccgtcccg ccgcccatga actcgttgcc 120
ccgcgagcag cgccgtcgtt aggccgtccc cgtcactctc gctcgccccc atccccgggc 180
agccattccc ccctacccac gtctggccgg ggactggacg tcgtgtcgaa gcgccttccc 240
cagtccgcgt cgcgctgcga ccctggaagc gggcgccgcc gccaacgaga ggaggagccc 300
cggccgcggg gcccagcagc cactgagcca gcaggcggga tcgaggccgg caacatggcg 360
agcgcttcgt accacatctc caacttgctg gaaaaaatga catccagcga caaggacttc 420
aggtttatgg ctacaaatga tctgatgaca gaactgcaga aagactccat caagctggat 480
gatgacagcg aaaggaaagt cgtaaagatg attctgaagt tgctggagga taaaaatggc 540
gaagtgcaga acttagctgt gaaatgtctt ggtcctttag tgagtaaagt gaaagagtac 600
caagttgaga cgattgtaga caccctctgt acaaacatgc tttctgacaa ggagcagctt 660
cgagatattt ccagtattgg ccttaaaaca gtaattggag aactccctcc agcttccagt 720
ggctctgcat tagctgctaa tgtatgtaaa aagattactg gacgccttac cagtgcaata 780
gcaaagcagg aagatgtttc tgttcagcta gaagctttag atattatggc tgatatgttg 840
agcaggcaag gaggactgct tgttaatttc catccttcaa ttctgacctg tctacttccc 900
cagctgacta gccctagact tgcagtcagg aaaagaacaa ttattgctct tggccaccta 960
gttatgagct gcggaaacat agtttttgta gaccttattg aacatctgtt gtcagagttg 1020
tccaaaaatg actccatgtc aacaacaagg acctacatac agtgtattgc tgctattagt 1080
aggcaagctg gccatagaat aggtgaatac cttgaaaaga taattccttt ggtggtaaaa 1140
ttttgtaatg tagatgatga tgaattaaga gaatactgca ttcaagcttt tgaatcattt 1200
gtgagaagat gccctaagga agtttatcct catgtttcta ccattataaa catttgtcta 1260
aaatacctta cctatgatcc aaattacaac tatgatgatg aagatgaaga tgagaatgct 1320
atggatgctg atggcggaga tgacgatgac caagggagtg acgatgaata cagtgatgac 1380
gatgacatga gttggaaagt gaggcgtgcg gctgccaagt gcctggatgc cgtagttagc 1440
acacggcatg agatgctccc ggaattctac aagaccgtct ctcctgcact gatatccaga 1500
tttaaagagc gcgaagagaa tgtaaaggca gatgtttttc atgcatacct ttctcttttg 1560
aagcaaactc gtccagtaca aagttggctg tgcgaccctg atgcaatgga gcaaggagaa 1620
acacccttaa caatgcttca gagtcaggtt cccaacattg ttaaagccct acataaacag 1680
atgaaagaaa aaagtgtaaa gacccgacag tgttgtttta acatgttaac tgaactggta 1740
aatgtgttgc ctggggcact aacccaacac attcctgtac ttgtaccagg aatcattttc 1800
tcactcaatg ataagtccag ctcatcgaat ctgaagattg atgcattgtc ctgtctgtat 1860
gtgatcctct gtaaccactc tccgcaagtt ttccacccgc atgttcaggc tttggtccct 1920
ccagtggtgg cttgtgttgg tgacccattt tacaagatca catcagaagc ccttcttgtc 1980
actcagcagc ttgtcaaagt aatccgtcct ctggaccaac cctcctcctt cgatgcaacg 2040
ccttacatca aagatctctt cacttgtaca attaagcgct taaaagcagc tgacattgat 2100
caggaagtca aggagagggc tatttcctgt atgggacaga ttatttgcaa tcttggagac 2160
aatttgggcc ctgacttatc aaatacactt cagattttct tggagagact caagaatgaa 2220
atcacccggc taacgacagt gaaagcactg accctgattg ctgggtcacc tttgaagata 2280
gatctgaggc ctgtgctggg agagggagtc cctatccttg cttcatttct caggaaaaat 2340
cagagagctt tgaaactggg gaccctctct gccctagata ttctcattaa aaactatagt 2400
gacagtttga cggccgccat gattgatgca gttctggatg agctccctcc tcttatcagc 2460
gaaagtgata tgcacgtgtc ccagatggct atcagcttcc ttactaccct ggcaaaagta 2520
tatccctcct ccctttcaaa gataagcgga tctattctca atgaactgat tggacttgta 2580
aggtcacctc tgctgcaggg aggagctctt agtgccatgc tagacttctt ccaagctttg 2640
gttgtcactg gaacaaacaa tctaggatac atggatttgc tgcgcatgct aacgggtcca 2700
gtttactctc agagcacagc tcttactcat aagcagtctt actattccat tgccaaatgt 2760
gtagctgccc ttactcgagc atgccctaaa gagggaccgg ctgtagtagg tcagtttatt 2820
caagatgtca agaactccag gtctacagat tccattcgtc tcttagcgct cctttctcta 2880
ggagaagttg gacaccatat tgacttaagt gggcagttgg agctaaaatc tgtaatatta 2940
gaggctttct cctctcctag tgaagaagtt aaatcagctg catcctatgc gttaggcagc 3000
atcagtgtag gcaacctccc tgagtatctg ccatttgtac tacaagaaat aaccagtcag 3060
cccaaaaggc agtacctgct gctgcattcc ctgaaggaaa tcattagctc tgcgtcagtg 3120
gtcggcctta agccgtatgt cgagaacatc tgggccttgc tgctaaagca ctgtgagtgc 3180
gcggaggagg gcaccagaaa cgtcgtcgcc gagtgtctag ggaagctcac tcttattgat 3240
cctgaaactc tcctcccgag gcttaaaggg tatttgatat cagggtcatc ctatgccaga 3300
agctcagtgg ttacagctgt gaagttcact atttctgacc accctcagcc cattgatcca 3360
ctgctcaaga actgcatagg tgattttcta aaaacgttgg aagacccaga tttgaatgta 3420
agaagagtgg ccttggtcac attcaattct gcagcccata acaagccgtc actgatacgg 3480
gaccttctgg attctgttct tccacatctt tacaatgaga caaaagttag gaaggaactt 3540

ataagagagg tagagatggg cccgtttaag cacacggttg atgacggcct ggacattaga 3600
aaggcagctt ttgagtgtat gtacacactt ctagacagct gtcttgacag actggatatc 3660
tttgaatttc taaatcatgt ggaagatggt ttgaaggacc attatgatat taagatgcta 3720
acatttttaa tgttggtgag actatctacc ctttgtccaa gtgcagtact acagaggttg 3780
gaccggcttg ttgagccact acgagctacg tgtacaacta aggtgaaggc gaactctgta 3840
aagcaggagt ttgaaaagca ggacgagtta aagcgctcgg ccatgagggc agtagctgca 3900
ctgctcacca ttccagaggc agagaagagc cctctcatga gtgagttcca gtcacagatc 3960
agctccaacc cggagctggc agccatcttt gaaagtatcc agaaagattc ttcgtccact 4020
aacttggaat caatggacac tagttagacg tgtgttccac atgaggacca ttacatttga 4080
ccgcacagtg cactgaaccg acaagttgac catagacacg ggagagaagt gtctagagct 4140
tcaggatgtt ccacttttat tttcattcat gggaagtttg ttcagctttc ttccattgtt 4200
gtttttgtag cttttacttc agaaatctgt atttccataa tccagaggtt gtaagaccac 4260
tgatggtttt agtggttagg gcaacgtttg aaatgggaac taaagtttgg atttatggaa 4320
tgtggaaata gggtccagta tctgtttgcc ctgtaatgtg taggattaaa atgttaaaac 4380
ttt 4383

<210> 46
<211> 220
<212> PRT
<213> Rattus norvegicus

<220>
<223> Chromodomain-helicase-DNA-binding protein

<400> 46
Met Gly Ala Asp Arg Asp Ser Pro Thr Leu Leu Pro Ala Gly Gly Ser
1               5                   10                  15

Asn Trp Leu Ala Gln Arg Phe His Cys Gln Asn Gly Cys Ile Leu Gly
            20                  25                  30

Asp Glu Met Gly Leu Gly Lys Thr Cys Gln Thr Ile Ala Leu Phe Ile
        35                  40                  45

Tyr Leu Ala Gly Arg Leu Asn Asp Glu Gly Pro Phe Leu Ile Leu Cys
    50                  55                  60

Pro Tyr Val Cys Phe Glu Gln Leu Glu Arg Arg Asn Ala Glu Ile Cys
65                  70                  75                  80

Ser Arg Ser Phe Leu Cys Asn Ile Cys Ser Asp Lys Glu Glu Arg Ala
                85                  90                  95

Cys Leu Gln Gln Asp Leu Lys Gln Glu Ser Arg Phe His Val Leu Leu
            100                 105                 110

Thr Thr Tyr Glu Ile Cys Leu Lys Asp Ala Ser Phe Leu Lys Ser Phe
        115                 120                 125

Pro Trp Ser Val Leu Val Val Asp Glu Ala His Arg Leu Lys Asn Gln
    130                 135                 140

Ser Ser Leu Leu His Lys Thr Leu Ser Glu Phe Ser Val Val Phe Ser
145                 150                 155                 160

Leu Leu Leu Thr Gly Thr Pro Ile Gln Asn Ser Leu Gln Glu Leu Tyr
                165                 170                 175

Ser Leu Leu Ser Phe Val Glu Pro Asp Leu Phe Ser Lys Glu Glu Trp
            180                 185                 190

Glu Ile Leu Phe Asn Ala Thr Arg Ile Leu Arg Lys Asn Leu Ser Gln
        195                 200                 205

Gln Val Asn Cys Thr Asn Ser Cys Ser His Phe Cys
    210                 215                 220


<210> 47
<211> 1147
<212> DNA
<213> Rattus norvegicus

<220>
<223> Chromodomain-helicase-DNA-binding protein

<400> 47
ggctactagc cgcgggggcc aagcccctgg cttcttactg cggcttcata ctgagggccg 60
agccgaggcg gcgcgggtgc aggagcagga cttacggcat gggggctgac agggattcac 120
ctacgctctt accagctgga gggagtaact ggctcgccca gcgcttccat tgtcagaatg 180
gctgtatcct gggagatgag atgggcctgg ggaagacctg ccagactatt gctctcttca 240
tttatttggc aggaagatta aatgatgaag ggccatttct gattctttgt ccctatgtct 300
gttttgagca actggaaaga agaaatgcag agatttgctc caggtctttc ctgtgtaaca 360
tatgcagcga caaggaggaa agagcctgcc ttcagcaaga cctgaaacag gagtcacgtt 420
ttcatgtgct actgactacc tatgagattt gcttgaaaga tgcatcattt ctaaaatcat 480
tcccttggag tgttcttgtt gtggatgaag ctcacaggtt gaaaaaccaa agctccctgc 540
tgcataagac cttgtcagag ttctcagtag tcttcagtct cctgttgacc ggaactccca 600
tccagaacag cctccaagag ctctactccc tcctcagttt tgtggagcct gatctctttt 660
ccaaggaaga gtgggagatt ttattcaacg ctaccaggat attgagaaag aatctgagtc 720
agcaagtgaa ctgcacaaac tcttgcagcc atttctgctg agcgagtgaa agctgaggta 780
gctacagagc ttcccaagaa gacagaagta gtgatatacc atggcatgtc cgcattgcag 840
aagaaatact acaaggccat tttgatgaaa gacctagatg catttgaaaa tgagacggca 900
aagaaagtta aactacagaa cattttgtcc cagcttcgaa agtgtgtgga tcacccatat 960
ttgtttgatg gtgtggagcc ggagcctttt gaagttggag accacctgac tgaggctagt 1020
gggaagcttc acctgctgga taagctacta gcattcctgt attctggggg ccatcgggtt 1080
ttacttttct cccaaatgac ccagatgttg gatattctcc aagactatat ggattacaga 1140
ggctacg                                                           1147


<210> 48
<211> 433
<212> PRT
<213> Mus musculus

<220>
<223> MuORC 4

<400> 48
Met Ser Ser Arg Lys Thr Lys Ser Asn Ala His Ala Glu Cys Leu Ser
1               5                   10                  15

Gln Val Gln Arg Ile Leu Arg Glu Arg Phe Cys His His Ser Pro His
            20                  25                  30

Ser Asn Leu Phe Gly Val Gln Val Gln Tyr Lys His Leu Ile Glu Leu
        35                  40                  45

Leu Lys Arg Thr Ala Ile Tyr Gly Glu Ser Asn Ser Val Leu Ile Val
    50                  55                  60

Gly Pro Arg Gly Ser Gly Lys Thr Thr Leu Leu Asn His Ala Leu Lys

65                  70                  75                  80

Glu Leu Met Glu Ile Glu Val Ser Glu Asn Val Ile Gln Val His Leu
                85                  90                  95

Asn Gly Leu Leu Gln Thr Asn Glu Lys Ile Ala Leu Lys Glu Ile Thr
            100                 105                 110

Arg Gln Leu Asn Leu Asp Asn Val Val Glu Asp Lys Val Phe Gly Ser
        115                 120                 125

Phe Ala Glu Asn Leu Ser Phe Leu Leu Glu Ala Leu Gln Lys Gly Asp
    130                 135                 140

Arg Thr Ser Ser Cys Pro Val Ile Phe Ile Leu Asp Glu Phe Asp Ile
145                 150                 155                 160

Phe Ala His Gln Lys Asn Gln Thr Leu Leu Tyr Asn Leu Phe Asp Ile
                165                 170                 175

Ser Gln Ser Ala Gln Thr Pro Val Ala Val Ile Gly Leu Thr Cys Arg
            180                 185                 190

Leu Asp Ile Leu Glu Leu Leu Glu Lys Arg Val Lys Ser Arg Phe Ser
        195                 200                 205

His Arg Gln Ile His Leu Met Asn Ser Phe Asp Phe Pro Gln Tyr Leu
    210                 215                 220

Lys Ile Phe Lys Glu Gln Leu Ser Leu Pro Ala Glu Phe Pro Asp Lys
225                 230                 235                 240

Ala Phe Ala Glu Arg Trp Asn Glu Asn Val His Cys Leu Ser Glu Asp
                245                 250                 255

Ser Thr Val Leu Glu Val Leu Gln Lys His Phe Ser Val Asn Lys Asn
            260                 265                 270

Leu Gln Ser Leu His Met Leu Leu Met Leu Ala Leu Asn Arg Val Thr
        275                 280                 285

Val Ser His Pro Phe Met Thr Ser Ala Asp Leu Met Glu Ala Gln His
    290                 295                 300

Met Cys Ser Leu Asp Ser Lys Ala Asn Ile Val His Gly Leu Ser Val
305                 310                 315                 320

Leu Glu Ile Cys Leu Ile Ile Ala Met Lys His Leu Asn Asp Ile Tyr
                325                 330                 335

Glu Glu Glu Pro Phe Asn Phe Gln Met Val Tyr Asn Glu Phe Gln Lys
            340                 345                 350

Phe Ile Gln Arg Lys Ala His Ser Val Tyr Asn Phe Glu Lys Pro Val
        355                 360                 365

Val Met Lys Ala Phe Glu His Leu Gln Gln Leu Glu Leu Ile Lys Pro
    370                 375                 380

Val Glu Arg Thr Ser Val Asn Ser Gln Arg Glu Tyr Gln Leu Val Lys
385                 390                 395                 400

Leu Leu Leu Asp Asn Thr Gln Ile Met Asn Ala Leu Gln Lys Tyr Ser
                405                 410                 415

Asn Cys Pro Thr Asp Val Arg Gln Trp Ala Thr Ser Ser Leu Ser Trp
            420                 425                 430

Leu

<210> 49
<211> 1654
<212> DNA
<213> Mus musculus

<220>
<223> MuORC 4

<400> 49
tgcggtgggc tgcagtagga gcagcgggac tgagaaggtg gtgaacatcc ttcggtcctc 60
accgtcggcg gcggccggct ttgcccgatt taggcaggct tgactgggag aggatttgaa 120
ttaatcgaga tgagcagtcg taaaaccaag agtaatgcac atgcagagtg tctttcacag 180
gtacaaagaa ttttacgtga aagattttgt catcatagtc cacatagcaa cctctttgga 240
gtgcaagtac aatacaagca tttgattgag ctactcaaaa gaactgctat ctatggagaa 300
agcaattctg tactcattgt tggaccacga ggatcaggaa agaccacatt attaaatcat 360
gctttgaaag aactcatgga aatagaagtg agtgaaaatg tgatacaagt tcacttaaat 420
ggactcctgc agacaaatga aaaaattgct ctgaaagaga tcacaagaca attaaatcta 480
gacaatgtag ttgaagataa agtttttgga agctttgctg aaaacctttc atttcttctg 540
gaagctttgc aaaaaggtga ccggactagc agttgcccag tgatcttcat actggatgaa 600
tttgatattt ttgctcatca gaaaaatcaa acactccttt ataatctttt tgacatttct 660
caatctgcac agacaccagt agcagttatt ggacttacat gtagattgga tattttggaa 720
ctcttagaaa agagagtgaa gtcacgattt tctcaccggc agatacattt aatgaattca 780
tttgattttc cacaatattt gaaaatattt aaagaacagt tatctctacc tgcagaattc 840
ccagataagg cttttgctga gagatggaat gagaatgttc actgtctctc tgaagattca 900
actgtgcttg aagtcctaca gaaacatttc agtgtcaaca aaaacttgca gtcattacac 960
atgctattga tgcttgcttt aaatcgagta accgtatcac acccatttat gacttcagca 1020
gatctgatgg aggcacagca tatgtgtagc ttggattcta aggcgaatat tgtacatggt 1080
ctgtcagtct tggaaatctg tcttataata gcaatgaaac atttaaatga catatatgaa 1140
gaggagccct ttaattttca aatggtgtat aatgaatttc agaaattcat tcaaagaaag 1200
gcccattctg tttataactt tgagaaacct gtggtcatga aggcatttga gcacttacaa 1260
cagttggaat taataaaacc cgtggaaaga acttcagtaa attctcagag agaataccag 1320
ctagtgaaac tacttttgga taatactcaa attatgaatg ctctacagaa atactccaac 1380
tgccctacag atgttaggca gtgggcaaca tcctcactaa gctggctgtg aagattttaa 1440
gtacatttct gtgaagacct taaagtactg ttctttattt aataagatac gctactacat 1500
tgctttgttt ataaatagtt tatgggaaat ttgcacttag tatgttttga ataatgacca 1560
cgcatggaca cagtttcatt aatttaaatt gcaccacaca agtagcagtt cataagaata 1620
aaatgtgctt attttaaaaa aaaaaaaaaa aaaa                              1654

<210> 50
<211> 1378
<212> PRT
<213> Rattus norvegicus

<220>
<223> Carboxypeptidase D precursor

<400> 50
Met Ala Ser Gly Trp Asp Glu Arg Pro Pro Trp Arg Leu Glu Ser Leu
1               5                   10                  15

Arg Leu Leu Pro Pro Pro Pro Leu Leu Leu Leu Leu Leu Leu Leu Arg
            20                  25                  30

Ser Ser Ala Gln Ala Ala His Ile Lys Lys Ala Glu Ala Thr Thr Thr
        35                  40                  45

Thr Val Gly Gly Ser Glu Ala Ala Glu Gly Gln Phe Asp His Tyr Tyr
    50                  55                  60

His Glu Ala Ala Leu Gly Glu Ala Leu Glu Ala Ala Ala Ala Ala Gly
65                  70                  75                  80

Pro Pro Gly Leu Ala Arg Leu Phe Ser Ile Gly Asn Ser Val Glu Gly
                85                  90                  95

Arg Pro Leu Trp Val Leu Arg Leu Thr Ala Gly Leu Gly Pro Pro Pro
            100                 105                 110

Thr Pro Ala Ala Val Gly Leu Asp Ala Ala Gly Pro Leu Leu Pro Gly
        115                 120                 125

Arg Pro Gln Val Lys Leu Val Gly Asn Met His Gly Asp Glu Thr Val
    130                 135                 140

Ser Arg Gln Val Leu Val Tyr Leu Ala Arg Glu Leu Ala Ser Gly Tyr
145                 150                 155                 160

Arg Arg Gly Asp Pro Arg Leu Val Arg Leu Leu Asn Thr Thr Asp Val
                165                 170                 175

Tyr Leu Leu Pro Ser Leu Asn Pro Asp Gly Phe Glu Arg Ala Arg Glu
            180                 185                 190

Gly Asp Cys Gly Leu Gly Asp Ser Gly Pro Pro Gly Thr Ser Gly Arg
        195                 200                 205

Asp Asn Ser Arg Gly Arg Asp Leu Asn Arg Ser Phe Pro Asp Gln Phe
    210                 215                 220

Ser Thr Gly Glu Pro Pro Ser Leu Asp Glu Val Pro Glu Val Arg Ala
225                 230                 235                 240

Leu Ile Asp Trp Ile Arg Arg Asn Lys Phe Val Leu Ser Gly Asn Leu
                245                 250                 255

His Gly Gly Ser Val Val Ala Ser Tyr Pro Phe Asp Asp Ser Pro Glu
            260                 265                 270

His Lys Thr Thr Gly Ile Tyr Ser Lys Thr Ser Asp Asp Glu Val Phe
        275                 280                 285

Arg Tyr Leu Ala Lys Ala Tyr Ala Ser Asn His Pro Ile Met Arg Thr
    290                 295                 300

Gly Glu Pro His Cys Pro Gly Asp Glu Glu Glu Thr Phe Lys Asp Gly
305                 310                 315                 320

Ile Thr Asn Gly Ala His Trp Tyr Asp Val Glu Gly Gly Met Gln Asp
                325                 330                 335

Tyr Asn Tyr Val Trp Ala Asn Cys Phe Glu Ile Thr Leu Glu Leu Ser
            340                 345                 350

Cys Cys Lys Tyr Pro Pro Ala Ser Gln Leu Arg Gln Glu Trp Glu Asn
        355                 360                 365

Asn Arg Glu Ser Leu Ile Thr Leu Ile Glu Lys Val His Ile Gly Ile
    370                 375                 380

Lys Gly Phe Val Lys Asp Ser Val Thr Gly Ser Gly Leu Glu Asn Ala
385                 390                 395                 400

Thr Ile Ser Val Ala Gly Ile Asn His Asn Ile Thr Thr Gly Arg Phe
                405                 410                 415

Gly Asp Phe His Arg Leu Leu Ile Pro Gly Ser Tyr Asn Leu Thr Ala
            420                 425                 430

Val Ser Pro Gly Tyr Met Pro Leu Thr Ile Asn Asn Ile Val Val Lys
        435                 440                 445

Glu Gly Pro Ala Thr Glu Ile Asp Phe Ser Leu Gln Pro Thr Val Met
    450                 455                 460

Ser Val Val Pro Asp Ser Thr Glu Ala Val Thr Thr Pro Gly Thr Val
465                 470                 475                 480

Ala Val Pro Asn Ile Pro Pro Gly Thr Pro Ser Ser His Gln Pro Ile
                485                 490                 495

Gln Pro Lys Asp Phe His His His His Phe Pro Asp Met Glu Ile Phe
            500                 505                 510

Leu Arg Arg Phe Ala Asn Glu Tyr Pro Asn Ile Thr Arg Leu Tyr Ser
        515                 520                 525

Leu Gly Lys Ser Val Glu Ser Arg Glu Leu Tyr Val Met Glu Ile Ser
    530                 535                 540

Asp Asn Pro Gly Val His Glu Pro Gly Glu Pro Glu Phe Lys Tyr Ile
545                 550                 555                 560

Gly Asn Met His Gly Asn Glu Val Val Gly Arg Glu Leu Leu Leu Asn
                565                 570                 575

Leu Ile Glu Tyr Leu Cys Lys Asn Phe Gly Thr Asp Pro Glu Val Thr
            580                 585                 590

Asp Leu Val Arg Ser Thr Arg Ile His Leu Met Pro Ser Met Asn Pro
        595                 600                 605

Asp Gly Tyr Glu Lys Ser Gln Glu Gly Asp Ser Ile Ser Val Val Gly
    610                 615                 620

Arg Asn Asn Ser Asn Asn Phe Asp Leu Asn Arg Asn Phe Pro Asp Gln
625                 630                 635                 640

Phe Val Pro Ile Thr Asp Pro Thr Gln Pro Glu Thr Ile Ala Val Met
                645                 650                 655

Ser Trp Val Lys Ala Tyr Pro Phe Val Leu Ser Ala Asn Leu His Gly

660                 665                 670

Gly Ser Leu Val Val Asn Tyr Pro Tyr Asp Asp Asn Glu Gln Gly Val
        675                 680                 685

Ala Thr Tyr Ser Lys Ser Pro Asp Asp Ala Val Phe Gln Gln Ile Ala
    690                 695                 700

Leu Ser Tyr Ser Lys Glu Asn Ser Gln Met Phe Gln Gly Arg Pro Cys
705                 710                 715                 720

Lys Asp Met Tyr Leu Asn Glu Tyr Phe Pro His Gly Ile Thr Asn Gly
                725                 730                 735

Ala Ser Trp Tyr Asn Val Pro Gly Gly Met Gln Asp Trp Asn Tyr Leu
            740                 745                 750

Gln Thr Asn Cys Phe Glu Val Thr Ile Glu Leu Gly Cys Val Lys Tyr
        755                 760                 765

Pro Phe Glu Lys Glu Leu Pro Lys Tyr Trp Glu Gln Asn Arg Arg Ser
    770                 775                 780

Leu Ile Gln Phe Met Lys Gln Val His Gln Gly Val Lys Gly Phe Val
785                 790                 795                 800

Leu Asp Ala Thr Asp Gly Arg Gly Ile Leu Asn Ala Thr Leu Ser Val
                805                 810                 815

Ala Glu Ile Asn His Pro Val Thr Thr Tyr Lys Ala Gly Asp Tyr Trp
            820                 825                 830

Arg Leu Leu Val Pro Gly Thr Tyr Lys Ile Thr Ala Ser Ala Arg Gly
        835                 840                 845

Tyr Asn Pro Val Thr Lys Asn Val Thr Val Arg Ser Glu Gly Ala Ile
    850                 855                 860

Gln Val Asn Phe Thr Leu Val Arg Ser Ser Thr Asp Ala Asn Asn Glu
865                 870                 875                 880

Ser Lys Lys Gly Lys Gly His Ser Thr Ser Thr Asp Asp Thr Ser Asp
                885                 890                 895

Pro Thr Ser Lys Glu Phe Glu Ala Leu Ile Lys His Leu Ser Ala Glu
            900                 905                 910

Asn Gly Leu Glu Gly Phe Met Leu Ser Ser Ser Ser Asp Leu Ala Leu
        915                 920                 925

Tyr Arg Tyr His Ser Tyr Lys Asp Leu Ser Glu Phe Leu Arg Gly Leu
    930                 935                 940

Val Met Asn Tyr Pro His Ile Thr Asn Leu Thr Thr Leu Gly Gln Ser
945                 950                 955                 960

Val Glu Tyr Arg His Ile Trp Ser Leu Glu Ile Ser Asn Lys Pro Asn
                965                 970                 975

Ile Ser Glu Pro Glu Glu Pro Lys Ile Arg Phe Val Ala Gly Ile His
            980                 985                 990

Gly Asn Ala Pro Val Gly Thr Glu Leu Leu Leu Ala Leu Ala Glu Phe
        995                 1000                1005

Leu Cys Leu Asn Tyr Lys Lys Asn Pro Val Val Thr Gln Leu Val Asp
    1010                1015                1020

Arg Thr Arg Ile Val Ile Val Pro Ser Leu Asn Pro Asp Gly Arg Glu
1025                1030                1035                1040

Arg Ala Gln Glu Lys Asp Cys Thr Ser Lys Thr Gly His Thr Asn Ala
                1045                1050                1055

Arg Gly Arg Asp Leu Asp Thr Asp Phe Thr Ser Asn Ala Ser Gln Pro
            1060                1065                1070

Glu Thr Lys Ala Ile Ile Glu Asn Leu Ile Gln Lys Gln Asp Phe Ser
        1075                1080                1085

Leu Ser Ile Ala Leu Asp Gly Gly Ser Val Leu Val Thr Tyr Pro Tyr
    1090                1095                1100

Asp Lys Pro Val Gln Thr Val Glu Asn Lys Glu Thr Leu Lys His Leu
1105                1110                1115                1120

Ala Ser Leu Tyr Ala Asn Asn His Pro Ser Met His Met Gly Gln Pro
                1125                1130                1135

Ser Cys Pro Asn Asn Ser Asp Glu Asn Ile Pro Gly Gly Val Met Arg
            1140                1145                1150

Gly Ala Glu Trp His Ser His Leu Gly Ser Met Lys Asp Tyr Ser Val
        1155                1160                1165

Thr Tyr Gly His Cys Pro Glu Ile Thr Val Tyr Thr Ser Cys Cys Tyr
    1170                1175                1180

Phe Pro Ser Ala Ala Gln Leu Pro Ala Leu Trp Ala Glu Asn Lys Lys
1185                1190                1195                1200

Ser Leu Leu Ser Met Leu Val Glu Val His Lys Gly Val His Gly Leu
                1205                1210                1215

Val Lys Asp Lys Thr Gly Lys Pro Ile Ser Lys Ala Val Ile Val Leu
            1220                1225                1230

Asn Glu Gly Ile Arg Val His Thr Lys Glu Gly Gly Tyr Phe His Val
        1235                1240                1245

Leu Leu Ala Pro Gly Val His Asn Ile Asn Ala Ile Ala Asp Gly Tyr
    1250                1255                1260

Gln Gln Gln His Ser Gln Val Phe Val His His Asp Ala Ala Ser Ser
1265                1270                1275                1280

Val Val Ile Val Phe Asp Thr Asp Asn Arg Ile Phe Gly Leu Pro Arg
                1285                1290                1295

Glu Leu Val Val Thr Val Ser Gly Ala Thr Met Ser Ala Leu Ile Leu
            1300                1305                1310

Thr Ala Cys Ile Ile Trp Cys Ile Cys Ser Ile Lys Ser Asn Arg His
      1315                1320                1325

Lys Asp Gly Phe His Arg Leu Arg Gln His His Asp Glu Tyr Glu Asp
   1330                1335                1340

Glu Ile Arg Met Met Ser Thr Gly Ser Lys Lys Ser Leu Leu Ser His
1345                1350                1355                1360

Glu Phe Gln Asp Glu Thr Asp Thr Glu Glu Glu Thr Leu Tyr Ser Ser
                1365                1370                1375

Lys His

<210> 51
<211> 4377
<212> DNA
<213> Rattus norvegicus

<220>
<223> Carboxypeptidase D precursor

<400> 51
tgcggagccg ctggagcgga gcggtgttag cggcgttgct ggaagatggc gagcggctgg 60
gatgagcggc cgccctggcg gctggaaagc ctgcggctgc taccgccgcc accgctgctg 120
ctgctactgc tgctattgag gagctcggcc caggcggcgc acatcaagaa ggctgaggcg 180
accacgacga ccgtcggcgg ctccgaggcg gccgagggcc agtttgacca ctactaccac 240
gaagcggcgc tgggcgaggc actggaggcg gcagcggccg cggggccgcc tggcctggcc 300
cgtctcttca gcatcggcaa ttcggtggag ggccgaccgc tgtgggtgct gcgcctcacg 360
gctggcctgg ggccgccgcc cacccccgcc gccgtggggc tggacgccgc ggggccgctg 420
ctgcccggcc gaccgcaggt gaagctggtg ggtaacatgc acggcgacga gacggtgtcg 480
cgccaggtgt tggtctacct ggcccgtgag ctggcgtcgg gctaccgccg cggggacccg 540
cgcctggtcc gcctgctcaa caccaccgac gtatatttgc tgcccagcct gaatccggat 600
ggcttcgaac gcgctcgcga gggtgactgc ggcctcggcg acagcggtcc gcctgggacc 660
agcggccgag acaacagccg aggccgcgac ctcaaccgca gcttccctga ccagttcagc 720
acgggggagc caccttccct ggatgaggtg ccagaggtgc gcgccctcat cgactggatc 780
cgcaggaaca agtttgtgct ttctggaaat cttcatggtg gctcagtggt agcaagctat 840
ccttttgatg attctcccga gcataagacc actggaatct atagcaaaac ctccgatgat 900
gaagtctttc gatacttggc gaaagcttat gcttctaacc accctataat gagaactggt 960
gaaccccatt gtccaggaga tgaagaggag actttcaaag acgggatcac gaacggtgcg 1020
cactggtatg atgtggaagg tggtatgcaa gattacaact atgtgtgggc caactgtttt 1080
gagatcacat tagaattatc ttgctgcaaa tacccacctg cttctcagct tcgacaagaa 1140
tgggagaaca accgtgagtc tttgatcaca ttgattgaaa aggtccacat tggaattaaa 1200
ggatttgtta aagactcagt cacaggctct gggttggaga atgcaaccat ctcagtggct 1260
ggtattaatc ataatatcac aactgggaga ttcggtgatt tccacagact gctcattcct 1320
ggaagttaca atcttacagc ggtttcacct gggtatatgc cgttgactat taataatata 1380
gtggtgaaag aaggaccagc cacagagata gatttctccc ttcaaccaac tgtgatgtca 1440
gtagtgcctg actcgactga ggctgtgaca actcccggca cagttgccgt tcctaatatt 1500
cctcctggaa caccatcgtc ccatcagcca attcagccta aagactttca ccaccaccat 1560
tttcctgaca tggaaatctt cttgagaaga tttgccaatg aatatcctaa catcactcgc 1620
ctttattctc tgggaaagtc cgtagagtcg agagaactgt atgtgatgga gatatctgac 1680
aacccaggcg tccatgaacc aggtgaacca gaatttaaat atattggaaa tatgcatgga 1740
aatgaagtgg ttggaagaga actgctattg aatctcatcg aatacctctg caagaacttt 1800
ggaacagacc ctgaagtaac agatttggtt cgcagcacta gaattcacct catgccatcc 1860
atgaatcctg atgggtatga gaagtcccaa gaaggagact cgataagtgt agttggcaga 1920
aacaacagca acaactttga cctaaaccga aatttcccag accagtttgt tccgatcaca 1980
gatcctaccc aacctgaaac tatcgctgtg atgagctggg tgaaggccta tccgtttgtg 2040
ctgtcggcca atctgcatgg aggttccttg gtggttaact acccttatga tgataatgaa 2100
caaggagttg ccacatacag caaatcacca gatgacgccg tgtttcaaca aatagcactt 2160

tcttattcca aggaaaactc gcagatgttc caaggtagac cttgcaaaga catgtacctt 2220
aatgagtatt ttcctcatgg cataacgaat ggagcgagtt ggtataatgt cccaggtggt 2280
atgcaggact ggaactattt acagacgaat tgctttgaag taaccatcga actaggttgt 2340
gtgaaatatc cattcgagaa ggaactgcca aagtactggg agcaaaaccg gagatctcta 2400
atccagttta tgaaacaggt tcatcagggt gtcaaaggat ttgttcttga tgccacagat 2460
ggtaggggca tattaaatgc caccctcagt gttgctgaaa ttaatcaccc agtgacgact 2520
tacaaagctg gagattactg gcgtctcttg gtcccaggga catataaaat cacagcatct 2580
gctcgagggt ataaccccgt caccaagaac gtgaccgtca ggagtgaagg tgctattcag 2640
gtcaacttca cacttgttcg atcttcaaca gatgcaaaca atgagtcaaa gaaaggaaag 2700
gggcatagca ccagcactga tgacaccagc gacccaactt ctaaggagtt tgaagcactg 2760
atcaaacacc tttcggctga gaatggcttg gaaggcttca tgttaagctc gtcctcagac 2820
ctggctctgt atcgatacca ttcctacaaa gacttatccg agttcctgag aggactcgta 2880
atgaactacc cacacattac aaaccttacc actttgggac agagtgttga atatcgtcac 2940
atttggtccc ttgaaatctc caataagccc aacatatcag aacctgaaga accgaagatt 3000
cgttttgttg ctggtatcca tggaaacgct ccagttggca ctgaactgct tttggctctg 3060
gcagaatttc tttgcctgaa ttacaaaaag aatccagttg ttacccaatt ggttgacagg 3120
actagaattg tgattgtccc ctctctgaat ccagatggga gagagcgagc acaggagaaa 3180
gactgtacct cgaagactgg gcacacaaat gcccgtggca gggacctgga cacagacttc 3240
acaagtaatg cttcccaacc tgagaccaaa gccatcattg aaaacttgat tcaaaaacag 3300
gatttcagcc tttccattgc tttagatggt ggttctgtgc tggtcacata tccgtacgac 3360
aagccagtcc agacagtgga aaacaaagaa acgttaaagc atctggcgtc tctttatgca 3420
aataaccacc catcaatgca catgggtcag cccagttgcc caaataattc agatgagaac 3480
ataccaggag gagtgatgcg gggagcagag tggcacagtc acctgggcag catgaaggat 3540
tatagtgtca cctatggcca ttgcccagaa atcacagtat acacaagctg ctgctacttc 3600
ccaagtgcag ctcaactccc tgccctatgg gcagagaaca agaagtctct cctcagcatg 3660
ctggtggagg tccacaaagg agtccatggg ttagtaaaag acaagactgg taaaccaatc 3720
tctaaagcag taattgtact caatgaagga ataagagtac acacaaaaga ggggggttat 3780
ttccatgtgc ttttagctcc tggggtccat aacatcaatg ccatcgctga tggatatcag 3840
caacagcatt cacaggtctt tgtgcatcat gatgcagcca gttctgtggt gatagtcttt 3900
gatacagata accggatatt tggtttgcca agggaacttg tggtaactgt gtcaggtgcc 3960
accatgtcag cgctgatcct cacggcttgc atcatttggt gcatctgctc tatcaagtct 4020
aacagacaca aagatggctt ccaccggctg cggcagcatc atgatgaata tgaagatgaa 4080
attcgcatga tgtcaactgg ctctaagaag tcccttttaa gccatgagtt ccaggatgaa 4140
acggacacag aggaagagac attatattct agcaaacatt gagaagcacg gtttgcatat 4200
ctccctgctt aagtaccagg caaatgacag ctcctcttgg gagacactgc agtaagaaga 4260
gagattcctt cctccttcaa agagctttgg gaagttagcc tgctaaattc atactctctg 4320
aatgtcacag gcagttttga acttcccttc cttaaaagta ctcttaacct tttcctt 4377

<210> 52
<211> 452
<212> PRT
<213> Rattus norvegicus

<220>
<223> Ich-1

<400> 52
Met Ala Ala Ser Ser Gly Arg Ser Gln Ser Ser Leu His Arg Lys Gly
1               5                   10                  15

Leu Met Ala Ala Asp Arg Arg Ser Arg Ile Leu Ala Val Cys Gly Met
            20                  25                  30

His Pro Asp His Gln Glu Thr Leu Lys Lys Asn Arg Val Val Leu Ala
        35                  40                  45

Lys Gln Leu Leu Leu Ser Glu Leu Leu Glu His Leu Leu Glu Lys Asp
    50                  55                  60

Ile Ile Thr Leu Glu Met Arg Glu Leu Ile Gln Ala Lys Gly Gly Ser

65                  70                  75                  80

Phe Ser Gln Asn Val Glu Leu Leu Asn Leu Leu Pro Lys Arg Gly Pro
                85                  90                  95

Gln Ala Phe Asp Ala Phe Cys Glu Ala Leu Arg Glu Thr Arg Gln Gly
            100                 105                 110

His Leu Glu Asp Leu Leu Leu Thr Thr Leu Ser Asp Ile Gln His Ile
        115                 120                 125

Leu Pro Pro Leu Ser Cys Asp Tyr Asp Ser Ser Leu Pro Phe Ser Val
    130                 135                 140

Cys Glu Ser Cys Pro Pro His Lys Gln Ser Arg Leu Ser Thr Asp Thr
145                 150                 155                 160

Met Glu His Ser Leu Asp Asn Gly Asp Gly Pro Pro Cys Leu Gln Val
                165                 170                 175

Lys Pro Cys Thr Pro Glu Phe Tyr Gln Ala His Tyr Gln Leu Ala Tyr
            180                 185                 190

Arg Leu Gln Ser Gln Pro Arg Gly Leu Ala Leu Val Met Ser Asn Val
        195                 200                 205

His Phe Thr Gly Glu Lys Asp Leu Glu Phe Arg Ser Gly Gly Asp Val
    210                 215                 220

Asp His Thr Thr Leu Val Thr Leu Phe Lys Leu Leu Gly Tyr Asn Val
225                 230                 235                 240

His Val Leu Tyr Asp Gln Thr Ala Gln Glu Met Gln Glu Lys Leu Gln
                245                 250                 255

Asn Phe Ala Gln Leu Pro Ala His Arg Val Thr Asp Ser Cys Ile Val
            260                 265                 270

Ala Leu Leu Ser His Gly Val Glu Gly Gly Ile Tyr Gly Val Asp Gly
        275                 280                 285

Lys Leu Leu Gln Leu Gln Glu Val Phe Arg Leu Phe Asp Asn Ala Asn
    290                 295                 300

Cys Pro Ser Leu Gln Asn Lys Pro Lys Met Phe Phe Ile Gln Ala Cys
305                 310                 315                 320

Arg Gly Asp Glu Thr Asp Arg Gly Val Asp Gln Gln Asp Gly Lys Asn
                325                 330                 335

His Ala Gln Ser Pro Gly Cys Glu Glu Ser Asp Ala Gly Lys Glu Glu
            340                 345                 350

Leu Met Lys Met Arg Leu Pro Thr Arg Ser Asp Met Ile Cys Gly Tyr
        355                 360                 365

Ala Cys Leu Lys Gly Asn Ala Ala Met Arg Asn Thr Lys Arg Gly Ser
    370                 375                 380

Trp Tyr Ile Glu Ala Leu Thr Gln Val Phe Ser Glu Arg Ala Cys Asp
385                 390                 395                 400

Met His Val Ala Asp Met Leu Val Lys Val Asn Ala Leu Ile Lys Glu
                405                 410                 415

Arg Glu Gly Tyr Ala Pro Gly Thr Glu Phe His Arg Cys Lys Glu Met
            420                 425                 430

Ser Glu Tyr Cys Ser Thr Leu Cys Gln Gln Leu Tyr Leu Phe Pro Gly
        435                 440                 445

Tyr Pro Pro Thr
    450


<210> 53
<211> 4377
<212> DNA
<213> Rattus norvegicus

<220>
<223> Ich-1

<400> 53
tgcggagccg ctggagcgga gcggtgttag cggcgttgct ggaagatggc gagcggctgg 60
gatgagcggc cgccctggcg gctggaaagc ctgcggctgc taccgccgcc accgctgctg 120
ctgctactgc tgctattgag gagctcggcc caggcggcgc acatcaagaa ggctgaggcg 180
accacgacga ccgtcggcgg ctccgaggcg gccgagggcc agtttgacca ctactaccac 240
gaagcggcgc tgggcgaggc actggaggcg gcagcggccg cggggccgcc tggcctggcc 300
cgtctcttca gcatcggcaa ttcggtggag ggccgaccgc tgtgggtgct gcgcctcacg 360
gctggcctgg ggccgccgcc cacccccgcc gccgtggggc tggacgccgc ggggccgctg 420
ctgcccggcc gaccgcaggt gaagctggtg ggtaacatgc acggcgacga gacggtgtcg 480
cgccaggtgt tggtctacct ggcccgtgag ctggcgtcgg gctaccgccg cggggacccg 540
cgcctggtcc gcctgctcaa caccaccgac gtatatttgc tgcccagcct gaatccggat 600
ggcttcgaac gcgctcgcga gggtgactgc ggcctcggcg acagcggtcc gcctgggacc 660
agcggccgag acaacagccg aggccgcgac ctcaaccgca gcttccctga ccagttcagc 720
acgggggagc caccttccct ggatgaggtg ccagaggtgc gcgccctcat cgactggatc 780
cgcaggaaca agtttgtgct ttctggaaat cttcatggtg gctcagtggt agcaagctat 840
ccttttgatg attctcccga gcataagacc actggaatct atagcaaaac ctccgatgat 900
gaagtctttc gatacttggc gaaagcttat gcttctaacc accctataat gagaactggt 960
gaaccccatt gtccaggaga tgaagaggag actttcaaag acgggatcac gaacggtgcg 1020
cactggtatg atgtggaagg tggtatgcaa gattacaact atgtgtgggc caactgtttt 1080
gagatcacat tagaattatc ttgctgcaaa tacccacctg cttctcagct tcgacaagaa 1140
tgggagaaca accgtgagtc tttgatcaca ttgattgaaa aggtccacat tggaattaaa 1200
ggatttgtta aagactcagt cacaggctct gggttggaga atgcaaccat ctcagtggct 1260
ggtattaatc ataatatcac aactgggaga ttcggtgatt tccacagact gctcattcct 1320
ggaagttaca atcttacagc ggtttcacct gggtatatgc cgttgactat taataatata 1380
gtggtgaaag aaggaccagc cacagagata gatttctccc ttcaaccaac tgtgatgtca 1440
gtagtgcctg actcgactga ggctgtgaca actcccggca cagttgccgt tcctaatatt 1500
cctcctggaa caccatcgtc ccatcagcca attcagccta aagactttca ccaccaccat 1560
tttcctgaca tggaaatctt cttgagaaga tttgccaatg aatatcctaa catcactcgc 1620
ctttattctc tgggaaagtc cgtagagtcg agagaactgt atgtgatgga gatatctgac 1680
aacccaggcg tccatgaacc aggtgaacca gaatttaaat atattggaaa tatgcatgga 1740
aatgaagtgg ttggaagaga actgctattg aatctcatcg aatacctctg caagaacttt 1800
ggaacagacc ctgaagtaac agatttggtt cgcagcacta gaattcacct catgccatcc 1860
atgaatcctg atgggtatga gaagtcccaa gaaggagact cgataagtgt agttggcaga 1920
aacaacagca acaactttga cctaaaccga aatttcccag accagtttgt tccgatcaca 1980
gatcctaccc aacctgaaac tatcgctgtg atgagctggg tgaaggccta tccgtttgtg 2040
ctgtcggcca atctgcatgg aggttccttg gtggttaact acccttatga tgataatgaa 2100
caaggagttg ccacatacag caaatcacca gatgacgccg tgtttcaaca aatagcactt 2160
tcttattcca aggaaaactc gcagatgttc caaggtagac cttgcaaaga catgtacctt 2220
aatgagtatt ttcctcatgg cataacgaat ggagcgagtt ggtataatgt cccaggtggt 2280

atgcaggact ggaactattt acagacgaat tgctttgaag taaccatcga actaggttgt 2340
gtgaaatatc cattcgagaa ggaactgcca aagtactggg agcaaaaccg gagatctcta 2400
atccagttta tgaaacaggt tcatcagggt gtcaaaggat ttgttcttga tgccacagat 2460
ggtaggggca tattaaatgc caccctcagt gttgctgaaa ttaatcaccc agtgacgact 2520
tacaaagctg gagattactg gcgtctcttg gtcccaggga catataaaat cacagcatct 2580
gctcgagggt ataaccccgt caccaagaac gtgaccgtca ggagtgaagg tgctattcag 2640
gtcaacttca cacttgttcg atcttcaaca gatgcaaaca atgagtcaaa gaaaggaaag 2700
gggcatagca ccagcactga tgacaccagc gacccaactt ctaaggagtt tgaagcactg 2760
atcaaacacc tttcggctga gaatggcttg gaaggcttca tgttaagctc gtcctcagac 2820
ctggctctgt atcgatacca ttcctacaaa gacttatccg agttcctgag aggactcgta 2880
atgaactacc cacacattac aaaccttacc actttgggac agagtgttga atatcgtcac 2940
atttggtccc ttgaaatctc caataagccc aacatatcag aacctgaaga accgaagatt 3000
cgttttgttg ctggtatcca tggaaacgct ccagttggca ctgaactgct tttggctctg 3060
gcagaatttc tttgcctgaa ttacaaaaag aatccagttg ttacccaatt ggttgacagg 3120
actagaattg tgattgtccc ctctctgaat ccagatggga gagagcgagc acaggagaaa 3180
gactgtacct cgaagactgg gcacacaaat gcccgtggca gggacctgga cacagacttc 3240
acaagtaatg cttcccaacc tgagaccaaa gccatcattg aaaacttgat tcaaaaacag 3300
gatttcagcc tttccattgc tttagatggt ggttctgtgc tggtcacata tccgtacgac 3360
aagccagtcc agacagtgga aaacaaagaa acgttaaagc atctggcgtc tctttatgca 3420
aataaccacc catcaatgca catgggtcag cccagttgcc caaataattc agatgagaac 3480
ataccaggag gagtgatgcg gggagcagag tggcacagtc acctgggcag catgaaggat 3540
tatagtgtca cctatggcca ttgcccagaa atcacagtat acacaagctg ctgctacttc 3600
ccaagtgcag ctcaactccc tgccctatgg gcagagaaca agaagtctct cctcagcatg 3660
ctggtggagg tccacaaagg agtccatggg ttagtaaaag acaagactgg taaaccaatc 3720
tctaaagcag taattgtact caatgaagga ataagagtac acacaaaaga ggggggttat 3780
ttccatgtgc ttttagctcc tggggtccat aacatcaatg ccatcgctga tggatatcag 3840
caacagcatt cacaggtctt tgtgcatcat gatgcagcca gttctgtggt gatagtcttt 3900
gatacagata accggatatt tggtttgcca agggaacttg tggtaactgt gtcaggtgcc 3960
accatgtcag cgctgatcct cacggcttgc atcatttggt gcatctgctc tatcaagtct 4020
aacagacaca aagatggctt ccaccggctg cggcagcatc atgatgaata tgaagatgaa 4080
attcgcatga tgtcaactgg ctctaagaag tcccttttaa gccatgagtt ccaggatgaa 4140
acggacacag aggaagagac attatattct agcaaacatt gagaagcacg gtttgcatat 4200
ctccctgctt aagtaccagg caaatgacag ctcctcttgg gagacactgc agtaagaaga 4260
gagattcctt cctccttcaa agagctttgg gaagttagcc tgctaaattc atactctctg 4320
aatgtcacag gcagttttga acttcccttc cttaaaagta ctcttaacct tttcctt 4377

<210> 54
<211> 339
<212> PRT
<213> Rattus norvegicus

<220>
<223> Cathepsin B

<400> 54
Met Trp Trp Ser Leu Ile Pro Leu Ser Cys Leu Leu Ala Leu Thr Ser
1               5                   10                  15

Ala His Asp Lys Pro Ser Ser His Pro Leu Ser Asp Asp Met Ile Asn
            20                  25                  30

Tyr Ile Asn Lys Gln Asn Thr Thr Trp Gln Ala Gly Arg Asn Phe Tyr
        35                  40                  45

Asn Val Asp Ile Ser Tyr Leu Lys Lys Leu Cys Gly Thr Val Leu Gly
    50                  55                  60

Gly Pro Asn Leu Pro Glu Arg Val Gly Phe Ser Glu Asp Ile Asn Leu
65                  70                  75                  80

Pro Glu Ser Phe Asp Ala Arg Glu Gln Trp Ser Asn Cys Pro Thr Ile
                85                  90                  95

Ala Gln Ile Arg Asp Gln Gly Ser Cys Gly Ser Cys Trp Ala Phe Gly
            100                 105                 110

Ala Val Glu Ala Met Ser Asp Arg Ile Cys Ile His Thr Asn Gly Arg
        115                 120                 125

Val Asn Val Glu Val Ser Ala Glu Asp Leu Leu Thr Cys Cys Gly Ile
    130                 135                 140

Gln Cys Gly Asp Gly Cys Asn Gly Gly Tyr Pro Ser Gly Ala Trp Asn
145                 150                 155                 160

Phe Trp Thr Arg Lys Gly Leu Val Ser Gly Gly Val Tyr Asn Ser His
                165                 170                 175

Ile Gly Cys Leu Pro Tyr Thr Ile Pro Pro Cys Glu His His Val Asn
            180                 185                 190

Gly Ser Arg Pro Pro Cys Thr Gly Glu Gly Asp Thr Pro Lys Cys Asn
        195                 200                 205

Lys Met Cys Glu Ala Gly Tyr Ser Thr Ser Tyr Lys Glu Asp Lys His
    210                 215                 220

Tyr Gly Tyr Thr Ser Tyr Ser Val Ser Asp Ser Glu Lys Glu Ile Met
225                 230                 235                 240

Ala Glu Ile Tyr Lys Asn Gly Pro Val Glu Gly Ala Phe Thr Val Phe
                245                 250                 255

Ser Asp Phe Leu Thr Tyr Lys Ser Gly Val Tyr Lys His Glu Ala Gly
            260                 265                 270

Asp Val Met Gly Gly His Ala Ile Arg Ile Leu Gly Trp Gly Ile Glu
        275                 280                 285

Asn Gly Val Pro Tyr Trp Leu Val Ala Asn Ser Trp Asn Val Asp Trp
    290                 295                 300

Gly Asp Asn Gly Phe Phe Lys Ile Leu Arg Gly Glu Asn His Cys Gly
305                 310                 315                 320

Ile Glu Ser Glu Ile Val Ala Gly Ile Pro Arg Thr Gln Gln Tyr Trp
                325                 330                 335

Gly Arg Phe

<210> 55
<211> 1904
<212> DNA
<213> Rattus norvegicus

<220>
<223> Cathepsin B

<400> 55

gagcgactgc gggcccaggt tgtcgcgctg cgctcggtga gtacaggatc cagcttccag 60
gatgtggtgg tccttgatcc ctctctcttg tctgctggca ctgaccagtg cccatgacaa 120
gccttcctca cacccactgt cggacgacat gattaactat atcaacaaac agaatacaac 180
atggcaggct ggacgcaact tctacaatgt tgacataagc tatctgaaga agctgtgtgg 240
aactgtcctg ggtggaccca acctgccgga aagggttggg ttcagcgagg acataaatct 300
acctgaatcc tttgatgcac gggaacagtg gtccaattgc ccgaccatcg cacagatcag 360
agaccagggg tcctgtggct cttgttgggc atttggggca gtggaagcca tgtctgaccg 420
aatctgcatt cacaccaatg gccgagtcaa tgtggaggtg tctgctgagg acctgcttac 480
ctgctgtggt atccagtgtg gggatggctg taatggtggc tatccctctg gagcatggaa 540
cttctggact agaaaaggcc tggtttctgg tggagtatac aattctcata taggctgctt 600
accctacacc atccctccct gtgaacacca tgtcaatggc tcccgtcccc catgcactgg 660
agagggagat actcccaagt gcaacaagat gtgtgaggct ggctactcca catcctacaa 720
ggaagataag cactatgggt acacttccta cagtgtgtct gacagcgaga aggagatcat 780
ggcggaaatc tacaaaaatg gcccagtgga gggtgctttt actgtgtttt ctgacttctt 840
gacttacaaa tcaggcgtat acaagcatga agccggtgat gtgatgggag gccatgccat 900
ccgcattctg ggctggggaa tagagaatgg agtaccctac tggctggtag caaactcctg 960
gaacgttgac tggggtgata atggtttctt taaaatcctc agaggagaga accactgtgg 1020
aattgaatca gaaatcgtgg ctggaatccc acgcactcag cagtactggg gaagattcta 1080
atctgctggg acttgattgt atagtcctta ggagactttt tccaaaattt agcaatgcag 1140
gcagggagtg aggcagatag gagtctttga ttctttgagt tcacctaaca tgcatgaagc 1200
tttcagcaag gtttgggtga ctggaccaga gctgcccccc atcagagcca ccctacctgg 1260
ctcccttcta gcctatccca tgacagtgat cacagcctac atcctagcct ctccctagac 1320
tagtgctgtg tgtagcgcct gctgctgtgg ctctgtctgc catccctccc acccccacct 1380
cagcatcccc agaccaaggg ctttggctgc aggatttcca aaggaatgaa caccaactac 1440
atgatgagag gacagatgcc acccgccagc tgcaccttga agctagtcac ttctaggact 1500
gtggcaggct ttgatggccc aatgcagttc tctggagaaa actacctttc cccaaggcat 1560
ctgcacccat tgacaatggt aatgtgccca tctctccttg gtcctgccct caaccgatgc 1620
ttttccagtc agggttttgt tttttgtttt gttttgtgta cctcaactga gttatgaaga 1680
tttgtactgg ttttacagat catctcatcg tatggaaaag aacaagcttc gtggtcagtt 1740
tactgggtga ccggcagaca ccacaatcaa actagtctgg ggaaaacctg cttttttgtt 1800
gtaggtgcca cgtaaccctg tcagtttgac aaggaatgac cgtgccaata aaccaattct 1860
ccctctgctt ggaaaaaaaa aaaaaaaaaa aaaaaaaaaa aaaa 1904

<210> 56
<211> 334
<212> PRT
<213> Rattus norvegicus

<220>
<223> Cathepsin L

<400> 56
Met Thr Pro Leu Leu Leu Leu Ala Val Leu Cys Leu Gly Thr Ala Leu
1               5                   10                  15

Ala Thr Pro Lys Phe Asp Gln Thr Phe Asn Ala Gln Trp His Gln Trp
            20                  25                  30

Lys Ser Thr His Arg Arg Leu Tyr Gly Thr Asn Glu Glu Glu Trp Arg
        35                  40                  45

Arg Ala Val Trp Glu Lys Asn Met Arg Met Ile Gln Leu His Asn Gly
    50                  55                  60

Glu Tyr Ser Asn Gly Lys His Gly Phe Thr Met Glu Met Asn Ala Phe
65                  70                  75                  80

Gly Asp Met Thr Asn Glu Glu Phe Arg Gln Ile Val Asn Gly Tyr Arg
                85                  90                  95

His Gln Lys His Lys Lys Gly Arg Leu Phe Gln Glu Pro Leu Met Leu
            100                 105                 110

Gln Ile Pro Lys Thr Val Asp Trp Arg Glu Lys Gly Cys Val Thr Pro
        115                 120                 125

Val Lys Asn Gln Gly Gln Cys Gly Ser Cys Trp Ala Phe Ser Ala Ser
    130                 135                 140

Gly Cys Leu Glu Gly Gln Met Phe Leu Lys Thr Gly Lys Leu Ile Ser
145                 150                 155                 160

Leu Ser Glu Gln Asn Leu Val Asp Cys Ser His Asp Gln Gly Asn Gln
                165                 170                 175

Gly Cys Asn Gly Gly Leu Met Asp Phe Ala Phe Gln Tyr Ile Lys Glu
            180                 185                 190

Asn Gly Gly Leu Asp Ser Glu Glu Ser Tyr Pro Tyr Glu Ala Lys Asp
        195                 200                 205

Gly Ser Cys Lys Tyr Arg Ala Glu Tyr Ala Val Ala Asn Asp Thr Gly
    210                 215                 220

Phe Val Asp Ile Pro Gln Gln Glu Lys Ala Leu Met Lys Pro Val Ala
225                 230                 235                 240

Thr Val Gly Pro Ile Ser Val Ala Met Asp Ala Ser His Pro Ser Leu
                245                 250                 255

Gln Phe Tyr Ser Ser Gly Ile Tyr Tyr Glu Pro Asn Cys Ser Ser Lys
            260                 265                 270

Asp Leu Asp His Gly Val Leu Val Val Gly Tyr Gly Tyr Glu Gly Thr
        275                 280                 285

Asp Ser Asn Lys Asp Lys Tyr Trp Leu Val Lys Asn Ser Trp Gly Lys
    290                 295                 300

Glu Trp Gly Met Asp Gly Tyr Ile Lys Ile Ala Lys Asp Arg Asn Asn
305                 310                 315                 320

His Cys Gly Leu Ala Thr Ala Ala Ser Tyr Pro Ile Val Asn
                325                 330


<210> 57
<211> 1386
<212> DNA
<213> Rattus norvegicus

<220>
<223> Cathepsin L

<400> 57
aattccgcgg cgacctccgg ggatccgagt ttgcagacta cgtgtgtgcg cagctagcca 60
cctcaggtgt ttgaaccatg acccctttac tcctcctggc tgtcctctgc ttgggaacag 120
ccttagccac tccaaaattt gatcaaacgt ttaatgcaca gtggcaccag tggaagtcca 180
cacacagaag actgtatggc acgaatgagg aagagtggag gagagcagtg tgggagaaga 240
acatgaggat gatccagcta cacaatgggg agtacagcaa cgggaagcac ggctttacca 300
tggagatgaa cgccttcggt gacatgacca atgaggaatt caggcagata gtgaatggct 360

atcgccacca gaagcacaag aagggaaggt tatttcagga acctctgatg ctgcagatcc 420
ccaagactgt ggactggaga gaaaagggtt gtgtgactcc tgtgaagaat cagggccagt 480
gtggttcttg ctgggctttt agcgcatcgg gttgcctaga aggacagatg ttccttaaga 540
ctggcaaact gatctcactg agtgaacaga accttgtgga ctgttctcac gatcaaggca 600
atcagggctg taatggaggc ctgatggatt ttgctttcca gtacattaag gaaaatggag 660
gtctggactc agaggagtct tatccctatg aagcaaagga tggatcttgt aaatacagag 720
ctgagtatgc tgtggctaac gacacagggt ttgtggatat ccctcagcaa gagaaagccc 780
tcatgaagcc tgtagcgacg gtggggccta tttctgttgc catggatgca agccatccgt 840
ctctccagtt ctatagttca ggtatctact atgaacccaa ctgtagcagc aaggacctcg 900
accatggggt tctggtggtt ggctatggtt atgaaggaac agattcaaat aaggataaat 960
actggcttgt caaaaacagc tggggtaaag aatggggtat ggatggctac atcaaaatag 1020
ccaaagaccg gaacaaccac tgcggacttg ccaccgcagc cagctatcct atcgtgaatt 1080
gatggacagc gataataagg actgacggac actacatccg aaggagttca tcttaaaact 1140
gaccaaaccc gtctctgagt gagaccatgg tacttgaatc gttcaggatc caagtcacga 1200
tttaaattct gttgacattt ttacatgggt taaatgttac cactacttaa aactcctgtt 1260
ataaacagct ttataatatt ggacacttaa tgcttaattc tgattctgga atatttgttt 1320
tataaaagtt gtataaaact ttctttacct tttaaaaata aattttagct cagtgcaaaa 1380
aaaaaa 1386

<210> 58
<211> 803
<212> PRT
<213> Rattus norvegicus

<220>
<223> Dipeptidyl aminopeptidase-related protein

<400> 58
Met Thr Thr Ala Lys Glu Pro Ser Ala Ser Gly Lys Ser Val Gln Gln
1               5                   10                  15

Gln Asp Gln Glu Leu Val Gly Ser Asn Pro Pro Gln Arg Asn Trp Lys
            20                  25                  30

Gly Ile Ala Ile Ala Leu Leu Val Ile Leu Val Ile Cys Ser Leu Ile
        35                  40                  45

Val Thr Ser Val Ile Leu Leu Thr Pro Ala Glu Asp Thr Ser Leu Ser
    50                  55                  60

Gln Lys Lys Lys Val Thr Val Glu Asp Leu Phe Ser Glu Asp Phe Lys
65                  70                  75                  80

Ile His Asp Pro Glu Ala Lys Trp Ile Ser Asp Lys Glu Phe Ile Tyr
                85                  90                  95

Arg Glu Arg Lys Gly Ser Val Ile Leu Arg Asn Val Glu Thr Asn Asn
            100                 105                 110

Ser Thr Val Leu Ile Glu Gly Lys Lys Ile Glu Ser Leu Arg Ala Ile
        115                 120                 125

Arg Tyr Glu Ile Ser Pro Asp Lys Glu Tyr Ala Leu Phe Ser Tyr Asn
    130                 135                 140

Val Glu Pro Val Tyr Gln His Ser His Thr Gly Tyr Tyr Val Leu Ser
145                 150                 155                 160

Lys Ile Pro His Gly Asp Pro Gln Ser Leu Asp Pro Pro Glu Val Ser
                165                 170                 175

Asn Ala Lys Leu Gln Tyr Ala Gly Trp Gly Pro Lys Gly Gln Gln Leu
            180                 185                 190

Ile Phe Ile Phe Glu Asn Asn Ile Tyr Tyr Cys Ala His Val Gly Lys
        195                 200                 205

Gln Ala Ile Arg Val Val Ser Thr Gly Lys Glu Gly Val Ile Tyr Asn
    210                 215                 220

Gly Leu Ser Asp Trp Leu Tyr Glu Glu Glu Ile Leu Lys Ser His Ile
225                 230                 235                 240

Ala His Trp Trp Ser Pro Asp Gly Thr Arg Leu Ala Tyr Ala Thr Ile
                245                 250                 255

Asn Asp Ser Arg Val Pro Leu Met Glu Leu Pro Thr Tyr Thr Gly Ser
            260                 265                 270

Val Tyr Pro Thr Val Lys Pro Tyr His Tyr Pro Lys Ala Gly Ser Glu
        275                 280                 285

Asn Pro Ser Ile Ser Leu His Val Ile Gly Leu Asn Gly Pro Thr His
    290                 295                 300

Asp Leu Glu Met Met Pro Pro Asp Asp Pro Arg Met Arg Glu Tyr Tyr
305                 310                 315                 320

Ile Thr Met Val Lys Trp Ala Thr Ser Thr Lys Val Ala Val Thr Trp
                325                 330                 335

Leu Asn Arg Ala Gln Asn Val Ser Ile Leu Thr Leu Cys Asp Ala Thr
            340                 345                 350

Thr Gly Val Cys Thr Lys Lys His Glu Asp Glu Ser Glu Ala Trp Leu
        355                 360                 365

His Arg Gln Asn Glu Glu Pro Val Phe Ser Lys Asp Gly Arg Lys Phe
    370                 375                 380

Phe Phe Val Arg Ala Ile Pro Gln Gly Gly Arg Gly Lys Phe Tyr His
385                 390                 395                 400

Ile Thr Val Ser Ser Ser Gln Pro Asn Ser Ser Asn Asp Asn Ile Gln
                405                 410                 415

Ser Ile Thr Ser Gly Asp Trp Asp Val Thr Glu Ile Leu Thr Tyr Asp
            420                 425                 430

Glu Lys Arg Asn Lys Leu Tyr Phe Leu Ser Thr Glu Asp Leu Pro Arg
        435                 440                 445

Arg Arg His Leu Tyr Ser Ala Asn Thr Val Asp Asp Phe Asn Arg Gln
    450                 455                 460

Cys Leu Ser Cys Asp Leu Val Glu Asn Cys Thr Tyr Val Ser Ala Ser
465                 470                 475                 480

Phe Ser His Asn Met Asp Phe Phe Leu Leu Lys Cys Glu Gly Pro Gly
                485                 490                 495

Val Pro Thr Val Thr Val His Asn Thr Thr Asp Lys Arg Arg Met Phe
        500                 505                 510

Asp Leu Glu Ala Asn Glu Gln Val Gln Lys Ala Ile Tyr Asp Arg Gln
        515                 520                 525

Met Pro Lys Ile Glu Tyr Arg Lys Ile Glu Val Glu Asp Tyr Ser Leu
    530                 535                 540

Pro Met Gln Ile Leu Lys Pro Ala Thr Phe Thr Asp Thr Ala His Tyr
545                 550                 555                 560

Pro Leu Leu Leu Val Val Asp Gly Thr Pro Gly Ser Gln Ser Val Ser
                565                 570                 575

Glu Arg Phe Glu Val Thr Trp Glu Thr Val Leu Val Ser Ser His Gly
            580                 585                 590

Ala Val Val Val Lys Cys Asp Gly Arg Gly Ser Gly Phe Gln Gly Thr
        595                 600                 605

Lys Leu Leu His Glu Val Arg Arg Arg Leu Gly Phe Leu Glu Glu Lys
    610                 615                 620

Asp Gln Met Glu Ala Val Arg Thr Met Leu Lys Glu Gln Tyr Ile Asp
625                 630                 635                 640

Lys Thr Arg Val Ala Val Phe Gly Lys Asp Tyr Gly Gly Tyr Leu Ser
                645                 650                 655

Thr Tyr Ile Leu Pro Ala Lys Gly Glu Asn Gln Gly Gln Thr Phe Thr
            660                 665                 670

Cys Gly Ser Ala Leu Ser Pro Ile Thr Asp Phe Lys Leu Tyr Ala Ser
        675                 680                 685

Ala Phe Ser Glu Arg Tyr Leu Gly Leu His Gly Leu Asp Asn Arg Ala
    690                 695                 700

Tyr Glu Met Thr Lys Leu Ala His Arg Val Ser Ala Leu Glu Asp Gln
705                 710                 715                 720

Gln Phe Leu Ile Ile His Ala Thr Ala Asp Glu Lys Ile His Phe Gln
                725                 730                 735

His Thr Ala Glu Leu Ile Thr Gln Leu Ile Lys Gly Lys Ala Asn Tyr
            740                 745                 750

Ser Leu Gln Ile Tyr Pro Asp Glu Ser His Tyr Phe His Ser Val Ala
        755                 760                 765

Leu Lys Gln His Leu Tyr Arg Ser Ile Ile Gly Phe Phe Val Glu Cys
    770                 775                 780

Phe Arg Ile Gln Asp Lys Leu Pro Thr Ala Thr Ala Lys Glu Asp Glu
785                 790                 795                 800

Glu Glu Asp

<210> 59
<211> 2672
<212> DNA
<213> Rattus norvegicus

<220>
<223> Dipeptidyl aminopeptidase-related protein

<400> 59
ctcttcttct gccgagcccg tctctgctct ttgcatttaa agagcaaact aggctgctct 60
ccccagctcc ctccccccac cgccccccaa aaacctggtc attttggaag tatggactaa 120
aatcgctctc ctcctcaccc taccattccg atcccggtgc ctccaaagtc acagtcagac 180
ccctcctgtg tctggggtgg cgagtgcccg gacagaccat gaccaccgcc aaggagccca 240
gcgcctcagg aaaatctgta cagcagcagg atcaggagct ggtggggagt aatcctccac 300
agagaaactg gaaaggaatc gccattgcac tgcttgtcat ccttgtcatc tgctccctga 360
ttgtcacctc agtcatcctg ctgaccccag cggaagatac cagtctgtct cagaagaaga 420
aggtgactgt ggaagatctt ttcagtgaag acttcaaaat ccatgaccca gaggcaaagt 480
ggataagcga taaggaattc atctacagag aacggaaagg gagcgtcata ctgcggaacg 540
ttgagacaaa taattccacg gtgttaatag aaggcaaaaa aattgaatcg ttaagagcca 600
tcagatatga aatatcccca gataaagagt atgcgttgtt ttcctataat gtggaaccgg 660
tataccaaca ctcccacact ggatattatg ttctgagcaa aattcctcac ggggaccctc 720
agagtctgga cccaccagaa gtcagcaacg cgaagcttca gtatgcaggg tggggcccga 780
aaggccagca gctgatattt atctttgaga acaacatcta ctactgtgcc catgttggaa 840
agcaggcgat tcgagtggtc tctacgggga aggagggtgt gatctacaat ggcctcagcg 900
actggctgta tgaagaggag attctgaaga gccacatcgc acattggtgg tcgccagatg 960
gtacaaggct cgcctatgcc accatcaatg attcccgagt tccacttatg gagctgccaa 1020
cctacacggg ctctgtctac cccactgtga agccctacca ctatcctaag gctggcagtg 1080
aaaaccccag catctccctc cacgtcatcg gcttgaatgg acccacccac gacctggaga 1140
tgatgccacc tgacgacccc cgcatgagag agtattacat caccatggtg aagtgggcca 1200
ccagcaccaa ggttgctgtg acctggctga accgggccca gaatgtgtcc atcctgaccc 1260
tctgtgatgc caccacaggt gtctgcacta agaaacacga ggatgaaagt gaagcttggc 1320
tccacagaca gaatgaagag cccgtgttct ccaaggatgg ccggaagttt ttctttgtca 1380
gagcgatccc acagggaggc cggggtaaat tctaccacat cactgtatcc tcatcccagc 1440
ccaacagcag caatgacaac atccagtcca ttacctctgg ggactgggat gtgaccgaga 1500
tcctgaccta cgatgagaag cggaataagc tctatttcct aagcacagag gacctgccac 1560
gaagacgaca cctctacagc gccaacacgg tagacgactt caacaggcag tgtctgtcat 1620
gtgacctggt ggagaactgc acctatgtca gtgcttcctt cagtcataac atggacttct 1680
tcctgctcaa gtgtgaaggc cctggcgtac ccactgttac tgtgcacaat accacggata 1740
agagaagaat gtttgacttg gaagcaaatg agcaagtgca gaaagccatc tacgatcggc 1800
agatgcctaa aattgaatac cggaaaattg aagtggaaga ttacagcttg ccaatgcaaa 1860
tcttgaagcc ggccaccttc actgacacag cccactaccc tttattgctc gtggtggatg 1920
gtactccagg gagtcagagc gtgtcagaga ggtttgaagt gacctgggag acagtgttgg 1980
tgagcagcca tggagcagtg gtggtcaagt gtgacggccg aggcagcggc tttcagggaa 2040
ccaagctctt gcacgaagta cggaggcggc taggcttcct ggaggagaag gaccagatgg 2100
aagctgtgag aaccatgctg aaggagcagt acattgacaa gactcgggta gctgtgtttg 2160
ggaaggatta tggtgggtac ctgagtactt acatcctccc agccaaggga gaaaaccaag 2220
gccagacttt cacctgcggc tctgcactct ctccgataac ggacttcaaa ctctatgcct 2280
cagcattttc cgagaggtac cttggcctcc atggactcga caacagagcg tatgagatga 2340
ccaagctggc acacagagtg tcagcactgg aggaccagca gttcctgatc atccatgcta 2400
cggctgacga aaaaatccat ttccagcaca cagcagagct catcacgcag ctaatcaagg 2460
gaaaggccaa ttacagctta cagatatacc cagatgaaag tcactacttt cacagcgtgg 2520
cgctcaaaca gcacctgtac aggtccatca tcggattctt tgtggaatgt ttcaggatcc 2580
aggacaagct acctacagcc acagcgaaag aggacgagga ggaggactaa ccacgcttga 2640
gacctaaaca cagacatggc tctttctaca ac 2672

<210> 60
<211> 255
<212> PRT
<213> Rattus norvegicus

<220>
<223> Proteasome subunit C8

<400> 60
Met Ser Ser Ile Gly Thr Gly Tyr Asp Leu Ser Ala Ser Thr Phe Ser
1               5                   10                  15

Pro Asp Gly Arg Val Phe Gln Val Glu Tyr Ala Met Lys Ala Val Glu
            20                  25                  30

Asn Ser Ser Thr Ala Ile Gly Ile Arg Cys Lys Asp Gly Val Val Phe
        35                  40                  45

Gly Val Glu Lys Leu Val Leu Ser Lys Leu Tyr Glu Glu Gly Ser Asn
    50                  55                  60

Lys Arg Leu Phe Asn Val Asp Arg His Val Gly Met Ala Val Ala Gly
65                  70                  75                  80

Leu Leu Ala Asp Ala Arg Ser Leu Ala Asp Ile Ala Arg Glu Glu Ala
                85                  90                  95

Ser Asn Phe Arg Ser Asn Phe Gly Tyr Asn Ile Pro Leu Lys His Leu
            100                 105                 110

Ala Asp Arg Val Ala Met Tyr Val His Ala Tyr Thr Leu Tyr Ser Ala
        115                 120                 125

Val Arg Pro Phe Gly Cys Ser Phe Met Leu Gly Ser Tyr Ser Val Asn
    130                 135                 140

Asp Gly Ala Gln Leu Tyr Met Ile Asp Pro Ser Gly Val Ser Tyr Gly
145                 150                 155                 160

Tyr Trp Gly Cys Ala Ile Gly Lys Ala Arg Gln Ala Ala Lys Thr Glu
                165                 170                 175

Ile Glu Lys Leu Gln Met Lys Glu Met Thr Cys Arg Asp Val Val Lys
            180                 185                 190

Glu Val Ala Lys Ile Ile Tyr Ile Val His Asp Glu Val Lys Asp Lys
        195                 200                 205

Ala Phe Glu Leu Glu Leu Ser Trp Val Gly Glu Leu Thr Lys Gly Arg
    210                 215                 220

His Glu Ile Val Pro Lys Asp Val Arg Glu Glu Ala Glu Lys Tyr Ala
225                 230                 235                 240

Lys Glu Ser Leu Lys Glu Glu Asp Glu Ser Asp Asp Asp Asn Met
                245                 250                 255


<210> 61
<211> 897
<212> DNA
<213> Rattus norvegicus

<220>
<223> Proteasome subunit C8

<400> 61
cgttctgtgg cctgccgggt cagcaccatg agctccattg gcactgggta tgacctgtca 60
gcctctacat tctctcctga tggaagagtt ttccaagttg aatatgctat gaaggctgtg 120
gaaaacagta gtacagctat tgggatcaga tgtaaagatg gtgttgtgtt tggggtagaa 180
aaattagtcc tttctaaact ttatgaagaa ggctccaata aacgtctttt caatgttgat 240
cgacatgttg gaatggcagt ggcaggtttg ttggcagatg ctcgttcctt ggcagacata 300
gcaagagaag aagcgtccaa ctttaggtct aactttggct ataacattcc tctaaaacat 360
cttgcagaca gagtggccat gtatgtacac gcgtatacac tctacagtgc tgttagacct 420
tttggctgca gtttcatgtt ggggtcttac agtgtgaatg atggtgcaca gctctatatg 480
atcgatccat caggtgtttc atatggttac tggggctgtg ccattggcaa agccaggcaa 540
gctgcaaaga cagaaataga aaagcttcag atgaaggaaa tgacctgccg tgatgtagtt 600
aaagaagttg caaaaataat ttacatagta catgatgaag ttaaggataa agcttttgaa 660
ctagagctca gctgggttgg tgaattaact aaaggaagac atgaaattgt tcccaaagac 720
gtaagagagg aagcagagaa atatgccaag gaatctttga aggaagaaga tgaatcagat 780
gacgacaata tgtaacattt caccatcctc tggtgtccat ttctgtgaca gttgcatatg 840
gctagcacca tggattatat atacccactg accaattttc attaaatttt gtcttat 897

<210> 62
<211> 1080
<212> PRT
<213> Mus musculus

<220>
<223> Deubiquitinating enzyme 8

<400> 62
   Met Pro Ala Val Ala Ser Val Pro Lys Glu Leu Tyr Leu Ser Ser Ser
   1               5                   10                  15
   Leu Lys Asp Leu Asn Lys Lys Thr Glu Val Lys Pro Glu Lys Thr Ser
               20                  25                  30
   Thr Lys Asn Tyr Ile His Ser Ala Gln Lys Ile Phe Lys Thr Ala Glu
           35                  40                  45
   Glu Cys Arg Leu Asp Arg Asp Glu Glu Arg Ala Tyr Val Leu Tyr Met
       50                  55                  60
   Lys Tyr Val Ala Val Tyr Asn Leu Ile Lys Lys Arg Pro Asp Phe Lys
   65                  70                  75                  80
   Gln Gln Gln Asp Tyr Tyr Leu Ser Ile Leu Gly Pro Ala Asn Ile Lys
                   85                  90                  95
   Lys Ala Ile Glu Glu Ala Glu Arg Leu Ser Glu Ser Leu Lys Leu Arg
               100                 105                 110
   Tyr Glu Glu Ala Glu Val Arg Lys Gln Leu Glu Glu Lys Asp Arg Arg
           115                 120                 125
   Glu Glu Glu Gln Leu Gln Gln Gln Lys Arg Pro Glu Met Gly Arg Glu
       130                 135                 140
   Asp Ser Gly Ala Ala Ala Lys Arg Ser Val Glu Asn Leu Leu Asp Ser
   145                 150                 155                 160
   Lys Thr Lys Thr Gln Arg Ile Asn Gly Glu Lys Ser Glu Gly Ala Ala
                   165                 170                 175
   Ala Ala Glu Arg Gly Ala Ile Thr Ala Lys Glu Leu Tyr Thr Met Met
   180 185 190
   Met Asp Lys Asn Thr Ser Leu Ile Ile Met Asp Ala Arg Lys Ile Gln
   195 200 205
   Asp Tyr Gln His Ser Cys Ile Leu Asp Ser Leu Ser Val Pro Glu Glu
   210 215 220
   Ala Ile Ser Pro Gly Val Thr Ala Ser Trp Ile Glu Ala Asn Leu Ser
   225 230 235 240
   Asp Asp Ser Lys Asp Thr Trp Lys Lys Arg Gly Ser Val Asp Tyr Val
   245 250 255
   Val Leu Leu Asp Trp Phe Ser Ser Ala Lys Asp Leu Leu Leu Gly Thr
   260 265 270
   Thr Leu Arg Ser Leu Lys Asp Ala Leu Phe Lys Trp Glu Ser Lys Thr
   275 280 285
   Val Leu Arg His Glu Pro Leu Val Leu Glu Gly Gly Tyr Glu Asn Trp
   290 295 300
   Leu Leu Cys Tyr Pro Gln Phe Thr Thr Asn Ala Lys Val Thr Pro Pro
   305 310 315 320
   Pro Arg Ser Arg Ala Glu Glu Val Ser Val Ser Leu Asp Phe Thr Tyr
   325 330 335
   Pro Ser Leu Glu Glu Pro Val Pro Ser Lys Leu Pro Thr Gln Met Pro
   340 345 350
   Pro Pro Pro Ile Glu Thr Asn Glu Lys Ala Leu Leu Val Thr Asp Gln
   355 360 365
   Asp Glu Lys Leu Arg Leu Ser Thr Gln Pro Ala Leu Ala Gly Pro Gly
   370 375 380
   Ala Ala Pro Arg Ala Glu Ala Ser Pro Ile Ile Gln Pro Ala Pro Ala
   385 390 395 400
   Thr Lys Ser Val Pro Gln Val Asp Arg Thr Lys Lys Pro Ser Val Lys
   405 410 415
   Leu Pro Glu Asp His Arg Ile Lys Ser Glu Asn Thr Asp Gln Ser Gly
   420 425 430
   Arg Val Leu Ser Asp Arg Ser Thr Lys Pro Val Phe Pro Ser Pro Thr
   435 440 445
   Thr Met Leu Thr Asp Glu Glu Lys Ala Arg Ile His Gln Glu Thr Ala
   450 455 460
   Leu Leu Met Glu Lys Asn Lys Gln Glu Lys Glu Leu Trp Asp Lys Gln
   465 470 475 480
   Gln Lys Glu Gln Lys Glu Lys Leu Arg Arg Glu Glu Gln Glu Arg Lys
   485 490 495
   Ala Gly Lys Thr Gln Asp Ala Asp Glu Arg Asp Ser Thr Glu Asn Gln
   500 505 510
   His Lys Ala Lys Asp Gly Gln Glu Lys Lys Asp Ser Lys Gln Thr Lys
           515                 520                 525
   Thr Glu Asp Arg Glu Leu Ser Ala Asp Gly Ala Gln Glu Ala Thr Gly
       530                 535                 540
   Thr Gln Arg Gln Ser Lys Ser Glu His Glu Ala Ser Asp Ala Lys Val
   545                 550                 555                 560
   Pro Val Glu Gly Lys Arg Cys Pro Thr Ser Glu Ala Gln Lys Arg Pro
                   565                 570                 575
   Ala Asp Val Ser Pro Ala Ser Val Ser Gly Glu Leu Asn Ala Gly Lys
               580                 585                 590
   Ala Gln Arg Glu Pro Leu Thr Arg Ala Arg Ser Glu Glu Met Gly Arg
           595                 600                 605
   Ile Val Pro Gly Leu Pro Leu Gly Trp Ala Lys Phe Leu Asp Pro Ile
       610                 615                 620
   Thr Gly Thr Phe Arg Tyr Tyr His Ser Pro Thr Asn Thr Val His Met
   625                 630                 635                 640
   Tyr Pro Pro Glu Met Ala Pro Ser Ser Ala Pro Pro Ser Thr Pro Pro
                   645                 650                 655
   Thr His Lys Val Lys Pro Gln Val Pro Ala Glu Arg Asp Arg Glu Pro
               660                 665                 670
   Ser Lys Leu Lys Arg Ser Tyr Ser Ser Pro Asp Ile Thr Gln Ala Leu
           675                 680                 685
   Gln Glu Glu Glu Lys Arg Arg Pro Ala Val Thr Pro Met Val Asn Arg
       690                 695                 700
   Glu Asn Lys Pro Pro Cys Tyr Pro Lys Ala Glu Ile Ser Arg Leu Ser
   705                 710                 715                 720
   Ala Ser Gln Ile Arg Asn Leu Asn Pro Val Phe Gly Gly Ser Gly Pro
                   725                 730                 735
   Ala Leu Thr Gly Leu Arg Asn Leu Gly Asn Thr Cys Tyr Met Asn Ser
               740                 745                 750
   Ile Leu Gln Cys Leu Cys Asn Ala Pro His Leu Ala Asp Tyr Phe Asn
           755                 760                 765
   Arg Asn Cys Tyr Gln Asp Asp Ile Asn Arg Ser Asn Leu Leu Gly His
       770                 775                 780
   Lys Gly Glu Val Ala Glu Glu Phe Gly Ile Ile Met Lys Ala Leu Trp
   785                 790                 795                 800
   Thr Gly Gln Tyr Arg Tyr Ile Ser Pro Lys Asp Phe Lys Val Thr Ile
                   805                 810                 815
   Gly Lys Ile Asn Asp Gln Phe Ala Gly Ser Ser Gln Gln Asp Ser Gln
               820                 825                 830
   Glu Leu Leu Leu Phe Leu Met Asp Gly Leu His Glu Asp Leu Asn Lys
           835                 840                 845
   Ala Asp Asn Arg Lys Arg His Lys Glu Glu Asn Asn Glu His Leu Asp
       850                 855                 860
   Asp Leu Gln Ala Ala Glu His Ala Trp Gln Lys His Lys Gln Leu Asn
   865                 870                 875                 880
   Glu Ser Ile Ile Val Ala Leu Phe Gln Gly Gln Phe Lys Ser Thr Val
                   885                 890                 895
   Gln Cys Leu Thr Cys Arg Arg Arg Ser Arg Thr Phe Glu Ala Phe Met
               900                 905                 910
   Tyr Leu Ser Leu Pro Leu Ala Ser Thr Ser Lys Cys Thr Leu Gln Asp
           915                 920                 925
   Cys Leu Arg Leu Phe Ser Lys Glu Glu Lys Leu Thr Asp Asn Asn Arg
       930                 935                 940
   Phe Tyr Cys Ser His Cys Arg Ala Arg Arg Asp Ser Leu Lys Lys Ile
   945                 950                 955                 960
   Glu Ile Trp Lys Leu Pro Pro Val Leu Leu Val His Leu Lys Arg Phe
                   965                 970                 975
   Ser Tyr Asp Gly Arg Trp Lys Gln Lys Leu Gln Thr Ser Val Asp Phe
               980                 985                 990
   Pro Leu Glu Asn Leu Asp Leu Ser Gln Tyr Val Ile Gly Pro Lys Asn
           995                 1000                1005
   Ser Leu Lys Lys Tyr Asn Leu Phe Ser Val Ser Asn His Tyr Gly Gly
       1010                1015                1020
   Leu Asp Gly Gly His Tyr Thr Ala Tyr Cys Lys Asn Ala Ala Arg Gln
   1025                1030                1035                1040
   Arg Trp Phe Lys Phe Asp Asp His Glu Val Ser Asp Ile Ser Val Ser
                   1045                1050                1055
   Ser Val Arg Ser Ser Ala Ala Tyr Ile Leu Phe Tyr Thr Ser Leu Gly
               1060                1065                1070
   Pro Arg Ile Thr Asp Val Ala Thr
           1075                1080


<210> 63
<211> 3970
<212> DNA
<213> Mus musculus

<220>
<223> Deubiquitinating enzyme 8

<400> 63
gcgagtctta cttcatcatg cctgctgtag cttcagttcc taaagaactc tacctcagtt 60
cttcactaaa agacctcaat aagaagaccg aagttaaacc tgagaaaacc agcaccaaga 120
attatataca cagcgcgcag aagatcttca agacagcaga agaatgcaga ctagatcgtg 180

atgaggaaag ggcctatgtg ctttatatga aatatgtggc agtttataat cttatcaaaa 240
agagacctga tttcaagcaa cagcaggatt attatctttc aatacttgga cctgcaaaca 300
tcaaaaaagc tattgaagaa gctgaaagac tctccgaaag ccttaaacta agatacgaag 360
aggctgaagt tcggaaacaa cttgaagaaa aggacagacg ggaggaagag cagctgcagc 420
aacagaaaag gccggagatg ggagagaggg acagcggtgc ggcagccaaa cgctctgtgg 480
aaaatttact ggattccaaa accaaaaccc aaaggattaa cggcgagaag agtgaaggag 540
ctgcagctgc agagagagga gccatcacag caaaggaact atatacgatg atgatggata 600
aaaacacaag cttaattata atggatgctc gaaaaataca ggattatcag cattcctgta 660
tcttggattc tctcagtgtt cctgaagaag ctatcagtcc aggagtcact gctagttgga 720
ttgaagcaaa cctctcagat gattctaaag acacatggaa aaagaggggg agtgtggact 780
atgtggtcct tctcgactgg tttagttcag cgaaagattt gctgcttggg accactctac 840
ggagtctgaa agatgcactt ttcaagtggg aaagtaaaac tgtcctgcgc catgagcctt 900
tggtgttgga gggcggctat gaaaactggc tgctttgcta cccgcagttt acaaccaatg 960
ctaaggtcac tccaccccct cggagcagag ctgaagaggt gtctgtctca ttggatttta 1020
cttatccctc attggaagaa ccagttcctt ccaaacttcc tacccagatg ccacctcctc 1080
ctatagaaac aaatgaaaag gcactgttgg taactgatca agatgaaaag ctgagactgt 1140
caacccagcc agctctagct ggacctggtg cggctcccag agctgaagcc tcacccataa 1200
ttcagccagc gcctgctaca aagagtgttc cacaggttga tcgtacgaaa aaaccgtcag 1260
tcaagttgcc tgaagatcat agaataaaat ctgaaaatac agatcagagt ggaagagttc 1320
tttctgatcg atccaccaag ccagtatttc cctctccaac caccatgtta acagatgaag 1380
aaaaggctcg tattcatcaa gaaactgccc ttcttatgga aaagaataaa caggagaagg 1440
aactttggga caagcagcag aaggaacaga aagagaagct gagaagggag gaacaagagc 1500
gcaaagctgg aaagacacag gatgcagatg aacgtgactc cactgagaat cagcacaaag 1560
caaaggatgg acaagagaaa aaagacagca aacagaccaa gacggaagac agagagcttt 1620
cagcagacgg ggcccaggaa gccacaggaa cacaaagaca aagtaagagt gagcatgaag 1680
cttctgatgc taaggtacct gtggaaggta aaaggtgtcc cacgtcagag gcgcagaaga 1740
ggccagcaga tgtgtcccct gcatccgtgt caggagagct gaatgcaggc aaggctcagc 1800
gagaaccttt gacgagagca cgaagtgaag aaatggggag aattgtgccg ggactgcctt 1860
tgggctgggc caagtttctt gatccaatca ccgggacctt tcgttactac cattccccca 1920
caaacacagt tcatatgtat ccacctgaaa tggctccttc gtctgcacct ccttccaccc 1980
cgcccactca caaagtcaag ccccaggtcc ctgccgagcg ggacagggag ccatcgaaac 2040
tgaagcgctc ctactcctca ccagatatca ctcaggccct gcaggaggag gagaagagga 2100
ggccggcagt gaccccgatg gtcaaccggg aaaacaagcc accatgttac cctaaagctg 2160
agatttcgag gctttctgct tctcagattc ggaacctcaa tcctgtattt ggaggatcag 2220
gaccagctct tactggactt cgtaatttgg gaaatacttg ttacatgaac tcaatcttgc 2280
agtgcctgtg taatgctcca catctggctg attatttcaa ccgaaactgc taccaggatg 2340
atatcaacag gtcaaatttg ttggggcata aaggtgaagt ggcagaagaa tttggtataa 2400
tcatgaaagc actgtggaca ggacagtata gatacatcag tccaaaggac tttaaagtca 2460
ccattggtaa gattaatgac cagtttgcag gctccagcca acaggattca caagagctgc 2520
ttctgttcct catggatggc ctgcatgagg atctgaataa ggctgacaat cggaagaggc 2580
acaaggaaga gaacaacgag cacctggatg acctgcaggc ggccgagcac gcctggcaga 2640
agcacaagca gctcaacgag tccatcatcg tggccctgtt ccagggccag ttcaagtcca 2700
cagtgcagtg cctcacctgc cgcaggaggt cgcgcacctt cgaggccttc atgtacttgt 2760
ctttgccgct agcatccaca agtaaatgta ctctacagga ctgccttaga ttattttcca 2820
aagaagaaaa gcttacagat aacaacagat tttactgcag ccattgccga gctcggcggg 2880
attctctaaa gaaaatagaa atctggaaat tacctcctgt gctgttagtg cacctgaaac 2940
gattttccta tgacggcagg tggaagcaga agctgcaaac atccgtggat ttcccattgg 3000
aaaatcttga cctgtcacag tacgttattg gtccaaaaaa cagcttgaag aaatataact 3060
tgttttctgt ttcaaaccac tacggcgggc tagacggagg ccactacaca gcctactgta 3120
agaacgcggc aaggcagcgc tggtttaagt ttgatgacca tgaagtttct gatatctctg 3180
tgtcttctgt gaggtcatca gcagcttata tcctctttta tacttccctg ggaccacgca 3240
taactgatgt agccacatag agaggagtag gctgtcggct tattgacgtc tacaaattgc 3300
agcctcacat ctaatgctta caaagataat ggatcgctgc agctgctatg tagagggatt 3360
ccagaacact ggggctgtgc tactagcacc atataattca ggccagtgct gtttatcaaa 3420
agaaagaaag aaagaaaaca gcctgacatt taacaactat catagtacgc attccttaca 3480
ggagtcattt atttcaaaac aactttttga gtctgcccca aagttaaaat aattcattag 3540
tcagacattt attattctct tggtaccaaa ccatcataac ctccattttc ctattcactg 3600
ctccagcctc ttctcatttc tagccccagc tcagtgcact ctgaatgctc tagaatgacc 3660
tagctcagat aggaatgtat atgtacatat ttattgcact gttgcacatc agactgctgg 3720
acacagttta acatagcctt ctgtgaaagc ctagaaagga tttttctttt ttcttttggc 3780
ctcttctagg tagctgcaat gctttcttaa ggaaatgaca gggcaaaact atttttctgt 3840

tggcttccgg ctgtatttgt gcactaaatc tttattctaa acaaatggaa actttaattt 3900
tttaaaaata attcatttac atctacatta aaatcctaaa aaaaaaaaaa aaaaaaaaaa 3960
aaaaaaaaaa 3970

<210> 64
<211> 1203
<212> DNA
<213> Homo sapiens

<220>
<223> Ubiquitin-binding enzyme E2

<400> 64
actcgtgcgt gaggcgagag gagccggaga cgagaccaga ggccgaactc gggttctgac 60
aagatggccg ggctgccccg caggatcatc aaggaaaccc agcgtttgct ggcagaacca 120
gttcctggca tcaaagccga accagatgag agcaacgccc gttattttca tgtggtcatt 180
gctggccctc aggattcccc ctttgaggga gggactttta aacttgaact attccttcca 240
gaagaatacc caatggcagc ccctaaagta cgtttcatga ccaaaattta tcatcctaat 300
gtagacaagt tgggaagaat atgtttagat attttgaaag ataagtggtc cccagcactg 360
cagatccgca cagttctgct atcgatccag gccttgttaa gtgctcccaa tccagatgat 420
ccattagcaa atgatgtagc ggagcagtgg aagaccaacg aagcccaagc catagaaaca 480
gctagagcat ggactaggct atatgccatg aataatattt aaattgatac gatcatcaag 540
tgtgcatcac ttctcctgtt ctgccaagac ttcctcctct ttgtttgcat ttaatggaca 600
cagtcttaga aacattacag aataaaaaag cccagacatc ttcagtcctt tggtgattaa 660
atgcacatta gcaaatctat gtcttgtcct gattcactgt cataaagcat gagcagaggc 720
tagaagtatc atctggattg ttgtgaaacg tttaaaagca gtggcccctc cctgctttta 780
ttcatttccc ccatcctggt ttaagtataa agcactgtga atgaaggtag ttgtcaggtt 840
agctgcaggg gtgtgggtgt ttttatttta ttttatttta ttttattttt gaggggggag 900
gtagtttaat tttatgggct cctttccccc ttttttggtg atctaattgc attggttaaa 960
agcagctaac caggtcttta gaatatgctc tagccaagtc taactttatt tagacgctgt 1020
agatggacaa gcttgattgt tggaaccaaa atgggaacat taaacaaaca tcacagccct 1080
cactaataac attgctgtca agtgtagatt ccccccttca aaaaaagctt gtgaccattt 1140
tgtatggctt gtctggaaac ttctgtaaat cttatgtttt agtaaaatat tttttgttat 1200
tct 1203

<210> 65
<211> 475
<212> PRT
<213> Mus musculus

<220>
<223> Tryptophan t-RNA ligase

<400> 65
Met Ala Asp Met Pro Ser Gly Glu Ser Cys Thr Ser Pro Leu Glu Leu
1               5                   10                  15

Phe Asn Ser Ile Ala Thr Gln Gly Glu Leu Val Arg Ser Leu Lys Ala
            20                  25                  30

Gly Asn Ala Pro Lys Asp Glu Ile Asp Ser Ala Val Lys Met Leu Leu
        35                  40                  45

Ser Leu Lys Met Ser Tyr Lys Ala Ala Met Gly Glu Glu Tyr Lys Ala
    50                  55                  60

Gly Cys Pro Pro Gly Asn Pro Thr Ala Gly Arg Asn Cys Asp Ser Asp
65                  70                  75                  80

Ala Thr Lys Ala Ser Glu Asp Phe Val Asp Pro Trp Thr Val Arg Thr
                85                  90                  95

Ser Ser Ala Lys Gly Ile Asp Tyr Asp Lys Leu Ile Val Gln Pro Gly
            100                 105                 110

Ser Ser Lys Ile Asp Lys Glu Leu Ile Asn Arg Ile Glu Arg Ala Thr
        115                 120                 125

Gly Gln Arg Pro His Arg Phe Leu Arg Arg Gly Ile Phe Phe Ser His
    130                 135                 140

Arg Asp Met Asn Gln Ile Leu Asp Ala Tyr Glu Asn Lys Lys Pro Phe
145                 150                 155                 160

Tyr Leu Tyr Thr Gly Arg Gly Pro Ser Ser Glu Ala Met His Leu Gly
                165                 170                 175

His Leu Val Pro Phe Ile Phe Thr Lys Trp Leu Glu Asp Val Phe Asn
            180                 185                 190

Val Pro Leu Val Ile Gln Met Ser Asp Asp Glu Lys Tyr Leu Trp Lys
        195                 200                 205

Asp Leu Thr Leu Glu Gln Ala Tyr Ser Tyr Thr Val Glu Asn Ala Lys
    210                 215                 220

Asp Ile Ile Ala Cys Gly Phe Asp Ile Asn Lys Thr Phe Ile Phe Ser
225                 230                 235                 240

Asp Leu Glu Tyr Met Gly Gln Ser Pro Gly Phe Tyr Arg Asn Val Val
                245                 250                 255

Lys Ile Gln Lys His Val Thr Phe Asn Gln Val Lys Gly Ile Phe Gly
            260                 265                 270

Phe Thr Asp Ser Asp Cys Ile Gly Lys Ser Ser Phe Pro Ala Val Gln
        275                 280                 285

Ala Ala Pro Ser Phe Ser Asn Ser Phe Pro Lys Ile Phe Arg Asp Arg
    290                 295                 300

Thr Asp Ile Gln Cys Leu Ile Pro Cys Ala Ile Asp Gln Asp Pro Tyr
305                 310                 315                 320

Phe Arg Met Thr Arg Asp Val Ala Pro Arg Ile Gly His Pro Lys Pro
                325                 330                 335

Ala Leu Leu His Ser Thr Phe Phe Pro Ala Leu Gln Gly Ala Gln Thr
            340                 345                 350

Lys Met Ser Ala Ser Asp Pro Asn Ser Ser Ile Phe Leu Thr Asp Thr
        355                 360                 365

Ala Lys Gln Ile Lys Ser Lys Val Asn Lys His Ala Phe Ser Gly Gly
    370                 375                 380

Arg Asp Thr Val Glu Glu His Arg Gln Phe Gly Gly Asn Cys Glu Val
385                 390                 395                 400

Asp Val Ser Phe Met Tyr Leu Thr Phe Phe Leu Glu Asp Asp Asp Arg

405                 410                 415

Leu Glu Gln Ile Arg Lys Asp Tyr Thr Ser Gly Ala Met Leu Thr Gly
            420                 425                 430

Glu Leu Lys Lys Thr Leu Ile Asp Val Leu Gln Pro Leu Ile Ala Glu
        435                 440                 445

His Gln Ala Arg Arg Lys Ala Val Thr Glu Glu Thr Val Lys Glu Phe
    450                 455                 460

Met Thr Pro Arg Gln Leu Ser Phe His Phe Gln
465                 470                 475

<210> 66
<211> 2786
<212> DNA
<213> Mus musculus

<220>
<223> Tryptophan t-RNA ligase

<400> 66
cagctgtcag cttgagcacc cttggactac acagccagga gtcctgtctc aactgctgaa 60
cacactactc acaagccagc tgcaggcctc ggtcctagaa gatggcagac atgcccagtg 120
gcgagtcctg cacctctcca ctggagctgt tcaacagcat agctactcaa ggggagctcg 180
tgaggtccct caaagctgga aatgcaccaa aagatgagat tgattctgca gtaaagatgt 240
tgttatcatt aaaaatgagc tataaggccg ccatggggga ggagtacaag gctggctgcc 300
ctcctgggaa cccaacagct gggagaaatt gtgactctga tgccacaaaa gccagcgagg 360
attttgtgga tccatggacg gtccggacaa gcagtgcaaa aggcatcgac tatgacaagc 420
tcattgttca gcccggaagc agcaaaattg acaaagagct gataaaccga atagagcgag 480
ccactggcca aagaccgcac cgcttcctgc gccggggcat cttcttctcc caccgagata 540
tgaatcaaat tctggatgcc tatgaaaata agaagccatt ctacctgtac acaggcaggg 600
gcccctcctc cgaagcaatg catctgggtc atctcgtccc gtttattttc acaaagtggc 660
tagaggatgt gttcaatgtg cctttggtca tccagatgtc tgacgacgag aagtacctgt 720
ggaaggactt gactctggag caggcatata gctacaccgt agaaaatgca aaggacatca 780
ttgcctgtgg cttcgacatc aacaaaactt tcatcttctc tgatcttgag tatatggggc 840
agagcccggg cttctacagg aacgtggtga agattcagaa gcacgtcacc ttcaaccaag 900
tgaaaggcat tttcggcttt acggacagtg actgcatcgg gaagagcagt tttcctgctg 960
tccaggccgc gccatcgttc agcaactctt tcccgaagat cttccgagac aggacagata 1020
tccagtgcct catcccgtgt gccattgacc aggatccgta cttcagaatg acaagggacg 1080
tggcccccag gatcggccat cctaaacctg ccctgctgca ctcgaccttc ttccctgccc 1140
tgcagggcgc tcagaccaag atgagtgcca gtgaccccaa ctcctccatc ttcctcactg 1200
acacggccaa gcagatcaag agcaaggtca acaagcatgc attttctgga ggaagagata 1260
ctgtggagga gcacaggcag tttgggggca actgtgaagt ggatgtatcc ttcatgtatc 1320
tgaccttctt ccttgaagat gacgacaggc tggagcagat cagaaaggat tacaccagcg 1380
gagccatgct caccggggag ctcaagaaga cgctcataga cgtccttcag cccctgatcg 1440
cagagcacca ggcccgacgc aaggcggtca ctgaggagac cgtgaaggag ttcatgacgc 1500
cccggcagct gtccttccac ttccagtaat gtctcttttc tgtggcaagc ctgtctgtaa 1560
cccaccctgt caaatcaagt cacactccca ataggcttct cacgtggtaa ttcctggacc 1620
tgggtctctg tgcacgtgtc gatcctggat gctgtttctt ctctgatgtc tccccgtctg 1680
ttgtctgcta cgggtgatca agcgatgcta atgttgtgtg gtcagataga accccagccc 1740
gcagtctctc ccacaaagag cccagaggta gtgcttccct ggcccacaga agactgtcca 1800
gctagcagcc tggggttcca gaacaaattc tgaaatgtat tccagttttt ctttaaagtg 1860
cgtgtgcccg gttggccacg gtggggtgca ctgtatcatc tctgttctca ggaggctgag 1920
acacgaggtc tgagccagcc tggtagtaat ctgcctttga ttctgctgct gtggagtcac 1980
aatgtcatta ctgtaaaact gaacactgct gtgaggaact gacaaggaga catgtagtgg 2040
acatgccgct gctccagccc acgccccccc ccacacacgc acacacactt acatgtaatg 2100
tgtgcaaact ttgctttata cttgctaaga agccatcagc tttgttctga aggtgtagct 2160
cagtagtaga gcactcgcct agcaccctgc tggcccgggt tcagtctcct gccccacgaa 2220

gccgggcgga taactgagct ctctgctttt aaccctgatt atggaccagg ctgggtcttt 2280
ccactaacct gaggctgagg tagctaacct ccagctcccg tgtgccgtcc actccaaaat 2340
catacgttgc gcccttggtg tactaacttc cccacagatg gcccagactc caccctgtga 2400
cgtgcagggc tgcagctcct gcccccgggt cctgaggacg gccttgttcc ctttgcaggt 2460
gcttctgctt tgacacttga ctgacgggga gggcaggcca gagcagtgtg ctggccatgc 2520
ctcaggccgt tgaccatagc tagacagaaa agaacagcag ccgtccaggc cgcagcgttc 2580
tcctctgggc gggctgcatg gaatgagcct ccactcagag tcttggaaaa gcagaagccg 2640
tacctcaggg aaagcccctg gttgaagtgt ctgtcctctc tgaagcaaca agggccactc 2700
tgagtgttct ctccttgcat catttttagg aaaaaacaat aaactttgtt gagctccaaa 2760
aaaaaaaaaa aaaaaaaaaa aaaaaa 2786

<210> 67
<211> 364
<212> PRT
<213> Rattus norvegicus

<220>
<223> Aldolase A

<400> 67
Met Pro His Pro Tyr Pro Ala Leu Thr Pro Glu Gln Lys Lys Glu Leu
1               5                   10                  15

Ala Asp Ile Ala His Arg Ile Val Ala Pro Gly Lys Gly Ile Leu Ala
            20                  25                  30

Ala Asp Glu Ser Thr Gly Ser Ile Ala Lys Arg Leu Gln Ser Ile Gly
        35                  40                  45

Thr Glu Asn Thr Glu Glu Asn Arg Arg Phe Tyr Arg Gln Leu Leu Leu
    50                  55                  60

Thr Ala Asp Asp Arg Val Asn Pro Cys Ile Gly Gly Val Ile Leu Phe
65                  70                  75                  80

His Glu Thr Leu Tyr Gln Lys Ala Asp Asp Gly Arg Pro Phe Pro Gln
                85                  90                  95

Val Ile Lys Ser Lys Gly Gly Val Val Gly Ile Lys Val Asp Lys Gly
            100                 105                 110

Val Val Pro Leu Ala Gly Thr Asn Gly Glu Thr Thr Thr Gln Gly Leu
        115                 120                 125

Asp Gly Leu Ser Glu Arg Cys Ala Gln Tyr Lys Lys Asp Gly Ala Asp
    130                 135                 140

Phe Ala Lys Trp Arg Cys Val Leu Lys Ile Gly Glu His Thr Pro Ser
145                 150                 155                 160

Ser Leu Ala Ile Met Glu Asn Ala Asn Val Leu Ala Arg Tyr Ala Ser
                165                 170                 175

Ile Cys Gln Gln Asn Gly Ile Val Pro Ile Val Glu Pro Glu Ile Leu
            180                 185                 190

Pro Asp Gly Asp His Asp Leu Lys Arg Cys Gln Tyr Val Thr Glu Lys
        195                 200                 205

Val Leu Ala Ala Val Tyr Lys Ala Leu Ser Asp His His Val Tyr Leu

210                 215                 220

Glu Gly Thr Leu Leu Lys Pro Asn Met Val Thr Pro Gly His Ala Cys
225                 230                 235                 240

Thr Gln Lys Phe Ser Asn Glu Glu Ile Ala Met Ala Thr Val Thr Ala
                245                 250                 255

Leu Arg Arg Thr Val Pro Pro Ala Val Pro Gly Val Thr Phe Leu Ser
            260                 265                 270

Gly Gly Gln Ser Glu Glu Glu Ala Ser Ile Asn Leu Asn Ala Ile Asn
        275                 280                 285

Lys Cys Pro Leu Leu Lys Pro Trp Ala Leu Thr Phe Ser Tyr Gly Arg
    290                 295                 300

Ala Leu Gln Ala Ser Ala Leu Lys Ala Trp Gly Gly Lys Lys Glu Asn
305                 310                 315                 320

Leu Lys Ala Ala Gln Glu Glu Tyr Ile Lys Arg Ala Leu Ala Asn Ser
                325                 330                 335

Leu Ala Cys Gln Gly Lys Tyr Thr Pro Ser Gly Gln Ser Gly Ala Ala
            340                 345                 350

Ala Ser Glu Ser Leu Phe Ile Ser Asn His Ala Tyr
        355                 360


<210> 68
<211> 1442
<212> DNA
<213> Rattus norvegicus

<220>
<223> Aldolase A

<400> 68
gtccccccca ccccagctga ataggctgcg ttctcttgga acgcgccgca gaacgaggtt 60
ctgtgaccct agccgcgttc cctccttagt tcctttcgcc tacccacccg cgtacccgac 120
agacccaccc cgtcctgtgc caggaaagcg ctgccaccgg caccatgccc cacccatacc 180
cagcactgac cccggagcag aagaaggagc tggctgacat cgctcaccga attgtagctc 240
cgggcaaggg catcctggct gcagacgagt ccactggaag cattgccaag cgcctgcagt 300
ccattggcac cgagaacacc gaggagaaca ggcgcttcta ccgccaactg ctgctgactg 360
ccgatgaccg tgtgaatccc tgcattggag gggtgatcct tttccacgag acactgtacc 420
agaaggcaga tgatggccgt cccttccccc aagttatcaa gtccaagggt ggtgttgtgg 480
gcattaaggt agataagggt gtagtgcccc tggctggaac caatggcgag accactactc 540
aagggctgga cgggctgtct gagcgctgtg cccagtataa gaaggatgga gccgactttg 600
ccaagtggcg ctgtgtgcta aagattgggg agcatactcc ctcgtccctc gccatcatgg 660
aaaatgccaa tgttctggcc cgttacgcta gcatctgcca gcagaatggc attgtaccca 720
ttgtggagcc tgaaattctc cctgatgggg accatgactt gaagcgctgc cagtatgtaa 780
ctgagaaggt actggcagct gtctacaagg ctctgagtga ccaccatgtc tatctggaag 840
gcacactgct gaagcccaac atggtcaccc ctggccatgc ttgcacccag aaattttcca 900
atgaggaaat tgccatggca accgtcacag cacttcgtcg aacagtgccc cctgccgtcc 960
ctggggtcac tttcctgtct ggagggcaga gtgaggaaga ggcatccatc aacctcaatg 1020
ctatcaacaa gtgtcccctg ctgaagccat gggccttgac tttctcctat ggccgagccc 1080
tgcaggcctc tgctctaaag gcttggggtg ggaagaagga gaacctgaag gcagcccagg 1140
aggagtacat caagcgagcc ctggccaaca gcctcgcttg tcaaggaaag tacactccaa 1200
gtggccagtc tggagccgca gccagtgaat ctctcttcat ctctaaccat gcctactaac 1260
cagagctgat ctaaggctgc tccatcgaca ctccaggccc ctgcctaccc acttgctatt 1320

gaagaggggc cttcaggctc tttcccatca ctcttgctgc cctcgtgtgt gcagtgttgt 1380
ctgtgaatgc taaatctgcc atcccttcca gcccactgcc aataaacagc tatttaaggg 1440
gg 1442

<210> 69
<211> 887
<212> PRT
<213> Rattus norvegicus

<220>
<223> 3-hydroxy-3-methylglutaryl coenzyme A reductase

<400> 69
Met Leu Ser Arg Leu Phe Arg Met His Gly Leu Phe Val Ala Ser His
1               5                   10                  15

Pro Trp Glu Val Ile Val Gly Thr Val Thr Leu Thr Ile Cys Met Met
            20                  25                  30

Ser Met Asn Met Phe Thr Gly Asn Asn Lys Ile Cys Gly Trp Asn Tyr
        35                  40                  45

Glu Cys Pro Lys Phe Glu Glu Asp Val Leu Ser Ser Asp Ile Ile Ile
    50                  55                  60

Leu Thr Ile Thr Arg Cys Ile Ala Ile Leu Tyr Ile Tyr Phe Gln Phe
65                  70                  75                  80

Gln Asn Leu Arg Gln Leu Gly Ser Lys Tyr Ile Leu Gly Ile Ala Gly
                85                  90                  95

Leu Phe Thr Ile Phe Ser Ser Phe Val Phe Ser Thr Val Val Ile His
            100                 105                 110

Phe Leu Asp Lys Glu Leu Thr Gly Leu Asn Glu Ala Leu Pro Phe Phe
        115                 120                 125

Leu Leu Leu Ile Asp Leu Ser Arg Ala Ser Ala Leu Ala Lys Phe Ala
    130                 135                 140

Leu Ser Ser Asn Ser Gln Asp Glu Val Arg Glu Asn Ile Ala Arg Gly
145                 150                 155                 160

Met Ala Ile Leu Gly Pro Thr Phe Thr Leu Asp Ala Leu Val Glu Cys
                165                 170                 175

Leu Val Ile Gly Val Gly Thr Met Ser Gly Val Arg Gln Leu Glu Ile
            180                 185                 190

Met Cys Cys Phe Gly Cys Met Ser Val Leu Ala Asn Tyr Phe Val Phe
        195                 200                 205

Met Thr Phe Phe Pro Ala Cys Val Ser Leu Val Leu Glu Leu Ser Arg
    210                 215                 220

Glu Ser Arg Glu Gly Arg Pro Ile Trp Gln Leu Ser His Phe Ala Arg
225                 230                 235                 240

Val Leu Glu Glu Glu Glu Asn Lys Pro Asn Pro Val Thr Gln Arg Val
                245                 250                 255

Lys Met Ile Met Ser Leu Gly Leu Val Leu Val His Ala His Ser Arg
            260                 265                 270

Trp Ile Ala Asp Pro Ser Pro Gln Asn Ser Thr Ala Glu Gln Ser Lys
        275                 280                 285

Val Ser Leu Gly Leu Ala Glu Asp Val Ser Lys Arg Ile Glu Pro Ser
    290                 295                 300

Val Ser Leu Trp Gln Phe Tyr Leu Ser Lys Met Ile Ser Met Asp Ile
305                 310                 315                 320

Glu Gln Val Ile Thr Leu Ser Leu Ala Leu Leu Leu Ala Val Lys Tyr
                325                 330                 335

Ile Phe Phe Glu Gln Ala Glu Thr Glu Ser Thr Leu Ser Leu Lys Asn
            340                 345                 350

Pro Ile Thr Ser Pro Val Val Thr Pro Lys Lys Ala Gln Asp Asn Cys
        355                 360                 365

Cys Arg Arg Glu Pro Leu Leu Val Arg Arg Asn Gln Lys Leu Ser Ser
    370                 375                 380

Val Glu Glu Asp Pro Gly Val Asn Gln Asp Arg Lys Val Glu Val Ile
385                 390                 395                 400

Lys Pro Leu Val Ala Glu Ala Glu Thr Ser Gly Arg Ala Thr Phe Val
                405                 410                 415

Leu Gly Ala Ser Ala Ala Ser Pro Pro Leu Ala Leu Gly Ala Gln Glu
            420                 425                 430

Pro Gly Ile Glu Leu Pro Ser Glu Pro Arg Pro Asn Glu Glu Cys Leu
        435                 440                 445

Gln Ile Leu Glu Ser Ala Glu Lys Gly Ala Lys Phe Leu Ser Asp Ala
    450                 455                 460

Glu Ile Ile Gln Leu Val Asn Ala Lys His Ile Pro Ala Tyr Lys Leu
465                 470                 475                 480

Glu Thr Leu Met Glu Thr His Glu Arg Gly Val Ser Ile Arg Arg Gln
                485                 490                 495

Leu Leu Ser Ala Lys Leu Ala Glu Pro Ser Ser Leu Gln Tyr Leu Pro
            500                 505                 510

Tyr Arg Asp Tyr Asn Tyr Ser Leu Val Met Gly Ala Cys Cys Glu Asn
        515                 520                 525

Val Ile Gly Tyr Met Pro Ile Pro Val Gly Val Ala Gly Pro Leu Cys
    530                 535                 540

Leu Asp Gly Lys Glu Tyr Gln Val Pro Met Ala Thr Thr Glu Gly Cys
545                 550                 555                 560

Leu Val Ala Ser Thr Asn Arg Gly Cys Arg Ala Ile Ser Leu Gly Gly
                565                 570                 575

Gly Ala Ser Ser Arg Val Leu Ala Asp Gly Met Ser Arg Gly Pro Val
            580                 585                 590

Val Arg Leu Pro Arg Ala Cys Asp Ser Ala Glu Val Lys Ser Trp Leu
        595                 600                 605

Glu Thr Pro Glu Gly Phe Ala Val Val Lys Glu Ala Phe Asp Ser Thr
    610                 615                 620

Ser Arg Phe Ala Arg Leu Gln Lys Leu His Val Thr Leu Ala Gly Arg
625                 630                 635                 640

Asn Leu Tyr Ile Arg Leu Gln Ser Lys Thr Gly Asp Ala Met Gly Met
                645                 650                 655

Asn Met Ile Ser Lys Gly Thr Glu Lys Ala Leu Leu Lys Leu Gln Glu
            660                 665                 670

Gly Val Pro Glu Leu Gln Ile Leu Ala Val Ser Gly Asn Tyr Cys Thr
        675                 680                 685

Asp Lys Lys Pro Ala Ala Ile Asn Trp Ile Glu Gly Arg Gly Lys Thr
    690                 695                 700

Val Val Cys Glu Ala Val Ile Pro Ala Lys Val Val Arg Glu Val Leu
705                 710                 715                 720

Lys Thr Thr Thr Glu Ala Met Val Asp Val Asn Ile Asn Lys Asn Leu
                725                 730                 735

Val Gly Ser Ala Met Ala Gly Ser Ile Gly Gly Tyr Asn Leu His Ala
            740                 745                 750

Ala Asn Ile Val Thr Ala Ile Tyr Ile Ala Cys Gly Gln Asp Ala Ala
        755                 760                 765

Gln Asn Val Gly Ser Ser Asn Cys Ile Thr Leu Met Glu Ala Ser Gly
    770                 775                 780

Pro Thr Asn Glu Asp Leu Tyr Ile Ser Cys Thr Met Pro Ser Ile Glu
785                 790                 795                 800

Ile Gly Thr Val Gly Gly Gly Thr Asn Leu Leu Pro Gln Gln Ala Cys
                805                 810                 815

Leu Gln Met Leu Gly Val Gln Gly Ala Cys Lys Asp Asn Pro Gly Glu
            820                 825                 830

Asn Ala Arg Gln Leu Ala Arg Ile Val Cys Gly Thr Val Met Ala Gly
        835                 840                 845

Glu Leu Ser Leu Met Ala Ala Leu Ala Ala Gly His Leu Val Arg Ser
    850                 855                 860

His Met Val His Asn Arg Ser Lys Ile Asn Leu Gln Asp Leu Gln Gly
865                 870                 875                 880

Thr Cys Thr Lys Lys Ala Ala
                885

<210> 70
<211> 4346
<212> DNA
<213> Rattus norvegicus

<220>
<223> 3-hydroxy-3-methylglutaryl coenzyme A reductase

<220>
<221> misc_feature
<222> 31
<223> n is a or g or c or t

<400> 70
   ataagcgcag ttccttccgc cctggtctcc nttggctgga gacggcggaa gggcggcttg 60
   gtggcctcca ttgagatccg gaggatccaa ggactgtgta gctacaatgt tgtcaagact 120
   tttccgtatg catggcctct ttgtggcctc ccatccctgg gaggtaattg tgggaacggt 180
   gacacttact atctgtatga tgtccatgaa catgttcacc ggcaacaaca agatctgtgg 240
   ttggaattat gagtgcccaa aatttgaaga ggacgtgctg agcagcgaca tcatcatcct 300
   cacgataacc cggtgcatcg ccatcctgta catctacttc cagttccaga acctgcgtca 360
   gcttgggtca aagtacattt tgggtattgc cggcctcttc acaattttct caagtttcgt 420
   cttcagcact gtcgtcattc atttcctcga caaagaattg acaggcttaa atgaagcttt 480
   gccctttttc ctgctcttga ttgacctttc tagagcgagt gcattggcca agtttgccct 540
   gagttcaaac tcacaggatg aagtaaggga gaatatagcg cgtgggatgg cgatcctggg 600
   ccccacgttc acccttgacg ctctggtgga atgtcttgtg attggagttg gcaccatgtc 660
   aggggtgcgg cagcttgaga tcatgtgctg ctttggctgt atgtccgtgc ttgccaacta 720
   ctttgtcttc atgacattct tcccagcctg cgtgtccctg gtcctagagc tttctcggga 780
   aagccgtgag ggtcgtccaa tttggcagct cagccatttt gccagagttt tagaagaaga 840
   agagaataaa ccaaacccag taacccaaag ggtcaagatg atcatgtctt taggcctggt 900
   tcttgttcac gctcacagtc gctggatagc tgatccttct cctcagaaca gcacagcaga 960
   acagtctaag gtttccttgg gtctggctga agatgtgtcc aagagaattg agccgagtgt 1020
   ttctctctgg cagttttacc tctccaagat gatcagcatg gacatcgagc aagtgattac 1080
   cctgagctta gcgttgcttt tggctgtcaa gtatattttc tttgaacaag cagagacaga 1140
   atcaacactc tcattaaaaa atcctatcac atctcctgtc gtgaccccaa agaaagctca 1200
   agacaactgt tgtagacgtg agcctctgct tgtgaggagg aaccagaagc tttcgtcagt 1260
   ggaggaggat ccaggagtga accaagacag aaaagttgag gttataaaac ctttagtggc 1320
   agaagccgag acttcgggca gagctacgtt tgtgcttggc gcctctgcag ccagccctcc 1380
   attggccctg ggggcacagg agcctgggat cgaactcccc agcgagcctc gacctaatga 1440
   agagtgtcta cagatactgg agagtgcaga gaaaggtgcg aagttcctta gtgatgcaga 1500
   gatcatccag ttggtcaatg ctaagcacat cccagcctac aaactggaaa ccctcatgga 1560
   gacgcacgag cgtggtgtgt ctattcgccg gcagctcctc tccgccaagc ttgcagagcc 1620
   atcttctctg cagtacctgc cttacagaga ctataattac tccttggtga tgggagcttg 1680
   ctgtgagaac gtgatcggat atatgcccat ccctgttgga gtggcaggac ctctgtgcct 1740
   ggatggaaaa gagtaccagg tgccaatggc aacaacagaa ggttgtcttg tggccagcac 1800
   gaacagaggc tgcagagcga tcagtcttgg tggaggtgcc agcagccggg tccttgcaga 1860
   tgggatgagc cgaggcccag tggtgcgtct tcctcgtgct tgtgactcag cagaggtgaa 1920
   gagctggctt gaaacacctg aagggtttgc agtggtaaag gaggccttcg acagcacgag 1980
   cagatttgca cgtctacaga aacttcacgt gacgctggca ggacgcaacc tctacatccg 2040
   tctccagtcc aaaacggggg acgccatggg gatgaacatg atttccaagg gtacggagaa 2100
   agcacttctg aagctgcaag agggcgtgcc ggagctgcag atactggcgg tcagtggtaa 2160
   ctattgcacc gacaagaaac ctgctgccat aaactggatc gaagggagag gaaagactgt 2220
   ggtttgtgaa gctgtcattc cagccaaggt ggtgagagaa gtattaaaga cgactacgga 2280
   agctatggtt gacgtaaaca ttaacaagaa tcttgtgggc tctgccatgg ctggtagcat 2340
   aggaggctac aacctccatg ctgccaacat cgtcactgcc atctacattg catgtggcca 2400
   ggatgcagca cagaatgtgg ggagttcaaa ctgtattacg ttaatggaag caagtggtcc 2460
   cacaaatgaa gacttataca tcagctgtac catgccgtct atagagatcg gaaccgtggg 2520
   tggtgggacc aaccttctac ctcagcaagc ctgcctgcag atgctaggtg ttcaaggggc 2580
   gtgcaaagac aatcctggag aaaatgcacg gcagcttgca cgaattgtgt gtggcactgt 2640
   gatggctggt gagttgtcct tgatggcagc attggcagca ggacatcttg tcagaagtca 2700
   catggttcac aacagatcaa agataaattt acaagatctg caaggaacat gcaccaagaa 2760
   ggcagcttga gcatcctgac atactggact gaaacacggg cattgggttc tccaggacta 2820
   acatgaaatc tgtgaattaa aaatgtcagt gcagtgtctt gtggaagatg aacgtgatca 2880
   gtgaggcagg cacctgcttg gtttctggcc tctttagaga cgtctgcggt cctttgcaca 2940
   gactcctcag acgtgggaac tatggttctt tccgtgccgt gttctagaaa atctcatgtg 3000
   gatgtcatgt gctctgagca ccacagatgt gactgcagct cgtttctgaa agctgccaca 3060
   agctggaagc tggtgttttg acgaaatgat ggatcctggt gatcagtgtg gggctcacct 3120
   ccaatgggtt aaaatggagt tttaaatgac actgtagctg acagaacttc tgatttttat 3180
   ttattcagtc tgggttgtag aactttgcaa tctaagttta ttttttgtaa actaataatt 3240
   catttggtgc tggtctattg atttttgggg acctacttct tcatggaaga attactttta 3300
   ttctcaaact acagaataat gtgctaagta gtgctaaata gttctcgacg aagaaaagag 3360
   tcgctgtgtt catctcagtc tttgttcaga gaggccttta gctgtgcaca gtctgcacta 3420
   aagacacaga gctcttagtg ttagagcaga gtacaagcag gtcggacctc tcaggactta 3480
   gagtgtggga gcagttttta aagaaaacaa ccattgctct aagccaactt tcttgggaga 3540
   cattttaact tatttagctg aattctagat ttcttggtaa actatcaaat ctgtatatgt 3600
   tgtaataaag tgtctaatgc taggagttta ttggaagtgt ttaagaaata aaagtactca 3660
   tcagctttta cacggataaa atgctctagc ttgggccaga gaagacctcg ctcaggagca 3720
   ttggtccggg aaagcctggc ttgcttgccg agcctaatga agggaagtca gcctacagag 3780
   ccagtgatgg agccacatga atggccctgg agctgtgtgc cttgttcctg tggccaggag 3840
   ttaggtgact gaatcattta tgggctcctt tggtggaccc cataaaagct cttagcttcc 3900
   ctcagggggg gtcagcagag ttgttgaatc ttaatttttt ttttaatgta ccagtttgtg 3960
   ataaataata ataaagagct ccttattttg tattctatct aatgcttcgt ttggtcttgg 4020
   aagcggacgt ctgtgtagaa gacggactct gaaagtgagc atcgatgacg cgcgcgcgcg 4080
   cgactgacgc tagtgatggc tagatatgtg gaaggtagct tcgtgtgtat gtaaagtttc 4140
   cagttgcgcc tcttgtgact tttcagccag taccatttta tttacctgag cttgtcgtgg 4200
   gaagtggtag taaaccgcat tgagtgttga tgctggtgac ttgtgatcag tgtcatcgtg 4260
   ctaagtccat gttttgtaca attactaaat tgtatacatt ttgttataga atactttttc 4320
   cagttgagta attatgaaag gaagtt 4346

<210> 71
<211> 610
<212> PRT
<213> Rattus norvegicus

<220>
<223> NADH subunit 5

<400> 71
Met Asn Met Met Thr Ile Leu Ile Leu Met Ile Leu Asp Leu Leu Thr
1               5                   10                  15

Thr Pro Ile Thr Phe Ser Met Thr Ile Leu Thr Lys Leu Met Tyr Phe
            20                  25                  30

Arg His Met Leu Ile Thr Ser Ile Lys Phe Ser Phe Leu Leu Ser Leu
        35                  40                  45

Leu Pro Leu Leu Leu Phe Phe His His Asn Thr Glu Tyr Met Ile Thr
    50                  55                  60

Asn Trp His Trp Leu Thr Ile Asn Ser Ile Lys Leu Thr Met Ser Phe
65                  70                  75                  80

Lys Ile Asp Tyr Phe Ser Ile Leu Phe Leu Ser Val Ser Leu Phe Val
                85                  90                  95

Thr Trp Ser Ile Met Gln Phe Ser Ser Trp Tyr Met His Ser Asp Pro
            100                 105                 110

His Ile Asn Arg Phe Ile Lys Tyr Leu Met Met Phe Leu Asn Asn Met
        115                 120                 125

Leu Ile Leu Thr Ser Ala Asn Asn Leu Phe Gln Leu Phe Ile Gly Trp
    130                 135                 140

Glu Gly Val Gly Ile Met Ser Phe Leu Leu Ile Gly Trp Trp Tyr Gly
145                 150                 155                 160

Leu Ala Asp Ala Asn Thr Ala Ala Leu Gln Ala Ile Leu Tyr Asn Arg
                165                 170                 175

Val Gly Asp Ile Gly Phe Ile Leu Ala Met Thr Trp Phe Cys Leu Asn
            180                 185                 190

Met Asn Ser Trp Glu Leu Gln Gln Ile Phe Leu Thr Asn Thr Asn Asn
        195                 200                 205

Asn Leu Val Pro Leu Thr Gly Leu Leu Ile Ala Ala Thr Gly Lys Ser
    210                 215                 220

Ala Gln Phe Gly Leu His Pro Trp Leu Pro Ser Ala Met Glu Gly Pro
225                 230                 235                 240

Thr Pro Val Ser Ala Leu Leu His Ser Ser Thr Met Val Val Ala Gly
                245                 250                 255

Ile Phe Leu Met Ile Arg Phe His Pro Leu Thr Ser Asn Asn Ser Thr
            260                 265                 270

Ile Met Thr Ala Met Leu Cys Leu Gly Ala Ile Thr Thr Leu Phe Thr
        275                 280                 285

Ala Ile Cys Ala Leu Thr Gln Asn Asp Ile Lys Lys Ile Val Ala Phe
    290                 295                 300

Ser Thr Ser Ser Gln Leu Gly Leu Met Met Val Thr Leu Gly Ile Asn
305                 310                 315                 320

Gln Pro Tyr Leu Ala Phe Leu His Ile Cys Thr His Ala Phe Phe Lys
                325                 330                 335

Ala Met Leu Phe Met Cys Ser Gly Ser Ile Ile His Ile Leu Asn Asp
            340                 345                 350

Glu Gln Asp Ile Arg Lys Met Gly Asn Met Met Lys Ala Met Pro Phe
        355                 360                 365

Thr Ser Ser Cys Leu Ile Ile Gly Ser Leu Ala Leu Thr Gly Met Pro
    370                 375                 380

Phe Leu Thr Gly Phe Tyr Ser Lys Asp Leu Ile Ile Glu Ala Ile Asn
385                 390                 395                 400

Thr Cys Asn Thr Asn Ala Trp Ala Leu Met Ile Thr Leu Ile Ala Thr
                405                 410                 415

Ser Met Thr Ala Val Tyr Ser Met Arg Ile Ile Tyr Phe Val Thr Met
            420                 425                 430

Thr Lys Pro Arg Tyr Ser Pro Leu Ile Thr Ile Asn Glu Asn Asn Pro
        435                 440                 445

Asn Leu Ile Asn Pro Ile Lys Arg Leu Ala Leu Gly Ser Ile Leu Ala

450                 455                 460

Gly Phe Leu Ile Ser Leu Asn Ile Pro Pro Thr Asn Ile Gln Ile Leu
465                 470                 475                 480

Thr Met Pro Trp His Leu Lys Met Thr Ala Leu Leu Ile Thr Ile Leu
                485                 490                 495

Gly Phe Ala Ile Ala Leu Glu Leu Asn Asn Leu Thr Leu Asn Leu Ser
            500                 505                 510

Met Ser Lys Pro Thr Lys Leu Ser Ser Phe Ser Thr Ser Leu Gly Tyr
        515                 520                 525

Tyr Pro Pro Ile Met Pro Arg Ile Ile Pro Gln Lys Thr Leu Asn Ser
    530                 535                 540

Ser Tyr Lys Leu Ser Leu Asn Leu Leu Asp Leu Ile Trp Ser Glu Lys
545                 550                 555                 560

Thr Ile Pro Lys Ser Thr Ser Ile Thr Gln Thr Gln Leu Ser Lys Met
                565                 570                 575

Met Ser Asn Gln Lys Gly Leu Ile Lys Leu Tyr Phe Leu Ser Phe Leu
            580                 585                 590

Ile Thr Ile Ser Leu Ile Phe Ile Leu His Thr Leu Asn Pro Glu Trp
        595                 600                 605

Phe Gln
    610


<210> 72
<211> 300
<212> PRT
<213> Rattus norvegicus

<220>
<223> Electron transfer flavoprotein alpha-subunit

<400> 72
Glu Gly Thr Ala Gly Leu Thr Arg Thr Glu Thr Met Phe Arg Ala Ala
  1               5                  10                  15

Ala Pro Gly Gln Leu Arg Arg Ala Ala Ser Leu Leu Arg Phe Gln Ser
             20                  25                  30

Thr Leu Val Ile Ala Glu His Ala Asn Asp Ser Leu Ala Pro Ile Thr
         35                  40                  45

Leu Asn Thr Ile Thr Ala Ala Gly Arg Leu Gly Gly Glu Val Ser Cys
     50                  55                  60

Leu Val Ala Gly Thr Lys Cys Asp Lys Val Val Gln Asp Leu Cys Lys
 65                  70                  75                  80

Val Ala Gly Val Ala Lys Val Leu Val Ala Gln His Asp Ala Tyr Lys
                 85                  90                  95

Gly Leu Leu Pro Glu Glu Leu Thr Pro Leu Ile Leu Glu Thr Gln Lys

            100                 105                 110

Gln Phe Ser Tyr Thr His Ile Val Ala Gly Ala Ser Ala Phe Gly Lys
        115                 120                 125

Asn Leu Leu Pro Arg Val Ala Ala Lys Leu Asn Val Ala Pro Val Ser
    130                 135                 140

Asp Ile Ile Glu Ile Lys Ser Pro Asp Thr Phe Val Arg Thr Ile Tyr
145                 150                 155                 160

Ala Ala Asn Ala Leu Cys Thr Val Lys Cys Asp Glu Lys Val Lys Val
                165                 170                 175

Phe Ser Val Arg Gly Thr Ser Phe Glu Ala Ala Ala Ala Ser Gly Gly
            180                 185                 190

Ser Ala Ser Ser Glu Lys Ala Pro Ser Ser Ser Ser Ala Gly Ile Ser
        195                 200                 205

Glu Trp Leu Asp Gln Lys Leu Thr Lys Ser Asp Arg Pro Glu Leu Thr
    210                 215                 220

Gly Ala Lys Val Val Val Ser Gly Gly Arg Gly Leu Lys Ser Gly Glu
225                 230                 235                 240

Asn Phe Lys Leu Gln Tyr Asp Leu Ala Asp Gln Leu His Ala Ala Val
                245                 250                 255

Gly Ala Ser Arg Ala Ala Val Asp Ala Gly Phe Val Pro Asn Asp Met
            260                 265                 270

Gln Val Gly Gln Thr Gly Lys Ile Val Ala Pro Glu Leu Tyr Ile Ala
        275                 280                 285

Val Gly Ile Ser Gly Ala Ile Gln His Leu Ala Gly
    290                 295                 300

<210> 73
<211> 1241
<212> DNA
<213> Rattus norvegicus

<220>
<223> Electron transfer flavoprotein alpha-subunit

<400> 73
gagggtaccg ctgggctgac tagaacagag acaatgtttc gagcagcggc gcctgggcag 60
ctccggcggg cggcctcgtt gctccgtttt cagagcacct tggtaatagc tgagcatgcg 120
aatgattctc tagcacctat tactctaaac actatcactg cagctggacg tcttggaggt 180
gaagtgtcct gcttagtagc tggaacaaaa tgtgacaagg tggtacagga tctctgtaaa 240
gtagcaggcg tggcaaaggt tctggtggct cagcatgatg cgtacaaagg ccttcttcca 300
gaggaactca caccattgat tttggaaact cagaagcagt tcagttacac acacatcgtt 360
gctggagcat ctgcttttgg aaagaacctt ctgcccagag tagcagccaa acttaatgtt 420
gccccagttt ctgacatcat tgagatcaag tcacctgaca catttgtgag aactatctat 480
gcagcaaatg cattgtgtac agtgaaatgt gatgagaaag tgaaggtgtt ttctgttcga 540
ggaacatctt ttgaggctgc agcagcaagt ggaggtagtg ccagttcaga aaaggcacca 600
agttcttcat cagcaggaat atcagagtgg cttgaccaga aattgacaaa aagtgaccga 660
ccagagctaa ctggtgccaa agtggtggta tctggtggtc ggggcttgaa gagtggagag 720
aactttaaac tgcagtatga cttggcagat cagctgcatg ctgcagttgg tgcttcccgt 780

gctgctgttg atgctggctt tgttcccaat gacatgcaag ttggtcagac aggaaaaata 840
gtagctccag aactttatat tgctgttgga atatctggag ccatccagca tttagctgga 900
tgaaggacag caagacaatt gtggctatta acaaagaccc agaagctcca attttccaag 960
tggcagatta tggaatagtt gcagatttat ttaaggtggt tcctgaaatg acagaaatac 1020
tgaagaaaaa ataagtcaaa atcatatctt aaggaaactg tcaaaagtat tcagattgaa 1080
atcatagcaa cttcataatc caaagaaaga aaatatgcta acagcagcta caatgccttt 1140
tgatgaatca attattatat tcctttttaa ttttttgtga ttccaaacaa ttatttttac 1200
attttctaat tctataataa aaaatataat aaagcgaatt c 1241

<210> 74
<211> 587
<212> PRT
<213> Rattus norvegicus

<220>
<223> Erythroid-specific delta-aminolevulinate synthase

<400> 74
Met Val Ala Ala Ala Met Leu Leu Arg Ser Cys Pro Val Leu Ser Lys
1               5                   10                  15

Gly Pro Thr Gly Leu Leu Gly Lys Val Ala Lys Thr Tyr Gln Phe Leu
            20                  25                  30

Phe Gly Ile Gly Arg Cys Pro Ile Leu Ala Thr Gln Gly Pro Thr Cys
        35                  40                  45

Ser Gln Ile His Leu Lys Ala Thr Lys Ala Gly Ala Asp Ser Pro Ser
    50                  55                  60

Trp Thr Lys Ser His Cys Pro Phe Met Leu Ser Glu Leu Gln Asp Arg
65                  70                  75                  80

Lys Ser Lys Ile Val Gln Arg Ala Ala Pro Glu Val Gln Glu Asp Val
                85                  90                  95

Lys Thr Phe Lys Thr Asp Leu Leu Ser Phe Met Glu Ser Thr Thr Arg
            100                 105                 110

Ser Gln Ser Val Pro Arg Phe Gln Asp Pro Glu Gln Thr Gly Gly Ala
        115                 120                 125

Pro Pro Leu Leu Ile Gln Asn Asn Met Thr Gly Ser Gln Ala Phe Gly
    130                 135                 140

Tyr Asp Gln Phe Phe Arg Asp Lys Ile Met Glu Lys Lys Gln Asp His
145                 150                 155                 160

Thr Tyr Arg Val Phe Lys Thr Val Asn Arg Trp Ala Asn Ala Tyr Pro
                165                 170                 175

Phe Ala Gln His Phe Ser Glu Ala Ser Met Asp Ser Lys Asp Val Ser
            180                 185                 190

Val Trp Cys Ser Asn Asp Tyr Leu Gly Ile Ser Arg His Pro Arg Val
        195                 200                 205

Leu Gln Ala Ile Glu Glu Thr Leu Lys Asn His Gly Ala Gly Ala Gly
    210                 215                 220

Gly Thr Arg Asn Ile Ser Gly Thr Ser Lys Phe His Val Glu Leu Glu
225                 230                 235                 240

Gln Glu Leu Ala Glu Leu His His Lys Asp Ser Ala Leu Leu Phe Ser
                245                 250                 255

Ser Cys Phe Val Ala Asn Asp Ser Thr Leu Phe Thr Leu Ala Lys Leu
            260                 265                 270

Leu Pro Gly Cys Glu Ile Tyr Ser Asp Ala Gly Asn His Ala Ser Met
        275                 280                 285

Ile Gln Gly Ile Arg Asn Ser Gly Ala Val Lys Phe Val Phe Arg His
    290                 295                 300

Asn Asp Pro Gly His Leu Lys Lys Leu Leu Glu Lys Ser Asp Pro Lys
305                 310                 315                 320

Thr Pro Lys Ile Val Ala Phe Glu Thr Val His Ser Met Asp Gly Ala
                325                 330                 335

Ile Cys Pro Leu Glu Glu Leu Cys Asp Val Ala His Gln Tyr Gly Ala
            340                 345                 350

Leu Thr Phe Val Asp Glu Val His Ala Val Gly Leu Tyr Gly Thr Arg
        355                 360                 365

Gly Ala Gly Ile Gly Glu Arg Asp Gly Ile Met His Lys Leu Asp Ile
    370                 375                 380

Ile Ser Gly Thr Leu Gly Lys Ala Phe Gly Cys Val Gly Gly Tyr Ile
385                 390                 395                 400

Ala Ser Thr Arg Asp Leu Val Asp Met Val Arg Ser Tyr Ala Ala Gly
                405                 410                 415

Phe Ile Phe Thr Thr Ser Leu Pro Pro Met Val Leu Ser Gly Ala Leu
            420                 425                 430

Glu Ser Val Arg Leu Leu Lys Gly Glu Glu Gly Gln Ala Leu Arg Arg
        435                 440                 445

Ala His Gln Arg Asn Val Lys His Met Arg Gln Leu Leu Met Asp Arg
    450                 455                 460

Gly Phe Pro Val Ile Pro Cys Pro Ser His Ile Ile Pro Ile Arg Val
465                 470                 475                 480

Gly Asn Ala Ala Leu Asn Ser Lys Ile Cys Asp Leu Leu Leu Ala Lys
                485                 490                 495

His Ser Ile Tyr Val Gln Ala Ile Asn Tyr Pro Thr Val Pro Arg Gly
            500                 505                 510

Glu Glu Leu Leu Arg Leu Ala Pro Ser Pro His His Ser Pro Gln Met
        515                 520                 525

Met Glu Asn Phe Val Glu Lys Leu Leu Leu Ala Trp Thr Glu Val Gly
    530                 535                 540

Leu Pro Leu Gln Asp Val Ser Val Ala Ala Cys Asn Phe Cys Arg Arg

545                 550                 555                 560

Pro Val His Phe Glu Leu Met Ser Glu Trp Glu Arg Ser Tyr Phe Gly
                565                 570                 575

Asn Met Gly Pro Gln Tyr Val Thr Thr Tyr Ala
            580                 585


<210> 75
<211> 1899
<212> DNA
<213> Rattus norvegicus

<220>
<223> Erythroid-specific delta-aminolevulinate synthase

<400> 75
gactttgggc tcaggatggt ggcagcagct atgttgctac ggtcctgtcc agtgctctct 60
aagggcccca caggcctcct aggcaaagtg gctaaaactt accagttcct atttggtatt 120
ggacgctgcc ccatcctggc cactcaagga ccaacctgtt ctcaaatcca tcttaaggca 180
accaaggctg gagcagactc tccgtcttgg actaagagcc attgtccttt catgctgtca 240
gaactccaag acaggaagag caagattgtg cagagggcag ctccagaagt ccaagaggat 300
gtcaagactt tcaagacaga cctgctgagc ttcatggagt caactacccg aagccaatca 360
gtgcctcgtt tccaggatcc agagcagact ggaggggcac ctcccctgct gattcagaat 420
aatatgactg gaagccaggc ttttggttat gaccaatttt ttagagacaa gatcatggaa 480
aagaagcagg accacacata ccgtgtgttc aagactgtga atcgctgggc taatgcctac 540
ccctttgccc aacacttctc cgaggcatct atggactcaa aggatgtttc tgtctggtgc 600
agtaatgact atttgggcat aagcaggcac cctcgtgtct tgcaggccat agaggagacc 660
ctgaagaatc atggagctgg agctgggggc actcgcaata tctcaggtac aagcaagttt 720
catgtggagc ttgaacagga gctggctgaa ctacatcata aggactcggc tctgctcttc 780
tcctcctgtt ttgtggccaa tgattctact ctctttacac tggccaagct tctgccaggg 840
tgtgagatct actcagacgc aggcaatcat gcctccatga tccaaggcat tcgtaacagt 900
ggtgcagtca agtttgtctt cagacacaat gacccaggcc acctgaagaa acttcttgag 960
aagtccgatc ccaagacacc aaaaattgtg gcttttgaga ctgttcactc catggatggt 1020
gccatctgtc ctctggagga attgtgtgat gtggcccacc agtacggggc cctgaccttc 1080
gtggatgaag tccatgccgt aggactatat ggaacccggg gtgcagggat cggggagcgt 1140
gatggaatta tgcacaagct tgacatcatc tctggaactc ttggcaaggc ctttggctgt 1200
gtcggtggct atatcgccag cactcgggac ttggtggaca tggtgcgctc ctacgctgca 1260
ggcttcatct ttaccacttc actgcctccc atggtgctct ctggagctct agagtctgtg 1320
cgtctactca agggagagga gggtcaagcc ctgaggcgtg cacaccagcg caacgtcaaa 1380
catatgcgcc agctactaat ggacaggggc tttcctgtta tcccctgtcc cagccacatc 1440
atccccataa gggtgggtaa tgcggcactc aacagcaaga tctgtgatct tctgctcgcc 1500
aagcacagca tctatgtgca ggccatcaac tacccaactg tgcctcgtgg tgaggagctg 1560
ctgcgcttgg ccccctcccc ccaccacagc cctcagatga tggaaaactt tgtggagaag 1620
ttgctgctgg cctggactga ggtggggttg cccctccaag atgtgtctgt ggctgcatgc 1680
aacttctgcc gtcgtcctgt gcactttgag cttatgagcg agtgggagcg atcctacttt 1740
gggaacatgg gaccccaata tgttactacc tatgcctaag gagccagctg ccttagatgc 1800
caaccccacc tgcactcccc ctggggatgg tcttcctcct actctctgct ttgctgtgtg 1860
cttctagctg acttgattct aaaaataaag agcaacctg                        1899


<210> 76
<211> 559
<212> PRT
<213> Rattus norvegicus

<220>
<223> Polypeptide GalNAc transferase

<400> 76

Met Arg Lys Phe Ala Tyr Cys Lys Val Val Leu Ala Thr Ser Leu Val
1               5                   10                  15

Trp Val Leu Leu Asp Met Phe Leu Leu Leu Tyr Phe Ser Glu Cys Asn
            20                  25                  30

Lys Cys Glu Glu Lys Lys Glu Arg Gly Leu Pro Ala Gly Asp Val Leu
        35                  40                  45

Glu Leu Val Gln Lys Pro His Glu Gly Pro Gly Glu Met Gly Lys Pro
    50                  55                  60

Val Val Ile Pro Lys Glu Asp Gln Glu Lys Met Lys Glu Met Phe Lys
65                  70                  75                  80

Ile Asn Gln Phe Asn Leu Met Ala Ser Glu Met Ile Ala Phe Asn Arg
                85                  90                  95

Ser Leu Pro Asp Val Arg Leu Glu Gly Cys Lys Thr Lys Val Tyr Pro
            100                 105                 110

Asp Ser Leu Pro Thr Thr Ser Val Val Ile Val Phe His Asn Glu Ala
        115                 120                 125

Trp Ser Thr Leu Leu Arg Thr Val His Ser Val Ile Asn Arg Ser Pro
    130                 135                 140

Arg His Met Ile Glu Glu Ile Val Leu Val Asp Asp Ala Ser Glu Arg
145                 150                 155                 160

Asp Phe Leu Lys Arg Pro Leu Glu Ser Tyr Val Lys Lys Leu Lys Val
                165                 170                 175

Pro Val His Val Ile Arg Met Glu Gln Arg Ser Gly Leu Ile Arg Ala
            180                 185                 190

Arg Leu Lys Gly Ala Ala Val Ser Lys Gly Gln Val Ile Thr Phe Leu
        195                 200                 205

Asp Ala His Cys Glu Cys Thr Val Gly Trp Leu Glu Pro Leu Leu Ala
    210                 215                 220

Arg Ile Lys His Asp Arg Arg Thr Val Val Cys Pro Ile Ile Asp Val
225                 230                 235                 240

Ile Ser Asp Asp Thr Phe Glu Tyr Met Ala Gly Ser Asp Met Thr Tyr
                245                 250                 255

Gly Gly Phe Asn Trp Lys Leu Asn Phe Arg Trp Tyr Pro Val Pro Gln
            260                 265                 270

Arg Glu Met Asp Arg Arg Lys Gly Asp Arg Thr Leu Pro Val Arg Thr
        275                 280                 285

Pro Thr Met Ala Gly Gly Leu Phe Ser Ile Asp Arg Asp Tyr Phe Gln
    290                 295                 300

Glu Ile Gly Thr Tyr Asp Ala Gly Met Asp Ile Trp Gly Gly Glu Asn
305                 310                 315                 320

Leu Glu Ile Ser Phe Arg Ile Trp Gln Cys Gly Gly Thr Leu Glu Ile

325                 330                 335

Val Thr Cys Ser His Val Gly His Val Phe Arg Lys Ala Thr Pro Tyr
            340                 345                 350

Thr Phe Pro Gly Gly Thr Gly Gln Ile Ile Asn Lys Asn Asn Arg Arg
        355                 360                 365

Leu Ala Glu Val Trp Met Asp Glu Phe Lys Asn Phe Phe Tyr Ile Ile
    370                 375                 380

Ser Pro Gly Val Thr Lys Val Asp Tyr Gly Asp Ile Ser Ser Arg Val
385                 390                 395                 400

Gly Leu Arg His Lys Leu Gln Cys Lys Pro Phe Ser Trp Tyr Leu Glu
                405                 410                 415

Asn Ile Tyr Pro Asp Ser Gln Ile Pro Arg His Tyr Phe Ser Leu Gly
            420                 425                 430

Glu Ile Arg Asn Val Glu Thr Asn Gln Cys Leu Asp Asn Met Ala Arg
        435                 440                 445

Lys Glu Asn Glu Lys Val Gly Ile Phe Asn Cys His Gly Met Gly Gly
    450                 455                 460

Asn Gln Val Phe Ser Tyr Thr Ala Asn Lys Glu Ile Arg Thr Asp Asp
465                 470                 475                 480

Leu Cys Leu Asp Val Ser Lys Leu Asn Gly Pro Val Thr Met Leu Lys
                485                 490                 495

Cys His His Leu Lys Gly Asn Gln Leu Trp Glu Tyr Asp Pro Val Lys
            500                 505                 510

Leu Thr Leu Gln His Val Asn Ser Asn Gln Cys Leu Asp Lys Ala Thr
        515                 520                 525

Glu Glu Asp Ser Gln Val Pro Ser Ile Arg Asp Cys Thr Gly Ser Arg
    530                 535                 540

Ser Gln Gln Trp Leu Leu Arg Asn Val Thr Leu Pro Glu Ile Phe
545                 550                 555


<210> 77
<211> 1838
<212> DNA
<213> Rattus norvegicus

<220>
<223> Polypeptide GalNAc transferase

<400> 77
   atatgactgt gatttctgat gaaaccagat tggaataatt ttcatsatat ttgtatattt 60
   atatatatat attttaaaat tttacatttg acttaaagtg ccatgagaaa atttgcatat 120
   tgcaaggtgg ttctagccac ctccttggtt tgggtactct tggatatgtt cctgctgctt 180
   tacttcagtg aatgcaacaa atgtgaagag aagaaggagc gagggcttcc tgctggggat 240
   gttctagagc tggtgcagaa gcctcacgaa ggtcctggag aaatgggaaa accagtcgtc 300
   attcctaaag aggatcagga aaagatgaaa gagatgttca aaatcaacca gttcaaccta 360
   atggcgagtg agatgattgc attcaacaga tccttaccag acgtgaggct agaggggtgt 420
   aaaacaaaag tgtacccaga tagccttcct acgacaagcg tggtgattgt ctttcacaat 480
   gaggcgtgga gcaccctcct gcgaacagtc cacagtgtca ttaaccgctc accaagacac 540
   atgatagaag agattgttct tgtagacgat gccagcgaaa gagacttttt gaaaagacct 600
   ctagagagtt atgtgaaaaa gcttaaagtg ccagttcatg taattcgaat ggaacaacgt 660
   tctgggttga tcagagctag attaaaagga gctgctgtgt ccaaaggcca agtgatcacc 720
   ttcttagatg ctcactgcga gtgcacagta gggtggctgg agcctctgtt agcccggatc 780
   aaacatgaca ggaggacagt ggtgtgtcct atcatcgatg tgatcagtga tgacactttt 840
   gagtacatgg cgggctctga tatgacctat ggtggattca actggaagct caatttccgt 900
   tggtatcctg ttccccaacg agaaatggac agaaggaaag gtgaccggac tcttcctgtc 960
   agaacaccta caatggctgg aggcctgttt tcaatagaca gagattactt tcaggaaatt 1020
   ggaacatatg atgctggaat ggatatttgg ggaggagaaa acctagaaat ttcctttagg 1080
   atttggcagt gtggagggac tctggaaata gtgacgtgct cccacgttgg ccatgtgttt 1140
   cggaaagcta cgccgtacac gttcccgggt ggcacggggc agatcatcaa caagaataac 1200
   agacggctcg cggaggtgtg gatggatgaa ttcaagaatt tcttctacat aatttctcca 1260
   ggtgttacaa aggtagatta tggtgatata tcatcaagag tcggtctgag acataaacta 1320
   cagtgcaagc ctttctcttg gtacctagaa aatatttatc ccgattctca aattcctcgt 1380
   cactatttct cattgggaga gatacgaaat gtggaaacaa atcaatgtct agataacatg 1440
   gctagaaaag agaatgaaaa ggttggaatt tttaattgtc atggtatggg aggcaatcag 1500
   gttttctctt acactgccaa caaagaaatt agaacagatg acctttgttt ggacgtttcc 1560
   aaacttaatg gcccagttac catgctcaaa tgccaccacc taaaaggcaa ccagctctgg 1620
   gagtatgacc cagtgaaatt aaccctgcag catgtgaaca gtaaccagtg cctggataaa 1680
   gccacagaag aggacagcca ggtgcccagc atcagagact gcactggaag ccggtcccag 1740
   cagtggcttc ttcgaaatgt caccctccca gaaatattct gagaccaaat tacaacaaag 1800
   aaaggattga ctgggctacc tcagcataag ccgaattc 1838

<210> 78
<211> 354
<212> PRT
<213> Rattus norvegicus

<220>
<223> Arginase II

<400> 78
Met Phe Leu Arg Ser Ser Val Ser Arg Leu Leu His Gly Gln Ile Pro
1               5                   10                  15

Cys Ala Leu Thr Arg Ser Val His Ser Val Ala Val Val Gly Ala Pro
            20                  25                  30

Phe Ser Arg Gly Gln Lys Lys Lys Gly Val Glu Tyr Gly Pro Ala Ala
        35                  40                  45

Ile Arg Glu Ala Gly Leu Leu Lys Arg Leu Ser Met Leu Gly Cys His
    50                  55                  60

Ile Lys Asp Phe Gly Asp Leu Ser Phe Thr Asn Val Pro Lys Asp Asp
65                  70                  75                  80

Pro Tyr Asn Asn Leu Val Val Tyr Pro Arg Ser Val Gly Ile Ala Asn
                85                  90                  95

Gln Glu Leu Ala Glu Val Val Ser Arg Ala Val Ser Gly Gly Tyr Ser
            100                 105                 110

Cys Val Thr Leu Gly Gly Asp His Ser Leu Ala Ile Gly Thr Ile Ser
        115                 120                 125

Gly His Ala Arg His His Pro Asp Leu Cys Val Ile Trp Val Asp Ala
    130                 135                 140

His Ala Asp Ile Asn Thr Pro Leu Thr Thr Val Ser Gly Asn Ile His
145                 150                 155                 160

Gly Gln Pro Leu Ser Phe Leu Ile Arg Glu Leu Gln Asp Lys Val Pro
                165                 170                 175

Gln Leu Pro Gly Phe Ser Trp Ile Lys Pro Cys Leu Ser Pro Pro Asn
            180                 185                 190

Leu Val Tyr Ile Gly Leu Arg Asp Val Glu Pro Ala Glu His Phe Ile
        195                 200                 205

Leu Lys Ser Phe Asp Ile Gln Tyr Phe Ser Met Arg Asp Ile Asp Arg
    210                 215                 220

Leu Gly Ile Gln Lys Val Met Glu Gln Thr Phe Asp Arg Leu Ile Gly
225                 230                 235                 240

Lys Arg Lys Arg Pro Ile His Leu Ser Phe Asp Ile Asp Ala Phe Asp
                245                 250                 255

Pro Lys Leu Ala Pro Ala Thr Gly Thr Pro Val Val Gly Gly Leu Thr
            260                 265                 270

Tyr Arg Glu Gly Leu Tyr Ile Thr Glu Glu Ile His Ser Thr Gly Leu
        275                 280                 285

Leu Ser Ala Leu Asp Leu Val Glu Val Asn Pro His Leu Ala Thr Ser
    290                 295                 300

Glu Glu Glu Ala Lys Ala Thr Ala Ser Leu Ala Val Asp Val Ile Ala
305                 310                 315                 320

Ser Ser Phe Gly Gln Thr Arg Glu Gly Gly His Ile Ala Tyr Asp His
                325                 330                 335

Leu Pro Thr Pro Ser Ser Pro His Glu Ser Glu Lys Glu Glu Cys Val
            340                 345                 350

Arg Ile

<210> 79
<211> 1463
<212> DNA
<213> Rattus norvegicus

<220>
<223> Arginase II

<400> 79
cgcgcttgcc tagtgaagct gcgaacgtgt gggaggctgc actcactcga ggtcctgagt 60
tgcgcggagc tgcttctgct agggcgatcg cctccctgca atcatgttcc tgaggagcag 120
cgtctcccgt ctcctccacg ggcaaattcc ttgtgccctg acgagatccg tccactctgt 180
agctgtagtc ggagcccctt tctctcgggg acagaaaaag aaaggagtgg aatatggccc 240
agccgccatc cgagaagctg gcttgctgaa gaggctttct atgttgggat gccatataaa 300
agactttgga gacttgagtt ttactaacgt tcccaaagat gatccctaca ataatctggt 360
cgtgtatcct cgctcagtgg gcattgccaa ccaggaactg gctgaggtgg ttagtagagc 420
tgtgtcaggt ggctacagct gtgtcacact gggaggagac cacagcctgg caatcggtac 480

cattagtggc catgcccgac accacccaga tctctgtgtc atctgggttg atgctcatgc 540
tgacattaat acacccctca ccactgtatc aggaaatatc catgggcagc ctctttcctt 600
tctcatcaga gaactacaag acaaggtacc acaactgcca ggattttcct ggatcaaacc 660
ttgcctctct cccccaaatc ttgtatacat tggcttacga gatgtggagc ctgccgaaca 720
ctttatttta aagagttttg acatccagta tttctccatg agagatattg atcgacttgg 780
tatccagaag gtcatggaac agacatttga tcggctgatt ggcaagagga agaggccgat 840
ccacctgagc tttgacatag atgcatttga ccctaagctg gctccagcca caggaacccc 900
tgtggtaggg gggctgacct acagagaagg actgtacatt actgaagaaa tacatagcac 960
agggttgctg tcggctctgg atcttgttga agtcaatcct catttggcca cttctgagga 1020
agaggccaag gctacagcca gcctagcagt ggatgtgatt gcttcaagtt ttggtcagac 1080
aagagaaggc ggacacattg cctatgacca ccttcctact cctagttcac cacatgaatc 1140
agaaaaggaa gaatgtgtga gaatttagga aacactgtac tctggcacct ttcacgacag 1200
cattccagag ttgggaggca tttaaaggga caaagagtaa atggctgtct ggatccaata 1260
ttgccttaat gagaacatct gtgcactctc acaagtgtag agtccccttc tctattttgg 1320
tcacaatact atcactgtaa atgtatctgt tgggtttttg tttctgaagt ttacaagcta 1380
ttgttattat acacgtgtgt ttgaaggagt cataaacagc atttattacc ttaaaaaaaa 1440
aaaaaaaaaa aaaaaaaaaa aaa 1463

<210> 80
<211> 270
<212> PRT
<213> Rattus norvegicus

<220>
<223> ATP synthase gamma subunit

<400> 80
Arg Asp Ile Thr Arg Arg Leu Lys Ser Ile Lys Asn Ile Gln Lys Ile
1               5                   10                  15

Thr Lys Ser Met Lys Met Val Ala Ala Ala Lys Tyr Ala Arg Ala Glu
            20                  25                  30

Arg Glu Leu Lys Pro Ala Arg Val Tyr Gly Thr Gly Ser Leu Ala Leu
        35                  40                  45

Tyr Glu Lys Ala Glu Ile Lys Gly Pro Glu Asp Lys Lys Lys His Leu
    50                  55                  60

Ile Ile Gly Val Ser Ser Asp Arg Gly Leu Cys Gly Ala Ile His Ser
65                  70                  75                  80

Ser Val Ala Lys Gln Met Lys Asn Asp Met Ala Ala Leu Thr Ala Ala
                85                  90                  95

Gly Lys Glu Val Met Ile Val Gly Ile Gly Glu Lys Ile Lys Ser Ile
            100                 105                 110

Leu Tyr Arg Thr His Ser Asp Gln Phe Leu Val Ser Phe Lys Asp Val
        115                 120                 125

Gly Arg Lys Pro Pro Thr Phe Gly Asp Ala Ser Val Ile Ala Leu Glu
    130                 135                 140

Leu Leu Asn Ser Gly Tyr Glu Phe Asp Glu Gly Ser Ile Ile Phe Asn
145                 150                 155                 160

Gln Phe Lys Ser Val Ile Ser Tyr Lys Thr Glu Glu Lys Pro Ile Phe
                165                 170                 175

Ser Phe Ser Thr Val Val Ala Ala Glu Asn Met Ser Ile Tyr Asp Asp
            180                 185                 190

Ile Asp Ala Asp Val Leu Gln Asn Tyr Gln Glu Tyr Asn Leu Ala Asn
        195                 200                 205

Ile Ile Tyr Tyr Ser Leu Lys Glu Ser Thr Thr Ser Glu Gln Ser Ala
    210                 215                 220

Arg Met Thr Ala Met Asp Asn Ala Ser Lys Asn Ala Ser Asp Met Ile
225                 230                 235                 240

Asp Lys Leu Thr Leu Thr Phe Asn Arg Thr Arg Gln Ala Val Ile Thr
                245                 250                 255

Lys Glu Leu Ile Glu Ile Ile Ser Gly Ala Ala Ala Leu Asp
            260                 265                 270


<210> 81
<211> 975
<212> DNA
<213> Rattus norvegicus

<220>
<223> ATP synthase gamma-subunit

<400> 81
cgagacatta ccaggagact gaagtccatc aaaaacatcc agaagattac caagtctatg 60
aagatggtgg cagcagcgaa gtacgcccgg gctgagcggg agctgaagcc agctcgagtg 120
tatgggacag gctccttggc tctgtatgag aaggctgaga ttaagggacc tgaggacaag 180
aagaagcacc tcattattgg tgtgtcctcg gatcgaggcc tttgtggtgc tattcattcc 240
tccgtggcta aacaaatgaa gaatgacatg gctgccctca cagcagctgg gaaagaagtt 300
atgattgttg gaattggtga aaaaatcaag agcatacttt ataggactca ctctgatcag 360
tttttggtgt ccttcaaaga cgtggggcgg aagcccccta cttttggaga tgcatcggtc 420
attgccttgg agttgttaaa ttcgggatat gaatttgatg aaggctctat tatctttaat 480
cagttcaagt ctgttatctc ctacaagaca gaagagaagc ccatcttctc tttcagtacc 540
gtcgtggctg ctgagaacat gagcatttat gatgacattg atgctgacgt gctgcagaat 600
taccaggaat acaatctggc caacatcatc tactactccc tgaaggagtc caccaccagt 660
gagcagagtg ccaggatgac cgccatggac aacgccagca agaacgcctc tgatatgatt 720
gataaattga cgctgacttt caaccgcacc cgccaggccg tcatcacgaa ggagctgata 780
gagatcatct ccggggctgc tgctctggat taatgagaat caagttacat cctcagacaa 840
gaggtgaaga aggaaaaggc agctgatttt aactgattgc tgtccttgtt agaagaaact 900
tggtccactg agtcacaaag acagcaggat gtttgtaaat tatcttacaa taaacaactt 960
acaatgagaa aaacg                                                   975


<210> 82
<211> 285
<212> PRT
<213> Rattus norvegicus

<220>
<223> N-G, N-G dimethylarginine dimethylaminohydrolase

<400> 82
Met Ala Gly Leu Ser His Pro Ser Val Phe Gly Arg Ala Thr His Ala
1               5                   10                  15

Val Val Arg Ala Pro Pro Glu Ser Leu Cys Arg His Ala Leu Arg Arg
            20                  25                  30

Ser Gln Gly Glu Glu Val Asp Phe Ala Arg Ala Glu Arg Gln His Gln
        35                  40                  45

Leu Tyr Val Gly Val Leu Gly Ser Lys Leu Gly Leu Gln Val Val Gln
    50                  55                  60

Leu Pro Ala Asp Glu Ser Leu Pro Asp Cys Val Phe Val Glu Asp Val
65                  70                  75                  80

Ala Val Val Cys Glu Glu Thr Ala Leu Ile Thr Arg Pro Gly Ala Pro
                85                  90                  95

Ser Arg Arg Lys Glu Val Asp Met Met Lys Glu Ala Leu Glu Lys Leu
            100                 105                 110

Gln Leu Asn Ile Val Glu Met Lys Asp Glu Asn Ala Thr Leu Asp Gly
        115                 120                 125

Gly Asp Val Leu Phe Thr Gly Arg Glu Phe Phe Val Gly Leu Ser Lys
    130                 135                 140

Arg Thr Asn Gln Arg Gly Ala Glu Ile Leu Ala Asp Thr Phe Lys Asp
145                 150                 155                 160

Tyr Ala Val Ser Thr Val Pro Val Ala Asp Ser Leu His Leu Lys Ser
                165                 170                 175

Phe Cys Ser Met Ala Gly Pro Asn Leu Ile Ala Ile Gly Ser Ser Glu
            180                 185                 190

Ser Ala Gln Lys Ala Leu Lys Ile Met Gln Gln Met Ser Asp His Arg
        195                 200                 205

Tyr Asp Lys Leu Thr Val Pro Asp Asp Met Ala Ala Asn Cys Ile Tyr
    210                 215                 220

Leu Asn Ile Pro Ser Lys Gly His Val Leu Leu His Arg Thr Pro Glu
225                 230                 235                 240

Glu Tyr Pro Glu Ser Ala Lys Val Tyr Glu Lys Leu Lys Asp His Leu
                245                 250                 255

Leu Ile Pro Val Ser Asn Ser Glu Met Glu Lys Val Asp Gly Leu Leu
            260                 265                 270

Thr Cys Cys Ser Val Phe Ile Asn Lys Lys Thr Asp Ser
        275                 280                 285

<210> 83
<211> 3008
<212> DNA
<213> Rattus norvegicus

<220>
<223> N-G, N-G dimethylarginine dimethylaminohydrolase

<220>
<221> misc_feature
<222> 2411, 2412

<223> n is a or g or c or t

<400> 83
ccacatgatt tattacaatc atctcctcaa tagccacact atttatttta tttcaattaa 60
aaatttcttc ccaaaccttt cctgcacctc cctcacccaa aactatagcc acagaaaaaa 120
cgaataaccc ttgagaatca aaatgaacga aaatctattt gcctctttca ttacccccac 180
aataataggt ctaccaattg ttgtgaccat tattatgttc ccatcaattc tattcccatc 240
atcagaacgc ctaatcagca accgactaca ctcatttcaa cactgactaa tcaaacttat 300
catcaaacaa ataatgttaa tcggagccgc cgggcgctcg caggctcgca ggctcgcagg 360
ttgggcgcgc gctgctctcg ggaagacccg gccaccgagc caaaccccgc cgccgcctct 420
ccgcgcgagc catggccggc ctcagccacc cctcggtctt cggccgggcc acccacgccg 480
tggtgcgggc tccgcccgag tccctgtgcc gccacgcgct gaggcgctcc cagggcgaag 540
aggtggattt cgctcgcgct gagcgccagc accagctcta cgtgggcgtg ctgggcagca 600
agctggggct gcaggtggtg cagctgccgg ccgacgagag cctgcctgac tgcgtgttcg 660
tggaggacgt ggccgtggtg tgcgaggaga ccgccctcat cacccgcccc ggggcgccta 720
gccgcaggaa ggaggttgac atgatgaaag aggctttgga aaaacttcag ctcaacatag 780
tagagatgaa agatgaaaat gcaaccttag atggtgggga cgtcctattc acaggcagag 840
agttttttgt gggcctttcc aaaaggacaa atcaacgagg tgctgagatc ttggctgata 900
ctttcaagga ctacgcagtt tccacagtcc ccgtggccga ttctttgcat ttaaagagtt 960
tctgcagcat ggctggcccc aacctgatcg caatagggtc cagtgaatct gcgcagaagg 1020
ccctcaagat catgcaacag atgagtgacc accgttatga caagctcact gtaccggacg 1080
acatggccgc caactgtata tatttaaata tccccagcaa agggcatgtc ttgctgcacc 1140
gaaccccaga agagtaccca gaaagcgcaa aggtttatga gaagctcaag gaccatctac 1200
tgatccctgt gagcaattct gagatggaaa aggtggacgg cttgctcacc tgctgctccg 1260
tttttattaa caagaagaca gactcttgag cggaagagtc cctcccgagc ggccggcgag 1320
tggcctaggc caggctgatg atggctctgt acccattcct cctgtttcct ttgacaatct 1380
actgtgccac taactcttgt ttacaaagaa ttccaatctt aatcgcctca tttccccaca 1440
cccccctctt cctcacgggg ccccctaacc tgtgcacaca tttgctcaat gaactaagca 1500
acctaggaaa tacagaacta acgaatcatt ggaatgtgat tgatagcgtg gaggcactca 1560
ctccagcctc tgtgtcctta gtgtgggaat agtttagatg cagggcatcc tgaaggactg 1620
acctcctggg cagacagata aacaagcaag ggactttctt tgcttcatga gttgcgtttg 1680
gttcctgtga gagtcctgag aacgtggatt ttttcccatt cccctctctt ctcacctcta 1740
aaacactttc ttccaggtct tcctttggcc ttcctcactt ctttcgtaat caccttgaac 1800
ctggatttat ccaaggtctc gaactctgga taccctccta acgtccaccc tgtctacgtg 1860
cagtcctctc ctcctcacag ccacaacttc tgccaccacc agcaaatcag agtggctggt 1920
tggagacgag gaacctcaca gaggcacatc ctgacagggt ttcccattag cacacctagg 1980
aaggtaaatt caggtggttg atacaggatg ttagatacag aagcctagaa acacccccat 2040
gggtacatct cagagagact gagtcactgc catgattttg cctcatgact ggtcctatag 2100
gagcgtggag gaaagtcttt ctccacccca ccacctcccc ctttttcagc ttccttctga 2160
ctcaaccttg tatctacccc cttagaatca ccatgatttt gaaaagtcat ttccttcaaa 2220
agaggcagaa catctaatga ttgttgacgg tctggtaaga gctagaggga actgcctcca 2280
cccaaggcct aggagcacaa tcaggaaatg gttcctaaag tcaacttcaa acaatgtgtg 2340
agtctccttt gaaatatcca tgactgttta ctgtggaatt caaccgaaac ttcaaattac 2400
acaactttca nnagacgcag gtctctcaca gggaacaata ccacagcaaa ggaattcctt 2460
tcttgaaagc tgaaacaggc caaggacatg gtaccttaca aggcacggtg acccagccag 2520
gcatctcatc aaacttctgg tcacttctac cttggctgta tagcctgcag tctgtcattc 2580
gatatttaaa gttggggaga caaaggctat gtcagctcta gctctcctta cctctctttg 2640
tgatcagtgt ttcttctagt gaaggcaaga gagactgggc ctcatctcgt ccccctcagt 2700
ggccggaagt gagaaaatca agacttcctt taccgccctt ctcttagttg gcaacctctc 2760
tgaagaggtc tccatctctc ccttgctgtc taactctaga ctcatatcgc atgccaatat 2820
ctaactgtac accctgtgaa tgaaccatat cctggtcact gtcacatttc acagcgtatc 2880
acaggtgaac agttatgcct tctccagggc aagggaatat gagacagttg cctaaattca 2940
taaagaaaaa ttgttctcag ccattatggt tgccttgagg ggttaaagtt gaccccacct 3000
acactagc 3008

<210> 84
<211> 515
<212> PRT
<213> Mus musculus

<220>
<223> Glucocerebrosidase

<400> 84
Met Ala Ala Arg Leu Ile Gly Phe Phe Leu Phe Gln Ala Val Ser Trp
1               5                   10                  15

Ala Tyr Gly Ala Gln Pro Cys Ile Pro Lys Ser Phe Gly Tyr Ser Ser
            20                  25                  30

Val Val Cys Val Cys Asn Ala Ser Tyr Cys Asp Ser Leu Asp Pro Val
        35                  40                  45

Thr Leu Pro Ala Leu Gly Thr Phe Ser Arg Tyr Glu Ser Thr Arg Arg
    50                  55                  60

Gly Arg Arg Met Glu Leu Ser Val Gly Ala Ile Gln Ala Asn Arg Thr
65                  70                  75                  80

Gly Thr Gly Leu Leu Leu Thr Leu Gln Pro Glu Lys Lys Phe Gln Lys
                85                  90                  95

Val Lys Gly Phe Gly Gly Ala Met Thr Asp Ala Thr Ala Leu Asn Ile
            100                 105                 110

Leu Ala Leu Ser Pro Pro Thr Gln Lys Leu Leu Leu Arg Ser Tyr Phe
        115                 120                 125

Ser Thr Asn Gly Ile Glu Tyr Asn Ile Ile Arg Val Pro Met Ala Ser
    130                 135                 140

Cys Asp Phe Ser Ile Arg Val Tyr Thr Tyr Ala Asp Thr Pro Asn Asp
145                 150                 155                 160

Phe Gln Leu Ser Asn Phe Ser Leu Pro Glu Glu Asp Thr Lys Leu Lys
                165                 170                 175

Ile Pro Leu Ile His Gln Ala Leu Lys Met Ser Ser Arg Pro Ile Ser
            180                 185                 190

Leu Phe Ala Ser Pro Trp Thr Ser Pro Thr Trp Leu Lys Thr Asn Gly
        195                 200                 205

Arg Val Asn Gly Lys Gly Ser Leu Lys Gly Gln Pro Gly Asp Ile Phe
    210                 215                 220

His Gln Thr Trp Ala Asn Tyr Phe Val Lys Phe Leu Asp Ala Tyr Ala
225                 230                 235                 240

Lys Tyr Gly Leu Arg Phe Trp Ala Val Thr Ala Glu Asn Glu Pro Thr
                245                 250                 255

Ala Gly Leu Phe Thr Gly Tyr Pro Phe Gln Cys Leu Gly Phe Thr Pro
            260                 265                 270

Glu His Gln Arg Asp Phe Ile Ser Arg Asp Leu Gly Pro Ala Leu Ala
        275                 280                 285

Asn Ser Ser His Asp Val Lys Leu Leu Met Leu Asp Asp Gln Arg Leu
    290                 295                 300

Leu Leu Pro Arg Trp Ala Glu Val Val Leu Ser Asp Pro Glu Ala Ala
305                 310                 315                 320

Lys Tyr Val His Gly Ile Ala Val His Trp Tyr Met Asp Phe Leu Ala
                325                 330                 335

Pro Ala Lys Ala Thr Leu Gly Glu Thr His Arg Leu Phe Pro Asn Thr
            340                 345                 350

Met Leu Phe Ala Ser Glu Ala Cys Val Gly Ser Lys Phe Trp Glu Gln
        355                 360                 365

Ser Val Arg Leu Gly Ser Trp Asp Arg Gly Met Gln Tyr Ser His Ser
    370                 375                 380

Ile Ile Thr Asn Leu Leu Tyr His Val Thr Gly Trp Thr Asp Trp Asn
385                 390                 395                 400

Leu Ala Leu Asn Pro Glu Gly Gly Pro Asn Trp Val Arg Asn Phe Val
                405                 410                 415

Asp Ser Pro Ile Ile Val Asp Ile Pro Lys Asp Ala Phe Tyr Lys Gln
            420                 425                 430

Pro Met Phe Tyr His Leu Gly His Phe Ser Lys Phe Ile Pro Glu Gly
        435                 440                 445

Ser Gln Arg Val Ala Leu Val Ala Ser Glu Ser Thr Asp Leu Glu Thr
    450                 455                 460

Val Ala Leu Leu Arg Pro Asp Gly Ser Ala Val Val Val Val Leu Asn
465                 470                 475                 480

Arg Ser Ser Glu Asp Val Pro Leu Thr Ile Ser Asp Pro Asp Leu Gly
                485                 490                 495

Phe Leu Glu Thr Val Ser Pro Gly Tyr Ser Ile His Thr Tyr Leu Trp
            500                 505                 510

Arg Arg Gln
        515

<210> 85
<211> 1684
<212> DNA
<213> Mus musculus

<220>
<223> Glucocerebrosidase

<400> 85
ccacagaaac ttccacagtc ttctcattcg ctggtcctga gtagctccag tttccaagat 60
cttctggaga gaagtgtgct ggtgtcatgg ctgccaggct catcggattc ttcctatttc 120
aggcggtatc ttgggcatat ggtgcccaac cctgcatccc caaaagcttt ggctacagct 180
cagtggtctg tgtctgtaat gcatcgtact gtgactctct tgaccccgtg accttaccgg 240
ctctgggtac cttcagccgt tacgagagca ctcgacgtgg acgtcggatg gagctgagtg 300
tcggggccat ccaggccaat cgcactggca cagggttact actcactttg cagccagaaa 360
agaagttcca gaaagtgaaa ggatttggag gcgccatgac agacgccact gcgctcaaca 420
tccttgcttt gtccccacct actcagaagc tgctactcag atcctacttc tctaccaacg 480
gaattgaata taacatcatc cgggtaccca tggccagttg tgacttctcc atccgtgtct 540

atacctatgc tgacacccct aacgacttcc agttatccaa cttcagcctc ccagaagaag 600
acaccaagct caagataccc ctgattcacc aagccctgaa gatgtcctca cgccccattt 660
cactctttgc cagtccctgg acatcaccca cttggctcaa gaccaatgga agagtgaatg 720
ggaaggggtc gctcaagggt cagccagggg atatctttca ccagacctgg gccaattact 780
ttgtcaagtt tctggatgct tatgctaagt atggcctaag attctgggca gtgacagcgg 840
agaatgaacc tacagcaggg ctcttcacgg ggtacccctt ccagtgcctg ggcttcactc 900
ctgaacacca gagagacttc atttcccgtg acctagggcc agcccttgcc aacagttccc 960
atgatgtgaa gctactcatg ctagatgacc aacgcttgct gctaccccgc tgggcagagg 1020
tggtgctctc tgatccagag gcagctaagt acgttcatgg cattgctgtt cactggtata 1080
tggatttcct ggctccagcc aaagctactt taggagagac acaccgcttg ttccccaaca 1140
caatgctctt tgcttcggag gcctgcgtgg gctccaagtt ctgggaacag agtgttcggt 1200
tgggctcctg ggatcgaggg atgcagtaca gtcacagcat cattacgaac ctcctttacc 1260
acgtaactgg atggacggac tggaaccttg ccctgaatcc tgaaggaggg cccaactggg 1320
tccgcaactt tgtcgatagc cccattattg tcgacatccc caaagacgca ttttacaaac 1380
agcccatgtt ctaccatctt ggccacttca gcaagttcat tcccgaggga tcccagagag 1440
tggcgttggt ggccagtgag agcactgact tggaaacagt agcactgtta cgccctgacg 1500
gctctgcagt tgtggtcgtg ttaaaccgat cttcggagga tgtccctctt accatcagtg 1560
atcctgacct gggcttcctg gagaccgtgt cacctggcta ctccattcac acttacctgt 1620
ggcgtcgcca gtgatggaac tgacacctga gcaggcacct gggcttagca tggacattaa 1680
aggg 1684

<210> 86
<211> 552
<212> PRT
<213> Homo sapiens

<220>
<223> N-acetylglucosamine-6-sulphatase

<400> 86

<210> 87
<211> 2379
<212> DNA
<213> Homo sapiens

<220>
<223> N-acetylglucosamine-6-sulphatase

<400> 87
   ggaattccgg tcggcctctc gcccttcagc tacctgtgcg tccctccgtc ccgtcccgtc 60
   ccggggtcac cccggagcct gtccgctatg cggctcctgc ctctagcccc aggtcggctc 120
   cggcggggca gcccccgcca cctgccctcc tgcagcccag cgctgctact gctggtgctg 180
   ggcggctgcc tgggggtctt cggggtggct gcgggaaccc ggaggcccaa cgtggtgctg 240
   ctcctcacgg acgaccagga cgaagtgctc ggcggcatga caccactaaa gaaaaccaaa 300
   gctctcatcg gagagatggg gatgactttt tccagtgctt atgtgccaag tgctctctgc 360
   tgccccagca gagccagtat cctgacagga aagtacccac ataatcatca cgttgtgaac 420
   aacactctgg aggggaactg cagtagtaag tcctggcaga agatccaaga accaaatact 480
   ttcccagcaa ttctcagatc aatgtgtggt tatcagacct tttttgcagg gaaatattta 540
   aatgagtacg gagccccaga tgcaggtgga ctagaacacg ttcctctggg ttggagttac 600
   tggtatgcct tggaaaagaa ttctaagtat tataattaca ccctgtctat caatgggaag 660
   gcacggaagc atggtgaaaa ctatagtgtg gactacctga cagatgtttt ggctaatgtc 720
   tccttggact ttctggacta caagtccaac tttgagccct tcttcatgat gatcgccact 780
   ccagcgcctc attcgccttg gacagctgca cctcagtacc agaaggcttt ccagaatgtc 840
   tttgcaccaa gaaacaagaa cttcaacatc catggaacga acaagcactg gttaattagg 900
   caagccaaga ctccaatgac taattcttca atacagtttt tagataatgc atttaggaaa 960
   aggtggcaaa ctctcctctc agttgatgac cttgtggaga aactggtcaa gaggctggag 1020
   ttcactgggg agctcaacaa cacttacatc ttctatacct cagacaatgg ctatcacaca 1080
   ggacagtttt ccttgccaat agacaagaga cagctgtatg agtttgatat caaagttcca 1140
   ctgttggttc gaggacctgg gatcaaacca aatcagacaa gcaagatgct ggttgccaac 1200
   attgacttgg gtcctactat tttggacatt gctggctacg acctaaataa gacacagatg 1260
   gatgggatgt ccttattgcc cattttgaga ggtgccagta acttgacctg gcgatcagat 1320
   gtcctggtgg aataccaagg agaaggccgt aacgtcactg acccaacatg cccttccctg 1380
   agtcctggcg tatctcaatg cttcccagac tgtgtatgtg aagatgctta taacaatacc 1440
   tatgcctgtg tgaggacaat gtcagcattg tggaatttgc agtattgcga gtttgatgac 1500
   caggaggtgt ttgtagaagt ctataatctg actgcagacc cagaccagat cactaacatt 1560
   gctaaaacca tagacccaga gcttttagga aagatgaact atcggttaat gatgttacag 1620
   tcctgttctg ggccaacctg tcgcactcca ggggtttttg accccggata caggtttgac 1680
   ccccgtctca tgttcagcaa tcgcggcagt gtcaggactc gaagattttc caaacatctt 1740
   ctgtagcgac ctcacacagc ctctgcagat ggatccctgc acgcctcttt ctgatgaagt 1800
   gattgtagta ggtgtctgta gctagtcttc aagaccacac ctggaagagt ttctgggctg 1860
   gctttaagtc ctgtttgaaa aagcaaccca gtcagctgac ttcctcgtgc aatgtgttaa 1920
   actgtgaact ctgcccatgt gtcaggagtg gctgtctctg gtctcttcct ttagctgaca 1980
   aggacactcc tgaggtcttt gttctcactg tatttttttt atcctggggc cacagttctt 2040
   gattattcct cttgtggtta aagactgaat ttgtaaaccc attcagataa atggcagtac 2100
   tttaggacac acacaaacac acagatacac cttttgatat gtaagcttga cctaaagtca 2160
   aaggacctgt gtagcatttc agattgagca cttcactatc aaaaatacta acatcacatg 2220
   gcttgaagag taaccatcag agctgaatca tccaagtaag aacaagtacc attgttgatt 2280
   gataagtaga gatacatttt ttatgatgtt catcacagtg tggtaaggtt gcaaattcaa 2340
   aacatgtcac ccaagctctg ttcatgtttt tgtgaattc 2379

<210> 88
<211> 3225
<212> DNA
<213> Rattus norvegicus

<220>
<223> 12S rRNA

<400> 88
gtcaacaaac ccccccaccc cctacacctg aaacttcaat gccaaacccc aaaaacatta 60
aagcaagaat taaataaaac aaaaagctaa cttaattctt aaaaggcttc tccgttctag 120
tagaccacaa aattttaact taaatcttag cattggtaaa atttcccgac acaaaatctt 180
tcctccctaa ctaaaccctc tttacttgct ctaaccctca gaaaattcca catacaccaa 240
agttaatgta gcttataata aagcaaagca ctgaaaatgc ttagatggat tcaaaaatcc 300
cataaacaca aaggtttggt cctggcctta taattaattg gaggtaagat tacacatgca 360
aacatccata aaccggtgta aaatccctta aagatttgcc taaaacttaa ggagagggca 420
tcaagcacat aatatagctc aagacgcctt gcctagccac acccccacgg gactcagcag 480
tgataaatat taagcaatga acgaaagttt gactaagcta gtacctctca gggttggtaa 540
atttcgtgcc agccaccgcg gtcatacgat taacccaaac taattatttt cggcgtaaaa 600
cgtgccaact ataaatctca taatagaatt aaaatccaac ttatatgtga aaattcattg 660
ttaggaccta agcccaataa cgaaagtaat tctaatcatt tatataatgc acgatagcta 720
agacccaaac tgggattaga taccccacta tgcttagccc taaaccttaa taattaaacc 780
tacaaaatta tttgccagag aactactagc tacagcttaa aacacaaagg acttggcggt 840
actttatatc cgtctagagg agcctgttct ataatcgata aaccccgttc taccttaccc 900
cttctcgcta attcagccta tataccgcca tcttcagcaa accctaaaaa ggcactaagt 960
tagcacaaga caacataaaa cgttaggtca aggtgtagcc aatgaagcgg aaagaaatgg 1020
gctacatttt cttttcccag agaacattac gaaaccctta tgaaactaaa ggacaaagga 1080
ggatttagta gtaaattaag aatagagagc ttaattgaat agagcaatga agtacgcaca 1140
caccgcccgt caccctcctc aaattagatt gacattcaca tatacataat ttcactaaca 1200
aatttatgag aggagataag tcgtaacaag gtaagcatac tggaaagtgt gcttggaata 1260
atcacagtgt agcttaatca caaagcatct ggcctacacg cagaagaatt cataaaaaat 1320
gaacactttg aactaaccct agccctacaa ccaaccaaca taactaaacc cccacataaa 1380
ctaaaacatt taactcaaaa agtattggag aaagaaattt acttaccaag agctatagag 1440
aaagtaccgc aagggaaatg atgaaagact aatttaaagt aaaaacaaga caaagattaa 1500
acctgtacct tttgcataat gaattaacta gaaaatcctt aacaaaaaga atttaagcta 1560
agaaccccga aaccaaacga gctacctaaa aacaatttca tgaatcaacc cgtctatgta 1620
gcaaaatagt gggaagattt ttaggtagag gtgaaaagcc tatcgagctt ggtgatagct 1680
ggttgcccaa aaaagaattt cagttcaaac tttaagcttc catcagaaca acaaatcaaa 1740
atgtaaactt aaaatatagc caaaagaggg acagctcttt aggaaacgga aaaaacctta 1800
aatagtgaat aaacaactac aatcacttaa ccattgtagg cttaaaagca agccatcaat 1860
aaagaaagcg tcaagccaca tcatcttaca cacacactaa ttccacaaac ctcaaaaatt 1920
ccaaaattac aaattgggct aaatctatga tcctagtgaa tactgttaat atgtgaacaa 1980
gaaccaatcc accaagcaca agtgctaaga caaccggata accattgtta attattgaat 2040
cataggcata acccaacaat agaattacct atccctaact cgttagccca acacaggcgt 2100
gctttaagga aagtttaaaa aagtaaagga actcggcaaa cacgaacccc gcctgtttac 2160
caaaaacatc tcctctagca taacaagtat tagtggcatc gcctgcccag tgactaaagt 2220
tccacggccg cggtatcccg accgtgcaaa ggtaggataa tcacttgttc cttaattagg 2280
gactagaatg aatggctaaa cgagggtcca accgtctctt acttgcaatc agtgaaattg 2340
accttccagt gaaggggcgg actcataata aaagacgaga agaccctatg gagctttaat 2400
ttactaattt caatttatat aaaaaaacct aatgggcgaa aacaacaaaa ttatgaacta 2460
ccaaatttcg gttggggtga cctcggagaa taaaaaatcc tccgaatgat tataaccgag 2520
tcggtaaccg tgtccgaccc agtcaagtaa tactaatatc ttattgaccc aattattgat 2580
caacggacca agttacccta gggtaacagc gcgacctatt taagagttca tatcgacaat 2640
tagggtttac gacctcgatg ttggatcagg acatcccaat ggtgcagaag ctattaatgg 2700
ttcgtttgtt caacgattaa agtcctacgt gatctaagtc cggcaatcca ggtcggtttc 2760
tatctattta caatttctcc cagttacgaa aggacaagag aaatggagac caaccaatcc 2820

taggcttcca accaatttag aaaaacttaa taaagtatat atgtacaata aataacctta 2880
gacccaagtt attagggtgg cagagccaag taattgcgta agacttaaaa ccttgttccc 2940
agaggttcaa atcctctccc taatagtgta ctttattaat atcctaacac tcctaatccc 3000
aatcttaatt gccataggcc ttctcaccct agtagaacgg aaaatcctag gctacataca 3060
attacgcaaa ggccccaaca acgaaggccc atatggtaaa ctacaaccat ttgcagatgc 3120
cataaaacta ttcataaaag aacccatacg ccctctaacc acctcaatat cactatttat 3180
tatcgcccca accctctccc ttacactagc tctaagccta tgatt 3225

<210> 89
<211> 840
<212> PRT
<213> Rattus norvegicus

<220>
<223> Ischemia responsive 94 kD protein

<400> 89
Met Ser Val Val Gly Ile Asp Leu Gly Phe Gln Ser Cys Tyr Val Ala
1               5                   10                  15

Val Ala Arg Ala Gly Gly Ile Glu Thr Ile Ala Asn Glu Tyr Ser Asp
            20                  25                  30

Arg Cys Thr Pro Ala Cys Val Ser Phe Gly Pro Lys Asn Arg Ser Val
        35                  40                  45

Gly Ala Ala Ala Lys Ser Gln Val Ile Ser Asn Ala Lys Asn Thr Val
    50                  55                  60

Gln Gly Phe Lys Arg Phe His Gly Arg Ala Phe Ser Asp Pro Phe Val
65                  70                  75                  80

Glu Ala Glu Lys Ser Asn Leu Ala Tyr Asp Ile Val Gln Leu Pro Thr
                85                  90                  95

Gly Leu Thr Gly Ile Lys Val Thr Tyr Met Glu Glu Glu Arg Asn Phe
            100                 105                 110

Thr Thr Glu Gln Val Thr Ala Met Leu Leu Ser Lys Leu Lys Glu Thr
        115                 120                 125

Ala Glu Ser Val Leu Lys Lys Pro Val Val Asp Cys Val Val Ser Val
    130                 135                 140

Pro Ser Phe Tyr Thr Asp Ala Glu Arg Arg Ser Val Met Asp Ala Thr
145                 150                 155                 160

Gln Ile Ala Gly Leu Asn Cys Leu Arg Leu Met Asn Glu Thr Thr Ala
                165                 170                 175

Val Ala Leu Ala Tyr Gly Ile Tyr Lys Gln Asp Leu Pro Ala Leu Glu
            180                 185                 190

Glu Lys Pro Arg Asn Val Val Phe Val Asp Met Gly His Ser Ala Tyr
        195                 200                 205

Gln Val Ser Val Cys Ala Phe Asn Arg Gly Lys Leu Lys Val Leu Ala
    210                 215                 220

Thr Ala Phe Asp Thr Thr Leu Gly Gly Arg Lys Phe Asp Glu Val Leu

225                 230                 235                 240

Val Asn His Phe Cys Glu Glu Phe Gly Lys Lys Tyr Lys Leu Asp Ile
                245                 250                 255

Lys Ser Lys Val Arg Ala Leu Leu Arg Leu Ser Gln Glu Cys Glu Lys
            260                 265                 270

Leu Lys Lys Leu Met Ser Ala Asn Ala Ser Asp Leu Pro Leu Ser Ile
        275                 280                 285

Glu Cys Phe Met Asn Asp Ile Asp Val Ser Gly Thr Met Asn Arg Gly
    290                 295                 300

Lys Phe Leu Glu Met Cys Asp Asp Leu Leu Ala Arg Val Glu Pro Pro
305                 310                 315                 320

Leu Arg Ser Ile Leu Asp Gln Ser Lys Leu Lys Lys Glu Asp Ile Tyr
                325                 330                 335

Ala Val Glu Ile Val Gly Gly Ala Thr Arg Ile Pro Ala Val Lys Glu
            340                 345                 350

Lys Ile Ser Lys Phe Phe Gly Lys Glu Leu Ser Thr Thr Leu Asn Ala
        355                 360                 365

Asp Glu Ala Val Thr Arg Gly Cys Ala Leu Gln Cys Ala Ile Leu Ser
    370                 375                 380

Pro Ala Phe Lys Val Arg Glu Phe Ser Ile Thr Asp Val Val Pro Tyr
385                 390                 395                 400

Pro Ile Ser Leu Arg Trp Asn Ser Pro Ala Glu Glu Gly Ser Ser Asp
                405                 410                 415

Cys Glu Val Phe Pro Lys Asn His Ala Ala Pro Phe Ser Lys Val Leu
            420                 425                 430

Thr Phe Tyr Arg Lys Glu Pro Phe Thr Leu Glu Ala Tyr Tyr Ser Ser
        435                 440                 445

Pro Gln Asp Leu Pro Tyr Pro Asp Pro Ala Ile Ala Gln Phe Ser Val
    450                 455                 460

Gln Lys Val Thr Pro Gln Ser Asp Gly Ser Ser Ser Lys Val Lys Val
465                 470                 475                 480

Lys Val Arg Val Asn Val His Gly Ile Phe Ser Val Ser Ser Ala Ala
                485                 490                 495

Leu Val Glu Val His Lys Ser Glu Glu Ser Glu Glu Pro Met Glu Thr
            500                 505                 510

Asp Gln Asn Ala Lys Glu Glu Glu Lys Met Gln Val Asp Gln Glu Glu
        515                 520                 525

Pro His Thr Glu Glu Gln Gln Pro Gln Thr Pro Ala Glu Asn Lys Ala
    530                 535                 540

Glu Ser Glu Glu Met Glu Thr Ser Gln Ala Gly Ser Lys Asp Lys Lys
545                 550                 555                 560

Met Asp Gln Pro Pro Gln Ala Lys Lys Ala Lys Val Lys Thr Ser Thr
                565                 570                 575

Val Asp Leu Pro Ile Glu Ser Gln Leu Leu Trp Gln Leu Asp Arg Glu
            580                 585                 590

Met Leu Gly Leu Tyr Thr Glu Asn Glu Gly Lys Met Ile Met Gln Asp
        595                 600                 605

Lys Leu Glu Lys Glu Arg Asn Asp Ala Lys Asn Ala Val Glu Glu Tyr
    610                 615                 620

Val Tyr Glu Met Arg Asp Lys Leu Ser Gly Glu Tyr Glu Lys Phe Val
625                 630                 635                 640

Ser Glu Asp Asp Arg Asn Asn Phe Thr Leu Lys Leu Glu Asp Thr Glu
                645                 650                 655

Asn Trp Leu Tyr Glu Asp Gly Glu Asp Gln Pro Lys Gln Val Tyr Val
            660                 665                 670

Asp Lys Leu Ala Glu Leu Arg Thr Leu Gly Gln Pro Ile Lys Thr Arg
        675                 680                 685

Phe Gln Glu Ser Glu Glu Arg Pro Lys Leu Phe Glu Glu Leu Gly Lys
    690                 695                 700

Gln Ile Gln Gln Tyr Met Lys Val Ile Ser Ser Phe Lys Asn Lys Glu
705                 710                 715                 720

Asp Gln Tyr Glu His Leu Asp Ala Ala Asp Met Thr Lys Val Glu Lys
                725                 730                 735

Ser Thr Asn Glu Ala Met Glu Trp Met Asn Ser Lys Leu Asn Leu Gln
            740                 745                 750

Asn Lys Gln Ser Leu Thr Ala Asp Pro Val Val Lys Thr Lys Glu Ile
        755                 760                 765

Glu Ala Lys Ile Lys Glu Leu Thr Asn Ile Cys Ser Pro Ile Ile Ser
    770                 775                 780

Lys Pro Lys Pro Lys Val Glu Pro Pro Lys Glu Glu Pro Lys His Ala
785                 790                 795                 800

Glu Gln Asn Gly Pro Val Asp Gly Gln Gly Asp Asn Pro Gly Thr Gln
                805                 810                 815

Ala Ala Glu His Gly Ala Asp Thr Ala Val Pro Ser Asp Gly Asp Lys
            820                 825                 830

Lys Leu Pro Glu Met Asp Ile Asp
        835                 840

<210> 90
<211> 4521
<212> DNA
<213> Rattus norvegicus

<220>
<223> Ischemia responsive 94 kD protein

<400> 90
   cggcgcctct tctgcggccg tggagccgga gccggcctga gcggtagagg cccgtctttt 60
   tcaaggaccc aagcatcctc gtggacggtc gtccggcttc gcccgcccag ccgcggtgct 120
   ccatcagccc acgcggcgcg ccctctccac gcgccaaccc cgtcgggttc cgtgtgcagt 180
   gtcggcggcc ggccctgggc ccgagcccga gcagtagccg gcgccatgtc ggtggtgggc 240
   atagacctgg gctttcagag ctgctatgtc gctgtggccc gcgccggcgg cattgagacg 300
   atcgctaatg agtatagcga ccgctgcacg ccggcttgtg tttctttcgg tcctaagaat 360
   cgttcagttg gagcagcagc aaagagccag gtaatttcca atgccaagaa cacagttcaa 420
   gggtttaaaa gatttcatgg gcgtgcattc tcagatccat ttgtggaagc agaaaaatct 480
   aaccttgcat atgatattgt acagttgccc actggattaa cgggcataaa ggttacatat 540
   atggaagaag agaggaattt taccactgaa caagtgactg ccatgctctt gtcaaaactg 600
   aaggagacag cagaaagtgt tcttaagaag cctgttgtag actgtgttgt ttcggttcct 660
   agtttctaca ctgatgcaga acgacggtca gtgatggatg ccacacagat tgctggtctc 720
   aattgcttgc ggttaatgaa tgaaacaact gcagttgctc ttgcttatgg aatctataaa 780
   caagaccttc ctgccttaga agaaaaacca agaaatgtag tttttgtaga tatgggacat 840
   tctgcctatc aagtttctgt atgtgcattt aatagaggaa aactgaaagt tttggccaca 900
   gcttttgata caacattggg aggcagaaaa tttgatgagg tgttggtaaa tcacttctgt 960
   gaagaatttg gaaagaaata caagctagac ataaaatcta aagttcgtgc tttattgcga 1020
   ctctctcaag agtgtgagaa actcaagaaa ttaatgagtg cgaatgcttc agacctccct 1080
   ttgagtattg aatgctttat gaacgatatt gatgtatcgg gaaccatgaa tagaggcaaa 1140
   tttttggaga tgtgtgatga tctcttagca agagtggagc cgccacttcg tagcatcttg 1200
   gaccaatcca agttaaagaa agaagatatt tatgcagtgg agatagttgg tggagccaca 1260
   cggatccctg ccgtgaaaga gaagatcagc aagttctttg gtaaagaact cagcacaaca 1320
   ttaaatgcgg atgaagcagt cactcgaggc tgtgctctgc agtgtgcaat cttatcacct 1380
   gctttcaaag tcagagaatt ttctatcact gatgtggtac catatccaat atctttgagg 1440
   tggaattctc cagctgaaga ggggtcaagt gactgtgaag tcttccccaa aaaccatgct 1500
   gctccttttt ccaaagtcct caccttttat agaaaggaac cgttcactct ggaggcctac 1560
   tatagttctc ctcaggattt gccctatcca gatcctgcta tagctcagtt ttcagttcag 1620
   aaagtcactc cacagtctga tggctccagc tcaaaagtga aagtcaaggt ccgggtaaat 1680
   gtgcatggca ttttcagtgt gtccagtgcg gccttagtag aggtgcacaa gtccgaggag 1740
   agcgaggagc caatggagac tgatcagaat gcaaaggaag aagagaagat gcaggtggac 1800
   caggaagaac cacatactga agagcaacag ccacagacac cagcagaaaa caaggcggag 1860
   tctgaggaga tggagacctc tcaagctgga tcaaaagata aaaagatgga ccaaccacct 1920
   caagcaaaga aggcaaaagt gaagaccagc accgtggacc tgcccattga gagccagctg 1980
   ctgtggcagc tggaccggga aatgctcggc ctgtacacag agaatgaggg taagatgatc 2040
   atgcaggata aactggagaa ggaacggaat gacgccaaaa atgcggtgga agagtatgtg 2100
   tatgagatga gagacaaact tagtggtgaa tatgagaagt ttgtgagtga agatgatcgt 2160
   aataatttta ctttgaaatt agaggataca gaaaactggc tgtatgaaga tggagaagat 2220
   caaccaaagc aagtttatgt agacaaattg gctgagttaa gaactttagg tcaacctatt 2280
   aagacacggt tccaggaatc tgaagaaaga ccaaaattat ttgaagaatt agggaagcaa 2340
   atccaacagt atatgaaagt gatcagctct ttcaaaaaca aggaggacca gtatgaacac 2400
   ttggatgctg ctgacatgac aaaggtagag aaaagcacca atgaggcgat ggagtggatg 2460
   aatagtaagc tgaacctgca gaacaaacag agcctgacgg cggatccagt tgtcaagaca 2520
   aaagagattg aagctaaaat taaggagctg acaaatattt gcagtcctat aatttcaaag 2580
   cccaaaccca aagtggaacc ccctaaggag gaaccaaaac atgctgagca gaatggacca 2640
   gtggatggcc aaggagacaa cccaggcacc caagctgctg agcacggtgc agacacagct 2700
   gtgccttctg atggtgacaa gaagcttcct gagatggaca ttgattgatt ccaacacttg 2760
   tttatattaa aacagactat tataaagctt taagttgtca actttgttct gaatatcaac 2820
   tagagcaagc aaatactgga gatttcttag tcagttttta ggggatctcg gggtggggta 2880
   tataggtaat atacagagca ttttgacttc taaatagtta gatccagaga tgaagtgcac 2940
   tgttctctct tcattatgat cctgtttaag agcccagcta gtctttctga gctcttgcct 3000
   ccctccttgt tttaatcatg cttctacaag gtgaagtgtg tttggggaaa ctgagctcta 3060
   acaaaagctg tgagagccac taggcaggct tttcctcaaa catgacagaa gttcaaatta 3120
   tgcttggaaa ggctgagtgg agatcaccac cagcattctc tgcctgtggg gctgtgggac 3180
   tgtggggctg gtgcttcctc catttctgag ggtctgagaa tggcccagca tgcattaaca 3240
   caggagagcg cagagcgcag cagggcctgc tctcattgcc ctctattccc agccctgtct 3300
   tcctcttccg tccgcaagca gtataaatgc catgtgccct ctgcacttag agtgagcctt 3360
   agcgtatcaa ggggctttga cgtggctccc cgtacttcct gaaagtattc actgacactg 3420
   aagcgtcaga gtctagtgta accttaaggt aggttgtaaa taacagttgg ctgtaggcat 3480
   ggtaagtctg agcagcccat ccttagtgtg gtgggaaggt ctgtaccctg acctgctcca 3540
   cggtagatgg tgtatgtaaa ttagaggttt cgatggaaca ggatgagagc tcaaggttct 3600
   ttgtttggta ttatttgtgt taatgtgtct tgaatctgcc aatctgtatg ttaaaacagc 3660
   tgacagattg taaagctatt gccctggaca agtctgaaat tagtgtaaaa gaaaacctca 3720
   tcgttaactg cagccgctgt cagggtacca tatattcctg gaacaagtct atggggaaga 3780
   gatgttctgc agacaaaggg acggatgcct atggtagagg tcagagttgg ttacctgcct 3840
   tagttacaag tgttacgttc tgggtctttg taatcactga tctctgtaac agagcctctg 3900
   tttattcttg cttgttggaa ataagcttaa accatgcata gttagaatta ctatgcttct 3960
   gtctacacag gaagtgctgt agctctcaag tgctctagag gccattggtt ggagaggaag 4020
   gaatgcatgc ttatgttatg taagcaactg gactcccgct cagtacaggc ctggagctcc 4080
   aggccgagtg catactctgc tgctttgagt gctgcaggag tgcccactgg cctggctgtg 4140
   aggctgatag ctttttcaag tgcaggctgc aggattaagc caactcttat gcaactgact 4200
   gtccttaatg atgatctcat aaacatttgt gtgtgggggt gggagggttt gggtgtcggg 4260
   atggggaatg aatttgaagt ctaaagttaa agctagaaat cagatgatca gcttctgctc 4320
   tgtctatata gacgattgga tttatgtgga ggcaggccag atggcaactg atgacttgtg 4380
   tgcaaacaag catttatcca ggttgcaata tccaaattgg tttgttaaaa actctaggtc 4440
   acattcttac actaagaaaa acagtaaaca tgagacaaag tttaggaaaa actaataaaa 4500
   gaaacctgtc ttaacttgaa a 4521

<210> 91
<211> 443
<212> PRT
<213> Rattus norvegicus

<220>
<223> Signalosome subunit 2

<400> 91
Met Ser Asp Met Glu Asp Asp Phe Met Cys Asp Asp Glu Glu Asp Tyr
1               5                   10                  15

Asp Leu Glu Tyr Ser Glu Asp Ser Asn Ser Glu Pro Asn Val Asp Leu
            20                  25                  30

Glu Asn Gln Tyr Tyr Asn Ser Lys Ala Leu Lys Glu Asp Asp Pro Lys
        35                  40                  45

Ala Ala Leu Ser Ser Phe Gln Lys Val Leu Glu Leu Glu Gly Glu Lys
    50                  55                  60

Gly Glu Trp Gly Phe Lys Ala Leu Lys Gln Met Ile Lys Ile Asn Phe
65                  70                  75                  80

Lys Leu Thr Asn Phe Pro Glu Met Met Asn Arg Tyr Lys Gln Leu Leu
                85                  90                  95

Thr Tyr Ile Arg Ser Ala Val Thr Arg Asn Tyr Ser Glu Lys Ser Ile
            100                 105                 110

Asn Ser Ile Leu Asp Tyr Ile Ser Thr Ser Lys Gln Met Asp Leu Leu
        115                 120                 125

Gln Glu Phe Tyr Glu Thr Thr Leu Glu Ala Leu Lys Asp Ala Lys Asn
    130                 135                 140

Asp Arg Leu Trp Phe Lys Thr Asn Thr Lys Leu Gly Lys Leu Tyr Leu
145                 150                 155                 160

Glu Arg Glu Glu Tyr Gly Lys Leu Gln Lys Ile Leu Arg Gln Leu His

165                 170                 175

Gln Ser Cys Gln Thr Asp Asp Gly Glu Asp Asp Leu Lys Lys Gly Thr
            180                 185                 190

Gln Leu Leu Glu Ile Tyr Ala Leu Glu Ile Gln Met Tyr Thr Ala Gln
        195                 200                 205

Lys Asn Asn Lys Lys Leu Lys Ala Leu Tyr Glu Gln Ser Leu His Ile
    210                 215                 220

Lys Ser Ala Ile Pro His Pro Leu Ile Met Gly Val Ile Arg Glu Cys
225                 230                 235                 240

Gly Gly Lys Met His Leu Arg Glu Gly Glu Phe Glu Lys Ala His Thr
                245                 250                 255

Asp Phe Phe Glu Ala Phe Lys Asn Tyr Asp Glu Ser Gly Ser Pro Arg
            260                 265                 270

Arg Thr Thr Cys Leu Lys Tyr Leu Val Leu Ala Asn Met Leu Met Lys
        275                 280                 285

Ser Gly Ile Asn Pro Phe Asp Ser Gln Glu Ala Lys Pro Tyr Lys Asn
    290                 295                 300

Asp Pro Glu Ile Leu Ala Met Thr Asn Leu Val Ser Ala Tyr Gln Asn
305                 310                 315                 320

Asn Asp Ile Thr Glu Phe Glu Lys Ile Leu Lys Thr Asn His Ser Asn
                325                 330                 335

Ile Met Asp Asp Pro Phe Ile Arg Glu His Ile Glu Glu Leu Leu Arg
            340                 345                 350

Asn Ile Arg Thr Gln Val Leu Ile Lys Leu Ile Lys Pro Tyr Thr Arg
        355                 360                 365

Ile His Ile Pro Phe Ile Ser Lys Glu Leu Asn Ile Asp Val Ala Asp
    370                 375                 380

Val Glu Ser Leu Leu Val Gln Cys Ile Leu Asp Asn Thr Ile His Gly
385                 390                 395                 400

Arg Ile Asp Gln Val Asn Gln Leu Leu Glu Leu Asp His Gln Lys Arg
                405                 410                 415

Gly Gly Ala Arg Tyr Thr Ala Leu Asp Lys Trp Thr Asn Gln Leu Asn
            420                 425                 430

Ser Leu Asn Gln Ala Val Val Ser Lys Leu Ala
        435                 440


<210> 92
<211> 1929
<212> DNA
<213> Rattus norvegicus

<220>
<223> Signalosome subunit 2

<400> 92
gcacgagctg acatggagga tgatttcatg tgcgatgatg aggaggacta cgacctggag 60
tactctgaag acagtaactc tgagcccaat gtggatttgg aaaatcagta ctataattcc 120
aaagcactga aagaagatga cccaaaagca gcattaagca gcttccaaaa ggttttggag 180
cttgaaggtg agaagggaga atggggattc aaagcgctaa aacagatgat caagattaac 240
ttcaagttga caaactttcc agaaatgatg aacagatata agcaactatt gacctatatt 300
cggagtgcag tcaccaggaa ctattctgaa aagtccatta attctatcct tgattatatc 360
tccacttcta aacagatgga tttactgcag gaattttatg aaacaacact ggaagctttg 420
aaagatgcta agaatgatag actgtggttt aagacaaaca caaagcttgg aaaattatat 480
ttagaacgag aagaatatgg aaagcttcaa aaaattttac gacagttaca tcagtcttgt 540
cagactgatg atggagaaga tgacctgaaa aaaggtaccc agttattaga aatctatgct 600
ttggaaattc aaatgtacac tgcacagaag aacaacaaaa agcttaaagc actctatgag 660
caatcacttc acatcaagtc tgccatccct cacccactaa tcatgggtgt catcagagaa 720
tgcggtggta agatgcactt gagagaaggt gaatttgaaa aggcacacac tgattttttt 780
gaagccttca agaattatga tgaatcagga agcccaagac gaaccacttg tttaaaatat 840
ttggttttag caaatatgct aatgaaatca ggaataaatc cgtttgactc acaagaggcc 900
aagccgtata aaaatgatcc agaaattcta gcaatgacaa atttagtaag tgcctatcag 960
aataatgaca tcactgaatt tgaaaagatt ctgaaaacaa atcacagcaa catcatggat 1020
gatcctttca taagagagca cattgaagaa cttttacgaa acatcagaac acaagtcctc 1080
ataaagttaa ttaagcctta cacaagaata catattcctt ttatttctaa ggagctaaac 1140
atagacgtag ctgatgtgga gagcttgctg gtgcagtgca tactggataa cactattcat 1200
ggccgaattg atcaagtcaa ccagctcctt gaactggatc atcagaagag gggtggtgcc 1260
cgatacactg cgctagataa atggaccaac caactaaatt ctctgaacca ggctgtggtc 1320
agtaaactgg cttaacggag accgagcttt gcggacatgc ttaaggcaac agtgcagaga 1380
tgtgaccctt gaggactgga aagacaaagc tccagtgggt tggtgtgtcc tgaaagggat 1440
ggagttatgg cagaagtgct tttgtgatca actggtttgt ggtttgctgc tgcatttatc 1500
ccaagaaaac agctttaatc tccagaagaa aaccaaaaca ccatgggatt tatgctgtgt 1560
tgacatcttg ccctaaacat acaacaccat agtaatttgt catgggcgac atgatcagag 1620
agaagatttt tgtcatgatt ttaaaaacac tgacacgcta ctgttggtta aatttaaaca 1680
tgttgtacct gcagaaattc tctcacaaat aacctgcaat aacttgaaat tcataccctt 1740
ttgaacactt ccttttctca tgtataaatg aaaatgtttg ctgcattttg caaaatgtca 1800
attctccaaa actgtgtccg tctatttctg ttcttgcagc tagtaaaggt tagacaaccc 1860
gaattctcag tggcacgctg tcacctaggg ctcaagcagc aaaataaaca gtgcagctca 1920
gaaattaaa 1929

<210> 93
<211> 777
<212> PRT
<213> Rattus norvegicus

<220>
<223> rS-Rex-s

<400> 93
Met Ala Ala Pro Pro Asp Leu Gln Asp Glu Pro Leu Ser Pro Ala Asn
1               5                   10                  15

Pro Gly Ser Gln Leu Phe Gly Gly Arg Gly Glu Gly Glu Glu Ala Thr
            20                  25                  30

Pro Lys Gly Ala Arg Pro Ala Gln Gln Asp Gly Glu Pro Ala Trp Gly
        35                  40                  45

Ser Gly Ala Gly Ala Gly Val Val Ser Ser Arg Gly Leu Cys Ser Gly
    50                  55                  60

Pro Ala Arg Ser Pro Pro Val Ala Met Glu Thr Ala Ser Thr Gly Val
65                  70                  75                  80

Ala Ala Val Pro Asp Ala Leu Asp His Ser Ser Ser Pro Thr Leu Lys
                85                  90                  95

Asp Gly Glu Gly Ala Cys Tyr Thr Ser Leu Ile Ser Asp Ile Cys Tyr
            100                 105                 110

Pro Pro Arg Glu Asp Ser Ala Tyr Phe Thr Gly Ile Leu Gln Lys Glu
        115                 120                 125

Asn Gly His Ile Thr Thr Ser Glu Ser Pro Glu Glu Leu Gly Thr Pro
    130                 135                 140

Gly Pro Ser Leu Pro Glu Val Pro Gly Thr Glu Pro His Gly Leu Leu
145                 150                 155                 160

Ser Ser Asp Ser Gly Ile Glu Met Thr Pro Ala Glu Ser Thr Glu Val
                165                 170                 175

Asn Lys Ile Leu Ala Asp Pro Leu Asp Gln Met Lys Ala Glu Ala Cys
            180                 185                 190

Lys Tyr Ile Asp Ile Thr Arg Pro Gln Glu Ala Lys Gly Gln Glu Glu
        195                 200                 205

Gln Ser Pro Gly Leu Glu Asp Lys Asp Leu Asp Phe Lys Asp Lys Asp
    210                 215                 220

Ser Glu Val Ser Thr Lys Pro Glu Gly Val His Ala Pro Asn Gln Pro
225                 230                 235                 240

Ser Pro Val Glu Gly Lys Leu Ile Lys Asp Asn Leu Phe Glu Glu Ser
                245                 250                 255

Thr Phe Ala Pro Tyr Ile Asp Glu Leu Ser Asp Glu Gln His Arg Met
            260                 265                 270

Ser Leu Val Thr Ala Pro Val Lys Ile Thr Leu Thr Glu Ile Gly Pro
        275                 280                 285

Pro Val Met Thr Ala Thr His Glu Thr Ile Pro Glu Lys Gln Asp Leu
    290                 295                 300

Cys Leu Lys Pro Ser Pro Asp Thr Val Pro Thr Val Thr Val Ser Glu
305                 310                 315                 320

Pro Glu Asp Asp Ser Pro Gly Ser Val Thr Pro Pro Ser Ser Gly Thr
                325                 330                 335

Glu Pro Ser Ala Ala Glu Ser Gln Gly Lys Gly Ser Val Ser Glu Asp
            340                 345                 350

Glu Leu Ile Ala Ala Ile Lys Glu Ala Lys Gly Leu Ser Tyr Glu Thr
        355                 360                 365

Thr Glu Ser Pro Arg Pro Val Gly Gln Ala Ala Asp Arg Pro Lys Val
    370                 375                 380

Lys Ala Arg Ser Gly Leu Pro Thr Ile Pro Ser Ser Leu Asp Gln Glu
385                 390                 395                 400

Ala Ser Ser Ala Glu Ser Gly Asp Ser Glu Ile Glu Leu Val Ser Glu

405                 410                 415

Asp Pro Met Ala Ser Glu Asp Ala Leu Pro Ser Gly Tyr Val Ser Phe
            420                 425                 430

Gly His Val Ser Gly Pro Pro Pro Ser Pro Ala Ser Pro Ser Ile Gln
        435                 440                 445

Tyr Ser Ile Leu Arg Glu Glu Arg Glu Ala Glu Leu Asp Ser Glu Leu
    450                 455                 460

Ile Ile Glu Ser Cys Asp Ala Ser Ser Ala Ser Glu Glu Ser Pro Lys
465                 470                 475                 480

Arg Glu Gln Asp Ser Pro Pro Met Lys Pro Gly Val Leu Asp Ala Ile
                485                 490                 495

Arg Glu Glu Thr Ser Ser Arg Ala Thr Glu Glu Arg Ala Pro Ser His
            500                 505                 510

Gln Gly Pro Val Glu Pro Asp Pro Ile Leu Ser Phe Thr Pro Val Thr
        515                 520                 525

Leu Gln Ser Arg Pro Glu Pro Ser Ser Gly Asp Gly Ala Pro Val Pro
    530                 535                 540

Glu Pro Pro Lys Ser Gln Gln Gln Lys Pro Glu Glu Glu Ala Val Ser
545                 550                 555                 560

Ser Ser Gln Ser Pro Ala Ala Thr Glu Ile Pro Gly Pro Leu Gly Ser
                565                 570                 575

Asp Leu Val Pro Pro Leu Pro Phe Phe Asn Lys Gln Lys Ala Ile Asp
            580                 585                 590

Leu Leu Tyr Trp Arg Asp Ile Lys Gln Thr Gly Ile Val Phe Gly Ser
        595                 600                 605

Phe Leu Leu Leu Leu Phe Ser Leu Thr Gln Phe Ser Val Val Ser Val
    610                 615                 620

Val Ala Tyr Leu Ala Leu Ala Ala Leu Ser Ala Thr Ile Ser Phe Arg
625                 630                 635                 640

Ile Tyr Lys Ser Val Leu Gln Ala Val Gln Lys Thr Asp Glu Gly His
                645                 650                 655

Pro Phe Lys Ala Tyr Leu Glu Leu Glu Ile Thr Leu Ser Gln Glu Gln
            660                 665                 670

Ile Gln Lys Tyr Thr Asp Cys Leu Gln Leu Tyr Val Asn Ser Thr Leu
        675                 680                 685

Lys Glu Leu Arg Arg Leu Phe Leu Val Gln Asp Leu Val Asp Ser Leu
    690                 695                 700

Lys Phe Ala Val Leu Met Trp Leu Leu Thr Tyr Val Gly Ala Leu Phe
705                 710                 715                 720

Asn Gly Leu Thr Leu Leu Leu Met Ala Val Val Ser Met Phe Thr Leu
                725                 730                 735

Pro Val Val Tyr Val Lys His Gln Ala Gln Val Asp Gln Tyr Leu Gly
            740                 745                 750

Leu Val Arg Thr His Ile Asn Thr Val Val Ala Lys Ile Gln Ala Lys
        755                 760                 765

Ile Pro Gly Ala Lys Arg His Ala Glu
    770                 775

<210> 94
<211> 1502
<212> DNA
<213> Rattus norvegicus

<220>
<223> rS-Rex-s

<400> 94
gcgagtctgc agccgcgcgg ccaccagagc ccaccgccgc cgcagccggg cagcgacgca 60
gagtgcaagc tcagcgactg gaggagagcc acggcgagcc cgccaagccc gcaccatgca 120
ggccactgcc gattccacca agatggactg tgtgtggagc aactggaaaa gtcaggctat 180
tgaccttctg tactggcggg acatcaagca gacggggatt gtgttcggga gcttcctgct 240
gctgctcttc tccctgaccc agttcagcgt tgtgagcgtc gtcgcctacc tggccctggc 300
tgccctctct gccaccatca gcttccgcat ctacaagtcc gttctacaag ctgtgcagaa 360
aacagatgag ggtcaccctt tcaaggccta cctggagctg gagatcaccc tgtcccagga 420
gcagatccag aagtacacag actgcctgca gctatacgtg aacagcactc tgaaggagct 480
acggaggctc ttcctggtcc aggacctggt ggattcctta aaatttgcag tcctcatgtg 540
gctcctgacc tacgttggcg cactcttcaa tggcctgacc ctgctgctta tggctgtggt 600
ttcgatgttt actctacctg tggtatatgt taagcaccag gcacaggttg accaatatct 660
gggacttgtg aggactcaca taaacaccgt tgtggcaaag atccaggcta aaatccccgg 720
cgccaagagg cacgctgagt agatggttgc cccaggagaa gccggacatg aatgggggtg 780
tctggagtgg taacagctct ctccttcctc gttactgcga attgattgtt tcccttcccc 840
agtaccataa tcttagaggc aaacttcgag acaggtgttt tcaggctttt cctgtactct 900
taggatattt gagtcccccc gccccttgtc aagcactaaa gtatagagtg tgttagatgt 960
ggtttttaat tttgtgttgc taaaaaaaaa agtgcatgat ggtgagagct cacgttctcc 1020
ttcgtccctt gctgtcctcc ccctgcaatg cttctgtggc ttctactgtc cctgtggcta 1080
ggcccttctt gccaagtgcg ctgacacata gtgggtcgct tatcatgtcc ttgggttgct 1140
ggttggattc atctctaata acactatata gaatcatagg ccaatgtttt cgcattttac 1200
ccccccacac acagaacata aaaattaata taaaaccgtt gaccgtgtat atggtcgagc 1260
cgttttgtat aagctaagaa tgatcgaatt aaagaataac tccaaaaatc gaccgcacag 1320
tacttttatt gtatgggatg ccctgggctg atttacatgt atggtaacta aagtgccagc 1380
atgttgactt tattacgatt tgtatttctt cctctgtagt tcctaacgga ctcaatcaca 1440
gactctgggt atttgcactt atgtacttga tactgaatgc atcaataaat gtgactcaat 1500
gt                                                                 1502

<210> 95
<211> 212
<212> DNA
<213> Rattus norvegicus

<220>
<223> Haemoglobin beta chain

<400> 95
aggctgctgg ttgtctaccc ttggacccag aggtactttg atagctttgg ggacctgtcc 60
tctgcctctg ctatcatggg taaccctaag gtgaaggccc atggcaagaa ggtgataaac 120
gccttcaatg atggcctgaa acacttggac aacctcaagg gcacctttgc tcatctgagt 180
gaactccact gtaagggcct gcatgtggat cc                                212

<210> 96
<211> 433
<212> DNA
<213> Rattus norvegicus

<220>
<223> PMSG-induced ovarian mRNA

<220>
<221> misc_feature
<222> 377
<223> n is a or g or c or t

<400> 96
acaaaaaact gtttattgct gtgacagcca ccagaagctg tcaaggagcc cattgctgtg 60
aggggccagg cagccagtgg atgggctgaa cgccagtctc acgagtcaac caacctgctg 120
ctggccccgc ccacccagca ataggctgtc acactgatta aaacccaacc aggagatagg 180
aaacccccat aggttcacct tcctaggtct catgacctag acgaagaaga gtgccccaaa 240
tacccctcca aaataagacc tgccctggtc cctcatattt ctgaggatcc tggggtgtca 300
tacagccctg cagcaagcca gagttgcagc cccaaggaag taccagctcc tctctgttcc 360
acaggctgtc ccaagantcc agcccaaggt attcagctgc ctctagaatg caggggggga 420
atcactagga ata 433


<210> 97
<211> 1230
<212> PRT
<213> Rattus norvegicus

<220>
<223> TBP-interacting protein

<400> 97
   Met Ala Ser Ala Ser Tyr His Ile Ser Asn Leu Leu Glu Lys Met Thr
   1               5                   10                  15
   Ser Ser Asp Lys Asp Phe Arg Phe Met Ala Thr Asn Asp Leu Met Thr
               20                  25                  30
   Glu Leu Gln Lys Asp Ser Ile Lys Leu Asp Asp Asp Ser Glu Arg Lys
           35                  40                  45
   Val Val Lys Met Ile Leu Lys Leu Leu Glu Asp Lys Asn Gly Glu Val
       50                  55                  60
   Gln Asn Leu Ala Val Lys Cys Leu Gly Pro Leu Val Ser Lys Val Lys
   65                  70                  75                  80
   Glu Tyr Gln Val Glu Thr Ile Val Asp Thr Leu Cys Thr Asn Met Leu
                   85                  90                  95
   Ser Asp Lys Glu Gln Leu Arg Asp Ile Ser Ser Ile Gly Leu Lys Thr
               100                 105                 110
   Val Ile Gly Glu Leu Pro Pro Ala Ser Ser Gly Ser Ala Leu Ala Ala
           115                 120                 125
   Asn Val Cys Lys Lys Ile Thr Gly Arg Leu Thr Ser Ala Ile Ala Lys
       130                 135                 140
   Gln Glu Asp Val Ser Val Gln Leu Glu Ala Leu Asp Ile Met Ala Asp
   145                 150                 155                 160
   Met Leu Ser Arg Gln Gly Gly Leu Leu Val Asn Phe His Pro Ser Ile
                   165                 170                 175
   Leu Thr Cys Leu Leu Pro Gln Leu Thr Ser Pro Arg Leu Ala Val Arg
               180                 185                 190
   Lys Arg Thr Ile Ile Ala Leu Gly His Leu Val Met Ser Cys Gly Asn
           195                 200                 205
   Ile Val Phe Val Asp Leu Ile Glu His Leu Leu Ser Glu Leu Ser Lys
       210                 215                 220
   Asn Asp Ser Met Ser Thr Thr Arg Thr Tyr Ile Gln Cys Ile Ala Ala
   225                 230                 235                 240
   Ile Ser Arg Gln Ala Gly His Arg Ile Gly Glu Tyr Leu Glu Lys Ile
                   245                 250                 255
   Ile Pro Leu Val Val Lys Phe Cys Asn Val Asp Asp Asp Glu Leu Arg
               260                 265                 270
   Glu Tyr Cys Ile Gln Ala Phe Glu Ser Phe Val Arg Arg Cys Pro Lys
           275                 280                 285
   Glu Val Tyr Pro His Val Ser Thr Ile Ile Asn Ile Cys Leu Lys Tyr
       290                 295                 300
   Leu Thr Tyr Asp Pro Asn Tyr Asn Tyr Asp Asp Glu Asp Glu Asp Glu
   305                 310                 315                 320
   Asn Ala Met Asp Ala Asp Gly Gly Asp Asp Asp Asp Gln Gly Ser Asp
                   325                 330                 335
   Asp Glu Tyr Ser Asp Asp Asp Asp Met Ser Trp Lys Val Arg Arg Ala
               340                 345                 350
   Ala Ala Lys Cys Leu Asp Ala Val Val Ser Thr Arg His Glu Met Leu
           355                 360                 365
   Pro Glu Phe Tyr Lys Thr Val Ser Pro Ala Leu Ile Ser Arg Phe Lys
       370                 375                 380
   Glu Arg Glu Glu Asn Val Lys Ala Asp Val Phe His Ala Tyr Leu Ser
   385                 390                 395                 400
   Leu Leu Lys Gln Thr Arg Pro Val Gln Ser Trp Leu Cys Asp Pro Asp
                   405                 410                 415
   Ala Met Glu Gln Gly Glu Thr Pro Leu Thr Met Leu Gln Ser Gln Val
               420                 425                 430
   Pro Asn Ile Val Lys Ala Leu His Lys Gln Met Lys Glu Lys Ser Val
           435                 440                 445
   Lys Thr Arg Gln Cys Cys Phe Asn Met Leu Thr Glu Leu Val Asn Val
       450                 455                 460
   Leu Pro Gly Ala Leu Thr Gln His Ile Pro Val Leu Val Pro Gly Ile
   465                 470                 475                 480
   Ile Phe Ser Leu Asn Asp Lys Ser Ser Ser Ser Asn Leu Lys Ile Asp
                   485                 490                 495
   Ala Leu Ser Cys Leu Tyr Val Ile Leu Cys Asn His Ser Pro Gln Val
               500                 505                 510
   Phe His Pro His Val Gln Ala Leu Val Pro Pro Val Val Ala Cys Val
           515                 520                 525
   Gly Asp Pro Phe Tyr Lys Ile Thr Ser Glu Ala Leu Leu Val Thr Gln
       530                 535                 540
   Gln Leu Val Lys Val Ile Arg Pro Leu Asp Gln Pro Ser Ser Phe Asp
   545                 550                 555                 560
   Ala Thr Pro Tyr Ile Lys Asp Leu Phe Thr Cys Thr Ile Lys Arg Leu
                   565                 570                 575
   Lys Ala Ala Asp Ile Asp Gln Glu Val Lys Glu Arg Ala Ile Ser Cys
               580                 585                 590
   Met Gly Gln Ile Ile Cys Asn Leu Gly Asp Asn Leu Gly Pro Asp Leu
           595                 600                 605
   Ser Asn Thr Leu Gln Ile Phe Leu Glu Arg Leu Lys Asn Glu Ile Thr
       610                 615                 620
   Arg Leu Thr Thr Val Lys Ala Leu Thr Leu Ile Ala Gly Ser Pro Leu
   625                 630                 635                 640
   Lys Ile Asp Leu Arg Pro Val Leu Gly Glu Gly Val Pro Ile Leu Ala
                   645                 650                 655
   Ser Phe Leu Arg Lys Asn Gln Arg Ala Leu Lys Leu Gly Thr Leu Ser
               660                 665                 670
   Ala Leu Asp Ile Leu Ile Lys Asn Tyr Ser Asp Ser Leu Thr Ala Ala
           675                 680                 685
   Met Ile Asp Ala Val Leu Asp Glu Leu Pro Pro Leu Ile Ser Glu Ser
       690                 695                 700
   Asp Met His Val Ser Gln Met Ala Ile Ser Phe Leu Thr Thr Leu Ala
   705                 710                 715                 720
   Lys Val Tyr Pro Ser Ser Leu Ser Lys Ile Ser Gly Ser Ile Leu Asn
                   725                 730                 735
   Glu Leu Ile Gly Leu Val Arg Ser Pro Leu Leu Gln Gly Gly Ala Leu
               740                 745                 750
   Ser Ala Met Leu Asp Phe Phe Gln Ala Leu Val Val Thr Gly Thr Asn
           755                 760                 765
   Asn Leu Gly Tyr Met Asp Leu Leu Arg Met Leu Thr Gly Pro Val Tyr
       770                 775                 780
   Ser Gln Ser Thr Ala Leu Thr His Lys Gln Ser Tyr Tyr Ser Ile Ala
   785                 790                 795                 800
   Lys Cys Val Ala Ala Leu Thr Arg Ala Cys Pro Lys Glu Gly Pro Ala
                   805                 810                 815
   Val Val Gly Gln Phe Ile Gln Asp Val Lys Asn Ser Arg Ser Thr Asp
               820                 825                 830
   Ser Ile Arg Leu Leu Ala Leu Leu Ser Leu Gly Glu Val Gly His His
           835                 840                 845
   Ile Asp Leu Ser Gly Gln Leu Glu Leu Lys Ser Val Ile Leu Glu Ala
       850                 855                 860
   Phe Ser Ser Pro Ser Glu Glu Val Lys Ser Ala Ala Ser Tyr Ala Leu
   865                 870                 875                 880
   Gly Ser Ile Ser Val Gly Asn Leu Pro Glu Tyr Leu Pro Phe Val Leu
                   885                 890                 895
   Gln Glu Ile Thr Ser Gln Pro Lys Arg Gln Tyr Leu Leu Leu His Ser
               900                 905                 910
   Leu Lys Glu Ile Ile Ser Ser Ala Ser Val Val Gly Leu Lys Pro Tyr
           915                 920                 925
   Val Glu Asn Ile Trp Ala Leu Leu Leu Lys His Cys Glu Cys Ala Glu
       930                 935                 940
   Glu Gly Thr Arg Asn Val Val Ala Glu Cys Leu Gly Lys Leu Thr Leu
   945                 950                 955                 960
   Ile Asp Pro Glu Thr Leu Leu Pro Arg Leu Lys Gly Tyr Leu Ile Ser
                   965                 970                 975
   Gly Ser Ser Tyr Ala Arg Ser Ser Val Val Thr Ala Val Lys Phe Thr
               980                 985                 990
   Ile Ser Asp His Pro Gln Pro Ile Asp Pro Leu Leu Lys Asn Cys Ile
           995                 1000                1005
   Gly Asp Phe Leu Lys Thr Leu Glu Asp Pro Asp Leu Asn Val Arg Arg
       1010                1015                1020
   Val Ala Leu Val Thr Phe Asn Ser Ala Ala His Asn Lys Pro Ser Leu
   1025                1030                1035                1040
   Ile Arg Asp Leu Leu Asp Ser Val Leu Pro His Leu Tyr Asn Glu Thr
                   1045                1050                1055
   Lys Val Arg Lys Glu Leu Ile Arg Glu Val Glu Met Gly Pro Phe Lys
               1060                1065                1070
   His Thr Val Asp Asp Gly Leu Asp Ile Arg Lys Ala Ala Phe Glu Cys
           1075                1080                1085
   Met Tyr Thr Leu Leu Asp Ser Cys Leu Asp Arg Leu Asp Ile Phe Glu
       1090                1095                1100
   Phe Leu Asn His Val Glu Asp Gly Leu Lys Asp His Tyr Asp Ile Lys
   1105                1110                1115                1120
   Met Leu Thr Phe Leu Met Leu Val Arg Leu Ser Thr Leu Cys Pro Ser
               1125                1130                1135
   Ala Val Leu Gln Arg Leu Asp Arg Leu Val Glu Pro Leu Arg Ala Thr
           1140                1145                1150
   Cys Thr Thr Lys Val Lys Ala Asn Ser Val Lys Gln Glu Phe Glu Lys
       1155                1160                1165
   Gln Asp Glu Leu Lys Arg Ser Ala Met Arg Ala Val Ala Ala Leu Leu
    1170                1175                1180
   Thr Ile Pro Glu Ala Glu Lys Ser Pro Leu Met Ser Glu Phe Gln Ser
   1185                1190                1195                1200
   Gln Ile Ser Ser Asn Pro Glu Leu Ala Ala Ile Phe Glu Ser Ile Gln
               1205                1210                1215
   Lys Asp Ser Ser Ser Thr Asn Leu Glu Ser Met Asp Thr Ser
           1220                1225                1230

<210> 98
<211> 4383
<212> DNA
<213> Rattus norvegicus

<220>
<223> TBP-interacting protein

<400> 98
tctggcctct tgccctaaga agcgagtgcg gagggagtgg gagcgagact gcccgaacgc 60
aagtgtctgg cttcccggcg aggcccaggc gtccgtcccg ccgcccatga actcgttgcc 120
ccgcgagcag cgccgtcgtt aggccgtccc cgtcactctc gctcgccccc atccccgggc 180
agccattccc ccctacccac gtctggccgg ggactggacg tcgtgtcgaa gcgccttccc 240
cagtccgcgt cgcgctgcga ccctggaagc gggcgccgcc gccaacgaga ggaggagccc 300
cggccgcggg gcccagcagc cactgagcca gcaggcggga tcgaggccgg caacatggcg 360
agcgcttcgt accacatctc caacttgctg gaaaaaatga catccagcga caaggacttc 420
aggtttatgg ctacaaatga tctgatgaca gaactgcaga aagactccat caagctggat 480
gatgacagcg aaaggaaagt cgtaaagatg attctgaagt tgctggagga taaaaatggc 540
gaagtgcaga acttagctgt gaaatgtctt ggtcctttag tgagtaaagt gaaagagtac 600
caagttgaga cgattgtaga caccctctgt acaaacatgc tttctgacaa ggagcagctt 660
cgagatattt ccagtattgg ccttaaaaca gtaattggag aactccctcc agcttccagt 720
ggctctgcat tagctgctaa tgtatgtaaa aagattactg gacgccttac cagtgcaata 780
gcaaagcagg aagatgtttc tgttcagcta gaagctttag atattatggc tgatatgttg 840
agcaggcaag gaggactgct tgttaatttc catccttcaa ttctgacctg tctacttccc 900
cagctgacta gccctagact tgcagtcagg aaaagaacaa ttattgctct tggccaccta 960
gttatgagct gcggaaacat agtttttgta gaccttattg aacatctgtt gtcagagttg 1020
tccaaaaatg actccatgtc aacaacaagg acctacatac agtgtattgc tgctattagt 1080
aggcaagctg gccatagaat aggtgaatac cttgaaaaga taattccttt ggtggtaaaa 1140
ttttgtaatg tagatgatga tgaattaaga gaatactgca ttcaagcttt tgaatcattt 1200
gtgagaagat gccctaagga agtttatcct catgtttcta ccattataaa catttgtcta 1260
aaatacctta cctatgatcc aaattacaac tatgatgatg aagatgaaga tgagaatgct 1320
atggatgctg atggcggaga tgacgatgac caagggagtg acgatgaata cagtgatgac 1380
gatgacatga gttggaaagt gaggcgtgcg gctgccaagt gcctggatgc cgtagttagc 1440
acacggcatg agatgctccc ggaattctac aagaccgtct ctcctgcact gatatccaga 1500
tttaaagagc gcgaagagaa tgtaaaggca gatgtttttc atgcatacct ttctcttttg 1560
aagcaaactc gtccagtaca aagttggctg tgcgaccctg atgcaatgga gcaaggagaa 1620
acacccttaa caatgcttca gagtcaggtt cccaacattg ttaaagccct acataaacag 1680
atgaaagaaa aaagtgtaaa gacccgacag tgttgtttta acatgttaac tgaactggta 1740

aatgtgttgc ctggggcact aacccaacac attcctgtac ttgtaccagg aatcattttc 1800
tcactcaatg ataagtccag ctcatcgaat ctgaagattg atgcattgtc ctgtctgtat 1860
gtgatcctct gtaaccactc tccgcaagtt ttccacccgc atgttcaggc tttggtccct 1920
ccagtggtgg cttgtgttgg tgacccattt tacaagatca catcagaagc ccttcttgtc 1980
actcagcagc ttgtcaaagt aatccgtcct ctggaccaac cctcctcctt cgatgcaacg 2040
ccttacatca aagatctctt cacttgtaca attaagcgct taaaagcagc tgacattgat 2100
caggaagtca aggagagggc tatttcctgt atgggacaga ttatttgcaa tcttggagac 2160
aatttgggcc ctgacttatc aaatacactt cagattttct tggagagact caagaatgaa 2220
atcacccggc taacgacagt gaaagcactg accctgattg ctgggtcacc tttgaagata 2280
gatctgaggc ctgtgctggg agagggagtc cctatccttg cttcatttct caggaaaaat 2340
cagagagctt tgaaactggg gaccctctct gccctagata ttctcattaa aaactatagt 2400
gacagtttga cggccgccat gattgatgca gttctggatg agctccctcc tcttatcagc 2460
gaaagtgata tgcacgtgtc ccagatggct atcagcttcc ttactaccct ggcaaaagta 2520
tatccctcct ccctttcaaa gataagcgga tctattctca atgaactgat tggacttgta 2580
aggtcacctc tgctgcaggg aggagctctt agtgccatgc tagacttctt ccaagctttg 2640
gttgtcactg gaacaaacaa tctaggatac atggatttgc tgcgcatgct aacgggtcca 2700
gtttactctc agagcacagc tcttactcat aagcagtctt actattccat tgccaaatgt 2760
gtagctgccc ttactcgagc atgccctaaa gagggaccgg ctgtagtagg tcagtttatt 2820
caagatgtca agaactccag gtctacagat tccattcgtc tcttagcgct cctttctcta 2880
ggagaagttg gacaccatat tgacttaagt gggcagttgg agctaaaatc tgtaatatta 2940
gaggctttct cctctcctag tgaagaagtt aaatcagctg catcctatgc gttaggcagc 3000
atcagtgtag gcaacctccc tgagtatctg ccatttgtac tacaagaaat aaccagtcag 3060
cccaaaaggc agtacctgct gctgcattcc ctgaaggaaa tcattagctc tgcgtcagtg 3120
gtcggcctta agccgtatgt cgagaacatc tgggccttgc tgctaaagca ctgtgagtgc 3180
gcggaggagg gcaccagaaa cgtcgtcgcc gagtgtctag ggaagctcac tcttattgat 3240
cctgaaactc tcctcccgag gcttaaaggg tatttgatat cagggtcatc ctatgccaga 3300
agctcagtgg ttacagctgt gaagttcact atttctgacc accctcagcc cattgatcca 3360
ctgctcaaga actgcatagg tgattttcta aaaacgttgg aagacccaga tttgaatgta 3420
agaagagtgg ccttggtcac attcaattct gcagcccata acaagccgtc actgatacgg 3480
gaccttctgg attctgttct tccacatctt tacaatgaga caaaagttag gaaggaactt 3540
ataagagagg tagagatggg cccgtttaag cacacggttg atgacggcct ggacattaga 3600
aaggcagctt ttgagtgtat gtacacactt ctagacagct gtcttgacag actggatatc 3660
tttgaatttc taaatcatgt ggaagatggt ttgaaggacc attatgatat taagatgcta 3720
acatttttaa tgttggtgag actatctacc ctttgtccaa gtgcagtact acagaggttg 3780
gaccggcttg ttgagccact acgagctacg tgtacaacta aggtgaaggc gaactctgta 3840
aagcaggagt ttgaaaagca ggacgagtta aagcgctcgg ccatgagggc agtagctgca 3900
ctgctcacca ttccagaggc agagaagagc cctctcatga gtgagttcca gtcacagatc 3960
agctccaacc cggagctggc agccatcttt gaaagtatcc agaaagattc ttcgtccact 4020
aacttggaat caatggacac tagttagacg tgtgttccac atgaggacca ttacatttga 4080
ccgcacagtg cactgaaccg acaagttgac catagacacg ggagagaagt gtctagagct 4140
tcaggatgtt ccacttttat tttcattcat gggaagtttg ttcagctttc ttccattgtt 4200
gtttttgtag cttttacttc agaaatctgt atttccataa tccagaggtt gtaagaccac 4260
tgatggtttt agtggttagg gcaacgtttg aaatgggaac taaagtttgg atttatggaa 4320
tgtggaaata gggtccagta tctgtttgcc ctgtaatgtg taggattaaa atgttaaaac 4380
ttt 4383

<210> 99
<211> 625
<212> PRT
<213> Rattus norvegicus

<220>
<223> CRM1 protein

<400> 99
Asp Thr Asp Ser Ile Asn Leu Tyr Lys Asn Met Arg Glu Thr Leu Val
1               5                   10                  15

Tyr Leu Thr His Leu Asp Tyr Val Asp Thr Glu Ile Ile Met Thr Lys
            20                  25                  30

Lys Leu Gln Asn Gln Val Asn Gly Thr Glu Trp Ser Trp Lys Asn Leu
        35                  40                  45

Asn Thr Leu Cys Trp Ala Ile Gly Ser Ile Ser Gly Ala Met His Glu
    50                  55                  60

Glu Asp Glu Lys Arg Phe Leu Val Thr Val Ile Lys Asp Leu Leu Gly
65                  70                  75                  80

Leu Cys Glu Gln Lys Arg Gly Lys Asp Asn Lys Ala Ile Ile Ala Ser
                85                  90                  95

Asn Ile Met Tyr Ile Val Gly Gln Tyr Pro Arg Phe Leu Arg Ala His
            100                 105                 110

Trp Lys Phe Leu Lys Thr Val Val Asn Lys Leu Phe Glu Phe Met His
        115                 120                 125

Glu Thr His Asp Gly Val Gln Asp Met Ala Cys Asp Thr Phe Ile Lys
    130                 135                 140

Ile Ala Gln Lys Cys Arg Arg His Phe Val Gln Val Gln Val Gly Glu
145                 150                 155                 160

Val Met Pro Phe Ile Asp Glu Ile Leu Asn Asn Ile Asn Thr Ile Ile
                165                 170                 175

Cys Asp Leu Gln Pro Gln Gln Val His Thr Phe Tyr Glu Ala Val Gly
            180                 185                 190

Tyr Met Ile Gly Ala Gln Thr Asp Gln Thr Val Gln Glu His Leu Ile
        195                 200                 205

Glu Lys Tyr Met Leu Leu Pro Asn Gln Val Trp Asp Ser Ile Ile Gln
    210                 215                 220

Gln Ala Thr Lys Asn Val Asp Ile Leu Lys Asp Pro Glu Thr Val Lys
225                 230                 235                 240

Gln Leu Gly Ser Ile Leu Lys Thr Asn Val Arg Ala Cys Lys Ala Val
                245                 250                 255

Gly His Pro Phe Val Ile Gln Leu Gly Arg Ile Tyr Leu Asp Met Leu
            260                 265                 270

Asn Val Tyr Lys Cys Leu Ser Glu Asn Ile Ser Ala Ala Ile Gln Ala
        275                 280                 285

Asn Gly Glu Met Val Thr Lys Gln Pro Leu Ile Arg Ser Met Arg Thr
    290                 295                 300

Val Lys Arg Glu Thr Leu Lys Leu Ile Ser Gly Trp Val Ser Arg Ser
305                 310                 315                 320

Asn Asp Pro Gln Met Val Ala Glu Asn Phe Val Pro Pro Leu Leu Asp
                325                 330                 335

Ala Val Leu Ile Asp Tyr Gln Arg Asn Val Pro Ala Ala Arg Glu Pro
            340                 345                 350

Glu Val Leu Ser Thr Met Ala Ile Ile Val Asn Lys Leu Gly Gly His
            355                 360                 365

Ile Thr Ala Glu Ile Pro Gln Ile Phe Asp Ala Val Phe Glu Cys Thr
        370                 375                 380

Leu Asn Met Ile Asn Lys Asp Phe Glu Glu Tyr Pro Glu His Arg Thr
385                 390                 395                 400

Asn Phe Phe Leu Leu Leu Gln Ala Val Asn Ser His Cys Phe Pro Ala
                405                 410                 415

Phe Leu Ala Ile Pro Pro Ala Gln Phe Lys Leu Val Leu Asp Ser Ile
            420                 425                 430

Ile Trp Ala Phe Lys His Thr Met Arg Asn Val Ala Asp Thr Gly Leu
        435                 440                 445

Gln Ile Leu Phe Thr Leu Leu Gln Asn Val Ala Gln Glu Glu Ala Ala
    450                 455                 460

Ala Gln Ser Phe Tyr Gln Thr Tyr Phe Cys Asp Ile Leu Gln His Ile
465                 470                 475                 480

Phe Ser Val Val Thr Asp Thr Ser His Thr Ala Gly Leu Thr Met His
                485                 490                 495

Ala Ser Ile Leu Ala Tyr Met Phe Asn Leu Val Glu Glu Gly Lys Ile
            500                 505                 510

Ser Thr Pro Leu Asn Pro Gly Ser Pro Val Ser Asn Gln Met Phe Ile
        515                 520                 525

Gln Asp Tyr Val Ala Asn Leu Leu Lys Ser Ala Phe Pro His Leu Gln
    530                 535                 540

Asp Ala Gln Val Lys Leu Phe Val Thr Gly Leu Phe Ser Leu Asn Gln
545                 550                 555                 560

Asp Ile Pro Ala Phe Lys Glu His Leu Arg Asp Phe Leu Val Gln Ile
                565                 570                 575

Lys Glu Phe Ala Gly Glu Asp Thr Ser Asp Leu Phe Leu Glu Glu Arg
            580                 585                 590

Glu Thr Ala Leu Arg Gln Ala Gln Glu Glu Lys His Lys Leu Gln Met
        595                 600                 605

Ser Val Pro Gly Ile Leu Asn Pro His Glu Ile Pro Glu Glu Met Cys
    610                 615                 620

Asp
625


<210> 100
<211> 2099
<212> DNA
<213> Rattus norvegicus

<220>

<223> CRM1 protein

<400> 100
gatactgatt ccataaattt gtataagaat atgagagaaa cattagttta tcttacccat 60
ctggattatg tagatacgga aataataatg actaagaaac ttcaaaatca agtgaatggt 120
acagaatggt catggaaaaa tttgaataca ctgtgctggg caataggctc catcagtggg 180
gcgatgcacg aggaggatga gaagcggttc cttgtcactg ttatcaagga cctcttagga 240
ttatgtgagc agaagagagg caaggataat aaagccatca ttgcatcgaa tatcatgtac 300
atagtaggcc agtacccacg gtttctgaga gcacactgga aatttttgaa gacagtagta 360
aacaagcttt tcgaattcat gcatgaaacc catgatggag tccaggacat ggcttgtgac 420
acatttatta aaatagctca aaaatgccgt agacattttg ttcaggttca agttggagaa 480
gtaatgccgt ttattgatga gattttaaac aacataaata ctataatttg tgatcttcag 540
cctcagcagg ttcatacctt ttatgaagca gtggggtaca tgattggtgc acagacagac 600
cagacagttc aagagcattt gatagaaaaa tacatgctac tccctaatca agtatgggac 660
agtataattc aacaggcaac caaaaatgtg gatatcctga aagaccccga gacagtcaag 720
cagctgggga gcatactaaa aactaatgtg agagcctgca aggctgttgg ccaccccttt 780
gtgattcagc tggggaggat ttacttagac atgctcaatg tgtacaagtg cctcagtgaa 840
aacatttctg cagccattca agctaacggt gagatggtta caaagcaacc actgattaga 900
agtatgcgaa cagtaaaaag ggaaacttta aagttaatat ctggttgggt gagccgttcc 960
aatgatcctc agatggtagc tgaaaacttt gttcctcctc tgttggatgc ggttcttatt 1020
gattaccaga gaaatgttcc agctgctaga gagccagaag tgctcagtac tatggccatt 1080
attgtcaaca agctcggtgg acatataaca gctgaaatac ctcagatttt tgatgcagtt 1140
tttgagtgca cattgaatat gataaataag gattttgaag agtatccaga acacagaacc 1200
aactttttct tgttacttca agctgtcaat tctcactgtt tcccagcatt ccttgctatt 1260
ccacctgccc agttcaagct tgttttggat tctattattt gggcttttaa acatactatg 1320
aggaatgtcg cagatacagg cttacagata ctttttacac ttttacaaaa tgttgcacaa 1380
gaagaagctg cagcgcagag tttttaccaa acgtactttt gtgacattct tcagcatatc 1440
ttttctgttg tgacagacac ctcacatact gcgggtctaa caatgcatgc atcaattctc 1500
gcatatatgt ttaatttggt tgaagaagga aagataagca caccgctaaa tcccggaagt 1560
ccagttagca accagatgtt tattcaagac tacgtggcta atctccttaa gtctgcgttc 1620
cctcacctac aagatgctca agtaaagctc tttgtgacag gacttttcag cttaaatcaa 1680
gatattcctg ctttcaaaga acatctaagg gatttcttag ttcaaataaa ggagtttgca 1740
ggtgaagaca catcggatct atttttggaa gagagagaaa cagctcttcg acaggctcag 1800
gaggagaaac ataaacttca aatgtctgta cctggaatcc ttaacccaca tgaaatccca 1860
gaagaaatgt gtgactaaat ctgaagtccc gaagccccgc taggctcttt tccctgtgca 1920
gcgtttgtcc tgggagagca gcagatgggt tctctgtcga ccaaaatgat gccaatttgt 1980
aaattaaaat gtcacctagt gaaaaaaaaa aaaaaaaaaa aaaaaaaaaa aaaaaaaaaa 2040
aaaaaaaaaa aaaaaaaaaa aaaaaaaaaa aaaaaaaaaa aaaaaaaaaa aaaaaaaaa 2099


<210> 101
<211> 1029
<212> DNA
<213> Mus musculus

<220>
<223> SGC10

<400> 101
ggaaaacacc acctgggtta tttcaggccg aacatgtcca gcaacagcta aggtggctct 60
gtgagttctt gggaccacat gcactgaccc ctctgccctt gctctttcta ggagaggcat 120
gctgcagagg ttcgcaggaa caaggaactg caggttgaac tgtctggctg aagcaatggc 180
gtgtatggca agccctccca tagtaaatcc ccctgcctat attataatgg atcatgcgat 240
atcagtatgg gaagtgtatg acatggttta aaaagaactc attatgaaaa acaaaacaaa 300
aaacagaatc aaaaataaaa taaaaatcaa tgcggtctct ttgcagaatg ttttgcttga 360
tgtttaaaaa agtaccttgg atcttatttt gcaaatattt acatttttgt taaaaaatac 420
aagtattgca ttatgcaagt tatttcatga tcttacatgt cctgtaacag gcttttgacg 480
ttgtgtcttt ccactcagat ggatttgcta ggtctgttct cttttggaag ccccactcag 540
tccctgccct tttcattcca accgaaaaat gaggtcagta gacagtctat ggtgctagaa 600
acccaccatt gcctaatgac ctagatgact ccgtaatctc tgagcgtgac caagaccaca 660
cctagatcgt gggtactgtg tgtccatgtg aacctccaca tttgctcgta atctgcttcc 720

tgcctagaag cgcaaagcca ggctaaggga atctaccagt catagcactt atgtctgctc 780
ctcatggatt acgcgctaca catgtgtttg ggttttagaa acaggtgcat gtgaagacaa 840
atctgagctc agtcagggta gaagcgaaag gctgaacctt ccaacgtgct gatcacaaac 900
gcaaagccag agggttcaag cagccatggg attgttcttc atgaagtggg cttatttctc 960
tcattttagt tattatgatg ggatgaatta attgacatgt cgggtggctt atctgtgggt 1020
gccatgggt 1029

<210> 102
<211> 892
<212> PRT
<213> Rattus norvegicus

<220>
<223> Myosin heavy chain

<400> 102
Pro Pro Gly Cys Arg Asn Ser Ala Arg Gly Lys Leu Glu Gly Asp Leu
1               5                   10                  15

Lys Leu Ala Gln Glu Ser Thr Met Asp Val Glu Asn Asp Lys Gln Gln
            20                  25                  30

Leu Asp Glu Lys Leu Lys Lys Lys Glu Phe Glu Met Ser Asn Leu Gln
        35                  40                  45

Ser Lys Ile Glu Asp Glu Gln Ala Leu Gly Met Gln Leu Gln Lys Lys
    50                  55                  60

Ile Lys Glu Leu Gln Ala Arg Ile Glu Glu Leu Glu Glu Glu Ile Glu
65                  70                  75                  .80

Ala Glu Arg Ala Ser Arg Ala Lys Ala Glu Lys Gln Arg Ser Asp Leu
                85                  90                  95

Ser Arg Glu Leu Glu Glu Ile Ser Glu Arg Leu Glu Glu Ala Gly Gly
            100                 105                 110

Ala Thr Ser Ala Gln Ile Glu Met Asn Lys Lys Arg Glu Ala Glu Phe
        115                 120                 125

Gln Lys Met Arg Arg Asp Leu Glu Glu Ala Thr Leu Gln His Glu Ala
    130                 135                 140

Thr Ala Ala Thr Leu Arg Lys Lys His Ala Asp Ser Val Ala Glu Leu
145                 150                 155                 160

Gly Glu Gln Ile Asp Asn Leu Gln Arg Val Lys Gln Lys Leu Glu Lys
                165                 170                 175

Glu Lys Ser Glu Met Lys Met Glu Ile Asp Asp Leu Ala Ser Asn Met
            180                 185                 190

Glu Val Ile Ser Lys Ser Lys Gly Asn Leu Glu Lys Met Cys Arg Thr
        195                 200                 205

Leu Glu Asp Gln Val Ser Glu Leu Lys Thr Lys Glu Glu Glu Gln Gln
    210                 215                 220

Arg Leu Ile Asn Glu Leu Thr Ala Gln Arg Gly Arg Leu Gln Thr Glu
225                 230                 235                 240

Ser Gly Glu Tyr Ser Arg Gln Leu Asp Glu Lys Asp Ser Leu Val Ser
245 250 255

Gln Leu Ser Arg Gly Lys Gln Ala Phe Thr Gln Gln Ile Glu Glu Leu
260 265 270

Lys Arg Gln Leu Glu Glu Glu Val Lys Ala Lys Ser Ala Leu Ala His
275 280 285

Ala Leu Gln Ser Ser Arg His Asp Cys Asp Leu Leu Arg Glu Gln Tyr
290 295 300

Glu Glu Glu Gln Glu Ala Lys Ala Glu Leu Gln Arg Ala Met Ser Lys
305 310 315 320

Ala Asn Ser Glu Val Ala Gln Trp Arg Thr Lys Tyr Glu Thr Asp Ala
325 330 335

Ile Gln Arg Thr Glu Glu Leu Glu Glu Ala Lys Lys Lys Leu Ala Gln
340 345 350

Arg Leu Gln Asp Ala Glu Glu His Val Glu Ala Val Asn Ala Lys Cys
355 360 365

Ala Ser Leu Glu Lys Thr Lys Gln Arg Leu Gln Asn Glu Val Glu Asp
370 375 380

Leu Met Ile Asp Val Glu Arg Thr Asn Ala Ala Cys Ala Ala Leu Asp
385 390 395 400

Lys Lys Gln Arg Asn Phe Asp Lys Ile Leu Ala Glu Trp Lys Gln Lys
405 410 415

Tyr Glu Glu Thr His Ala Glu Leu Glu Ala Ser Gln Lys Glu Ser Arg
420 425 430

Ser Leu Ser Thr Glu Leu Phe Lys Ile Lys Asn Ala Tyr Glu Glu Ser
435 440 445

Leu Asp Gln Leu Glu Thr Leu Lys Arg Glu Asn Lys Asn Leu Gln Gln
450 455 460

Glu Ile Ser Asp Leu Thr Glu Gln Ile Ala Glu Gly Gly Lys Arg Ile
465 470 475 480

His Glu Leu Glu Lys Ile Lys Lys Gln Ile Glu Gln Glu Lys Ser Glu
485 490 495

Leu Gln Ala Ala Leu Glu Glu Ala Glu Ala Ser Leu Glu His Glu Glu
500 505 510

Gly Lys Ile Leu Arg Ile Gln Leu Glu Leu Asn Gln Val Lys Ser Glu
515 520 525

Ile Asp Arg Lys Ile Ala Glu Lys Asp Glu Glu Ile Asp Gln Leu Lys
530 535 540

Arg Asn His Ile Arg Val Val Glu Ser Met Gln Ser Thr Leu Asp Ala
545 550 555 560

Glu Ile Arg Ser Arg Asn Asp Ala Ile Arg Ile Lys Lys Lys Met Glu
                565                 570                 575

Gly Asp Leu Asn Glu Met Glu Ile Gln Leu Asn His Ser Asn Arg Met
            580                 585                 590

Ala Ala Glu Ala Leu Arg Asn Tyr Arg Asn Thr Gln Gly Ile Leu Lys
        595                 600                 605

Asp Thr Gln Leu His Leu Asp Asp Ala Leu Arg Gly Gln Glu Asp Leu
    610                 615                 620

Lys Glu Gln Leu Ala Met Val Glu Arg Arg Ala Asn Leu Leu Gln Ala
625                 630                 635                 640

Glu Ile Glu Glu Leu Arg Ala Thr Leu Glu Gln Thr Glu Arg Ser Arg
                645                 650                 655

Lys Ile Ala Glu Gln Glu Leu Leu Asp Ala Ser Glu Arg Val Gln Leu
            660                 665                 670

Leu His Thr Gln Asn Thr Ser Leu Ile Asn Thr Lys Lys Lys Leu Glu
        675                 680                 685

Thr Asp Ile Ser Gln Ile Gln Gly Glu Met Glu Asp Ile Val Gln Glu
    690                 695                 700

Ala Arg Asn Ala Glu Glu Lys Ala Lys Lys Ala Ile Thr Asp Ala Ala
705                 710                 715                 720

Met Met Ala Glu Glu Leu Lys Lys Glu Gln Asp Thr Ser Ala His Leu
                725                 730                 735

Glu Arg Met Lys Lys Asn Leu Glu Gln Thr Val Lys Asp Leu Gln His
            740                 745                 750

Arg Leu Asp Glu Ala Glu Gln Leu Ala Leu Lys Gly Gly Lys Lys Gln
        755                 760                 765

Ile Gln Lys Leu Glu Ala Arg Val Arg Glu Leu Glu Gly Glu Val Glu
    770                 775                 780

Asn Glu Gln Lys Arg Asn Val Glu Ala Ile Lys Gly Leu Arg Lys His
785                 790                 795                 800

Glu Arg Arg Val Lys Glu Leu Thr Tyr Gln Thr Glu Glu Asp Arg Lys
                805                 810                 815

Asn Val Leu Arg Leu Gln Asp Leu Val Asp Lys Leu Gln Ser Lys Val
            820                 825                 830

Lys Ala Tyr Lys Arg Gln Ala Glu Glu Ala Glu Glu Gln Ser Asn Val
        835                 840                 845

Asn Leu Ala Lys Phe Arg Lys Ile Gln His Glu Leu Glu Glu Ala Glu
    850                 855                 860

Glu Arg Ala Asp Ile Ala Glu Ser Gln Val Asn Lys Leu Arg Val Lys
865                 870                 875                 880

Ser Arg Glu Val His Thr Lys Ile Ile Ser Glu Glu

885 890

<210> 103
<211> 2924
<212> DNA
<213> Rattus norvegicus

<220>
<223> Myosin heavy chain

<400> 103
cccccgggct gcaggaattc ggcacgaggg aaactcgagg gggacctaaa actggcccaa 60
gagtccacaa tggacgtaga aaatgacaaa cagcagctcg atgagaaact caaaaagaaa 120
gagtttgaaa tgagcaacct gcagagcaag atcgaggatg aacaggccct cggcatgcag 180
ctgcagaaga agatcaagga gttgcaggct cgcattgagg agctggagga ggaaatcgag 240
gcagagcggg cctccagggc caaagcagag aagcagcgct ctgacctctc ccgggaactg 300
gaggagatca gcgagcggct ggaagaagcc ggcggggcca cttcagccca gatcgagatg 360
aacaagaaga gagaggccga gttccagaaa atgcgcaggg acctggagga ggccaccctg 420
cagcacgaag ccacagcagc caccctgagg aagaagcacg cggacagcgt ggctgagctc 480
ggggagcaga tcgacaacct gcagcgggtg aagcagaagc tggagaagga gaagagcgag 540
atgaagatgg agatcgacga cctcgctagc aacatggagg tcatttccaa atccaaggga 600
aaccttgaga agatgtgccg cactctggag gaccaggtga gcgagctgaa gaccaaggag 660
gaggagcagc agcggctcat caatgagctg accgcgcaga gggggcgtct gcagacagag 720
tcaggtgaat attcacgcca gctggatgaa aaggattcat tagtttctca gctctccagg 780
ggcaaacagg cattcactca acagattgag gagctgaaga ggcagcttga agaggaagta 840
aaggccaaga gtgccctggc tcatgctctt cagtcctccc gccatgactg tgaccttctg 900
cgggaacagt atgaagaaga acaggaagcc aaggctgaac tgcagagggc catgtccaag 960
gccaacagtg aagtggccca gtggaggacg aaatatgaga cggatgccat ccagcgcacg 1020
gaagaactgg aggaggccaa gaagaagctg gctcagcgtc tgcaggatgc tgaggagcac 1080
gtagaagccg tgaacgccaa gtgtgcttcc ctggagaaga cgaagcagag gctgcagaat 1140
gaggtggagg acctcatgat tgatgtggag aggaccaacg ctgcctgtgc cgccctggac 1200
aagaagcaga gaaacttcga caagatcctg gcagaatgga aacagaagta tgaggaaacg 1260
catgctgaac ttgaagcctc ccagaaggag tcccgctctc tcagcacaga gttgttcaag 1320
attaagaatg cttatgagga atctttagac caacttgaaa ccttgaaaag ggaaaataag 1380
aatttgcaac aggagatctc tgacctcact gaacagattg cagaaggagg gaaacgtatc 1440
catgaactgg aaaaaataaa gaagcaaatt gagcaagaaa aatccgagct ccaggctgct 1500
ttagaggaag cagaggcatc tctggagcac gaggagggaa agatcctgcg catccagctg 1560
gagctgaacc aagtcaagtc tgagatcgac aggaagattg ctgagaagga tgaggaaatt 1620
gaccagctga agagaaacca cattcgagtc gtggagtcca tgcagagcac gctggatgcc 1680
gagatcagga gcaggaatga cgccatcagg atcaagaaga agatggaggg agacctcaat 1740
gagatggaaa tccagctgaa ccactccaac cgcatggctg ctgaggccct gagaaactac 1800
aggaacaccc aaggcatcct caaggacacc cagttgcacc tggatgacgc tctccggggc 1860
caggaggacc tgaaggagca gctggccatg gtggagcgca gagccaacct gctgcaggct 1920
gagatcgagg agctgcgggc cactctggag cagactgaga ggagcaggaa gattgcagag 1980
caggagctgc tggacgccag tgagcgcgtg cagctcctcc acacccagaa caccagcctc 2040
atcaacacca agaagaagct ggagacagac atttcccaga tccagggaga gatggaggac 2100
atcgtccagg aagcccgcaa tgcagaagag aaggccaaga aagccatcac tgacgccgcc 2160
atgatggcgg aggagctgaa gaaggagcag gacaccagcg cccatctgga gcggatgaag 2220
aagaacctgg agcagacggt gaaggacctg cagcaccgtc tggacgaggc tgagcagctg 2280
gcgctgaagg gcggcaagaa gcagatccag aaactggagg ccagggtccg tgaacttgaa 2340
ggagaagttg aaaacgaaca gaagcgcaat gttgaagcta tcaagggtct gcgcaaacat 2400
gagagaagag tgaaagaact cacttaccaa actgaggaag accgcaagaa cgttctcagg 2460
ctccaggacc ttgtggacaa actgcaatca aaggtcaaag catacaagag acaagccgag 2520
gaagcggagg aacaatccaa cgtcaacctg gccaagttcc gcaagatcca gcacgagctg 2580
gaggaagccg aggagcgggc tgacatcgcc gagtcccagg tcaacaagct gcgggtgaag 2640
agccgcgagg ttcacaccaa aatcataagc gaagagtgat cgatccaaag caggaaagtg 2700
accaaagaga tgagcaaaat gtgaagatct ttgtcactcc attttgtact tacgactttg 2760
ggagataaaa aatttatctg ccaaaaaaaa aaaaaaaaaa aaaaaaaaaa aaaaaaaaaa 2820
aaaaaaaaaa aaaaaaaaaa aaaaaaaaaa aaaaaaaaaa aaaaaaaaaa aaaaaaaaaa 2880
aaaaaaaaaa aaaaaaaaaa aaaaaactcg aggggggccc cggt 2924

<210> 104
<211> 428
<212> PRT
<213> Rattus norvegicus

<220>
<223> KIAA 1157

<400> 104
Gly Lys Ser Thr His Asn Glu Asp Gln Ala Ser Cys Glu Val Leu Thr
1               5                   10                  15

Val Lys Lys Lys Ala Gly Ala Val Thr Ser Thr Pro Asn Arg Asn Ser
            20                  25                  30

Ser Lys Arg Arg Ser Ser Leu Pro Asn Gly Glu Gly Leu Gln Leu Lys
        35                  40                  45

Glu Asn Ser Glu Ser Glu Gly Val Ser Cys His Tyr Trp Ser Leu Phe
    50                  55                  60

Asp Gly His Ala Gly Ser Gly Ala Ala Val Val Ala Ser Arg Leu Leu
65                  70                  75                  80

Gln His His Ile Thr Glu Gln Leu Gln Asp Ile Val Asp Ile Leu Lys
                85                  90                  95

Asn Ser Ala Val Leu Pro Pro Thr Cys Leu Gly Glu Glu Pro Glu Asn
            100                 105                 110

Thr Pro Ala Asn Ser Arg Thr Leu Thr Arg Ala Ala Ser Leu Arg Gly
        115                 120                 125

Gly Val Gly Ala Pro Gly Ser Pro Ser Thr Pro Pro Thr Arg Phe Phe
    130                 135                 140

Thr Glu Lys Lys Ile Pro His Glu Cys Leu Val Ile Gly Ala Leu Glu
145                 150                 155                 160

Ser Ala Phe Lys Glu Met Asp Leu Gln Ile Glu Arg Glu Arg Ser Ser
                165                 170                 175

Tyr Asn Ile Ser Gly Gly Cys Thr Ala Leu Ile Val Ile Cys Leu Leu
            180                 185                 190

Gly Lys Leu Tyr Val Ala Asn Ala Gly Asp Ser Arg Ala Ile Ile Ile
        195                 200                 205

Arg Asn Gly Glu Ile Ile Pro Met Ser Ser Glu Phe Thr Pro Glu Thr
    210                 215                 220

Glu Arg Gln Arg Leu Gln Tyr Leu Ala Phe Met Gln Pro His Leu Leu
225                 230                 235                 240

Gly Asn Glu Phe Thr His Leu Glu Phe Pro Arg Arg Val Gln Arg Lys
                245                 250                 255

Glu Leu Gly Lys Lys Met Leu Tyr Arg Asp Phe Asn Met Thr Gly Trp
            260                 265                 270

Ala Tyr Lys Thr Ile Glu Asp Glu Asp Leu Lys Phe Pro Leu Ile Tyr
        275                 280                 285

Gly Glu Gly Lys Lys Ala Arg Val Met Ala Thr Ile Gly Val Thr Arg
    290                 295                 300

Gly Leu Gly Asp His Asp Leu Lys Val His Asp Ser Asn Ile Tyr Ile
305                 310                 315                 320

Lys Pro Phe Leu Ser Ser Ala Pro Glu Val Arg Ile Tyr Asp Leu Ser
                325                 330                 335

Lys Tyr Asp His Gly Ser Asp Asp Val Leu Ile Leu Ala Thr Asp Gly
            340                 345                 350

Leu Trp Asp Val Leu Ser Asn Glu Glu Val Ala Glu Ala Ile Thr Gln
        355                 360                 365

Phe Leu Pro Asn Cys Asp Pro Asp Asp Pro His Arg Tyr Thr Leu Ala
    370                 375                 380

Ala Gln Asp Leu Val Met Arg Ala Arg Gly Val Leu Lys Asp Arg Gly
385                 390                 395                 400

Trp Arg Ile Ser Asn Asp Arg Leu Gly Ser Gly Asp Asp Ile Ser Val
                405                 410                 415

Tyr Val Ile Pro Leu Ile His Gly Asn Lys Leu Ser
            420                 425

<210> 105
<211> 5791
<212> DNA
<213> Rattus norvegicus

<220>
<223> KIAA 1157

<400> 105
gggaagagca cacacaatga agaccaagcc agctgtgagg tgctcactgt gaagaagaag 60
gcaggggccg tgacctcaac cccaaacagg aactcatcca agagacggtc ctcccttccc 120
aatggggaag ggctgcagct gaaggagaac tcggaatccg agggtgtttc ctgccactat 180
tggtcgctgt ttgacgggca cgcggggtcc ggggccgcgg tggtggcgtc acgcctgctg 240
cagcaccaca tcacggagca gctgcaggac atcgtggaca tcctgaagaa ctccgccgtc 300
ctgcccccta cctgcctggg ggaggagcct gagaacacgc ccgccaacag ccggactctg 360
acccgggcag cctccctgcg cggaggggtg ggggccccgg gctcccccag cacgcccccc 420
acacgcttct ttaccgagaa gaagattccc catgagtgcc tggtcatcgg agcgcttgaa 480
agtgcattca aggaaatgga cctacagata gaacgagaga ggagttcata taatatatct 540
ggtggctgca cggccctcat tgtgatttgc cttttgggga agctgtatgt tgcaaatgct 600
ggggatagca gggccataat catcagaaat ggagaaatta tccccatgtc ttcagaattt 660
acccccgaga cggagcgcca gcgacttcag tacctggcat tcatgcagcc tcacttgctg 720
ggaaatgagt tcacacattt ggagtttcca aggagagtac agagaaagga gcttggaaag 780
aagatgctct acagggactt taatatgaca ggctgggcat acaaaaccat tgaggatgag 840
gacttgaagt tcccccttat atatggagaa ggcaagaagg cccgggtaat ggcaactatt 900
ggagtgacca ggggacttgg ggaccatgac ctgaaggtgc atgactccaa catctacatt 960
aaaccattcc tgtcttcagc tccagaggta agaatctacg atctttcaaa atatgatcat 1020
ggatcagatg atgtgctgat cttggccact gatggactct gggacgtttt atcaaatgaa 1080
gaagtagcag aagcaatcac tcagtttctt cctaactgtg atccagatga tcctcacagg 1140
tacacactgg cagctcagga cctggtgatg cgtgcccggg gtgtgctgaa ggacagagga 1200

tggcggatat ctaatgaccg actgggctca ggagacgaca tttctgtata tgtcattcct 1260
ttaatacatg gaaacaagct gtcatgaaaa tggcccaggg gattgggagg acagagggga 1320
agaaagctgg gatgcctctt ggcaggacgg aactgggaag tgccccagct gagttccaag 1380
tgatgcagtc tcttcccagc ccaagcgggg agttcatggc caaaagacta tgcttcaaga 1440
tgaccctttg gtttccattt cttctttagt aacaggtcaa ctcaacaaga gcaaaacaca 1500
aaggctgcta ccaagtgttg ttgtatttca gttcctttca taggcctccg aggtggccat 1560
tgactatttg gggtatatat gtcatattta ttttatctag agtagctggg gcagccattt 1620
tcaggtgtaa atggcagagg actcttcagc ctgtcaagct gccagcttat ctacgggtta 1680
aaaagtgctg cattggaaag tagggggtca tgcctcaaaa tgtaagtaag tgcccacctt 1740
ctaggaagcc tgaggtttat ttcagggatt gccgtctgcc ccccgccccc cttctctttt 1800
tttcttctct gtttctattc ttttatggca gtggtggagt gaggcaggga tttttttttt 1860
ttttttttcg tgtttttgac attccttgaa tctgtttttt attccccttc cacagaacag 1920
gcctgggact ttccaacacc ctgctaagga agttctgtgt ccaagtccca cccaggctgg 1980
gttgtcccca cctcctccag cccacacagc ccaggcagca tccgggccag tgccctgcat 2040
gacagagggt ctttgttgtg taatgtttgt tcccaagttg cattttctaa ccgaatcagt 2100
gtgttttcat gaaactgagt gtttctgtgg accagtagtt cctctgttgt cttcagtggt 2160
cttcctgtgt ggctcaaggg ttctctgtga gagtctggat tttcatttct ggaatggctg 2220
gccccatccc acttttctgt atcatgggga cacatataaa gcagtgttta atagagcagt 2280
ttaagaagtt gcttgcatct gttggttcac catggctcat ctggggacca ttttggattc 2340
atgtttcatg gcttgtgact gtccccaagc ccactccaaa caaagtgtaa ggatcagagt 2400
tctgtcaagg agcagcagtt ctgctctccc catcatcttt gtgcaaggcc cctcgggggg 2460
cactttaata aaagaatttg aaatgggttg actggccatt ctcatgctgt gctccctgtc 2520
tcttctcttc tctaaagaat catgtcccag ctcctcaagg tccctctatg gttccacatc 2580
tgagtgttcg ccacaagagc agcagcagca ggcacagtgc atgccatatc tacctgctgc 2640
ttctctgctg ggaggaatgg ccaagtagat tataaaactc acttctgtct cttaggcaga 2700
cttgtacggc cacaaaatta cctagtcttc ttcctgctga gctactgagg tattgccacc 2760
attttgacaa ctttgagtaa ttaaaacact cttctgaccc aaaaaggaaa aaaggtcact 2820
gacgtgaccc ccccagcatg ctagagagct aattccagtt ctcatatttg tttgaatttc 2880
ttcccagagg agaggatagg aacctctcct ccagggcagt aaatcacctg catttctgga 2940
gttgtcggta ttgtattcga aaaggcctgg agcccctcct gctcaggaaa gaactcattc 3000
cagggtgtgg agacagtgcc gtctggcagg tgaaatactg tgggaattca cgccaccagg 3060
tgtttgtgca agtgttggcc tgggaagaat gggacttcgg ccttgtcagg agttgtcttc 3120
atctgcagca cgtttcttcc tcctgcagta gatcttagct accccagata tctctatgga 3180
gagaagtttg tggaaaatgc tttgcttcgt ggcagagtct gatgctgtag gaaaaccttc 3240
gggcatgtga cagcagtgtg gtccactccc tgttctgccc tggcgctcag agtcatgtgt 3300
aagtaggaaa cctgagcaag tcttccgtgg aggaccctga gctgccgtct ttgggatcct 3360
tcctgtgtcc ccaccgtctt tcatttattt gctttcctgg gcctctatct gggccctacc 3420
ttgagcttct ccagttttat tcaagccacc agagtaagaa tttgggtgta gatgtcacaa 3480
ctaccttcta ctcaattcac caattcattt actgctatgg cacgtctcag gaataactct 3540
agaaacctct aaatcgaaat attataaaat cttgagcact tagtcctgct ggttttagtt 3600
agaaaggcat ccaggaattg ttttcctacg cccccttgag tggaaagatc ttagttagaa 3660
gataaagtca agtttgtgtt caggggatgg gaggaagact ataaataaga tgaagaaatc 3720
aaaagtagga aacatgatgt aaacgaagca tggcagatct gtccagcact gatattgctc 3780
tataaattga gcttactcag ttttggcctt atttttttac ccaggcccca tgtcacccag 3840
tcctaaaaca gtaaccgtgt ctacataacg ggttggcccc tggtgcatcc ctggaaaagt 3900
caaaggacgc acacttcgaa attctgcaga acgtatttat acatggttca gaaatcttgc 3960
gtatctgact tatagccaaa tctgcttgct cgaatagcct cagaggaagt cttgtttaat 4020
aaaaaccttt tgatttccta gtcaagtctt tatggttgtc tcgaggggtg tgtggctact 4080
ttaatgaaag gctttcctgc tctaaatctc tttgctgggc tgggcctctt cagactatct 4140
ggtgaaactc ctttccttag aacaaactca gtccgtccat gctctgtggc attttgctag 4200
atgataacca aagccttatt cctgtagcca gtgtcagcag tcagagaggt ggagggtgtg 4260
ttctgctgtg gttatgcata cctatctgct gttcttgagg tgtaaaagga aaggtgaaaa 4320
tcgggccagg ccaagtactc agctgtctta ataggatgaa gccttaagca gtggaaattt 4380
cagttatttt ccacagtatt ccattttgga ggatttgggg tgtttacttt ttaaattctt 4440
gaacaactta acctccatga ggctttgtga agtcagctgt gaccaccctc ctcttactgt 4500
gttctcagta ttcattcact tccagggaag aatgacagcc acagggagat ggtggtgggc 4560
aagaatgaga gtcccaggat ccagatttag cctcagatct tccccattca ggaagggttt 4620
tccatttaac aagagcacta gtatgaaaac attagggaca aatctcccat gtctttgaaa 4680
ttcggattct cctcttgaga tccccttcct cacctgccaa tcaactttat aaggccacaa 4740
gtggtcactg gttttccttc cacaggtttg aggttctcag ctttccttaa gcgacccagc 4800
agctccgctg ttttcagagt gaatatgtta agctttgatg agattctatt ttcagtaagt 4860

tagtgcttct gggacacttg gagaaagctg tgagagtcat tgtctacgca aagaacaacg 4920
aagctgatcc taaaagtgat ccaatctaag aaaatggtaa aacgagctct ggccacagca 4980
cagaatttta tgtgaggaac tcagattttt gaagacttaa caattgcaga gaaaggttgc 5040
agcctgcaca ccatagccca cctctctgag cagactttgg ttttgtgtgg tgacgtggca 5100
catgtttgta cactgggatt tttcaaagga cgctacgcga gcagactgac ttgcctcttc 5160
tgtgagcact gtggcttttg tcagatggag tgccggtctg cagaggactg ctctttcgaa 5220
tccacagtgt tatctgtgta aatagcttta atttttcttc tgtgtcttag gtgaagtttt 5280
gttcatgtag caaccaggta gacagtgacc aaataaggct gtaaatgtgc tgtagttttc 5340
tactgtgatg tacttgaagg agaacctgtg tcctctactt ttctgatctc ccacaagtat 5400
tttgtgtttg tttcctgagt cctgaggtta ttattttact cctgttttgc ccccagtttt 5460
ctttgttttt tttctggaga cccagggagg cccatggtgg agatcatttg taaggaatgg 5520
atcatggtct gggtttccaa aactacccta gtacagtgaa tgagagaaat ctgcctggaa 5580
attgtttcag aaccatgtac ctttatgctt tgtgattgtg aaacattgac ttttttgtaa 5640
ccccaaaatg aaaactgttt agtaaagggg atctattttg tgtgttttga aacttaggtg 5700
caatgtcccc tggaaaaagc taaagaaatg tatatgttca atgacatttt aaaataaaat 5760
attatatata tgtatatacg acatattcag c 5791

<210> 106
<211> 1253
<212> PRT
<213> Mus musculus

<220>
<223> SHYC

<400> 106
Met Ala Ala Gln Val Thr Leu Glu Asp Ala Leu Ser Asn Val Asp Leu
1               5                   10                  15

Leu Glu Glu Leu Pro Leu Pro Asp Gln Gln Pro Cys Ile Glu Pro Pro
            20                  25                  30

Pro Ser Ser Leu Leu Tyr Gln Pro Asn Phe Asn Thr Asn Phe Glu Asp
        35                  40                  45

Arg Asn Ala Phe Val Thr Gly Ile Ala Arg Tyr Ile Glu Gln Ala Thr
    50                  55                  60

Val His Ser Ser Met Asn Glu Met Leu Glu Glu Gly Gln Glu Tyr Ala
65                  70                  75                  80

Val Met Leu Tyr Thr Trp Arg Ser Cys Ser Arg Ala Ile Pro Gln Val
                85                  90                  95

Lys Cys Asn Glu Gln Pro Asn Arg Val Glu Ile Tyr Glu Lys Thr Val
            100                 105                 110

Glu Val Leu Glu Pro Glu Val Thr Lys Leu Met Asn Phe Met Tyr Phe
        115                 120                 125

Gln Arg Asn Ala Ile Glu Arg Phe Cys Gly Glu Val Arg Arg Leu Cys
    130                 135                 140

His Ala Glu Arg Arg Lys Asp Phe Val Ser Glu Ala Tyr Leu Ile Thr
145                 150                 155                 160

Leu Gly Lys Phe Ile Asn Met Phe Gly Val Leu Asp Glu Leu Lys Asn
                165                 170                 175

Met Lys Cys Ser Val Lys Asn Asp His Ser Ala Tyr Lys Arg Ala Ala

180                 185                 190

Gln Phe Leu Arg Lys Met Ala Asp Pro Gln Ser Ile Gln Glu Ser Gln
        195                 200                 205

Asn Leu Ser Met Phe Leu Ala Asn His Asn Lys Ile Thr Gln Ser Leu
    210                 215                 220

Gln Gln Gln Leu Glu Val Ile Ser Gly Tyr Glu Glu Leu Leu Ala Asp
225                 230                 235                 240

Ile Val Asn Leu Cys Val Asp Tyr Tyr Glu Asn Arg Met Tyr Leu Thr
                245                 250                 255

Pro Ser Glu Lys His Met Leu Leu Lys Val Met Gly Phe Gly Leu Tyr
            260                 265                 270

Leu Met Asp Gly Ser Val Ser Asn Ile Tyr Lys Leu Asp Ala Lys Lys
        275                 280                 285

Arg Ile Asn Leu Ser Lys Ile Asp Lys Tyr Phe Lys Gln Leu Gln Val
    290                 295                 300

Val Pro Leu Phe Gly Asp Met Gln Ile Glu Leu Ala Arg Tyr Ile Lys
305                 310                 315                 320

Thr Ser Ala His Tyr Glu Glu Asn Lys Ser Arg Trp Thr Cys Ala Ser
                325                 330                 335

Ser Ser Ser Ser Pro Gln Tyr Asn Ile Cys Glu Gln Met Ile Gln Ile
            340                 345                 350

Arg Glu Asp His Met Arg Phe Ile Ser Glu Leu Ala Arg Tyr Ser Asn
        355                 360                 365

Ser Glu Val Val Thr Gly Ser Gly Arg Gln Glu Ala Gln Lys Thr Asp
    370                 375                 380

Ala Glu Tyr Arg Lys Leu Phe Asp Leu Ala Leu Gln Gly Leu Gln Leu
385                 390                 395                 400

Leu Ser Gln Trp Ser Ala His Ala Met Glu Val Tyr Ser Trp Lys Leu
                405                 410                 415

Val His Pro Thr Asp Lys Tyr Ser Asn Lys Asp Cys Pro Asp Asn Ala
            420                 425                 430

Glu Glu Tyr Glu Arg Ala Thr Arg Tyr Asn Tyr Thr Thr Glu Glu Lys
        435                 440                 445

Phe Ala Leu Val Glu Val Ile Ala Met Ile Lys Gly Leu Gln Val Leu
    450                 455                 460

Met Gly Arg Met Glu Ser Val Phe Asn His Ala Ile Arg His Thr Val
465                 470                 475                 480

Tyr Ala Ala Leu Gln Asp Phe Ser Gln Val Thr Leu Arg Glu Pro Leu
                485                 490                 495

Arg Gln Ala Ile Lys Lys Lys Lys Asn Val Ile Gln Ser Val Leu Gln
            500                 505                 510

Ala Ile Arg Lys Thr Val Cys Asp Trp Glu Thr Gly His Glu Pro Phe
        515                 520                 525

Asn Asp Pro Ala Leu Arg Gly Glu Lys Asp Pro Lys Ser Gly Phe Asp
    530                 535                 540

Ile Lys Val Pro Arg Arg Ala Val Gly Pro Ser Ser Thr Gln Leu Tyr
545                 550                 555                 560

Met Val Arg Thr Met Leu Glu Ser Leu Ile Ala Asp Lys Ser Gly Ser
                565                 570                 575

Lys Lys Thr Leu Arg Ser Ser Leu Glu Gly Pro Thr Ile Leu Asp Ile
            580                 585                 590

Glu Lys Phe His Arg Glu Ser Phe Phe Tyr Thr His Leu Ile Asn Phe
        595                 600                 605

Ser Glu Thr Leu Gln Gln Cys Cys Asp Leu Ser Gln Leu Trp Phe Arg
    610                 615                 620

Glu Phe Phe Leu Glu Leu Thr Met Gly Arg Arg Ile Gln Phe Pro Ile
625                 630                 635                 640

Glu Met Ser Met Pro Trp Ile Leu Thr Asp His Ile Leu Glu Thr Lys
                645                 650                 655

Glu Ala Ser Met Met Glu Tyr Val Leu Tyr Ser Leu Asp Leu Tyr Asn
            660                 665                 670

Asp Ser Ala His Tyr Ala Leu Thr Lys Phe Asn Lys Gln Phe Leu Tyr
        675                 680                 685

Asp Glu Ile Glu Ala Glu Val Asn Leu Cys Phe Asp Gln Phe Val Tyr
    690                 695                 700

Lys Leu Ala Asp Gln Ile Phe Ala Tyr Tyr Lys Val Met Ala Gly Ser
705                 710                 715                 720

Leu Leu Leu Asp Lys Arg Leu Arg Ser Glu Cys Lys Asn Gln Gly Ala
                725                 730                 735

Thr Ile His Leu Pro Pro Ser Asn Arg Tyr Glu Thr Leu Leu Lys Gln
            740                 745                 750

Arg His Val Gln Leu Leu Gly Arg Ser Ile Asp Leu Asn Arg Leu Ile
        755                 760                 765

Thr Gln Arg Val Ser Ala Ala Met Tyr Lys Ser Leu Glu Leu Ala Ile
    770                 775                 780

Gly Arg Phe Glu Ser Glu Asp Leu Thr Ser Val Val Glu Leu Asp Gly
785                 790                 795                 800

Leu Leu Glu Ile Asn Arg Met Thr His Lys Leu Leu Ser Arg Tyr Leu
                805                 810                 815

Thr Leu Asp Ser Phe Asp Ala Met Phe Arg Glu Ala Asn His Asn Val
            820                 825                 830

Ser Ala Pro Tyr Gly Arg Ile Thr Leu His Val Phe Trp Glu Leu Asn
        835                 840                 845

Tyr Asp Phe Leu Pro Asn Tyr Cys Tyr Asn Gly Ser Thr Asn Arg Phe
    850                 855                 860

Val Arg Thr Val Leu Pro Phe Ser Gln Glu Phe Gln Arg Asp Lys Gln
865                 870                 875                 880

Pro Asn Ala Gln Pro Gln Tyr Leu His Gly Ser Lys Ala Leu Asn Leu
                885                 890                 895

Ala Tyr Ser Ser Ile Tyr Gly Ser Tyr Arg Asn Phe Val Gly Pro Pro
            900                 905                 910

His Phe Gln Val Ile Cys Arg Leu Leu Gly Tyr Gln Gly Ile Ala Val
        915                 920                 925

Val Met Glu Glu Leu Leu Lys Val Val Lys Ser Leu Leu Gln Gly Thr
    930                 935                 940

Ile Leu Gln Tyr Val Lys Thr Leu Met Glu Val Met Pro Lys Ile Cys
945                 950                 955                 960

Arg Leu Pro Arg His Glu Tyr Gly Ser Pro Gly Ile Leu Glu Phe Phe
                965                 970                 975

His His Gln Leu Lys Asp Ile Ala Glu Tyr Ala Glu Leu Lys Thr Val
            980                 985                 990

Cys Phe Gln Asn Leu Arg Glu Val Gly Asn Ala Val Leu Phe Cys Leu
        995                 1000                1005

Leu Ile Glu Gln Ser Leu Ser Leu Glu Glu Val Cys Asp Leu Leu His
    1010                1015                1020

Ala Ala Pro Phe Gln Asn Ile Leu Pro Arg Ile His Val Lys Glu Gly
1025                1030                1035                1040

Glu Arg Val Asp Ala Lys Met Lys Arg Leu Glu Ser Lys Tyr Ala Pro
                1045                1050                1055

Leu His Leu Val Pro Leu Ile Glu Arg Leu Gly Thr Pro Gln Gln Ile
            1060                1065                1070

Ala Ile Ala Arg Glu Gly His Leu Leu Thr Lys Glu Arg Leu Cys Cys
        1075                1080                1085

Gly Leu Ser Met Phe Glu Val Ile Leu Thr Arg Ile Arg Thr Phe Leu
    1090                1095                1100

Asp Asp Pro Ile Trp Arg Gly Pro Leu Pro Ser Asn Gly Val Met His
1105                1110                1115                1120

Val Asp Glu Cys Val Glu Phe His Arg Leu Trp Ser Ala Met Gln Phe
                1125                1130                1135

Val Tyr Cys Ile Pro Val Gly Thr His Glu Phe Thr Val Glu Gln Cys
            1140                1145                1150

Phe Gly Asp Gly Leu His Trp Ala Gly Cys Met Ile Ile Val Leu Leu

1155                1160                1165

Gly Gln Gln Arg Arg Phe Ala Val Leu Asp Phe Cys Tyr His Leu Leu
    1170                1175                1180

Lys Val Gln Lys His Asp Gly Lys Asp Glu Ile Ile Lys Asn Val Pro
1185                1190                1195                1200

Leu Lys Lys Met Val Glu Arg Ile Arg Lys Phe Gln Ile Leu Asn Asp
                1205                1210                1215

Glu Ile Ile Thr Ile Leu Asp Lys Tyr Leu Lys Ser Gly Asp Gly Glu
            1220                1225                1230

Thr Thr Pro Val Glu His Val Arg Cys Phe Gln Pro Pro Ile His Gln
        1235                1240                1245

Ser Leu Ala Ser Ser
    1250

<210> 107
<211> 4112
<212> DNA
<213> Mus musculus

<220>
<223> SHYC

<400> 107
ccgccctgga caaccgaagc ggcgcagacc aggatggctg cccaagtgac actagaggac 60
gcactgtcca atgtggacct cctggaggag ctgcctctgc ctgaccagca gccctgcatc 120
gagcccccac cgtcctcgct gctctatcag ccaaatttca acaccaactt tgaagacaga 180
aatgcatttg tcactggcat tgcaagatac atcgaacaag caactgtcca ctctagcatg 240
aacgagatgc tagaggaagg ccaagagtat gctgtcatgc tgtacacctg gaggagctgc 300
tcccgggcca tccctcaggt gaagtgcaac gagcagccga atagagttga aatttatgag 360
aaaaccgtgg aagtccttga acccgaggtc acaaaactga tgaattttat gtactttcag 420
agaaatgcca tcgagcggtt ctgtggggaa gtgagacgcc tgtgtcatgc tgagaggaga 480
aaggactttg tttctgaagc ctacttgatc accctgggca aatttatcaa catgtttggt 540
gtgttggatg agctgaagaa catgaagtgc agtgtgaaga atgaccactc tgcatataag 600
agggctgctc agtttttacg taaaatggca gatccacaat ccatccagga gtcacagaat 660
ctgtccatgt tcctggccaa ccacaacaag attacacaat ctctgcagca gcagcttgag 720
gtcatttctg gctatgaaga gctcctggca gacatcgtga atctgtgtgt ggattactat 780
gagaacagga tgtacttgac acccagcgaa aaacacatgc ttctcaaagt catgggattt 840
ggcctgtacc tgatggatgg gagtgtgagt aacatctaca aactagatgc caagaaaaga 900
ataaatttat ccaaaatcga caagtatttc aagcagctgc aggtggttcc actctttgga 960
gatatgcaaa tagaactggc aagatacatc aagaccagcg cacactatga ggagaataag 1020
tcccggtgga cctgcgcgtc ctccagcagc agcccgcagt acaacatctg tgagcagatg 1080
atccagatcc gtgaggacca tatgcgtttc atctccgagc tggcacgcta cagcaatagc 1140
gaggtagtca cagggtctgg ccggcaggaa gcccagaaga cagatgctga gtaccggaag 1200
ctcttcgatc tggctctgca gggcctgcag cttctgtcgc agtggagcgc tcacgcgatg 1260
gaagtgtatt cctggaaact tgtacatcca acagacaagt actccaacaa ggactgcccc 1320
gacaacgctg aggagtacga gcgagccaca cgctacaact acaccactga ggagaagttt 1380
gccctggtgg aggtgattgc catgatcaaa ggcctgcagg tgctgatggg caggatggag 1440
agtgtgttca atcacgccat cagacacacg gtttacgctg cgcttcagga cttctcccag 1500
gtgaccctca gggaaccact gcgacaggcc atcaagaaga aaaagaacgt aatccagagt 1560
gtcctacagg ccatcaggaa gactgtgtgt gactgggaga ctgggcatga gccctttaac 1620
gacccagctc tgcggggaga gaaggaccct aagagtggct ttgacatcaa ggtgccacgc 1680
cgtgctgtgg gaccctccag cacgcagctt tacatggtga gaactatgct agagtccctc 1740
attgcagaca aaagtggttc caagaaaacc ttgagaagta gccttgaggg gcccaccata 1800
ttggacatag aaaaatttca tcgagaatca ttcttctaca ctcacttgat aaatttcagt 1860

gaaacactgc agcagtgctg tgacctttcc cagctgtggt tccgagagtt cttcctggag 1920
ctgaccatgg gcaggaggat ccagttcccc attgagatgt ctatgccctg gattctgact 1980
gaccacatcc tagagaccaa ggaggcatca atgatggagt acgtcctcta ctccttggac 2040
ctgtacaacg acagtgccca ctatgcgctc accaagttca acaagcagtt tctctatgat 2100
gagattgagg cagaggtgaa tctgtgtttt gaccaatttg tttataaact agcagaccag 2160
atatttgcat attacaaggt tatggcagga agtttgcttc ttgacaaaag gttacggtca 2220
gaatgcaaaa atcagggagc cacgatccac ctccccccat ctaaccgcta tgagacgctg 2280
ttgaagcaaa ggcacgtgca gctgctcggg agatccattg atctcaaccg tctgattaca 2340
cagcgcgttt cagcagccat gtacaaatct ctagaactgg caattggacg gtttgaaagt 2400
gaagatttaa catcagtagt cgagctagat ggactcttag aaatcaaccg tatgacacac 2460
aagctgttga gcaggtacct cacactggac agctttgatg ccatgttccg ggaagccaac 2520
cacaacgtgt ctgcacctta tggaagaatt accctccacg tcttctggga gctaaactat 2580
gacttcttgc ccaactactg ctataatggc tctaccaacc ggtttgttcg gacagtatta 2640
ccattttctc aggaatttca aagagacaaa cagcctaacg cacagcccca gtatttgcat 2700
ggatccaagg ctttgaacct agcctactca agcatttatg gcagctaccg gaactttgtg 2760
gggcctccgc acttccaagt catctgccgg ctacttggct accagggcat tgcagtagtc 2820
atggaagagc tgctgaaggt tgtcaagagc ctgctgcaag gcacaatcct gcagtacgtg 2880
aaaactctga tggaagtgat gcccaagatc tgccggcttc cgcgacatga gtacggctct 2940
cctggcatcc tggagttctt ccaccaccag ctgaaggaca ttgcggagta tgcagagctg 3000
aaaaccgtat gcttccagaa cctgcgggag gtgggcaatg ctgttctctt ctgcctgctt 3060
attgagcaaa gcctgtcttt agaagaagtc tgtgacctgt tgcatgcagc tcctttccag 3120
aatatcttac ctcgaatcca tgtaaaagag ggggagagag ttgatgccaa aatgaaaaga 3180
ctagaatcca agtatgcccc actgcacctt gtcccactga ttgaaaggct gggcacccca 3240
cagcaaattg caattgcaag agaggggcac ttgctaacca aggagcgtct ctgttgtggt 3300
ctgtccatgt ttgaagtcat cctgacacgg atccggacct ttctggatga tcccatctgg 3360
cgtgggcccc tacccagcaa tggtgtcatg cacgtggatg agtgtgtgga gtttcacaga 3420
ctatggagtg ccatgcagtt tgtttactgc attcctgtag ggacacacga gtttacagtg 3480
gagcagtgtt ttggagatgg gctccactgg gctggctgca tgatcattgt acttcttgga 3540
cagcagcggc gctttgctgt gttggatttc tgctatcatc ttctcaaagt tcagaaacat 3600
gatggcaaag atgagatcat caaaaatgtg ccattgaaga agatggtgga gaggatccgc 3660
aagttccaga ttctcaacga tgaaatcatc actatcctgg acaagtactt gaaatctggt 3720
gatggcgaga cgacacctgt ggagcatgta cgctgcttcc agccacccat ccaccagtcc 3780
ctagccagta gctgagggct aagctcacag catcccagta actgatggca tgtttgtctt 3840
tatgtaaact atattgaaat ttttaggggc tattttccat tatgtctgaa cctacttttg 3900
atctgaaagc ttaactttat aaaatttact tatttttcta tactaaaatt gtatatgcct 3960
ttggaattgg gaagtctgag aatacggtta ctagttgttg ccattacggt attaacaagt 4020
attttcatgc cgaatattcc agcagctatt ataatgctaa atcactcatc tgtacgagca 4080
taaccactaa ttgtattaaa aaaaaataaa aa 4112

<210> 108
<211> 3738
<212> DNA
<213> Rattus norvegicus

<220>
<223> MAP-2

<400> 108
tgaatccttc tcatctagca ctgaagtacc atttaggcat gagctcttgg caggagtggg 60
ctctgagcag gtgtcatatt cattctttac aaaccataga taaataatct catcccccaa 120
ctgtagtaat tgttacaatt ttatataaaa aaaaagaata gtatatgcag aaaatgacct 180
gaattccatc cgcaacaaga gcacatggct ttacatggat acaattggac aattattttc 240
gctctgctct gttttgcatg gagtattatt attattatta tttttaaaat tgcattttta 300
cctttcatgt gcctgaaggc tatccaatac attctgaaag gccttgttaa aaatccaagc 360
tgctcatttc cctattctgt ttatggttgg aaagattaaa aactatccat tgtcgcttac 420
tacaggttca aggaaatcaa ggagtctgag tgcaggaagg aaagagcatg taagagacac 480
attttttaaa gtgttatttt gtataaatgg gaagaaagat gcaactaagt tattgatgtt 540
tgggacctgg atgattatat cagagtatgc cgttccaata aattatttaa ctaaaagcta 600
gattaaagca tgtgagcggt gtttgaagaa gtggtgtgga agtgcgtctc ttaggattga 660
tgcactttcg ttaggatggg ctcgagtctg attagaatgt cagttgattg gctagatttg 720

tgtccacaca gtcagtttca tgcccccatt ccatctgttt gatacagtat tatagatata 780
aatatatata tatatttctc tgtggccatt tgtgatactt cctcatatac ttgaatatga 840
tacttcttta ttcacagtat ctgtgtctcc tgcacccttt ggtgttgcaa ttttaggtat 900
gtgaaagtag atgttagcag ggttctctcc ctatttaaaa atacatttta caaagacaaa 960
gagtttgagc atgaagttgc tttctgtaac aactcaaagc tgtaaccctg ttttagtgcc 1020
agaatcaagt ccttcctgtg atgctagaac attttatttt tctttgcttc caccaacatg 1080
gagtttgtgg agtgggtcca gttatgcata aaggggttta cagattgttg gtctatggat 1140
ttatctcatg tttaattact gaatagtttg ggttttatcc ctctaattat tcatgaaacc 1200
aatcccttac tattcatttc ttctctttct cttttccttt ttccatttgc taaagttgaa 1260
agtaggaaaa tagttttgga tatcttttct tgttctccaa atagtatctg ttcattaaat 1320
aattctctga tagaagatat ttggcttcct tctcccaaag acctcttcaa aaatgagaaa 1380
tacaacatat taagaatata tatatatata tatatatata tattctttga gacacagata 1440
tctaaatcca tctctccagg attctacatg aacatggtat gatttttttt attttatata 1500
cagtctcaac atcacaattc attaatttca tttacaaatc atactaagtg aataagcaag 1560
agtcaatggc ccaaaagaag acaaagcatt atcaaaccaa gtggactttt cccttaacat 1620
ttaatttgtt ctttttcttg ttctcaggat tggtagactg aatctgaagg gttaaatgta 1680
aaaagttggc taggtgtttt atgtctttga aacaagccaa gccctgtcta taaaggagac 1740
ggtgaacatg aacaagacag agacgtacag gtgtgtctga gacagaaggc aagtggatgc 1800
gaaggcagag aggaaacagg tggcagttta gtgatccatt gagtattatg gccaatgtga 1860
cagccacatg gaaagcttag catcaaacga agacattctt cacagctagt aggtacacac 1920
acagacacag acacagacac acacacacac acacacacac acacacacac acacacacag 1980
agagagagag agagagagac acacacacac acacagagag agagagagac acacacacac 2040
acagagagag agagagagag acagagagag agagagagac acacacacgt ctttgtaaat 2100
tgttttttta atatataact ttaaaactac catgtaatgg ttgctttcac tctggacaat 2160
ccattcctta cctactcttg aatgcacaag aaatggaaga aggaacgtct gtgtgtggag 2220
aaggccagtc ctacagtttt ggtagacaag gattaaagtt atctaggaaa aagagagtta 2280
atagatgatc cgtgtggttt aaactatgta gaagtaattt caacccagtt tattataaaa 2340
attaaggcta agcaaaggtt tttttaagtg ttcaagttca ttttcttacg atgtgtattc 2400
aaaataaaat tatgtaagcc ccaagttaat agatctagca ctaaaatatc atttttctaa 2460
tgttaataca attataatgg atacaagtcc ttgttctatg tgaaaatgtg attcacacat 2520
gaatgtaaag tcaacacaag aaggacctga attttttgta ccagacagag acagagaaat 2580
gcacaggcta aaattcactt ccttatggga atgtgggatg gatcccacct tacctactta 2640
agataatgac tcaaattaag ctttttggac accacttttg tggggataca catacgctga 2700
tctagaaatg aaaggcgcac agctacattt ctagatccac taatgccagt ttctctttgg 2760
cttcagcctt tgagaacctg ttcaagaata cgtaagtatc cagagctctg aagagttcga 2820
aggccaactt tttcagtgaa ctcacacact ctgggtctcc tgcaactgac aattgggtac 2880
cttgcaacaa tgcgggaagg atccgagttt atgatgagtt tcaaaggccg tgttcactta 2940
ggaactgact ctctctggat ctgcctgctg cgttccagcg agatgacggg ctgaaatccc 3000
acccatagga agacacctgt gcaattccag ctcagtttgg ctgaaggtaa ctaaagaaga 3060
aggtccagta aatggaagaa tagactacag ctggccttct ccacacacac acgcacacac 3120
acgcacgcac gcacacacac agatgtggga tagaagagag atcagcacca aaatgtagca 3180
ttatatcagt agtagtaaag aaataatata aatgtgaatg attctgactt agctgcattt 3240
atagttttct tctcattttt catgccacag gggaaaaaaa ataaattatt tatagtagaa 3300
gtagcaaggc aaaagtagat ttatcatttg gaaaggcaaa tttcaggcat ttcttcaaga 3360
ctggtgtgct aaaattaata ctgttgttgt gtttttacac ctagaagttt tagcctggac 3420
atgtagattg aggccaagtc cctatgtagg aaaaagtttt tttcaaaacc cctaaaaaaa 3480
tgccaatcat gaaagtccac ttcaacttta gcaaaaaaat tcaacaaaaa gtatactctg 3540
tatgctggga tcccaaggtt ccaacacacc atctttgggg gggttctttc ttctgataat 3600
tctagagcct gttaccatag aaaggaattt cttcaatggc tgattgtagt tagttcatgt 3660
tttcaatcca atttgcaaat gtatttgttg ctgtatagtg attgttttgc aaaataaaat 3720
tgcttgtcat ctaactac 3738

<210> 109
<211> 1726
<212> PRT
<213> Rattus norvegicus

<220>
<223> Chromatin structure regulator

<400> 109
Met Ser Asp Phe Val Glu Ser Glu Ala Glu Glu Ser Glu Glu Glu Tyr
1               5                   10                  15

Asn His Glu Gly Glu Val Val Pro Arg Val Thr Lys Lys Phe Val Glu
            20                  25                  30

Glu Glu Asp Asp Asp Glu Glu Glu Glu Glu Glu Asn Leu Asp Asp Gln
        35                  40                  45

Asp Glu Arg Gly Asn Leu Lys Asp Phe Ile Asn Asp Asp Asp Asp Glu
    50                  55                  60

Glu Glu Gly Glu Glu Asp Glu Gly Ser Asp Ser Gly Asp Ser Glu Asp
65                  70                  75                  80

Asp Val Gly His Lys Lys Arg Lys Arg Pro Ser Phe Asp Asp Arg Leu
                85                  90                  95

Glu Asp Asp Asp Phe Asp Leu Ile Glu Glu Asn Leu Gly Val Lys Val
            100                 105                 110

Lys Arg Gly Gln Lys Tyr Arg Arg Val Lys Lys Met Ser Asp Asp Asp
        115                 120                 125

Glu Asp Asp Glu Glu Glu Tyr Gly Lys Glu Glu His Glu Lys Glu Ala
    130                 135                 140

Ile Ala Gly Glu Ile Phe Gln Asp Glu Glu Gly Glu Glu Gly Gln Glu
145                 150                 155                 160

Ala Val Glu Ala Pro Met Ala Pro Pro Asp Glu Glu Glu Glu Asp Asp
                165                 170                 175

Glu Glu Ser Asp Ile Asp Asp Phe Ile Val Asp Asp Asp Gly Gln Pro
            180                 185                 190

Leu Lys Lys Pro Lys Trp Arg Lys Lys Leu Pro Gly Tyr Thr Asp Ala
        195                 200                 205

Ala Leu Gln Glu Ala Gln Glu Ile Phe Gly Val Asp Phe Asp Tyr Asp
    210                 215                 220

Glu Phe Glu Lys Tyr Asn Glu Tyr Asp Glu Glu Leu Glu Glu Asp Tyr
225                 230                 235                 240

Glu Tyr Glu Asp Asp Glu Thr Glu Gly Glu Ile Arg Val Arg Pro Lys
                245                 250                 255

Lys Thr Thr Lys Lys Arg Val Ser Arg Arg Ser Ile Phe Glu Met Tyr
            260                 265                 270

Glu Pro Ser Glu Leu Glu Ser Ser His Leu Thr Asp Gln Asp Asn Glu
        275                 280                 285

Ile Arg Ala Thr Asp Leu Pro Glu Arg Phe Gln Leu Arg Ser Ile Pro
    290                 295                 300

Val Lys Ala Ala Glu Asp Asp Glu Leu Glu Glu Glu Ala Asp Trp Ile
305                 310                 315                 320

Tyr Arg Asn Ala Phe Ala Thr Pro Thr Ile Ser Leu Gln Asp Ser Cys
                325                 330                 335

Asp Tyr Leu Asp Arg Gly Gln Pro Thr Ser Ser Phe Ser Arg Lys Gly
            340                 345                 350

Pro Ser Thr Val Gln Lys Ile Lys Glu Ala Leu Gly Phe Met Arg Asn
        355                 360                 365

Gln His Phe Glu Val Pro Phe Ile Ala Phe Tyr Arg Lys Glu Tyr Val
    370                 375                 380

Glu Pro Glu Leu His Ile Asn Asp Leu Trp Arg Val Trp Gln Trp Asp
385                 390                 395                 400

Glu Lys Trp Thr Gln Leu Arg Ile Arg Lys Glu Asn Leu Thr Arg Leu
                405                 410                 415

Phe Glu Lys Met Gln Ala Tyr Gln Tyr Glu Gln Ile Ser Ala Asp Pro
            420                 425                 430

Asp Lys Pro Leu Ala Asp Gly Ile Arg Ala Leu Asp Thr Thr Asp Met
        435                 440                 445

Glu Arg Leu Lys Asp Val Gln Ser Met Asp Glu Leu Lys Asp Val Tyr
    450                 455                 460

Asn His Phe Leu Leu Tyr Tyr Gly Arg Asp Ile Pro Lys Met Gln Asn
465                 470                 475                 480

Ala Ala Lys Ala Ser Arg Lys Lys Leu Lys Arg Ile Lys Glu Asp Gly
                485                 490                 495

Asp Glu Glu Gly Glu Gly Glu Glu Ala Glu Asp Glu Glu Gln Arg Gly
            500                 505                 510

Pro Glu Leu Lys Gln Ala Ser Arg Arg Asp Met Tyr Thr Ile Cys Gln
        515                 520                 525

Ser Ala Gly Leu Asp Gly Leu Ala Lys Lys Phe Gly Leu Thr Pro Glu
    530                 535                 540

Gln Phe Gly Glu Asn Leu Arg Asp Ser Tyr Gln Arg His Glu Thr Glu
545                 550                 555                 560

Gln Phe Pro Ala Glu Pro Leu Glu Leu Ala Lys Asp Tyr Val Cys Ser
                565                 570                 575

Gln Phe Pro Thr Pro Glu Ala Val Leu Glu Gly Ala Arg Tyr Met Val
            580                 585                 590

Ala Leu Gln Ile Ala Arg Glu Pro Leu Val Arg Gln Val Leu Arg Gln
        595                 600                 605

Ala Phe Gln Glu Arg Ala Lys Leu Asn Ile Thr Pro Thr Lys Lys Gly
    610                 615                 620

Arg Lys Asp Val Asp Glu Ala His Tyr Ala Tyr Ser Phe Lys Tyr Leu
625                 630                 635                 640

Lys Asn Lys Pro Val Lys Glu Leu Arg Asp Asp Gln Phe Leu Lys Ile

                645                 650                 655

Gly Leu Ala Glu Asp Glu Gly Leu Leu Thr Ile Asp Ile Ser Ile Asp
            660                 665                 670

Met Lys Gly Val Glu Gly Tyr Gly Asn Asp Gln Thr Tyr Phe Glu Glu
        675                 680                 685

Ile Lys Gln Phe Tyr Tyr Arg Asp Glu Phe Ser His Gln Val Gln Glu
    690                 695                 700

Trp Asn Arg Gln Arg Thr Met Ala Ile Glu Arg Ala Leu Gln Gln Phe
705                 710                 715                 720

Leu Tyr Val Gln Met Ala Lys Glu Leu Lys Asn Lys Leu Leu Ala Glu
                725                 730                 735

Ala Arg Glu Ser Val Val Lys Ala Cys Ser Arg Lys Leu Tyr Asn Trp
            740                 745                 750

Leu Arg Val Ala Pro Tyr Arg Pro Asp Gln Gln Val Glu Glu Asp Asp
        755                 760                 765

Asp Phe Met Asp Glu Asn Gln Gly Lys Gly Ile Arg Val Leu Gly Ile
    770                 775                 780

Ala Phe Ser Ser Ala Arg Asp His Pro Val Phe Cys Ala Leu Val Asn
785                 790                 795                 800

Gly Glu Gly Glu Val Thr Asp Phe Leu Arg Leu Pro His Phe Thr Lys
                805                 810                 815

Arg Arg Thr Ala Trp Arg Glu Glu Glu Arg Glu Lys Lys Ala Gln Asp
            820                 825                 830

Ile Glu Thr Leu Lys Lys Phe Leu Val Asn Lys Lys Pro His Val Val
        835                 840                 845

Thr Ile Ala Gly Glu Asn Arg Asp Ala Gln Met Leu Thr Glu Asp Val
    850                 855                 860

Lys Arg Ile Val His Glu Leu Asp Gln Gly Gln Gln Leu Ser Ser Ile
865                 870                 875                 880

Gly Val Glu Leu Val Asp Asn Glu Leu Ala Ile Leu Tyr Met Asn Ser
                885                 890                 895

Lys Lys Ser Glu Ala Glu Phe Arg Asp Tyr Pro Pro Val Leu Arg Gln
            900                 905                 910

Ala Val Ser Leu Ala Arg Arg Ile Gln Asp Pro Leu Ile Glu Phe Ala
        915                 920                 925

Gln Val Cys Ser Ser Asp Glu Asp Ile Leu Cys Leu Lys Phe His Pro
    930                 935                 940

Leu Gln Glu His Val Val Lys Glu Glu Leu Leu Asn Ala Leu Tyr Cys
945                 950                 955                 960

Glu Phe Ile Asn Arg Val Asn Glu Val Gly Val Asp Val Asn Arg Ala
                965                 970                 975

Ile Ala His Pro Tyr Ser Gln Ala Leu Ile Gln Tyr Val Cys Gly Leu
            980                 985                 990

Gly Pro Arg Lys Gly Thr His Leu Leu Lys Ile Leu Lys Gln Asn Asn
        995                 1000                1005

Thr Arg Leu Glu Ser Arg Thr Gln Leu Val Thr Met Cys His Met Gly
    1010                1015                1020

Pro Lys Val Phe Met Asn Cys Ala Gly Phe Leu Lys Ile Asp Thr Ala
1025                1030                1035                1040

Ser Leu Gly Asp Ser Thr Asp Ser Tyr Ile Glu Val Leu Asp Gly Ser
                1045                1050                1055

Arg Val His Pro Glu Thr Tyr Glu Trp Ala Arg Lys Met Ala Val Asp
            1060                1065                1070

Ala Leu Glu Tyr Asp Glu Ser Ala Glu Asp Ala Asn Pro Ala Gly Ala
        1075                1080                1085

Leu Glu Glu Ile Leu Glu Asn Pro Glu Arg Leu Lys Asp Leu Asp Leu
    1090                1095                1100

Asp Ala Phe Ala Glu Glu Leu Glu Arg Gln Gly Tyr Gly Asp Lys His
1105                1110                1115                1120

Ile Thr Leu Tyr Asp Ile Arg Ala Glu Leu Ser Cys Arg Tyr Lys Asp
                1125                1130                1135

Leu Arg Thr Ala Tyr Arg Ser Pro Asn Thr Glu Glu Ile Phe Asn Met
            1140                1145                1150

Leu Thr Lys Glu Thr Pro Glu Thr Phe Tyr Ile Gly Lys Leu Ile Ile
        1155                1160                1165

Cys Asn Val Thr Gly Ile Ala His Arg Arg Pro Gln Gly Glu Ser Tyr
    1170                1175                1180

Asp Gln Ala Ile Arg Asn Asp Glu Thr Gly Leu Trp Gln Cys Pro Phe
1185                1190                1195                1200

Cys Gln Gln Asp Asn Phe Pro Glu Leu Ser Glu Val Trp Asn His Phe
                1205                1210                1215

Asp Ser Gly Ser Cys Pro Gly Gln Ala Ile Gly Val Lys Thr Arg Leu
            1220                1225                1230

Asp Asn Gly Val Thr Gly Phe Ile Pro Thr Lys Phe Leu Ser Asp Lys
        1235                1240                1245

Val Val Lys Arg Pro Glu Glu Arg Val Lys Val Gly Met Thr Val His
    1250                1255                1260

Cys Arg Ile Met Lys Ile Asp Ile Glu Lys Phe Ser Ala Asp Leu Thr
1265                1270                1275                1280

Cys Arg Thr Ser Asp Leu Met Asp Arg Asn Asn Glu Trp Lys Leu Pro
                1285                1290                1295

Lys Asp Thr Tyr Tyr Asp Phe Asp Ala Glu Ala Ala Asp His Lys Gln
            1300                1305                1310

Glu Glu Asp Met Lys Arg Lys Gln Gln Arg Thr Thr Tyr Ile Lys Arg
        1315                1320                1325

Val Ile Ala His Pro Ser Phe His Asn Ile Asn Phe Lys Gln Ala Glu
    1330                1335                1340

Lys Met Met Glu Thr Met Asp Gln Gly Asp Val Ile Ile Arg Pro Ser
1345                1350                1355                1360

Ser Lys Gly Glu Asn His Leu Thr Val Thr Trp Lys Val Ser Ala Gly
                1365                1370                1375

Ile Tyr Gln His Val Asp Val Arg Glu Glu Gly Lys Glu Asn Ala Phe
            1380                1385                1390

Ser Leu Gly Ala Thr Leu Trp Ile Asn Ser Glu Glu Phe Glu Asp Leu
        1395                1400                1405

Asp Glu Ile Val Ala Arg Tyr Val Gln Pro Met Ala Ser Phe Ala Arg
    1410                1415                1420

Asp Leu Leu Asn His Lys Tyr Tyr Gln Asp Cys Ser Gly Gly Asp Arg
1425                1430                1435                1440

Lys Lys Leu Glu Glu Leu Leu Ile Lys Thr Lys Lys Glu Lys Pro Thr
                1445                1450                1455

Phe Ile Pro Tyr Phe Ile Cys Ala Cys Lys Glu Leu Pro Gly Lys Phe
            1460                1465                1470

Leu Leu Gly Tyr Gln Pro Arg Gly Lys Pro Arg Ile Glu Tyr Val Thr
        1475                1480                1485

Val Thr Pro Glu Gly Phe Arg Tyr Arg Gly Gln Ile Phe Pro Thr Val
    1490                1495                1500

Asn Gly Leu Phe Arg Trp Phe Lys Asp His Tyr Gln Asp Pro Val Pro
1505                1510                1515                1520

Gly Ile Thr Pro Ser Ser Ser Asn Arg Thr Arg Thr Pro Ala Ser Ile
                1525                1530                1535

Asn Ala Thr Pro Ala Asn Ile Asn Leu Ala Asp Leu Thr Arg Ala Val
            1540                1545                1550

Asn Ala Leu Pro Gln Asn Met Thr Ser Gln Met Phe Ser Ala Ile Ala
        1555                1560                1565

Ala Val Thr Gly Gln Gly Gln Asn Pro Asn Ala Thr Pro Ala Gln Trp
    1570                1575                1580

Ala Ser Ser Gln Tyr Gly Tyr Gly Gly Ser Gly Gly Gly Ser Ser Ala
1585                1590                1595                1600

Tyr His Val Phe Pro Thr Pro Ala Gln Gln Pro Val Ala Thr Pro Leu
                1605                1610                1615

Met Thr Pro Ser Tyr Ser Tyr Thr Thr Pro Ser Gln Pro Ile Thr Thr

1620                1625                1630

Pro Gln Tyr His Gln Leu Gln Ala Ser Thr Thr Pro Gln Ser Thr Gln
        1635                1640                1645

Ala Gln Pro Gln Pro Ser Ser Ser Ser Arg Gln Arg Gln Gln Gln Pro
    1650                1655                1660

Lys Ser Asn Ser His Ala Ala Ile Asp Trp Gly Lys Met Ala Glu Gln
1665                1670                1675                1680

Trp Leu Gln Glu Lys Glu Ala Glu Arg Arg Lys Gln Lys Gln Arg Leu
                1685                1690                1695

Thr Pro Arg Pro Ser Pro Ser Pro Met Ile Glu Ser Thr Pro Met Ser
            1700                1705                1710

Ile Ala Gly Asp Ala Thr Pro Leu Leu Asp Glu Met Asp Arg
        1715                1720                1725


<210> 110
<211> 5923
<212> DNA
<213> Rattus norvegicus

<220>
<223> Chromatin structure regulator

<400> 110
ttaacgcagc agcggcgacg gtggtggtgg tggtggcggc ggctgcggag gccttgcggt 60
gggtccggac tatccttttg cagtctcttg atcggcgact ccattttctt cggtgggagc 120
ttagcgtagc ggagaagcag cggccttcaa gcagtcttca aggaggctgt gagaagcagc 180
aacaatgtct gattttgtgg aaagtgaggc cgaagagtca gaagaagaat acaatcatga 240
aggcgaggtg gtacctagag tcaccaagaa gtttgtggaa gaggaggatg atgatgagga 300
ggaggaagag gagaatctag atgatcaaga tgagcgaggc aacctcaaag actttatcaa 360
tgacgatgat gatgaagaag aaggggagga ggatgagggc agcgactctg gggactcaga 420
agacgatgtt ggccacaaga agagaaaacg cccatctttt gatgaccgcc tggaagatga 480
tgattttgac ctcattgagg agaatttggg tgttaaagtc aaacgaggac aaaaataccg 540
acgtgtcaaa aaaatgtctg atgatgatga agatgatgaa gaggaatatg gcaaagagga 600
acacgagaag gaggccattg caggggaaat cttccaagat gaagaagggg aagaaggcca 660
ggaggctgta gaggccccca tggcccctcc tgacgaggag gaggaagatg atgaggagtc 720
agacattgac gatttcattg tggatgatga tggacagcct ctgaaaaaac ccaaatggcg 780
aaaaaagctc cctggatata cagatgcagc cctgcaggaa gcccaggaga tcttcggtgt 840
ggactttgac tatgatgaat ttgagaaata caatgaatac gatgaagaac tggaagaaga 900
ctatgagtac gaggatgatg agactgaagg tgagatccga gtgcgcccca agaagaccac 960
caagaaacgt gtgagtcgca ggagcatctt tgagatgtac gagcctagtg agctggagag 1020
cagccacctc acagaccagg acaatgaaat ccgagccact gacttgcctg agaggttcca 1080
gctccgctcc atcccagtca aggcagctga ggatgatgag ctagaagagg aagctgactg 1140
gatctataga aatgccttcg caacaccgac catctccctc caggacagct gtgattacct 1200
agaccgtggg cagccaacta gtagcttcag tcgtaaaggg cccagcacag ttcagaagat 1260
caaagaggcc ctgggcttca tgcggaatca gcattttgag gtgcctttca ttgccttcta 1320
ccgaaaggag tatgtggaac ctgagctgca catcaatgac ctgtggaggg tgtggcagtg 1380
ggatgagaag tggactcagc tgagaatccg caaagagaac ctaacacggc tgtttgagaa 1440
gatgcaggct tatcagtatg agcaaatctc tgctgaccct gacaaacctc ttgctgacgg 1500
catccgggct ctagatacta ctgacatgga gaggctcaaa gatgttcagt caatggatga 1560
actgaaagac gtctacaacc atttcttact ttactacggg cgtgacatcc ccaagatgca 1620
gaatgctgcc aaggctagcc ggaagaagct gaaacgcata aaggaggatg gcgatgaaga 1680
aggtgaaggt gaggaggctg aagatgagga acagagggga ccagagctca agcaagcctc 1740
ccgccgagac atgtatacca tctgccagag tgccgggcta gatggcctgg ccaaaaagtt 1800
tggcctcaca cctgagcagt ttggggagaa cctccgtgac agctaccagc gccacgagac 1860

agagcagttc cctgcagagc ctttggagct ggccaaagat tacgtgtgca gccagttccc 1920
tacaccagaa gctgtgcttg aaggtgcccg atacatggtt gcactacaga ttgccaggga 1980
gccccttgtc agacaggtgc tgagacaagc ctttcaggag agagccaagc taaacataac 2040
cccaaccaag aaaggtagaa aggatgtgga tgaggcccac tacgcttact cttttaagta 2100
cttaaagaac aagcctgtta aagagctgag agacgaccag ttcctcaaga taggcctggc 2160
tgaggatgaa gggctgctca ccattgacat cagcatagat atgaagggag tggaagggta 2220
tggtaatgac cagacatatt ttgaggagat caagcagttt tactaccggg atgagttcag 2280
ccaccaagta caggaatgga accggcagcg cactatggcc attgagcgag ctctgcagca 2340
gttcctctac gtgcaaatgg ccaaagaact taagaacaag ctgctagctg aggccaggga 2400
gagtgttgtg aaggcctgta gccgaaagct ctacaactgg ttgagagtgg caccctaccg 2460
tccagatcag caggtagagg aggacgacga ctttatggat gagaaccagg ggaagggcat 2520
ccgagtccta ggcattgctt tctcctctgc cagagatcac cctgtgttct gtgccctagt 2580
caatggtgaa ggagaagtga ctgatttcct ccgcctgccc cattttacca agcggcgaac 2640
tgcatggaga gaagaggagc gggaaaagaa ggctcaagat attgaaaccc taaagaagtt 2700
tcttgtgaat aagaagcctc atgtggtcac tatcgcagga gagaacaggg atgcccagat 2760
gctgactgag gatgtgaagc ggatagtgca tgagctggac caaggccaac agctgtcttc 2820
tattggggtg gagctggttg acaatgagct ggccattctg tacatgaaca gcaagaaatc 2880
agaggcagag ttccgggatt atccaccagt tttgagacaa gctgtctctc tagcccggcg 2940
tatccaagac cctctgatag aatttgccca ggtgtgcagc tcagatgaag acatcctgtg 3000
tctcaagttt catcccttgc aggagcatgt tgtgaaagag gagctgctta acgccttgta 3060
ctgtgaattc atcaaccggg tcaatgaggt cggggtggat gtcaaccgtg ccattgccca 3120
tccttacagc caggccttga tccagtatgt ctgtggcctg ggaccccgaa aagggaccca 3180
cctgctgaag attttgaaac agaacaacac tcgactcgag agccggactc agctggtcac 3240
catgtgtcat atgggtccca aagtcttcat gaattgtgct ggcttcctca agatcgacac 3300
tgcttctctg ggggacagca ctgactcata cattgaagtt cttgatggct ctcgagtcca 3360
ccctgaaacg tacgagtggg ccaggaagat ggcagtggat gccttagaat acgatgagtc 3420
tgcagaagat gccaatcctg ctggagccct tgaggaaatc ttggaaaacc cagagcggct 3480
gaaggattta gatcttgatg cctttgcaga agaactagag agacagggct atggtgacaa 3540
gcacatcaca ctgtatgaca tccgagcaga gctcagttgt aggtataagg acctccggac 3600
agcctaccgc tctcctaaca cagaggagat cttcaatatg ttaaccaaag agacgccaga 3660
gaccttctac attggaaagc ttatcatctg caatgttacc ggcattgctc acaggcgccc 3720
acagggtgag agctatgacc aggccatccg aaatgatgag acagggctgt ggcagtgccc 3780
cttctgtcaa caagacaact tccctgagct aagcgaggtc tggaaccatt ttgacagtgg 3840
atcatgccct ggtcaggcca ttggtgtgaa aacacggcta gacaatggcg tcaccggctt 3900
catccctacc aaattcctca gtgacaaggt ggtgaagagg ccagaggagc gagtgaaggt 3960
ggggatgact gttcactgcc ggatcatgaa gatcgatatt gaaaagttca gtgcagacct 4020
gacctgccgg acctcagacc tcatggacag gaacaatgag tggaaactgc ccaaggacac 4080
ctactatgac tttgatgcgg aagctgcaga ccacaagcag gaagaggata tgaaacgaaa 4140
gcagcagcgg accacataca tcaagcgagt gattgcacac ccgtccttcc ataacataaa 4200
tttcaagcaa gcagaaaaga tgatggagac catggaccaa ggggatgtta tcattcggcc 4260
cagcagtaaa ggtgaaaacc atctgacggt gacctggaaa gtcagtgctg gcatctacca 4320
gcacgtggac gtgcgggaag agggcaagga gaacgccttc agcctgggag ccactctttg 4380
gatcaacagt gaggaatttg aagacttgga tgagattgtt gcccgctatg tccagcccat 4440
ggcttccttt gctcgagacc ttctgaacca caaatattac caggactgta gtggtgggga 4500
ccgaaagaaa ttagaggagc tgctcatcaa aactaagaag gagaagccca ccttcatccc 4560
ttacttcatc tgtgcctgca aggagctgcc cggcaagttc ctcctgggat accagccccg 4620
gggtaaaccc aggatagaat atgtaacagt gactcctgaa ggattccggt accggggcca 4680
gattttccca actgtgaatg gactcttcag gtggtttaag gatcactatc aggatcctgt 4740
accaggcatc actcccagta gcagtaacag aacacggacc cctgcttcta tcaatgctac 4800
cccagccaac atcaatcttg cagatctcac acgggctgtg aatgccttgc ctcagaacat 4860
gacttcacag atgttcagtg ccattgctgc tgtgacaggc caggggcaga accccaatgc 4920
taccccagcc cagtgggcat ccagccagta tggctacggt ggcagcggag gaggcagcag 4980
cgcctaccat gtgttcccaa caccagccca gcagccagtg gccacaccac taatgacccc 5040
aagctactcc tatactaccc cgagccagcc catcaccacc ccacaatacc accagctcca 5100
ggccagcacc accccacagt ctacacaggc ccagccccag ccttcctcca gctcccgaca 5160
acggcaacag cagccaaagt ccaacagcca tgcagctatc gactggggca agatggccga 5220
gcagtggcta caggaaaagg aggcagaacg acggaaacag aaacagcggc tgaccccacg 5280
accctctccc agccccatga tcgagagcac ccccatgtcc attgctggcg atgcgacccc 5340
actcctggac gagatggatc ggtagggggc ctgttcctcg gactctggtt acctctgaag 5400
ctcccaagag gcctggccac ttatggcctc actccctgcc cctctttttt ttttgtccat 5460
aaagtggcgt gaaatgacgt tctttttagt ggtcagcctg gctggatggt ggccttgtga 5520

gctggtgtgc accgagcagc agctgttcca ctccgggctt gtgctaacca cctgttctgg 5580
ggccaggctg ggagagcagt atggcaggga ggagagcaaa ggtggagcag tctgtaatgt 5640
ctctggaagc cctcacctga gacaggccat catgaggcat gcagctcaat tgtctatgtc 5700
cccagcctgt gtggggacac cgatgctatt tattcagttt ggggcaggag ggaaagtagc 5760
tctgatcctg atggcaagac cttggctgtc aaagcaggac tgtgagggca tgtggagcag 5820
tgctgagctg ccagcccctg cctgcctgcc tgcctgcctt ccctcatcac ctaacctcac 5880
ctgcttctcg cctgtcattt gaataaacag tgtttgtaca gag 5923

<210> 111
<211> 459
<212> PRT
<213> Mus musculus

<220>
<223> Zinc finger protein ZPR1

<400> 111
Met Ser Ala Ser Gly Ala Val Gln Pro Gly His Pro Gly Ala Ala Val
1               5                   10                  15

Gly Pro Ser Pro Ala Ala Ala Ala Ser Pro Ala Thr Gly Pro Leu Phe
            20                  25                  30

Arg Pro Leu Ser Ala Glu Asp Glu Glu Gln Gln Pro Thr Glu Ile Glu
        35                  40                  45

Ser Leu Cys Met Asn Cys Tyr Arg Asn Gly Thr Thr Arg Leu Leu Leu
    50                  55                  60

Thr Lys Ile Pro Phe Phe Arg Glu Ile Ile Val Ser Ser Phe Ser Cys
65                  70                  75                  80

Glu His Cys Gly Trp Asn Asn Thr Glu Ile Gln Ser Ala Gly Arg Ile
                85                  90                  95

Gln Asp Gln Gly Val Arg Tyr Thr Leu Thr Val Arg Ser Gln Glu Asp
            100                 105                 110

Met Asn Arg Glu Val Val Lys Thr Asp Ser Ala Thr Thr Arg Ile Pro
        115                 120                 125

Glu Leu Asp Phe Glu Ile Pro Ala Phe Ser Gln Lys Gly Ala Leu Thr
    130                 135                 140

Thr Val Glu Gly Leu Ile Ser Arg Ala Ile Ser Gly Leu Glu Gln Asp
145                 150                 155                 160

Gln Pro Thr Arg Arg Ala Val Glu Gly Ala Ile Ala Glu Arg Ile Asp
                165                 170                 175

Glu Phe Ile Gly Lys Leu Lys Asp Leu Lys Gln Met Ala Ser Pro Phe
            180                 185                 190

Thr Leu Val Ile Asp Asp Pro Ser Gly Asn Ser Phe Val Glu Asn Pro
        195                 200                 205

His Ala Pro Gln Lys Asp Asn Ala Leu Val Ile Thr Tyr Tyr Asp Arg
    210                 215                 220

Thr Pro Gln Gln Ala Glu Met Leu Gly Leu Gln Ala Glu Ala Pro Glu

225                 230                 235                 240

Glu Lys Ala Glu Glu Glu Asp Leu Arg Asn Glu Val Leu Gln Phe Asn
                245                 250                 255

Thr Asn Cys Pro Glu Cys Asn Ala Pro Ala Gln Thr Asn Met Lys Leu
            260                 265                 270

Val Gln Ile Pro His Phe Lys Glu Val Ile Ile Met Ala Thr Asn Cys
        275                 280                 285

Glu Asn Cys Gly His Arg Thr Asn Glu Val Lys Ser Gly Gly Ala Val
    290                 295                 300

Glu Pro Leu Gly Thr Arg Ile Thr Leu His Ile Thr Asp Pro Ser Asp
305                 310                 315                 320

Met Thr Arg Asp Leu Leu Lys Ser Glu Thr Cys Ser Val Glu Ile Pro
                325                 330                 335

Glu Leu Glu Phe Glu Leu Gly Met Ala Val Leu Gly Gly Lys Phe Thr
            340                 345                 350

Thr Leu Glu Gly Leu Leu Lys Asp Ile Arg Glu Leu Val Thr Lys Asn
        355                 360                 365

Pro Phe Thr Leu Gly Asp Ser Ser Asn Pro Asp Gln Ser Glu Lys Leu
    370                 375                 380

Gln Glu Phe Ser Gln Lys Leu Gly Gln Ile Ile Glu Gly Lys Met Lys
385                 390                 395                 400

Ala His Phe Ile Met Asn Asp Pro Ala Gly Asn Ser Tyr Leu Gln Asn
                405                 410                 415

Val Tyr Ala Pro Glu Asp Asp Pro Glu Met Lys Val Glu Arg Tyr Lys
            420                 425                 430

Arg Thr Phe Asp Gln Asn Glu Glu Leu Gly Leu Asn Asp Met Lys Thr
        435                 440                 445

Glu Gly Tyr Glu Ala Gly Leu Ala Pro Gln Arg
    450                 455


<210> 112
<211> 2162
<212> DNA
<213> Mus musculus

<220>
<223> Zinc finger protein ZPR1

<400> 112
   ccgctccccg ttcctccaca accacaacct ttctttttca aagaagattt gccccgggga 60
   agccgccgcg ggagcaacgc gtggggagat gtctgccagc ggggctgtgc agccgggaca 120
   cccgggggcc gccgtcgggc cctcgcccgc tgcggctgcg tcaccagcca ccgggccttt 180
   gttccggccc ctcagcgccg aagatgagga gcaacagccc accgagatcg agtcactgtg 240
   catgaactgt taccggaacg gcacgacgcg acttctgctc accaagatcc ccttctttag 300
   agaaatcatc gtgagctcct tttcctgcga acactgtggc tggaacaaca cggagatcca 360
   gtctgcaggc aggatccagg accagggagt gcgctacacc ttgaccgtga gaagccaaga 420
   ggacatgaac agagaagtgg taaagacaga ctctgccacc acaaggatcc ccgagctgga 480
   ttttgagatt ccagccttca gccagaaggg agctctgacc actgttgaag gactcatcag 540
   ccgtgcgatc tctggcctgg aacaggatca gcccacacga cgggcagtgg aaggtgccat 600
   cgcagagaga attgatgagt tcattggcaa actgaaggac ctaaagcaaa tggcttcccc 660
   tttcacactg gtcattgatg atccctcggg aaacagcttt gtagaaaacc cacatgctcc 720
   ccagaaagat aatgccttgg tgatcacata ctatgaccga accccacagc aagctgagat 780
   gctggggctc caagcagaag caccagagga gaaggcggaa gaggaagacc ttagaaacga 840
   agtgctccag ttcaacacta actgcccaga gtgcaacgct ccggctcaga ccaacatgaa 900
   gcttgtccaa atcccccact ttaaagaggt tatcatcatg gccaccaact gtgagaattg 960
   tgggcatcgg actaatgagg tgaagtccgg aggagctgta gaacctttgg gtaccaggat 1020
   caccctccac atcacagatc cctcagacat gaccagagac ctcctcaagt ctgagacctg 1080
   tagtgtggaa atcccagagc ttgagtttga actgggaatg gctgtacttg ggggcaagtt 1140
   caccactcta gaaggactgc tgaaagacat ccgagaactg gtaaccaaga acccattcac 1200
   actgggcgac agctctaatc ctgaccagtc agagaaactg caggagttta gccagaagtt 1260
   gggccagatc atcgagggca agatgaaggc ccactttatc atgaatgatc cagcaggaaa 1320
   cagttatctg cagaatgtgt atgcacctga agacgatcca gagatgaagg tcgagcggta 1380
   caaacgcacc tttgaccaaa atgaggagct cgggctcaat gacatgaaga cagagggcta 1440
   tgaggcgggc ctggccccac agcggtagca gtggccagct cactggccag ctacagtgcc 1500
   actcacactg cagggttatc tgttactgtg gggaactgac gaggagtgct caagccctcg 1560
   tccatggtga agaggttacc acttgagtta gaaatgtaag cacccaagat tagcagctga 1620
   cggacgaggc agctgcagcc ctactgtgct ccttgacctt cttttggagg ttttaaagtc 1680
   ggcgtgagaa gaatcccaga aacaccaggc ggtctgccat caccgtttgc ctgtcagctc 1740
   tctgacctcc agtgctggac ctttgaagtc tggggaagtg aaatacaagt ttctgctggc 1800
   tctgggcatg tgaagtactg actcagcagg gcaaggatgt cggaggggcc gagcaggcac 1860
   aggtgaagac gccacattaa agtgatggcc tttaaacgaa aggagaacaa ctatccagac 1920
   tcctacctcc cacatggaag aaaccgccac ctcatcaagt taataaagaa aaagaaaaga 1980
   agggaggtcc agagtcattc ccacattctg ttggaggagg agagaaggat gttcactact 2040
   tggtgcatac aggcatgcac acggacagac aggtgcgtgc acacacacaa ccacacacaa 2100
   acacaggttt gctgatggaa cattattata caattctgag cttacataaa aaaaaaaaaa 2160
   aa 2162

<210> 113
<211> 1324
<212> PRT
<213> Rattus norvegicus

<220>
<223> KIAA0627

<400> 113
Gln Ala Glu Pro Ser Gly Ala Gly Thr Glu Leu Tyr Leu Phe Ala Val
1               5                   10                  15

Leu Phe Asp Leu Leu Val Ser Glu Ser Ser Leu Asp His Leu Met Ala
            20                  25                  30

Met Gly Asp Asp Lys Ser Phe Asp Asp Glu Glu Ser Val Asp Gly Asn
        35                  40                  45

Arg Pro Ser Ser Ala Ala Ser Ala Phe Lys Val Pro Ala Pro Lys Thr
    50                  55                  60

Ser Gly Asn Pro Ala Asn Ser Ala Arg Lys Pro Gly Ser Ala Gly Gly
65                  70                  75                  80

Pro Lys Val Gly Gly Ala Ser Lys Glu Gly Gly Ala Gly Ala Val Asp
                85                  90                  95

Glu Asp Asp Phe Ile Lys Ala Phe Thr Asp Val Pro Ser Ile Gln Ile
            100                 105                 110

Tyr Ser Ser Arg Glu Leu Glu Glu Thr Leu Asn Lys Ile Arg Glu Ile
        115                 120                 125

Leu Ser Asp Asp Lys His Asp Trp Asp Gln Arg Ala Asn Ala Leu Lys
    130                 135                 140

Lys Ile Arg Ser Leu Leu Val Ala Gly Ala Ala Gln Tyr Asp Cys Phe
145                 150                 155                 160

Phe Gln His Leu Arg Leu Leu Asp Gly Ala Leu Lys Leu Ser Ala Lys
                165                 170                 175

Asp Leu Arg Ser Gln Val Val Arg Glu Ala Cys Ile Thr Val Ala His
            180                 185                 190

Leu Ser Thr Val Leu Gly Asn Lys Phe Asp His Gly Ala Glu Ala Ile
        195                 200                 205

Val Pro Thr Leu Phe Asn Leu Val Pro Asn Ser Ala Lys Val Met Ala
    210                 215                 220

Thr Ser Gly Cys Ala Ala Ile Arg Phe Ile Ile Arg His Thr His Val
225                 230                 235                 240

Pro Arg Leu Ile Pro Leu Ile Thr Ser Asn Cys Thr Ser Lys Ser Val
                245                 250                 255

Pro Val Arg Arg Arg Ser Phe Glu Phe Leu Asp Leu Leu Leu Gln Glu
            260                 265                 270

Trp Gln Thr His Ser Leu Glu Arg His Ala Ala Val Leu Val Glu Thr
        275                 280                 285

Ile Lys Lys Gly Ile His Asp Ala Asp Ala Glu Ala Arg Val Glu Ala
    290                 295                 300

Arg Lys Thr Tyr Met Gly Leu Arg Asn His Phe Pro Gly Glu Ala Glu
305                 310                 315                 320

Thr Leu Tyr Asn Ser Leu Glu Pro Ser Tyr Gln Lys Ser Leu Gln Thr
                325                 330                 335

Tyr Leu Lys Ser Ser Gly Ser Val Ala Ser Leu Pro Gln Ser Asp Arg
            340                 345                 350

Ser Ser Ser Ser Ser Gln Glu Ser Leu Asn Arg Pro Phe Ser Ser Lys
        355                 360                 365

Trp Ser Thr Ala Asn Pro Ser Thr Val Ala Gly Arg Val Ser Ala Gly
    370                 375                 380

Ser Ser Lys Ala Ser Ser Leu Pro Gly Ser Leu Gln Arg Ser Arg Ser
385                 390                 395                 400

Asp Ile Asp Val Asn Ala Ala Ala Gly Ala Lys Ala His His Ala Ala
                405                 410                 415

Gly Gln Ser Val Arg Arg Gly Arg Leu Gly Ala Gly Ala Leu Asn Ala
            420                 425                 430

Gly Ser Tyr Ala Ser Leu Glu Asp Thr Ser Asp Lys Leu Asp Gly Thr
        435                 440                 445

Ala Ser Glu Asp Gly Arg Val Arg Ala Lys Leu Ser Ala Pro Leu Ala
    450                 455                 460

Gly Met Gly Asn Ala Lys Ala Asp Ser Arg Gly Arg Ser Arg Thr Lys
465                 470                 475                 480

Met Val Ser Gln Ser Gln Pro Gly Ser Arg Ser Gly Ser Pro Gly Arg
                485                 490                 495

Val Leu Thr Thr Thr Ala Leu Ser Thr Val Ser Ser Gly Val Gln Arg
            500                 505                 510

Val Leu Val Asn Ser Ala Ser Ala Gln Lys Arg Ser Lys Ile Pro Arg
        515                 520                 525

Ser Gln Gly Cys Ser Arg Glu Ala Ser Pro Ser Arg Leu Ser Val Ala
    530                 535                 540

Arg Ser Ser Arg Ile Pro Arg Pro Ser Val Ser Gln Gly Cys Ser Arg
545                 550                 555                 560

Glu Ala Ser Arg Glu Ser Ser Arg Asp Thr Ser Pro Val Arg Ser Phe
                565                 570                 575

Gln Pro Leu Ala Ser Arg His His Ser Arg Ser Thr Gly Ala Leu Tyr
            580                 585                 590

Ala Pro Glu Val Tyr Gly Ala Ser Gly Pro Gly Tyr Gly Ile Ser Gln
        595                 600                 605

Ser Ser Arg Leu Ser Ser Ser Val Ser Ala Met Arg Val Leu Asn Thr
    610                 615                 620

Gly Ser Asp Val Glu Glu Ala Val Ala Asp Ala Leu Lys Lys Pro Ala
625                 630                 635                 640

Arg Arg Arg Tyr Glu Ser Tyr Gly Met His Ser Asp Asp Asp Ala Asn
                645                 650                 655

Ser Asp Ala Ser Ser Ala Cys Ser Glu Arg Ser Tyr Ser Ser Arg Asn
            660                 665                 670

Gly Ser Ile Pro Thr Tyr Met Arg Gln Thr Glu Asp Val Ala Glu Val
        675                 680                 685

Leu Asn Arg Cys Ala Ser Ser Asn Trp Ser Glu Arg Lys Glu Gly Leu
    690                 695                 700

Leu Gly Leu Gln Asn Leu Leu Lys Asn Gln Arg Thr Leu Ser Arg Val
705                 710                 715                 720

Glu Leu Lys Arg Leu Cys Glu Ile Phe Thr Arg Met Phe Ala Asp Pro
                725                 730                 735

His Gly Lys Arg Val Phe Ser Met Phe Leu Glu Thr Leu Val Asp Phe
            740                 745                 750

Ile Gln Val His Lys Asp Asp Leu Gln Asp Trp Leu Phe Val Leu Leu

755                 760                 765

Thr Gln Leu Leu Lys Lys Met Gly Ala Asp Leu Leu Gly Ser Val Gln
    770                 775                 780

Ala Lys Val Gln Lys Ala Leu Asp Val Thr Arg Glu Ser Phe Pro Asn
785                 790                 795                 800

Asp Leu Gln Phe Asn Ile Leu Met Arg Phe Thr Val Asp Gln Thr Gln
                805                 810                 815

Thr Pro Ser Leu Lys Val Lys Val Ala Ile Leu Lys Tyr Ile Glu Thr
            820                 825                 830

Leu Ala Lys Gln Met Asp Pro Gly Asp Phe Ile Asn Ser Ser Glu Thr
        835                 840                 845

Arg Leu Ala Val Ser Arg Val Ile Thr Trp Thr Thr Glu Pro Lys Ser
    850                 855                 860

Ser Asp Val Arg Lys Ala Ala Gln Ser Val Leu Ile Ser Leu Phe Glu
865                 870                 875                 880

Leu Asn Thr Pro Glu Phe Thr Met Leu Leu Gly Ala Leu Pro Lys Thr
                885                 890                 895

Phe Gln Asp Gly Ala Thr Lys Leu Leu His Asn His Leu Arg Asn Thr
            900                 905                 910

Gly Asn Gly Thr Gln Ser Ser Met Gly Ser Pro Leu Thr Arg Pro Thr
        915                 920                 925

Pro Arg Ser Pro Ala Asn Trp Ser Ser Pro Leu Thr Ser Pro Thr Asn
    930                 935                 940

Thr Ser Gln Asn Thr Leu Ser Pro Ser Ala Phe Asp Tyr Asp Thr Glu
945                 950                 955                 960

Asn Met Asn Ser Glu Asp Ile Tyr Ser Ser Leu Arg Gly Val Thr Glu
                965                 970                 975

Ala Ile Gln Asn Phe Ser Phe Arg Ser Gln Glu Asp Met Asn Glu Pro
            980                 985                 990

Leu Lys Arg Asp Ser Lys Lys Asp Asp Gly Asp Ser Met Cys Gly Gly
        995                 1000                1005

Pro Gly Met Ser Asp Pro Arg Ala Gly Gly Asp Ala Thr Asp Ser Ser
    1010                1015                1020

Gln Thr Ala Leu Asp Asn Lys Ala Ser Leu Leu His Ser Met Pro Thr
1025                1030                1035                1040

His Ser Ser Pro Arg Ser Arg Asp Tyr Asn Pro Tyr Asn Tyr Ser Asp
                1045                1050                1055

Ser Ile Ser Pro Phe Asn Lys Ser Ala Leu Lys Glu Ala Met Phe Asp
            1060                1065                1070

Asp Asp Ala Asp Gln Phe Pro Asp Asp Leu Ser Leu Asp His Ser Asp
        1075                1080                1085

Leu Val Ala Glu Leu Leu Lys Glu Leu Ser Asn His Asn Glu Arg Val
1090                1095                1100

Glu Glu Arg Lys Ile Ala Leu Tyr Glu Leu Met Lys Leu Thr Gln Glu
1105                1110                1115                1120

Glu Ser Phe Ser Val Trp Asp Glu His Phe Lys Thr Ile Leu Leu Leu
                1125                1130                1135

Leu Leu Glu Thr Leu Gly Asp Lys Glu Pro Thr Ile Arg Ala Leu Ala
            1140                1145                1150

Leu Lys Val Leu Arg Glu Ile Leu Arg His Gln Pro Ala Arg Phe Lys
        1155                1160                1165

Asn Tyr Ala Glu Leu Thr Val Met Lys Thr Leu Glu Ala His Lys Asp
    1170                1175                1180

Pro His Lys Glu Val Val Arg Ser Ala Glu Glu Ala Ala Ser Val Leu
1185                1190                1195                1200

Ala Thr Ser Ile Ser Pro Glu Gln Cys Ile Lys Val Leu Cys Pro Ile
                1205                1210                1215

Ile Gln Thr Ala Asp Tyr Pro Ile Asn Leu Ala Ala Ile Lys Met Gln
            1220                1225                1230

Thr Lys Val Ile Glu Arg Val Ser Lys Glu Thr Leu Asn Leu Leu Leu
        1235                1240                1245

Pro Glu Ile Met Pro Gly Leu Ile Gln Gly Tyr Asp Asn Ser Glu Ser
    1250                1255                1260

Ser Val Arg Lys Ala Cys Val Phe Cys Leu Val Ala Val His Ala Val
1265                1270                1275                1280

Ile Gly Asp Glu Leu Lys Pro His Leu Ser Gln Leu Thr Gly Ser Lys
                1285                1290                1295

Met Lys Leu Leu Asn Leu Tyr Ile Lys Arg Ala Gln Thr Gly Ser Gly
            1300                1305                1310

Gly Ala Asp Pro Thr Thr Asp Val Ser Gly Gln Ser
        1315                1320


<210> 114
<211> 5614
<212> DNA
<213> Rattus norvegicus

<220>
<223> KIAA0627

<400> 114
gcaggccgag cccagtggcg caggcactga gctgtactta tttgctgtgc tatttgacct 60
cttggtttca gagagctccc tggaccatct aatggctatg ggagatgata aaagcttcga 120
tgatgaagaa tcagtggatg gaaataggcc atcatcagct gcatcagcct tcaaggttcc 180
tgcacctaaa acatccggaa atcctgccaa cagtgcaagg aagcctggtt cagcaggtgg 240
ccctaaggtt ggaggtgctt ctaaggaagg aggtgctgga gcagttgatg aagatgattt 300

tataaaagct tttacagatg tcccttctat tcagatttat tctagtcgag aactcgaaga 360
aacattaaat aaaatcaggg aaattttgtc agatgataaa catgactggg atcagcgtgc 420
caatgcactg aagaaaattc gatcactgct tgttgctgga gctgcacagt atgattgctt 480
ttttcaacat ttacgattgt tggatggagc acttaaactt tcagctaagg atcttagatc 540
ccaggtggtt agagaagctt gtattactgt agcccacctt tcaacagttt tgggaaacaa 600
gtttgatcat ggcgctgaag ccattgtacc tacacttttt aatctcgtcc ccaatagtgc 660
aaaagtcatg gcaacttctg gatgtgcagc aatcagattt atcattcggc atactcatgt 720
acccagactt atacctttaa taacaagcaa ttgcacatca aaatcagttc ccgtgaggag 780
acgttcattt gaatttttag atttattgtt gcaagagtgg cagactcatt cattggaaag 840
acatgcagcc gtcttggttg aaactattaa aaagggaatt catgatgctg acgctgaggc 900
cagagtggag gcaagaaaga catacatggg tcttagaaac cactttcctg gtgaagctga 960
aacattatat aattcccttg agccatctta tcagaagagt cttcaaactt acttaaagag 1020
ttctggcagt gtagcatctc ttccacaatc agacaggtcc tcatccagct cacaggaaag 1080
tctcaatcgc cctttttctt ccaaatggtc tacagcaaat ccatcaactg tggctggaag 1140
agtatcagca ggcagcagca aagccagttc ccttccagga agcctgcagc gttcacgaag 1200
tgacattgat gtgaatgctg ctgcaggtgc caaggcacat catgctgctg gacagtctgt 1260
gcgaaggggg cgcttaggtg caggtgccct gaatgcaggt tcctatgcgt cactagagga 1320
tacttctgac aagctggatg gaacagcatc tgaagatggc cgggtgagag caaaactttc 1380
agcaccactt gctggcatgg gaaatgccaa ggcagattct agaggaagaa gtcgaacaaa 1440
aatggtgtct caatcacagc ctggtagccg gtctgggtct ccaggaagag ttctgaccac 1500
aacagccctg tccactgtga gctctggtgt tcaaagagtc ctggtcaatt cagcctcagc 1560
acaaaaaaga agcaagatac cacggagcca gggctgtagc agagaggcta gtccatctag 1620
gctttcagtg gcccgaagca gtcgtattcc tcgaccaagt gtgagtcaag gatgcagccg 1680
ggaagctagt cgggagagca gcagagacac aagtcctgtt cgctcttttc agcccctcgc 1740
ctccagacac cattccagat caactggtgc cctctacgcc cccgaagtgt atggggcctc 1800
aggtccaggt tatgggatca gccaatcaag tcgactgtcg tcttctgtta gtgccatgcg 1860
agtcctgaac acaggttctg atgtggagga ggcggtggca gatgccttga aaaaaccagc 1920
tcgaagaaga tatgaatcat atggaatgca ttcagatgat gacgccaaca gcgatgcatc 1980
tagtgcttgt tcagaacgct cctatagttc tcgaaatggt agtattccta catatatgag 2040
gcagacggaa gatgtggcag aagtcctcaa tagatgtgct agttccaatt ggtcagaaag 2100
gaaagaaggc ctcctaggtc tgcagaactt attaaaaaat cagagaacac taagtcgagt 2160
tgaactgaaa agattatgtg aaattttcac aagaatgttt gctgaccctc atggcaagag 2220
agtattcagc atgtttttgg agactctagt ggatttcata caagtccaca aagatgatct 2280
tcaagattgg ttgtttgtac tgctgacaca actactaaaa aaaatgggtg ctgatttgct 2340
tggatctgtt caggcaaaag ttcagaaagc ccttgatgtt acaagagagt cttttccaaa 2400
tgatcttcag ttcaatattc taatgagatt tacagttgat cagacccaga caccaagctt 2460
aaaggtgaag gttgctatcc ttaaatacat agaaactctg gccaaacaga tggatccagg 2520
agattttata aattccagtg aaactcgcct agcagtgtct cgggtcatca cttggacaac 2580
agaacccaaa agttctgatg ttcggaaggc agcacagtca gtgctgattt cattatttga 2640
actcaatacc ccagagttta caatgttatt aggagcttta ccaaaaactt ttcaggatgg 2700
tgctaccaag cttcttcata atcaccttcg aaacactggc aatggaaccc agagttccat 2760
ggggagtcct ttgacaagac caacaccacg atcaccagct aactggtcca gtcctcttac 2820
ttctcctacc aatacatcac agaatacttt atctccaagt gcatttgatt atgacacaga 2880
aaatatgaac tctgaagata tttatagctc tcttagaggt gtcactgaag caatccagaa 2940
tttcagcttc cgtagccaag aagatatgaa tgagccattg aaaagggatt ctaaaaaaga 3000
tgatggcgat tcaatgtgtg gtggtcctgg gatgtctgac ccaagagcag gaggtgatgc 3060
tactgactca agtcaaacag ctcttgataa taaagcttca ttgctccatt caatgcctac 3120
tcactcctct ccacgctctc gagactataa tccatataac tattcagata gcatcagtcc 3180
cttcaacaag tctgccctca aggaagccat gtttgatgat gatgctgacc agtttcctga 3240
cgatctttcc ctagatcatt ctgacctagt tgcagagttg ttgaaggagc tgtctaacca 3300
taatgagcgt gtagaagaaa gaaaaattgc cctctatgaa cttatgaaac tgacacagga 3360
agaatctttt agtgtttggg atgaacactt caaaacaata ttgcttttat tgcttgaaac 3420
gcttggagat aaagagccta caatcagggc tttggcatta aaggttttaa gagaaatcct 3480
aaggcatcaa ccagcaagat ttaaaaacta tgcagaattg actgtcatga aaacattgga 3540
agcacataaa gatcctcata aggaggtggt gagatctgct gaggaagcgg catcagtgtt 3600
ggccacttca attagtccag agcagtgcat caaagtgctt tgtcctatca ttcaaactgc 3660
agactaccca attaatctgg ctgcaatcaa aatgcaaaca aaagtgatag agagagtgtc 3720
caaggaaacc ctaaacctgc ttttgccaga gattatgcca ggtctaatac agggttatga 3780
taattcagag agcagtgttc ggaaagcttg tgtcttctgc ctggtggctg ttcatgcggt 3840
aattggtgat gaactaaaac cacatctcag tcaacttact ggcagtaaaa tgaagctact 3900
gaatctttac atcaaacgtg cacaaacagg ttctggagga gctgatccca ctactgatgt 3960

ttctggacaa agttagtgaa gctcatcaca gcgaaccagg tctctcaaaa gaaaggacag 4020
atagaccacc ctcatcaatg aaaggaagtt ctcaaacaca tcctttggaa cttactattg 4080
tttcccagtt ttagtttttt gtttcgtttc gttttgtatt ttctgtaaca gaggactatc 4140
ctcagtctgc atgtaacttt tatgatagtt attccaaatt caagaagaag cagtattaac 4200
atcaactgat cgacacaaag taatttttaa tttaattcat catttcacat gtttgtactt 4260
tgtcttccca ttaacctttg ccagtgttat gattgtataa atttttttaa atgctggtta 4320
aacaggaatg cttaaagctt taaaagttta acagtctaaa acatttttgc ttttattcaa 4380
ctgcagaata atatttttat tgctactttg agttttgttt cgtatcatgt cctatgctag 4440
aaatatttaa atgatgtgaa acaaagcagg actaatttga actacagctg gactccgttt 4500
gtgtgatggt gatacatgtc attagttgca acttctttgg ggtgatctat agtttgaaaa 4560
ctaaaacctc aaagacagat gttacagaat cagccagttc tgtaaaactg atattgtcta 4620
ttggttattg atcttgccat ctttatttaa aaccatgtcc cttctatgat cccttaagaa 4680
agctgcacca aatcatctgc ctgttttttc ttgatactta ctgaaataga aggttttatt 4740
gcagggttta ttttggtttg tttatatctt tgttgtgaat gatgcttttt tgtatttatt 4800
aatatcaaat tcacttatga ataaacttga taatggaaac ggacaaaaaa aatcaagtgc 4860
gtgtgtgtcc ttgaccgtct tctgtttctc acgtaataaa caaattatcg agacatggga 4920
gtgaccagca ccttttcttt aaatggtgga acctggtttc cttttaccat gaaattgtct 4980
tacttgaaaa tattgatcct gatgagagag aagatggtgc caaggctgtc tttgtataat 5040
gggctcaaat tctctacctc ttcagggcta atacttttaa ctgagctgct gcctatagtg 5100
tcttttggaa aactacttaa agggtgattt tctgttactt tttagcaaat ttttttaatc 5160
acctcttgct acacccattc ttttcatgtg cagccgactc aaaaattacc agttttggtg 5220
aaaggctaaa ttagataatt tggaaccagg atactaatga tttctcatct ttactttttt 5280
ttaatcctaa tataaagtga atttgattga aaaggcaaat agctattagg gaagcagttt 5340
gccattgttg cagagttatc tgtactttgt ttaactgaaa aaaatgtaga aatatatgta 5400
aagaatttaa gacaagagta ctgaatggat gatttgtcat aggctttccc ctttctttct 5460
gttctagcag caggaaaagt ttctctatat cctctccctc tacctgtaac aatttgtttt 5520
tctactgtta attacattgt gtatttatag ttctatgctt actgttgtgc atatactggc 5580
aataaaactg tacataacat tacttgaaaa agtt 5614

<210> 115
<211> 463
<212> PRT
<213> Rattus norvegicus

<220>
<223> Statin-related protein

<400> 115
Met Gly Lys Glu Lys Thr His Ile Asn Ile Val Val Ile Gly His Val
1               5                   10                  15

Asp Ser Gly Lys Ser Thr Thr Thr Gly His Leu Ile Tyr Lys Cys Gly
            20                  25                  30

Gly Ile Asp Lys Arg Thr Ile Glu Lys Phe Glu Lys Glu Ala Ala Glu
        35                  40                  45

Met Gly Lys Gly Ser Phe Lys Tyr Ala Trp Val Leu Asp Lys Leu Lys
    50                  55                  60

Ala Glu Arg Glu Arg Gly Ile Thr Ile Asp Ile Ser Leu Trp Lys Phe
65                  70                  75                  80

Glu Thr Thr Lys Tyr Tyr Ile Thr Ile Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Val Ala Ala Gly Val Gly Glu Phe Glu Ala Gly Ile Ser

115                 120                 125

Lys Asn Gly Gln Thr Arg Glu His Ala Leu Leu Ala Tyr Thr Leu Gly
    130                 135                 140

Val Lys Gln Leu Ile Val Gly Val Asn Lys Met Asp Ser Thr Glu Pro
145                 150                 155                 160

Ala Tyr Ser Glu Lys Arg Tyr Asp Glu Ile Val Lys Glu Val Ser Ala
                165                 170                 175

Tyr Ile Lys Lys Ile Gly Tyr Asn Pro Ala Thr Val Pro Phe Val Pro
            180                 185                 190

Ile Ser Gly Trp His Gly Asp Asn Met Leu Glu Pro Ser Pro Asn Met
        195                 200                 205

Pro Trp Phe Lys Gly Trp Lys Val Glu Arg Lys Glu Gly Asn Ala Ser
    210                 215                 220

Gly Val Ser Leu Leu Glu Ala Leu Asp Thr Ile Leu Pro Pro Thr Arg
225                 230                 235                 240

Pro Thr Asp Lys Pro Leu Arg Leu Pro Leu Gln Asp Val Tyr Lys Ile
                245                 250                 255

Gly Gly Ile Gly Thr Val Pro Val Gly Arg Val Glu Thr Gly Ile Leu
            260                 265                 270

Arg Pro Gly Met Val Val Thr Phe Ala Pro Val Asn Ile Thr Thr Glu
        275                 280                 285

Val Lys Ser Val Glu Met His His Glu Ala Leu Ser Glu Ala Leu Pro
    290                 295                 300

Gly Asp Asn Val Gly Phe Asn Val Lys Asn Val Ser Val Lys Asp Ile
305                 310                 315                 320

Arg Arg Gly Asn Val Cys Gly Asp Ser Lys Ala Asp Pro Pro Gln Glu
                325                 330                 335

Ala Ala Gln Phe Thr Ser Gln Val Ile Ile Leu Asn His Pro Gly Gln
            340                 345                 350

Ile Ser Ala Gly Tyr Ser Pro Val Ile Asp Cys His Thr Ala His Ile
        355                 360                 365

Ala Cys Lys Phe Ala Glu Leu Lys Glu Lys Ile Asp Arg Arg Ser Gly
    370                 375                 380

Lys Lys Leu Glu Asp Asn Pro Lys Ser Leu Lys Ser Gly Asp Ala Ala
385                 390                 395                 400

Ile Val Glu Met Val Pro Gly Lys Pro Met Cys Val Glu Ser Phe Ser
                405                 410                 415

Gln Tyr Pro Pro Leu Gly Arg Phe Ala Val Arg Asp Met Arg Gln Thr
            420                 425                 430

Val Ala Val Gly Val Ile Lys Asn Val Glu Lys Lys Ser Gly Gly Ala
        435                 440                 445

Gly Lys Val Thr Lys Ser Ala Gln Lys Ala Gln Lys Ala Gly Lys
    450                 455                 460

<210> 116
<211> 1719
<212> DNA
<213> Rattus norvegicus

<220>
<223> Statin-related protein

<400> 116
cagcgccacc agtccctctg actgagacct cggctctaga gttcctgccc agcaaattcc 60
ctcaacccag agccagagcc cccacagtgc cagcccgtcc ctcactcagg cagaatgggc 120
aaggagaaga cacacatcaa cattgtggtc attggccacg tggactcagg caagtctacc 180
acgacaggcc atctcatcta caaatgtggc ggcatcgaca agcggaccat cgagaagttt 240
gagaaggagg cagcagagat ggggaagggc tcctttaaat atgcctgggt gctggacaag 300
ctgaaggcag agcgggagcg cggcatcacc atcgacatct ccctctggaa gtttgagacc 360
accaagtact acatcaccat cattgatgct ccaggccacc gggacttcat caagaacatg 420
attactggca catcccaggc cgactgtgca gtactgatcg tggcagcggg cgtgggcgag 480
tttgaggcgg gcatctccaa aaacgggcaa acccgggaac acgcactcct ggcctacact 540
ctgggtgtga aacagctcat tgtgggtgtc aacaagatgg actccacgga accagcctac 600
agcgagaagc gctacgatga gattgtcaag gaggtcagcg cctacatcaa gaagatcggc 660
tacaacccag ccacggtgcc cttcgtgccc atctcgggct ggcacggtga caacatgctg 720
gaaccttcac ctaatatgcc atggttcaag ggctggaagg tagagcgtaa ggaaggaaat 780
gcaagcggcg tgtccctgct ggaagccctg gacacaatcc tgccccccac ccgccccact 840
gacaagcccc ttcgtctacc gctgcaggat gtgtacaaga ttggtggcat tgggactgta 900
cctgtgggca gagtggagac cggtatcctc cgtcctggta tggtggtgac ctttgcgcca 960
gtcaacatca ccacagaggt gaagtctgtg gaaatgcacc acgaggcgct cagcgaggcc 1020
ctgcctggtg acaatgtcgg gttcaacgtg aagaatgtgt ctgtcaagga cattcgccgg 1080
ggcaatgtct gtggggacag caaagctgac ccccctcagg aggccgccca gttcacctct 1140
caggttatca tcctgaacca ccctgggcaa atcagcgctg gctactcgcc agtcatcgac 1200
tgtcacacag cccacatcgc ctgcaagttt gccgagctaa aggagaagat tgaccgtcgc 1260
tctggcaaga agctggagga caatcccaag tccctgaagt ctggggatgc cgccattgtt 1320
gagatggtcc caggaaaacc catgtgtgtg gagagcttct cacagtaccc acccctcggc 1380
cgcttcgccg tgcgcgacat gcggcagact gtggccgtgg gcgtcatcaa gaacgtggag 1440
aagaagagcg gcggcgccgg caaggtcacc aagtccgcac agaaggcgca gaaagcgggc 1500
aagtgaaccg gcgcccggcg tggccctccc caacgcgccc gccgcgcccc cagccccgcc 1560
cccggcactg gccccgtccc ctccccgccc cagccggccc ctccgccccg cccccccgcc 1620
aggcgcatgt ctgcacctcc gcttgtaaga ggctctacgt cagcgactgg atgctcgcca 1680
tcaaagtcca gtggaaattc ttcaagagga aaagcgcgg 1719

<210> 117
<211> 467
<212> PRT
<213> Rattus norvegicus

<220>
<223> Dynactin subunit p62

<400> 117
Met Ala Ser Leu Leu Gln Ser Glu Arg Val Leu Tyr Leu Val Gln Gly
1               5                   10                  15

Glu Lys Lys Val Arg Ala Pro Leu Ser Gln Leu Tyr Phe Cys Arg Tyr
            20                  25                  30

Cys Ser Glu Leu Arg Ser Leu Glu Cys Val Ser His Glu Val Asp Ser

        35                  40                  45

His Tyr Cys Pro Ser Cys Leu Glu Asn Met Pro Ser Ala Glu Ala Lys
    50                  55                  60

Leu Lys Lys Asn Arg Cys Ala Asn Cys Phe Asp Cys Pro Gly Cys Met
65                  70                  75                  80

His Thr Leu Ser Thr Arg Ala Thr Ser Ile Ser Thr Gln Leu Pro Asp
                85                  90                  95

Asp Pro Ala Lys Thr Thr Met Lys Lys Ala Tyr Tyr Leu Ala Cys Gly
            100                 105                 110

Phe Cys Arg Trp Thr Ser Arg Asp Val Gly Met Ala Asp Lys Ser Val
        115                 120                 125

Ala Ser Gly Gly Trp Gln Glu Pro Glu Asn Pro His Ala Gln Arg Met
    130                 135                 140

Asn Lys Leu Ile Glu Tyr Tyr Gln Gln Leu Ala Gln Lys Glu Lys Val
145                 150                 155                 160

Glu Arg Asp Arg Lys Lys Leu Ala Arg Arg Arg Asn Tyr Met Pro Leu
                165                 170                 175

Ala Phe Ser Gln His Thr Ile His Val Val Asp Lys Tyr Ser Leu Gly
            180                 185                 190

Thr Arg Leu Gln Arg Pro Arg Ala Gly Ala Ser Ile Ser Thr Leu Ala
        195                 200                 205

Gly Leu Ser Leu Arg Glu Gly Glu Asp Gln Lys Glu Val Lys Ile Glu
    210                 215                 220

Pro Ala Gln Ala Val Ala Glu Val Glu Pro Leu Pro Glu Asp Tyr Tyr
225                 230                 235                 240

Thr Arg Pro Val Asn Leu Thr Glu Val Thr Thr Leu Gln Gln Arg Leu
                245                 250                 255

Leu Gln Pro Asp Leu Gln Pro Val Ser Ala Ser Gln Leu Tyr Pro Arg
            260                 265                 270

His Lys His Leu Leu Ile Lys Arg Ser Leu Arg Cys Arg Lys Cys Glu
        275                 280                 285

His Asn Leu Ser Lys Pro Glu Phe Asn Pro Thr Ser Ile Lys Phe Lys
    290                 295                 300

Ile Gln Leu Val Ala Val Asn Tyr Ile Pro Glu Val Arg Ile Met Ser
305                 310                 315                 320

Ile Pro Asn Leu Arg Tyr Met Lys Glu Ser Gln Val Leu Leu Thr Leu
                325                 330                 335

Thr Asn Pro Val Glu Asn Leu Thr His Val Thr Leu Leu Glu Cys Glu
            340                 345                 350

Glu Gly Asp Pro Asp Asn Ile Asn Ser Thr Ala Lys Val Val Val Pro
        355                 360                 365

Pro Lys Glu Leu Ile Leu Ala Gly Lys Asp Ala Ala Ala Glu Tyr Asp
    370                 375                 380

Glu Leu Ala Glu Pro Gln Asp Phe Gln Asp Asp Pro Asp Ile Val Ala
385                 390                 395                 400

Phe Arg Lys Ala Asn Lys Val Gly Ile Phe Ile Lys Val Thr Pro Gln
                405                 410                 415

Arg Glu Glu Gly Asp Val Thr Val Cys Phe Lys Met Lys His Asp Phe
            420                 425                 430

Lys Asn Leu Ala Ala Pro Ile Arg Pro Met Glu Glu Ser Asp Gln Gly
        435                 440                 445

Thr Glu Val Ile Trp Leu Thr Gln His Val Glu Leu Ser Phe Gly Pro
    450                 455                 460

Leu Leu Pro
465


<210> 118
<211> 2573
<212> DNA
<213> Rattus norvegicus

<220>
<223> Dynactin subunit p62

<220>
<221> misc_feature
<222> 1767
<223> n is a or g or c or t

<400> 118
gcacgaggcc ggactgtgtc acgtgacggg tgcgccgggc gttggctcct ccctcaccaa 60
gatggcgtcc ttgctgcagt cggagcgggt tctctatctc gtccaaggag aaaagaaggt 120
tcgggccccg ctctcacagc tctacttctg ccgctactgc agtgagcttc gatcgctgga 180
atgtgtgtca cacgaggtgg actctcatta ttgtcccagc tgcttagaaa acatgccatc 240
agctgaagcc aaattaaaaa agaatagatg tgccaactgc tttgactgtc ctggatgcat 300
gcacaccctc tccacccgag ctacaagcat ctccacacag cttccagacg acccagccaa 360
aaccactatg aagaaagcct attacctggc ctgtggattt tgtcgatgga catctagaga 420
tgtgggcatg gcagacaaat ctgtagctag tggtggttgg caagaaccag aaaatcctca 480
tgcacaacgg atgaacaaat tgattgaata ttaccagcag cttgctcaga aggaaaaggt 540
tgaacgagat cgcaagaaac tggcacggcg aagaaattac atgcctttgg ctttttcgca 600
acacactatt catgtagtgg ataagtacag ccttgggacc aggcttcagc gaccacgagc 660
tggtgcatcc attagtacac ttgccggact ttcccttaga gaaggagaag accagaagga 720
ggtaaagatt gagccagctc aggccgtcgc tgaagtggag cctctgcctg aagactatta 780
cacacgacca gtgaacctga cagaggtgac cacccttcag cagcgcctct tacagcctga 840
cctgcagcct gtctcagcct cgcagcttta tcctcgacac aagcaccttc tgatcaaacg 900
gtccctgcgc tgtcggaaat gtgaacacaa tttgagcaaa ccagaattta atccaacatc 960
tatcaaattc aaaatccagt tggttgctgt caattatatt ccagaagtga gaatcatgtc 1020
gatccccaat cttcgctaca tgaaggaaag ccaggtcctc ttgactctta caaatccagt 1080
ggagaacctc acccatgtaa ctctgctgga gtgcgaagag ggggaccctg ataacatcaa 1140
cagcactgct aaggtggtgg tgcctcccaa agagctcatc ttagccggca aggatgcagc 1200
agcagagtac gatgagctgg cagagcccca ggacttccag gatgaccctg acattgtagc 1260
tttcagaaaa gccaacaaag taggcatctt catcaaagtc accccacagc gtgaggaagg 1320
ggacgtgacc gtgtgcttca agatgaaaca tgattttaaa aacctggctg cccccatccg 1380
ccctatggaa gaaagcgacc agggcacaga agtcatttgg ctcactcagc atgtggaact 1440

gagctttggc cctcttcttc cttaaaacaa agctacctgg tgggcagatc ccacccagcc 1500
atgtcactac agctctcaac agtgtggaag ctgctgcttc atcagacatg tttataaaga 1560
tgtactactg aatcctgtgg agtggagggc aggccagggt tcggagcaca gggtaactgc 1620
tgttgacacc agtaagcctg tgtctctctg agccctgcac attgagtaac agcatcgttc 1680
catcacagta aaggagtagc tgttccatgg agggcattgt atttactcgc taagaaacgt 1740
gctgtgcgcc tgtcacagtt tgacagnaag ccttagggtg ataatccaga gcagaccttg 1800
tctttgtgcc gcagctttgg tctcggtgtt ccaacggctt tctgtatttc tgtatgccgt 1860
taaggataac cctgaaatca taacccttca ctggccttgg ccctggctct cactttgccg 1920
atgatttcca tttgacagag cagtaggact gtcattgtgt ctctaaccta gtgatggcag 1980
ggcaacctgt cgctttccta aggcactctg tctcttccaa aggaacacag tatacctgtc 2040
acttgattgt cattttcctc attagtatat gggacttgta agtaagaatt tgaatggcaa 2100
gcagttacat gtcatgaaat tcatttgttt taatattacc acaataccca ggggctaagg 2160
ctgcctgcca cagggagaca aactttatga atcttactat ctgccctcct ctgttctgtt 2220
ttttgtttgt ttgtttgttt gtttgttttt cagatgggct cgctccgtgt aactctcgct 2280
ggcctgaact caacctgcta ggactaaaga tccttgcaac atgtccagct cgctgccctg 2340
ttctataaat caagaaattg ctctaacgtt ggggctttta gaaagagctg ggacagttta 2400
tatggacact ttgctgcatc acttctggaa tgcagaaaac attttaaact aacttttagc 2460
atgatccaaa ctaatgttct ttctgctgaa ttgaaggaaa aattaactgt caatccctta 2520
aatttaaaac tggacacaaa gaaaaagaac tttcccttaa tgaaaaaaaa aaa 2573

<210> 119
<211> 1616
<212> PRT
<213> Rattus norvegicus

<220>
<223> Phosphacan

<400> 119
Met Arg Ile Leu Gln Ser Phe Leu Ala Cys Val Gln Leu Leu Cys Val
1               5                   10                  15

Cys Arg Leu Asp Trp Ala Tyr Gly Tyr Tyr Arg Gln Gln Arg Lys Leu
            20                  25                  30

Val Glu Glu Ile Gly Trp Ser Tyr Thr Gly Ala Leu Asn Gln Lys Asn
        35                  40                  45

Trp Gly Lys Lys Tyr Pro Ile Cys Asn Ser Pro Lys Gln Ser Pro Ile
    50                  55                  60

Asn Ile Asp Glu Asp Leu Thr Gln Val Asn Val Asn Leu Lys Lys Leu
65                  70                  75                  80

Lys Phe Gln Gly Trp Glu Lys Pro Ser Leu Glu Asn Thr Phe Ile His
                85                  90                  95

Asn Thr Gly Lys Thr Val Glu Ile Asn Leu Thr Asn Asp Tyr Tyr Leu
            100                 105                 110

Ser Gly Gly Leu Ser Glu Lys Val Phe Lys Ala Ser Lys Met Thr Phe
        115                 120                 125

His Trp Gly Lys Cys Asn Val Ser Ser Glu Gly Ser Glu His Ser Leu
    130                 135                 140

Glu Gly Gln Lys Phe Pro Leu Glu Met Gln Ile Tyr Cys Phe Asp Ala
145                 150                 155                 160

Asp Arg Phe Ser Ser Phe Glu Glu Thr Val Lys Gly Lys Gly Arg Leu

165                 170                 175

Arg Ala Leu Ser Ile Leu Phe Glu Ile Gly Val Glu Glu Asn Leu Asp
            180                 185                 190

Tyr Lys Ala Ile Ile Asp Gly Thr Glu Ser Val Ser Arg Phe Gly Lys
        195                 200                 205

Gln Ala Ala Leu Asp Pro Phe Ile Leu Gln Asn Leu Leu Pro Asn Ser
    210                 215                 220

Thr Asp Lys Tyr Tyr Ile Tyr Asn Gly Ser Leu Thr Ser Pro Pro Cys
225                 230                 235                 240

Thr Asp Thr Val Glu Trp Ile Val Phe Lys Asp Thr Val Ser Ile Ser
                245                 250                 255

Glu Ser Gln Leu Ala Val Phe Cys Glu Val Leu Thr Met Gln Gln Ser
            260                 265                 270

Gly Tyr Val Met Leu Met Asp Tyr Leu Gln Asn Asn Phe Arg Glu Gln
        275                 280                 285

Gln Tyr Lys Phe Ser Arg Gln Val Phe Ser Ser Tyr Thr Gly Lys Glu
    290                 295                 300

Glu Ile His Glu Ala Val Cys Ser Ser Glu Pro Glu Asn Val Gln Ala
305                 310                 315                 320

Asp Pro Glu Asn Tyr Thr Ser Leu Leu Ile Thr Trp Glu Arg Pro Arg
                325                 330                 335

Val Val Tyr Asp Thr Met Ile Glu Lys Phe Ala Val Leu Tyr Gln Pro
            340                 345                 350

Leu Glu Gly Asn Asp Gln Thr Lys His Glu Phe Leu Thr Asp Gly Tyr
        355                 360                 365

Gln Asp Leu Gly Ala Ile Leu Asn Asn Leu Ile Pro Asn Met Ser Tyr
    370                 375                 380

Val Leu Gln Ile Val Ala Ile Cys Ser Asn Gly Leu Tyr Gly Lys Tyr
385                 390                 395                 400

Ser Asp Gln Leu Ile Val Asp Met Pro Thr Glu Asp Ala Glu Leu Asp
                405                 410                 415

Leu Phe Pro Glu Leu Ile Gly Thr Glu Glu Ile Ile Lys Glu Glu Asn
            420                 425                 430

Tyr Gly Lys Gly Asn Glu Glu Asp Thr Gly Leu Asn Pro Gly Arg Asp
        435                 440                 445

Ser Ala Thr Asn Gln Ile Arg Lys Lys Glu Pro Gln Val Ser Thr Thr
    450                 455                 460

Thr His Tyr Asn His Met Gly Thr Lys Tyr Asn Glu Ala Lys Thr Asn
465                 470                 475                 480

Arg Ser Pro Thr Arg Gly Ser Glu Phe Ser Gly Lys Ser Asp Val Leu
                485                 490                 495

Asn Thr Ser Leu Asn Pro Thr Ser Gln Gln Val Ala Glu Phe Asn Pro
            500                 505                 510

Glu Arg Glu Met Ser Leu Pro Ser Gln Ile Gly Thr Asn Leu Pro Pro
        515                 520                 525

His Ser Val Glu Gly Thr Ser Ala Ser Leu Asn Ser Gly Ser Lys Thr
    530                 535                 540

Leu Leu Val Phe Pro Gln Met Asn Leu Ser Gly Thr Ala Glu Ser Leu
545                 550                 555                 560

Asn Met Val Ser Ile Thr Glu Tyr Lys Glu Val Ser Ala Asp Leu Ser
                565                 570                 575

Glu Glu Glu Asn Leu Leu Thr Asp Phe Lys Leu Asp Ser Gly Ala Asp
            580                 585                 590

Asp Ser Ser Gly Ser Ser Pro Ala Ser Ser Thr Val Pro Phe Ser Thr
        595                 600                 605

Asp Asn Leu Ser His Gly Tyr Thr Ser Ser Ser Asp Thr Pro Glu Ala
    610                 615                 620

Val Thr Tyr Asp Val Leu Arg Pro Glu Ser Thr Arg Asn Ala Leu Glu
625                 630                 635                 640

Asp Ser Ala Pro Ser Gly Ser Glu Glu Ser Leu Lys Asp Pro Ser Leu
                645                 650                 655

Glu Gly Ser Val Trp Phe Pro Gly Ser Thr Asp Leu Thr Thr Gln Ser
            660                 665                 670

Glu Thr Gly Ser Gly Arg Glu Gly Phe Leu Gln Val Asn Ser Thr Asp
        675                 680                 685

Phe Gln Val Asp Glu Ser Arg Glu Thr Thr Glu Thr Phe Ser Pro Asp
    690                 695                 700

Ala Thr Ala Ser Arg Gly Pro Ser Val Thr Asp Met Glu Met Pro His
705                 710                 715                 720

Tyr Ser Thr Phe Ala Tyr Pro Pro Thr Glu Val Thr Ser His Ala Phe
                725                 730                 735

Thr Pro Ser Ser Arg Pro Leu Asp Leu Ala Pro Thr Ser Asn Ile Leu
            740                 745                 750

His Ser Gln Thr Thr Gln Pro Val Tyr Asn Gly Glu Thr Pro Leu Gln
        755                 760                 765

Pro Ser Tyr Ser Ser Glu Val Phe Pro Leu Val Thr Pro Leu Leu Leu
    770                 775                 780

Asp Asn Gln Thr Leu Asn Thr Thr Pro Ala Ala Ser Ser Ser Asp Ser
785                 790                 795                 800

Ala Leu His Ala Thr Pro Val Phe Pro Ser Val Gly Val Ser Phe Asp
                805                 810                 815

Ser Ile Leu Ser Ser Tyr Asp Asp Ala Pro Leu Leu Pro Phe Ser Ser
                820                 825                 830

Ala Ser Phe Ser Ser Asp Leu Phe His His Leu His Thr Val Ser Gln
        835                 840                 845

Thr Leu Pro Gln Val Thr Ser Ala Ala Glu Arg Asp Glu Leu Ser Leu
    850                 855                 860

His Ala Ser Leu Leu Val Ala Gly Gly Asp Leu Leu Leu Glu Pro Ser
865                 870                 875                 880

Leu Val Gln Tyr Ser Asp Val Met Ser His Gln Val Thr Ile His Ala
                885                 890                 895

Ala Ser Asp Thr Leu Glu Phe Gly Ser Glu Ser Ala Val Leu Tyr Lys
            900                 905                 910

Thr Ser Met Val Ser Gln Ile Glu Ser Pro Ser Ser Asp Val Val Met
        915                 920                 925

His Ala Tyr Ser Ser Gly Pro Glu Thr Ser Tyr Ala Ile Glu Gly Ser
    930                 935                 940

His His Val Leu Thr Val Ser Ser Ser Ser Ala Ile Pro Val His Asp
945                 950                 955                 960

Ser Val Gly Val Ala Asp Gln Gly Ser Leu Leu Ile Asn Pro Ser His
                965                 970                 975

Ile Ser Leu Pro Glu Ser Ser Phe Ile Thr Pro Thr Ala Ser Leu Leu
            980                 985                 990

Gln Leu Pro Pro Ala Leu Ser Gly Asp Gly Glu Trp Ser Gly Ala Ser
        995                 1000                1005

Ser Asp Ser Glu Leu Leu Leu Pro Asp Thr Asp Gly Leu Arg Thr Leu
    1010                1015                1020

Asn Met Ser Ser Pro Val Ser Val Ala Asp Phe Thr Tyr Thr Thr Ser
1025                1030                1035                1040

Val Ser Gly Asp Asp Ile Lys Pro Leu Ser Lys Gly Glu Met Met Tyr
                1045                1050                1055

Gly Asn Glu Thr Glu Leu Lys Met Ser Ser Phe Ser Asp Met Ala Tyr
            1060                1065                1070

Pro Ser Lys Ser Thr Val Val Pro Lys Met Ser Asp Ile Val Asn Lys
        1075                1080                1085

Trp Ser Glu Ser Leu Lys Glu Thr Ser Val Ser Val Ser Ser Ile Asn
    1090                1095                1100

Ser Val Phe Thr Glu Ser Leu Val Tyr Pro Ile Thr Lys Val Phe Asp
1105                1110                1115                1120

Gln Glu Ile Ser Arg Val Pro Glu Ile Ile Phe Pro Val Lys Pro Thr
                1125                1130                1135

His Thr Ala Ser Gln Ala Ser Gly Asp Thr Trp Leu Lys Pro Gly Leu

1140                1145                1150

Ser Thr Asn Ser Glu Pro Ala Leu Ser Asp Thr Ala Ser Ser Glu Val
        1155                1160                1165

Ser His Pro Ser Thr Gln Pro Leu Leu Tyr Glu Ala Ala Ser Pro Phe
    1170                1175                1180

Asn Thr Glu Ala Leu Leu Gln Pro Ser Phe Pro Ala Ser Asp Val Asp
1185                1190                1195                1200

Thr Leu Leu Lys Thr Ala Leu Pro Ser Gly Pro Arg Asp Pro Val Leu
                1205                1210                1215

Thr Glu Thr Pro Met Val Glu Gln Ser Ser Ser Ser Val Ser Leu Pro
            1220                1225                1230

Leu Ala Ser Glu Ser Ala Ser Ser Lys Ser Thr Leu His Phe Thr Ser
        1235                1240                1245

Val Pro Val Leu Asn Met Ser Pro Ser Asp Val His Pro Thr Ser Leu
    1250                1255                1260

Gln Arg Leu Thr Val Pro His Ser Arg Glu Glu Tyr Phe Glu Gln Gly
1265                1270                1275                1280

Leu Leu Lys Ser Lys Ser Pro Gln Gln Val Leu Pro Ser Leu His Ser
                1285                1290                1295

His Asp Glu Phe Phe Gln Thr Ala His Leu Asp Ile Ser Gln Ala Tyr
            1300                1305                1310

Pro Pro Lys Gly Arg His Ala Phe Ala Thr Pro Ile Leu Ser Ile Asn
        1315                1320                1325

Glu Pro Gln Asn Thr Leu Ile Asn Arg Leu Val Tyr Ser Glu Asp Ile
    1330                1335                1340

Phe Met His Pro Glu Ile Ser Ile Thr Asp Lys Ala Leu Thr Gly Leu
1345                1350                1355                1360

Pro Thr Thr Val Ser Asp Val Leu Ile Ala Thr Asp His Ser Val Pro
                1365                1370                1375

Leu Gly Ser Gly Pro Ile Ser Met Thr Thr Val Ser Pro Asn Arg Asp
            1380                1385                1390

Asp Ser Val Thr Thr Thr Lys Leu Leu Leu Pro Ser Lys Ala Thr Ser
        1395                1400                1405

Lys Pro Thr His Ser Ala Arg Ser Asp Ala Asp Leu Val Gly Gly Gly
    1410                1415                1420

Glu Asp Gly Asp Asp Tyr Asp Asp Asp Asp Tyr Asp Asp Ile Asp Ser
1425                1430                1435                1440

Asp Arg Phe Pro Val Asn Lys Cys Met Ser Cys Ser Pro Tyr Arg Glu
                1445                1450                1455

Ser Gln Glu Lys Val Met Asn Asp Ser Asp Thr Gln Glu Ser Ser Leu
            1460                1465                1470

Val Asp Gln Ser Asp Pro Ile Ser His Leu Leu Ser Glu Asn Thr Glu
        1475                1480                1485

Glu Glu Asn Gly Gly Thr Gly Val Thr Arg Val Asp Lys Ser Pro Asp
    1490                1495                1500

Lys Ser Pro Pro Pro Ser Met Leu Pro Gln Lys His Asn Asp Gly Arg
1505                1510                1515                1520

Glu Asp Arg Asp Ile Gln Met Gly Ser Ala Val Leu Pro His Thr Pro
                1525                1530                1535

Gly Ser Lys Ala Trp Ala Val Leu Thr Ser Asp Glu Glu Ser Gly Ser
            1540                1545                1550

Gly Gln Gly Thr Ser Asp Ser Leu Asn Asp Asn Glu Thr Ser Thr Asp
        1555                1560                1565

Phe Ser Phe Pro Asp Val Asn Glu Lys Asp Ala Asp Gly Val Leu Glu
    1570                1575                1580

Ala Asp Asp Thr Gly Ile Ala Pro Gly Ser Pro Arg Ser Ser Thr Pro
1585                1590                1595                1600

Ser Val Thr Ser Gly His Ser Gly Val Ser Asn Ser Ser Glu Ala Gly
                1605                1610                1615

<210> 120
<211> 6801
<212> DNA
<213> Rattus norvegicus

<220>
<223> Phosphacan

<400> 120
cctagaccct ggccagtcac cggcgtcccc tgtctcggtg ttcccactct ctgcacccta 60
agctgtaccc ctgcggctgg cgaggggccg cggacccggc tggagatgcg aatcctgcag 120
agcttcctcg cgtgcgttca gctactgtgc gtgtgtcgcc tggactgggc ttatggatac 180
tacagacaac agagaaaact tgttgaagag attggctggt cctatacagg agcactaaat 240
caaaaaaatt ggggaaagaa atatccaata tgtaatagcc caaagcagtc tcctattaat 300
attgatgaag atcttacaca agtaaatgtg aatcttaaga aactgaaatt tcagggttgg 360
gaaaaaccat ccttggaaaa cacgtttatt cacaacactg ggaaaacagt ggaaataaat 420
ctcaccaatg actactatct cagtggagga ctttcagaaa aggtcttcaa ggcaagcaaa 480
atgacttttc actgggggaa atgcaatgtg tcatcggaag gatcggaaca cagcttagaa 540
gggcagaagt ttccactcga gatgcaaatc tactgcttcg atgcggaccg attttcaagt 600
tttgaggaaa cagttaaagg aaaaggaagg ttaagggctc tatccatttt atttgagatt 660
ggagttgaag aaaatttgga ttacaaagcc attattgatg ggactgaaag tgttagtcgt 720
tttggaaagc aggctgcctt agatccgttc atcttgcaga acctcctgcc aaactccact 780
gacaagtact acatttacaa cggatcactg acatcccctc cctgcacaga caccgtcgaa 840
tggattgttt ttaaggatac agttagcatc tctgaaagcc agctggctgt attttgtgaa 900
gttctcacaa tgcaacagtc cgggtatgtc atgttgatgg attacttaca aaacaatttc 960
cgagaacaac agtacaagtt ttccaggcag gtgttttctt cgtacactgg gaaggaagag 1020
attcatgaag cagtgtgcag ttcagaacca gaaaatgtgc aagctgaccc tgagaattac 1080
accagccttc tgatcacatg ggaaaggcct cgggttgttt atgacacaat gattgagaag 1140
tttgcggttc tgtaccagcc actggaggga aacgaccaaa ccaagcatga gtttttaaca 1200

gatggctatc aggacttggg tgccattctc aataacttaa tacctaacat gagttatgtg 1260
ctccaaatag tggccatatg ctctaatggc ctttatggaa agtacagtga ccaattgata 1320
gtcgacatgc ctactgagga tgctgaactt gacctctttc ctgaattaat tggaactgaa 1380
gaaataatca aggaggaaaa ctatggaaaa ggcaatgaag aagacactgg cttgaatccc 1440
ggtagagaca gtgccacaaa ccaaataagg aaaaaggaac cccaggtttc taccacaact 1500
cactataatc acatggggac taaatataat gaagccaaga ctaaccgatc tccaacgaga 1560
ggatctgaat tctctggaaa gagtgatgtt ctcaacacat ccctgaatcc tacttcccaa 1620
caggttgctg aattcaatcc agaaagagaa atgtccttgc cttctcagat tggaactaac 1680
ctgccaccac acagtgtgga aggcacctca gcctccttaa acagtggctc taaaactctc 1740
cttgtcttcc cacagatgaa cttgtctggg actgcagaat ccttaaatat ggtttccata 1800
acagagtaca aagaggtgtc tgctgacctc agtgaggaag aaaacttact gactgatttc 1860
aagctcgata gtggagcaga tgattcgtca ggctctagcc ctgcatcctc cactgtcccc 1920
ttttccacag ataatctatc ccatggatat acgtcttctt cagacacgcc cgaggcggtc 1980
acgtatgatg tccttaggcc agaatctacg agaaatgctc tagaggattc ggctccatca 2040
ggttcagaag aatcactaaa ggatccctct cttgaaggga gtgtgtggtt ccctggatcc 2100
acagacctaa caacacagtc tgagactgga tctgggagag agggctttct ccaagttaac 2160
tccacggact tccaagttga tgaatcgagg gagacaactg agacattttc tccagatgct 2220
accgcgtccc ggggtccttc ggtcacagat atggaaatgc cacattattc tacctttgcc 2280
taccccccga ctgaagtaac atcacatgct ttcactccgt cctccagacc acttgatttg 2340
gctcccacta gcaacatcct ccattcgcag acaactcaac cagtatacaa tggtgagaca 2400
cctcttcaac cttcctacag tagtgaagtc tttcctctag tcaccccttt gttgcttgac 2460
aatcagaccc tcaacactac ccctgctgct tcaagtagtg attcggcctt gcatgctacg 2520
cctgtattcc ccagtgttgg tgtgtcattt gactccatcc tgtcttccta tgatgatgca 2580
cctctgctcc cattttcctc tgcttccttc agtagtgact tgtttcacca tctgcatacg 2640
gtttctcaaa cccttccgca agttacttca gctgctgaga gggatgagct gtctttgcat 2700
gcttctctgc tggtggctgg gggtgatttg ctgttagagc ccagccttgt tcagtattct 2760
gatgtgatgt cacatcaggt cactattcat gctgcttcgg acacattgga atttggtagt 2820
gagtctgctg tcctttataa aacgtctatg gtttctcaaa tcgaatcacc cagcagtgat 2880
gtcgttatgc atgcatattc gtcagggcct gaaacttctt atgccattga gggctcccac 2940
cacgtgctca ctgtttcttc cagttctgca atacctgtgc atgattctgt cggtgtagct 3000
gatcaggggt ccttacttat caatcctagc catatatcac tgcctgagtc ctcatttatt 3060
actccaactg catcattact gcagcttcct cctgccctct ctggtgatgg ggagtggtct 3120
ggagcctcct ctgatagtga attgctttta cctgacacag atgggctgag aactcttaac 3180
atgtcttcac ctgtttctgt agctgatttt acatacacga catctgtgtc tggcgatgat 3240
attaagccgc tttctaaagg tgaaatgatg tatggaaatg agaccgaact gaaaatgtct 3300
tctttcagtg acatggcata cccttctaaa agcacagtcg tgccaaagat gtctgatatt 3360
gtaaataagt ggagtgaatc tttaaaagaa acctctgttt ccgtatctag cataaacagc 3420
gtgtttacag agtctcttgt ttatcccata actaaggttt ttgatcagga gattagtcga 3480
gttccagaga ttatcttccc agttaaacct acacacacag catctcaagc atctggtgac 3540
acttggctta aacccgggct tagcacaaac tcagagcctg cgctctctga cactgcttct 3600
agtgaagtat cacacccttc aacacagccc ttgctctatg aggccgcatc tccttttaat 3660
acggaagcat tgctgcaacc ttcctttccg gcttctgatg ttgacacctt gcttaaaact 3720
gcccttccct ctgggcctcg tgatccagtg ctgactgaaa cccccatggt tgagcaaagt 3780
agctcttccg tatctcttcc cctggcatca gagtctgctt caagcaaaag cacgctgcac 3840
tttacatctg taccagttct caatatgtca ccttctgatg tgcaccccac ttcacttcaa 3900
cgcttaacag ttcctcactc gagggaggaa tattttgaac aaggtttgct taagagcaaa 3960
agtccccagc aagtcctgcc gtccttgcac agccatgacg agtttttcca aactgcacat 4020
ctggacatta gccaggccta ccctccaaaa ggaaggcatg catttgctac tcctatttta 4080
tcaatcaatg aaccacaaaa tacacttata aacaggcttg tgtattctga ggacattttc 4140
atgcaccctg aaatttctat tactgataag gcacttactg gtctaccaac gaccgtttct 4200
gatgtactta tagctactga ccattctgtt ccattaggaa gtgggcccat ttccatgaca 4260
actgtttctc ccaacagaga tgattctgtg accacaacca agttgcttct tccttctaaa 4320
gctacttcta agccgactca tagtgccaga tctgatgccg atttagtagg aggtggtgaa 4380
gatggtgatg actatgatga tgatgattat gatgacatag atagtgatcg ctttcccgta 4440
aataagtgta tgtcatgttc accctataga gaatcacagg aaaaggtaat gaatgactcg 4500
gacacccaag aaagcagtct tgtggatcag agtgacccaa tttcacattt gctctctgag 4560
aataccgaag aagaaaatgg aggcacgggt gtaactaggg tggataaaag tcctgataag 4620
tcaccaccac caagtatgct accccagaag cacaatgatg gaagagagga ccgtgacatt 4680
cagatgggta gtgctgtcct tcctcacacc ccaggatcta aagcatgggc agttttgaca 4740
agtgatgaag agagtgggtc agggcaaggc acctcagata gccttaatga taatgagact 4800
tccacagatt tcagtttccc agatgttaat gaaaaggatg ctgatggtgt cctggaagca 4860

gatgacacag gcatagctcc gggatctcca cggtcctcca caccatctgt tactagtggg 4920
cattcaggag tatccaacag ttcagaggca ggttagttat gagcaaaggg atagaacgag 4980
atgtgctgat tttcttgcta tgaaagtaaa aatagaagag tcacggaaga aaagaattct 5040
ggtccaccag cagatcttca ctttctaatc agaacgttca atacatttat agctcttata 5100
aatatggttt tactcatgat ttcgaatgaa tatccaacat attttggtta ttgattttta 5160
ttatcttcaa aatgcattgt gtgctgttac taatccctat tcctattgtg tgtagtatca 5220
actatgtatg cataatacgc cattagaata ttacaacgca gtgtgctgtg ttaagacagt 5280
ttaaatgttc atacttgtat tctgaaacaa gttgtacaca aactctattg gatttaaaat 5340
ctcagtaata tgctataagc acccatccac caatggtgga cacttcacat gtgcacaaca 5400
gcttggagag cttctcaaaa gaactacagg gctaagattt gatagctaag gaaggttttt 5460
ataaacatca tactgctttt aaactatcct tgagcttctt ttttgttgcc taaagagggc 5520
agtataaacc accaaaatac attctacttt gtgtttgtta cagtattcca acttactaat 5580
cgacatgaat atgcagaaga tgcataggta ttgtatgaca agatgtaaaa gaaaaagcct 5640
acagcctttt ctgtagagat gcatttcatt gttaatatat tttatgcaaa tgggtttatt 5700
agccagatgt ttcttggagc agactgcaac ctagaataca gatgtgtgct ttaaatgtca 5760
atggtaacaa aattcgaaat tccattaagt gtttaaaaaa aaaactaggt gattccattg 5820
aattcacggc aagaatttta taatttgctt tgattaagtg atgaggtttt tttttaatgc 5880
acaaggaaag tatttaaatc ttagcaagtc aaaagataag ataatgtata atgtgacatt 5940
gggtaataat tgcacagagc ttaatattaa aaatccacag tatagagcca atctcattaa 6000
aatatatatt ctctttgtta ttttcatcat tcacattagt tgtaaactct ttagtatggc 6060
agcaattaag agattatata tttagataag acagtttctt tcttttctgt attctgacgt 6120
gatctccact taacgttgaa tttcagcttc aatatatgaa ctgatgtgtg agaaggagta 6180
atgagtccat gggtcactgt agtcaggaat gttgttacat atgtgaattg ttatctgtcc 6240
tcaattatag cacttgtttt tctttctata tttttagatt tatctgaaag ggaaagatcg 6300
tgaacatact ctaatggaca gaccatgcat atacatatat cccaaagcac tatatctaga 6360
attatcgaag aaagttattt ctcttagatg tactaattag atttatttaa ctgttaagag 6420
taagttcccc cactaatgaa aacacagcaa tatggataaa acatccatct tcaggaaata 6480
atacagttaa tgaagggagt aggatcaaat agaattaggg aaaacaattt ttacctgttt 6540
ttaaagctgt atctgtaaaa ctaattcaaa gtcagtctta tccaattatt gaatattccc 6600
tagtctgaaa ctcatcattt atggtacttc taaattgtag aaaaactgac atgatgtttc 6660
ctaaactaat ataaataggg caaatataga tgagagtgaa caaagactgt aagatacttg 6720
ctctggttca cataaagagt ccatccaaaa ctaaccctgg gaatgtatta ttagtgtctg 6780
tctcttagaa catgcttaca t 6801

<210> 121
<211> 530
<212> PRT
<213> Homo sapiens

<220>
<223> EP70-P-iso

<400> 121
Met Arg Pro Leu Pro Gly Ala Leu Gly Val Ala Ala Ala Ala Leu Trp
1               5                   10                  15

Leu Leu Leu Leu Leu Leu Pro Arg Thr Arg Ala Asp Glu His Glu His
            20                  25                  30

Thr Tyr Gln Asp Lys Glu Glu Val Val Leu Trp Met Asn Thr Val Gly
        35                  40                  45

Pro Tyr His Asn Arg Gln Glu Thr Tyr Lys Tyr Phe Ser Leu Pro Phe
    50                  55                  60

Cys Val Gly Ser Lys Lys Ser Ile Ser His Tyr His Glu Thr Leu Gly
65                  70                  75                  80

Glu Ala Leu Gln Gly Val Glu Leu Glu Phe Ser Gly Leu Asp Ile Lys
                85                  90                  95

Phe Lys Asp Asp Val Met Pro Ala Thr Tyr Cys Glu Ile Asp Leu Asp
            100                 105                 110

Lys Glu Lys Arg Asp Ala Phe Val Tyr Ala Ile Lys Asn His Tyr Trp
        115                 120                 125

Tyr Gln Met Tyr Ile Asp Asp Leu Pro Ile Trp Gly Ile Val Gly Glu
    130                 135                 140

Ala Asp Glu Asn Gly Glu Asp Tyr Tyr Leu Trp Thr Tyr Lys Lys Leu
145                 150                 155                 160

Glu Ile Gly Phe Asn Gly Asn Arg Ile Val Asp Val Asn Leu Thr Ser
                165                 170                 175

Glu Gly Lys Val Lys Leu Gly Ser Lys Tyr Tyr Asn Pro Asp Val Ile
            180                 185                 190

Phe Ser Lys Met Glu Lys Ser Asp Val Lys Phe Glu Asp Arg Phe Asp
        195                 200                 205

Asn Ile Leu Ile Val Leu Phe Ser His Arg Ile His Trp Phe Ser Ile
    210                 215                 220

Phe Asn Ser Phe Met Met Val Ile Phe Leu Val Gly Leu Val Ser Met
225                 230                 235                 240

Ile Leu Met Arg Thr Leu Arg Lys Asp Tyr Ala Arg Tyr Ser Lys Glu
                245                 250                 255

Glu Glu Met Asp Asp Met Asp Arg Asp Leu Gly Asp Glu Tyr Gly Trp
            260                 265                 270

Lys Gln Val His Gly Asp Val Phe Arg Pro Ser Ser His Pro Leu Ile
        275                 280                 285

Phe Ser Ser Leu Ile Gly Ser Gly Cys Gln Ile Phe Ala Val Ser Leu
    290                 295                 300

Ile Val Ile Ile Val Ala Met Ile Glu Asp Leu Tyr Thr Glu Arg Gly
305                 310                 315                 320

Ser Met Leu Ser Thr Ala Ile Phe Val Tyr Ala Ala Thr Ser Pro Val
                325                 330                 335

Asn Gly Tyr Phe Gly Gly Ser Leu Tyr Ala Arg Gln Gly Gly Arg Arg
            340                 345                 350

Trp Ile Lys Gln Met Phe Ile Gly Ala Phe Leu Ile Pro Ala Met Gly
        355                 360                 365

Val His Cys Leu Leu His Gln Phe His Ser His Leu Leu Pro Cys Phe
    370                 375                 380

Lys Ser His Ser Phe Trp Asn Asn Gly Gly Arg Leu Leu His Leu Phe
385                 390                 395                 400

Phe Cys Tyr Ser Ser Ser Lys Ser Cys Trp Tyr Asn Thr Trp Pro Lys
                405                 410                 415

Ser Val Arg Ser Ala Gln Leu Ser Leu Ser Cys Gln Cys Cys Ala Ser
            420                 425                 430

Ser Tyr Thr Gly Glu Lys Met Val His Gly Ala Ala Val Ile Val Cys
        435                 440                 445

Leu Gly Gly Ile Leu Pro Phe Gly Ser Ile Phe Ile Glu Met Tyr Phe
    450                 455                 460

Ile Phe Thr Ser Phe Trp Ala Tyr Lys Ile Tyr Tyr Val Tyr Gly Phe
465                 470                 475                 480

Met Met Leu Val Leu Val Ile Leu Cys Ile Val Thr Val Cys Val Thr
                485                 490                 495

Ile Val Cys Thr Tyr Phe Leu Leu Asn Ala Glu Asp Tyr Arg Trp Gln
            500                 505                 510

Trp Thr Ser Phe Leu Ser Ala Ala Ser Thr Ala Ile Tyr Val Tyr Met
        515                 520                 525

Tyr Ser
    530

<210> 122
<211> 3389
<212> DNA
<213> Homo sapiens

<220>
<223> EP70-P-iso

<400> 122
ggcacgaggg gcgcggagga tgaggccgct gcctggcgct cttggcgtgg cggccgccgc 60
gctgtggctg ctgctgctgc tgctgccccg gacccgggcg gacgagcacg aacacacgta 120
tcaagataaa gaggaagttg tcttatggat gaatactgtt gggccctacc ataatcgtca 180
agaaacatat aagtactttt cacttccatt ctgtgtgggg tcaaaaaaaa gtatcagtca 240
ttaccatgaa actctgggag aagcacttca aggggttgaa ttggaattta gtggtctgga 300
tattaaattt aaagatgatg tgatgccagc cacttactgt gaaattgatt tagataaaga 360
aaagagagat gcatttgtat atgccataaa aaatcattac tggtaccaga tgtacataga 420
tgatttacca atatggggta ttgttggtga ggctgatgaa aatggagaag attactatct 480
ttggacctat aaaaaacttg aaataggttt taatggaaat cgaattgttg atgttaatct 540
aactagtgaa ggaaaggtga aactgggttc caaatactat aatccagatg tcatattcag 600
taaaatggaa aagtcagatg tgaaatttga agatcgattt gacaatatct tgatcgtcct 660
tttttcacat cggattcatt ggttttcaat tttcaactcc ttcatgatgg tgatcttctt 720
ggtgggctta gtttcaatga ttttaatgag aacattaaga aaagattatg ctcggtacag 780
taaagaggaa gaaatggatg atatggatag agacctagga gatgaatatg gatggaaaca 840
ggtgcatgga gatgtattta gaccatcaag tcacccactg atattttcct ctctgattgg 900
ttctggatgt cagatatttg ctgtgtctct catcgttatt attgttgcaa tgatagaaga 960
tttatatact gagaggggat caatgctcag tacagccata tttgtctatg ctgctacgtc 1020
tccagtgaat ggttattttg gaggaagtct gtatgctaga caaggaggaa ggagatggat 1080
aaagcagatg tttattgggg cattccttat cccagctatg ggtgtgcact gccttcttca 1140
tcaatttcat agccatttat taccatgctt caagagccat tccttttgga acaatggtgg 1200
ccgtttgttg catctgtttt tttgttattc ttcctctaaa tcttgttggt acaatacttg 1260
gccgaaatct gtcaggtcag cccaactttc cttgtcgtgt caatgctgtg cctcgtccta 1320
taccggagaa aaaatggttc atggagctgc ggttattgtt tgcctgggtg gaattttacc 1380
ttttggttca atctttattg aaatgtattt catcttcacg tctttctggg catataagat 1440
ctattatgtc tatggcttca tgatgctggt gctggttatc ctgtgcattg tgactgtctg 1500
tgtgactatt gtgtgcacat attttctact aaatgcagaa gattaccggt ggcaatggac 1560
aagttttctc tctgctgcat caactgcaat ctatgtttac atgtattcct tttactacta 1620

ttttttcaaa acaaagatgt atggcttatt tcaaacatca ttttactttg gatatatggc 1680
ggtatttagc acagccttgg ggataatgtg tggagcgatt ggttacatgg gaacaagtgc 1740
ctttgtccga aaaatctata ctaatgtgaa aattgactag agacccaaga aaacctggaa 1800
ctttggatca atttcttttt cataggggtg gaacttgcac agcaaaaaca aacaaacgca 1860
agaagagatt tgggctttaa cacactgggt actttgtggg tctctctttc gtcggtggct 1920
taaagtaaca tctatttcca ttgatcctag gttcttcctg actgctttct ccaactgttc 1980
acagcaaatg cttggatttt atgcagtagg cattactaca gtacatggct aatcttccca 2040
aaaactagct cattaaagat gaaatagacc agctctcttc agtgaagagg acaaatagtt 2100
tatttaaagc atttgttcca ataaaataaa tagagggaaa cttggatgct aaaattacat 2160
gaataggaat cttcctggca cttagtgttt ctatgttatt gaaaaatgat gttccagaaa 2220
gattactttt ttcctcttat ttttactgcc attgtcgacc tattgtggga catttttata 2280
tattgaatct gggttctttt ttgacttttt ttttttccca atccaacagc atcctttttt 2340
ttaaaagaga gaattagaaa atattaaatc ctgcatgtaa tatatctgct gtcatcttag 2400
ttggaccaac ttcccattta tttatcttaa aactatacag ttacatctta attccatcca 2460
aagaagatac agtttgaaga cagaagtgta ctctctacaa tgcaatttac tgtacagtta 2520
gaaagcaaag tgttaaatgg agaagatact tgtttttatt aaacattttg agatttagat 2580
aaactacatt ttaactgaat gtctaaagtg attatctttt ttccccccaa gttagtctta 2640
aatcttttgg gtttgaatga aggttttaca taagaaatta ttaaaaacaa ggggggtggg 2700
taataaatgt atataacatt aaataatgta acgtaggtgt agattcccaa atgcatttgg 2760
atgtacagat cgactacaga gtactttttt cttatgatga ttggtgtaga aatgtgtgat 2820
ttgggtgggc ttttacatct tgcctaccat tgcatgaaac attggggttt cttcaaaatg 2880
tgtgtgtcat acttcttttg ggaggggggt tgttttcttc tgtttatttt ctgagactcc 2940
tacaggagcc aaatttgtaa tttagagaca cttaattttg ttaatcctgt ctgggacact 3000
taagtaacat ctaaagcatt attgctttag aatgttcaaa taaaatttcc tgaccaaatt 3060
gttttgtgga aatagatgtg tttgcaattt gaagatatct ttctgtccag aaggcaaaat 3120
taccgaatgc catttttaaa agtatgctat aaactatgct actctcatac aggggacccg 3180
tattttaaaa tctccagact tgcttacatc tagattatcc agcacaatca taaagtgaat 3240
gacaaaccct ttgaatgaaa ttgtggcaca aaatctgttc aggttggtgt accgtgtaaa 3300
gtggggatgg ggtaaaagtg gttaacgtac tgttggatca acaaataaag gttacagttt 3360
tgtaagagaa aaaaaaaaaa aaaaaaaaa 3389

<210> 123
<211> 1027
<212> PRT
<213> Drosophila melanogaster

<220>
<223> ISWI protein

<400> 123
Met Ser Lys Thr Asp Thr Ala Ala Val Glu Ala Thr Glu Glu Asn Ser
1               5                   10                  15

Asn Glu Thr Thr Ser Asp Ala Ala Thr Ser Ser Ser Gly Glu Lys Glu
            20                  25                  30

Ala Glu Phe Asp Asn Lys Ile Glu Ala Asp Arg Ser Arg Arg Phe Asp
        35                  40                  45

Phe Leu Leu Lys Gln Thr Glu Ile Phe Thr His Phe Met Thr Asn Ser
    50                  55                  60

Ala Lys Ser Pro Thr Lys Pro Lys Gly Arg Pro Lys Lys Ile Lys Asp
65                  70                  75                  80

Lys Asp Lys Glu Lys Asp Val Ala Asp His Arg His Arg Lys Thr Glu
                85                  90                  95

Gln Glu Glu Asp Glu Glu Leu Leu Ala Glu Asp Ser Ala Thr Lys Glu
            100                 105                 110

Ile Phe Arg Phe Asp Ala Ser Pro Ala Tyr Ile Lys Ser Gly Glu Met
        115                 120                 125

Arg Asp Tyr Gln Ile Arg Gly Leu Asn Trp Met Ile Ser Leu Tyr Glu
    130                 135                 140

Asn Gly Ile Asn Gly Ile Leu Ala Asp Glu Met Gly Leu Gly Lys Thr
145                 150                 155                 160

Leu Gln Thr Ile Ser Leu Leu Gly Tyr Leu Lys His Phe Lys Asn Gln
                165                 170                 175

Ala Gly Pro His Ile Val Ile Val Pro Lys Ser Thr Leu Gln Asn Trp
            180                 185                 190

Val Asn Glu Phe Lys Lys Trp Cys Pro Ser Leu Arg Ala Val Cys Leu
        195                 200                 205

Ile Gly Asp Gln Asp Thr Arg Asn Thr Phe Ile Arg Asp Val Leu Met
    210                 215                 220

Pro Gly Glu Trp Asp Val Cys Val Thr Ser Tyr Glu Met Cys Ile Arg
225                 230                 235                 240

Glu Lys Ser Val Phe Lys Lys Phe Asn Trp Arg Tyr Leu Val Ile Asp
                245                 250                 255

Glu Ala His Arg Ile Lys Asn Glu Lys Ser Lys Leu Ser Glu Ile Leu
            260                 265                 270

Arg Glu Phe Lys Thr Ala Asn Arg Leu Leu Ile Thr Gly Thr Pro Leu
        275                 280                 285

Gln Asn Asn Leu His Glu Leu Trp Ala Leu Leu Asn Phe Leu Leu Pro
    290                 295                 300

Asp Val Phe Asn Ser Ser Glu Asp Phe Asp Glu Trp Phe Asn Thr Asn
305                 310                 315                 320

Thr Cys Leu Gly Asp Asp Ala Leu Ile Thr Arg Leu His Ala Val Leu
                325                 330                 335

Lys Pro Phe Leu Leu Arg Arg Leu Lys Ala Glu Val Glu Lys Arg Leu
            340                 345                 350

Lys Pro Lys Lys Glu Met Lys Ile Phe Val Gly Leu Ser Lys Met Gln
        355                 360                 365

Arg Asp Trp Tyr Thr Lys Val Leu Leu Lys Asp Ile Asp Val Val Asn
    370                 375                 380

Gly Ala Gly Lys Val Glu Lys Met Arg Leu Gln Asn Ile Leu Met Gln
385                 390                 395                 400

Leu Arg Lys Cys Thr Asn His Pro Tyr Leu Phe Asp Gly Ala Glu Pro
                405                 410                 415

Gly Pro Pro Tyr Thr Thr Asp Thr His Leu Val Tyr Asn Ser Gly Lys
            420                 425                 430

Met Ala Ile Leu Asp Lys Leu Leu Pro Lys Leu Gln Glu Gln Gly Ser
        435                 440                 445

Arg Val Leu Ile Phe Ser Gln Met Thr Arg Met Leu Asp Ile Leu Glu
    450                 455                 460

Asp Tyr Cys His Trp Arg Asn Tyr Asn Tyr Cys Arg Leu Asp Gly Gln
465                 470                 475                 480

Thr Pro His Glu Asp Arg Asn Arg Gln Ile Gln Glu Phe Asn Met Asp
                485                 490                 495

Asn Ser Ala Lys Phe Leu Phe Met Leu Ser Thr Arg Ala Gly Gly Leu
            500                 505                 510

Gly Ile Asn Leu Ala Thr Ala Asp Val Val Ile Ile Tyr Asp Ser Asp
        515                 520                 525

Trp Asn Pro Gln Met Asp Leu Gln Ala Met Asp Arg Ala His Arg Ile
    530                 535                 540

Gly Gln Lys Lys Gln Val Arg Val Phe Arg Leu Ile Thr Glu Ser Thr
545                 550                 555                 560

Val Glu Glu Lys Ile Val Glu Arg Ala Glu Val Lys Leu Arg Leu Asp
                565                 570                 575

Lys Met Val Ile Gln Gly Gly Arg Leu Val Asp Asn Arg Ser Asn Gln
            580                 585                 590

Leu Asn Lys Asp Glu Met Leu Asn Ile Ile Arg Phe Gly Ala Asn Gln
        595                 600                 605

Val Phe Ser Ser Lys Glu Thr Asp Ile Thr Asp Glu Asp Ile Asp Val
    610                 615                 620

Ile Leu Glu Arg Gly Glu Ala Lys Thr Ala Glu Gln Lys Ala Ala Leu
625                 630                 635                 640

Asp Ser Leu Gly Glu Ser Ser Leu Arg Thr Phe Thr Met Asp Thr Asn
                645                 650                 655

Gly Glu Ala Gly Thr Ser Ser Val Tyr Gln Phe Glu Gly Glu Asp Trp
            660                 665                 670

Arg Glu Lys Gln Lys Leu Asn Ala Leu Gly Asn Trp Ile Glu Pro Pro
        675                 680                 685

Lys Arg Glu Arg Lys Ala Asn Tyr Ala Val Asp Ala Tyr Phe Arg Glu
    690                 695                 700

Ala Leu Arg Val Ser Glu Pro Lys Ala Pro Lys Ala Pro Arg Pro Pro
705                 710                 715                 720

Lys Gln Pro Ile Val Gln Asp Phe Gln Phe Phe Pro Pro Arg Leu Phe
                725                 730                 735

Glu Leu Leu Asp Gln Glu Ile Tyr Tyr Phe Arg Lys Thr Val Gly Tyr
            740                 745                 750

Lys Val Pro Lys Asn Thr Glu Leu Gly Ser Asp Ala Thr Lys Val Gln

        755                 760                 765

Arg Glu Glu Gln Arg Lys Ile Asp Glu Ala Glu Pro Leu Thr Glu Glu
    770                 775                 780

Glu Ile Gln Glu Lys Glu Asn Leu Leu Ser Gln Gly Phe Thr Ala Trp
785                 790                 795                 800

Thr Lys Arg Asp Phe Asn Gln Phe Ile Lys Ala Asn Glu Lys Tyr Gly
                805                 810                 815

Arg Asp Asp Ile Asp Asn Ile Ala Lys Asp Val Glu Gly Lys Thr Pro
            820                 825                 830

Glu Glu Val Ile Glu Tyr Asn Ala Val Phe Trp Glu Arg Cys Thr Glu
        835                 840                 845

Leu Gln Asp Ile Glu Arg Ile Met Gly Gln Ile Glu Arg Gly Glu Gly
    850                 855                 860

Lys Ile Gln Arg Arg Leu Ser Ile Lys Lys Ala Leu Asp Gln Lys Met
865                 870                 875                 880

Ser Arg Tyr Arg Ala Pro Phe His Gln Leu Arg Leu Gln Tyr Gly Asn
                885                 890                 895

Asn Lys Gly Lys Asn Tyr Thr Glu Ile Glu Asp Arg Phe Leu Val Cys
            900                 905                 910

Met Leu His Lys Leu Gly Phe Asp Lys Glu Asn Val Tyr Glu Glu Leu
        915                 920                 925

Arg Ala Ala Ile Arg Ala Ser Pro Gln Phe Arg Phe Asp Trp Phe Ile
    930                 935                 940

Lys Ser Arg Thr Ala Leu Glu Leu Gln Arg Arg Cys Asn Thr Leu Ile
945                 950                 955                 960

Thr Leu Ile Glu Arg Glu Asn Ile Glu Leu Glu Glu Lys Glu Arg Ala
                965                 970                 975

Glu Lys Lys Lys Lys Ala Pro Lys Gly Ser Val Ser Ala Gly Ser Gly
            980                 985                 990

Ser Ala Ser Ser Asn Thr Pro Ala Pro Ala Pro Gln Pro Lys Ala Ser
        995                 1000                1005

Gln Lys Arg Lys Ser Glu Val Val Ala Thr Ser Ser Asn Ser Lys Lys
    1010                1015                1020

Lys Lys Lys
1025

<210> 124
<211> 3700
<212> DNA
<213> Drosophila melanogaster

<220>
<223> ISWI protein

<400> 124
agttcgtatt ctcgatttgt ttgccgccac cgtcgctgcc gcagccaact tcgcgcattc 60
atcgcataat ctagcgatag cacgcgcaga tttcccacca ttcattaaaa aaaatcgcat 120
gtaattattt acaatttgtt taagcgaaac aaacattgtt ttgtgtaaat tgccttttat 180
ttatatactg aaagtataac taacaggttg cacactcgat tttcagttca cattaaacga 240
caccagtgtg cagcacgctt gattggcgag gggaaatttg tggaaaaatt aagttcattt 300
agagacagac gccggcattg caaatatgtc caaaacagat acagctgccg tggaggcaac 360
cgaagagaac tcgaacgaga cgacttcaga tgcggccacc agttcatccg gtgaaaagga 420
ggctgagttc gacaacaaaa tcgaggctga tcgcagtagg cgctttgatt tcctgctaaa 480
gcagacggag atattcaccc acttcatgac taacagcgct aagagtccca cgaagcctaa 540
gggtagaccc aagaagatca aagacaagga caaggaaaag gatgtggccg atcatcgtca 600
tcgcaagaca gagcaggagg aggatgagga gttgctggcg gaagactcgg ccaccaagga 660
gatctttcgc ttcgatgcct cacccgccta catcaaaagt ggagagatgc gtgactacca 720
gattcgcggt cttaactgga tgatttcgct ttacgaaaat ggtatcaatg gaattctggc 780
cgatgaaatg ggtctaggaa agaccctgca gaccatatct ctgctgggtt acctcaagca 840
tttcaaaaat caagctggac cacacatcgt catcgtgcca aagtcaacgc ttcagaattg 900
ggtaaatgag tttaaaaagt ggtgtccttc cctcagagcc gtctgcctta ttggtgacca 960
ggacacccgt aacaccttca ttagagatgt gctcatgcct ggcgagtggg acgtttgcgt 1020
gacctcctat gagatgtgta tccgcgagaa gtctgtattc aagaagttca actggcgcta 1080
tttggtcatc gacgaggcgc atcgtatcaa gaacgagaag tcgaagctgt cggagattct 1140
gcgagagttt aagaccgcta atcgtctact tatcacgggt actccgctgc agaataacct 1200
ccacgagctg tgggccctgc ttaatttcct gctgcccgat gtgtttaatt cgtcagagga 1260
ttttgacgaa tggttcaaca cgaacacctg cctgggtgac gatgcattga ttacgcgttt 1320
gcatgccgtg ctgaaacctt tcctgctccg tcgtctaaag gccgaagtgg agaagcgtct 1380
gaagccgaag aaggagatga aaatatttgt gggtctatcc aagatgcaac gcgactggta 1440
caccaaggtg ctgcttaagg acattgatgt agtgaacggt gctggcaaag tggagaagat 1500
gcgactgcag aacatcctta tgcagctccg caagtgcacc aaccacccat atttgtttga 1560
tggcgccgag cccggtccgc catacaccac ggacacgcat ttggtgtata actccggaaa 1620
gatggctatt ctggacaagc tgctgcccaa gctccaagag cagggatcgc gtgtgttgat 1680
tttctcacaa atgacgagga tgttggatat cctcgaggac tactgtcact ggcgcaacta 1740
caactattgc cgcctggatg gtcagacgcc gcacgaagat cgtaacaggc agattcagga 1800
atttaacatg gacaacagcg ccaagtttct cttcatgttg tccacccgag ccggtggttt 1860
gggtatcaat ttggctaccg ctgatgttgt catcatttac gactcggatt ggaatcctca 1920
aatggatttg caagctatgg atcgtgctca tcgtattggt caaaagaagc aagtgcgcgt 1980
tttccggctg atcaccgaaa gtacagtgga ggagaagatc gtggagagag cagaggtcaa 2040
gctccgtctg gacaagatgg tcatccaggg gggcagattg gttgacaacc gctccaatca 2100
gttgaacaag gatgaaatgc ttaatataat ccgttttgga gctaaccaag tgttcagctc 2160
taaggagaca gacattaccg atgaggacat cgatgttatt ttggagcgcg gtgaggccaa 2220
gacggccgag caaaaggcag cactggacag tctgggcgag agttcgctgc ggacgttcac 2280
aatggacaca aacggcgagg caggaacttc ttccgtatat caattcgagg gtgaggattg 2340
gcgcgagaag caaaagctaa atgcgctggg caactggatc gagccaccga agcgtgaacg 2400
caaagccaac tatgctgtgg atgcctattt ccgcgaggct ctccgtgtct ccgaacccaa 2460
ggcaccgaag gctccccgcc cacccaagca gcctatcgtt caggactttc agttcttccc 2520
accccgtctg tttgagctgc tcgaccagga aatctactat ttccgcaaga ctgttggtta 2580
caaggtgccc aagaacacgg aactaggatc ggatgccacc aaagtgcagc gcgaggagca 2640
gcgcaagatc gatgaggcag agccgcttac cgaggaagag atccaggaga aggagaatct 2700
actctcacag ggtttcactg cctggaccaa gcgcgatttc aaccagttca tcaaggctaa 2760
cgaaaagtac ggtcgggatg atattgacaa cattgccaag gacgtggagg gcaagactcc 2820
ggaggaggtt atcgagtaca acgccgtgtt ttgggagcga tgcactgagt tgcaggatat 2880
tgagcgaata atgggacaga ttgagcgtgg agagggcaag attcaacggc gattgtctat 2940
taagaaggct ttggatcaaa agatgtcgcg gtatcgcgcc cccttccatc agttgcggct 3000
gcaatatggt aataacaagg gcaagaatta cactgaaata gaggatcgct ttctggtatg 3060
catgcttcac aagttgggct ttgacaagga gaatgtctac gaagagctgc gagcggctat 3120
aagagcttct ccgcaattcc gctttgactg gttcatcaaa tctagaaccg ctttggagct 3180
acagcgtcgc tgcaacacgt tgattaccct tattgaacgt gaaaacattg agctggaaga 3240
gaaagaacgc gccgagaaaa agaaaaaggc accaaagggc agcgtgtccg ctggaagtgg 3300
aagtgccagc tcgaacactc cagcacccgc gccccaaccg aaggccagtc aaaagcggaa 3360
gagcgaggtg gtggcaacca gttccaactc gaaaaagaag aagaagtaaa gcgacaaagc 3420
aaacagatat ttttctgaga ttcggctcta cactcagcac taggcagtta aatagttaag 3480
acaagcgatt acttaaacat attttactct atatagttaa tagttcatat ctcacgccgg 3540

aagcaatatc acaccaaccc cacccccaaa atctgaagtt tttttaaaca cccgcctaca 3600
catccgacta cattactatt gcgtgccgct gtattctatt ccaaataatt gtattgtgtt 3660
ctaagactat aatcattata tttatataaa tcaacacagc 3700

<210> 125
<211> 406
<212> PRT
<213> Homo sapiens

<220>
<223> SNX6

<400> 125
Met Met Glu Gly Leu Asp Asp Gly Pro Asp Phe Leu Ser Glu Glu Asp
1               5                   10                  15

Arg Gly Leu Lys Ala Ile Asn Val Asp Leu Gln Ser Asp Ala Ala Leu
            20                  25                  30

Gln Val Asp Ile Ser Asp Ala Leu Ser Glu Arg Asp Lys Val Lys Phe
        35                  40                  45

Thr Val His Thr Lys Ser Ser Leu Pro Asn Phe Lys Gln Asn Glu Phe
    50                  55                  60

Ser Val Val Arg Gln His Glu Glu Phe Ile Trp Leu His Asp Ser Phe
65                  70                  75                  80

Val Glu Asn Glu Asp Tyr Ala Gly Tyr Ile Ile Pro Pro Ala Pro Pro
                85                  90                  95

Arg Pro Asp Phe Asp Ala Ser Arg Glu Lys Leu Gln Lys Leu Gly Glu
            100                 105                 110

Gly Glu Gly Ser Met Thr Lys Glu Glu Phe Thr Lys Met Lys Gln Glu
        115                 120                 125

Leu Glu Ala Glu Tyr Leu Ala Ile Phe Lys Lys Thr Val Ala Met His
    130                 135                 140

Glu Val Phe Leu Cys Arg Val Ala Ala His Pro Ile Leu Arg Arg Asp
145                 150                 155                 160

Leu Asn Phe His Val Phe Leu Glu Tyr Asn Gln Asp Leu Ser Val Arg
                165                 170                 175

Gly Lys Asn Lys Lys Glu Lys Leu Glu Asp Phe Phe Lys Asn Met Val
            180                 185                 190

Lys Ser Ala Asp Gly Val Ile Val Ser Gly Val Lys Asp Val Asp Asp
        195                 200                 205

Phe Phe Glu His Glu Arg Thr Phe Leu Leu Glu Tyr His Asn Arg Val
    210                 215                 220

Lys Asp Ala Ser Ala Lys Ser Asp Arg Met Thr Arg Ser His Lys Ser
225                 230                 235                 240

Ala Ala Asp Asp Tyr Asn Arg Ile Gly Ser Ser Leu Tyr Ala Leu Gly
                245                 250                 255

Thr Gln Asp Ser Thr Asp Ile Cys Lys Phe Phe Leu Lys Val Ser Glu
            260                 265                 270

Leu Phe Asp Lys Thr Arg Lys Ile Glu Ala Arg Val Ser Ala Asp Glu
        275                 280                 285

Asp Leu Lys Leu Ser Asp Leu Leu Lys Tyr Tyr Leu Arg Glu Ser Gln
    290                 295                 300

Ala Ala Lys Asp Leu Leu Tyr Arg Arg Ser Arg Ser Leu Val Asp Tyr
305                 310                 315                 320

Glu Asn Ala Asn Lys Ala Leu Asp Lys Ala Arg Ala Lys Asn Lys Asp
                325                 330                 335

Val Leu Gln Ala Glu Thr Ser Gln Gln Leu Cys Cys Gln Lys Phe Glu
            340                 345                 350

Lys Ile Ser Glu Ser Ala Lys Gln Glu Leu Ile Asp Phe Lys Thr Arg
        355                 360                 365

Arg Val Ala Ala Phe Arg Lys Asn Leu Val Glu Leu Ala Glu Leu Glu
    370                 375                 380

Leu Lys His Ala Lys Gly Asn Leu Gln Leu Leu Gln Asn Cys Leu Ala
385                 390                 395                 400

Val Leu Asn Gly Asp Thr
                405

<210> 126
<211> 1969
<212> DNA
<213> Homo sapiens

<220>
<223> SNX6

<400> 126
cgggtcgacc cacgcgtccg cgcctcggag caaccatgat ggaaggcctg gacgacggcc 60
cggacttcct ctcagaagag gaccgcggac ttaaagcaat aaatgtagat cttcaaagtg 120
atgctgctct gcaggtggac atttctgatg ctcttagtga gcgggataaa gtaaaattca 180
ctgttcacac aaagagttca ttgccaaatt ttaaacaaaa cgagttttca gttgttcggc 240
aacatgagga atttatctgg cttcatgatt cctttgttga aaatgaagac tatgcaggtt 300
atatcattcc accagcacca ccaagacctg attttgatgc ttcaagggaa aaactacaga 360
agcttggtga aggagaaggg tcaatgacga aggaagaatt cacaaagatg aaacaggaac 420
tggaagctga atatttggca atattcaaga agacagttgc gatgcatgaa gtgttcctgt 480
gtcgtgtggc agcacatcct attttgagaa gagatttaaa tttccatgtc ttcttggaat 540
ataatcaaga tttgagtgtg cgaggaaaaa ataaaaaaga gaaacttgaa gacttcttta 600
aaaacatggt taaatcagca gatggagtaa tcgtttcagg agtaaaggat gtagatgatt 660
tctttgagca cgaacgaaca tttcttttgg aatatcataa ccgagttaag gatgcatctg 720
ctaaatctga tagaatgaca agatcccaca aaagtgctgc agatgattac aatagaattg 780
gttcttcatt atatgcttta ggaactcagg attctacaga tatatgcaag ttttttctca 840
aagtttcaga actgttcgat aaaacaagaa aaatagaagc acgagtgtct gctgatgaag 900
acctcaaact ttctgatctt ttaaaatatt acttaagaga atctcaagct gctaaggatc 960
tcctgtatcg aaggtctagg tcactagtgg attatgaaaa tgctaataaa gcactggata 1020
aagcaagagc aaaaaataaa gatgttctac aggccgaaac ttcccaacaa ttatgttgtc 1080
agaaatttga aaaaatatct gagtctgcaa aacaagaact tatagatttt aagacaagaa 1140
gagttgctgc attcagaaaa aatttagtgg aactggcaga gttagaactg aagcatgcaa 1200

agggtaatct acagttgctg cagaactgcc tggcagtgtt aaatggagac acataagcca 1260
cactccgcct tcctgttaaa aagggctgcc ttccttcaaa ttttattttt gttttcttaa 1320
tgatgttaag catttatgct cactggaaac aaacaaaaag cagctgaaaa agtgcatcaa 1380
ctcctctttt tctgagaaac atggagcagc gcacgcccag gcgatgccag tctgtgtgcc 1440
gtgatgccgc actgtgttcc ccatgacagt ggtccatcat cgtgcactcg tcatactcag 1500
aagtccaaag ttcattcttc tttaaagtag cctctataac tctgtttatt ttataaatag 1560
tattccttat ggctgccact cttatttacc tttaaataat ttctgaaatt taaccttttc 1620
agaatgcatt gttgaaacaa gataaagatt gccttttttg aattttttaa attttgtttt 1680
taaaagcata taccacctta gttcattcat gtatcctggt aaagcatctt aatcagactt 1740
atttttaatt actgaatatt tcttagacgt tttgggacag attttatgta atctttataa 1800
gtatgatttc tgaagaaaag caaatgcatt agtatgtttg ccttaaactt gtagactaaa 1860
ccaagtattg taaaataaac agcgataaca gtgatagttt ttaactctat ggtcattgta 1920
tcactctgga aaatgtggag tagctgtaat aaatctactc ctgtattat 1969

<210> 127
<211> 140
<212> PRT
<213> Homo sapiens

<220>
<223> Profilin II

<400> 127
Met Ala Gly Trp Gln Ser Tyr Val Asp Asn Leu Met Cys Asp Gly Cys
1               5                   10                  15

Cys Gln Glu Ala Ala Ile Val Gly Tyr Cys Asp Ala Lys Tyr Val Trp
            20                  25                  30

Ala Ala Thr Ala Gly Gly Val Phe Gln Ser Ile Thr Pro Ile Glu Ile
        35                  40                  45

Asp Met Ile Val Gly Lys Asp Arg Glu Gly Phe Phe Thr Asn Gly Leu
    50                  55                  60

Thr Leu Gly Ala Lys Lys Cys Ser Val Ile Arg Asp Ser Leu Tyr Val
65                  70                  75                  80

Asp Gly Asp Cys Thr Met Asp Ile Arg Thr Lys Ser Gln Gly Gly Glu
                85                  90                  95

Pro Thr Tyr Asn Val Ala Val Gly Arg Ala Gly Arg Ala Leu Val Ile
            100                 105                 110

Val Met Gly Lys Glu Gly Val His Gly Gly Thr Leu Asn Lys Lys Ala
        115                 120                 125

Tyr Glu Leu Ala Leu Tyr Leu Arg Arg Ser Asp Val
    130                 135                 140

<210> 128
<211> 1693
<212> DNA
<213> Homo sapiens

<220>
<223> Profilin II

<400> 128

gaagggctcg aagatggccg gttggcagag ctacgtggat aacctgatgt gcgatggctg 60
ctgccaggag gccgccattg tcggctactg cgacgccaaa tacgtctggg cagccacggc 120
cgggggcgtc tttcagagca ttacgccaat agaaatagat atgattgtag gaaaagaccg 180
ggaaggtttc tttaccaacg gtttgactct tggcgcgaag aaatgctcag tgatcagaga 240
tagtctatac gtcgatggtg actgcacaat ggacatccgg acaaagagtc aaggtgggga 300
gccaacatac aatgtggctg tcggcagagc tggtagagca ttggttatag tcatgggaaa 360
ggaaggtgtc cacggaggca cacttaacaa gaaagcatat gaactcgctt tatacctgag 420
gaggtctgat gtgtaagcag cctctcccca tctacctagc aactgtcttc atcaacaacc 480
ctaattatgg tcacaatgct accaaactgt agatggtagc taatttttct ttacctattt 540
tctaatgtca tgattcctgt ttgcccaatg gatcatttgt atgttaacca ctgtatgtaa 600
ccaaccctta tctggcaaca taattgcagc acaataatga tttgcatgat accttgaaat 660
tggggggagg gggcatgcca agttgggcat cactttgtct tagcaattaa tgggatattg 720
attactaaaa taagttaata ttaagcaagg tgccggttgt acaatctctg atcagtgtct 780
tttcagcact ttgagcattt acttggctca tttagtcttc cttttgtagc gcatggttgg 840
gaggaaaaag tgcatgcatc attccttcac tcttctcttt ttcccgcccc cccctccctt 900
cgcacatagg catttggttt gcttccatct ttttttatgc agtgcctgtt tttttttaac 960
caattaaaat cccttttgtt gatgagctat tgagagctgc agtagtttgc ttttagtatt 1020
gttgttgcac ttgagcagag acaaaccttt attcatagtg tctacaggac atatgaagag 1080
tgcaatggca aaacaagagc aaaaagcact tcctcccatg accttacagt aaccatactg 1140
attgaatccc cagggacatt ccatcattgc aatagctcag atttttcttc ctttttcttt 1200
gcacaccagc tctactcttt agtaaaattg taaaaggctg ccattatgga cattaggtat 1260
cccaacataa ccatctggag tgtgtccagt ttgttcttca taggaccaat ttttatttgc 1320
agcttgagtt tttatatgaa gttgcattat tgtggacttg gctgtccttg aatttttttc 1380
atatgtattc tgtgccatac tattgttaaa atgaactgtt gctattgtga gatggatttt 1440
aactgaccta ttaagggttt ctttcgaatg gcactacttt agggacattc tagtatttgc 1500
ttctattgtt tgggccttgt ggataatgta cagatttaaa aacaaatctt gttgctgatt 1560
tgtccatttc tttccctgca ctttgttaca tctgggatac agtctaactc atctgattta 1620
atatgcattt aaaaaaatgc cataactatt aaacaccttg tttacagaca gatgaaataa 1680
atttattcca acc 1693

<210> 129
<211> 442
<212> PRT
<213> Mus musculus

<220>
<223> Testican

<400> 129
Met Pro Ala Ile Ala Val Leu Ala Ala Ala Ala Ala Ala Trp Cys Phe
1               5                   10                  15

Leu Gln Val Asp Ser Arg His Leu Asp Ala Leu Ala Gly Gly Ala Ala
            20                  25                  30

Leu Asn Asn Ala Asn Phe Leu Asp Asn Asp Gln Trp Leu Ser Thr Val
        35                  40                  45

Ser Gln Tyr Asp Arg Asp Lys Tyr Trp Asn Arg Phe Arg Asp Glu Val
    50                  55                  60

Glu Asp Asp Tyr Phe Arg Asn Trp Asn Pro Asn Lys Pro Phe Asp Gln
65                  70                  75                  80

Ala Leu Asp Pro Ser Lys Asp Pro Cys Leu Lys Val Lys Cys Ser Pro
                85                  90                  95

His Lys Val Cys Val Thr Gln Asp Tyr Gln Thr Ala Leu Cys Val Ser
            100                 105                 110

Arg Lys His Leu Leu Pro Arg Gln Lys Lys Gly Asn Val Ala His Lys
        115                 120                 125

His Trp Leu Gly Pro Ser Asn Leu Val Lys Cys Lys Pro Cys Pro Val
    130                 135                 140

Ala Gln Ser Ala Met Val Cys Gly Ser Asp Gly His Thr Tyr Thr Ser
145                 150                 155                 160

Lys Cys Lys Leu Glu Phe His Ala Cys Ser Thr Gly Lys Ser Leu Asn
                165                 170                 175

Ser Leu Cys Asp Gly Pro Cys Pro Cys Leu Pro Glu Pro Glu Pro Leu
            180                 185                 190

Lys Pro Lys Ala Glu Lys Ser Ala Cys Thr Asp Lys Glu Leu Arg Asn
        195                 200                 205

Leu Ala Ser Arg Leu Lys Asp Trp Phe Gly Ala Leu His Glu Asp Ala
    210                 215                 220

Asn Arg Val Ile Lys Pro Thr Ser Ser Asp Gly Ala Gln Gly Arg Phe
225                 230                 235                 240

Asp Thr Ser Ile Leu Pro Ile Cys Lys Asp Ser Leu Gly Trp Met Phe
                245                 250                 255

Asn Lys Leu Asp Met Asn Tyr Asp Leu Leu Leu Asp His Ser Glu Ile
            260                 265                 270

Asn Ala Ile Tyr Leu Asp Lys Tyr Glu Pro Cys Ile Lys Pro Leu Phe
        275                 280                 285

Asn Ser Cys Asp Ser Phe Lys Asp Gly Lys Leu Ser Asn Asn Glu Trp
    290                 295                 300

Cys Tyr Cys Phe Gln Lys Pro Ala Gly Leu Pro Cys Gln Asn Glu Met
305                 310                 315                 320

Asn Arg Ile Gln Lys Leu Ser Lys Gly Lys Ser Leu Leu Gly Ala Phe
                325                 330                 335

Ile Pro Arg Cys Asn Glu Glu Gly Tyr Tyr Lys Ala Thr Gln Cys His
            340                 345                 350

Gly Ser Thr Gly Gln Cys Trp Cys Val Asp Lys Tyr Gly Asn Glu Leu
        355                 360                 365

Ala Gly Ser Arg Lys Gln Gly Thr Val Ser Cys Glu Glu Glu Gln Glu
    370                 375                 380

Thr Ser Gly Asp Phe Gly Ser Gly Gly Ser Val Val Leu Leu Asp Asp
385                 390                 395                 400

Leu Glu Asp Glu Arg Asp Val Gly Pro Lys Asp Lys Glu Gly Lys Leu
                405                 410                 415

Arg Val Arg Thr Arg Ala Val Arg Glu Asp Asp Glu Asp Glu Asp Asp
            420                 425                 430

Asp Lys Glu Asp Glu Val Gly Tyr Ile Trp

        435                 440

<210> 130
<211> 4343
<212> DNA
<213> Mus musculus

<220>
<223> Testican

<400> 130
   gtgggctcac aaagcggccg gacgcgcggc ggcggcaggt gcgcaaggga acgagccggg 60
   catcagcctt cccagccacc gcggtacagg aaccccagca actcgggcgc cgggactcgg 120
   gcgggctccc aagatgccag cgatcgcggt gctcgcggcg gccgccgcgg cgtggtgctt 180
   cctccaagtg gacagccggc acctggacgc gctggccggt ggcgcggccc tcaacaacgc 240
   caatttccta gacaatgacc agtggctgag cactgtctcc cagtatgacc gtgacaagta 300
   ctggaaccgc ttccgagatg aagttgagga tgactatttc agaaactgga atcccaacaa 360
   gcccttcgac caagccctgg acccatccaa ggacccctgc ctgaaggtga aatgcagccc 420
   gcacaaagta tgtgtgaccc aggactacca gacggctctg tgtgtcagcc gcaagcacct 480
   gttgccaagg cagaagaagg gcaatgtggc tcacaaacac tggcttggac cttccaatct 540
   ggttaagtgc aagccttgcc ccgtggcgca gtcagcgatg gtctgcggct ctgacggcca 600
   cacgtacacg tccaagtgca agttggaatt ccacgcttgt tctacaggca aaagcctcaa 660
   ctccctctgt gatgggccct gtccgtgtct gcctgagcct gagccactga agcccaaagc 720
   agagaagagt gcctgcacgg acaaggagct gcggaacctc gcctcccggc tgaaggactg 780
   gttcggggct cttcatgagg acgccaatag agtcatcaag cctaccagct ctgatggagc 840
   ccaaggcagg tttgacacca gcatcttacc catttgcaag gactccttgg gttggatgtt 900
   caacaagttg gacatgaact atgacctgtt gctggaccac tcagagatca atgccatcta 960
   cctagacaaa tatgagccct gcatcaagcc tctcttcaac tcgtgcgact ccttcaagga 1020
   cggcaaactc tccaacaatg agtggtgtta ctgcttccag aagccagcgg gtctcccttg 1080
   ccagaatgaa atgaacagaa ttcagaagct aagcaagggg aaaagcctac tgggggcctt 1140
   catccctcga tgtaacgagg agggctacta caaagccaca cagtgccacg gcagcacggg 1200
   gcagtgctgg tgtgtggata aatatgggaa cgagctggcc ggctccagga aacagggcac 1260
   tgtaagctgc gaagaggagc aggaaacctc cggggacttc ggcagtggag gctccgtggt 1320
   cctgctggat gacctagagg atgagcggga cgtgggacca aaggacaaag aagggaagct 1380
   gagggtgcgc acccggccgg tgcgggaaga tgatgaggat gaagatgacg acaaagaaga 1440
   tgaggtcggc tacatatggt agtgcccacg aggaagagga cacacttttg gcacagatct 1500
   gcaagtcgtt tcctttgcct gcatttgtat ctaagactcc gaggcaccgg ggtctcttct 1560
   ccactgttga tctctgaacg gggcctgagg tttggaagac cctcttccca gagtgactga 1620
   atttgcatac ggttgtgtgg gagaaaggat gttatttttg ttccttgttt tcgttttcat 1680
   tggatgagag gacaggctgg cttagttaaa gccgcctcct ctccctgtga ggttttcgca 1740
   actagcatgt gatttgtgtg gaatccaaca gtgcggggag ccccaccctc tcaagcgtca 1800
   aagaccctct ctgattaccc acgtcggtgg ttcctacagc atggttccca gcgtcttatg 1860
   atgtctgcgt gcttttctcg tgtcctgtag atgttgtgga aaacatacca ggctgtccct 1920
   tttccctgtc tctaccacct ctggtgttga ttattaaaaa ttaattttca tgtcactgta 1980
   tctgacttcc tacaaacgac agccttaatt cagtccaagt ccctttggag aattcatttt 2040
   ctttcttaaa aaaaataaga tctcgacacc cccccccccc tgcgcccaaa ggtgactgtg 2100
   cttgcatgac ttgcatacac acacctttca gtctagtgtc tggttgtata ccaaaaacac 2160
   cttagataag caatggcttt tagtcagttg ctccagaccg agaacacaaa ccggggtatg 2220
   aaattcctct tagagtccgg tttgcactag ctcttcgtgt gtgtgtgtag cctgcatccc 2280
   ggggaggctc ttctatgcgg tctcagagtg actggaagcc tttttgattt ggatgagcct 2340
   ccatttgcct aaatgtgttt ttacccacag aagccatgca attcccacgt gggcagcttc 2400
   acaagccatt tcttcacttc cgcgcacagc tcccctcagg atacagtgta cccacctgcg 2460
   gcagcttcct tccctatcca cccaagggct ttttcctacc gtgttcccca ctgagcccct 2520
   gctccccaag aagacagcat cattataaag cttagaagaa aatttaccct tcttcagtgt 2580
   ctcctttgac cccctttcta tccacaaatc atgacccagg gtatgagact ttaaaggttt 2640
   ctcgtgttcc cccattattt actagggacc cccaatggaa gcccagaaag agattcctag 2700
   tagcacaaga ggctagtctc ctcatcagca gccatctccc cgacacatgc tgaccttagt 2760
   tgacacacgg ctgaggccat tttactgttt gtcctgagag ctcgttattg gcatctggtt 2820
   tcactagtga tatcatctca atacttttca gatttctttc atacaagtac caaacagcaa 2880
   tcttcagtgc gggccccgtt cagctcaggg ccaatcccca gctctgtctt cttgcttcct 2940
   gcctagctcc ttgccctaac cagccacagc ctgccagaca cactctcttc atggtccttg 3000
   ccttccccca tcctgagttt tagccagtcc acaagacaga tccatcaact caggctacaa 3060
   aggtatttgg ttaaaccgag atcagggagc agagccctaa actggctttc tgtatttagg 3120
   aagccaccta caattcgaat tccccttctt gaataggaca ggcaaagtga catcagccct 3180
   cttgggaaat gaatataccc ggtgcccttt acccctggcc tgggatacac acgcagagcc 3240
   agggtgctga gattctgtgt gcttcctcct tatggagatg ggaacctgct ttggaatctc 3300
   agtgccatcc cccccgcccc caccccctat tttgtttgtt tttttcttcc cattctcaaa 3360
   atggatcatc ctttagaaaa caggtgctta atcacagcta aattgtaata gaatacatta 3420
   aaaagacata ttagaaatct cagtttaggc catccatata agccattgtt gttatttcct 3480
   atgagcttag gggaaaaaaa aacatgcaca agagaacaaa ttaacatggc aaattgattt 3540
   ctctctgaga aacttctagg aacccaatga gccacttact ttgtctaatt atcttatgac 3600
   aagtgtcaca ttaagatgac acttagaagt cctcaacatt atcttaatgg tgaagaagag 3660
   tactcaggag gcaattccca ggaaggtaat gagatgtcat ttccagagac attccaagaa 3720
   gatattttga ttcattaaaa tattaaatca aaagccctcc tacgtgtgga gcccacatca 3780
   acaagacagg ccagtgccgc ttctcatggc cccactttgg gttttaaaga tggctattta 3840
   taatggtact ttaaagttag acttttgttc tgtttcctga gaaagagtgc ttccactggt 3900
   cttctggaag gaccctttca gtctaatcat caggcgagca tatgagactt tttaagcata 3960
   tgcggctgta atgataacca cttgcttagg caggcactga gctgagtccc cacttagctt 4020
   tagaaaacct tcagttgatt ctgttactct aattctacaa actgttctta ctaacattcc 4080
   tgtgacatcc agtttaaacc ctagaattct cttccaccag ggttcattgt ctgccatatg 4140
   actatgtttc tacagatatt ctcagcatga ggatggcttc acatggcttt ctctcagtac 4200
   tccctttatg cccacgaccc tgtgtataca ccaaatgatg atttgtctgt ccaggtctac 4260
   cctctgcttg tcccagaacg ccattgacat tgactttggt ttggttttta tcttgaccac 4320
   actgtacagt aacatccaag agc 4343

<210> 131
<211> 358
<212> PRT
<213> Mus musculus

<220>
<223> E124

<400> 131
Met Val Trp Gly His Phe Pro Leu Ala Val Phe Asp Ser Leu Gly Ser
1               5                   10                  15

Gly Glu Met Ala Asp Ser Val Lys Thr Phe Leu Gln Asp Leu Gly Arg
            20                  25                  30

Gly Ile Lys Asp Ser Ile Trp Gly Ile Cys Thr Ile Ser Lys Leu Asp
        35                  40                  45

Ala Arg Ile Gln Gln Lys Arg Glu Glu Gln Arg Arg Arg Arg Ala Ser
    50                  55                  60

Ser Leu Leu Ala Gln Arg Arg Pro Gln Ser Val Glu Arg Lys Gln Glu
65                  70                  75                  80

Ser Glu Pro Arg Ile Val Ser Arg Ile Phe Gln Cys Cys Ala Trp Asn
                85                  90                  95

Gly Gly Val Phe Trp Phe Ser Leu Leu Leu Phe Tyr Arg Val Phe Ile
            100                 105                 110

Pro Val Leu Gln Ser Val Thr Ala Arg Ile Ile Gly Asp Pro Ser Leu
        115                 120                 125

His Gly Asp Val Trp Ser Trp Leu Glu Phe Phe Leu Thr Ser Ile Phe
    130                 135                 140

Ser Ala Leu Trp Val Leu Pro Leu Phe Val Leu Ser Lys Val Val Asn
145                 150                 155                 160

Ala Ile Trp Phe Gln Asp Ile Ala Asp Leu Ala Phe Glu Val Ser Gly
                165                 170                 175

Arg Lys Pro His Pro Phe Pro Ser Val Ser Lys Ile Ile Ala Asp Met
            180                 185                 190

Leu Phe Asn Leu Leu Leu Gln Ala Leu Phe Leu Ile Gln Gly Met Phe
        195                 200                 205

Val Ser Leu Phe Pro Ile His Leu Val Gly Gln Leu Val Ser Leu Leu
    210                 215                 220

His Met Ser Leu Leu Tyr Ser Leu Tyr Cys Phe Glu Tyr Arg Trp Phe
225                 230                 235                 240

Asn Lys Gly Ile Glu Met His Gln Arg Leu Ser Asn Ile Glu Arg Asn
                245                 250                 255

Trp Pro Tyr Tyr Phe Gly Phe Gly Leu Pro Leu Ala Phe Leu Thr Ala
            260                 265                 270

Met Gln Ser Ser Tyr Ile Ile Ser Gly Cys Leu Phe Ser Ile Leu Phe
        275                 280                 285

Pro Leu Phe Ile Ile Ser Ala Asn Glu Ala Lys Thr Pro Gly Lys Ala
    290                 295                 300

Tyr Leu Phe Gln Leu Arg Leu Phe Ser Leu Val Val Phe Leu Ser Asn
305                 310                 315                 320

Arg Leu Phe His Lys Thr Val Tyr Leu Gln Ser Ala Leu Ser Ser Ser
                325                 330                 335

Ser Ser Ala Glu Lys Phe Pro Ser Pro His Pro Ser Pro Ala Lys Leu
            340                 345                 350

Lys Ala Ala Ala Gly His
        355


<210> 132
<211> 2119
<212> DNA
<213> Mus musculus

<220>
<223> E124

<400> 132
tgaacccggg acgcgaaagg cggcaggggc aggcctggcg gcggcgcgcg ggactcaggc 60
tttccccagg ccctccatga tgaatggttt gggggcactt ccctctagct gtatttgata 120
gtctgggcag tggagagatg gctgacagtg tcaaaacctt tctgcaggac cttggcaggg 180
gaatcaaaga ctccatctgg ggcatctgta ccatctcaaa gctagatgct cggatccagc 240
agaagagaga ggaacagcgt cgaagaaggg caagtagcct cttggcccag aggagacccc 300
agagtgtaga gcggaagcaa gagagtgaac cacgtattgt tagtagaatt ttccagtgtt 360
gtgcttggaa tggtggagta ttctggttca gtctcctctt gttttatcga gtgtttattc 420
ctgtacttca gtcagtaaca gcccggatta ttggagatcc atcacttcat ggagatgttt 480

ggtcatggct ggaattcttc ctcacatcaa ttttcagtgc tctttgggtg ctccccctgt 540
ttgtgcttag caaagttgtg aatgccattt ggttccaaga tatagctgac ttggcatttg 600
aagtatcagg gaggaaacct catccattcc ccagtgtcag caaaataatt gctgacatgc 660
tcttcaacct tttgctacag gcacttttcc ttattcaggg gatgtttgtg agtctcttcc 720
ccatccatct tgtgggtcag ctggttagtc tgctgcatat gtctcttctc tattcactgt 780
actgctttga gtaccgttgg ttcaacaaag gaattgaaat gcaccagcga ttgtcgaaca 840
tagaaaggaa ttggccttac tactttgggt ttggcttgcc cttggctttc ctcacagcaa 900
tgcaatcctc ctacattatc agtggctgcc tcttttctat cctgtttcct ttattcatca 960
tcagcgccaa tgaagcaaag actcctggaa aagcatatct tttccagttg cgcctattct 1020
ccttggtggt ctttttaagc aacagacttt tccacaagac cgtctacctg cagtcagccc 1080
tgagcagctc gtcctctgca gagaaattcc cttcgccaca tccttctccg gccaaactga 1140
aagctgctgc aggccattga gccctgctgt caaaggggtg ggtgggactg ggtggaggat 1200
gtggcagctc ttttctctgt tttcctcccc ctgccgtgga aggcagaacc cactgccaag 1260
ggccctctgc atagtccctt gtctttgaat tggaatcttc ctgactccag tatatggatt 1320
tttaccacca ccctaggtct gtaaggacca gttttccagc tgttttttta gcacttgcca 1380
gctcctgtgc ctggactgat tgatttgagt actttttttc ccctttcctt gtgtcattta 1440
ccctcccact tcctcctgcc ttccagcacc cctggatgaa tgggctttgt aattttaact 1500
gttgtatttt gtgaatttgt tgttactgtt tttctgtgaa gcacatacat gtatgtggga 1560
ggtaaagggg cattccagtt gctccagtca ctccctctat agccatactg tcttgttttc 1620
tgtaactcag gttaggtttt ggtctctatt ctctgctgca gaaaaggaaa gaaggagtgg 1680
gggaaatgga gcctgaagag ttggggcaga tagacctcag ccaaactggc tgggttttga 1740
ggagtcatgt tctttcttcc cttgaagggg aaagagtttt ttccactggt ccatttaaag 1800
tttcccagct atggggtggt accagttctg gacaagtgcc actgcatcat agtatgctcg 1860
gagaatctga accttactct gaagatgaaa tttactgttg ccactgccag gtcacactgg 1920
tgttttaagg aatactgggt gcttcatata ggaactgaag gggtaaactt actaaaccat 1980
tcaacctgtg attggtgatg ttttcctgtc attttaagag tcgacacatg ggtggggggg 2040
cagatgtaaa aaaacttgta caattttaaa atatcacaat taaacgtgag ctggtttccc 2100
aaaaaaaaaa aaaaaaaaa 2119

<210> 133
<211> 845
<212> PRT
<213> Rattus norvegicus

<220>
<223> Matrin 3

<400> 133
Met Ser Lys Ser Phe Gln Gln Ser Ser Leu Gly Arg Asp Ser Gln Gly
1               5                   10                  15

His Gly Arg Asp Leu Ser Ala Ala Gly Ile Gly Leu Leu Ala Ala Ala
            20                  25                  30

Thr Gln Ser Leu Ser Met Pro Ala Ser Leu Gly Arg Met Asn Gln Gly
        35                  40                  45

Thr Ala Arg Leu Ala Ser Leu Met Asn Leu Gly Met Ser Ser Ser Leu
    50                  55                  60

Asn Gln Gln Gly Ala His Ser Ala Leu Ser Ser Ala Ser Thr Ser Ser
65                  70                  75                  80

His Asn Leu Gln Ser Ile Phe Asn Ile Gly Ser Arg Gly Pro Leu Pro
                85                  90                  95

Leu Ser Ser Gln His Arg Gly Asp Thr Asp Gln Ala Ser Asn Ile Leu
            100                 105                 110

Ala Ser Phe Gly Leu Ser Ala Arg Asp Leu Asp Glu Leu Ser Arg Tyr

115                 120                 125

Pro Glu Asp Lys Ile Thr Pro Glu Asn Leu Pro Gln Ile Leu Leu Gln
    130                 135                 140

Leu Lys Arg Arg Arg Thr Glu Glu Gly Pro Thr Leu Ser Tyr Gly Arg
145                 150                 155                 160

Asp Gly Arg Ser Ala Thr Arg Glu Pro Pro Tyr Arg Val Pro Arg Asp
                165                 170                 175

Asp Trp Glu Glu Lys Arg His Phe Arg Arg Asp Ser Phe Asp Asp Arg
            180                 185                 190

Gly Pro Ser Leu Asn Pro Val Leu Asp Tyr Asp His Gly Ser Arg Ser
        195                 200                 205

Gln Glu Ser Gly Tyr Tyr Asp Arg Met Asp Tyr Glu Asp Asp Arg Leu
    210                 215                 220

Arg Asp Gly Glu Arg Cys Arg Asp Asp Ser Phe Phe Gly Glu Thr Ser
225                 230                 235                 240

His Asn Tyr His Lys Phe Asp Ser Glu Tyr Glu Arg Met Gly Arg Gly
                245                 250                 255

Pro Gly Pro Leu Gln Glu Arg Ser Leu Phe Glu Lys Lys Arg Gly Ala
            260                 265                 270

Pro Pro Ser Ser Asn Ile Glu Asp Phe His Gly Leu Leu Pro Lys Gly
        275                 280                 285

Tyr Pro His Leu Cys Ser Ile Cys Asp Leu Pro Val His Ser Asn Lys
    290                 295                 300

Glu Trp Ser Gln His Ile Asn Gly Ala Ser His Ser Arg Arg Cys Gln
305                 310                 315                 320

Leu Leu Leu Glu Ile Tyr Pro Glu Trp Asn Pro Asp Asn Asp Thr Gly
                325                 330                 335

His Thr Met Gly Asp Pro Phe Met Leu Gln Gln Ser Thr Asn Pro Ala
            340                 345                 350

Pro Gly Ile Leu Gly Pro Pro Pro Pro Ser Phe His Leu Gly Gly Pro
        355                 360                 365

Ala Val Gly Pro Arg Gly Asn Leu Gly Ala Gly Asn Gly Asn Leu Gln
    370                 375                 380

Gly Pro Arg His Met Gln Lys Gly Arg Val Glu Thr Ser Arg Val Val
385                 390                 395                 400

His Ile Met Asp Phe Gln Arg Gly Lys Asn Leu Arg Tyr Gln Leu Leu
                405                 410                 415

Gln Leu Val Glu Pro Phe Gly Val Ile Ser Asn His Leu Ile Leu Asn
            420                 425                 430

Lys Ile Asn Glu Ala Phe Ile Glu Met Ala Thr Thr Glu Asp Ala Gln
        435                 440                 445

Ala Ala Val Asp Tyr Tyr Thr Thr Thr Pro Ala Leu Val Phe Gly Lys
    450                 455                 460

Pro Val Arg Val His Leu Ser Gln Lys Tyr Lys Arg Ile Lys Lys Pro
465                 470                 475                 480

Glu Gly Lys Pro Asp Glu Lys Phe Asp Gln Lys Gln Glu Leu Gly Arg
                485                 490                 495

Val Ile His Leu Ser Asn Leu Pro His Ser Gly Tyr Ser Asp Ser Ala
            500                 505                 510

Val Leu Lys Leu Ala Glu Pro Tyr Gly Lys Ile Lys Asn Tyr Ile Leu
        515                 520                 525

Met Arg Met Lys Ser Gln Ala Phe Ile Glu Met Glu Thr Arg Glu Asp
    530                 535                 540

Ala Met Ala Met Val Asp His Cys Leu Lys Lys Ala Leu Trp Phe Gln
545                 550                 555                 560

Gly Arg Cys Val Lys Val Asp Leu Ser Glu Lys Tyr Lys Lys Leu Val
                565                 570                 575

Leu Arg Ile Pro Asn Arg Gly Ile Asp Leu Leu Lys Lys Asp Lys Ser
            580                 585                 590

Arg Lys Arg Ser Tyr Ser Pro Asp Gly Lys Glu Ser Pro Ser Asp Lys
        595                 600                 605

Lys Ser Lys Thr Asp Gly Ala Gln Lys Thr Glu Asn Pro Ala Glu Gly
    610                 615                 620

Lys Glu Gln Glu Glu Lys Ser Gly Glu Asp Gly Glu Lys Asp Thr Lys
625                 630                 635                 640

Asp Asp Gln Thr Glu Gln Glu Pro Ser Met Leu Leu Glu Ser Glu Asp
                645                 650                 655

Glu Leu Leu Val Asp Glu Glu Glu Ala Ala Ala Leu Ile Glu Ser Gly
            660                 665                 670

Ser Ser Val Gly Asp Glu Thr Asp Leu Ala Asn Leu Gly Asp Val Ser
        675                 680                 685

Ser Asp Gly Lys Lys Glu Pro Ser Asp Lys Ala Val Lys Lys Asp Ala
    690                 695                 700

Ser Ala Thr Ser Lys Lys Lys Leu Lys Lys Val Asp Lys Ile Glu Glu
705                 710                 715                 720

Leu Asp Gln Glu Asn Glu Ala Ala Leu Glu Asn Gly Ile Lys Asn Glu
                725                 730                 735

Glu Asn Thr Glu Pro Gly Ala Glu Ser Ala Glu Asn Ala Asp Asp Pro
            740                 745                 750

Asn Lys Asp Ala Ser Asp Asn Ser Asp Gly Gln Asn Asp Glu Asn Lys
        755                 760                 765

Glu Asp Tyr Thr Ile Pro Asp Glu Tyr Arg Ile Gly Pro Tyr Gln Pro
    770                 775                 780

Asn Val Pro Gly Gly Ile Asp Tyr Val Ile Pro Lys Thr Gly Phe Tyr
785                 790                 795                 800

Cys Lys Leu Cys Ser Leu Phe Tyr Thr Asn Glu Glu Val Ala Lys Asn
                805                 810                 815

Thr His Cys Ser Ser Leu Pro His Tyr Gln Lys Leu Lys Lys Phe Leu
            820                 825                 830

Asn Lys Leu Ala Glu Glu Arg Arg Gln Lys Lys Glu Ser
        835                 840                 845


<210> 134
<211> 3761
<212> DNA
<213> Rattus norvegicus

<220>
<223> Matrin 3

<220>
<221> misc_feature
<222> 6
<223> n is a or g or c or t

<400> 134
tdatdnactg agagaggtat ctctcctttt ccctctccct tccctaagag ttgtctgctg 60
gttctcagct tgaagaagat tctacagtcc ttattgatcc tttttcttgg cgttaccatt 120
tttgaagcaa agttgaccta gctttctagt ttgagctttc tttttggccg tcttttaaaa 180
catttttttt tttgatctat aaaatagaca agagatagtt ctacaatgtc caagtcattc 240
cagcagtcat ctctcggtag ggattcacag ggtcatgggc gtgacctgtc tgcagcagga 300
ataggccttc ttgctgctgc tacccagtct ttaagtatgc cagcatctct tggaaggatg 360
aaccagggta ctgcacgcct tgctagctta atgaatcttg gaatgagttc ttcattgaat 420
caacaaggag ctcatagtgc actgtcttct gctagtactt cttcccataa tttgcagtct 480
atatttaaca ttggaagtag aggtccactc cctttgtctt ctcaacaccg tggagataca 540
gaccaggcca gtaatatttt ggccagcttt ggtctgtctg ctagagactt agatgaactg 600
agtcgttatc cagaggacaa gattactcct gagaacttgc cccaaattct tctacagctt 660
aaaaggagga gaactgaaga aggccctaca ttgagttatg gtagagatgg cagatctgct 720
acacgggagc caccatacag agtacctagg gatgattggg aagaaaaaag gcattttaga 780
agagatagtt ttgatgatcg tggtcctagt ctcaacccag tgcttgatta tgaccatgga 840
agtcgttctc aagaatctgg ttattatgac agaatggatt atgaagatga cagattaaga 900
gatggagaaa ggtgtaggga tgattctttt tttggtgaga cctcgcataa ctatcataaa 960
tttgacagtg agtatgagag aatgggacgt ggtcctggcc ccttacaaga gagatctctc 1020
tttgagaaaa agaggggcgc tcctccaagt agcaatattg aagacttcca tggactctta 1080
ccgaagggtt atccccatct gtgctctata tgtgatttgc cagttcattc taataaggag 1140
tggagtcaac atatcaatgg agcaagtcac agtcgtcgat gccagcttct tcttgaaatc 1200
tacccagaat ggaatcctga caatgatact ggacacacaa tgggtgatcc attcatgctg 1260
cagcagtcaa caaacccagc accgggaatt ctgggaccgc cacctccttc atttcatctt 1320
ggaggaccag cagttggacc aagaggaaat ctgggtgctg gtaatggaaa cctacaggga 1380
ccaagacaca tgcaaaaagg cagagtggaa accagccgag ttgttcacat tatggatttt 1440
cagcgaggaa aaaacttgag ataccagcta ttacaactgg tagaaccatt tggagtcatt 1500
tcaaatcatc tgattctaaa taaaattaat gaggcattta ttgaaatggc gaccacagaa 1560
gatgctcagg ctgctgtgga ttattataca actacaccag cattagtgtt tggcaagcca 1620
gtgagagttc atttatccca gaagtataag agaataaaga aacctgaagg aaaaccagat 1680
gagaagtttg atcaaaagca agaacttgga cgtgtgatac atctcagcaa tttacctcat 1740
tctggctatt ctgacagtgc tgtcctcaag cttgcagagc cttatgggaa aataaaaaat 1800
tatatattga tgaggatgaa aagtcaggct tttattgaga tggaaaccag agaagatgca 1860

atggcaatgg ttgaccactg tctaaagaag gcactttggt ttcagggaag atgtgttaaa 1920
gttgacttgt ctgagaaata taaaaaactg gtgctgagga ttcctaacag aggcattgac 1980
ttactgaaaa aagataaatc tcggaaaaga tcctattctc cagatggaaa agaatctcca 2040
agtgataaga agtccaaaac tgatggtgct cagaagactg agaatccagc tgagggtaaa 2100
gaacaagaag agaagtcggg tgaggatggt gagaaagaca ccaaggatga ccagacagaa 2160
caggagccca gtatgcttct tgaatctgaa gatgaattgc ttgtcgatga ggaagaagct 2220
gcagcactaa tagaaagtgg aagctcagtg ggtgatgaga ctgatcttgc taatttaggg 2280
gatgtatctt ctgatgggaa aaaggagcct tcagataaag ctgtgaaaaa agatgcaagt 2340
gcaacatcca agaaaaaact taaaaaggtg gacaagattg aggaacttga tcaagaaaat 2400
gaagctgcat tggaaaatgg gattaaaaat gaggaaaata cagaaccagg tgctgaatct 2460
gctgagaatg ctgatgatcc aaacaaagac gcaagtgata attcagatgg ccaaaatgat 2520
gagaacaagg aggactatac aattccagat gagtatagaa ttggaccata tcagcctaat 2580
gttcctggtg gtatagacta tgtgatacct aaaacagggt tttactgtaa gctgtgttca 2640
ctcttttata caaatgaaga agttgcaaag aatactcatt gcagcagcct tcctcattat 2700
cagaaattaa agaaatttct gaataaattg gcagaagaac gcaggcagaa gaaggaaagt 2760
taagatgtac aagaagcttt tatttcaaag aaaacaatgg ttctttgatt ttaatgttaa 2820
ccttttttaa atacaatact gatagttaga aaactactgt gactcttttg ttttagtgga 2880
aaataataga tgtctgttca tgtgtttaag tgttatagca aaaagtacac atacggttaa 2940
gttaatgaaa aagtttttgt ttcatcagtg acaacagaca aaagtacttc ctagagattt 3000
gctgttgtaa gtaagctatg gaaaaacaac ttgcaccact aagagaaaga taacatagaa 3060
ccattcagaa aactgaaatt tagtagttta aggcttcaaa gaaatgtcaa cattttgatt 3120
ccattcaata aagtacaaaa ccaatgtatt ttttattact tttcatctga aacagtccac 3180
attttaatct gagccttgca gacttttcat ttgtgacgtt tgtaagctgt ttcggttgca 3240
ttgcattttt agagaactta attaacgtga gaattcaatt gcaggtgcag ttttctttta 3300
atgttcatgc tgtttagatg ataagatata ttaagtaatt ggctttagat ttgtaatttt 3360
tttcctctga gttcctgcta ggttttgtat tgtagtagtc aatgttattt cagtgaaatg 3420
taaaaatatt accgttctct ttgaccagta ttaatttctt gagaacctat tccttgtcac 3480
ttgaatcctg tagctgtcaa acatctccgg tataagcaac atttgatttt tgaagtgtgt 3540
aaaccatcta tattttcaag atgtaatttt acatttctgc attttaaaag tttggccata 3600
attctagaag cacgttctaa ttatgtacct acacatgtga cctatgtgag cagaaatttg 3660
catgtataat ctgtgtttac ttgtaactct ctggttatat actgcttata tctgtggatt 3720
caagttactg aagtgaatac caataaaaaa aaaaacaagc t 3761

<210> 135
<211> 1523
<212> PRT
<213> Homo sapiens

<220>
<223> KIAA0287

<400> 135
Val Gln Thr Leu Gly Gly Trp Val Gln Pro Glu Thr Arg Thr Lys Glu
1               5                   10                  15

Glu Ile Ile Glu Leu Leu Val Leu Glu Gln Tyr Leu Thr Ile Ile Pro
            20                  25                  30

Glu Lys Leu Lys Pro Trp Val Arg Ala Lys Lys Pro Glu Asn Cys Glu
        35                  40                  45

Lys Leu Val Thr Leu Leu Glu Asn Tyr Lys Glu Met Tyr Gln Pro Glu
    50                  55                  60

Asp Asp Asn Asn Ser Asp Val Thr Ser Asp Asp Asp Met Thr Arg Asn
65                  70                  75                  80

Arg Arg Glu Ser Ser Pro Pro His Ser Val His Ser Phe Ser Gly Asp
                85                  90                  95

Arg Asp Trp Asp Arg Arg Gly Arg Ser Arg Asp Met Glu Pro Arg Asp
            100                 105                 110

Arg Trp Ser His Thr Arg Asn Pro Arg Ser Arg Met Pro Pro Arg Asp
        115                 120                 125

Leu Ser Leu Pro Val Val Ala Lys Thr Ser Phe Glu Met Asp Arg Glu
    130                 135                 140

Asp Asp Arg Asp Ser Arg Ala Tyr Glu Ser Arg Ser Gln Asp Ala Glu
145                 150                 155                 160

Ser Tyr Gln Asn Val Val Asp Leu Ala Glu Asp Arg Lys Pro His Asn
                165                 170                 175

Thr Ile Gln Asp Asn Met Glu Asn Tyr Arg Lys Leu Leu Ser Leu Gly
            180                 185                 190

Val Gln Leu Ala Glu Asp Asp Gly His Ser His Met Thr Gln Gly His
        195                 200                 205

Ser Ser Arg Ser Lys Arg Ser Ala Tyr Pro Ser Thr Ser Arg Gly Leu
    210                 215                 220

Lys Thr Met Pro Glu Ala Lys Lys Ser Thr His Arg Arg Gly Ile Cys
225                 230                 235                 240

Glu Asp Glu Ser Ser His Gly Val Ile Met Glu Lys Phe Ile Lys Asp
                245                 250                 255

Val Ser Arg Ser Ser Lys Ser Gly Arg Ala Arg Glu Ser Ser Asp Arg
            260                 265                 270

Ser Gln Arg Phe Pro Arg Met Ser Asp Asp Asn Trp Lys Asp Ile Ser
        275                 280                 285

Leu Asn Lys Arg Glu Ser Val Ile Gln Gln Arg Val Tyr Glu Gly Asn
    290                 295                 300

Ala Phe Arg Gly Gly Phe Arg Phe Asn Ser Thr Leu Val Ser Arg Lys
305                 310                 315                 320

Arg Val Leu Glu Arg Lys Arg Arg Tyr His Phe Asp Thr Asp Gly Lys
                325                 330                 335

Gly Ser Ile His Asp Gln Lys Gly Cys Pro Arg Lys Lys Pro Phe Glu
            340                 345                 350

Cys Gly Ser Glu Met Arg Lys Ala Met Ser Val Ser Ser Leu Ser Ser
        355                 360                 365

Leu Ser Ser Pro Ser Phe Thr Glu Ser Gln Pro Ile Asp Phe Gly Ala
    370                 375                 380

Met Pro Tyr Val Cys Asp Glu Cys Gly Arg Ser Phe Ser Val Ile Ser
385                 390                 395                 400

Glu Phe Val Glu His Gln Ile Met His Thr Arg Glu Asn Leu Tyr Glu
                405                 410                 415

Tyr Gly Glu Ser Phe Ile His Ser Val Ala Val Ser Glu Val Gln Lys

            420                 425                 430

Ser Gln Val Gly Gly Lys Arg Phe Glu Cys Lys Asp Cys Gly Glu Thr
        435                 440                 445

Phe Asn Lys Ser Ala Ala Leu Ala Glu His Arg Lys Ile His Ala Arg
    450                 455                 460

Gly Tyr Leu Val Glu Cys Lys Asn Gln Glu Cys Glu Glu Ala Phe Met
465                 470                 475                 480

Pro Ser Pro Thr Phe Ser Glu Leu Gln Lys Ile Tyr Gly Lys Asp Lys
                485                 490                 495

Phe Tyr Glu Cys Arg Val Cys Lys Glu Thr Phe Leu His Ser Ser Ala
            500                 505                 510

Leu Ile Glu His Gln Lys Ile His Phe Gly Asp Asp Lys Asp Asn Glu
        515                 520                 525

Arg Glu His Glu Arg Glu Arg Glu Arg Glu Arg Gly Glu Thr Phe Arg
    530                 535                 540

Pro Ser Pro Ala Leu Asn Glu Phe Gln Lys Met Tyr Gly Lys Glu Lys
545                 550                 555                 560

Met Tyr Glu Cys Lys Val Cys Gly Glu Thr Phe Leu His Ser Ser Ser
                565                 570                 575

Leu Lys Glu His Gln Lys Ile His Thr Arg Gly Asn Pro Phe Glu Asn
            580                 585                 590

Lys Gly Lys Val Cys Glu Glu Thr Phe Ile Pro Gly Gln Ser Leu Lys
        595                 600                 605

Arg Arg Gln Lys Thr Tyr Asn Lys Glu Lys Leu Cys Asp Phe Thr Asp
    610                 615                 620

Gly Arg Asp Ala Phe Met Gln Ser Ser Glu Leu Ser Glu His Gln Lys
625                 630                 635                 640

Ile His Ser Arg Lys Asn Leu Phe Glu Gly Arg Gly Tyr Glu Lys Ser
                645                 650                 655

Val Ile His Ser Gly Pro Phe Thr Glu Ser Gln Lys Ser His Thr Ile
            660                 665                 670

Thr Arg Pro Leu Glu Ser Asp Glu Asp Glu Lys Ala Phe Thr Ile Ser
        675                 680                 685

Ser Asn Pro Tyr Glu Asn Gln Lys Ile Pro Thr Lys Glu Asn Val Tyr
    690                 695                 700

Glu Ala Lys Ser Tyr Glu Arg Ser Val Ile His Ser Leu Ala Ser Val
705                 710                 715                 720

Glu Ala Gln Lys Ser His Ser Val Ala Gly Pro Ser Lys Pro Lys Val
                725                 730                 735

Met Ala Glu Ser Thr Ile Gln Ser Phe Asp Ala Ile Asn His Gln Arg
            740                 745                 750

Val Arg Ala Gly Gly Asn Thr Ser Glu Gly Arg Glu Tyr Ser Arg Ser
        755                 760                 765

Val Ile His Ser Leu Val Ala Ser Lys Pro Pro Arg Ser His Asn Gly
    770                 775                 780

Asn Glu Leu Val Glu Ser Asn Glu Lys Gly Glu Ser Ser Ile Tyr Ile
785                 790                 795                 800

Ser Asp Leu Asn Asp Lys Arg Gln Lys Ile Pro Ala Arg Glu Asn Pro
                805                 810                 815

Cys Glu Gly Gly Ser Lys Asn Arg Asn Tyr Glu Asp Ser Val Ile Gln
            820                 825                 830

Ser Val Phe Arg Ala Lys Pro Gln Lys Ser Val Pro Gly Glu Gly Ser
        835                 840                 845

Gly Glu Phe Lys Lys Asp Gly Glu Phe Ser Val Pro Ser Ser Asn Val
    850                 855                 860

Arg Glu Tyr Gln Lys Ala Arg Ala Lys Lys Lys Tyr Ile Glu His Arg
865                 870                 875                 880

Ser Asn Glu Thr Ser Val Ile His Ser Leu Pro Phe Gly Glu Gln Thr
                885                 890                 895

Phe Arg Pro Arg Gly Met Leu Tyr Glu Cys Gln Glu Cys Gly Glu Cys
            900                 905                 910

Phe Ala His Ser Ser Asp Leu Thr Glu His Gln Lys Ile His Asp Arg
        915                 920                 925

Glu Lys Pro Ser Gly Ser Arg Asn Tyr Glu Trp Ser Val Ile Arg Ser
    930                 935                 940

Leu Ala Pro Thr Asp Pro Gln Thr Ser Tyr Ala Gln Glu Gln Tyr Ala
945                 950                 955                 960

Lys Glu Gln Ala Arg Asn Lys Cys Lys Asp Phe Arg Gln Phe Phe Ala
                965                 970                 975

Thr Ser Glu Asp Leu Asn Thr Asn Gln Lys Ile Tyr Asp Gln Glu Lys
            980                 985                 990

Ser His Gly Glu Glu Ser Gln Gly Glu Asn Thr Asp Gly Glu Glu Thr
        995                 1000                1005

His Ser Glu Glu Thr His Gly Gln Glu Thr Ile Glu Asp Pro Val Ile
    1010                1015                1020

Gln Gly Ser Asp Met Glu Asp Pro Gln Lys Asp Asp Pro Asp Asp Lys
1025                1030                1035                1040

Ile Tyr Glu Cys Glu Asp Cys Gly Leu Gly Phe Val Asp Leu Thr Asp
                1045                1050                1055

Leu Thr Asp His Gln Lys Val His Ser Arg Lys Cys Leu Val Asp Ser
            1060                1065                1070

Arg Glu Tyr Thr His Ser Val Ile His Thr His Ser Ile Ser Glu Tyr
        1075                1080                1085

Gln Arg Asp Tyr Thr Gly Glu Gln Leu Tyr Glu Cys Pro Lys Cys Gly
    1090                1095                1100

Glu Ser Phe Ile His Ser Ser Phe Leu Phe Glu His Gln Arg Ile His
1105                1110                1115                1120

Glu Gln Asp Gln Leu Tyr Ser Met Lys Gly Cys Asp Asp Gly Phe Ile
                1125                1130                1135

Ala Leu Leu Pro Met Lys Pro Arg Arg Asn Arg Ala Ala Glu Arg Asn
            1140                1145                1150

Pro Ala Leu Ala Gly Ser Ala Ile Arg Cys Leu Leu Cys Gly Gln Gly
        1155                1160                1165

Phe Ile His Ser Ser Ala Leu Asn Glu His Met Arg Leu His Arg Glu
    1170                1175                1180

Asp Asp Leu Leu Glu Gln Ser Gln Met Ala Glu Glu Ala Ile Ile Pro
1185                1190                1195                1200

Gly Leu Ala Leu Thr Glu Phe Gln Arg Ser Gln Thr Glu Glu Arg Leu
                1205                1210                1215

Phe Glu Cys Ala Val Cys Gly Glu Ser Phe Val Asn Pro Ala Glu Leu
            1220                1225                1230

Ala Asp His Val Thr Val His Lys Asn Glu Pro Tyr Glu Tyr Gly Ser
        1235                1240                1245

Ser Tyr Thr His Thr Ser Phe Leu Thr Glu Pro Leu Lys Gly Ala Ile
    1250                1255                1260

Pro Phe Tyr Glu Cys Lys Asp Cys Gly Lys Ser Phe Ile His Ser Thr
1265                1270                1275                1280

Val Leu Thr Lys His Lys Glu Leu His Leu Glu Glu Glu Glu Glu Asp
                1285                1290                1295

Glu Ala Ala Ala Ala Ala Ala Ala Ala Ala Gln Glu Val Glu Ala Asn
            1300                1305                1310

Val His Val Pro Gln Val Val Leu Arg Ile Gln Gly Leu Asn Val Glu
        1315                1320                1325

Ala Ala Glu Pro Glu Val Glu Ala Ala Glu Pro Glu Val Glu Ala Ala
    1330                1335                1340

Glu Pro Glu Val Glu Ala Ala Glu Pro Asn Gly Glu Ala Glu Gly Pro
1345                1350                1355                1360

Asp Gly Glu Ala Ala Glu Pro Ile Gly Glu Ala Gly Gln Pro Asn Gly
                1365                1370                1375

Glu Ala Glu Gln Pro Asn Gly Asp Ala Asp Glu Pro Asp Gly Ala Gly
            1380                1385                1390

Ile Glu Asp Pro Glu Glu Arg Ala Glu Glu Pro Glu Gly Lys Ala Glu

1395                1400                1405

Glu Pro Glu Gly Asp Ala Asp Glu Pro Asp Gly Val Gly Ile Glu Asp
    1410                1415                1420

Pro Glu Glu Gly Glu Asp Gln Glu Ile Gln Val Glu Glu Pro Tyr Tyr
1425                1430                1435                1440

Asp Cys His Glu Cys Thr Glu Thr Phe Thr Ser Ser Thr Ala Phe Ser
                1445                1450                1455

Glu His Leu Lys Thr His Ala Ser Met Ile Ile Phe Glu Pro Ala Asn
            1460                1465                1470

Ala Phe Gly Glu Cys Ser Gly Tyr Ile Glu Arg Ala Ser Thr Ser Thr
        1475                1480                1485

Gly Gly Ala Asn Gln Ala Asp Glu Lys Tyr Phe Lys Cys Asp Val Cys
    1490                1495                1500

Gly Gln Leu Phe Asn Asp Arg Leu Ser Leu Ala Arg His Gln Asn Thr
1505                1510                1515                1520

His Thr Gly

<210> 136
<211> 5994
<212> DNA
<213> Homo sapiens

<220>
<223> KIAA0287

<400> 136
gaggtttggg aggcgcggga gatgtccacc ctgggctggt ggcgccgccg ggcgccgggc 60
gccatgaggg tgcgctaggc ggctgttcgt gcccgaggct gcgcagcact gaggtgagct 120
ttgccttctt gatcttccgt ccttcttgga gacgactggc gagaggaaga gggactaggt 180
ccaaacgcta ggtggctggg tccagccgga gacccgcacc aaggaggaga tcatcgagct 240
cttggtcctt gagcagtacc tgaccatcat ccctgaaaag ctcaagcctt gggtgcgagc 300
aaaaaagccg gagaactgtg agaagctcgt cactctgctg gagaattaca aggagatgta 360
ccaaccagaa gacgacaaca acagtgacgt gaccagcgac gacgacatga cccggaacag 420
aagagagtcc tcaccacctc actcagtcca ttctttcagt ggtgaccggg actgggaccg 480
gaggggcaga agcagagaca tggagccacg agaccgctgg tcccacacca ggaacccaag 540
aagcaggatg cctccgcggg atctttccct tcctgtggtg gcgaaaacaa gctttgaaat 600
ggacagagag gacgacaggg actccagggc ttatgagtcc cgatctcagg atgctgaatc 660
ataccaaaat gtggtggacc tcgctgagga caggaaacct cacaacacaa tccaggacaa 720
catggaaaac tacaggaagc tgctctccct cggagtgcag cttgctgaag acgatggcca 780
ctcccacatg acgcagggcc actcatcaag atccaagaga agtgcctacc caagcaccag 840
tcgaggtcta aaaactatgc ctgaagccaa aaaatcaacc caccggcggg ggatttgtga 900
agatgaatct tcccacggag tgataatgga aaaattcatc aaggatgtgt cacgcagttc 960
caaatcggga agagcaaggg agtcaagcga ccggtcacag agattcccca gaatgtcaga 1020
tgataactgg aaggacattt cattgaacaa gagggagtca gtgatccagc agcgggttta 1080
tgaagggaat gcatttaggg gaggctttag gtttaattca acccttgttt ccagaaagag 1140
agttcttgaa agaaagaggc gctatcattt tgacacagat gggaagggct cgattcacga 1200
tcaaaaaggc tgtcccagga agaagccctt tgaatgtggt agtgagatga gaaaagccat 1260
gagcgtgagc agcctgagca gcctcagctc ccctcctttt accgagtcac agccaattga 1320
ttttggggca atgccatatg tatgtgatga gtgtgggagg tcgttcagtg tcatctcaga 1380
atttgttgag caccagatca tgcatactag agagaacctc tatgagtatg gtgagtcctt 1440
tatccacagt gtggctgtca gtgaagttca gaaaagtcag gttggaggga aacgttttga 1500

atgtaaggac tgtggagaga ccttcaataa gagtgccgcc ttggctgaac atcggaagat 1560
tcatgctaga ggttatcttg tggaatgtaa gaatcaggaa tgtgaggaag ccttcatgcc 1620
tagccccacc tttagtgagc ttcagaaaat atatggcaaa gacaaattct acgagtgcag 1680
ggtgtgtaag gaaaccttcc ttcatagttc tgccctgatt gagcaccaga aaatccactt 1740
tggggatgac aaagataatg agcgtgaaca tgaacgtgaa cgtgaacgtg agcgcgggga 1800
aacctttagg cccagcccag cccttaatga gtttcagaaa atgtatggta aagagaaaat 1860
gtacgaatgt aaggtgtgtg gggagacttt ccttcatagc tcatccctga aagaacatca 1920
gaaaatccat actagaggga acccatttga aaacaagggt aaagtgtgtg aggaaacctt 1980
tattcctggt cagtccctta aaaggcgtca gaaaacttac aataaggaga agctctgtga 2040
ctttacagat ggccgggatg ccttcatgca aagctcagag ctcagtgagc atcagaaaat 2100
tcattctcga aagaacctct ttgaaggcag agggtatgag aaatctgtca ttcatagtgg 2160
gccattcact gaatctcaga agagtcatac tataacaaga cctcttgaaa gtgatgagga 2220
cgaaaaggcg ttcaccatta gctctaaccc ctatgaaaac cagaagattc ccactaagga 2280
aaatgtctac gaggcaaaat catatgagag gtctgttatt catagcttag cctctgtgga 2340
agctcagaaa agtcacagtg tagcagggcc cagtaaacca aaagtaatgg cagagtctac 2400
cattcagagc ttcgatgcta tcaaccatca gagagttcgt gctggaggga acacctctga 2460
aggaagggaa tacagtaggt ctgttatcca tagcttagtg gcttccaaac ctccaagaag 2520
tcacaatgga aatgaattgg tggaatctaa tgagaaggga gaatcctcca tttatatctc 2580
agaccttaat gataagcgac agaagattcc tgccagagag aacccttgtg aagggggcag 2640
taagaatcgc aactatgaag actctgtcat acagagtgta ttccgtgcca aacctcagaa 2700
aagtgttcct ggagagggat ctggtgagtt taagaaggat ggcgaattct ctgttcccag 2760
ctcaaatgtc cgtgaatacc agaaggctcg tgctaaaaag aaatacattg agcataggag 2820
caatgagacc tctgtaattc actctctgcc ttttggtgaa caaacatttc gccctcgagg 2880
gatgctctat gaatgtcagg agtgtgggga gtgctttgct catagctctg acctcactga 2940
gcaccagaag attcatgata gggagaagcc ctctggaagc agaaactatg aatggtctgt 3000
cattcgcagc ttggccccta ctgaccctca aacaagttac gcccaagagc agtatgctaa 3060
agagcaagcg cggaacaaat gtaaggactt cagacaattt tttgctacca gcgaagacct 3120
caacacaaac cagaaaatct atgaccaaga gaagtctcat ggcgaggagt ctcaaggcga 3180
gaatactgat ggggaggaga cccacagcga ggagacccat ggtcaggaga caattgaaga 3240
ccctgtcatt caaggctcag acatggaaga ccctcagaag gatgaccctg atgacaaaat 3300
ctatgaatgt gaggactgtg gcctgggctt tgtggatctc acagacctca cagaccatca 3360
gaaagtccac agcaggaagt gcctggttga cagtcgggag tacacacatt ctgtaattca 3420
cacccattcc atcagcgagt atcagagaga ttacactgga gagcagctgt atgaatgtcc 3480
aaagtgtggg gaatctttta ttcatagctc attccttttc gagcatcaga gaatccatga 3540
acaagaccag ttgtattcca tgaaggggtg tgatgatggt tttattgccc tcttgcccat 3600
gaagccacgg aggaatcgtg ctgcagagag gaatcctgct cttgctgggt cggccattcg 3660
atgccttttg tgtggacaag gcttcattca tagctctgcc cttaatgagc atatgagact 3720
tcatagggaa gatgatttac tggagcagag ccagatggct gaggaagcta tcattccagg 3780
cttagccctc actgagtttc agagaagtca gaccgaagag agactctttg aatgtgcagt 3840
ctgtggagaa tctttcgtca acccagcaga acttgcagat cacgtaactg ttcataagaa 3900
tgagccctat gagtacgggt cctcctatac tcacacctca tttcttactg agcccctcaa 3960
aggagctata ccattctatg aatgcaagga ttgtggtaag tcctttattc atagcacagt 4020
cctcactaaa cataaggagc ttcatctgga agaagaagaa gaagatgaag cagcagcagc 4080
tgcagcagca gcagcccagg aagttgaagc caatgtccat gttccacaag tagttctgag 4140
gattcagggc ttaaacgtag aggctgctga gccagaagtg gaggctgccg agccagaagt 4200
ggaggctgct gagccagaag tggaggctgc tgagccaaac ggagaggctg aagggccaga 4260
tggagaggct gcagagccca ttggagaggc tggacagcca aatggagagg ccgagcagcc 4320
aaatggggat gctgatgagc cagatggtgc aggtattgaa gacccagaag aaagagctga 4380
agagccagag ggaaaagctg aagagccaga gggagatgcc gacgagcctg acggtgtggg 4440
aattgaagac ccagaagaag gtgaagatca agagattcag gtagaagaac catactatga 4500
ctgccatgaa tgcacagaaa ccttcacttc cagcacagca ttcagtgaac acctgaaaac 4560
tcatgccagc atgatcatat ttgagcctgc aaatgccttt ggggagtgct caggctacat 4620
cgaacgtgcc agcaccagca caggtggtgc caatcaagct gatgagaagt acttcaaatg 4680
tgacgtctgt gggcagctct tcaatgaccg cctgtccctc gccagacacc agaataccca 4740
cactggctga gggcatgggg taaaggttag aaaaccttca cctaggactt gacccttacc 4800
aaaccacaga gaatccaaac caatccatga taatgtcagt aggagactta accttagtgt 4860
gttacacacc tgacttaaca tctctaaact cagattgaaa agagaccgaa tgtgcagatt 4920
ccacagtctt aagctttccc cttcagatgt cagtgtctgc atgtgggaaa gccatagcac 4980
acatcttacc tttccaagta atcagattga gaaaacccta tgagtattcc agactacaga 5040
gtttgcccaa atcaactgta aatgacactt gtgtaacgta tatatagtgt ttcatgaggt 5100
gtatataaaa tagcaaatta tgacagaaca gtgatcacat atatttggat ttatatgata 5160

tacagttaca gtttactctg cagaggtacc ttacctggta ttctttgaat tttttttttt 5220
tttggaggag gaagagagca acaaatttga ttatattttt aagtgtctta gatcctgaga 5280
aagatttatt gtgcattatt tgaaccctgt caatatcttt ttgagtaatt gttttgtttc 5340
ttacccttaa atagtcttgt gaagctgtag gcatgataga taacatggct tttactcctt 5400
actgtttgaa aagataagta ctttagcttc tttctgcagc catttcatct gcaccaacac 5460
tttggaacct aatactgtgt aaggctttac aatatacgga ttggcttttt gtgacccaga 5520
ttgattggtt gccacatgtt atgtttgttg aagtggttct catgcaaaaa tattacacat 5580
ttgtgttctg ggtttttttt tttttttaac caactcaata tgtgtttgat gatagtgaat 5640
tgataaaacc cgaagctttt ccctgtaaat cttacatctt tgcctttaaa gaatgggtta 5700
caaccatcac tagatcacag tagtgcctaa tgaaggttga gaaccgtagg agaggctctc 5760
atgctgtaaa taatgttgca ggctaataac ctttcatcac ttcctttgtg cgcttcctgc 5820
cttaagtgac aagtagcaac atggcttggg tcccctgtgc agcatcagct tatgctgcca 5880
caagtcagtt tgcaccctag gtgcccagga gctagtatcc ttagatcttt ctatcgctaa 5940
cttaattctc ttcgttattt atctgaccct ctaactccat gtctaacttg catt 5994

<210> 137
<211> 225
<212> PRT
<213> Mus musculus

<220>
<223> Rho-GDI3

<400> 137
Met Leu Gly Leu Asp Ala Cys Glu Leu Gly Glu Gln Leu Leu Glu Leu
1               5                   10                  15

Leu Arg Leu Ala Leu Cys Ala Arg Val Leu Leu Ala Asp Lys Asp Gly
            20                  25                  30

Glu Ser Thr Pro Ser Asp Glu Val Leu Asp Glu Ile Val Pro Glu Tyr
        35                  40                  45

Gln Ala Pro Gly Lys Lys Ser Met Leu Ala Ile Trp Gln Leu Asp Pro
    50                  55                  60

Gly Asp Val Ser Leu Val Lys Tyr Lys Gln Ala Leu Leu Gly Pro Leu
65                  70                  75                  80

Pro Pro Ile Met Asp Pro Ser Leu Pro Asn Val Gln Val Thr Arg Leu
                85                  90                  95

Thr Leu Leu Thr Glu Gln Ala Pro Gly Pro Ile Ile Met Asp Leu Thr
            100                 105                 110

Gly Asp Leu Asp Ala Leu Lys Asn Gln Val Phe Val Leu Lys Glu Gly
        115                 120                 125

Ile Glu Tyr Lys Val Lys Ile Thr Phe Lys Val Asn Lys Glu Ile Val
    130                 135                 140

Ser Gly Leu Lys Cys Leu His His Thr Tyr Arg Arg Gly Leu Arg Val
145                 150                 155                 160

Asp Lys Ala Ile Phe Met Val Gly Ser Tyr Gly Pro Arg Ala Gln Glu
                165                 170                 175

Tyr Glu Phe Val Thr Ser Val Glu Glu Ala Pro Arg Gly Ala Leu Ala
            180                 185                 190

Arg Gly Leu Tyr Val Val Arg Ser Leu Phe Thr Asp Asp Asp Arg Leu
        195                 200                 205

Asn His Leu Ser Trp Glu Trp His Leu His Val Cys Gln Asp Trp Lys
    210                 215                 220

Asp
225


<210> 138
<211> 865
<212> DNA
<213> Mus musculus

<220>
<223> Rho-GDI3

<400> 138
ccgagcgggc ggcggctccg cggcgggccg cggctaccat gctgggcctg gacgcgtgtg 60
agctggggga gcagctgttg gagctgctcc ggctggcgct gtgcgcccga gtcctcttgg 120
ctgacaagga tggggagtca actccatcag atgaggtgct ggacgaaatt gtaccagagt 180
accaggcccc tgggaagaag agcatgctgg caatctggca actggacccc ggggacgtga 240
gcttggtcaa gtacaagcag gcgctgctgg gacccctgcc acccattatg gaccccagcc 300
tgcccaacgt gcaggtgact aggctgacac tgctgacgga gcaggctcca gggcctatca 360
tcatggacct tacaggagac ctagatgcac tgaaaaacca ggtgtttgtc ctgaaggagg 420
gcattgaata caaggtgaag atcaccttca aggtcaacaa ggagattgtc agtggcctca 480
aatgtctgca tcacacctat cgtcggggac tgcgtgtgga caaggccatc ttcatggtgg 540
gaagctacgg ccccagagcc caggagtatg aatttgtgac ttcagtggag gaagcaccaa 600
gaggagcatt ggcacgtggc ctctacgtgg tcaggtccct cttcactgat gacgacaggc 660
tgaaccacct gtcctgggag tggcacctcc atgtctgcca ggactggaag gactgaaccc 720
cacttggggt ctgtattccc aatttcttgt cagttgcaga gacctgcaag cattccctag 780
acctcccagt tggtgaccag atgtccccca ctctctgctg tgtcccccct gcctggtgcc 840
tattaaatgt taccttgtct tgcca                                        865


<210> 139
<211> 758
<212> PRT
<213> Homo sapiens

<220>
<223> Motor protein

<400> 139
Met Leu Arg Ala Cys Gln Leu Ser Gly Val Thr Ala Ala Ala Gln Ser
  1               5                  10                  15

Cys Leu Cys Gly Lys Phe Val Leu Arg Pro Leu Arg Pro Cys Arg Arg
             20                  25                  30

Tyr Ser Thr Ser Gly Ser Ser Gly Leu Thr Thr Gly Lys Ile Ala Gly
         35                  40                  45

Ala Gly Leu Leu Phe Val Gly Gly Gly Ile Gly Gly Thr Ile Leu Tyr
     50                  55                  60

Ala Lys Trp Asp Ser His Phe Arg Glu Ser Val Glu Lys Thr Ile Pro
 65                  70                  75                  80

Tyr Ser Asp Lys Leu Phe Glu Met Val Leu Gly Pro Ala Ala Tyr Asn

85                  90                  95

Val Pro Leu Pro Lys Lys Ser Ile Gln Ser Gly Pro Leu Lys Ile Ser
            100                 105                 110

Ser Val Ser Glu Val Met Lys Glu Ser Lys Gln Pro Ala Ser Gln Leu
        115                 120                 125

Gln Lys Gln Lys Gly Asp Thr Pro Ala Ser Ala Thr Ala Pro Thr Glu
    130                 135                 140

Ala Ala Gln Ile Ile Ser Ala Ala Gly Asp Thr Leu Ser Val Pro Ala
145                 150                 155                 160

Pro Ala Val Gln Pro Glu Glu Ser Leu Lys Thr Asp His Pro Glu Ile
                165                 170                 175

Gly Glu Gly Lys Pro Thr Pro Ala Leu Ser Glu Glu Ala Ser Ser Ser
            180                 185                 190

Ser Ile Arg Glu Arg Pro Pro Glu Glu Val Ala Ala Arg Leu Ala Gln
        195                 200                 205

Gln Glu Lys Gln Glu Gln Val Lys Ile Glu Ser Leu Ala Lys Ser Leu
    210                 215                 220

Glu Asp Ala Leu Arg Gln Thr Ala Ser Val Thr Leu Gln Ala Ile Ala
225                 230                 235                 240

Ala Gln Asn Ala Ala Val Gln Ala Val Asn Ala His Ser Asn Ile Leu
                245                 250                 255

Lys Ala Ala Met Asp Asn Ser Glu Ile Ala Gly Glu Lys Lys Ser Ala
            260                 265                 270

Gln Trp Arg Thr Val Glu Gly Ala Leu Lys Glu Arg Arg Lys Ala Val
        275                 280                 285

Asp Glu Ala Ala Asp Ala Leu Leu Lys Ala Lys Glu Glu Leu Glu Lys
    290                 295                 300

Met Lys Ser Val Ile Glu Asn Ala Lys Lys Lys Glu Val Ala Gly Ala
305                 310                 315                 320

Lys Pro His Ile Thr Ala Ala Glu Gly Lys Leu His Asn Met Ile Val
                325                 330                 335

Asp Leu Asp Asn Val Val Lys Lys Val Gln Ala Ala Gln Ser Glu Ala
            340                 345                 350

Lys Val Val Ser Gln Tyr His Glu Leu Val Val Gln Ala Arg Asp Asp
        355                 360                 365

Phe Lys Arg Glu Leu Asp Ser Ile Thr Pro Glu Val Leu Pro Gly Trp
    370                 375                 380

Lys Gly Met Ser Val Ser Asp Leu Ala Asp Lys Leu Ser Thr Asp Asp
385                 390                 395                 400

Leu Asn Ser Leu Ile Ala His Ala His Arg Arg Ile Asp Gln Leu Asn
                405                 410                 415

Arg Glu Leu Ala Glu Gln Lys Ala Thr Glu Lys Gln His Ile Thr Leu
            420                 425                 430

Ala Leu Glu Lys Gln Lys Leu Glu Glu Lys Arg Ala Phe Asp Ser Ala
        435                 440                 445

Val Ala Lys Ala Leu Glu His His Arg Ser Glu Ile Gln Ala Glu Gln
    450                 455                 460

Asp Arg Lys Ile Glu Glu Val Arg Asp Ala Met Glu Asn Glu Met Arg
465                 470                 475                 480

Thr Gln Leu Arg Arg Gln Ala Ala Ala His Thr Asp His Leu Arg Asp
                485                 490                 495

Val Leu Arg Val Gln Glu Gln Glu Leu Lys Ser Glu Phe Glu Gln Asn
            500                 505                 510

Leu Ser Glu Lys Leu Ser Glu Gln Glu Leu Gln Phe Arg Arg Leu Ser
        515                 520                 525

Gln Glu Gln Val Asp Asn Phe Thr Leu Asp Ile Asn Thr Ala Tyr Ala
    530                 535                 540

Arg Leu Arg Gly Ile Glu Gln Ala Val Gln Ser His Ala Val Ala Glu
545                 550                 555                 560

Glu Glu Ala Arg Lys Ala His Gln Leu Trp Leu Ser Val Glu Ala Leu
                565                 570                 575

Lys Tyr Ser Met Lys Thr Ser Ser Ala Glu Thr Pro Thr Ile Pro Leu
            580                 585                 590

Gly Ser Ala Val Glu Ala Ile Lys Ala Asn Cys Ser Asp Asn Glu Phe
        595                 600                 605

Thr Gln Ala Leu Thr Ala Ala Ile Pro Pro Glu Ser Leu Thr Arg Gly
    610                 615                 620

Val Tyr Ser Glu Glu Thr Leu Arg Ala Arg Phe Tyr Ala Val Gln Lys
625                 630                 635                 640

Leu Ala Arg Arg Val Ala Met Ile Asp Glu Thr Arg Asn Ser Leu Tyr
                645                 650                 655

Gln Tyr Phe Leu Ser Tyr Leu Gln Ser Leu Leu Leu Phe Pro Pro Gln
            660                 665                 670

Gln Leu Lys Pro Pro Pro Glu Leu Cys Pro Glu Asp Ile Asn Thr Phe
        675                 680                 685

Lys Leu Leu Ser Tyr Ala Ser Tyr Cys Ile Glu His Gly Asp Leu Glu
    690                 695                 700

Leu Ala Ala Lys Phe Val Asn Gln Leu Lys Gly Glu Ser Arg Arg Val
705                 710                 715                 720

Ala Gln Asp Trp Leu Lys Glu Ala Arg Met Thr Leu Glu Thr Lys Gln
                725                 730                 735

Ile Val Glu Ile Leu Thr Ala Tyr Ala Ser Ala Val Gly Ile Gly Thr
            740                 745                 750

Thr Gln Val Gln Pro Glu
        755

<210> 140
<211> 2690
<212> DNA
<213> Homo sapiens

<220>
<223> Motor protein

<400> 140
cccggacaca cgcaagcacg cctccactta actcgcgccg ccgcggcagc tcgagtccac 60
cagcagcgcc gtccgcttga ccgagatgct gcgggcctgt cagttatcgg gtgtgaccgc 120
cgccgcccag agttgtctct gtgggaagtt tgtcctccgt ccattgcgac catgccgcag 180
atactctact tcaggcagct ctgggttgac tactggcaaa attgctggag ctggcctttt 240
gtttgttggt ggaggtattg gtggcactat cctatatgcc aaatgggatt cccatttccg 300
ggaaagtgta gagaaaacca taccttactc agacaaactc ttcgagatgg ttcttggtcc 360
tgcagcttat aatgttccat tgccaaagaa atcgattcag tcgggtccac taaaaatctc 420
tagtgtatca gaagtaatga aagaatctaa acagcctgcc tcacaactcc aaaaacaaaa 480
gggagatact ccagcttcag caacagcacc tacagaagcg gctcaaatta tttctgcagc 540
aggtgatacc ctgtcggtcc cagcccctgc agttcagcct gaggaatctt taaaaactga 600
tcaccctgaa attggtgaag gaaaacccac acctgcactt tcagaagaag catcctcatc 660
ttctataagg gagcgaccac ctgaagaagt tgcagctcgc cttgcacaac aggaaaaaca 720
agaacaagtt aaaattgagt ctctagccaa gagcttagaa gatgctctga ggcaaactgc 780
aagtgtcact ctgcaggcta ttgcagctca gaatgctgcg gtccaggctg tcaatgcaca 840
ctccaacata ttgaaagccg ccatggacaa ttctgagatt gcaggcgaga agaaatctgc 900
tcagtggcgc acagtggagg gtgcattgaa ggaacgcaga aaggcagtag atgaagctgc 960
cgatgccctt ctcaaagcca aagaagagtt agagaagatg aaaagtgtga ttgaaaatgc 1020
aaagaaaaaa gaggttgctg gggccaagcc tcatataact gctgcagagg gtaaacttca 1080
caacatgata gttgatctgg ataatgtggt caaaaaggtc caagcagctc agtctgaggc 1140
taaggttgta tctcagtatc atgagctggt ggtccaagct cgggatgact ttaaacgaga 1200
gctggacagt attactccag aagtccttcc tgggtggaaa ggaatgagtg tttcagactt 1260
agctgacaag ctctctactg atgatctgaa ctccctcatt gctcatgcac atcgtcgtat 1320
tgatcagctg aacagagagc tggcagaaca gaaggccacc gaaaagcagc acatcacgtt 1380
agccttggag aaacaaaagc tggaagaaaa gcgggcattt gactctgcag tagcaaaagc 1440
attagaacat cacagaagtg aaatacaggc tgaacaggac agaaagatag aagaagtcag 1500
agatgccatg gaaaatgaaa tgagaaccca gcttcgccga caggcagctg cccacactga 1560
tcacttgcga gatgtcctta gggtacaaga acaggaattg aagtctgaat ttgagcagaa 1620
cctgtctgag aaactctctg aacaagaatt acaatttcgt cgtctcagtc aagagcaagt 1680
tgacaacttt actctggata taaatactgc ctatgccaga ctcagaggaa tcgaacaggc 1740
tgttcagagc catgcagttg ctgaagagga agccagaaaa gcccaccaac tctggctttc 1800
agtggaggca ttaaagtaca gcatgaagac ctcatctgca gaaacaccta ctatcccgct 1860
gggtagtgcg gttgaggcca tcaaagccaa ctgttctgat aatgaattca cccaagcttt 1920
aaccgcagct atccctccag agtccctgac ccgtggggtg tacagtgaag agacccttag 1980
agcccgtttc tatgctgttc aaaaactggc ccgaagggta gcaatgattg atgaaaccag 2040
aaatagcttg taccagtact tcctctccta cctacagtcc ctgctcctat tcccacctca 2100
gcaactgaag ccgcccccag agctctgccc tgaggatata aacacattta aattactgtc 2160
atatgcttcc tattgcattg agcatggtga tctggagcta gcagcaaagt ttgtcaatca 2220
gctgaagggg gaatccagac gagtggcaca ggactggctg aaggaagccc gaatgaccct 2280
agaaacgaaa cagatagtgg aaatcctgac agcatatgcc agcgccgtag gaataggaac 2340
cactcaggtg cagccagagt gaggtttagg aagattttca taaagtcata tttcatgtca 2400
aaggaaatca gcagtgatag atgaagggtt cgcagcgaga gtcccggact tgtctagaaa 2460
tgagcaggtt tacaagtact gttctaaatg ttaacacctg ttgcatttat attctttcca 2520
tttgctatca tgtcagtgaa cgccaggagt gctttctttg caacttgtgt aacattttct 2580
gttttttcag gttttactga tgaggcttgt gaggccaatc aaaataatgt ttgtgatctc 2640
tactactgtt gattttgccc tcggagcaaa ctgaataaag caacaagatg 2690

<210> 141
<211> 220
<212> PRT
<213> Mus musculus

<220>
<223> Phosphatidylinositol transfer protein alpha

<400> 141
Met Val Leu Leu Lys Glu Tyr Arg Val Ile Leu Pro Val Ser Val Asp
1               5                   10                  15

Glu Tyr Gln Val Gly Gln Leu Tyr Ser Val Ala Glu Ala Ser Lys Asn
            20                  25                  30

Glu Thr Gly Gly Gly Glu Gly Val Glu Val Leu Val Asn Glu Pro Tyr
        35                  40                  45

Glu Lys Asp Asp Gly Glu Lys Gly Gln Tyr Thr His Lys Ile Tyr His
    50                  55                  60

Leu Gln Ser Lys Val Pro Thr Phe Val Arg Met Leu Ala Pro Glu Gly
65                  70                  75                  80

Ala Leu Asn Ile His Glu Lys Ala Trp Asn Ala Tyr Pro Tyr Cys Arg
                85                  90                  95

Thr Val Ile Thr Asn Glu Tyr Met Lys Glu Asp Phe Leu Ile Lys Ile
            100                 105                 110

Glu Thr Trp His Lys Pro Asp Leu Gly Thr Gln Glu Asn Val His Lys
        115                 120                 125

Leu Glu Pro Glu Ala Trp Lys His Val Glu Ala Ile Tyr Ile Asp Ile
    130                 135                 140

Ala Asp Arg Ser Gln Val Leu Ser Lys Asp Tyr Lys Ala Glu Glu Asp
145                 150                 155                 160

Pro Ala Lys Phe Lys Ser Val Lys Thr Gly Arg Gly Pro Leu Gly Pro
                165                 170                 175

Asn Trp Lys Gln Glu Leu Val Asn Gln Lys Asp Cys Pro Tyr Met Cys
            180                 185                 190

Ala Tyr Lys Leu Val Thr Val Lys Phe Lys Trp Trp Gly Leu Gln Asn
        195                 200                 205

Lys Val Glu Asn Phe Ile His Lys Gln Glu Lys Arg
    210                 215                 220


<210> 142
<211> 3724
<212> DNA
<213> Mus musculus

<220>
<223> Phosphatidylinositol transfer protein alpha

<400> 142
cggaaccgga gctccgggcg gcgtcggcag aggcgcggga ggtggtgagg ccagggcagg 60
gcgacaggag cccgcgcggc gaccgcagcg agagcagcgg ggacagagca gaccacgacg 120
aaggcgcaca ggcagcgggg ccgggccggg ccaccgggag agcggccacc gggtagcggg 180
cgggaggccg ggctggcagc tggcagccgc cgagccgcga agcgacatgg tgctgctcaa 240
ggagtatcgg gtcatcctgc ctgtgtctgt agatgagtat caagtggggc agctgtattc 300
tgtggctgaa gccagtaaaa atgaaactgg tggtggggaa ggtgtggagg tcctggtgaa 360
tgagccctac gagaaggacg atggcgagaa aggccagtac acacacaaga tctaccactt 420
acagagcaaa gtacccacct ttgttcgaat gctggcccca gaaggcgccc tgaatataca 480
cgagaaagcc tggaatgcct acccttactg cagaaccgtt attactaatg agtacatgaa 540
agaagatttt ctgattaaaa ttgaaacctg gcacaagcca gaccttggca cccaggagaa 600
tgtgcataaa ctggagcctg aggcatggaa acatgtggaa gctatatata tagacatcgc 660
tgatcgaagc caagtactta gcaaggatta caaggcagag gaagacccag caaaatttaa 720
atctgtcaaa acaggacgcg gacctttggg cccgaattgg aagcaagaac ttgtcaatca 780
gaaagactgc ccatatatgt gtgcatacaa actggttact gtcaagttca agtggtgggg 840
cttgcagaac aaagtggaaa actttataca taagcaagag aagcgtctgt ttacaaactt 900
tcacaggcag ctgttctgtt ggctcgataa atgggttgat ctgactatgg atgacattcg 960
gaggatggaa gaagagacga agagacagct ggatgagatg agacaaaagg acccagtgaa 1020
aggaatgaca gcagacgact agcgccaccg ccctttctgc actttttgca agacagtggt 1080
caacaggaag gcccagcagc tccaccctcc tgagtggcag gccatcttcg gacgcagcct 1140
ttctgtgctc attcttcagg caactttcaa tcccttacta tagctatttc tagatgccct 1200
ttaacattgt gaacaaatag accgtctggt attataaagc ctgtgtgtgc gggagcctga 1260
tcctctaaga tacatggtgt agctgctgtg tttacagctc atcctctctc cactgtgctc 1320
tgacccattt ctgtatgccc tcccctttta tttccaagtg atggtctcag tcttcccaat 1380
gagctgcctt ttgtgatgtg attatttaag ctcatttatt tcagccttgg gataaactga 1440
gctattttgc ttcctgggca gatctttggc aatgctgaat attcacttgg ggagagagga 1500
ggagttaaaa atacagagac acctaccccc agctcctcac aataggaatg tggcttcaga 1560
cccctgacct ctgctcctag taacctcttg tttgcgtgct ctaagcacca agaaggcacc 1620
caaggcccag ccagtctcag aaaatttagc aaataataaa taacccctac tcgagtcatt 1680
gcatctctgg ttaatattct tagaaatctt ggcttctcag ccctcctgtt gcccactctg 1740
tctggatctc tctgtagagc ctacaagacc ccaccactgc ccagctggag aactgtccag 1800
tatggcctgt tgacaccatt cagaccccac aggagcgctg tatgtccgtg ctcctttccc 1860
cttttgggct ggtgctgcca ctcagcaata atcctctctg tgctttctta gacccctgtc 1920
ctctgtgagg ctggctagga tgcaagagtc cttgtctttt catgctgcac aaacaagcat 1980
gcaaaaagct ttttatccct agcaaaacat gtaccttcct gtttctaatc actggacaag 2040
aatttgcaga taaagaactc agctcatcct gccccacact gagttctgtc ctggacaatc 2100
aaaagcaagc cgtgactgta gcctggaaca gttagcacag caggctgggg aagcccaggc 2160
tccaagggag tggaagctgc agagcactgg cttttctaga tgggcagaaa ctgaggatgc 2220
ctttctggtg ggagagtggg atagaattgt gtcacctgac atttcccaag ccttcttcag 2280
tcagtcagcc cagtgtgtag tgatccaagc ccatgattgc cagggtacct gttagagacc 2340
aaatctctac taggggagtg agaagtagga tggatcgttc tccttgtttt tgtctgtgtt 2400
ggccttactc atttctaagt gcaataaggc agtgtctcag ggactgcacc tgtgacatcc 2460
tgactggatg cttccctgtg gtcctcctgg aataagggta gacagatttg ggcccctcag 2520
catggttagc tctgaccttc cttgaggttc ttttaagacc aggagatgtc tggttactga 2580
cacctttcaa ggtattagtc tcctcacctt gtagagtctt taggcatgca cttggggcat 2640
cttggtgatt agcatatcag ctgctagtag catgggaaag ttggagaaga ggcattccta 2700
atggaaatgg aaaaaacata aatgttctca tataagctct gtattaccta tcaaatccta 2760
tttgtgctta aagatcttcc ttcagtaact aggttaaagt tcagatgtgt tatcttgaga 2820
gaagttgata gtctacgcag atagtttaca tgatatgagg gtcaaaagat atttgtctcc 2880
ttcagcactt cataagatct atacatatca gtcacattgg cctcaatcct ggcctgctct 2940
tacctgcctt gcgcccctgc tgttgcccac atttgaaagc atccaaatga tccgaactag 3000
aatttcctga gaccagcctg gatttagggc tcctgcccac ataaacacaa aggaggaatg 3060
tccctgtctt gttccatccc catgggtggt gttgctgctg gacaagtgtt ggcttttaag 3120
tatccccttt ttttacccac ccccaaaatt aactagcact caagagggct ggtgatgaaa 3180
ggctcacctc tggtgagaat ccctgagagt gcgacatgct tcttcagact ggctgcttcc 3240
attacatttc cagggtctgc tttactccca gcgggagcag cagtgtctct gcacaaggcc 3300
tgctgggctc cagccttctt gtggagagac actggggagg gacaggtcag ggtcactgct 3360
gtgggcatgt gtgtatcctt ccacattctt cctttcagtc ccccctggac ctctgacgtc 3420
cttgggttgg ttcttagtgc tacttctttt acagactaca ctttatagaa ctgattagat 3480
ttcctgttta tttaatgtga gtttgtgcct ttttgtctca atcttccaat acaggtatat 3540

tggggggaaa aaagcagtct aataaaatcc tagacagagt tttttggagt ctagcttatt 3600
ggtgatgttc ctatgtccat ttttcaactt agatgtcctt aaagctccta ctgatatctc 3660
cccagtttat ttacaggaga ggattatgag aagatacagt ggtatttctg acattttatg 3720
taaa 3724

<210> 143
<211> 172
<212> PRT
<213> Mus musculus

<220>
<223> Centrin

<400> 143
Met Ala Ser Asn Phe Lys Lys Thr Thr Met Ala Ser Ser Ala Gln Arg
1               5                   10                  15

Lys Arg Met Ser Pro Lys Pro Glu Leu Thr Glu Asp Gln Lys Gln Glu
            20                  25                  30

Ile Arg Glu Ala Phe Asp Leu Phe Asp Ala Asp Gly Thr Gly Thr Ile
        35                  40                  45

Asp Ile Lys Glu Leu Lys Val Ala Met Arg Ala Leu Gly Phe Glu Pro
    50                  55                  60

Lys Lys Glu Glu Ile Lys Lys Met Ile Ser Glu Ile Asp Lys Glu Gly
65                  70                  75                  80

Thr Gly Lys Met Asn Phe Ser Asp Phe Leu Thr Val Met Thr Gln Lys
                85                  90                  95

Met Ser Glu Lys Asp Thr Lys Glu Glu Ile Leu Lys Ala Phe Lys Leu
            100                 105                 110

Phe Asp Asp Asp Glu Thr Gly Lys Ile Ser Phe Lys Asn Leu Lys Arg
        115                 120                 125

Val Ala Lys Glu Leu Gly Glu Asn Leu Thr Asp Glu Glu Leu Gln Glu
    130                 135                 140

Met Ile Asp Glu Ala Asp Arg Asp Gly Asp Gly Glu Val Asn Glu Gln
145                 150                 155                 160

Glu Phe Leu Arg Ile Met Lys Lys Thr Ser Leu Tyr
                165                 170

<210> 144
<211> 11485
<212> DNA
<213> Mus musculus

<220>
<223> Centrin

<220>
<221> misc_feature
<222> 189, 594, 676, 1320, 1358, 1578, 2286, 2667, 2710, 2801
<223> n is a or g or c or t

<220>
<221> misc_feature
<222> 2914, 2947, 2979, 3146, 8782, 9732, 9975, 10163, 11383
<223> n is a or g or c or t

<400> 144
gctccctgta aaggaacaga gtgtgaagtt agactgtggc tacctcgagt tccttatctg 60
aggatagatt gtgctgatct gtgtaagaaa tacagactcc aagggcaaaa tatacttccc 120
agacaagtca attagtggaa ttaggaatcc tgatatgagt ccctgagttt tatgttgtca 180
ccacccacnt caggcttagc acactgtata agacctggcc tgatgatcta atggaaagag 240
aacaatccca tgaagctggc acctacttat ccaatggata cattaccctg agtaaatatt 300
gccaaaaaag ggcaggggtc ttgtaaaaaa acaaagactt ctctgtgcag aattcaaaga 360
catggcacag gatgaaagtg tttaggaggt aagccattat tttcctgggg ccatccaact 420
ggactctgtc ttttcacaga cctgagatgc cacatctttc tcactttttc atatctactt 480
acttttgaat ttgctatggg ccactcagga gccccttacc aagcccgagc ctagcttcta 540
ctgttccttt cagatttaag gtaaatccaa attgcccctt atccttctat ttcntcagta 600
cctgttggca atgctttctt cctgactcaa gtgacttcta ttgaattatc ctgttgttgt 660
tttagaagtt ggatcntgga aaacattcac atttcctgcc tacatccgag ttgctatagg 720
ggctcactca cttacttatt cacaagttgc caaacagcag ccactgtcag acagaagcca 780
actagaacag ttcagatctt caaagaaacc atcagcttgt atggaaaaca gaaagcttca 840
aagaatgata gagctgtggt tggatcctaa ggaggctaag cccatgagaa ctaatgtatg 900
atgcttagta acatgaaatt atctgaaaac ttgagtgcta aggccctaga atgagtgagt 960
taaagagagg atgtgagagt cttaaaagat agatcacatc caagggtggg aagaacatgt 1020
cctagtaacc gtgataagtt gcaagaacat aagaacaaag caaaattata ctcagacagg 1080
tcaaagaact tggctagggt cacagaatta gcgcataacc caggaagaaa tttatttgtc 1140
ctttctagcc tgctgggctg ccaccccaaa ggggactgat gaaaccagag atgttcactg 1200
taagagtata agcatatgtt ggcagagggg tgaattatga aaaactaagg gatacttatg 1260
gactacctga tagactaagg ttcactgaac actgaacatg tagtctttcc ttttccaggn 1320
actggtgaac acaaatatga ttcctgtcct cctgtgcnta ccttctagtg gagacaacaa 1380
agaaaaaagg ttaactttac agtggcttag gaggtgacaa gtgctactat aaaaatatgg 1440
agcaatatga ggggcccagg tacaaagtaa agagagtgat tacctttaca gagaaggtga 1500
caatagctga agaccaatac taggcttgct ggtgcatacc tttaatctaa gcactcagga 1560
ggcagtggca gatggatncc ctgtgaagtt tgagggcatc ctggtttaca tagtgagtgc 1620
caggtcagcc agaattacag agtgagaccc tatctcaaaa caaaaaaatt atatctacct 1680
gtgaaatttt ctcaaagaat taatcaaaat actaaaaaat tttaaaaagt tcatgggact 1740
gggtaaggtc agtgaaggaa tgtatcagcc gcaaaaatag tgccctgggt gagagtgagc 1800
cagaggggga acagtgtggg tgtgatggga gaaggaggaa tttcatggca ggctttgcag 1860
attatggtga gaactccagc tcatactgct gaggaagggc tgaggaggaa cagagaaaac 1920
agaggacaat tcaaggacgg aactgtgaga actcagaggc tggatatggg ggattgttgc 1980
tgcagtgaac aaaggagcca aacaagacac aaaagttttg aagccggttc ctggcttaaa 2040
ggaaagaggg caaagggcct atattacatt tatggggagc tgttaagtct caggcctttc 2100
tcagtttgag agactgatac tgtgccctta aagtccctaa actgattttc tttgctttga 2160
gggacgtaga acatagaatc tgtgttcgac cctgggcctt cattactcct ggcacagctc 2220
caggtagccc caacccgttg tgcactttgc cctttaattc tctttacatt ccatacttcc 2280
aaaacncctg tggctttgct ttcaagggag tctcattcag gaatgctcct tcccatgtga 2340
cttgagtgtc tgtacgtcta acaggacaca ttttgatgag ttttgaagat ctaaaagtcc 2400
ccagcccatt cccctgagag ctaaagtgac tcttgtagag ctaatgtaaa gacaaagcct 2460
catgtggatt gggtttctaa gccccagccc tgtgaaccac tgacacaata ctcagcatgc 2520
ccactccagt ttcgaggtct cagcttctcc tggcacagtc acccctccaa accatttggt 2580
catatcttgc tggcccctca gatgttctgt gtcccccaac tcggttttaa aatgggtccc 2640
acccattccc tcctacttcc tccaggntct gtcacccaca cgcagcagct tttaagggca 2700
agcccgcaan tcttacccgg tttagacact caaatgatcg acctcagctt gcagacaacc 2760
ggcaaccaga gaagtctggc tgaacacccg ccaccctttt ntactgatag atatgagccc 2820
acaatcagca gagcttcctt cataatgttg attggtctag cttggctctg gcgcaaccaa 2880
ttgacgctca cagcatgatt ttgcccaatg aggntagtct ttggctgccg acgcaccgcc 2940
ctcttgntct tgggcgagtt ttgccaggtt agccccacnt cttcgttccc aaaccaatgg 3000
gaagcgggct gtgcgcgcgt ggagcagtgg gaacgccgct gaggctgcga gtacgccgtt 3060
gccttaacaa ccggtggcag agtccgttgg gtgatggtaa gtgctaggga ttcggtggac 3120
cccacgcggc gtccccggtc ctttcntagg cctcggtgca gccttgctac ctttcttgca 3180
cggttcttgg cgttgacgca ctagttcttc cttggagctg tgtgccggaa gggtagaaga 3240

gaaagtacag ccgccagcag cttgccttgc ctgctctttc ccactacccc ctcctcgccc 3300
aagtcacctt cagccacccc cgcacaaact gcctctggac ctccgctggc tttcaggaaa 3360
cagcagggtg accattctat tgcttccctt agacttccgc ctcagtcaaa ctcaggcctc 3420
ctggccgcgg ccacccagtt aaagtttatg cattggtgct aagaggttta ctgtgttctg 3480
atggtcgcaa acgctttcag gagaggctgt gagaaagcac gcaggtgcaa atggtttgat 3540
ttttatcact ttgtaccggc ataattttta tcttagtaag tttacattta gccaaattta 3600
ggcataagta attggaggag cagagttctt gggaactggc accacaagta gatgttttaa 3660
gtacatttct aaaaccgaga gtacggaaat catatacagt tgatcatcca taactgtaat 3720
tggtttctca aagagctttt gatgatgctt ttttagattt aaaaatgact gaaattgaat 3780
ctcctaaagt gacacatgga ttaatggtac aattgcatga actttctgta gtataaactt 3840
taagacagtg gaggtcagag ttatgtcttc tcatgaaggc tatgttaaca ctagaaattc 3900
atattcatat attcacttgc tctatgttga tctatacaag aacaaggagg aaaatggtaa 3960
tatactattt taaaacaaaa actctcttct atttggtggc tccccaatga aatatagttg 4020
aatttagtca tatgagacat cacattaaga caaatttaca gttatattag aaaataacct 4080
cattattgcc aatttctttt tttataactt agtaaaaact gacatatata aattgaaata 4140
gttttaagtt gagagaaagt tttttcttca cagaggtttg tgctatttgt catttagagt 4200
tggggttaat tttattttag tttttacact tattattcct tggtggagaa gcacatttta 4260
gcctgagtgt ggcggtcaga gaataatttg gaagagttag ttctctcctt ttaccatgta 4320
agcatcgttt cccttttcat ctttgacaac gacaactgaa gcctgggtcc tggttcccac 4380
ttaggttatc aagcttggca gcaagaattc tcactggccc atggaagttt acgtaatgac 4440
ataagtacat tacaaacgat ggcacacctc attgaaaaag atatgtatgt ttagagctgt 4500
cataaataaa acaaggacta tggcttgtaa taatgtgctt ctgtgtttat atattgtgtg 4560
aaggcctcta attttaagaa gacaaccatg gcatccagtg cccagagaaa aaggatgagt 4620
cctaagcctg aacttactga agaccagaaa caggaaatcc gggaagcttt tgatctcttt 4680
gatgctgatg gaactggaac tatagacatc aaagaactga aggtaagctg cacacatcct 4740
tttctttgtt accttgtgtt tccttattgc ttgtctttta gctgttaccg ttcttctcct 4800
ttctgcctgc aaaaagtaac agacaagggt accctgtgta tattttacct gaaacaatgg 4860
tctaattgat aaagttgatt gcaaatatca attaaatcag ccttaagtgt tccctgatca 4920
tttccatcat tttatcaggt gtttggttaa tatcttcaat agtcattttt gtttttacgt 4980
tatgtatcta tttagatgat tctgttttca ttgtaggtgg caatgagggc cctgggcttt 5040
gagcccaaga aagaagaaat taagaaaatg ataagtgaaa ttgataaaga aggaacagga 5100
aaaatgaact tcagtgactt tttgacagtg atgactcaga aaatggtaag ttagttaaga 5160
taattagcat gctttctacc cataatggta gaaacatgag aatctagaca tacaaagagc 5220
tttatccaac aaaatctatc ccttccacat gggagaagat agtattttta attgagagga 5280
tctctacgtt tcatttggct ttctttaagg tattagcatt atcttgtttc atgtggccac 5340
atgataatca caagcagtaa aaacttttaa caccgagatt ttcaatgcta gtcatttttg 5400
gttttaattt attattatta aaaattaaat tatcatacaa taaatcaggt gtcaatctga 5460
cattttcaga caaatggttc acacttaaga ttggttccta gattattctt ccacctgaac 5520
ctcctggcaa gaaccttcct gaaatagctg aggtttgaac gctttcagaa attctgctct 5580
tctgcattac aaggactcag ggccctggta attctttgaa ggccattgtg aaaaatacct 5640
ctctcctcct ctgcctttat cagtctatct gttttagcaa tgtattttcc ctgactacaa 5700
ccctactcac tgcttctata ttttaaaaat gaatctagtg tctgcttgtt ttgtacagta 5760
ctttatatac aattatattt cctatgtaac ccctgagttt atattcactt tactaagtat 5820
gttttctttt tttttttttt ttttacaatg taccatactt gtttgcctgt gaccaaggac 5880
atatatcatg acagctatca ccaggacctt atgataatgt gggccatggc cggggaaatt 5940
aatctaagaa gatagttttc ctacaactcc cttcttggga ttagaactgg gcagcgttct 6000
atacaagaca aagcacagga gggacagtat agatacctgg ccagatccag tgtcaggtgt 6060
tagaaagatg agccccagct cactccagaa gctcttgtct tctacctgtg aaaacagtat 6120
atgactgttc ggctataata ggaacctgtc cttgccacat gatactttgc cccacccccc 6180
caacaagtcc tcatgtctat tttttaggac ttcttgactt tttcaaaagc aggcatcttt 6240
tttctactaa gttctgattt gattatggaa tagatgatgg cagaggaagt agaaaggaaa 6300
tatatgccac catgaatcag aggacctggc ttcctaacat tgttcaagtc aggtttttgg 6360
agacaaaaag atgaccgggg agtagcaaga gtggctggat ataagtaact atcacttttg 6420
tgttctactc cagtctgaga aagacactaa agaagaaatc ctgaaagctt tcaagttgtt 6480
cgatgatgat gagactggga aaatatcatt caagaatctt aagcgcgtgg ccaaggagct 6540
gggtgagaac ctgacagatg aagaactgca ggtgagaaaa agctatggct ttcgtgggtt 6600
ctattttctc tattttcctt ctttgaaagg aaagctagta tccctcatac ctgtggtgat 6660
aatttcatac ctgtggaccc tgtttcgata atacacatgt acaattgaag ttccctcatt 6720
tgggtttcag gtcagggtga ctttccagct tcttttcaat ggatgcatat gacagtcatt 6780
gagatttggc ttttaagtaa ctttcataga aaggggcata ctcacattaa atggaccata 6840
gtcatctttt ctcatagcat tatttgaagt ttcttctttt ttttcttttg ttttgttttt 6900

cgtttttttc aagacagggt ttctctgtgt agccctggct gtcctggaac acactctgta 6960
gaccaggccg gccttgaact cagaaatctg cttttgtctg cctcccaagt gctggaacta 7020
aaggtgtgcg ccaccacagc ctgtctgaag tttcttatct aattactttc agtttagttt 7080
gtgacttaat ctaagatgaa cccttatctt ctgtgcagga aatgattgat gaagctgatc 7140
gagatggaga tggtgaggtc aatgagcaag agttcctgcg catcatgaaa aagaccagcc 7200
tctattaaga tcagtgtttc tttttctact gcaagcacat gtgactagat ttagtgcctg 7260
gcattttgtg aaatatggct tttgaaagcc tccctccccc aacctggggt aactttagcc 7320
atgaccttga atctattttt gacttttgga agtgttcttt gatattaagt tctttgtaca 7380
tttacctgct gactaaatca acagggcaga acaagagcac atagagtggg gaaaagggtc 7440
tgaaagtgtg actgtggcca ccccaccttc cattacttca ccttgctcat gcatatccac 7500
tcgactgaaa acatgcagtg gcttcttagt tgtccatgta tccatgtcac cataagcagt 7560
ggttgttctt aaagtggttt tggttttcat ttgacaccac agtacagttt tctcttttac 7620
aaaacccaat gagtgcttgc tgtggcattt ggatgtgtgt taatgtattt gtttggtctt 7680
taaaaaaata aaacctttct taatttaagc tctcattctt taaaggatga gtatttttta 7740
gctttaactg tgagttcacc ttgtaaacaa ctgtgttgag gacagcctgg tacatatata 7800
caatttgtca tcggcctatg gattaaaaaa gggtaataat tctttgtaac ttttctgcca 7860
taaatagttt ttttgctttc agaacttgtg gatttttaca gctagtagaa tttcttatgc 7920
tctaagccta tagagtctta tgtgtaattt ctgcttggac aaaatggaga gggatgggca 7980
gtaggtaata ttcaggcaat cttatgagat tatttgtttt ataatatctc attgtacaag 8040
tgccctctgg gcttgcacag aattgcctat gagagaaccc aagtatgtgg cattgacatc 8100
taatccagga tgaagggtgg gaacaagaag actgaaggga aatggaaaat gaacaatgat 8160
taaatacata catgaaaatg tacctacata tatacacaca caaaacccac tataagagcc 8220
aggcaggtga tcaagatggc aatcccaatg gatgaggcag gagggttgcc atgactttta 8280
gtcatgctgg gcctacataa tgagtttcgt atcattctgg gtcacaaagt ctgatcctgt 8340
ctcaaagaag caaaacaaaa caagactcca cataaccaga tcggtttgaa tcatatcatc 8400
aatgggttat atgtacatat agttaaaaat ttgtagatat atttatttta tgggtatgat 8460
tattttgcca gcatgtatat atgcacatta tgtgtgagcc tggtgcctgc attagttaga 8520
agagggtata aaattctata taactagagt aagggacagt tgtgagtcac catgtaggtg 8580
ctgggtgctg gaaatcgaac ccaggtctta tgcaagagca acaaggcaag aattctctta 8640
accactaagt aaactctcca tactctttcc cactctctct gtttctgtgg ctcatttgtc 8700
tgtctgtctc tgtttctttt tcttcctctg tctgtctgtc tgtctctttc tctctctctc 8760
tctgtatgtg tgtgtgtaat anrtaaaggc ctccaattcc tcttgatcta acattcaact 8820
ttccaaggca ttttatattt tctgttgggg gcctgctaaa aagttgtgct cctgggcacg 8880
cctttatacg ttggcagtct tgaggatcta ggtgtactgg gataagtgag ggattactcc 8940
gtggaaaaca cagggagaca aaaagccaag aggcacctca gatttcagtt acaacatggg 9000
ttcacttagt tcctggatgg aaatttgggg caagctgctt ctcttagtgt ctcgaattcc 9060
ttgtacaacg aggagaactg ttgaatggag attttgtgca gtcaaatgaa aacttctcaa 9120
cagaaccttg cgctatgcta ttcaagtata agatgtttca gaattatctc attccatgaa 9180
acttacaatc attgtaccca aagtcattgg cagcattttt tgacagcatg ttttttagaa 9240
ggataaaaga tagttagaaa gactgacctt cagcccactt tactatgaag tatatagccc 9300
cacaaaatca atttattcct aatgggcaag taaaagttct ctatagtttg tgtatgcaca 9360
attatatttt gaaatatgcc tactttataa aatagtggaa tcaagctaat taccctgaga 9420
acattctcct actgtttaat gtttcccact ctgataataa atctctttag aatctcttga 9480
tggaggtgat tattgcatat catccctagt gacaaaagca aatataagga agtcaccatg 9540
tcctgcagtg cagacattct gtgattctca gtataggaaa cagatagaca ggatgagtca 9600
tccaagcagg gaggtggggc taaccaagga gctgtgagaa gaactggagc tgtgtaagtt 9660
cagttcatgt ggagccaggc aaggggcact gtagggattg ggcatgtggg atagaaaaag 9720
actgaggcag antgatcgat gggatgggcc tggttctatt tagcatgact gtaccagaga 9780
cttgggcact caggagaggg acaagttcag ccaattatct ctctctctct ctctctctct 9840
ctctctctct ctctctctct ctctctctgt gtgtgtgtgt gtgtgtgtgt gtgtaagagc 9900
agtagcaaag gatgggaaga atggacccag acacatttta taaagtagga agggatcaga 9960
agttgaagga accanggtga gttcagttca ttcaggacag aacacagtga tgggtggtgg 10020
ggaaggagag agagagaatg tagaagatag tctatgtgag gctgtagctg agacattttt 10080
gttgagtggg attcagttga agacagtaat gactaattga tgtgatctta ggcatgtagt 10140
gggaatgggg agggagcaca tanaagtttt ggaaagatgg tctggaggaa agaagaagtc 10200
cattcatgtt gggcttaaag acatagtaag tattcatgaa agatttttca atggttgagt 10260
ctttttctga agacacacca gtgttgagtt tagaatctat ttgagaaagc agtttaaaga 10320
gtaaggtagt taaattacag tcctcggaag acatagacac aaaacatagg caaaacactg 10380
taggagagct aaacgcagac tgacttcgct aggccacctt gaggtacttt taccttctac 10440
caggtcttac cttctttgga gactattttg cttcagtctc acatgagcac cttctgcttt 10500
ttttcttcct catcttgcta tggtttctct ggtggctaag gtaacaagag aacatgtgct 10560

tggaaacatg cctctttgga aagcctagta gttagactgt ctgtttagaa caggccacgt 10620
ggctacaagt tcctgagcat gtccattgac atctactagg aataactttc ctgaagttta 10680
atggcttctg aaagtgctga ccttgcaaac agtgtcaagt acaagaattt tgggctcccc 10740
tccagttctt ctcttgcttg ctttaactac actgtaggca agagctgttc aacttggttt 10800
catttttgta tcctttcaaa tactagaatt ttggtagcag ggttaaagga agtaatgaaa 10860
tcaaaaccac acttgaatag tagtagtgac ttggttacta gtccccagaa aacatgcagt 10920
attcagccta ctatacaaat aggcatccat ctgtttggcc agaattgctt gctgccctga 10980
tggtgttttt gaagtcaaga tacacacccc tacccgaccc ccactgccta atccaggaaa 11040
tgccctttct aagtcctcca ctgtgattga cattttgcag taagtcgtta cctcctccac 11100
aatgagtttc atgacttgtt ttgttcactg ttccatatgc tggaatttgc ttgagtatcc 11160
tgcaccttcc attgtagttg aagtaatcag tctgcccaga ttatagtcat gaagccatat 11220
taccagtcta ctgtgccatt ctgcaacata ttatttttct ttgtgtgtat atgtatgcat 11280
atgtatgcat agccatacat gcaggtctct atgtctgcag aagccaagaa agggcctcag 11340
atttcctgga gctgaagata taaagccatt gtgcaacacc tantatgggt gcaggagttt 11400
ggtcttgggt cctccacaag gagtaatatg caggttcctc ctcaagagcc ccaaatactt 11460
tttaccactg aactctctct tagga 11485

<210> 145
<211> 445
<212> PRT
<213> Rattus norvegicus

<220>
<223> Beta-tubulin

<400> 145
Met Arg Glu Ile Val His Ile Gln Ala Gly Gln Cys Gly Asn Gln Ile
1               5                   10                  15

Gly Pro Lys Phe Trp Glu Val Ile Ser Asp Glu His Gly Ile Asp Pro
            20                  25                  30

Thr Gly Ser Tyr His Gly Asp Ser Asp Leu Gln Leu Glu Arg Ile Asn
        35                  40                  45

Val Tyr Tyr Asn Glu Ala Ala Gly Asn Lys Tyr Val Pro Arg Ala Ile
    50                  55                  60

Leu Val Asp Leu Glu Pro Gly Thr Met Asp Ser Val Arg Ser Gly Pro
65                  70                  75                  80

Phe Gly Gln Ile Phe Arg Pro Asp Asn Phe Val Phe Gly Gln Ser Gly
                85                  90                  95

Ala Gly Asn Asn Trp Ala Lys Gly His Tyr Thr Glu Gly Ala Glu Leu
            100                 105                 110

Val Asp Ser Val Leu Asp Val Val Arg Lys Glu Ser Glu Ser Cys Asp
        115                 120                 125

Cys Leu Gln Gly Phe Gln Leu Thr His Ser Leu Gly Gly Gly Thr Gly
    130                 135                 140

Ser Gly Met Gly Thr Leu Leu Ile Ser Lys Ile Arg Glu Glu Tyr Pro
145                 150                 155                 160

Asp Arg Ile Met Asn Thr Phe Ser Val Met Pro Ser Pro Lys Val Ser
                165                 170                 175

Asp Thr Val Val Glu Pro Tyr Asn Ala Thr Leu Ser Val His Gln Leu

180                 185                 190

Val Glu Asn Thr Asp Glu Thr Tyr Cys Ile Asp Asn Glu Ala Leu Tyr
        195                 200                 205

Asp Ile Cys Phe Arg Thr Leu Lys Leu Thr Thr Pro Thr Tyr Gly Asp
    210                 215                 220

Leu Asn His Leu Val Ser Ala Thr Met Ser Gly Val Thr Thr Cys Leu
225                 230                 235                 240

Arg Phe Pro Gly Gln Leu Asn Ala Asp Leu Arg Lys Leu Ala Val Asn
                245                 250                 255

Met Val Pro Phe Pro Arg Leu His Phe Phe Met Pro Gly Phe Ala Pro
            260                 265                 270

Leu Thr Ser Arg Gly Ser Gln Gln Tyr Arg Ala Leu Thr Val Pro Glu
        275                 280                 285

Leu Thr Gln Gln Met Phe Asp Ser Lys Asn Met Met Ala Ala Cys Asp
    290                 295                 300

Pro Arg His Gly Arg Tyr Leu Thr Val Ala Ala Ile Phe Arg Gly Arg
305                 310                 315                 320

Met Ser Met Lys Glu Val Asp Glu Gln Met Leu Asn Val Gln Asn Lys
                325                 330                 335

Asn Ser Ser Tyr Phe Val Glu Trp Ile Pro Asn Asn Val Lys Thr Ala
            340                 345                 350

Val Cys Asp Ile Pro Pro Arg Gly Leu Lys Met Ser Ala Thr Phe Ile
        355                 360                 365

Gly Asn Ser Thr Ala Ile Gln Glu Leu Phe Lys Arg Ile Ser Glu Gln
    370                 375                 380

Phe Thr Ala Met Phe Arg Arg Lys Ala Phe Leu His Trp Tyr Thr Gly
385                 390                 395                 400

Glu Gly Met Asp Glu Met Glu Phe Thr Glu Ala Glu Ser Asn Met Asn
                405                 410                 415

Glu Leu Val Ser Glu Tyr Gln Gln Tyr Gln Asp Ala Thr Ala Asp Glu
            420                 425                 430

Gln Gly Glu Phe Glu Glu Glu Glu Gly Glu Asp Glu Ala
        435                 440                 445

<210> 146
<211> 1592
<212> DNA
<213> Rattus norvegicus

<220>
<223> Beta-tubulin

<400> 146
ccaacaccat gcgcgagatc gtgcacatcc aggcgggcca atgcggcaac cagatcggcc 60

ctaagttttg ggaggtgata agcgatgagc atggcatcga cccgacgggc agctaccatg 120
gcgacagtga cttgcagctg gagagaatca atgtgtacta caatgaagct gctggcaaca 180
aatatgtacc tcgggccatc ctagtggacc tggagccagg caccatggac tcagtgaggt 240
cgggaccatt cggccagatc ttcaggccag acaactttgt gttcggtcag agtggtgcag 300
gaaataactg ggcaaagggc cactacacag agggtgccga gctggtggac tctgtcctgg 360
atgtggtcag gaaggagtca gaaagctgtg actgtctcca gggctttcag ctgacccact 420
cattgggggg aggcactggc tcaggcatgg ggaccctgct catcagcaag atcagagaag 480
agtacccaga ccgcatcatg aacaccttca gcgtcatgcc ctcacccaag gtgtcggaca 540
ctgtggtgga gccctataat gccacccttt ccgtgcacca gctggtagag aacacagacg 600
aaacctactg catcgacaac gaggctctgt atgacatctg cttccgcacc ctgaagctga 660
ccacacccac ctatggcgat ctcaaccacc tggtgtcagc caccatgagt ggagtgacca 720
cctgcctgcg cttccctggc cagctgaacg cagacctgcg caagctggct gtgaacatgg 780
tgcctttccc acgcctgcac ttcttcatgc caggcttcgc acctctgacc agcaggggca 840
gccagcagta ccgagccctg acagtgcccg agctcaccca gcagatgttc gactccaaga 900
acatgatggc tgcctgcgac ccacgccatg gccgctacct gaccgtagcc gccattttcc 960
ggggccgcat gtccatgaag gaggtggatg agcagatgct caacgtgcag aacaagaaca 1020
gcagctactt cgtggaatgg atccccaaca atgtgaagac ggccgtgtgt gacatccctc 1080
ctcgtggcct caagatgtcc gccaccttca ttggcaacag caccgccatc caagagctgt 1140
tcaagcgcat ctcggagcag ttcactgcca tgttccggcg caaggccttc ctgcactggt 1200
acacgggcga gggcatggac gagatggagt tcaccgaggc ggagagcaac atgaatgagc 1260
tggtgtctga gtaccagcag taccaggatg ccacggctga tgagcagggc gagttcgagg 1320
aggaggaggg tgaggatgag gcttgagttc ccaggccaag caggttaggg aaagctgagg 1380
cgaaaggagg gggtgggggt cttaatctgt gaaaatacct tggcagttgg aagaaggaga 1440
atggtcttag gtttgtgctg ggtctctggt gctcttactg ttgcctctca cttttttctc 1500
tttttgtaat atcgatgacg tgatgtgatg cttgagatct ttctgaactc ctgttgtgat 1560
ggctgaaatc gcctgaacct ttgtgtccta aa 1592

<210> 147
<211> 237
<212> PRT
<213> Mus musculus

<220>
<223> Formin binding protein

<400> 147
Lys Ile His Cys Phe Arg Ser Leu Lys Arg Gly Gly Val Thr Pro Glu
1               5                   10                  15

Asp Phe Ser Asn Phe Pro Pro Glu Gln Arg Arg Lys Lys Leu Gln Gln
            20                  25                  30

Lys Val Asp Asp Leu Asn Arg Glu Ile Gln Lys Glu Thr Asp Gln Arg
        35                  40                  45

Asp Ala Ile Thr Lys Met Lys Asp Val Tyr Leu Lys Asn Pro Gln Met
    50                  55                  60

Gly Asp Pro Ala Ser Leu Asp Gln Lys Leu Thr Glu Val Thr Gln Asn
65                  70                  75                  80

Ile Glu Lys Leu Arg Leu Glu Ala Gln Lys Phe Glu Ala Trp Leu Ala
                85                  90                  95

Glu Val Glu Gly Arg Leu Pro Ala Arg Ser Glu Gln Ala Arg Arg Gln
            100                 105                 110

Ser Gly Leu Tyr Asp Gly Gln Thr His Gln Thr Val Thr Asn Cys Ala
        115                 120                 125

Gln Asp Arg Glu Ser Pro Asp Gly Ser Tyr Thr Glu Glu Gln Ser Gln
    130                 135                 140

Glu Ser Glu His Lys Val Leu Ala Pro Asp Phe Asp Asp Glu Phe Asp
145                 150                 155                 160

Asp Glu Glu Pro Leu Pro Ala Ile Gly Thr Cys Lys Ala Leu Tyr Thr
                165                 170                 175

Phe Glu Gly Gln Asn Glu Gly Thr Ile Ser Val Val Glu Gly Glu Thr
            180                 185                 190

Leu Ser Val Ile Glu Glu Asp Lys Gly Asp Gly Trp Thr Arg Ile Arg
        195                 200                 205

Arg Asn Glu Asp Glu Glu Gly Tyr Phe Pro Thr Ser Tyr Val Glu Val
    210                 215                 220

Tyr Leu Asp Lys Asn Ala Lys Gly Ala Lys Thr Tyr Ile
225                 230                 235


<210> 148
<211> 729
<212> DNA
<213> Mus musculus

<220>
<223> Formin binding protein

<400> 148
ccaaaatcca ctgcttccgg agcctaaagc gtgggggtgt cacaccagaa gacttcagca 60
acttcccacc tgagcagaga aggaaaaaac tacaacagaa agttgacgat ctcaatagag 120
agatacagaa ggagacggac cagagagatg ccatcaccaa aatgaaagat gtgtacctaa 180
agaaccctca gatgggagac ccagccagcc tggaccagaa gctcaccgaa gtcacccaga 240
acatagagaa actgcggctg gaggctcaga agtttgaggc ctggctggct gaggtagaag 300
gcagactccc agctcggagt gagcaggcac gccggcagag tggactgtat gatggccaga 360
cacaccagac ggtcactaac tgtgcccagg accgggagag cccagatggt agttacacag 420
aggagcaaag ccaggagagc gagcacaagg tcctggcccc ggatttcgac gatgaatttg 480
atgatgagga gccgcttcca gccataggga cctgcaaggc cctctacaca tttgaaggtc 540
agaacgaagg caccatttcc gtagttgaag gagagacgct gagcgtgatt gaagaggaca 600
aaggcgatgg gtggactcgc atccgcagaa atgaagacga ggagggttac ttccccactt 660
cctacgtcga agtctattta gacaaaaacg ccaaaggtgc taagacttat atttaatcca 720
cttaaaaaa                                                          729


<210> 149
<211> 472
<212> PRT
<213> Homo sapiens

<220>
<223> NY-REN-58

<400> 149
Met Arg Glu Arg Phe Arg Asn Leu Asp Glu Glu Val Glu Lys Tyr Arg
1               5                   10                  15

Ala Val Tyr Asn Lys Leu Arg Tyr Glu His Thr Phe Leu Lys Ser Glu
            20                  25                  30

Phe Glu His Gln Lys Glu Glu Tyr Ala Arg Ile Leu Asp Glu Gly Lys
        35                  40                  45

Ile Lys Tyr Glu Ser Glu Ile Ala Arg Leu Glu Glu Asp Lys Glu Glu
    50                  55                  60

Leu Arg Asn Gln Leu Leu Asn Val Asp Leu Thr Lys Asp Ser Lys Arg
65                  70                  75                  80

Val Glu Gln Leu Ala Arg Glu Lys Val Tyr Leu Cys Gln Lys Leu Lys
                85                  90                  95

Gly Leu Glu Ala Glu Val Ala Glu Leu Lys Ala Glu Lys Glu Asn Ser
            100                 105                 110

Glu Ala Gln Val Glu Asn Ala Gln Arg Ile Gln Val Arg Gln Leu Ala
        115                 120                 125

Glu Met Gln Ala Thr Val Arg Ser Leu Gly Ala Glu Lys Gln Ser Ala
    130                 135                 140

Asn Leu Arg Ala Glu Arg Leu Glu Lys Glu Leu Gln Ser Ser Ser Glu
145                 150                 155                 160

Gln Asn Thr Phe Leu Ile Asn Lys Leu His Lys Ala Glu Arg Glu Ile
                165                 170                 175

Asn Thr Leu Ser Ser Lys Val Lys Glu Leu Lys His Ser Asn Lys Leu
            180                 185                 190

Glu Ile Thr Asp Ile Lys Leu Glu Thr Ala Arg Ala Lys Ser Glu Leu
        195                 200                 205

Glu Arg Glu Arg Asn Lys Leu Gln Ser Glu Leu Asp Gly Leu Gln Ser
    210                 215                 220

Asp Asn Glu Ile Leu Lys Ala Ala Val Glu His His Lys Val Leu Leu
225                 230                 235                 240

Val Glu Lys Asp Arg Glu Leu Ile Arg Lys Val Gln Ala Ala Lys Glu
                245                 250                 255

Glu Gly Tyr Gln Lys Leu Val Val Leu Gln Asp Glu Lys Leu Glu Leu
            260                 265                 270

Glu Asn Arg Leu Ala Asp Leu Glu Lys Met Lys Val Glu His Asp Val
        275                 280                 285

Trp Arg Gln Ser Glu Lys Asp Gln Tyr Glu Glu Lys Leu Arg Ala Ser
    290                 295                 300

Gln Met Ala Glu Glu Ile Thr Arg Lys Glu Leu Gln Ser Val Arg Leu
305                 310                 315                 320

Lys Leu Gln Gln Gln Ile Val Thr Ile Glu Asn Ala Glu Lys Glu Lys
                325                 330                 335

Asn Glu Asn Ser Asp Leu Lys Gln Gln Ile Ser Ser Leu Gln Ile Gln
            340                 345                 350

Val Thr Ser Leu Ala Gln Ser Glu Asn Asp Leu Leu Asn Ser Asn Gln

355                 360                 365

Met Leu Lys Glu Met Val Glu Arg Leu Lys Gln Glu Cys Arg Asn Phe
    370                 375                 380

Arg Ser Gln Ala Glu Lys Ala Gln Leu Glu Ala Glu Lys Thr Leu Glu
385                 390                 395                 400

Glu Lys Gln Ile Gln Trp Leu Glu Glu Lys His Lys Leu His Asp Arg
                405                 410                 415

Ile Thr Asp Arg Glu Glu Lys Tyr Asn Gln Ala Lys Glu Lys Leu Gln
            420                 425                 430

Arg Ala Ala Ile Ala Gln Lys Lys Arg Lys Ser Leu His Glu Asn Lys
        435                 440                 445

Leu Lys Arg Leu Gln Glu Lys Val Glu Val Leu Glu Ala Lys Lys Glu
    450                 455                 460

Glu Leu Glu Thr Glu Ile Arg Ser
465                 470


<210> 150
<211> 2936
<212> DNA
<213> Homo sapiens

<220>
<223> NY-REN-58

<400> 150
cgtaaccctt agtcccaatg cctccgtaag cggagttgag tgggtgcctg tggttggagc 60
tgtggaggtg tccccggtgg cgagcgcggc cagaactgcg gtcacttaag ttttccgtgt 120
gcgggttgca aggagcgtgc gtgcgtctgg tatagggagg acacctctgg attgaggatc 180
ttatgaccta ctttagagga aggtataatt tggcttcctg agattctgcc ttagcaagaa 240
aggagtggga aatacccttg gaaagaaaac taaaacagta agaaagccaa aacttatttt 300
tacatggttg tcagcacatt taccgatatg gacacttttc ccaataattt tcctcctggt 360
ggagacagtg gattgacagg ttctcagtcg gagttccaga aaatgttaat tgatgaaagg 420
ttacgatgtg agcatcataa agctaattat cagacactga aggctgaaca cacaaggttg 480
cagaatgaac atgtaaagtt acaaaatgaa ctcaagcacc tgtttaatga aaagcaaact 540
cagcaggaaa aacttcagct cctgcttgaa gaactaagag gagaattagt agagaaaact 600
aaagatttag aagaaatgaa actgcagata ttaactccca aaaattggaa ttgctaagag 660
cccaaataca acaagaatta gaaactccaa tgagagaacg ttttaggaat ctagatgaag 720
aggtagaaaa gtatagagct gtatataata agcttcgcta tgaacataca tttctcaagt 780
cagaatttga acaccagaag gaagagtatg cacgtatttt agatgaagga aaaataaaat 840
atgaatcaga gattgcaaga ctggaggaag ataaagaaga actacgtaac cagctgctta 900
atgttgatct cacaaaagac agcaaacgag tggaacaact tgctcgagaa aaagtctatt 960
tgtgtcaaaa attaaaaggt ttagaggctg aagtagcgga attaaaggct gaaaaggaga 1020
attctgaggc tcaggtggaa aatgcccaaa gaatacaggt gcggcagttg gctgagatgc 1080
aggctacagt cagatccctg ggggctgaaa aacaatcagc taatttacgg gcagaacgct 1140
tggaaaaaga gctacaatca agcagtgaac aaaatacctt tttaattaat aaattgcata 1200
aagctgaacg agaaataaat acattgtcca gtaaagtaaa agaacttaaa cattcaaaca 1260
aactggaaat aacagacatc aaactggaga cagcaagagc taagagtgag ctagaaagag 1320
aaaggaataa gcttcaaagt gaactggatg gattacagtc agacaatgaa attctcaaag 1380
cagctgttga acatcacaaa gtgctcttag tagaaaagga tcgtgaatta atacgtaaag 1440
tacaagctgc caaagaagaa ggttatcaaa aacttgtggt attacaagat gaaaagttag 1500
aactcgagaa cagattagca gatttggaga aaatgaaagt ggaacatgat gtctggaggc 1560
aatctgaaaa ggatcagtat gaagagaaat tgcgggcttc acagatggca gaagagatca 1620
ccaggaagga gcttcagagt gttaggttaa aacttcagca acaaattgtg actattgaaa 1680

atgcagagaa ggaaaaaaat gaaaattctg acctaaaaca gcaaatcagt agtttgcaga 1740
tccaagtgac ttcacttgca cagtcagaga atgacttgct gaattcaaac caaatgctga 1800
aggaaatggt ggagagatta aaacaagaat gccgaaattt tagaagccaa gctgaaaaag 1860
cgcaactaga agctgaaaag acattggaag agaaacagat acagtggttg gaagaaaagc 1920
ataagcttca tgaccgtatc acagacagag aagaaaagta caatcaagct aaggagaaac 1980
tgcagcgagc tgcaattgcc cagaaaaaga gaaaatctct tcatgaaaac aaattgaaaa 2040
gactacaaga gaaagtagaa gtcttggagg caaagaaaga agaattggaa acagaaatca 2100
ggtcttaaat agacaaaatg ttcctttgaa gactatacaa ggcttcaaaa aagactaaaa 2160
gatatacaga gaagacataa tgaatttcga agtctaattt tggttcctaa catgcctcca 2220
acagcatcta tcaatcctgt tagctttcag tcatcagcca tggttccaag catggaacta 2280
ccatttcctc ctcatatgca ggaggaacaa catcaaaggg aactctctct acttcgcaaa 2340
agactagaag aactggaaac aacacaaaga aaacaactag aggaacttgg atcttccgga 2400
gaatgatgtt cttggagaac aggcagatca aaagaggtga agttggtgac tcagtaaaac 2460
ttgacatttt aacctgtggc atttagatac tttttactgt ttgccaaaac acttgaatgt 2520
gcctcaagaa aaggtaccta ctacatgctg tattgtatga ctgtcaggat tttaagatta 2580
tacaagtgaa gcattaaaag agaaattctc agagatattt agaatatttg acaatggttt 2640
gagaatgtaa aacaaaaagg aactagttag agtcaagttt taaaattttt actttgttga 2700
attttttttt ttggcatttt gagtgaaata taactatcat taattctctc ttcatctttg 2760
agatgcttgg ccataacagg gtccatacac atcttctggt ttactatata caaaaactgt 2820
agttgaaaaa agatgacaat ttaaaagtca gcctaaagaa tgtaaaggta tctatataca 2880
aaaggctacc ttttctaaaa tctgtgtgca cataattaaa gagcttaatt tttaaa 2936

<210> 151
<211> 583
<212> PRT
<213> Mus musculus

<220>
<223> Radixin

<400> 151
Met Pro Lys Pro Ile Asn Val Arg Val Thr Thr Met Asp Ala Glu Leu
1               5                   10                  15

Glu Phe Ala Ile Gln Pro Asn Thr Thr Gly Lys Gln Leu Phe Asp Gln
            20                  25                  30

Val Val Lys Thr Val Gly Leu Arg Glu Val Trp Phe Phe Gly Leu Gln
        35                  40                  45

Tyr Val Asp Ser Lys Gly Tyr Ser Thr Trp Leu Lys Leu Asn Lys Lys
    50                  55                  60

Val Thr Gln Gln Asp Val Lys Lys Glu Asn Pro Leu Gln Phe Lys Phe
65                  70                  75                  80

Arg Ala Lys Phe Phe Pro Glu Asp Val Ser Glu Glu Leu Ile Gln Glu
                85                  90                  95

Ile Thr Gln Arg Leu Phe Phe Leu Gln Val Lys Glu Ala Ile Leu Asn
            100                 105                 110

Asp Glu Ile Tyr Cys Pro Pro Glu Thr Ala Val Leu Leu Ala Ser Tyr
        115                 120                 125

Ala Val Gln Ala Lys Tyr Gly Asp Tyr Asn Lys Glu Ile His Lys Pro
    130                 135                 140

Gly Tyr Leu Ala Asn Asp Arg Leu Leu Pro Gln Arg Val Leu Glu Gln
145                 150                 155                 160

His Lys Leu Thr Lys Glu Gln Trp Glu Glu Arg Ile Gln Asn Trp His
                165                 170                 175

Glu Glu His Arg Gly Met Leu Arg Glu Asp Ser Met Met Glu Tyr Leu
            180                 185                 190

Lys Ile Ala Gln Asp Leu Glu Met Tyr Gly Val Asn Tyr Phe Glu Ile
        195                 200                 205

Lys Asn Lys Lys Gly Thr Glu Leu Trp Leu Gly Val Asp Ala Leu Gly
    210                 215                 220

Leu Asn Ile Tyr Glu His Asp Asp Lys Leu Thr Pro Lys Ile Gly Phe
225                 230                 235                 240

Pro Trp Ser Glu Ile Arg Asn Ile Ser Phe Asn Asp Lys Lys Phe Val
                245                 250                 255

Ile Lys Pro Ile Asp Lys Lys Ala Pro Asp Phe Val Phe Tyr Ala Pro
            260                 265                 270

Arg Leu Arg Ile Asn Lys Arg Ile Leu Ala Leu Cys Met Gly Asn His
        275                 280                 285

Glu Leu Tyr Met Arg Arg Arg Lys Pro Asp Thr Ile Glu Val Gln Gln
    290                 295                 300

Met Lys Ala Gln Ala Arg Glu Val Leu His Gln Lys Gln Leu Glu Arg
305                 310                 315                 320

Ala Gln Leu Glu Asn Glu Lys Lys Lys Arg Glu Ile Ala Glu Lys Glu
                325                 330                 335

Lys Glu Arg Ile Glu Arg Glu Lys Glu Glu Leu Met Glu Arg Leu Arg
            340                 345                 350

Gln Ile Glu Glu Gln Thr Val Lys Ala Gln Lys Glu Leu Glu Glu Gln
        355                 360                 365

Thr Arg Lys Ala Leu Glu Leu Glu Gln Glu Arg Gln Arg Ala Lys Glu
    370                 375                 380

Glu Ala Glu Arg Leu Asp Arg Glu Arg Arg Ala Ala Glu Glu Ala Lys
385                 390                 395                 400

Ser Ala Ile Ala Lys Gln Ala Ala Asp Gln Met Lys Asn Gln Glu Gln
                405                 410                 415

Leu Ala Ala Glu Leu Ala Glu Phe Thr Ala Lys Ile Ala Leu Leu Glu
            420                 425                 430

Glu Ala Lys Lys Lys Lys Glu Glu Glu Ala Thr Glu Trp Gln His Lys
        435                 440                 445

Ala Phe Ala Ala Gln Glu Asp Leu Glu Lys Thr Lys Glu Glu Leu Lys
    450                 455                 460

Thr Val Met Ser Ala Pro Pro Pro Pro Pro Pro Pro Pro Val Ile Pro
465                 470                 475                 480

Pro Thr Glu Asn Glu His Asp Glu Gln Asp Glu Asn Ser Ala Glu Ala
                485                 490                 495

Ser Ala Glu Leu Ser Ser Glu Gly Val Met Asn His Arg Ser Glu Glu
            500                 505                 510

Glu Trp Val Thr Glu Thr Gln Lys Asn Glu Arg Val Lys Lys Gln Leu
        515                 520                 525

Gln Ala Leu Ser Ser Glu Leu Ala Gln Ala Arg Asp Glu Thr Lys Lys
    530                 535                 540

Thr Gln Asn Asp Val Leu His Ala Glu Asn Val Lys Ala Gly Arg Asp
545                 550                 555                 560

Lys Tyr Lys Thr Leu Arg Gln Ile Arg Gln Gly Asn Thr Lys Gln Arg
                565                 570                 575

Ile Asp Glu Phe Glu Ala Met
            580

<210> 152
<211> 4238
<212> DNA
<213> Mus musculus

<220>
<223> Radixin

<220>
<221> misc_feature
<222> 2540
<223> n is a or g or c or t

<400> 152
ctcgcaccgc cgactctccg ctcgttcgcc gccagccacg acgcccccgc cgagccgcgc 60
ctcagccagc cggcgcgggc agggaaggag ccactcattt tcggggctat aattgtttat 120
agaagtgaag agtttctaaa gaaggtaaca aacaagaaaa agaaaatgcc gaagccaatc 180
aatgtaagag taactacaat ggacgctgag ctagaatttg ccatccagcc caatacaact 240
ggcaaacaac tttttgacca ggttgtaaaa acagttggct tacgtgaggt ctggtttttt 300
gggctgcaat atgtagacag caaaggatat tctacatggc ttaaattgaa taaaaaggta 360
acacagcaag atgttaaaaa agaaaatcct ttacagttca agtttagagc taaattcttt 420
cctgaagatg tttctgagga attgattcaa gaaataacac agagactttt cttcttgcaa 480
gttaaagaag ccatcttaaa tgatgagata tattgcccac cagaaactgc agttcttttg 540
gcttcgtatg ctgtccaagc caagtatgga gattataata aagagattca caaaccaggc 600
tatctggcta atgacagact cttaccacag cgtgttttgg aacaacacaa actgaccaaa 660
gagcagtggg aggagagaat acagaactgg catgaggaac acagaggcat gctaagggaa 720
gattcgatga tggaatactt gaagattgcg caagatctag agatgtatgg tgtcaactac 780
tttgaaatca agaataagaa gggtactgag ctgtggctag gtgttgatgc cttaggcctg 840
aatatttatg aacatgatga caagttaaca cctaaaattg gttttccctg gagtgaaatc 900
agaaatattt catttaatga caaaaaattc gttataaagc caattgacaa aaaggcacct 960
gattttgtct tctatgcacc tcgtctgaga atcaataaac gaattttggc cttatgtatg 1020
ggaaaccatg aactgtacat gcgaaggaga aagcctgata ctattgaagt acagcagatg 1080
aaggctcagg cccgggaggt tttgcatcag aagcagctag aaagggcaca attagagaat 1140
gaaaagaaga aacgagaaat agcagaaaag gaaaaggaaa gaatagaacg tgaaaaggaa 1200
gagctgatgg agcgtctgag gcagatcgaa gagcagacag tgaaagccca gaaagaacta 1260
gaagaacaga ctcggaaagc tctagaactg gagcaggagc gccagcgagc aaaagaggag 1320
gcagagcggc tagacaggga gcgccgggct gcagaagaag ccaagtctgc aatcgccaag 1380
caagctgctg accagatgaa gaaccaggag cagctggcag cagaacttgc cgaattcact 1440
gccaagattg cacttttaga agaagccaag aagaaaaagg aagaggaagc tactgagtgg 1500

cagcacaaag cttttgcagc tcaggaagac ttggaaaaga ccaaagaaga gctaaaaact 1560
gtgatgtctg cgccacctcc acctccccct cctccagtca tccctcccac ggagaacgag 1620
catgacgagc aggacgagaa cagtgcggag gccagcgccg agctgtccag cgagggggtg 1680
atgaaccacc gaagcgagga ggagtgggtc accgaaaccc agaagaatga acgcgtgaag 1740
aagcagctcc aggcattgag ttcagaatta gcccaagcta gagatgaaac caagaagaca 1800
cagaacgatg ttcttcacgc tgagaatgtc aaggccggcc gtgataagta caagaccctt 1860
cggcagattc gacaaggcaa tacgaagcag cgcatcgatg agtttgaagc catgtgagag 1920
ctgttcttgt gatccatgtc cttcataagc tgaaccacca ccagagaaaa gcaggcctgt 1980
gcagtgcaga tgcaatggaa cgcatcccac cttgccaaag cactcatacc agtcgactgt 2040
gctcactagc aggacactga agggtgtcct tcatcattaa ggcagtgtga tgtcatgttt 2100
ggtcattttt cttttgtcca gggaatggat aatgacattc tgtcacccac ccttttgtac 2160
atttttcact ttttgtttgt ttcatctttt tcagttgaat attaatacta atggccacgt 2220
ctgacaaatg tgtgtatgtg ctttgagcac tgtgtacatc ttaggataat ggtggacatt 2280
tcagtgcatg tttagctaat gaacttagtt ttctttgccc tttccatgtt gtaggctaca 2340
tacatctcca tgttcattct catgtttttt gtcagtgtga ccttctcatg cccagacatg 2400
atagagctgt aattctggtg tcacacggat cacttactgg aggtgtataa tatgagatag 2460
tgaggtcaga cagaccacag acaagacatg ggactacatc ataagatgat tagaatgttg 2520
gacatcgtag tatgcagccn gtggttagtt attacaaaga tcccttagtt actgtattgt 2580
ttgctttcct taagtgcagt catctctgat agcttgagtc cttaagtgca gccatctctg 2640
atagcttagt gatggttttc acgtcctgta gtcctatggt ggctttcctt gcctgttact 2700
ttagagtcta gccactagga gtcgcttcta agaaaattat tgatgaagtg gccttgtaag 2760
tacatggtct ctgaaaaaaa ttcaagtttt cttgttagga tgtggaatag aagataactt 2820
ggatgataac taattctaag ttgttgaagg gggaaaaaaa cccaaacctt ggcctttctg 2880
caagggcagc atatggaaac atcaactctg agaacaaaat actacctcac tctttacgta 2940
cgtggtgact gttcgcacag tctgtgctgc atgtctgtca cccatacgca gtctcaatca 3000
tggccttcac tgagtgcttt tgagatccca gtgctaatgt tttaactgcc tttctgttga 3060
actgtggggc tctgatttgt gtctagatta ttgtatattc tcactggtat gctgttccct 3120
ggggggtgtt ctgtgtccga cgctgagtga ccctttgtga gaaagacgtt gcctttgcag 3180
tacatgaagc atcccattct tcgttcttga tgggataggg atcgttattg tagcatattt 3240
ggtttctaaa tcactgctta tttttatttg caatctctaa aacaaaattt tatttcaatt 3300
acgtcttact gaagaagtct ttccagctag aaagatgttc tgacactgtc tgatcgtgaa 3360
gcacctgaat ttacagaaga tgcagaagcc tcgttttctc tgcgtggttg agctactttc 3420
catttggtat tatgcactca aacttaacat taatctatta aaattgccat tttattaagc 3480
aattttcacg cacagtagat tcaacttgtt ttttgtgctg ttttttgatt tttgctgact 3540
taaaaaggat taatttgggc agacattata aaaaggaaag ggttaatata tttttggact 3600
ctgtaggaca aaagagaact ggttaacctt gaagtgactg ttgtacagtg gctgtgcacg 3660
tgcttcagac tcatttgcga gggaatgctt tctgtatgag agggagacgg taggccttac 3720
cactcagacc tggacgtata agtgctcact tttccaacac atcacagcga tagtccttat 3780
tgctaaagga cttggcttcc ctcggagttt tactgattta tttttttctc tttgcattgt 3840
gtacattatc attgctacat cagttgttac agtgttgaaa ggctaccagt tatatttgct 3900
gttaatagtc tatttgtaga ttaggattaa agtgggttta atccattttt atagctgtat 3960
gaatttttct aaacaggaac agccatttgc aaaatgggtt ttcatagtga ttgcatcaat 4020
tataacattt atcattagca gttgagagca catgttcata tagcaatgta aaaacctact 4080
aatgagtatt tggtaatttc ccattagcat aattttatgt tgtacaagta aattacaatt 4140
ccatctctaa tcttggataa tactggttgg ttaacaataa agtgacaaaa gctcccgcct 4200
tgcaaaaaaa aaaaaaaaaa aaaaaaaaaa aaaaaaaa 4238

<210> 153
<211> 173
<212> PRT
<213> Rattus norvegicus

<220>
<223> Splicing factor 1 homolog

<400> 153
Leu Ile Thr Glu Met Val Ala Leu Asn Pro Asp Phe Lys Pro Pro Ala
1               5                   10                  15

Asp Tyr Lys Pro Pro Ala Thr Arg Val Ser Asp Lys Val Met Ile Pro

            20                  25                  30

Gln Asp Glu Tyr Pro Glu Ile Asn Phe Val Gly Leu Leu Ile Gly Pro
        35                  40                  45

Arg Gly Asn Thr Leu Lys Asn Ile Glu Lys Glu Cys Asn Ala Lys Ile
    50                  55                  60

Met Ile Arg Gly Lys Gly Ser Val Lys Glu Gly Lys Val Gly Arg Lys
65                  70                  75                  80

Asp Gly Gln Met Leu Pro Gly Glu Asp Glu Pro Leu His Ala Leu Val
                85                  90                  95

Thr Ala Asn Thr Met Glu Asn Val Lys Lys Ala Val Glu Gln Ile Arg
            100                 105                 110

Asn Ile Leu Lys Gln Gly Ile Glu Thr Pro Glu Asp Gln Asn Asp Leu
        115                 120                 125

Arg Lys Met Gln Leu Arg Glu Leu Ala Arg Leu Asn Gly Thr Leu Arg
    130                 135                 140

Glu Asp Asp Asn Arg Ile Leu Arg Pro Trp Gln Ser Ser Glu Thr Arg
145                 150                 155                 160

Ser Ile Thr Asn Thr Thr Val Cys Thr Lys Cys Gly Gly
                165                 170

<210> 154
<211> 519
<212> DNA
<213> Rattus norvegicus

<220>
<223> Splicing factor 1 homolog

<400> 154
ctcattacgg agatggttgc tctcaaccca gactttaaac cacctgcaga ttacaagcct 60
ccagcaacac gtgtgagcga taaagtaatg atcccccaag acgagtatcc agaaatcaat 120
tttgtgggtc tcctaattgg gcccagaggg aacaccctga agaacattga gaaggaatgc 180
aacgccaaga tcatgatacg gggaaaggga tcagtaaaag aagggaaagt tgggcgtaaa 240
gatggtcaga tgttgccagg agaagatgaa cctcttcatg ctctagtcac tgccaataca 300
atggagaatg tcaaaaaggc agtggaacag atcagaaaca tcctgaagca gggtattgaa 360
accccagagg accagaatga cctaaggaaa atgcagcttc gagagttagc tcgcttgaat 420
ggcactctac gggaagatga taacaggatc ttgagaccct ggcagagctc agagacacga 480
agcattacca acacgactgt gtgtactaag tgtggaggg 519

<210> 155
<211> 1196
<212> PRT
<213> Rattus norvegicus

<220>
<223> SH3-containing protein

<400> 155
Met Asn Thr Asp Ser Gly Gly Cys Ala Arg Lys Arg Ala Ala Met Ser
1               5                   10                  15

Val Thr Leu Thr Ser Val Lys Arg Val Gln Ser Ser Pro Asn Leu Leu
            20                  25                  30

Ala Ala Gly Arg Glu Ser His Ser Pro Asp Ser Ala Trp Arg Ser Tyr
        35                  40                  45

Asn Gly Arg Asn Pro Glu Thr Leu Asn Gly Asp Ala Thr Tyr Ser Ser
    50                  55                  60

Leu Ala Ala Lys Gly Phe Arg Ser Val Arg Pro Asn Leu Gln Asp Lys
65                  70                  75                  80

Lys Ser Pro Thr Gln Ser His Ile Thr Ile Asn Gly Asn Ser Gly Gly
                85                  90                  95

Ala Val Ser Pro Val Ser Tyr Tyr Gln Arg Pro Phe Ser Pro Ser Ala
            100                 105                 110

Tyr Ser Leu Pro Ala Ser Leu Asn Ser Ser Ile Ile Met Pro His Gly
        115                 120                 125

Arg Ser Leu Asp Ser Ala Glu Thr Tyr Ser Gln His Ala Gln Ser Leu
    130                 135                 140

Asp Gly Thr Met Gly Ser Ser Ile Pro Leu Tyr Arg Ser Ser Glu Glu
145                 150                 155                 160

Glu Lys Arg Val Thr Val Ile Lys Ala Pro His Tyr Pro Gly Ile Gly
                165                 170                 175

Pro Val Asp Glu Ser Gly Ile Pro Thr Ala Ile Arg Thr Thr Val Asp
            180                 185                 190

Arg Pro Lys Asp Trp Tyr Lys Thr Met Phe Lys Gln Ile His Met Val
        195                 200                 205

His Lys Pro Asp Glu Asp Thr Asp Met Tyr Asn Thr Pro Tyr Thr Tyr
    210                 215                 220

Asn Ala Gly Leu Tyr Asn Ser Pro Tyr Ser Ala Gln Ser His Pro Ala
225                 230                 235                 240

Ala Lys Thr Gln Thr Tyr Arg Pro Leu Ser Lys Ser His Ser Asp Asn
                245                 250                 255

Gly Thr Asp Ala Phe Lys Glu Ala Pro Ser Pro Val Pro Pro Pro His
            260                 265                 270

Val Pro Pro Arg Pro Arg Asp Gln Ser Ser Thr Glu Lys His Asp Trp
        275                 280                 285

Asp Pro Pro Asp Arg Lys Val Asp Thr Arg Lys Phe Arg Ser Glu Pro
    290                 295                 300

Arg Ser Ile Phe Glu Tyr Glu Pro Gly Lys Ser Ser Ile Leu Gln His
305                 310                 315                 320

Glu Arg Pro Val Ser Val Tyr Gln Ser Ser Ile Asp Arg Ser Leu Glu
                325                 330                 335

Arg Pro Ser Ser Ser Ala Ser Met Ala Gly Asp Phe Arg Lys Arg Arg
            340                 345                 350

Lys Ser Glu Pro Ala Val Gly Pro Pro Arg Gly Leu Gly Asp His Ser
        355                 360                 365

Ser Ser Arg Thr Ser Pro Gly Arg Ala Asp Leu Pro Gly Ser Ser Ser
    370                 375                 380

Thr Phe Thr Thr Ser Phe Ile Ser Ser Ser Pro Ser Ser Pro Ser Arg
385                 390                 395                 400

Ala Gln Gly Gly Asp Asp Ser Lys Met Cys Pro Pro Leu Cys Ser Tyr
                405                 410                 415

Ser Gly Leu Asn Gly Ser Pro Ser Ser Glu Leu Glu Cys Cys Gly Ala
            420                 425                 430

Tyr Arg Arg His Leu Asp Val Pro Gln Asp Ser Gln Arg Ala Ile Thr
        435                 440                 445

Phe Lys Asn Gly Trp Gln Met Ala Arg Gln Asn Ala Glu Ile Trp Ser
    450                 455                 460

Ser Thr Glu Glu Ala Val Ser Pro Lys Ile Lys Ser Arg Ser Cys Asp
465                 470                 475                 480

Asp Leu Leu Asn Asp Asp Cys Gly Ser Phe Pro Asp Pro Lys Thr Lys
                485                 490                 495

Ser Glu Ser Met Gly Ser Leu Leu Cys Asp Glu Gly Ser Lys Glu Ser
            500                 505                 510

Asp Pro Met Thr Trp Thr Ser Pro Tyr Ile Pro Glu Val Cys Gly Asn
        515                 520                 525

Ser Arg Ser Arg Leu Lys His Arg Ser Ala His Asn Ala Pro Gly Phe
    530                 535                 540

Leu Lys Met Tyr Lys Lys Met His Arg Ile Asn Arg Lys Asp Leu Met
545                 550                 555                 560

Asn Ser Glu Val Ile Cys Ser Val Lys Ser Arg Ile Leu Gln Tyr Glu
                565                 570                 575

Lys Glu Gln Gln His Arg Gly Leu Leu His Gly Trp Ser Gln Ser Ser
            580                 585                 590

Thr Glu Glu Val Pro Arg Asp Val Val Pro Thr Arg Ile Ser Glu Phe
        595                 600                 605

Glu Lys Leu Ile Gln Lys Ser Lys Ser Met Pro Asn Leu Gly Asp Glu
    610                 615                 620

Met Leu Ser Pro Val Thr Leu Glu Pro Pro Gln Asn Gly Leu Cys Pro
625                 630                 635                 640

Lys Arg Arg Phe Ser Ile Glu Ser Leu Leu Glu Glu Glu Thr Gln Val
                645                 650                 655

Arg His Pro Ser Gln Gly Gln Arg Ser Cys Lys Ser Asn Thr Leu Val

660                 665                 670

Pro Ile His Ile Glu Val Thr Ser Asp Glu Gln Pro Arg Thr His Met
        675                 680                 685

Glu Phe Ser Asp Ser Asp Gln Asp Gly Val Val Ser Asp His Ser Asp
    690                 695                 700

Asn Val His Val Glu Arg Ser Ser Phe Cys Ser Glu Ser Asp Phe Asp
705                 710                 715                 720

His Phe Ser Phe Thr Ser Ser Glu Ser Phe Tyr Gly Ser Ser His His
                725                 730                 735

His His His His His His His His Gly His Phe Ile Ser Ser Cys Lys
            740                 745                 750

Gly Arg Cys Pro Ala Ser Tyr Thr Arg Phe Thr Thr Met Leu Lys His
        755                 760                 765

Glu Arg Ala Lys His Glu Asn Ile Asp Arg Pro Arg Arg Gln Asp Met
    770                 775                 780

Asp Pro Gly Leu Ser Lys Leu Ala Phe Leu Val Ser Pro Val Pro Phe
785                 790                 795                 800

Arg Arg Lys Lys Val Leu Thr Pro Gln Lys Gln Thr Glu Gln Ala Lys
                805                 810                 815

Cys Lys Ala Ser Val Val Glu Ala Leu Asp Ser Ala Leu Lys Asp Ile
            820                 825                 830

Cys Asp Gln Ile Lys Ala Glu Lys Arg Arg Gly Ser Leu Pro Asp Asn
        835                 840                 845

Ser Ile Leu His Arg Leu Ile Ser Glu Leu Leu Pro Gln Ile Pro Lys
    850                 855                 860

Arg Asn Ser Ser Leu Asn Ala Leu Lys Arg Ser Pro Met His Gln Pro
865                 870                 875                 880

Phe His Pro Leu Pro Gln Asp Gly Ala Ile His Cys Pro Leu Tyr Gln
                885                 890                 895

Asn Asp Cys Gly Arg Met Pro His Ser Ala Ser Phe Pro Asp Val Asp
            900                 905                 910

Thr Thr Ser Ser Tyr His Ala Gln Asp Tyr Gly Ser Val Leu Ser Leu
        915                 920                 925

Gln Asp His Glu Ser Pro Arg Ser Tyr Ser Ser Thr Leu Thr Asp Leu
    930                 935                 940

Gly Arg Ser Val Ser Arg Glu Arg Arg Gly Thr Pro Glu Lys Glu Val
945                 950                 955                 960

Lys Leu Pro Ala Lys Ala Val Tyr Asp Phe Lys Ala Gln Thr Ser Lys
                965                 970                 975

Glu Leu Ser Phe Lys Lys Gly Asp Thr Val Tyr Ile Leu Arg Lys Ile
            980                 985                 990

Asp Gln Asn Trp Tyr Glu Gly Glu His His Gly Arg Val Gly Ile Phe
995                 1000                1005

Pro Ile Ser Tyr Val Glu Lys Leu Thr Pro Pro Glu Lys Ala Gln Pro
1010                1015                1020

Ala Arg Pro Pro Pro Pro Val Gln Pro Gly Glu Ile Gly Glu Ala Ile
1025                1030                1035                1040

Ala Lys Tyr Asn Phe Asn Ala Asp Thr Asn Val Glu Leu Ser Leu Arg
                1045                1050                1055

Lys Gly Asp Arg Ile Ile Leu Leu Lys Arg Val Asp Gln Asn Trp Tyr
            1060                1065                1070

Glu Gly Lys Ile Pro Gly Thr Asn Arg Gln Gly Ile Phe Pro Val Ser
        1075                1080                1085

Tyr Val Glu Val Val Lys Arg Asn Thr Lys Gly Ser Glu Asp Tyr Pro
1090                1095                1100

Asp Pro Pro Leu Pro His Ser Tyr Ser Ser Asp Arg Ile Tyr Ser Leu
1105                1110                1115                1120

Ser Ser Asn Lys Pro Gln Arg Pro Val Phe Ser His Glu Asn Ile Gln
                1125                1130                1135

Gly Gly Gly Glu Pro Phe Gln Ala Leu Tyr Asn Tyr Thr Pro Arg Asn
            1140                1145                1150

Glu Asp Glu Leu Glu Leu Arg Glu Ser Asp Val Val Asp Val Met Glu
        1155                1160                1165

Lys Cys Asp Asp Gly Trp Phe Val Gly Thr Ser Arg Arg Thr Lys Phe
1170                1175                1180

Phe Gly Thr Phe Pro Gly Asn Tyr Val Lys Arg Leu
1185                1190                1195


<210> 156
<211> 6331
<212> DNA
<213> Rattus norvegicus

<220>
<223> SH3-containing protein

<220>
<221> misc_feature
<222> 5164, 5218, 5650, 5663, 5916, 5948, 5961, 6030, 6051, 6127
<223> n is a or g or c or t

<220>
<221> misc_feature
<222> 6172, 6222, 6286, 6301, 6319
<223> n is a or g or c or t

<400> 156
gaattcggca cgaggaaaaa tctttggaga gaagagagcc acagtgagca cgctagtgca 60

caattattgc agccacgtgt gcccgcaagc tggccctgcg acaagctgtt gagtgctgtt 120
gcaattagct gattggagaa cggggactgc agggtgataa tgctgcgtct ccgctcgcgg 180
gcaccaggaa agggttttgt ctcgggaagg caagtcttcc ctgcacagtt atctcagcag 240
ctccctagct gaagagaact gggggctcta aagggagggg gtcgcactgt gcgagcacag 300
attctgtgcc aggctgttgc ttatgaaccg cacgtctggg aaagcaggtg tgtgctcgga 360
cgggcactgg gctggaacgc aggcggcggc tctcgggttc acctgcttcc tgttaacaga 420
ctgttggctc acagagcatc tgctcttaca cgctgaaact gcggctgaga aagggttccc 480
ggcatcccac ttgactgacg gaggcacttg gattggactt aatcttaaac ctctggaggt 540
caagaccttt taaaaagggc taaataaaca atctacatgt taaaggccag cgactcctac 600
ttcctctgtt ggagcaactg tgaagtccag cctcttctag gaaaactgaa gactttaata 660
acacaccgtt caaggtgaaa atgaatacag atagcggtgg gtgtgctcgc aaacgtgccg 720
ccatgtctgt cacgttaaca tctgtgaaga gagttcaaag ttctccaaac ctattggctg 780
cagggcgtga gtctcactct ccggactcag cttggagatc ttacaatggt cgaaatccag 840
agacactgaa cggagatgcc acatattcct ctcttgcagc aaaaggtttt agaagcgttc 900
gaccaaacct gcaagacaaa aagtcaccaa cccagagcca tatcactatc aacggcaact 960
ctggtggcgc cgtgagtcca gtgagttact atcagaggcc attctcccct tctgcatact 1020
ccctcccagc ctcactcaac tccagcatta tcatgccaca cggcaggtcg cttgattctg 1080
cggagacata ttcccagcat gcccagtcgc tagacggcac catgggaagc tccatcccac 1140
tctacagatc ctccgaggaa gagaagaggg tcacagtcat caaagccccg cattacccag 1200
ggatcggccc tgtggatgag tctggaatcc ccacagccat tagaacgaca gttgaccggc 1260
cgaaggactg gtacaagaca atgtttaaac aaattcacat ggtacacaag ccggatgagg 1320
acacagacat gtataatact ccttacacat acaatgcagg tctgtacaac tcgccctaca 1380
gtgctcagtc acatcctgct gcaaagaccc agacctacag acctctttcc aaaagccact 1440
cggacaatgg caccgatgct tttaaggagg caccctcacc agtgcctccc ccacacgttc 1500
caccacgacc aagagatcag tcttcaacag aaaagcatga ctgggatccc ccagacagaa 1560
aggtggacac caggaaattt cgatcggagc caaggagtat ttttgaatac gagcctggga 1620
agtcatccat cctgcagcac gaaagacccg tctccgtcta ccagtcttcc atagacagaa 1680
gcttggaaag acccagcagc tctgcaagca tggcgggtga ctttagaaaa cggaggaaga 1740
gtgaacctgc agtgggcccg cccaggggct tgggggatca cagttcaagc aggaccagcc 1800
ccggccgggc agacctccca ggatcaagtt ccacctttac cacgtctttc attagttctt 1860
ctccttcctc tccctcgaga gcacaaggtg gggatgatag caaaatgtgt ccgccccttt 1920
gcagttactc ggggctcaat ggctcgccct ctagtgagtt agagtgctgc ggcgcttata 1980
gaaggcactt ggacgtcccc caggactctc aaagggccat cactttcaag aacggctggc 2040
aaatggcccg gcaaaatgca gagatctgga gtagcactga agaggcggtt tcccccaaaa 2100
tcaaatcacg aagctgtgac gatctcctga atgatgactg cggcagcttc ccagacccta 2160
aaaccaagtc agaaagcatg ggttctctgt tatgtgacga aggctccaaa gagagcgacc 2220
ccatgacgtg gacttccccc tacatcccgg aagtgtgcgg gaacagcaga tctaggctca 2280
aacataggtc agcccataac gccccaggct tcctcaaaat gtacaagaaa atgcaccgca 2340
tcaaccgcaa ggatttgatg aactcggagg tcatttgctc tgtgaaatcc aggatccttc 2400
agtacgagaa ggaacagcag cacagggggc tgctccatgg atggagccag tcgtccaccg 2460
aggaggtgcc cagggacgtg gtacccactc gcatctcgga gtttgagaag ctgattcaga 2520
agtcaaagtc tatgcccaat ctaggagatg aaatgttatc tcctgtaacc ctagaacccc 2580
cacaaaatgg tttgtgcccc aagaggcgat tttctattga gtctctgctg gaggaggaaa 2640
ctcaggtccg acacccttct cagggtcagc gaagctgcaa gtcgaacacc ctggtaccca 2700
tccacatcga ggtcaccagc gatgagcaac ctagaacaca tatggagttt tccgacagtg 2760
accaagatgg ggttgtgtct gaccacagcg ataacgtcca cgtcgaaagg tcgtcctttt 2820
gtagtgaaag tgacttcgac cacttttcat tcacatcctc tgaaagtttc tacggatcca 2880
gccatcacca ccaccatcac caccaccatc acggacactt catcagttcc tgcaaaggcc 2940
gatgccccgc ttcttacact cgatttacca cgatgttaaa acacgaaaga gctaagcatg 3000
aaaatattga ccgacccaga aggcaagaca tggatcctgg cctatctaaa ctcgcgtttc 3060
tagtcagccc tgtgcctttc cgaaggaaaa aagttttgac tccccaaaaa caaactgagc 3120
aggcaaaatg caaagcctcg gtagttgagg ctctggactc tgcccttaaa gacatttgcg 3180
accaaataaa agctgaaaag cggagaggaa gcttgccgga caacagcatc ctgcacaggc 3240
ttattagtga actgctgcca cagattccta agaggaattc atctcttaat gctctaaaaa 3300
ggagccccat gcaccagcct ttccacccac tgcctcaaga tggtgctatt cattgtcccc 3360
tgtaccaaaa tgattgtggg agaatgcctc acagtgcctc tttcccagac gtggacacga 3420
ccagcagcta ccacgcacag gactatggta gtgtgctgag tctccaagat cacgagtccc 3480
ctagaagtta ctcgtctact ctgactgact tgggaagaag tgtatcacgg gaacgaagag 3540
gaactccaga aaaagaggta aaattgcctg caaaagctgt ctatgatttc aaagctcaga 3600
cttctaagga gctgtcattt aagaaaggag acaccgtcta catcctcagg aaaattgacc 3660
agaactggta tgagggggag caccacggaa gagtgggcat tttcccaatc tcatacgtag 3720

agaaactaac acccccagaa aaagcgcagc ccgcgagacc accaccccca gtccagccgg 3780
gagagattgg agaagccata gccaagtaca acttcaatgc agacacaaat gtggaactct 3840
ccctgagaaa gggtgacagg attattcttc tcaaaagagt tgatcaaaac tggtatgaag 3900
gtaaaatccc aggaaccaac agacaaggca tcttccctgt ctcctacgta gaagttgtca 3960
agaggaacac gaaaggttct gaggattacc ccgaccctcc tctaccccac agctactcca 4020
gtgatagaat ttacagccta agctccaata agccacagcg tcctgtgttc tctcacgaaa 4080
acattcaagg tggaggagaa ccgtttcagg ctctgtataa ctatactcct aggaatgaag 4140
atgagctgga actcagagaa agtgatgtcg tagatgtcat ggaaaagtgt gatgacggat 4200
ggttcgtggg aacttcaaga agaaccaaat tctttggtac ttttcctgga aactatgtca 4260
aaaggctgtg actcacctca ctcctaattt atgccacatt tcagccacac atctgcatta 4320
acccacctga aacgtcccag gaggcctgtt gctgcctcgc cttatggttt cccaatagcc 4380
cattaccatc tccatctgct gccaccaaat caccagcaga gggactgccg ctgtgagcct 4440
tagggaggct gggagcctta gagaaaagtg gcaaaactta cacccacata aatattcagt 4500
ctcctgcttt ctgccctgaa ctttgaaatg cctgtatatg gaatcagaat gaaaatgatc 4560
atactttcaa aaaagtgaaa taattaagga agaaagaaag agaaaagaaa tagagagact 4620
cttcaggagg ctgtctggcc tcatggctga atctccacct ctctggaagg tgtactgtcc 4680
tcaggaagcc tgaagattgt tttttttctg aaatgctatg gttccagttc tcactctcat 4740
ctaggcggta tattttcctt tcacgagttt gcctagcgct cgggtttaca ctacatgaca 4800
actatacttc ggctgttgtt tgcttgcact tattattcct tgtttcatgc acagtgatca 4860
caaaatccag agtgcctagg gaaggttcac tggttccact ggttcgagtg tgatttttgt 4920
tgactgcatt atattttcac acggggaggg gggtctttcc cctgcccact tttttgtgct 4980
tattagaagt gcaaacagtg agcaactgag agctcagcca caccacagga caaatccgtg 5040
ttgtgaattc gcattgctgt tttgtgtatt aaggtgtaat catcagcttc atggacaaca 5100
agctattagt gatttcttta cctgttaaaa cttacaggca gtgctagtga gttaggcaga 5160
aagntgacag taataccagt aggtgagctt cactgcgtgc atgctcacac gtttgagntt 5220
gtatgaggac atataattca tatgctatgt tgtacatttt atggaaatat aagagaatcc 5280
cacattattt tatagagtac ttcaggagca tcctaagtgt taaggctggc tttagcaagg 5340
attatgatca atacaactat ttttactaca ataattattt ttcttctatg agacccagaa 5400
tcctgactcc acttgcagac aggaatatat atgttgagcc tgactttttt ttctggtata 5460
tgtaaaatac ttcccaggaa tacattgggc acttttggga ataatggtta aatcattcag 5520
gttgtgcttc ctgcccccaa aacagatcta caaaatgata ccaaacctga aagatttaac 5580
ggatttacgg tgcctgcatt ccacacaacc tcacacttag ctttgtattt caaatgaatt 5640
tgcataaaan ctgttcactt tancacctta tagtcaaaac tttttatggc tttcctccca 5700
tgggcaatgc ttgatcttcc caacatataa actctggcat attttgttca tatgtttgtt 5760
cctttttggt tgtacagact atttacttgt tcagaaaaca tcgagatctc ccaatttgtt 5820
ctttaccccg cccttaaaag gaatttaaac tctttcagaa gatcgccctt caccacatct 5880
ccacagatca caagctaagg tgaatctgga atatancgtc tgcacaaaat tttgtgactc 5940
agaaaganct ttgtaactac nctgaaatac atataataac aatgttccag ttacagagga 6000
atattgttgg ggcaggaagt gaagaaacan cttcaagaaa cccactttac nctccagttc 6060
acaactagct ttatattaga aaaacttggg attggaaagt cagccagcca gccggccacc 6120
tgcagcnttg ttgataaatg aatacttttt cacaccattt atgaaaacaa ancttcaact 6180
ctgttgcctg ttatatttaa gaaaaattgc tgtttctact cnctgtatct gattttaaaa 6240
ggaaaaaaat attcacgcct ggctttcagg acattgactt tgaatnctta cgagcaaagg 6300
ncgttgtgtt tttcttgcnc gtgccgaatt c 6331

<210> 157
<211> 766
<212> PRT
<213> Homo sapiens

<220>
<223> Sperm-specific protein

<400> 157
Met Ile His Asn Tyr Met Glu His Leu Glu Arg Thr Lys Leu His Gln
1               5                   10                  15

Leu Ser Gly Ser Asp Gln Leu Glu Ser Thr Ala His Ser Arg Ile Arg
            20                  25                  30

Lys Glu Arg Pro Ile Ser Leu Gly Ile Phe Pro Leu Pro Ala Gly Asp
        35                  40                  45

Gly Leu Leu Thr Pro Asp Ala Gln Lys Gly Gly Glu Thr Pro Gly Ser
    50                  55                  60

Glu Gln Trp Lys Phe Gln Glu Leu Ser Gln Pro Arg Ser His Thr Ser
65                  70                  75                  80

Leu Lys Asp Glu Leu Ser Asp Val Ser Gln Gly Gly Ser Lys Ala Thr
                85                  90                  95

Thr Pro Ala Ser Thr Ala Asn Ser Asp Val Ala Thr Ile Pro Thr Asp
            100                 105                 110

Thr Pro Leu Lys Glu Glu Asn Glu Gly Phe Val Lys Val Thr Asp Ala
        115                 120                 125

Pro Asn Lys Ser Glu Ile Ser Lys His Ile Glu Val Gln Val Ala Gln
    130                 135                 140

Glu Thr Arg Asn Val Ser Thr Gly Ser Ala Glu Asn Glu Glu Lys Ser
145                 150                 155                 160

Glu Val Gln Ala Ile Ile Glu Ser Thr Pro Glu Leu Asp Met Asp Lys
                165                 170                 175

Asp Leu Ser Gly Tyr Lys Gly Ser Ser Thr Pro Thr Lys Gly Ile Glu
            180                 185                 190

Asn Lys Ala Phe Asp Arg Asn Thr Glu Ser Leu Phe Glu Glu Leu Ser
        195                 200                 205

Ser Ala Gly Ser Gly Leu Ile Gly Asp Val Asp Glu Gly Ala Asp Leu
    210                 215                 220

Leu Gly Met Gly Arg Glu Val Glu Asn Leu Ile Leu Glu Asn Thr Gln
225                 230                 235                 240

Leu Leu Glu Thr Lys Asn Ala Leu Asn Ile Val Lys Asn Asp Leu Ile
                245                 250                 255

Ala Lys Val Asp Glu Leu Thr Cys Glu Lys Asp Val Leu Gln Gly Glu
            260                 265                 270

Leu Glu Ala Val Lys Gln Ala Lys Leu Lys Leu Glu Glu Lys Asn Arg
        275                 280                 285

Glu Leu Glu Glu Glu Leu Arg Lys Ala Arg Ala Glu Ala Glu Asp Ala
    290                 295                 300

Arg Gln Lys Ala Lys Asp Asp Asp Asp Ser Asp Ile Pro Thr Ala Gln
305                 310                 315                 320

Arg Lys Arg Phe Thr Arg Val Glu Met Ala Arg Val Leu Met Glu Arg
                325                 330                 335

Asn Gln Tyr Lys Glu Arg Leu Met Glu Leu Gln Glu Ala Val Arg Trp
            340                 345                 350

Thr Glu Met Ile Arg Ala Ser Arg Glu Asn Pro Ala Met Gln Glu Lys

355                 360                 365

Lys Arg Ser Ser Ile Trp Gln Phe Phe Ser Arg Leu Phe Ser Ser Ser
    370                 375                 380

Ser Asn Thr Thr Lys Lys Pro Glu Pro Pro Val Asn Leu Lys Tyr Asn
385                 390                 395                 400

Ala Pro Thr Ser His Val Thr Pro Ser Val Lys Lys Arg Ser Ser Thr
                405                 410                 415

Leu Ser Gln Leu Pro Gly Asp Lys Ser Lys Ala Phe Asp Phe Leu Ser
            420                 425                 430

Glu Glu Thr Glu Ala Ser Leu Ala Ser Arg Arg Glu Gln Lys Arg Glu
        435                 440                 445

Gln Tyr Arg Gln Val Lys Ala His Val Gln Lys Glu Asp Gly Arg Val
    450                 455                 460

Gln Ala Phe Gly Trp Ser Leu Pro Gln Lys Tyr Lys Gln Val Thr Asn
465                 470                 475                 480

Gly Gln Gly Glu Asn Lys Met Lys Asn Leu Pro Val Pro Val Tyr Leu
                485                 490                 495

Arg Pro Leu Asp Lys Lys Asp Thr Ser Met Lys Leu Trp Cys Ala Val
            500                 505                 510

Gly Val Asn Leu Ser Gly Gly Lys Thr Arg Asp Gly Gly Ser Val Val
        515                 520                 525

Gly Ala Ser Val Phe Tyr Lys Asp Val Ala Gly Leu Asp Thr Glu Gly
    530                 535                 540

Ser Lys Gln Arg Ser Ala Ser Gln Ser Ser Leu Asp Lys Leu Asp Gln
545                 550                 555                 560

Glu Leu Lys Glu Gln Gln Lys Glu Leu Lys Asn Gln Glu Glu Leu Ser
                565                 570                 575

Ser Leu Val Trp Ile Cys Thr Ser Thr His Ser Ala Thr Lys Val Leu
            580                 585                 590

Ile Ile Asp Ala Val Gln Pro Gly Asn Ile Leu Asp Ser Phe Thr Val
        595                 600                 605

Cys Asn Ser His Val Leu Cys Ile Ala Ser Val Pro Gly Ala Arg Glu
    610                 615                 620

Thr Asp Tyr Pro Ala Gly Glu Asp Leu Ser Glu Ser Gly Gln Val Asp
625                 630                 635                 640

Lys Ala Ser Leu Cys Gly Ser Met Thr Ser Asn Ser Ser Ala Glu Thr
                645                 650                 655

Asp Ser Leu Leu Gly Gly Ile Thr Val Val Gly Cys Ser Ala Glu Gly
            660                 665                 670

Val Thr Gly Ala Ala Thr Ser Pro Ser Thr Asn Gly Ala Ser Pro Val
        675                 680                 685

Met Asp Lys Pro Pro Glu Met Glu Ala Glu Asn Ser Glu Val Asp Glu
    690                 695                 700

Asn Val Pro Thr Ala Glu Glu Ala Thr Glu Ala Thr Glu Gly Asn Ala
705                 710                 715                 720

Gly Ser Ala Glu Asp Thr Val Asp Ile Ser Gln Thr Gly Val Tyr Thr
                725                 730                 735

Glu His Val Phe Thr Asp Pro Leu Gly Val Gln Ile Pro Glu Asp Leu
            740                 745                 750

Ser Pro Val Tyr Gln Ser Arg Tyr Asn Asn Gly Ser Ser Thr
        755                 760                 765

<210> 158
<211> 2523
<212> DNA
<213> Homo sapiens

<220>
<223> Sperm-specific protein

<400> 158
gacaacttga gctgaaagcg aaaaactatg ctgaccagat tagcagactt gaagaaagag 60
aagcagaact gaagaaggaa tataatgcat tacatcaaag acacactgag atgatccata 120
attatatgga acatttagaa agaacaaaac ttcatcagct ctcagggagt gatcaactag 180
aatccacagc tcatagtaga attagaaaag aacgccctat atcattagga attttcccat 240
tacctgctgg agatggattg cttacacctg atgctcagaa aggaggagag acccctggat 300
ctgagcaatg gaaatttcag gaattaagtc aaccacgttc tcataccagc ctgaaggatg 360
agctttctga tgttagccaa ggcggatcta aagctaccac tccagcatca acagctaatt 420
cagatgtggc aacaattcct actgatactc ccttaaagga agaaaacgaa ggatttgtga 480
aggttacaga tgcgccaaat aaatcagaga taagcaaaca cattgaagta caggtagccc 540
aggaaactag aaatgtatct actggctctg ctgaaaatga agaaaagtca gaagttcaag 600
caatcatcga atctactcct gagctggata tggacaaaga tctcagtgga tataaaggtt 660
caagcactcc caccaaaggc atagagaaca aagcttttga tcgcaataca gaatctctct 720
ttgaagaact gtcttcagct ggctcaggcc taataggaga tgtggatgaa ggagcagatt 780
tactaggaat gggtcgggaa gttgagaatc ttatattaga aaatacacaa ctgttggaaa 840
ccaaaaatgc tttgaacata gtgaagaatg atttgatagc aaaagtggat gaactgacct 900
gtgagaaaga tgtgctgcaa ggggaattgg aggctgtgaa gcaagccaaa ctgaaactag 960
aggaaaagaa cagagaattg gaggaagagc ttaggaaagc tcgggcagaa gctgaagatg 1020
caaggcaaaa agcaaaagat gacgatgata gtgatattcc cacagcccag aggaaacggt 1080
ttactagagt agaaatggcc cgtgttctca tggagcgaaa ccagtataaa gagagattga 1140
tggagcttca ggaagctgtt cgatggacag agatgattcg ggcatcacga gaaaatccag 1200
ccatgcagga aaaaaaaagg tcaagcattt ggcagttttt cagccgactt ttcagctcct 1260
caagtaacac gactaagaag cctgaaccac ctgttaatct gaagtacaat gcacccacgt 1320
ctcatgttac tccgtccgtc aagaaaagaa gcagcacctt atctcagctc cctggggata 1380
agtccaaagc ctttgatttc cttagtgaag aaactgaagc tagtttagcc tcacgcagag 1440
aacaaaagag agagcagtat cgtcaggtaa aagcacatgt tcagaaggaa gacggtagag 1500
tgcaggcttt tggctggagt ctgcctcaga agtacaaaca ggtaaccaat ggtcaaggtg 1560
aaaataagat gaaaaattta cctgtgcctg tctatctcag acctctggat aaaaaagata 1620
catcaatgaa gctgtggtgt gctgttggag tcaatttatc tggtgggaag accagagatg 1680
gtggttctgt tgttggagca agtgtatttt acaaggatgt tgctggtttg gatacagaag 1740
gcagtaaaca gcgaagtgcc tctcagagta gtttagataa gttagatcag gaacttaagg 1800
aacagcagaa ggagttaaaa aatcaagaag aattatccag tctagtttgg atctgtacca 1860
gcactcattc ggctacaaaa gttcttatta ttgatgctgt tcaacctggc aacatcctag 1920
acagtttcac tgtttgcaac tctcatgttc tgtgcattgc aagtgtgcca ggtgcacgag 1980
aaacagacta ccctgcagga gaagatcttt cagaatctgg tcaggtagac aaagcatctt 2040
tatgtggaag tatgacaagc aacagctcag cagagacaga cagcctgtta ggaggcatca 2100

cagtggttgg ttgttctgca gaaggtgtga cgggagctgc cacttcccct agtacaaatg 2160
gtgcttctcc agtgatggat aaaccaccag aaatggaagc agaaaatagt gaggttgatg 2220
aaaatgttcc aacagcagaa gaagcaactg aagctacaga agggaatgcg gggtcagctg 2280
aagacacagt ggacatctcc caaactggcg tctacacaga gcatgtcttt acagatcctt 2340
tgggagttca gatcccagaa gacctctccc cagtgtatca gtcgaggtat aataatgggt 2400
catcaactta ggcaactggg tgaagtgcac aaataaaagg aaccaaaata atctcaaaca 2460
gtcaaaagat ttccactaat tagtgtcact gtggtggatt aaaagatacc tattcttgtg 2520
ata 2523

<210> 159
<211> 123
<212> PRT
<213> Rattus norvegicus

<220>
<223> Guanine-nucleotide-releasing protein

<400> 159
Met Glu Pro Cys Glu Leu Gln Asn Glu Leu Val Ser Ala Glu Gly Arg
1               5                   10                  15

Asn Arg Lys Ala Val Leu Cys Gln Arg Cys Gly Ser Arg Val Leu Gln
            20                  25                  30

Pro Gly Thr Ala Leu Phe Ser Arg Arg Gln Leu Phe Leu Pro Ser Met
        35                  40                  45

Arg Lys Lys Pro Asp Leu Val Asp Gly Ser Asn Pro Asp Gly Asp Val
    50                  55                  60

Leu Glu Glu His Trp Leu Val Asn Asp Met Phe Ile Phe Glu Asn Val
65                  70                  75                  80

Gly Phe Thr Lys Asp Val Gly Asn Val Lys Phe Leu Val Cys Ala Asp
                85                  90                  95

Cys Glu Ile Gly Pro Ile Gly Trp His Cys Leu Asp Asp Lys Asn Ser
            100                 105                 110

Phe Tyr Val Ala Leu Glu Arg Val Ser His Glu
        115                 120

<210> 160
<211> 2490
<212> DNA
<213> Rattus norvegicus

<220>
<223> Guanine-nucleotide-releasing protein

<400> 160
ggtagccact ttggacttat cttcaagaat gtgacatttg tggtaatatt ctttgcttcc 60
ccatggcctc accgtgccac aacagtgtta cgtgtttgac actcactgct tgtgcaccca 120
tcacttctga tgtatggcag ttttgggtca ataagatatt agtattactt tgactttgac 180
ttgtgagtct aggttaaaat cattcggagg attttttatt ctccgaggtc accccaaccg 240
aaatttttta gttcatattt attttgtttt agcccattag gttgttttta tataattgaa 300
ctagtaaatt aaagctccat agggcttctc cgtcttattg ggagattcca gcctcttcac 360
tggaaggtca atttcactga ttgaaagtaa gagacagttg aaccctcgtt tagccattca 420
ttctagtccc taattaagga acaagtgatt atgctacctt tgcacggtca ggataccgcg 480

gccgtttaac tttagtcact gggcaggcaa tgctctaata cttgttatgc tagaggtatg 540
tttttggtaa acaggcgggg ttcatgtttg ccgagttcct tttacttttt aatctttcct 600
taaagcacgc ctgtgttggg ctaacgagtt agggataggt aattctattg ttgggttagt 660
acctatgatt cgataattga caatggttat ccgggttgtc atacacttgt gctaggagag 720
gcaatggaac cctgcgagct gcagaacgag ctagtgtcag ccgaaggccg gaaccggaag 780
gcggtgctgt gccagcgttg tggctctcgg gtgctgcagc cagggactgc tcttttctct 840
cgccggcagc ttttcctccc ctccatgagg aagaagccgg atctggtgga cggcagcaat 900
cctgatggtg atgtcctcga ggaacactgg ctggttaatg acatgttcat ctttgagaac 960
gtgggcttta ccaaggacgt gggcaacgtc aagtttctag tctgtgcaga ctgtgagatt 1020
ggacccattg gctggcattg cctagatgac aagaacagct tctatgtggc cttggagcga 1080
gtttcccacg agtaactgag gggtttggga cttggctcca ccctagctat aaagctctgc 1140
ccacaagaac cgacactgat gtgtcagcag tgctggcaga cttcttggta ctaatataag 1200
caccggtgtt tgcccttgaa cttgcagagg gccggtcctg tttcctcagg cctcagagca 1260
tgtctcctac ttagtcctct ctgtcacatg tgtcctagag ccctttcccc tgacttggaa 1320
catggaagaa cacgcgagtg ctctaccagt gtagtctggt tccggcactc tgcctccgca 1380
cctcgatgcc aagctctctt cttctcagca agaacaacat tcatctttag ccatcacgac 1440
gggaactaag atgctaatat ttgaaactcc acctttcctc attctagaag ccaagtgtga 1500
tagggtgcac atctgtaatc ctaggtcttg agtgggtaga ggcaggaaga tcaggagttc 1560
aagggcagcc ttagctacat agactgggcc aagtgagatg ttgtctcaaa aaacataaag 1620
catacaagta tttgagagta atctcattgt atgtgaccta cttatctaca gtaatttatt 1680
ctttttaaaa gtaaaacatt tacagatgtc agtatttaaa cctcacttta gatacatttt 1740
tagtactgtg tttgagagcc ttttttccgg gtcaatagtg gcattttata atgaggacga 1800
acacgactcc tcttccctca gatcttgatc aggtttctct ttttagtcat tccttgcccc 1860
tgatgtcaac cctaaggaaa actcagccct agctgtgtgc cagactctct tgtaaacctg 1920
ttccacggac atttctattg ttatggacaa ggtgttacct tttcttatta ttagccaact 1980
taaatcttca atatgatttc cagttactct gtcatttctc ttctggagtg actcagtagc 2040
ttaaaaggac tctgtgtaaa caggttcaaa tactgtgtca tcatcccctc ctctgagggc 2100
ctaaattgag ataagtagag acttcattat tcctcatgcc agagcttagg aagtgggggc 2160
agatcaagag ttcaaggtca gcctcagtta ctataatatc aaattcaagg ccagcctgta 2220
ctatggagat cttgtttcaa atgaacaaaa cacaaaataa acaagtagga cctgcaaaag 2280
tgacacgtag ttaaaaatgt ttgctgctct ttcaggcgac ctgagttcag gaccttgcac 2340
cctcatggga ggtttattcc agttcaagga gatctgcgcc ttctggtccc tgcagatgcc 2400
ttcatgtgtg tggcacccat acaaacaagt gagcacactc gcgtacacac acacacacgc 2460
acgcacgcac gtgaataaaa attttacaaa 2490

<210> 161
<211> 112
<212> PRT
<213> Rattus norvegicus

<220>
<223> Intracellular calcium binding protein

<400> 161
Met Ala Ala Lys Thr Gly Ser Gln Leu Glu Arg Ser Ile Ser Thr Ile
1               5                   10                  15

Ile Asn Val Phe His Gln Tyr Ser Arg Lys Tyr Gly His Pro Asp Thr
            20                  25                  30

Leu Asn Lys Ala Glu Phe Lys Glu Met Val Asn Lys Asp Leu Pro Asn
        35                  40                  45

Phe Leu Lys Arg Glu Lys Arg Asn Glu Asn Leu Leu Arg Asp Ile Met
    50                  55                  60

Glu Asp Leu Asp Thr Asn Gln Asp Asn Gln Leu Ser Phe Glu Glu Cys
65                  70                  75                  80

Met Met Leu Met Gly Lys Leu Ile Phe Ala Cys His Glu Lys Leu His

                    85                  90                  95

Glu Asn Asn Pro Arg Gly His Asp His Arg His Gly Lys Gly Cys Gly
            100                 105                 110

<210> 162
<211> 494
<212> DNA
<213> Rattus norvegicus

<220>
<223> Intracellular calcium binding protein

<400> 162
cggcacgagc tccttagctt tgagcaagaa gatggctgcc aaaacaggat ctcagctgga 60
gcgcagcata agcaccatca tcaatgtttt ccatcagtac tctaggaagt atggacatcc 120
tgacaccctg aacaaggcgg aattcaaaga aatggtgaat aaggacttgc caaattttct 180
gaagagggag aaaagaaatg aaaatctcct aagagacatc atggaggacc tggacacaaa 240
ccaggacaat caactgtcct ttgaggagtg tatgatgctg atgggaaagt tgatctttgc 300
ctgtcatgag aagctgcatg agaacaaccc acgtgggcat gaccacaggc acggcaaagg 360
ctgtgggaag taattaagag gtcgccatgt aacatctgcc caaccaagtc taaagggaat 420
agcttactaa atgaccttgg ttctggggct gggaaataat ttaaaaatga ataaataaag 480
tctttatcca ttcc                                                   494

<210> 163
<211> 532
<212> PRT
<213> Rattus norvegicus

<220>
<223> PTB-like protein

<400> 163
Met Asp Gly Ile Val Thr Glu Val Ala Val Gly Val Lys Arg Gly Ser
1               5                   10                  15

Asp Glu Leu Leu Ser Gly Ser Val Leu Ser Ser Pro Asn Ser Asn Met
            20                  25                  30

Ser Gly Met Val Val Thr Ala Asn Gly Asn Asp Ser Lys Lys Phe Lys
        35                  40                  45

Gly Glu Asp Lys Met Asp Gly Ala Pro Ser Arg Val Leu His Ile Arg
    50                  55                  60

Lys Leu Pro Gly Glu Val Thr Glu Thr Glu Val Ile Ala Leu Gly Leu
65                  70                  75                  80

Pro Phe Gly Lys Val Thr Asn Ile Leu Met Leu Lys Gly Lys Asn Gln
                85                  90                  95

Ala Phe Leu Glu Leu Ala Thr Glu Glu Ala Ala Ile Thr Met Val Asn
            100                 105                 110

Tyr Tyr Ser Ala Val Thr Pro His Leu Arg Asn Gln Pro Ile Tyr Ile
        115                 120                 125

Gln Tyr Ser Asn His Lys Glu Leu Lys Thr Asp Asn Thr Leu Asn Gln
    130                 135                 140

Arg Ala Gln Val Val Leu Gln Ala Val Thr Ala Val Gln Thr Ala Asn
145                 150                 155                 160

Thr Pro Leu Ser Gly Thr Thr Val Ser Glu Ser Ala Val Thr Pro Ala
                165                 170                 175

Gln Ser Pro Val Leu Arg Ile Ile Ile Asp Asn Met Tyr Tyr Pro Val
            180                 185                 190

Thr Leu Asp Val Leu His Gln Ile Phe Ser Lys Phe Gly Ala Val Leu
        195                 200                 205

Lys Ile Ile Thr Phe Thr Lys Asn Asn Gln Phe Gln Ala Leu Leu Gln
    210                 215                 220

Tyr Gly Asp Pro Val Asn Ala Gln Gln Ala Lys Leu Ala Leu Asp Gly
225                 230                 235                 240

Gln Asn Ile Tyr Asn Ala Cys Cys Thr Leu Arg Ile Asp Phe Ser Lys
                245                 250                 255

Leu Val Asn Leu Asn Val Lys Tyr Asn Asn Asp Lys Ser Arg Asp Tyr
            260                 265                 270

Thr Arg Pro Asp Leu Pro Ser Gly Asp Gly Gln Pro Ala Leu Asp Pro
        275                 280                 285

Ala Ile Ala Ala Ala Phe Ala Lys Glu Thr Ser Leu Leu Ala Val Pro
    290                 295                 300

Gly Ala Leu Ser Pro Leu Ala Ile Pro Asn Ala Ala Ala Ala Ala Ala
305                 310                 315                 320

Ala Ala Ala Ala Gly Arg Val Gly Met Pro Gly Val Ser Ala Gly Gly
                325                 330                 335

Asn Thr Val Leu Leu Val Ser Asn Leu Asn Glu Glu Met Val Thr Pro
            340                 345                 350

Gln Ser Leu Phe Thr Leu Phe Gly Val Tyr Gly Asp Val Gln Arg Val
        355                 360                 365

Lys Ile Leu Tyr Asn Lys Lys Asp Ser Ala Leu Ile Gln Met Ala Asp
    370                 375                 380

Gly Asn Gln Ser Gln Leu Ala Met Asn His Leu Asn Gly Gln Lys Met
385                 390                 395                 400

Tyr Gly Lys Ile Ile Arg Val Thr Leu Ser Lys His Gln Thr Val Gln
                405                 410                 415

Leu Pro Arg Glu Gly Leu Asp Asp Gln Gly Leu Thr Lys Asp Phe Gly
            420                 425                 430

Asn Ser Pro Leu His Arg Phe Lys Lys Pro Gly Ser Lys Asn Phe Gln
        435                 440                 445

Asn Ile Phe Pro Pro Ser Ala Thr Leu His Leu Ser Asn Ile Pro Pro
    450                 455                 460

Ser Val Ala Glu Glu Asp Leu Arg Thr Leu Phe Ala Asn Thr Gly Gly
465                 470                 475                 480

Thr Val Lys Ala Phe Lys Phe Phe Gln Arg Asp His Lys Met Ala Leu
                485                 490                 495

Leu Gln Met Ala Thr Val Glu Glu Ala Ile Gln Ala Leu Ile Asp Leu
            500                 505                 510

His Asn Tyr Asn Leu Gly Glu Asn His His Leu Arg Val Ser Phe Ser
        515                 520                 525

Lys Ser Thr Ile
    530

<210> 164
<211> 3249
<212> DNA
<213> Rattus rattus

<220>
<223> PTB-like protein

<400> 164
   ctcgctggct gcgtggctcg gttcttggga gcgaagcttt gtccggttcg gcaatggacg 60
   gaattgtcac tgaggttgct gttggtgtga agagaggatc tgacgagcta ctctcaggca 120
   gtgttctcag tagtcccaac tctaatatga gtggcatggt agttacagcc aatggtaacg 180
   acagtaagaa atttaaagga gaagataaga tggacggggc gccctctcgt gtgcttcaca 240
   ttcgaaagtt acctggtgaa gtgactgaga ccgaagtaat tgcgttaggc ttaccttttg 300
   gtaaggtgac taacatcctt atgctgaaag gaaagaacca ggcatttttg gaactggcaa 360
   cagaggaagc agctattact atggttaatt actattctgc tgtgacacct catcttcgta 420
   accaaccaat ttacattcag tactccaatc acaaagaact aaagacagat aacacattaa 480
   accaacgtgc tcaagtagtt cttcaagctg tgacagctgt ccagacagca aatacacctc 540
   tcagtggcac cacagtcagt gagagtgcgg tgactccagc tcagagtcca gtgcttagaa 600
   taattattga caatatgtac taccctgtaa cacttgatgt ccttcaccaa atatttctca 660
   agtttggtgc tgtattgaag ataatcacat ttacaaaaaa caaccagttt caggctttgc 720
   tccagtatgg tgatccagta aacgctcaac aggcaaagct agccctagat ggtcaaaata 780
   tttataatgc ttgctgtacc ctaaggattg atttttccaa acttgtgaat ttgaatgtaa 840
   aatacaacaa tgataaaagt agggattata cacgacctga tcttccatct ggagacggcc 900
   agcctgcatt agacccagct attgctgcag catttgccaa ggagacgtcc cttttagctg 960
   tcccaggagc tctcagtcct ttggctattc ctaatgctgc tgcggcagct gctgccgctg 1020
   ccgctggccg agtgggcatg cctggagtct cagctggtgg caacacagtc ctgttggtta 1080
   gcaatttaaa tgaagagatg gttacgcccc aaagtctgtt taccctcttc ggtgtttatg 1140
   gcgatgtgca gcgcgtgaag attttgtaca ataagaaaga cagtgctctg atacaaatgg 1200
   ccgatgggaa ccagtcacag cttgccatga atcatcttaa tgggcagaag atgtatggaa 1260
   aaattattcg tgttactctc tctaaacatc agactgtgca actacctcga gagggacttg 1320
   atgatcaagg gctaacaaaa gattttggga attcacccct gcaccgtttt aaaaaaccgg 1380
   gatccaaaaa ctttcagaac attttccctc cttctgcaac ccttcacctg tccaacatcc 1440
   ctccttctgt agcagaagag gatctgcgaa ctctgtttgc caacaccggg ggcactgtga 1500
   aagcatttaa gttttttcaa agagatcaca aaatggctct tcttcagatg gcaacagtgg 1560
   aggaagctat tcaggctttg attgatcttc ataattataa ccttggtgaa aaccatcatc 1620
   tgagagtgtc tttctccaaa tcaacaattt aaggacggga gatgaagatg gcgggcagat 1680
   cccattgttt ggtgtcatca cctattgact gttcagaaaa gtggggacca gagttgattt 1740
   tttcatgctg ttctcattcc ttggttataa atgaaatggc gtatgtaagg cagagtgcta 1800
   actgctgtat ttcatctgtt cagtagggaa gccattttgt ctgttaaatt ttagtttaat 1860
   tttgcttctt tttttttttt ttcaacttag tgacatacgt gccttacaaa ggaaagctag 1920
   tgttgctatt gtgcatttac taggaaaaag gaattggttg tttagggcac attgttatgt 1980
   ggaaattaaa atctgtttag gcaggggtgt gtcaaaaggt taagtttttg tttctcctgc 2040
   ttggaactta ttttgaatta ttggcttatc acatttcttt ctatttaatc taataagata 2100
   cttgatactg aaagtgtaaa acagcatttt tagttcctac tatcttaggc tttattgctt 2160
   tgaaaacatt ggccttttgt atctcacaaa tctggtctag attcagttat gaatataggc 2220
   attagttaaa attaacaaga tgcagagtat taatttctta agacaagtga tttctgtaag 2280
   tttgagccct atgtggaaag cattgtggaa ccttaacctt tttgtacaca ctcttgtggg 2340
   acgtatcata taaatgtcag cactaagtaa tgtcttgttt gtggctgaat atttttcgta 2400
   gatgtttttg aagttgacat gacttatgtg catttaaata tatattgcca tccttagttt 2460
   gtaattaaga tttggaaaat ggttgtggat ttctgagcat gtgcagactg gtctagctag 2520
   ttcaggaact ggtgcatgta ttttcaaaga caaagaaaag tgtactgcga aaacttgcag 2580
   gaagattaat tttgtggcag ttttctaaaa ctgacaacca ggtgggacca aagtttatgt 2640
   gcctttagtc ttaatttacc ttgcattgta atattcagtt ttataaatct tcaaaatatt 2700
   ttgtatttag gaatagatct gactttaata aaaacatggc tcagaatcta caggtcaaat 2760
   ttatttgaac agttcttgtc aatctgaatt gttgattctg ttaaatgaca atactttttg 2820
   aaattgatgt acttagtttc aagattcata gattctgtta tctatgtaga cagaatggtc 2880
   atgtatattt tctattagtt gagtttttac atctttagaa atgtaaaatt cagtatagtt 2940
   tgaaagcggc acaattaaaa attaattttc taacaaagtt gggaggtctt gaatggttgt 3000
   ttaatttcat tttgtgtgta ctctgcttac ctctgtagca tgctcaataa acacttctgt 3060
   agctctgtat tcaccttttc tgtctttctc tgctgccttt tctctctctc ctttgttttc 3120
   actccactgt gcttctgaat tcatgtttac tctctgccag ggtgggaaag gagtaataat 3180
   actacaatcc tatggcttta taccatgaat aaatccagat gctgtgaaaa tgcaaaaaaa 3240
   aaaaaaaaa 3249

<210> 165
<211> 389
<212> PRT
<213> Spermophilus tridecemlineatus

<220>
<223> ATPase domain protein 44

<400> 165
Met Ala Asp Pro Arg Asp Lys Ala Leu Gln Asp Tyr Arg Lys Lys Leu
1               5                   10                  15

Leu Glu His Lys Glu Ile Asp Gly Arg Leu Lys Glu Leu Arg Glu Gln
            20                  25                  30

Leu Lys Glu Leu Thr Lys Gln Tyr Glu Lys Ser Glu Asn Asp Leu Lys
        35                  40                  45

Ala Leu Gln Ser Val Gly Gln Ile Val Gly Glu Val Leu Lys Gln Leu
    50                  55                  60

Thr Glu Glu Lys Phe Ile Val Lys Ala Thr Asn Gly Pro Arg Tyr Val
65                  70                  75                  80

Val Gly Cys Arg Arg Gln Leu Asp Lys Ser Lys Leu Lys Pro Gly Thr
                85                  90                  95

Arg Val Ala Leu Asp Met Thr Thr Leu Thr Ile Met Arg Tyr Leu Pro
            100                 105                 110

Arg Glu Val Asp Pro Leu Val Tyr Asn Met Ser His Glu Asp Pro Gly
        115                 120                 125

Asn Val Ser Tyr Ser Glu Ile Gly Gly Leu Ser Glu Gln Ile Arg Glu
    130                 135                 140

Leu Arg Glu Val Ile Glu Leu Pro Leu Thr Asn Pro Glu Leu Phe Gln
145                 150                 155                 160

Arg Val Gly Ile Ile Pro Pro Lys Gly Cys Leu Leu Tyr Gly Pro Pro
                165                 170                 175

Gly Thr Gly Lys Thr Leu Leu Ala Arg Ala Val Ala Ser Gln Leu Asp
            180                 185                 190

Cys Asn Phe Leu Lys Val Val Ser Ser Ser Ile Val Asp Lys Tyr Ile
        195                 200                 205

Gly Glu Ser Ala Arg Leu Ile Arg Glu Met Phe Asn Tyr Ala Arg Asp
    210                 215                 220

His Gln Pro Cys Ile Ile Phe Met Asp Glu Ile Asp Ala Ile Gly Gly
225                 230                 235                 240

Arg Arg Phe Ser Glu Gly Thr Ser Ala Asp Arg Glu Ile Gln Arg Thr
                245                 250                 255

Leu Met Glu Leu Leu Asn Gln Met Asp Gly Phe Asp Thr Leu His Arg
            260                 265                 270

Val Lys Met Ile Met Ala Thr Asn Arg Pro Asp Thr Leu Asp Pro Ala
        275                 280                 285

Leu Leu Arg Pro Gly Arg Leu Asp Arg Lys Ile His Ile Asp Leu Pro
    290                 295                 300

Asn Glu Gln Ala Arg Leu Asp Ile Leu Lys Ile His Ala Gly Pro Ile
305                 310                 315                 320

Thr Lys His Gly Glu Ile Asp Tyr Glu Ala Ile Val Lys Leu Ser Asp
                325                 330                 335

Gly Phe Asn Gly Ala Asp Leu Arg Asn Val Cys Thr Glu Ala Gly Met
            340                 345                 350

Phe Ala Ile Arg Ala Asp His Asp Phe Val Val Gln Glu Asp Phe Met
        355                 360                 365

Lys Ala Val Arg Lys Val Ala Asp Ser Lys Lys Leu Glu Ser Lys Leu
    370                 375                 380

Asp Tyr Lys Pro Val
385


<210> 166
<211> 1566
<212> DNA
<213> Spermophilus tridecemlineatus

<220>
<223> ATPase domain protein 44

<400> 166
cttctcatca tggcggaccc tagagataag gcgcttcagg attaccgcaa gaagctgctg 60
gagcacaagg agatcgacgg ccgtcttaag gagttaaggg aacaattaaa agaacttacc 120
aagcagtatg aaaagtctga aaatgatctg aaggcccttc aaagtgttgg gcagattgtg 180

ggtgaagtgc ttaagcaatt aactgaagaa aaattcattg ttaaagctac aaatggacca 240
agatatgttg tgggttgtcg tcgacagctt gacaaaagta agctgaagcc aggaacaaga 300
gttgctttgg atatgactac actaactatc atgagatatt tgccaagaga ggtggatcca 360
cttgtttaca acatgtctca tgaggaccct gggaatgttt cttattctga gattggaggt 420
ctgtcagaac agattcggga attaagagag gtaatagagt tacctcttac aaatccagaa 480
ttattccagc gtgtaggaat aatacctcca aaaggctgtt tgttatatgg accaccaggc 540
acaggaaaaa cactcttggc acgagctgtt gctagccaac tggactgcaa tttcttaaag 600
gttgtatcta gttctattgt agacaagtac attggtgaaa gtgctcgttt gattagagaa 660
atgtttaatt atgccaggga ccatcagcca tgcatcattt ttatggatga aatagatgct 720
attggtggtc gtcggttttc tgaaggtact tcagctgata gagagattca gagaacctta 780
atggagttac tgaatcaaat ggatggattt gatactctgc atagagttaa aatgatcatg 840
gctacaaaca gaccagatac actggaccct gctttgttac gtccaggaag attggataga 900
aaaatacata ttgatttacc aaatgaacaa gcaagattag atatattgaa aatccatgca 960
ggacccatta caaagcatgg tgaaatagat tatgaagcaa ttgtgaagct atcagacggc 1020
tttaatggag cagacctgag aaatgtttgc actgaagcag gtatgtttgc aattcgtgct 1080
gatcatgatt ttgtagttca agaagatttc atgaaagcag tcagaaaagt ggctgattct 1140
aagaagctag agtctaaatt ggactacaaa cctgtgtaat tttatcgtaa ggttttggat 1200
ggctgcatga cagacattga cttaatgtaa aaataaagtt aaggaaaata atgtacgtat 1260
tggcaatgat ctcattaaat gtgaatgaat aaaatatgta tgagtaacat aaaaattagt 1320
atgtaattca gtaattcacc ttttaaaatt gacacagaag aaatttgtat gtttgttaat 1380
gttgcattta ttgcagcaaa agttacaaaa agaagagtgt tgaagctttt cgtatttgcc 1440
atgtgagcat tttgtaaaac actgaaaaat agtttgagat agtagtacaa gagagcattt 1500
cttaagactt attttatatc atttgttttc ttcattctaa caagtttaat aaaatctgtt 1560
tgattt 1566

<210> 167
<211> 108
<212> PRT
<213> Homo sapiens

<220>
<223> BM-020

<400> 167
Met Ala Phe Gln Asn Ser Glu Tyr Gly Asp Asp Ser Ile Lys Lys Ala
1               5                   10                  15

Gln Asn Val Ile Asn Cys Phe Pro Lys Gln Trp Phe Met Asn Leu Lys
            20                  25                  30

Gly Glu Arg Thr Ile Ser Gln Leu Glu Asn Phe Cys Arg Tyr Leu Val
        35                  40                  45

His Leu Ala Asp Thr Ile Tyr Arg Asn Ser Ile Gly Cys Ser Asp Val
    50                  55                  60

Glu Lys Arg Asn Ala Arg Glu Asn Ile Lys Gln Ile Val Lys Leu Leu
65                  70                  75                  80

Ala Ser Val Arg Ala Leu Asp His Ala Met Ser Val Ala Ser Asp His
                85                  90                  95

Asn Val Lys Glu Phe Lys Ser Leu Ile Glu Gly Lys
            100                 105

<210> 168
<211> 1556
<212> DNA
<213> Homo sapiens

<220>
<223> BM-020

<400> 168
aataaaattc tgggccttac ttgttttttc aacgacagtt ttggtcttca gttaagctgt 60
taggcaattt tcttcagtgg tatggcattt tctcaaataa aactctgcaa gagttatcaa 120
tagatggttt attaaatcga tatattctca tggcttttca gaattcagaa tatggagatg 180
acagcatcaa aaaagcccaa aatgtaatca attgtttccc caaacaatgg ttcatgaatc 240
tgaaaggaga aaggactatt tctcagttag aaaacttttg ccgatacctt gtacacttag 300
cggatacaat ttatagaaat agtatcgggt gttctgatgt ggaaaaaaga aatgcaaggg 360
aaaacataaa acagatagta aaactccttg caagtgttcg agctttggat catgctatgt 420
ctgttgcaag tgaccacaat gtgaaagaat ttaagtcttt gatcgaagga aaatagatct 480
atgagactga aaaacctatt tgcttgatta ccattcccaa tgtgtaaatt ttgtatatat 540
gtaaagtttc ttaaactgta aaatagcaat tcttgtttta atacaaaaaa cattatattc 600
aaactgtgtc cttaaatgat atttcttcag aatgaaagga attcagatta tttctaggag 660
tctatgaatg ttttctttac tgcctgtcat actttgttta ctgaagtatt ttgtatggtc 720
atttaaatta accctctcag ttaattgtcc cctgtaaacg atgtgtgcag tgtaaattgt 780
gtaattatgc atatatatat ttataccacc atggacttac tttcacactg ggaaatttgt 840
ggtgtttttc cacagaggaa gccttttaac agcagaaaat tatccggtag caaattgtct 900
tagggaaatt actacactgt tgtaacaaaa gggctggcaa gtgactaaat gttcattacc 960
ttttggttag cgttgctatc actgtagctg tgctgcctta acattgagag ttgagttgtg 1020
gacttagtca ttgagtgaat gttgaacttt cttccttgaa tcagcgttag agtcaaatgt 1080
gggtttaatg tgaatgctaa atgaatatgc aacatagttt caaatggaac atagaaattt 1140
cacttttgaa aataataaac ccacaaccta aaaaaaaaaa attgcatggt tttgggctga 1200
ccgtcctgtc tcattcatga ttttactccc tgcccagctt gcagatacat gcatttgaat 1260
tttagtagtc tgtcatattt aaaatcccac atatgtgagt taaatatgca ataaagaata 1320
cgccttgctg attagccaca cccaggcaat ttaatatcca tggtcttcta taatgtttat 1380
accacattgt aaatattcag agaacttgtt ttaaagttta tgctttctga ttctgtgatt 1440
ttcaaaagag gaattttttt ggaaattaag gactatcgtt tgataattat atatgtaatt 1500
aagatttagc caagcattaa attaattttt aaatataaaa aaaaaaaaaa aaaaaa 1556

<210> 169
<211> 2649
<212> PRT
<213> Homo sapiens

<220>
<223> Pemphigoid antigen

<400> 169
Met His Ser Ser Ser Tyr Ser Tyr Arg Ser Ser Asp Ser Val Phe Ser
1               5                   10                  15

Asn Thr Thr Ser Thr Arg Thr Ser Leu Asp Ser Asn Glu Asn Leu Leu
            20                  25                  30

Leu Val His Cys Gly Pro Thr Leu Ile Asn Ser Cys Ile Ser Phe Gly
        35                  40                  45

Ser Glu Ser Phe Asp Gly His Arg Leu Glu Met Leu Gln Gln Ile Ala
    50                  55                  60

Asn Arg Val Gln Arg Asp Ser Val Ile Cys Glu Asp Lys Leu Ile Leu
65                  70                  75                  80

Ala Gly Asn Ala Leu Gln Ser Asp Ser Lys Arg Leu Glu Ser Gly Val
                85                  90                  95

Gln Phe Gln Asn Glu Ala Glu Ile Ala Gly Tyr Ile Leu Glu Cys Glu

            100                 105                 110

Asn Leu Leu Arg Gln His Val Ile Asp Val Gln Ile Leu Ile Asp Gly
        115                 120                 125

Lys Tyr Tyr Gln Ala Asp Gln Leu Val Gln Arg Val Ala Lys Leu Arg
    130                 135                 140

Asp Glu Ile Met Ala Leu Arg Asn Glu Cys Ser Ser Val Tyr Ser Lys
145                 150                 155                 160

Gly Arg Ile Leu Thr Thr Glu Gln Thr Lys Leu Met Ile Ser Gly Ile
                165                 170                 175

Thr Gln Ser Leu Asn Ser Gly Phe Ala Gln Thr Leu His Pro Ser Leu
            180                 185                 190

Thr Ser Gly Leu Thr Gln Ser Leu Thr Pro Ser Leu Thr Ser Ser Ser
        195                 200                 205

Met Thr Ser Gly Leu Ser Ser Gly Met Thr Ser Arg Leu Thr Pro Ser
    210                 215                 220

Val Thr Pro Ala Tyr Thr Pro Gly Phe Pro Ser Gly Leu Val Pro Asn
225                 230                 235                 240

Phe Ser Ser Gly Val Glu Pro Asn Ser Leu Gln Thr Leu Lys Leu Met
                245                 250                 255

Gln Ile Arg Lys Pro Leu Leu Lys Ser Ser Leu Leu Asp Gln Asn Leu
            260                 265                 270

Thr Glu Glu Glu Ile Asn Met Lys Phe Val Gln Asp Leu Leu Asn Trp
        275                 280                 285

Val Asp Glu Met Gln Val Gln Leu Asp Arg Thr Glu Trp Gly Ser Asp
    290                 295                 300

Leu Pro Ser Val Glu Ser His Leu Glu Asn His Lys Asn Val His Arg
305                 310                 315                 320

Ala Ile Glu Glu Phe Glu Ser Ser Leu Lys Glu Ala Lys Ile Ser Glu
                325                 330                 335

Ile Gln Met Thr Ala Pro Leu Lys Leu Thr Tyr Ala Glu Lys Leu His
            340                 345                 350

Arg Leu Glu Ser Gln Tyr Ala Lys Leu Leu Asn Thr Ser Arg Asn Gln
        355                 360                 365

Glu Arg His Leu Asp Thr Leu His Asn Phe Val Ser Arg Ala Thr Asn
    370                 375                 380

Glu Leu Ile Trp Leu Asn Glu Lys Glu Glu Glu Glu Val Ala Tyr Asp
385                 390                 395                 400

Trp Ser Glu Arg Asn Thr Asn Ile Ala Arg Lys Lys Asp Tyr His Ala
                405                 410                 415

Glu Leu Met Arg Glu Leu Asp Gln Lys Glu Glu Asn Ile Lys Ser Val
            420                 425                 430

Gln Glu Ile Ala Glu Gln Leu Leu Leu Glu Asn His Pro Ala Arg Leu
        435                 440                 445

Thr Ile Glu Ala Tyr Arg Ala Ala Met Gln Thr Gln Trp Ser Trp Ile
    450                 455                 460

Leu Gln Leu Cys Gln Cys Val Glu Gln His Ile Lys Glu Asn Thr Ala
465                 470                 475                 480

Tyr Phe Glu Phe Phe Asn Asp Ala Lys Glu Ala Thr Asp Tyr Leu Arg
                485                 490                 495

Asn Leu Lys Asp Ala Ile Gln Arg Lys Tyr Ser Cys Asp Arg Ser Ser
            500                 505                 510

Ser Ile His Lys Leu Glu Asp Leu Val Gln Glu Ser Met Glu Glu Lys
        515                 520                 525

Glu Glu Leu Leu Gln Tyr Lys Ser Thr Ile Ala Asn Leu Met Gly Lys
    530                 535                 540

Ala Lys Thr Ile Ile Gln Leu Lys Pro Arg Asn Ser Asp Cys Pro Leu
545                 550                 555                 560

Lys Thr Ser Ile Pro Ile Lys Ala Ile Cys Asp Tyr Arg Gln Ile Glu
                565                 570                 575

Ile Thr Ile Tyr Lys Asp Asp Glu Cys Val Leu Ala Asn Asn Ser His
            580                 585                 590

Arg Ala Lys Trp Lys Val Ile Ser Pro Thr Gly Asn Glu Ala Met Val
        595                 600                 605

Pro Ser Val Cys Phe Thr Val Pro Pro Pro Asn Lys Glu Ala Val Asp
    610                 615                 620

Leu Ala Asn Arg Ile Glu Gln Gln Tyr Gln Asn Val Leu Thr Leu Trp
625                 630                 635                 640

His Glu Ser His Ile Asn Met Lys Ser Val Val Ser Trp His Tyr Leu
                645                 650                 655

Ile Asn Glu Ile Asp Arg Ile Arg Ala Ser Asn Val Ala Ser Ile Lys
            660                 665                 670

Thr Met Leu Pro Gly Glu His Gln Gln Val Leu Ser Asn Leu Gln Ser
        675                 680                 685

Arg Phe Glu Asp Phe Leu Glu Asp Ser Gln Glu Ser Gln Val Phe Ser
    690                 695                 700

Gly Ser Asp Ile Thr Gln Leu Glu Lys Glu Val Asn Val Cys Lys Gln
705                 710                 715                 720

Tyr Tyr Gln Glu Leu Leu Lys Ser Ala Glu Arg Glu Glu Gln Glu Glu
                725                 730                 735

Ser Val Tyr Asn Leu Tyr Ile Ser Glu Val Arg Asn Ile Arg Leu Arg
            740                 745                 750

Leu Glu Asn Cys Glu Asp Arg Leu Ile Arg Gln Ile Arg Thr Pro Leu
        755                 760                 765

Glu Arg Asp Asp Leu His Glu Ser Val Phe Arg Ile Thr Glu Gln Glu
    770                 775                 780

Lys Leu Lys Lys Glu Leu Glu Arg Leu Lys Asp Asp Leu Gly Thr Ile
785                 790                 795                 800

Thr Asn Lys Cys Glu Glu Phe Phe Ser Gln Ala Ala Ala Ser Ser Ser
                805                 810                 815

Val Pro Thr Leu Arg Ser Glu Leu Asn Val Val Leu Gln Asn Met Asn
            820                 825                 830

Gln Val Tyr Ser Met Ser Ser Thr Tyr Ile Asp Lys Leu Lys Thr Val
        835                 840                 845

Asn Leu Val Leu Lys Asn Thr Gln Ala Ala Glu Ala Leu Val Lys Leu
    850                 855                 860

Tyr Glu Thr Lys Leu Cys Glu Glu Glu Ala Val Ile Ala Asp Lys Asn
865                 870                 875                 880

Asn Ile Glu Asn Leu Ile Ser Thr Leu Lys Gln Trp Arg Ser Glu Val
                885                 890                 895

Asp Glu Lys Arg Gln Val Phe His Ala Leu Glu Asp Glu Leu Gln Lys
            900                 905                 910

Ala Lys Ala Ile Ser Asp Glu Met Phe Lys Thr Tyr Lys Glu Arg Asp
        915                 920                 925

Leu Asp Phe Asp Trp His Lys Glu Lys Ala Asp Gln Leu Val Glu Arg
    930                 935                 940

Trp Gln Asn Val His Val Gln Ile Asp Asn Arg Leu Arg Asp Leu Glu
945                 950                 955                 960

Gly Ile Gly Lys Ser Leu Lys Tyr Tyr Arg Asp Thr Tyr His Pro Leu
                965                 970                 975

Asp Asp Trp Ile Gln Gln Val Glu Thr Thr Gln Arg Lys Ile Gln Glu
            980                 985                 990

Asn Gln Pro Glu Asn Ser Lys Thr Leu Ala Thr Gln Leu Asn Gln Gln
        995                 1000                1005

Lys Met Leu Val Ser Glu Ile Glu Met Lys Gln Ser Lys Met Asp Glu
    1010                1015                1020

Cys Gln Lys Tyr Ala Glu Gln Tyr Ser Ala Thr Val Lys Asp Tyr Glu
1025                1030                1035                1040

Leu Gln Thr Met Thr Tyr Arg Ala Met Val Asp Ser Gln Gln Lys Ser
                1045                1050                1055

Pro Val Lys Arg Arg Arg Met Gln Ser Ser Ala Asp Leu Ile Ile Gln
            1060                1065                1070

Glu Phe Met Asp Leu Arg Thr Arg Tyr Thr Ala Leu Val Thr Leu Met

1075                1080                1085

Thr Gln Tyr Ile Lys Phe Ala Gly Asp Ser Leu Lys Arg Leu Glu Glu
    1090                1095                1100

Glu Glu Ile Lys Arg Cys Lys Glu Thr Ser Glu His Gly Ala Tyr Ser
1105                1110                1115                1120

Asp Leu Leu Gln Arg Gln Lys Ala Thr Val Leu Glu Asn Ser Lys Leu
                1125                1130                1135

Thr Gly Lys Ile Ser Glu Leu Glu Arg Met Val Ala Glu Leu Lys Lys
            1140                1145                1150

Gln Lys Ser Arg Val Glu Glu Glu Leu Pro Lys Val Arg Glu Ala Ala
        1155                1160                1165

Glu Asn Glu Leu Arg Lys Gln Gln Arg Asn Val Glu Asp Ile Ser Leu
    1170                1175                1180

Gln Lys Ile Arg Ala Glu Ser Glu Ala Lys Gln Tyr Arg Arg Glu Leu
1185                1190                1195                1200

Glu Thr Ile Val Arg Glu Lys Glu Ala Ala Glu Arg Glu Leu Glu Arg
                1205                1210                1215

Val Arg Gln Leu Thr Ile Glu Ala Glu Ala Lys Arg Ala Ala Val Glu
            1220                1225                1230

Glu Asn Leu Leu Asn Phe Arg Asn Gln Leu Glu Glu Asn Thr Phe Thr
        1235                1240                1245

Arg Arg Thr Leu Glu Asp His Leu Lys Arg Lys Asp Leu Ser Leu Asn
    1250                1255                1260

Asp Leu Glu Gln Gln Lys Asn Lys Leu Met Glu Glu Leu Arg Arg Lys
1265                1270                1275                1280

Arg Asp Asn Glu Glu Glu Leu Leu Lys Leu Ile Lys Gln Met Glu Lys
                1285                1290                1295

Asp Leu Ala Phe Gln Lys Gln Val Ala Glu Lys Gln Leu Lys Glu Lys
            1300                1305                1310

Gln Lys Ile Glu Leu Glu Ala Arg Arg Lys Ile Thr Glu Ile Gln Tyr
        1315                1320                1325

Thr Cys Arg Glu Asn Ala Leu Pro Val Cys Pro Ile Thr Gln Ala Thr
    1330                1335                1340

Ser Cys Arg Ala Val Thr Gly Leu Gln Gln Glu His Asp Lys Gln Lys
1345                1350                1355                1360

Ala Glu Glu Leu Lys Gln Gln Val Asp Glu Leu Thr Ala Ala Asn Arg
                1365                1370                1375

Lys Ala Glu Gln Asp Met Arg Glu Leu Thr Tyr Glu Leu Asn Ala Leu
            1380                1385                1390

Gln Leu Glu Lys Thr Ser Ser Glu Glu Lys Ala Arg Leu Leu Lys Asp
        1395                1400                1405

Lys Leu Asp Glu Thr Asn Asn Thr Leu Arg Cys Leu Lys Leu Glu Leu
1410                1415                1420

Glu Arg Lys Asp Gln Ala Glu Lys Gly Tyr Ser Gln Gln Leu Arg Glu
1425                1430                1435                1440

Leu Gly Arg Gln Leu Asn Gln Thr Thr Gly Lys Ala Glu Glu Ala Met
                1445                1450                1455

Gln Glu Ala Ser Asp Leu Lys Lys Ile Lys Arg Asn Tyr Gln Leu Glu
            1460                1465                1470

Leu Glu Ser Leu Asn His Glu Lys Gly Lys Leu Gln Arg Glu Val Asp
        1475                1480                1485

Arg Ile Thr Arg Ala His Ala Val Ala Glu Lys Asn Ile Gln His Leu
    1490                1495                1500

Asn Ser Gln Ile His Ser Phe Arg Asp Glu Lys Glu Leu Glu Arg Leu
1505                1510                1515                1520

Gln Ile Cys Gln Arg Lys Ser Asp His Leu Lys Glu Gln Phe Glu Lys
                1525                1530                1535

Ser His Glu Gln Leu Leu Gln Asn Ile Lys Ala Glu Lys Glu Asn Asn
            1540                1545                1550

Asp Lys Ile Gln Arg Leu Asn Glu Glu Leu Glu Lys Ser Asn Glu Cys
        1555                1560                1565

Ala Glu Met Leu Lys Gln Lys Val Glu Glu Leu Thr Arg Gln Asn Asn
    1570                1575                1580

Glu Thr Lys Leu Met Met Gln Arg Ile Gln Ala Glu Ser Glu Asn Ile
1585                1590                1595                1600

Val Leu Glu Lys Gln Thr Ile Gln Gln Arg Cys Glu Ala Leu Lys Ile
                1605                1610                1615

Gln Ala Asp Gly Phe Lys Asp Gln Leu Arg Ser Thr Asn Glu His Leu
            1620                1625                1630

His Lys Gln Thr Lys Thr Glu Gln Asp Phe Gln Arg Lys Ile Lys Cys
        1635                1640                1645

Leu Glu Glu Asp Leu Ala Lys Ser Gln Asn Leu Val Ser Glu Phe Lys
    1650                1655                1660

Gln Lys Cys Asp Gln Gln Asn Ile Ile Ile Gln Asn Thr Lys Lys Glu
1665                1670                1675                1680

Val Arg Asn Leu Asn Ala Glu Leu Asn Ala Ser Lys Glu Glu Lys Arg
                1685                1690                1695

Arg Gly Glu Gln Lys Val Gln Leu Gln Gln Ala Gln Val Gln Glu Leu
            1700                1705                1710

Asn Asn Arg Leu Lys Lys Val Gln Asp Glu Leu His Leu Lys Thr Ile
        1715                1720                1725

Glu Glu Gln Met Thr His Arg Lys Met Val Leu Phe Gln Glu Glu Ser
    1730                1735                1740

Gly Lys Phe Lys Gln Ser Ala Glu Glu Phe Arg Lys Lys Met Glu Lys
1745                1750                1755                1760

Leu Met Glu Ser Lys Val Ile Thr Glu Asn Asp Ile Ser Gly Ile Arg
                1765                1770                1775

Leu Asp Phe Val Ser Leu Gln Gln Glu Asn Ser Arg Ala Gln Glu Asn
            1780                1785                1790

Ala Lys Leu Cys Glu Thr Asn Ile Lys Glu Leu Glu Arg Gln Leu Gln
        1795                1800                1805

Gln Tyr Arg Glu Gln Met Gln Gln Gly Gln His Met Glu Ala Asn His
    1810                1815                1820

Tyr Gln Lys Cys Gln Lys Leu Glu Asp Glu Leu Ile Ala Gln Lys Arg
1825                1830                1835                1840

Glu Val Glu Asn Leu Lys Gln Lys Met Asp Gln Gln Ile Lys Glu His
                1845                1850                1855

Glu His Gln Leu Val Leu Leu Gln Cys Glu Ile Gln Lys Lys Ser Thr
            1860                1865                1870

Ala Lys Asp Cys Thr Phe Lys Pro Asp Phe Glu Met Thr Val Lys Glu
        1875                1880                1885

Cys Gln His Ser Gly Glu Leu Ser Ser Arg Asn Thr Gly His Leu His
    1890                1895                1900

Pro Thr Pro Arg Ser Pro Leu Leu Arg Trp Thr Gln Glu Pro Gln Pro
1905                1910                1915                1920

Leu Glu Glu Lys Trp Gln His Arg Val Val Glu Gln Ile Pro Lys Glu
                1925                1930                1935

Val Gln Phe Gln Pro Pro Gly Ala Pro Leu Glu Lys Glu Lys Ser Gln
            1940                1945                1950

Gln Cys Tyr Ser Glu Tyr Phe Ser Gln Thr Ser Thr Glu Leu Gln Ile
        1955                1960                1965

Thr Phe Asp Glu Thr Asn Pro Ile Thr Arg Leu Ser Glu Ile Glu Lys
    1970                1975                1980

Ile Arg Asp Gln Ala Leu Asn Asn Ser Arg Pro Pro Val Arg Tyr Gln
1985                1990                1995                2000

Asp Asn Ala Cys Glu Met Glu Leu Val Lys Val Leu Thr Pro Leu Glu
                2005                2010                2015

Ile Ala Lys Asn Lys Gln Tyr Asp Met His Thr Glu Val Thr Thr Leu
            2020                2025                2030

Lys Gln Glu Lys Asn Pro Val Pro Ser Ala Glu Glu Trp Met Leu Glu
        2035                2040                2045

Gly Cys Arg Ala Ser Gly Gly Leu Lys Lys Gly Asp Phe Leu Lys Lys

2050                2055                2060

Gly Leu Glu Pro Glu Thr Phe Gln Asn Phe Asp Gly Asp His Ala Cys
2065                2070                2075                2080

Ser Val Arg Asp Asp Glu Phe Lys Phe Gln Gly Leu Arg His Thr Val
                2085                2090                2095

Thr Ala Arg Gln Leu Val Glu Ala Lys Leu Leu Asp Met Arg Thr Ile
            2100                2105                2110

Glu Gln Leu Arg Leu Gly Leu Lys Thr Val Glu Glu Val Gln Lys Thr
        2115                2120                2125

Leu Asn Lys Phe Leu Thr Lys Ala Thr Ser Ile Ala Gly Leu Tyr Leu
    2130                2135                2140

Glu Ser Thr Lys Glu Lys Ile Ser Phe Ala Ser Ala Ala Glu Arg Ile
2145                2150                2155                2160

Ile Ile Asp Lys Met Val Ala Leu Ala Phe Leu Glu Ala Gln Ala Ala
                2165                2170                2175

Thr Gly Phe Ile Ile Asp Pro Ile Ser Gly Gln Thr Tyr Ser Val Glu
            2180                2185                2190

Asp Ala Val Leu Lys Gly Val Val Asp Pro Glu Phe Arg Ile Arg Leu
        2195                2200                2205

Leu Glu Ala Glu Lys Ala Ala Val Gly Tyr Ser Tyr Ser Ser Lys Thr
    2210                2215                2220

Leu Ser Val Phe Gln Ala Met Glu Asn Arg Met Leu Asp Arg Gln Lys
2225                2230                2235                2240

Gly Lys His Ile Leu Glu Ala Gln Ile Ala Ser Gly Gly Val Ile Asp
                2245                2250                2255

Pro Val Arg Gly Ile Arg Val Pro Pro Glu Ile Ala Leu Gln Gln Gly
            2260                2265                2270

Leu Leu Asn Asn Ala Ile Leu Gln Phe Leu His Glu Pro Ser Ser Asn
        2275                2280                2285

Thr Arg Val Phe Pro Asn Pro Asn Asn Lys Gln Ala Leu Tyr Tyr Ser
    2290                2295                2300

Glu Leu Leu Arg Met Cys Val Phe Asp Val Glu Ser Gln Cys Phe Leu
2305                2310                2315                2320

Phe Pro Phe Gly Glu Arg Asn Ile Ser Asn Leu Asn Val Lys Lys Thr
                2325                2330                2335

His Arg Ile Ser Val Val Asp Thr Lys Thr Gly Ser Glu Leu Thr Val
            2340                2345                2350

Tyr Glu Ala Phe Gln Arg Asn Leu Ile Glu Lys Ser Ile Tyr Leu Glu
        2355                2360                2365

Leu Ser Gly Gln Gln Tyr Gln Trp Lys Glu Ala Met Phe Phe Glu Ser
    2370                2375                2380

Tyr Gly His Ser Ser His Met Leu Thr Asp Thr Lys Thr Gly Leu His
2385                2390                2395                2400

Phe Asn Ile Asn Glu Ala Ile Glu Gln Gly Thr Ile Asp Lys Ala Leu
                2405                2410                2415

Val Lys Lys Tyr Gln Glu Gly Leu Ile Thr Leu Thr Glu Leu Ala Asp
            2420                2425                2430

Ser Leu Leu Ser Arg Leu Val Pro Lys Lys Asp Leu His Ser Pro Val
        2435                2440                2445

Ala Gly Tyr Trp Leu Thr Ala Ser Gly Glu Arg Ile Ser Val Leu Lys
    2450                2455                2460

Ala Ser Arg Arg Asn Leu Val Asp Arg Ile Thr Ala Leu Arg Cys Leu
2465                2470                2475                2480

Glu Ala Gln Val Ser Thr Gly Gly Ile Ile Asp Pro Leu Thr Gly Lys
                2485                2490                2495

Lys Tyr Arg Val Ala Glu Ala Leu His Arg Gly Leu Val Asp Glu Gly
            2500                2505                2510

Phe Ala Gln Gln Leu Arg Gln Cys Glu Leu Val Ile Thr Gly Ile Gly
        2515                2520                2525

His Pro Ile Thr Asn Lys Met Met Ser Val Val Glu Ala Val Asn Ala
    2530                2535                2540

Asn Ile Ile Asn Lys Glu Met Gly Ile Arg Cys Leu Glu Phe Gln Tyr
2545                2550                2555                2560

Leu Thr Gly Gly Leu Ile Glu Pro Gln Val His Ser Arg Leu Ser Ile
                2565                2570                2575

Glu Glu Ala Leu Gln Val Gly Ile Ile Asp Val Leu Ile Ala Thr Lys
            2580                2585                2590

Leu Lys Asp Gln Lys Ser Tyr Val Arg Asn Ile Ile Cys Pro Gln Thr
        2595                2600                2605

Lys Arg Lys Leu Thr Tyr Lys Glu Ala Leu Glu Lys Ala Asp Phe Asp
    2610                2615                2620

Phe His Thr Gly Leu Lys Leu Leu Glu Val Ser Glu Pro Leu Met Thr
2625                2630                2635                2640

Gly Ile Ser Ser Leu Tyr Tyr Ser Ser
                2645

<210> 170
<211> 8684
<212> DNA
<213> Homo sapiens

<220>
<223> Pemphigoid antigen

<400> 170
gagctgccac ttttcaccgt tagaagtaga gctttttcca gacctcctac cttttagtct 60
actttgaaag gtgaaagaaa gaacatcgtt tcaggaataa aaatgcacag tagtagttat 120
agttaccgta gcagtgattc tgtgtttagt aacactacca gcactcgaac cagtcttgat 180
tcaaatgaaa atcttctctt ggttcattgt ggtccaacac tgatcaactc ttgcattagc 240
ttcggtagtg aatcctttga tggacacagg ttagaaatgt tgcaacagat tgccaacaga 300
gttcagaggg acagtgtcat ctgtgaagac aaactgattc ttgctggaaa tgctcttcag 360
tctgattcta aaagattaga atcaggagtg cagtttcaga atgaagcaga aattgctggg 420
tatatacttg aatgtgagaa ccttttacgc cagcatgtaa ttgatgtaca gattcttatt 480
gatggaaaat actaccaggc agatcaattg gtacagaggg ttgcaaaact gcgtgacgaa 540
attatggcct taaggaacga atgttcttct gtgtacagca aaggacgcat actgacaaca 600
gaacagacaa agctcatgat atcaggaatc actcaaagtt taaactcagg atttgcacag 660
accttacacc ctagtctgac ctcagggctg acccagagtt taacaccttc cctaacctct 720
tctagtatga cttctggcct gtcatcaggg atgacttccc gcctgactcc atctgtcact 780
ccagcttata cacctggttt cccatcagga ttagttccaa atttcagttc aggagtagag 840
ccaaattcat tgcaaacttt gaagttgatg cagatccgaa aaccccttct aaagtcttct 900
ttgctggatc aaaatttaac agaagaagaa atcaatatga aatttgttca ggatcttttg 960
aattgggttg atgagatgca ggtacaactg gaccgcactg agtggggctc agatttgcca 1020
agtgttgaaa gccatttaga aaatcataaa aatgttcata gagctattga agaatttgaa 1080
tctagtctca aagaagctaa aatcagtgag attcaaatga cagcacctct taaactgact 1140
tatgcagaaa agttgcacag attagagagt cagtatgcaa aactcttgaa tacatccagg 1200
aatcaagaac ggcaccttga tacactccat aattttgtaa gtcgtgcgac taatgaactt 1260
atttggttga atgaaaaaga agaggaggaa gttgcttatg actggagtga gagaaacacc 1320
aacatagcta ggaaaaaaga ttatcatgct gaattaatga gagaacttga tcaaaaggaa 1380
gaaaatatta aatcagttca ggagatagca gagcagctac ttctagaaaa tcatccagcc 1440
cggttaacta ttgaggccta cagagcggca atgcagacgc agtggagctg gatcttacag 1500
ctctgccagt gtgtggagca gcacataaag gagaacacag cgtatttcga gtttttcaat 1560
gatgccaaag aagctactga ttacttaagg aatctaaaag atgccattca gcggaagtac 1620
agctgtgata gatcaagcag cattcacaag ctagaagacc ttgttcagga atcaatggaa 1680
gagaaagaag aacttctgca gtacaaaagc actatagcaa acctaatggg aaaagcaaaa 1740
acaataattc aactgaagcc aaggaattct gactgtccac tcaaaacttc tattccgatc 1800
aaagctatct gtgactacag acaaattgag ataaccattt acaaagacga tgaatgtgtt 1860
ttggcgaata actctcatcg tgctaaatgg aaggtcatta gtcctactgg gaatgaggct 1920
atggtcccat ctgtgtgctt caccgttcct ccaccaaaca aagaagcggt ggaccttgcc 1980
aacagaattg agcaacagta tcagaatgtc ctgactcttt ggcatgagtc tcacataaac 2040
atgaagagtg tagtatcctg gcattatctc atcaatgaaa ttgatagaat tcgagctagc 2100
aatgtggctt caataaagac aatgctacct ggtgaacatc agcaagttct aagtaatcta 2160
caatctcgtt ttgaagattt tctggaagat agccaggaat cccaagtctt ttcaggctca 2220
gatataacac aactggaaaa ggaggttaat gtatgtaagc agtattatca agaacttctt 2280
aaatctgcag aaagagagga gcaagaggaa tcagtttata atctctacat ctctgaagtt 2340
cgaaacatta gacttcggtt agagaactgt gaagatcggc tgattagaca gattcgaact 2400
cccctggaaa gagatgattt gcatgaaagt gtgttcagaa tcacagaaca ggagaaacta 2460
aagaaagagc tggaacgact taaagatgat ttgggaacaa tcacaaataa gtgtgaggag 2520
tttttcagtc aagcagcagc ctcttcatca gtccctaccc tacgatcaga gcttaatgtg 2580
gtccttcaga acatgaacca agtctattct atgtcttcca cttacataga taagttgaaa 2640
actgttaact tggtgttaaa aaacactcaa gctgcagaag ccctcgtaaa actctatgaa 2700
actaaactgt gtgaagaaga agcagttata gctgacaaga ataatattga gaatctaata 2760
agtactttaa agcaatggag atctgaagta gatgaaaaga gacaggtatt ccatgcctta 2820
gaggatgagt tgcagaaagc taaagccatc agtgatgaaa tgtttaaaac gtataaagaa 2880
cgggaccttg attttgactg gcacaaagaa aaagcagatc aattagttga aaggtggcaa 2940
aatgttcatg tgcagattga caacaggtta cgggacttag agggcattgg caaatcactg 3000
aagtactaca gagacactta ccatccttta gatgattgga tccagcaggt tgaaactact 3060
cagagaaaga ttcaggaaaa tcagcctgaa aatagtaaaa ccctagccac acagttgaat 3120
caacagaaga tgctggtgtc cgaaatagaa atgaaacaga gcaaaatgga cgagtgtcaa 3180
aaatatgcag aacagtactc agctacagtg aaggactatg aattacaaac aatgacctac 3240
cgggccatgg tagattcaca acaaaaatct ccagtgaaac gccgaagaat gcagagttca 3300
gcagatctca ttattcaaga gttcatggac ctaaggactc gatatactgc cctggtcact 3360
ctcatgacac aatatattaa atttgctggt gattcattga agaggctgga agaggaggag 3420
attaaaaggt gtaaggagac ttctgaacat ggggcatatt cagatctgct tcagcgtcag 3480
aaggcaacag tgcttgagaa tagcaaactt acaggaaaga taagtgagtt ggaaagaatg 3540
gtagctgaac taaagaaaca aaagtcccga gtagaggaag aacttccgaa ggtcagggag 3600

gctgcagaaa atgaattgag aaagcagcag agaaatgtag aagatatctc tctgcagaag 3660
ataagggctg aaagtgaagc caagcagtac cgcagggaac ttgaaaccat tgtgagagag 3720
aaggaagccg ctgaaagaga actggagcgg gtgaggcagc tcaccataga ggccgaggct 3780
aaaagagctg ccgtggaaga gaacctcctg aattttcgca atcagttgga ggaaaacacc 3840
tttaccagac gaacactgga agatcatctt aaaagaaaag atttaagtct caatgatttg 3900
gagcaacaaa aaaataaatt aatggaagaa ttaagaagaa agagagacaa tgaggaagaa 3960
ctcttgaagc tgataaagca gatggaaaaa gaccttgcat ttcagaaaca ggtagcagag 4020
aaacagttga aagaaaagca gaaaattgaa ttggaagcaa gaagaaaaat aactgaaatt 4080
cagtatacat gtagagaaaa tgcattgcca gtgtgtccga tcacacaggc tacatcatgc 4140
agggcagtaa cgggtctcca gcaagaacat gacaagcaga aagcagaaga actcaaacag 4200
caggtagatg aactaacagc tgccaataga aaggctgaac aagacatgag agagctgaca 4260
tatgaactta atgccctcca gcttgaaaaa acgtcatctg aggaaaaggc tcgtttgcta 4320
aaagataaac tagatgaaac aaataataca ctcagatgcc ttaagttgga gctggaaagg 4380
aaggatcagg cggagaaagg gtattctcaa caactcagag agcttggtag gcaattgaat 4440
caaaccacag gtaaagctga agaagccatg caagaagcta gtgatctcaa gaaaataaag 4500
cgcaattatc agttagaatt agaatctctt aatcatgaaa aagggaaact acaaagagaa 4560
gtagacagaa tcacaagggc acatgctgta gctgagaaga atattcagca tttaaattca 4620
caaattcatt cttttcgaga tgagaaagaa ttagaaagac tacaaatctg ccagagaaaa 4680
tcagatcatc taaaagaaca atttgagaaa agccatgagc agttgcttca aaatatcaaa 4740
gctgaaaaag aaaataatga taaaatccaa aggctcaatg aagaattgga gaaaagtaat 4800
gagtgtgcag agatgctaaa acaaaaagta gaggagctta ctaggcagaa taatgaaacc 4860
aaattaatga tgcagagaat tcaggcagaa tcagagaata tagttttaga gaaacaaact 4920
atccagcaaa gatgtgaagc actgaaaatt caggcagatg gttttaaaga tcagctacgc 4980
agcacaaatg aacacttgca taaacagaca aaaacagagc aggattttca aagaaaaatt 5040
aaatgcctag aagaagacct ggcgaaaagt caaaatttgg taagtgaatt taagcaaaag 5100
tgtgaccaac agaacattat catccagaat accaagaaag aagttagaaa tctgaatgcg 5160
gaactgaatg cttccaaaga agagaagcga cgcggggagc agaaagttca gctacaacaa 5220
gctcaggtgc aagagttaaa taacaggttg aaaaaagtac aagacgaatt acacttaaag 5280
accatagagg agcagatgac ccacagaaag atggttctgt ttcaggaaga atctggtaaa 5340
ttcaaacaat cagcagagga gtttcggaag aagatggaaa aattaatgga gtccaaagtc 5400
atcactgaaa atgatatttc aggcattagg cttgactttg tgtctcttca acaagaaaac 5460
tctagagccc aagaaaatgc taagctttgt gaaacaaaca ttaaagaact tgaaagacag 5520
cttcaacagt atcgtgaaca aatgcagcaa gggcagcaca tggaagcaaa tcattaccaa 5580
aaatgtcaga aacttgagga tgagctgata gcccagaagc gtgaggttga aaacctgaag 5640
caaaaaatgg accaacagat caaagagcat gaacatcaat tagttttgct ccagtgtgaa 5700
attcaaaaaa agagcacagc caaagactgt accttcaaac cagattttga gatgacagtg 5760
aaggagtgcc agcactctgg agagctgtcc tctagaaaca ctggacacct tcacccaaca 5820
cccagatccc ctctgttgag atggactcaa gaaccacagc cattggaaga gaagtggcag 5880
catcgggttg ttgaacagat acccaaagaa gtccaattcc agccaccagg ggctccactc 5940
gagaaagaga aaagccagca gtgttactct gagtactttt ctcagacaag caccgagtta 6000
cagataactt ttgatgagac aaaccccatt acaagactgt ctgaaattga gaagataaga 6060
gaccaagccc tgaacaattc tagaccacct gttaggtatc aagataacgc atgtgaaatg 6120
gaactggtga aggttttgac acccttagag atagctaaga acaagcagta tgatatgcat 6180
acagaagtca caacattaaa acaagaaaag aacccagttc ccagtgctga agaatggatg 6240
cttgaagggt gcagagcatc tggtggactc aagaaagggg atttccttaa gaagggctta 6300
gaaccagaga ccttccagaa ctttgatggt gatcatgcat gttcagtcag ggatgatgaa 6360
tttaaattcc aagggcttag gcacactgtg actgccaggc agttggtgga agctaagctt 6420
ctggacatga gaacaattga gcagctgcga ctcggtctta agactgttga agaagttcag 6480
aaaactctta acaagtttct gacgaaagcc acctcaattg cagggcttta cctagaatct 6540
acaaaagaaa agatttcatt tgcctcagcg gccgagagaa tcataataga caaaatggtg 6600
gctttggcat ttttagaagc tcaggctgca acaggtttta taattgatcc catttcaggt 6660
cagacatatt ctgttgaaga tgcagttctt aaaggagttg ttgaccccga attcagaatt 6720
aggcttcttg aggcagagaa ggcagctgtg ggatattctt attcttctaa gacattgtca 6780
gtgtttcaag ctatggaaaa tagaatgctt gacagacaaa aaggtaaaca tatcttggaa 6840
gcccagattg ccagtggggg tgtcattgac cctgtgagag gcattcgtgt tcctccagaa 6900
attgctctgc agcaggggtt gttgaataat gccatcttac agtttttaca tgagccatcc 6960
agcaacacaa gagttttccc taatcccaat aacaagcaag ctctgtatta ctcagaatta 7020
ctgcgaatgt gtgtatttga tgtagagtcc caatgctttc tgtttccatt tggggagagg 7080
aacatttcca atctcaatgt caagaaaaca catagaattt ctgtagtaga tactaaaaca 7140
ggatcagaat tgaccgtgta tgaggctttc cagagaaacc tgattgagaa aagtatatat 7200
cttgaacttt cagggcagca atatcagtgg aaggaagcta tgttttttga atcctatggg 7260

cattcttctc atatgctgac tgatactaaa acaggattac acttcaatat taatgaggct 7320
atagagcagg gaacaattga caaagccttg gtcaaaaagt atcaggaagg cctcatcaca 7380
cttacagaac ttgctgattc tttgctgagc cggttagtcc ccaagaaaga tttgcacagt 7440
cctgttgcag ggtattggct gactgctagt ggggaaagga tctctgtact aaaagcctcc 7500
cgtagaaatt tggttgatcg gattactgcc ctccgatgcc ttgaagccca agtcagtaca 7560
gggggcataa ttgatcctct tactggcaaa aagtaccggg tggccgaagc tttgcataga 7620
ggcctggttg atgaggggtt tgcccagcag ctgcgacagt gtgaattagt aatcacaggg 7680
attggccatc ccatcactaa caaaatgatg tcagtggtgg aagctgtgaa tgcaaatatt 7740
ataaataagg aaatgggaat ccgatgtttg gaatttcagt acttgacagg agggttgata 7800
gagccacagg ttcactctcg gttatcaata gaagaggctc tccaagtagg tattatagat 7860
gtcctcattg ccacaaaact caaagatcaa aagtcatatg tcagaaatat aatatgccct 7920
cagacaaaaa gaaagttgac atataaagaa gccttagaaa aagctgattt tgatttccac 7980
acaggactta aactgttaga agtatctgag cccctgatga caggaatttc tagcctctac 8040
tattcttcct aatgggacat gtttaaataa ctgtgcaagg ggtgatgcag gctggttcat 8100
gccacttttt cagagtatga tgatatcggc tacatatgca gtctgtgaat tatgtaacat 8160
actctatttc ttgagggctg caaattgcta agtgctcaaa atagagtaag ttttaaattg 8220
aaaattacat aagatttaat gcccttcaaa tggtttcatt tagccttgag aatggttttt 8280
tgaaacttgg ccacactaaa atgttttttt ttttttacgt agaatgtggg ataaacttga 8340
tgaactccaa gttcacagtg tcatttcttc agaactcccc ttcattgaat agtgatcatt 8400
tattaaatga taaattgcac tcgctgaaag agcacgtcat gaagcaccat ggaatcaaag 8460
agaaagatat aaattcgttc ccacagcctt caagctgcag tgttttagat tgcttcaaaa 8520
aatgaaaaag ttttgccttt ttcgatatag tgaccttctt tgcatattaa aatgtttacc 8580
acaatgtccc atttctagtt aagtcttcgc acttgaaagc taacattatg aatattatgt 8640
gttggaggag gggaaggatt ttcttcattc tgtgtatttt ccgg 8684

<210> 171
<211> 871
<212> PRT
<213> Rattus norvegicus

<220>
<223> Copg2

<400> 171
   Met Ile Lys Lys Phe Asp Lys Lys Asp Glu Glu Ser Gly Ser Gly Ser
   1               5                   10                  15
   Asn Pro Phe Gln His Leu Glu Lys Ser Ala Val Leu Gln Glu Ala Arg
               20                  25                  30
   Ile Phe Asn Glu Thr Pro Ile Asn Pro Arg Arg Cys Leu His Ile Leu
           35                  40                  45
   Thr Lys Ile Leu Tyr Leu Leu Asn Gln Gly Glu His Phe Gly Thr Met
       50                  55                  60
   Glu Ala Thr Glu Ala Phe Phe Ala Met Thr Arg Leu Phe Gln Ser Asn
   65                  70                  75                  80
   Asp Gln Thr Leu Arg Arg Met Cys Tyr Leu Thr Ile Lys Glu Met Ala
                   85                  90                  95
   Thr Ile Ser Glu Asp Val Ile Ile Val Thr Ser Ser Leu Thr Lys Asp
               100                 105                 110
   Met Thr Gly Lys Glu Asp Val Tyr Arg Gly Pro Ala Ile Arg Ala Leu
           115                 120                 125
   Cys Arg Ile Thr Asp Gly Thr Met Leu Gln Ala Val Glu Arg Tyr Met
       130                 135                 140
   Lys Gln Ala Ile Val Asp Lys Val Ser Ser Val Ala Ser Ser Ala Leu
   145                 150                 155                 160
   Val Ser Ser Leu His Met Met Lys Ile Ser Tyr Asp Val Val Lys Arg
                   165                 170                 175
   Trp Ile Asn Glu Ala Gln Glu Ala Ala Ser Ser Asp Asn Ile Met Val
               180                 185                 190
   Gln Tyr His Ala Leu Gly Val Leu Tyr His Leu Arg Lys Asn Asp Arg
           195                 200                 205
   Leu Ala Val Ser Lys Met Leu Asn Lys Phe Thr Lys Ser Gly Leu Lys
       210                 215                 220
   Ser Gln Phe Ala Tyr Cys Met Leu Ile Arg Ile Ala Ser Arg Leu Leu
   225                 230                 235                 240
   Lys Glu Ser Glu Asp Gly His Glu Ser Pro Leu Phe Asp Phe Ile Glu
                   245                 250                 255
   Ser Cys Leu Arg Asn Lys His Glu Met Val Ile Tyr Glu Ala Ala Ser
               260                 265                 270
   Ala Ile Ile His Leu Pro Asn Cys Thr Ala Arg Glu Leu Ala Pro Ala
           275                 280                 285
   Val Ser Val Leu Gln Leu Phe Cys Ser Ser Pro Lys Pro Ala Leu Arg
       290                 295                 300
   Tyr Ala Ala Val Arg Thr Leu Asn Lys Val Ala Met Lys His Pro Ser
   305                 310                 315                 320
   Ala Val Thr Ala Cys Asn Leu Asp Leu Glu Asn Leu Ile Thr Asp Ser
                   325                 330                 335
   Asn Arg Ser Ile Ala Thr Leu Ala Ile Thr Thr Leu Leu Lys Thr Gly
               340                 345                 350
   Ser Glu Ser Ser Val Asp Arg Leu Met Lys Gln Ile Ser Ser Phe Val
           355                 360                 365
   Ser Glu Ile Ser Asp Glu Phe Lys Val Val Val Val Gln Ala Ile Ser
       370                 375                 380
   Ala Leu Cys His Lys Tyr Pro Arg Lys His Ser Val Met Met Thr Phe
   385                 390                 395                 400
   Leu Ser Asn Met Leu Arg Asp Asp Gly Gly Phe Glu Tyr Lys Lys Ala
                   405                 410                 415
   Ile Val Asp Cys Ile Ile Ser Ile Val Glu Glu Asn Pro Glu Ser Lys
               420                 425                 430
   Glu Ala Gly Leu Ala His Leu Cys Glu Phe Ile Glu Asp Cys Glu His
           435                 440                 445
   Thr Val Leu Ala Thr Lys Ile Leu His Leu Leu Gly Lys Glu Gly Pro
       450                 455                 460
   Arg Thr Pro Val Pro Ser Lys Tyr Ile Arg Phe Ile Phe Asn Arg Val
   465                 470                 475                 480
   Val Leu Glu Asn Glu Ala Val Arg Ala Ala Ala Val Ser Ala Leu Ala
                   485                 490                 495
   Lys Phe Gly Ala Gln Asn Glu Ser Leu Leu Pro Ser Ile Leu Val Leu
               500                 505                 510
   Leu Gln Arg Cys Met Met Asp Thr Asp Asp Glu Val Arg Asp Arg Ala
           515                 520                 525
   Thr Phe Tyr Leu Asn Val Leu Gln Gln Arg Gln Met Ala Leu Asn Ala
       530                 535                 540
   Thr Tyr Ile Phe Asn Gly Leu Thr Val Ser Ile Pro Gly Met Glu Lys
   545                 550                 555                 560
   Ala Leu His Gln Tyr Thr Leu Glu Pro Ser Glu Lys Pro Phe Asp Met
                   565                 570                 575
   Lys Ser Ile Pro Leu Ala Met Ala Pro Val Phe Glu Gln Lys Ser Glu
               580                 585                 590
   Ile Thr Leu Val Thr Pro Lys Pro Glu Lys Leu Ala Pro Ser Arg Gln
           595                 600                 605
   Asp Ile Phe Gln Glu Gln Leu Ala Ala Ile Pro Glu Phe Met Asn Leu
       610                 615                 620
   Gly Pro Leu Phe Lys Ser Ser Glu Pro Val Gln Leu Thr Glu Ala Glu
   625                 630                 635                 640
   Thr Glu Tyr Phe Val Arg Cys Val Lys His Met Phe Thr Asp His Ile
                   645                 650                 655
   Val Phe Gln Phe Asp Cys Thr Asn Thr Leu Asn Asp Gln Leu Leu Glu
               660                 665                 670
   Lys Val Thr Val Gln Met Glu Pro Ser Asp Ser Tyr Glu Val Leu Cys
           675                 680                 685
   Cys Ile Pro Ala Pro Ser Leu Pro Tyr Asn Gln Pro Gly Ile Cys Tyr
       690                 695                 700
   Thr Leu Val Arg Leu Pro Asp Glu Asp Pro Thr Ala Val Ala Gly Thr
   705                 710                 715                 720
   Phe Ser Cys Thr Met Lys Phe Thr Val Arg Asp Cys Asp Pro Asn Thr
                   725                 730                 735
   Gly Val Pro Asp Glu Asp Gly Tyr Asp Asp Glu Tyr Val Leu Glu Asp
               740                 745                 750
   Leu Glu Val Thr Val Ser Asp His Ile Gln Lys Ile Leu Lys Pro Asn
           755                 760                 765
   Phe Ala Ala Ala Trp Glu Glu Val Gly Asp Ala Phe Glu Lys Glu Glu
       770                 775                 780
   Thr Phe Ala Leu Ser Ser Thr Lys Thr Leu Glu Glu Ala Val Asn Asn
   785                 790                 795                 800
   Ile Ile Thr Phe Leu Gly Met Gln Pro Cys Glu Arg Ser Asp Lys Val
                   805                 810                 815
   Pro Glu Asn Lys Asn Ser His Ser Leu Tyr Leu Ala Gly Val Tyr Arg
               820                 825                 830
   Gly Gly Tyr Asp Leu Leu Val Arg Ser Arg Leu Ala Leu Ala Asp Gly
           835                 840                 845
   Val Thr Met Gln Val Thr Val Arg Ser Lys Glu Arg Thr Pro Val Asp
       850                 855                 860
   Val Ile Leu Ala Ser Val Gly
   865                 870


<210> 172
<211> 3068
<212> DNA
<213> Rattus norvegicus

<220>
<223> Copg2

<400> 172
atgattaaaa aattcgacaa gaaggacgag gagtctggta gtggttccaa tcctttccag 60
catttggaga agagtgctgt cttgcaggag gcacgcatat tcaacgaaac gcccatcaat 120
ccaagaagat gcttgcatat cttgacaaag attctttact tactcaacca gggtgaacac 180
ttcggaacta tggaagctac agaagccttc tttgcaatga ctcgattgtt tcaatctaat 240
gatcaaacgc tgaggcggat gtgctacctc accatcaagg aaatggccac catctctgag 300
gatgtgatca ttgtcacaag cagtctgacc aaggacatga ctggaaagga agatgtatac 360
cgaggcccag ccatcagggc tctctgcaga atcacagatg gaacaatgtt gcaagccgtt 420
gaaaggtaca tgaagcaggc cattgtggat aaagtatcca gtgtagccag ctcagcgctg 480
gtctcttcct tacacatgat gaagataagc tatgatgtgg ttaaacgatg gataaatgaa 540
gcccaagaag ctgcatcaag tgacaatatc atggtgcagt accatgcact aggagtgctc 600
tatcacctta ggaagaatga tcggcttgct gtctccaaga tgttaaataa gtttaccaaa 660
tctggtctca aatcacagtt tgcatactgt atgctgattc gaattgctag tcgcttactg 720
aaagaaagcg aggatggtca tgaaagtcca ctctttgatt tcattgagag ctgcttgcga 780
aataaacacg aaatggttat ttatgaagct gcttctgcca ttattcatct tcccaactgt 840
actgcaagag agctggcacc tgctgtctca gttcttcaac ttttctgtag ctctcctaaa 900
ccagccttga gatatgcagc tgtgaggacc ctgaacaagg tggcaatgaa gcacccctct 960
gctgttactg cctgcaatct ggacttagaa aacttaatca cagactcaaa caggagcatt 1020
gctaccctgg ccattacaac attgctcaaa acaggaagcg agagcagtgt agacaggctt 1080
atgaagcaga tatcttcttt tgtgtctgaa atctcagatg agttcaaggt ggtggtcgtg 1140
caggccatca gtgctctctg tcacaagtac cctcggaagc acagcgtcat gatgactttt 1200
ctttccaaca tgcttcgaga tgatggaggt tttgaataca aaaaggccat tgtggactgc 1260
ataatcagca ttgtggaaga gaaccctgag agtaaagaag ctggtctagc ccacctctgt 1320
gaattcattg aggactgtga acataccgtt ctggctacta aaattctcca cttattgggc 1380
aaagagggcc ctagaactcc tgttccctcc aagtatatca gattcatttt taatagggtg 1440
gtcctggaga atgaggctgt cagagctgct gctgtgagtg ctttggctaa atttggggct 1500
cagaatgaga gccttctccc cagcatcctt gtactcttac aaaggtgtat gatggatact 1560
gatgatgagg tacgggaccg agctaccttc tacctgaatg tgctacagca gagacagatg 1620
gccctgaatg ctacatatat cttcaatggt ctgacagtct ctataccagg gatggaaaag 1680
gccttacacc agtatacatt ggagccttca gaaaaaccct ttgatatgaa gtcaattcct 1740
cttgctatgg ctcctgtctt tgagcagaag tcagaaatta cacttgtgac tcctaagcca 1800
gagaagttgg ctccttccag gcaagacatt ttccaagaac agttagctgc cattcctgag 1860
tttatgaacc tgggaccctt gtttaagtct tctgagcctg tgcagcttac agaagcagag 1920
acagagtatt tcgttcgctg tgtgaagcac atgtttactg accatattgt attccagttt 1980
gactgtacca acactctaaa tgaccagctg ctggagaaag taacagtgca gatggaacca 2040

tccgattcct atgaagtgct gtgctgtatc ccagctccca gcctccctta caatcagcca 2100
ggaatatgct acacacttgt tcgattgcca gatgaagatc ctacagcagt tgcaggcacc 2160
tttagctgta ccatgaagtt cacagtccgg gattgtgacc ctaatactgg agttccagat 2220
gaagatggat atgatgatga atatgtgctg gaagatcttg aagtaactgt gtctgatcat 2280
attcagaaga tactaaaacc taacttcgct gctgcctggg aagaggtggg agatgccttt 2340
gaaaaagaag aaacctttgc cctgagctct acaaaaaccc ttgaagaagc tgtcaacaac 2400
atcatcacat ttcttggtat gcagccatgt gagaggtcag ataaagtccc cgagaataag 2460
aactcccact ccctctatct agcaggtgta tacagaggtg gctatgactt attggtgcgg 2520
tccaggctgg ccctagctga tggagtgacc atgcaggtga ctgtgagaag caaagagaga 2580
acgcctgtgg atgttatctt ggcttctgtg ggataagttc ttgctgggaa gggtgatgtt 2640
gctatgccct ccctattggt ggtttttagg ctagtggcat gatcttccaa gaatcacaca 2700
taaagattaa taatgatgac tttgaagaag caaatccaag tgtttggccc tgcgccagca 2760
gatcaaacat ctgtctgtct gtctgtcttt gagcatgagc tttagagttt cttccctcat 2820
ctcatgtctc actcactcag cctcggtgcg acaatgcagc tttgagtggg cttgaaaatc 2880
agatactact tatgctattc tacagctgct tagcttcatt atatgcttct atttaatgtg 2940
tgaaaaactc ttggcaatgc tggaaaattt tatcccagaa gggtggaggg ggaggggaag 3000
ccggaatcca tttttgtcac agtttgtttt tattaataga aaataaacac tcattcattc 3060
caaaaaaa 3068

<210> 173
<211> 621
<212> PRT
<213> Homo sapiens

<220>
<223> F-box protein Fbx21

<400> 173
Met Ala Ala Ala Ala Val Asp Ser Ala Met Glu Val Val Pro Ala Leu
1               5                   10                  15

Ala Glu Glu Ala Ala Pro Glu Val Ala Gly Leu Ser Cys Leu Val Asn
            20                  25                  30

Leu Pro Gly Glu Val Leu Glu Tyr Ile Leu Cys Cys Gly Ser Leu Thr
        35                  40                  45

Ala Ala Asp Ile Gly Arg Val Ser Ser Thr Cys Arg Arg Leu Arg Glu
    50                  55                  60

Leu Cys Gln Ser Ser Gly Lys Val Trp Lys Glu Gln Phe Arg Val Arg
65                  70                  75                  80

Trp Pro Ser Leu Met Lys His Tyr Ser Pro Thr Asp Tyr Val Asn Trp
                85                  90                  95

Leu Glu Glu Tyr Lys Val Arg Gln Lys Ala Gly Leu Glu Ala Arg Lys
            100                 105                 110

Ile Val Ala Ser Phe Ser Lys Arg Phe Phe Ser Glu His Val Pro Cys
        115                 120                 125

Asn Gly Phe Ser Asp Ile Glu Asn Leu Glu Gly Pro Glu Ile Phe Phe
    130                 135                 140

Glu Asp Glu Leu Val Cys Ile Leu Asn Met Glu Gly Arg Lys Ala Leu
145                 150                 155                 160

Thr Trp Lys Tyr Tyr Ala Lys Lys Ile Leu Tyr Tyr Leu Arg Gln Gln
                165                 170                 175

Lys Ile Leu Asn Asn Leu Lys Ala Phe Leu Gln Gln Pro Asp Asp Tyr
            180                 185                 190

Glu Ser Tyr Leu Glu Gly Ala Val Tyr Ile Asp Gln Tyr Cys Asn Pro
        195                 200                 205

Leu Ser Asp Ile Ser Leu Lys Asp Ile Gln Ala Gln Ile Asp Ser Ile
    210                 215                 220

Val Glu Leu Val Cys Lys Thr Leu Arg Gly Ile Asn Ser Arg His Pro
225                 230                 235                 240

Ser Leu Ala Phe Lys Ala Gly Glu Ser Ser Met Ile Met Glu Ile Glu
                245                 250                 255

Leu Gln Ser Gln Val Leu Asp Ala Met Asn Tyr Val Leu Tyr Asp Gln
            260                 265                 270

Leu Lys Phe Lys Gly Asn Arg Met Asp Tyr Tyr Asn Ala Leu Asn Leu
        275                 280                 285

Tyr Met His Gln Val Leu Ile Arg Arg Thr Gly Ile Pro Ile Ser Met
    290                 295                 300

Ser Leu Leu Tyr Leu Thr Ile Ala Arg Gln Leu Gly Val Pro Leu Glu
305                 310                 315                 320

Pro Val Asn Phe Pro Ser His Phe Leu Leu Arg Trp Cys Gln Gly Ala
                325                 330                 335

Glu Gly Ala Thr Leu Asp Ile Phe Asp Tyr Ile Tyr Ile Asp Ala Phe
            340                 345                 350

Gly Lys Gly Lys Gln Leu Thr Val Lys Glu Cys Glu Tyr Leu Ile Gly
        355                 360                 365

Gln His Val Thr Ala Ala Leu Tyr Gly Val Val Asn Val Lys Lys Val
    370                 375                 380

Leu Gln Arg Met Val Gly Asn Leu Leu Ser Leu Gly Lys Arg Glu Gly
385                 390                 395                 400

Ile Asp Gln Ser Tyr Gln Leu Leu Arg Asp Ser Leu Asp Leu Tyr Leu
                405                 410                 415

Ala Met Tyr Pro Asp Gln Val Gln Leu Leu Leu Leu Gln Ala Arg Leu
            420                 425                 430

Tyr Phe His Leu Gly Ile Trp Pro Glu Lys Val Leu Asp Ile Leu Gln
        435                 440                 445

His Ile Gln Thr Leu Asp Pro Gly Gln His Gly Ala Val Gly Tyr Leu
    450                 455                 460

Val Gln His Thr Leu Glu His Ile Glu Arg Lys Lys Glu Glu Val Gly
465                 470                 475                 480

Val Glu Val Lys Leu Arg Ser Asp Glu Lys His Arg Asp Val Cys Tyr
                485                 490                 495

Ser Ile Gly Leu Ile Met Lys His Lys Arg Tyr Gly Tyr Asn Cys Val
            500                 505                 510

Ile Tyr Gly Trp Asp Pro Thr Cys Met Met Gly His Glu Trp Ile Arg
        515                 520                 525

Asn Met Asn Val His Ser Leu Pro His Gly His His Gln Pro Phe Tyr
    530                 535                 540

Asn Val Leu Val Glu Asp Gly Ser Cys Arg Tyr Ala Ala Gln Glu Asn
545                 550                 555                 560

Leu Glu Tyr Asn Val Glu Pro Gln Glu Ile Ser His Pro Asp Val Gly
                565                 570                 575

Arg Tyr Phe Ser Glu Phe Thr Gly Thr His Tyr Ile Pro Asn Ala Glu
            580                 585                 590

Leu Glu Ile Arg Tyr Pro Glu Asp Leu Glu Phe Val Tyr Glu Thr Val
        595                 600                 605

Gln Asn Ile Tyr Ser Ala Lys Lys Glu Asn Ile Asp Glu
    610                 615                 620


<210> 174
<211> 1866
<212> DNA
<213> Homo sapiens

<220>
<223> F-box protein Fbx21

<400> 174
atggcggcgg cagcagtcga cagcgcgatg gaggtggtgc cggcgctggc ggaggaggcc 60
gcgccggagg tagcgggcct cagctgcctc gtcaacctgc cgggtgaggt gctggagtac 120
atcctgtgct gcggctcgct gacggccgcc gacatcggcc gtgtctccag cacctgccgg 180
cggctgcgcg agctgtgcca gagcagcggg aaggtgtgga aggagcagtt ccgggtgagg 240
tggccttccc ttatgaaaca ctacagcccc accgactacg tcaattggtt ggaagagtat 300
aaagttcggc aaaaagctgg gttagaagcg cggaagattg tagcctcgtt ctcaaagagg 360
ttcttttcag agcacgttcc ttgtaatggc ttcagtgaca ttgagaacct tgaaggacca 420
gagatttttt ttgaggatga actggtgtgt atcctaaata tggaaggaag aaaagctttg 480
acctggaaat actacgcaaa aaaaattctt tactacctgc ggcaacagaa gatcttaaat 540
aatcttaagg cctttcttca gcagccagat gactatgagt cgtatcttga aggtgctgta 600
tatattgacc agtactgcaa tcctctctcc gacatcagcc tcaaagacat ccaggcccaa 660
attgacagca tcgtggagct tgtttgcaaa acccttcggg gcataaacag tcgccacccc 720
agcttggcct tcaaggcagg tgaatcatcc atgataatgg aaatagaact ccagagccag 780
gtgctggatg ccatgaacta tgtcctttac gaccaactga agttcaaggg gaatcgaatg 840
gattactata atgccctcaa cttatatatg catcaggttt tgattcgcag aacaggaatc 900
ccaatcagca tgtctctgct ctatttgaca attgctcggc agttgggagt cccactggag 960
cctgtcaact tcccaagtca cttcttatta aggtggtgcc aaggcgcaga aggggcgacc 1020
ctggacatct ttgactacat ctacatagat gcttttggga aaggcaagca gctgacagtg 1080
aaagaatgcg agtacttgat cggccagcac gtgactgcag cactgtatgg ggtggtcaat 1140
gtcaagaagg tgttacagag aatggtggga aacctgttaa gcctggggaa gcgggaaggc 1200
atcgaccagt cataccagct cctgagagac tcgctggatc tctatctggc aatgtacccg 1260
gaccaggtgc agcttctcct cctccaagcc aggctttact tccacctggg aatctggcca 1320
gagaaggtgc ttgacatcct ccagcacatc caaaccctag acccggggca gcacggggcg 1380
gtgggctacc tggtgcagca cactctagag cacattgagc gcaaaaagga ggaggtgggc 1440
gtagaggtga agctgcgctc cgatgagaag cacagagatg tctgctactc catcgggctc 1500
attatgaagc ataagaggta tggctataac tgtgtgatct acggctggga ccccacctgc 1560
atgatgggac acgagtggat ccggaacatg aacgtccaca gcctgccgca cggccaccac 1620

cagcctttct ataacgtgct ggtggaggac ggctcctgtc gatacgcagc ccaagaaaac 1680
ttggaatata acgtggagcc tcaagaaatc tcacaccctg acgtgggacg ctatttctca 1740
gagtttactg gcactcacta catcccaaac gcagagctgg agatccggta tccagaagat 1800
ctggagtttg tctatgaaac ggtgcagaat atttacagtg caaagaaaga gaacatagat 1860
gagtaa 1866

<210> 175
<211> 771
<212> PRT
<213> Homo sapiens

<220>
<223> KIAA0235

<400> 175
Glu Phe Ser Asn Pro Glu Thr Gln Asn Leu Asp Ala Met Glu Gln Val
1               5                   10                  15

Gly Leu Glu Ser Leu Gln Phe Asp Tyr Pro Gly Asn Gln Val Pro Met
            20                  25                  30

Asp Ser Ser Gly Ala Thr Val Gly Leu Phe Asp Tyr Asn Ser Gln Gln
        35                  40                  45

Gln Leu Phe Gln Arg Thr Asn Ala Leu Thr Val Gln Gln Leu Thr Ala
    50                  55                  60

Ala Gln Gln Gln Gln Tyr Ala Leu Ala Ala Ala Gln Gln Pro His Ile
65                  70                  75                  80

Ala Gly Val Phe Ser Ala Gly Leu Ala Pro Ala Ala Phe Val Pro Asn
                85                  90                  95

Pro Tyr Ile Ile Ser Ala Ala Pro Pro Gly Thr Asp Pro Tyr Thr Ala
            100                 105                 110

Ala Gly Leu Ala Ala Ala Ala Thr Leu Ala Gly Pro Ala Val Val Pro
        115                 120                 125

Pro Gln Tyr Tyr Gly Val Pro Trp Gly Val Tyr Pro Ala Asn Leu Phe
    130                 135                 140

Gln Gln Gln Ala Ala Ala Ala Ala Asn Asn Thr Ala Ser Gln Gln Ala
145                 150                 155                 160

Ala Ser Gln Ala Gln Pro Gly Gln Gln Gln Val Leu Arg Ala Gly Ala
                165                 170                 175

Gly Gln Arg Pro Leu Thr Pro Asn Gln Gly Gln Gln Gly Gln Gln Ala
            180                 185                 190

Glu Ser Leu Ala Ala Ala Ala Ala Ala Asn Pro Thr Leu Ala Phe Gly
        195                 200                 205

Gln Gly Leu Ala Thr Gly Met Pro Gly Tyr Gln Val Leu Ala Pro Thr
    210                 215                 220

Ala Tyr Tyr Asp Gln Thr Gly Ala Leu Val Val Gly Pro Gly Ala Arg
225                 230                 235                 240

Thr Gly Leu Gly Ala Pro Val Arg Leu Met Ala Pro Thr Pro Val Leu
                245                 250                 255

Ile Ser Ser Ala Ala Ala Gln Ala Ala Ala Ala Ala Ala Ala Gly Gly
            260                 265                 270

Thr Ala Ser Ser Leu Thr Gly Ser Thr Asn Gly Leu Phe Arg Pro Ile
        275                 280                 285

Gly Thr Gln Pro Pro Gln Gln Gln Gln Gln Gln Pro Ser Thr Asn Leu
    290                 295                 300

Gln Ser Asn Ser Phe Tyr Gly Ser Ser Ser Leu Thr Asn Ser Ser Gln
305                 310                 315                 320

Ser Ser Ser Leu Phe Ser His Gly Pro Gly Gln Pro Gly Ser Thr Ser
                325                 330                 335

Leu Gly Phe Gly Ser Gly Asn Ser Leu Gly Ala Ala Ile Gly Ser Ala
            340                 345                 350

Leu Ser Gly Phe Gly Ser Ser Gly Gly Leu Thr Asn Gly Ser Gly Arg
        355                 360                 365

Tyr Ile Ser Ala Ala Pro Gly Ala Glu Ala Lys Tyr Arg Ser Ala Ser
    370                 375                 380

Ser Thr Ser Ser Leu Phe Ser Ser Ser Ser Gln Leu Phe Pro Pro Ser
385                 390                 395                 400

Arg Leu Arg Tyr Asn Arg Ser Asp Ile Met Pro Ser Gly Arg Ser Arg
                405                 410                 415

Leu Leu Glu Asp Phe Arg Asn Asn Arg Phe Pro Asn Leu Gln Leu Arg
            420                 425                 430

Asp Leu Ile Gly His Ile Val Glu Phe Ser Gln Asp Gln His Gly Ser
        435                 440                 445

Arg Phe Ile Gln Gln Lys Leu Glu Arg Ala Thr Pro Ala Glu Arg Gln
    450                 455                 460

Met Val Phe Asn Glu Ile Leu Gln Ala Ala Tyr Gln Leu Met Thr Asp
465                 470                 475                 480

Val Phe Gly Asn Tyr Val Ile Gln Lys Phe Phe Glu Phe Gly Ser Leu
                485                 490                 495

Asp Gln Lys Leu Ala Leu Ala Thr Arg Ile Arg Gly His Val Leu Pro
            500                 505                 510

Leu Ala Leu Gln Met Tyr Gly Cys Arg Val Ile Gln Lys Ala Leu Glu
        515                 520                 525

Ser Ile Ser Ser Asp Gln Gln Ser Glu Met Val Lys Glu Leu Asp Gly
    530                 535                 540

His Val Leu Lys Cys Val Lys Asp Gln Asn Gly Asn His Val Val Gln
545                 550                 555                 560

Lys Cys Ile Glu Cys Val Gln Pro Gln Ser Leu Gln Phe Ile Ile Asp

                565                 570                 575

Ala Phe Lys Gly Gln Val Phe Val Leu Ser Thr His Pro Tyr Gly Cys
            580                 585                 590

Arg Val Ile Gln Arg Ile Leu Glu His Cys Thr Ala Glu Gln Thr Leu
        595                 600                 605

Pro Ile Leu Glu Glu Leu His Gln His Thr Glu Gln Leu Val Gln Asp
    610                 615                 620

Gln Tyr Gly Asn Tyr Val Ile Gln His Val Leu Glu His Gly Arg Pro
625                 630                 635                 640

Glu Asp Lys Ser Lys Ile Val Ser Glu Ile Arg Gly Lys Val Leu Ala
                645                 650                 655

Leu Ser Gln His Lys Phe Ala Ser Asn Val Val Glu Lys Cys Val Thr
            660                 665                 670

His Ala Ser Arg Ala Glu Arg Ala Leu Leu Ile Asp Glu Val Cys Cys
        675                 680                 685

Gln Asn Asp Gly Pro His Ser Ala Leu Tyr Thr Met Met Lys Asp Gln
    690                 695                 700

Tyr Ala Asn Tyr Val Val Gln Lys Met Ile Asp Met Ala Glu Pro Ala
705                 710                 715                 720

Gln Arg Lys Ile Ile Met His Lys Ile Arg Pro His Ile Thr Thr Leu
                725                 730                 735

Arg Lys Tyr Thr Tyr Gly Lys His Ile Leu Ala Lys Leu Glu Lys Tyr
            740                 745                 750

Tyr Leu Lys Asn Ser Pro Asp Leu Gly Pro Ile Gly Gly Pro Pro Asn
        755                 760                 765

Gly Met Leu
    770

<210> 176
<211> 5162
<212> DNA
<213> Homo sapiens

<220>
<223> KIAA0235

<400> 176
agaattttca aatcctgaaa ctcagaatct ggatgccatg gaacaagttg gtctggaatc 60
cttacagttt gactatcctg gtaatcaggt accaatggac tcttcaggag ctactgtagg 120
cctttttgac tacaattccc agcagcagct ctttcagagg actaatgcac taacagttca 180
acagttaact gcagctcaac agcagcaata tgcattagca gcagctcagc agccacatat 240
agctggtgta ttctcagcag gccttgctcc agctgcattt gtgccaaatc catacattat 300
tagtgctgct cctccaggga ccgatccgta tactgcagca ggattggctg cagcagctac 360
attagcaggt ccagcagtgg ttccacctca gtattacggc gttccatggg gggtgtatcc 420
agccaactta tttcagcagc aagctgcagc tgcggcaaat aacacagcca gtcagcaagc 480
agcatcacaa gctcagcctg gacagcaaca ggttctccgt gctggagcag gtcagcgtcc 540
tcttactccc aatcagggtc agcaagggca gcaagcagaa tcacttgcgg cagctgcagc 600

agcaaatcca acattggctt ttggtcaggg tcttgctact ggcatgccag gctatcaagt 660
actagctcca actgcctatt atgatcagac tggtgcctta gtggttggcc ctggagcaag 720
gactggcctt ggagctccag ttcggttaat ggctccaaca cctgttttaa ttagttcagc 780
agcagcacaa gctgcagcag cagcagcagc tggaggaact gcaagtagcc ttacaggcag 840
cacaaatggt ctgtttcggc caattggcac tcagccacca cagcagcagc aacagcagcc 900
aagcactaat ctgcaatcta attcatttta tggaagcagt tctttgacta atagctccca 960
gagtagttct ttattttctc atggacctgg tcaacctgga agtacatctc ttggctttgg 1020
aagtggtaac tctttgggtg ctgctatagg ctcagccctc agtggatttg gttcatcagg 1080
aggactgaca aatggtagtg gtcgatatat ctctgcagca cctggagcag aagcaaaata 1140
tcgaagtgct tcaagcactt ccagtctatt tagctccagc agccagctct ttcctccttc 1200
ccggcttcgg tataataggt ctgatattat gccttctggc cgcagtagat tattggaaga 1260
tttcagaaac aaccgcttcc caaaccttca gcttagagac ttgattggac atatagttga 1320
gttttctcaa gaccagcatg gttctagatt catacagcaa aaactagaga gagctactcc 1380
agctgagcga cagatggtat ttaatgaaat tctgcaagca gcctatcaat taatgactga 1440
tgtttttggc aactatgtta tacagaagtt ttttgagttt gggagtctgg atcaaaaatt 1500
agccctggct actcgtattc gtggtcatgt tctaccctta gccttgcaga tgtatggctg 1560
ccgcgttatt cagaaagcat tagaatctat ttcttctgac cagcagagtg aaatggtaaa 1620
ggagctggat ggtcatgtgc tcaaatgtgt gaaagatcag aatggaaacc atgttgtaca 1680
aaaatgtatc gaatgtgttc agccacagtc actacagttc atcattgatg ctttcaaggg 1740
acaagtattt gtgctttcaa ctcatcctta tggctgcaga gtaattcagc gcatcctaga 1800
gcattgcact gcagaacaga ccttacctat cttagaagaa ctccaccaac atacagagca 1860
gttggtacag gatcagtatg gcaattatgt tattcagcat gtactggaac acggtcgacc 1920
tgaagacaag agcaaaattg tttccgaaat caggggaaag gttttagccc tgagtcaaca 1980
caaatttgcc agcaatgtag tagaaaagtg tgttactcat gcctcccgtg ctgagagagc 2040
tttactgatt gacgaggttt gctgccagaa tgatggtcct cacagtgcct tatacaccat 2100
gatgaaggac cagtatgcca attacgtggt tcaaaagatg attgatatgg ctgaacctgc 2160
tcagagaaag ataatcatgc acaagattcg acctcacatt actactttgc gcaaatacac 2220
atacgggaag catatactgg ccaagttgga aaagtattat ttgaagaata gcccggacct 2280
aggacctatt ggaggaccac caaatggaat gctgtaaatt acaggagcaa gagaaagaag 2340
ataatttaac catgtgaaaa gaattttttt gtgcgtgaat tatcaaaaca caactcaact 2400
atgaatcttc aatttttttt taaagcaaaa ctatttattg actttattca tccatttgta 2460
aattttttaa ggttcttgtg tatatttggg gggtggggga tgaattataa attatattca 2520
gccctgagtg gagacctatc agattggatt gctggcaaag cacagaatgc ctgtatatga 2580
tgtaactgta tcaaaaataa aaagctgtca catattttgt aaatttttac cttgtaaagt 2640
cacaaaaata gtttttaaag gaaaaagtac agtattcttt taataaactg gctcacagtc 2700
tggtaggtct acaaccccat agcacaacag gtttatagag atgtatatag aattatagtc 2760
cttatttttt tcctttgcgt gaaacctttt ataacagatt aacaatcaac tgcataaata 2820
ttattaatat tttaaaaaga gttaagttgt attttgataa ttcacaaact atcatgcaaa 2880
taacgagtaa gtagacaaga ataaagtggt ttgagatgaa aagaacctaa cattatttac 2940
agtagatgtg gttttaatac aattactgcc ctaaaatgtc tctggcaatg tacagaaata 3000
ttgtatatac ttacatatgt aattgttgta agagttaaat acaaaatcat ggtgacactt 3060
ccaattaagt gcactaaatg aaaagttaag tcacttatta acttttcagt ttggtttgca 3120
atgagaaaga gtggaaattt gtattttgtt ttgcttatag aattacagac atgttgagga 3180
agtgttgagc tttattttgc tttttcatag aggcagaaag taggaaccag atagagatga 3240
aaaggggcca ctgaaaagtg aatttgatag ctcagcattt aagcatgatt acatattcag 3300
atagctcttt ttgctttcta taaatatatg cattgtgtgt gtagtaatag atgtaagttt 3360
acactttgaa aggaaatctt gtttcaatgt ttattataaa agccttgcta atttagtagt 3420
gatgctttcc ttggttgtac aggtgtacat ttgtaaacct tcatgctgta aatggaattt 3480
gttttatctc tttgggatac atttgcattt tagtgtacat ttacgtccct gccctctttg 3540
acctggcaat atagtgttgt ataatgtaaa tttatttctc caaatcgaga gtgatttttt 3600
aaaaattttt tatctttata tggtttcaga agtatgaacc agctttcttt ttattattgt 3660
gagatcattt tgttttataa catagttgtt gactgttaat atggacctgc tagaatttgg 3720
atcactttca attgaagtca gggtattgtg cataatagaa agtattggac tgagatattt 3780
ggttaccatg gaggccaatg cttttttcat cttattaaat gtgatgtgac ttttttcttt 3840
gtacagaaga gtactgtatt tttgaatagc ctactcccaa gtaagagcaa atctgtatga 3900
taacattttt tcctctggac ataagacata acagtaacac gatgtacatt tacaagcggc 3960
cttatgtaca tttcccaaca atctttttaa ggcaaaattg tgaccatatg tgtataatta 4020
aaatcgtttt taatcctttg cctatgaaaa tattttggaa aaaaacttgc tgtgtatatt 4080
cagtttctga aagataaaga aagtgctttg tattttgttg aagtcagtat tttgtataaa 4140
catttatgtt gacccactta tgttcagtgc tgaaaactaa aatgaacatg ctattctgtc 4200
agctgaatat ggaagagatc tttttttact agagatctgc agaagaaacg caatcttctg 4260

agcacaatat ggaatctaaa ggttttatca cttagttgtt catattatga acctaaaaat 4320
aatggcataa agtttgggga tgccaggcat actttttcat gtttggtgtt gagttatttt 4380
acttttctaa cccaacattc cttggtgaga ccattaaatc caaacacttg tcaccgttcc 4440
ttctcatagt cactctgggt catcagcatg tcccagtcac tgcagcaacg ccttgtgttt 4500
gtttcatttt tttaaaaccc acacaaagcc gctgtctcac tttttcctac tttaccaacc 4560
tcagagtatt tcggcccgta tcgaactttt gttctcagta tcagcccatg gtttcaggat 4620
caaagctgtc atgttggaga ttggtaatgg ctttcctgtc tttgtacagt tgaattccta 4680
gtcttccttc atccttgccc tctgttggca caggcattat ctctgcaatt ttagaaaatg 4740
acaagtagag aatactacat tgagaaacta aaccctcttc ttggggtcct gatactcatt 4800
cccatttgtc ccagtgctga caacccaatc ttcccaatac tttcaggcct gctctacaaa 4860
agtacctgtt cttgtagaaa ttttacagtc tgccattttg ggtgcccacc ccaattttta 4920
ccttttagta agttggcatg aaattttggt aaaatctgaa aatcacattt cagaataaaa 4980
caattgggca aaactaccta ggctttactc ttgagtgtct ccttttgata gggattgttt 5040
ctggaccagt ttgtctaagt cctggctctt attggttcat atgaaataat gttaacttca 5100
cttctttgta tattatgtat aaattagaaa atgaaaaatg tgtgaataac attgtatgaa 5160
at 5162

<210> 177
<211> 547
<212> PRT
<213> Homo sapiens

<220>
<223> KIAA 1212

<400> 177
Gln Gln Leu Asp Ile Thr Ser Thr Lys Leu Asn Asn Gln Cys Glu Leu
1               5                   10                  15

Leu Ser Gln Leu Lys Gly Asn Leu Glu Glu Glu Asn Arg His Leu Leu
            20                  25                  30

Asp Gln Ile Gln Thr Leu Met Leu Gln Asn Arg Thr Leu Leu Glu Gln
        35                  40                  45

Asn Met Glu Ser Lys Asp Leu Phe His Val Glu Gln Arg Gln Tyr Ile
    50                  55                  60

Asp Lys Leu Asn Glu Leu Arg Arg Gln Lys Glu Lys Leu Glu Glu Lys
65                  70                  75                  80

Ile Met Asp Gln Tyr Lys Phe Tyr Asp Pro Ser Pro Pro Arg Arg Arg
                85                  90                  95

Gly Asn Trp Ile Thr Leu Lys Met Arg Lys Leu Ile Lys Ser Lys Lys
            100                 105                 110

Asp Ile Asn Arg Glu Arg Gln Lys Ser Leu Thr Leu Thr Pro Thr Arg
        115                 120                 125

Ser Asp Ser Ser Glu Gly Phe Leu Gln Leu Pro His Gln Asp Ser Gln
    130                 135                 140

Asp Ser Ser Ser Val Gly Ser Asn Ser Leu Glu Asp Gly Gln Thr Leu
145                 150                 155                 160

Gly Thr Lys Lys Ser Ser Thr Met Asn Asp Leu Val Gln Ser Met Val
                165                 170                 175

Leu Ala Gly Gln Trp Thr Gly Ser Thr Glu Asn Leu Glu Val Pro Asp

            180                 185                 190

Asp Ile Ser Thr Gly Lys Arg Arg Lys Glu Leu Gly Ala Met Ala Phe
        195                 200                 205

Ser Thr Thr Ala Ile Asn Phe Ser Thr Val Asn Ser Ser Ala Gly Phe
    210                 215                 220

Arg Ser Lys Gln Leu Val Asn Asn Lys Asp Thr Thr Ser Phe Glu Asp
225                 230                 235                 240

Ile Ser Pro Gln Gly Val Ser Asp Asp Ser Ser Thr Gly Ser Arg Val
                245                 250                 255

His Ala Ser Arg Pro Ala Ser Leu Asp Ser Gly Arg Thr Ser Thr Ser
            260                 265                 270

Asn Ser Asn Asn Asn Ala Ser Leu His Glu Val Lys Ala Gly Ala Val
        275                 280                 285

Asn Asn Gln Ser Arg Pro Gln Ser His Ser Ser Gly Glu Phe Ser Leu
    290                 295                 300

Leu His Asp His Glu Ala Trp Ser Ser Ser Gly Ser Ser Pro Ile Gln
305                 310                 315                 320

Tyr Leu Lys Arg Gln Thr Arg Ser Ser Pro Val Leu Gln His Lys Ile
                325                 330                 335

Ser Glu Thr Leu Glu Ser Arg His His Lys Ile Lys Thr Gly Ser Pro
            340                 345                 350

Gly Ser Glu Val Val Thr Leu Gln Gln Phe Leu Glu Glu Ser Asn Lys
        355                 360                 365

Leu Thr Ser Val Gln Ile Lys Ser Ser Ser Gln Glu Asn Leu Leu Asp
    370                 375                 380

Glu Val Met Lys Ser Leu Ser Val Ser Ser Asp Phe Leu Gly Lys Asp
385                 390                 395                 400

Lys Pro Val Ser Cys Gly Leu Ala Arg Ser Val Ser Gly Lys Thr Pro
                405                 410                 415

Gly Asp Phe Tyr Asp Arg Arg Thr Thr Lys Pro Glu Phe Leu Arg Pro
            420                 425                 430

Gly Pro Arg Lys Thr Glu Asp Thr Tyr Phe Ile Ser Ser Ala Gly Lys
        435                 440                 445

Pro Thr Pro Gly Thr Gln Gly Lys Ile Lys Leu Val Lys Glu Ser Ser
    450                 455                 460

Leu Ser Arg Gln Ser Lys Asp Ser Asn Pro Tyr Ala Thr Leu Pro Arg
465                 470                 475                 480

Ala Ser Ser Val Ile Ser Thr Ala Glu Gly Thr Thr Arg Arg Thr Ser
                485                 490                 495

Ile His Asp Phe Leu Thr Lys Asp Ser Arg Leu Pro Ile Ser Val Asp
            500                 505                 510

Ser Pro Pro Ala Ala Ala Asp Ser Asn Thr Thr Ala Ala Ser Asn Val
        515                 520                 525

Asp Lys Val Gln Glu Ser Arg Asn Ser Lys Ser Arg Ser Arg Glu Gln
    530                 535                 540

Gln Ser Ser
545

<210> 178
<211> 4748
<212> DNA
<213> Homo sapiens

<220>
<223> KIAA 1212

<400> 178
   caacaattgg atattacatc aaccaagctg aataaccagt gtgagttgct aagccaactt 60
   aaaggaaatt tagaagaaga aaatcggcat ctactagatc aaattcagac attaatgcta 120
   cagaacagaa cacttttgga gcagaatatg gaaagcaagg atctttttca tgttgaacaa 180
   agacagtaca ttgataagtt aaatgaatta agacgtcaga aggagaaact agaagagaaa 240
   attatggatc aatacaaatt ttatgaccca tctcctccta gaaggagagg caactggatt 300
   actctaaaaa tgagaaaatt gataaagtct aagaaagata ttaatcggga acgccagaaa 360
   tctctaacat taacacccac ccgctcagac tccagtgaag gatttcttca gctccctcat 420
   caagacagtc aagatagttc ttcagtaggt tcaaactctt tagaagatgg ccagaccttg 480
   gggaccaaga aaagcagcac catgaatgac ctggtgcagt ccatggtcct agcaggacag 540
   tggacaggta gtactgagaa tttggaggtt cctgatgata tttcaacggg taaaaggaga 600
   aaagaattgg gagctatggc cttctctact acagccatca acttttcaac tgtcaactct 660
   tctgcaggct tcagatccaa gcagttggtt aataataaag atactacatc ctttgaagac 720
   ataagtccac aaggtgttag tgatgattct agtacgggat caagagttca tgcttcaaga 780
   ccagccagcc ttgatagtgg cagaacatcc actagcaata gcaataataa tgcttcacta 840
   catgaagtca aagcaggtgc agttaataac caaagcaggc cacaaagcca cagcagtgga 900
   gaatttagcc tgcttcatga ccatgaggct tggtccagca gtggtagcag tccaatccag 960
   tacttgaaaa gacagaccag atcaagccca gtgctccagc acaaaatatc tgaaacactg 1020
   gagagtcgac atcacaagat caaaactggt tcccctggaa gtgaagttgt tactctacaa 1080
   cagtttttgg aagaaagcaa taagcttacc tcagtacaga taaagtcctc aagtcaagag 1140
   aatcttttag atgaagtaat gaaaagtttg tctgtctctt ctgacttttt gggaaaagac 1200
   aaaccagtta gctgtggtct ggccaggtca gtaagtggaa aaaccccagg ggacttctat 1260
   gatagacgga caactaagcc tgagtttttg agacctggtc ctcgaaaaac tgaagatacc 1320
   tacttcatta gttctgcggg aaaacctaca ccaggcactc aaggaaaaat aaaattagta 1380
   aaagaatctt ctctgtcacg acaatcaaaa gatagtaacc cttatgcaac tttacctcgt 1440
   gcaagcagcg tgatctcaac tgccgaagga actacacgaa ggacaagcat ccatgatttt 1500
   ttgaccaagg acagtagact gcctatatca gttgattcac caccagctgc tgctgacagc 1560
   aacaccactg cagcatctaa tgtggacaaa gtacaagaaa gcagaaattc aaaaagcagg 1620
   tctagggagc aacaaagctc ctaattctat tacccactac atgacatgtg ggccaagtga 1680
   gagaaaagtg tccttcagtt tctcagtatg aagcctttat ttctgaagta acaagacacc 1740
   tagcaactat aggaatcatt tttaaaaatt tttaaggaga cttttaacag tccttcgtga 1800
   atagagcagg caagaaatac aaaccttcat tccttgaatc aaggagcact actggattca 1860
   actgccaaaa ttttttaaag gttttaggac ttactatacc ttgtactgtt aagatctact 1920
   gaataaagga cgttctctca ctaaggacca ggtgttttaa ggttaagtgt ttaaagaagt 1980
   actccaagaa caatctgctt ttttcatcat ttgttttatg aatttatcca tgtttgctta 2040
   atgcttctgc taagtgttag ccaaaatcta gccatttata tttagttgtg taaacctaaa 2100
   ttaaatgctg tagtattttg tggaatgtac tatatagcaa gatacagaga aaattgtttt 2160
   ggcatgtcag agccttattt ggttagcaga ctgcatgtgt tgatactttt tttttcttaa 2220
   agccaattat tttgatgcaa aagaaattca gtttataaga taaatctgaa aaatccataa 2280
   tgaaatagga gttataaaaa atttatagtg atattaatct ttccatattt cccattaagc 2340
   aacactaagc attcataagt taacccatgg taaagagtgt ttttctgaaa ctttttttta 2400
   gtaagatggt ttttcagcaa atggcattcc caagataaag ctgttgtgct ttaactcatt 2460
   tcttttcttt ggtattgggt tatgtatgcg tgtgcatttt tttaacttga gagctgactg 2520
   ttgcttaaga agttttctta tggcaaaaat aatgtaaata agttactatg atctgcattt 2580
   tgccagaaac tcatttataa ttaaggctat catttattaa tgattttttt ctcctttatg 2640
   atattacatt aaagttgata actgttattg gtacttttga aatatttgta tgtatttgtt 2700
   acctttaaac atttggaaga agcacaaaaa aatagattta gttaacccag ggaaacatca 2760
   atttttttag tagttccaat tttatatcac agttttattt tcttatgaaa tcaaaaaatg 2820
   cattgatact cattaatgca aattcattat ttaacatcaa tatcagagta atcttcaagg 2880
   tctgaaatga gaaacatact gactttttaa aattttaaca gtgtacttct taggctttca 2940
   ttaccagctc tgaagaactt tttggaataa ttccatattc catagtgtgt ggtttatgag 3000
   ttgtgggttt catcactaac ccagtaacca taagaaaagt ctctctctct ctctctctct 3060
   ctttctctct ccctctttct ctctctcttt cgtaggccag tagcaatgtt gtgttcacag 3120
   tctaatttcc aaaagaccat caataaaaaa gagagcatgt ttaaattgaa atggaactta 3180
   gagaacttga gcttacttac gtacttcaat gccaccggta acttaggttt taccaccaaa 3240
   tgctgttaac attaaatcat tttgaaaatc ttggatgaaa ggtgctatgt aaatggaaat 3300
   acaaaggatt cttactaaca tacaaaaata atgcacaaca gaaatatcta aaaccttttc 3360
   cgtagacttt gaaacatctc tctctgtcat aactccctgg attcaagtag cacattggta 3420
   ataggtatca gagcagtcta gagacaattg catgtcaaaa aatgtacatt catttttagg 3480
   tggataaaag taaacataga aattatgtta tggctaaata cagttagtgg gtaacttaga 3540
   tttatattag ctagcatcta atttgcacaa ctagaacaca tcccagaaca attactgaaa 3600
   agctgaaatt taatgggtgg tgatgtagcc caatgagggc gaatgacatt ccagcttgac 3660
   ctctccagaa cactaatatc ctaaaataca gaacatgctg ggttaagtgc attagtgctt 3720
   caagcagaaa atgctgaaaa caacgtgtaa agtactgaat ctgagtaggc tgaccctgag 3780
   aagggacaat taaagagaca accaagggaa cacattgaga ctacaaaaat atgaataatc 3840
   tcaattatat tcatcacact tttttcatac catttcaaga aacgactaga cagtagtaac 3900
   cacatgaata ttttactttc tccagtatac cttgagaagc aaactttgta ggaagccact 3960
   cttctcccct aaacaacttc tgccaaacaa taataaagcc aactggaaac gaatcggagc 4020
   cattttcatt ttcctaaccg gggcctgaca tgctttaaat tatctggctg tattctaaat 4080
   caacacctaa cccctcaagg aaactgaaga atcaatatac agggtaatag ctttggctca 4140
   gagctccaat aatgtgcttc agatctgtcc atgtggaaat gctttcatcc aaatttttaa 4200
   attggtggtt accaaagagt tcacaaaaca ggtttgtatg tagcaccttt catgcaaggc 4260
   atgcaaaaag cctattttaa aatcactgtg catattatag agttgtagcc acctcacaat 4320
   gaagtactac agcctgtgct gtcttaatgg tttatgtcag gaaatgaaaa agatactgta 4380
   ccaaatctgg aattacaatg gggagtaata atgtatacta aatgactttt gtattttaag 4440
   ttactttttg tgagtggtga atttttgtgt ttttcttttc agctacactt agtcctgaga 4500
   tgtatttttt ctttaagtct tgaatgaata caaaaggagc ccattttata atataaacct 4560
   tgatgtacat gttgagatat ttggacaatg aaaatgcctt aaaaggaatg catatggata 4620
   aagttgcact tataacaccc ttcaacaaaa tctaatttta aattgtcttt ttcttttcta 4680
   ttaagggttt tctttttcag tgtctaccat tgtacttata actgttatta aataccaaat 4740
   caaataat 4748

<210> 179
<211> 298
<212> PRT
<213> Mus musculus

<220>
<223> Osteoglycin

<400> 179
Met Glu Thr Val His Ser Thr Phe Leu Leu Leu Leu Phe Val Pro Leu
1               5                   10                  15

Thr Gln Gln Ala Pro Gln Ser Gln Leu Asp Ser His Val Asn Tyr Glu
            20                  25                  30

Tyr Ala Thr Gly Asn Ser Glu Glu Thr Lys Phe Ser Gln Asp Tyr Glu
        35                  40                  45

Asp Lys Tyr Leu Asp Gly Lys Ser Ile Lys Glu Lys Glu Thr Met Ile
    50                  55                  60

Ile Pro Asp Glu Lys Ser Leu Gln Leu Gln Lys Asp Glu Val Ile Pro
65                  70                  75                  80

Ser Leu Pro Thr Lys Lys Glu Asn Asp Glu Met Pro Thr Cys Leu Leu
                85                  90                  95

Cys Val Cys Leu Ser Gly Ser Val Tyr Cys Glu Glu Val Asp Ile Asp
            100                 105                 110

Ala Val Pro Pro Leu Pro Lys Glu Ser Ala Tyr Leu Tyr Ala Arg Phe
        115                 120                 125

Asn Lys Ile Lys Lys Leu Thr Ala Lys Asp Phe Ala Asp Met Pro Asn
    130                 135                 140

Leu Arg Arg Leu Asp Phe Thr Gly Asn Leu Ile Glu Asp Ile Glu Asp
145                 150                 155                 160

Gly Thr Phe Ser Lys Leu Ser Leu Leu Glu Glu Leu Thr Leu Ala Glu
                165                 170                 175

Asn Gln Leu Leu Arg Leu Pro Val Leu Pro Pro Lys Leu Thr Leu Leu
            180                 185                 190

Asn Ala Lys His Asn Lys Ile Lys Ser Lys Gly Ile Lys Ala Asn Thr
        195                 200                 205

Phe Lys Lys Leu Asn Lys Leu Ser Phe Leu Tyr Leu Asp His Asn Asp
    210                 215                 220

Leu Glu Ser Val Pro Pro Asn Leu Pro Glu Ser Leu Arg Val Ile His
225                 230                 235                 240

Leu Gln Phe Asn Ser Ile Ser Ser Leu Thr Asp Asp Thr Phe Cys Lys
                245                 250                 255

Ala Asn Asp Thr Arg Tyr Ile Arg Glu Arg Ile Glu Glu Ile Arg Leu
            260                 265                 270

Glu Gly Asn Pro Ile Ala Leu Gly Lys His Pro Asn Ser Phe Ile Cys
        275                 280                 285

Leu Lys Arg Leu Pro Ile Gly Ser Tyr Phe
    290                 295

<210> 180
<211> 1268
<212> DNA
<213> Mus musculus

<220>
<223> Osteoglycin

<400> 180
tcagtcactc ctaacttcag tcaccttgtg tctgctgaaa tggagactgt gcactctaca 60
tttctcctgc tactcttcgt gcctctgaca cagcaagcac cacagtcgca gctggactca 120
catgttaact atgagtatgc aacaggcaat tctgaagaaa ccaaatttag ccaggattat 180
gaagataaat acctggatgg gaaaagtatt aaggaaaaag aaactatgat aattcctgat 240
gagaaaagtc ttcagttaca aaaagatgaa gttataccat cattaccaac caagaaagaa 300

aatgatgaaa tgcccacatg cctgttgtgt gtctgcttaa gtggctctgt ctactgtgaa 360
gaagttgaca ttgatgctgt accaccattg ccaaaggaat cagcttatct ttatgcacga 420
ttcaacaaaa ttaaaaagct gactgccaag gattttgcag acatgccaaa cctaagaaga 480
cttgatttta cgggaaattt gatagaagac atagaagatg ggactttttc aaaactttct 540
ctgttagaag aacttacact tgctgaaaac caactactaa gacttccagt tcttcctcca 600
aagcttactt tacttaatgc caaacacaac aaaatcaaga gtaaaggaat taaagcaaac 660
acattcaaaa aactgaataa actctctttt ctctatttgg accataacga cctggaatct 720
gtgcctccta atttaccaga aagtctacgt gtaattcacc ttcagtttaa cagcatatct 780
tcacttacag atgatacatt ctgcaaggct aatgacactc gttacattcg ggagcgaatt 840
gaagagattc gcctggaggg caatccaatt gctctgggaa agcatccaaa cagttttatc 900
tgcttaaaaa gattacccat agggtcatac ttctaacccc atcagtgcag cttataacct 960
aaggtacatg tgcctaatgt ctaaaggaac agaaacatta gtttaacatt accgttttat 1020
ctcactatga tggaagttta tgtttaaaca aagtgtccaa aattgttgta tgtgactaca 1080
aaaataatcc agcctgaatc tcttagttca aaacaaaata ctgtgaaact aaacagcatt 1140
aagggtctcc ttgttgttat accaaaccac tatctaaata gcatgctttt acacagacac 1200
ttgcttgaga tacaccattc ccatgactaa tgaagtaaga aaattccaag aaacttgcaa 1260
ctgtttgt 1268

<210> 181
<211> 346
<212> PRT
<213> Homo sapiens

<220>
<223> SAM68-like phosphotyrosine protein alpha

<400> 181
Met Glu Glu Lys Tyr Leu Pro Glu Leu Met Ala Glu Lys Asp Ser Leu
1               5                   10                  15

Asp Pro Ser Phe Thr His Ala Leu Arg Leu Val Asn Gln Glu Ile Glu
            20                  25                  30

Lys Phe Gln Lys Gly Glu Gly Lys Asp Glu Glu Lys Tyr Ile Asp Val
        35                  40                  45

Val Ile Asn Lys Asn Met Lys Leu Gly Gln Lys Val Leu Ile Pro Val
    50                  55                  60

Lys Gln Phe Pro Lys Phe Asn Phe Val Gly Lys Leu Leu Gly Pro Arg
65                  70                  75                  80

Gly Asn Ser Leu Lys Arg Leu Gln Glu Glu Thr Leu Thr Lys Met Ser
                85                  90                  95

Ile Leu Gly Lys Gly Ser Met Arg Asp Lys Ala Lys Glu Glu Glu Leu
            100                 105                 110

Arg Lys Ser Gly Glu Ala Lys Tyr Phe His Leu Asn Asp Asp Leu His
        115                 120                 125

Val Leu Ile Glu Val Phe Ala Pro Pro Ala Glu Ala Tyr Ala Arg Met
    130                 135                 140

Gly His Ala Leu Glu Glu Ile Lys Lys Phe Leu Ile Pro Asp Tyr Asn
145                 150                 155                 160

Asp Glu Ile Arg Gln Ala Gln Leu Gln Glu Leu Thr Tyr Leu Asn Gly
                165                 170                 175

Gly Ser Glu Asn Ala Asp Val Pro Val Val Arg Gly Lys Pro Thr Leu
            180                 185                 190

Arg Thr Arg Gly Val Pro Ala Pro Ala Ile Thr Arg Gly Arg Gly Gly
        195                 200                 205

Val Thr Ala Arg Pro Val Gly Val Val Val Pro Arg Gly Thr Pro Thr
    210                 215                 220

Pro Arg Gly Val Leu Ser Thr Arg Gly Pro Val Ser Arg Gly Arg Gly
225                 230                 235                 240

Leu Leu Thr Pro Arg Ala Arg Gly Val Pro Pro Thr Gly Tyr Arg Pro
                245                 250                 255

Pro Pro Pro Pro Pro Thr Gln Glu Thr Tyr Gly Glu Tyr Asp Tyr Asp
            260                 265                 270

Asp Gly Tyr Gly Thr Ala Tyr Asp Glu Gln Ser Tyr Asp Ser Tyr Asp
        275                 280                 285

Asn Ser Tyr Ser Thr Pro Ala Gln Ser Gly Ala Asp Tyr Tyr Asp Tyr
    290                 295                 300

Gly His Gly Leu Ser Glu Glu Thr Tyr Asp Ser Tyr Gly Gln Glu Glu
305                 310                 315                 320

Trp Thr Asn Ser Arg His Lys Ala Pro Ser Ala Arg Thr Ala Lys Gly
                325                 330                 335

Val Tyr Arg Asp Gln Pro Tyr Gly Arg Tyr
            340                 345


<210> 182
<211> 1963
<212> DNA
<213> Homo sapiens

<220>
<223> SAM68-like phosphotyrosine protein alpha

<400> 182
gcgcggcgtg caggtcgcag ctgtggctcg ggtgctggca ggtgctggcg ggactggcgg 60
ggatcgccgt ggactgcctc aggggcgccg cagcccgctc ggcccgaggg cccgcctaac 120
cgcgccgccc gcgcccgctc ctcctcggcc cgcgcccgga gcgcgggggc cgccggcgct 180
gggtactcgg cggccaccgg ggatcggggc tgagcggtcg gttcccgccc ccgtgccgcc 240
gccgccgcct tccggccgac cgcccggctt ggccgctgct gccgcgtctg gcccccgggt 300
ccccgccgct gggggcgcgg gcggggtcgg gggttgccgg gcgccgcccc ccgtgcgcct 360
ggagtccaca tcccgggccc ggcggccggc gagcatggag gagaagtacc tgcccgagct 420
gatggcggag aaggactccc tggacccctc cttcacgcac gccctgcgcc tggtgaacca 480
agaaatagaa aagtttcaaa aaggagaagg caaggatgaa gaaaagtaca tcgatgtggt 540
gattaataag aacatgaagc tgggacagaa agtgttaatt cccgtaaaac agttccctaa 600
gttcaacttt gtggggaaac ttttgggtcc acgtggcaat tctctgaagc gtttacaaga 660
agaaaccttg acaaaaatgt ccatccttgg gaaaggttcc atgagagaca aggccaagga 720
agaagagttg aggaaaagtg gagaagcgaa gtacttccat ctcaatgatg atctccatgt 780
tctcattgaa gtgtttgccc cacctgcaga agcttatgcc aggatgggac atgctttgga 840
agaaatcaaa aagttcctca tccctgatta taatgatgag atcaggcaag cacagctcca 900
ggagttaaca tatttgaatg gtggttcaga aaatgcagat gttccagtgg ttcgagggaa 960
acccaccttg cgtacaagag gtgtaccagc cccagcaata accaggggaa ggggaggagt 1020
tacagcccgg ccagttggag ttgtagtacc acgagggacg ccaactccca gaggagtcct 1080

gtccacccga gggccagtga gtcggggaag aggacttctc actcccagag caagaggagt 1140
ccccccaact gggtacagac ctccaccgcc acccccgaca caagagactt atggagaata 1200
tgactatgat gatggatatg gcactgctta tgatgaacag agttatgatt cctatgataa 1260
cagctatagc accccagccc aaagtggtgc tgattactat gattacggac atggactcag 1320
tgaggagact tatgattcct acgggcaaga agagtggact aactcaagac acaaggcacc 1380
ttcagcgagg acagcaaagg gcgtctacag agaccagcca tatggcagat actgattgta 1440
ctgtctgatg ttgtgaaata gccaatctcc accagtcctg tatactgttc aaagtaattt 1500
ttttctatga acaatccctt tttaaataaa tcaaaatgct taaaatctga atggatggaa 1560
cttaaaacta ctttgttgaa acatcaacct gggcagaaaa aaagaaaaaa agacatgtaa 1620
aattttgtta tttccagtct gtatatgaaa aaataggtca tcaaaaggaa aaaaaataac 1680
tttgattaac tagtgttaaa caaaaaatag gtttactaaa tatgttaatc tattctttta 1740
acataagcct cacctttcat tttaaaggtt tccatagaat ttagttattt tatctttcag 1800
ccatatgcta gttttttttt ctcttgccaa catgcgtaaa aagggaagcc aattacaagt 1860
gcaaataatg tggtattctt ttgtaactca agtcttgaaa tgttctgtag tgttaagcaa 1920
agtctcctct tgcttgatac taaataaact tttgaaaaaa aaa 1963

<210> 183
<211> 178
<212> PRT
<213> Rattus norvegicus

<220>
<223> Ribosomal Protein L11

<400> 183

<210> 184
<211> 585
<212> DNA
<213> Rattus norvegicus

<220>
<223> Ribosomal Protein L11


<400> 184
   tcttcctgtt ctccaccatg gcgcaagatc agggtgaaaa ggagaacccc atgcgggaac 60
   ttcgtatccg caagctctgc ctcaatatct gcgtggggga gagtggagac agactgaccc 120
   gagcagccaa ggtgttggag cagctcacag gccagacccc tgtgttttcc aaagctagat 180
   acaccgtcag gtcctttggc atccggcgaa atgagaaaat cgctgttcac tgcactgtcc 240
   gtggagccaa ggcagaggag attctggaga aaggcctgaa ggtgcgagag tacgaattac 300
   ggaagaataa cttctcagat accggcaact ttggctttgg gattcaggaa cacattgacc 360
   taggcatcaa atatgaccca agcattggca tctatggcct ggatttctat gtggtgctgg 420
   gtaggccagg tttcagcatc gcagacaaga agcgcagaac aggctgcatt ggggccaaac 480
   acagaatcag caaagaggag gcaatgcgct ggttccagca gaagtatgat ggcatcatcc 540
   ttcctggcaa ataaacttta tccaaaggcc aataaagttt tctca 585


<210> 185
<211> 221
<212> PRT
<213> Rattus norvegicus

<220>
<223> Tspan-2

<400> 185
Met Gly Arg Phe Arg Gly Gly Leu Arg Cys Ile Lys Tyr Leu Leu Leu
1               5                   10                  15

Gly Phe Asn Leu Leu Phe Trp Leu Ala Gly Ser Ala Val Ile Ala Phe
            20                  25                  30

Gly Leu Trp Phe Arg Phe Gly Gly Thr Ile Lys Asp Leu Ser Ser Glu
        35                  40                  45

Glu Lys Ser Pro Glu Tyr Phe Tyr Val Gly Leu Tyr Val Leu Val Gly
    50                  55                  60

Ala Gly Ala Leu Met Met Ala Val Gly Phe Phe Gly Cys Cys Gly Ala
65                  70                  75                  80

Met Arg Glu Ser Gln Cys Val Leu Gly Ser Phe Phe Thr Cys Leu Leu
                85                  90                  95

Val Ile Phe Ala Ala Glu Val Thr Thr Gly Val Phe Ala Phe Ile Gly
            100                 105                 110

Lys Asp Val Ala Ile Arg His Val Gln Ser Met Tyr Glu Glu Ala Tyr
        115                 120                 125

Ser Asp Tyr Val Arg Asp Arg Gly Arg Gly Asn Gly Thr Leu Ile Thr
    130                 135                 140

Phe His Ser Ala Phe Gln Cys Cys Gly Lys Glu Ser Ser Glu Gln Val
145                 150                 155                 160

Gln Pro Thr Cys Pro Lys Glu Leu Pro Gly His Lys Asn Cys Ile Asp
                165                 170                 175

Lys Ile Glu Thr Ile Ile Ser Val Lys Leu Gln Leu Ile Gly Ile Val
            180                 185                 190

Gly Ile Gly Ile Ala Gly Leu Thr Ile Phe Gly Met Ile Phe Ser Met
        195                 200                 205

Val Leu Cys Cys Ala Ile Arg Asn Ser Arg Asp Val Ile
    210                 215                 220


<210> 186
<211> 3854
<212> DNA
<213> Rattus norvegicus

<220>
<223> Tspan-2

<400> 186
ggacgccgcc gccggggtcg cagcatgggg cgttttcgcg ggggcctgcg gtgcatcaag 60
tatttgctgc tcggcttcaa cctgctgttc tggctagccg gatcggccgt tattgcattt 120
ggactatggt ttcgatttgg aggtaccata aaggatttat catcagagga gaagtcccca 180
gagtatttct atgtggggct gtatgtgctg gttggagctg gagccctaat gatggctgtg 240
ggtttctttg ggtgctgtgg agccatgcgt gagtcacagt gtgtgctcgg atcttttttt 300
acctgtctgc tggtgatatt cgctgctgaa gtaaccactg gagtgtttgc atttataggc 360
aaggatgtag ctatacgaca cgtacagagc atgtatgagg aggcctacag cgattacgtt 420
agggacaggg gaaggggaaa tggaactctc attaccttcc actcagcatt tcagtgctgt 480
ggaaaagaaa gctctgaaca agtccagccc acgtgtccaa aggagcttcc aggccacaag 540
aattgcatcg acaaaattga aaccataatc agtgttaagc tccagctcat tggaattgtt 600
ggtattggga ttgcgggtct cacgatcttt ggcatgatat tcagcatggt gctctgctgt 660
gcaatacgga actcacgcga tgtgatttga agatacttca tgaaaattgc aatctagagt 720
gttcgtgcta attatcacaa gagctcgtgg ctcattttta atattcagga tattagaagc 780
taaaataatt ttctgtcaac tgtatatttg cacatgtatg tatgtttgat tcattactga 840
tttgcctcct ggagtgaatg tctctggttt ctggaatgca ttgcacatac tgatagctgt 900
ttcggaaacc ctagttcttg tatgcaagaa taacttattt aattgccaga aaaaagcaat 960
gagagatatt taaaacttgg ctattcatta aatgtaataa tgggtccatt tctcagattt 1020
tagccatgaa aatgagatac ataaagagaa tgcaggattt ctagttaatg gaagcaataa 1080
gatataagaa ttgaagggcc atagattgac tgtgtctgag ttgaatgtaa aggtttcttt 1140
aaaaaaaaaa ttctccccag aatacagtaa accacagttt tagaactaaa cacatctgta 1200
aaagtaaata tagcatggaa aaatataatt tgaaaaagtc atgctttctt agcatttaaa 1260
aacagaaaac ctctctctgg aaagagaagt cacatagaca agcatgtgcc ccataaaagc 1320
gagtgctttt tggactaaaa ataacaactt ttaaggggaa atcctgttat ccgaggtcat 1380
gtaaatgttg ctttagccct cttctctcct gactctgctt tgaactgctg gaagcctcac 1440
ctttccacaa gggctttccc tcatctcagt gcttctccag atgtggtgac agaggaaagc 1500
aagaactgtg cagtgtgaca gtgggggacc tcgctggcac tgtgcctctg gaatacctcg 1560
tagcttgagt ttacatactt tcctcattgt tttccatttt tcttatttat cagtgcagag 1620
aattcttcat agattaagaa atcagtttta ttctttaaaa taagtagacc ccctgtaact 1680
cagagtatgt ttagtagtav cctgactaaa ccaagataaa caccttcttc aatatatata 1740
cacgcagcaa aggcaagtta atgaatccaa aatgtttcat gcatacaggc tgcagcacaa 1800
atatattctt tcttagcaaa tccctgtgga ataaatgcct caggctctgt ggatacctga 1860
gtgattgatt ttaagtgggg aaacccacag cggggtctaa attggcacat tttttagaac 1920
agaacaactt gttaaaggat ataaattgtg aattcttaag attgtatttg accacacaaa 1980
aaaatgaagg tgtgagggat tctggaaacc atcagggaag cttctgtgcc cgctaagaac 2040
ataagacttt gatgtgtgct agaacaaagt accatgtttc aggaaacttt tacaagtgtt 2100
ttgctgagga cacacaactg ctgggatgtc ttgcccacag agtgccacct ctctcttttc 2160

agatatgctt gctactgctc ctagctttgg agaaaaactg agggagttaa aagtgtaact 2220
gtatgactac tttacctaca gatgcaaaag agtatttaaa aaacatcact tcttgatgtt 2280
tgtaccccag tggctgacaa aggccagctt ttcaggcaga caaagagcga tcagtccttc 2340
ttttagcttt agtctatctg tcttcctgaa gacagaggac aggctccaga gtgtactcag 2400
gttctccagc tcagagttat ttcccgttcc tcagaagtaa gtttcgttcc tcagtgagtg 2460
agtgagttta aagaaaacca cagtaactat agacagaaga ataatttttt aatgcagagg 2520
aataagtcta gaaatccgta tcctgtgtga ttagtgtacc tctctgaggg ttaagctttc 2580
caaagaatgt actgggtaaa aatagagaat tccatctctg agtgaagatg gatatatatc 2640
tttatgtgaa tggataaagc tatatctgta gagatatata acccctctgt cctgaaccta 2700
tacatacctt aaatacgtat ctcttaagga tatatacata tgtaacttaa tacatgtgtg 2760
ccctggtgga aactatggag ataaatgttt gtatacatac acatgtacat gtatgtaaat 2820
ttacagttat ttctgtttac ctgaactaat ttgagatcag acccaaacat tttaagttat 2880
tttaaattaa attttctatg tacttacact gtttgagtta tcttaaaatc ttactgtatc 2940
ttcttaacac attcatgtga cttattcaat gtgttaccta ccagttcacc caaacaatat 3000
agaaaaagca gactggtaac ccttccatgc tgttacttac agcgggttca aaagcaacac 3060
ccctagggtt ctaattacat atagttgtac ttcagctcac actagttcac ccaagttgtt 3120
cctccttcct ccaagaaaac ttggttatct gtggcttggc tgtggaggtg atcctgggtc 3180
caatgtggcc ctcctgtaca gacagccctc ctttccctct gaggaatcta atcgatgtag 3240
ctgggtgagg atgaggccca gagagatgat aataatctcg taatcagcaa agaggatagc 3300
gtgttctccc ttctgaaata agtttaccaa atatttgctc tcttgtcatt ggtaaaggtg 3360
gtttctatgg cccagaggcc agccacagcc cagctttcaa ggccccattg acatgctctt 3420
gaacaccaag cctgtttccc atacccagga aagagcaatt tatgtctctc gaaggtagag 3480
ccagcttaag ctcacgtgag tcaatttggt ttttgtgtgt attttaagat tgagattttc 3540
tatttttttt aaacatcctc catttttttt actataaaag gtattttgta tatagatgtc 3600
ttttaaaagt gaaaatacta caacaataca aaacattatt gtttggtgtt cttataacaa 3660
ctaagggatt cacagtccag atgctgcccc tggggaaaca gagaaacatg gcgaatacaa 3720
gtaatatatc ctgtggttcc caaagtgttg ttaacagaaa cattctttgt gtttgtggtg 3780
tgtttcctgt ctattttatg atttctttta cttctattaa acagcaataa aaaattaaaa 3840
aaaaaaaaaa aaaa 3854

<210> 187
<211> 801
<212> PRT
<213> Rattus norvegicus

<220>
<223> AMP deaminase isoform C

<400> 187
Met Arg Ala Pro Val Cys Val Gly Arg Gly Gly Lys Val Ile Gln Gly
1               5                   10                  15

Thr Ser Ser Thr Pro Gly Glu His Gln Arg Leu Leu Val Gly Leu Ala
            20                  25                  30

Thr Lys Thr Glu Met Pro Arg Gln Phe Pro Lys Leu Asn Met Ser Asp
        35                  40                  45

Leu Asp Glu His Val Arg Leu Leu Ala Glu Lys Val Phe Ala Lys Val
    50                  55                  60

Leu Arg Glu Glu Asp Ser Lys Asp Val Met Ser Leu Phe Thr Val Pro
65                  70                  75                  80

Lys Asp Cys Pro Ile Gly Gln Lys Glu Ala Lys Glu Arg Glu Leu Gln
                85                  90                  95

Lys Glu Leu Ala Glu Gln Lys Ser Val Glu Thr Ala Lys Arg Lys Lys
            100                 105                 110

Ser Phe Lys Met Ile Arg Ser Gln Ser Met Ser Leu Gln Met Pro Thr
        115                 120                 125

Gln Asp Trp Lys Gly Pro Pro Ser Val Ser Pro Ala Met Ser Pro Thr
    130                 135                 140

Thr Pro Leu Val Leu Gly Ala Ala Ser Lys Pro Gly Leu Ala Pro Tyr
145                 150                 155                 160

Asp Met Pro Glu Tyr Gln Arg Ala Thr Ile Ser Gly Asp Tyr Cys Ala
                165                 170                 175

Gly Ile Thr Met Glu Asp Tyr Glu Gln Ala Ala Lys Ser Leu Ala Lys
            180                 185                 190

Ala Leu Met Ile Arg Glu Lys Tyr Ala Arg Leu Ala Tyr His Arg Phe
        195                 200                 205

Pro Arg Thr Thr Ala Gln Tyr Leu Ala His Gln Gly Glu Ser Val Pro
    210                 215                 220

Leu Glu Glu Gly Leu Pro Asp Phe His Pro Pro Pro Leu Pro Gln Glu
225                 230                 235                 240

Asp Pro Tyr Cys Leu Asp Asp Ala Pro Pro Asn Leu Gly Tyr Leu Val
                245                 250                 255

Arg Met Gln Gly Gly Val Leu Phe Val Tyr Asp Asn Gln Thr Met Leu
            260                 265                 270

Glu Arg Gln Glu Pro His Ser Leu Pro Tyr Pro Asp Leu Glu Thr Tyr
        275                 280                 285

Ile Val Asp Met Ser His Ile Leu Ala Leu Ile Thr Asp Gly Pro Thr
    290                 295                 300

Lys Thr Tyr Cys His Arg Arg Leu Asn Phe Leu Glu Ser Lys Phe Ser
305                 310                 315                 320

Leu His Glu Met Leu Asn Glu Met Ser Glu Phe Lys Glu Leu Lys Ser
                325                 330                 335

Asn Pro His Arg Asp Phe Tyr Asn Val Arg Lys Val Asp Thr His Ile
            340                 345                 350

His Ala Ala Ala Cys Met Asn Gln Lys His Leu Leu Arg Phe Ile Lys
        355                 360                 365

Tyr Thr Tyr Gln Thr Glu Pro Asp Arg Thr Val Ala Glu Lys Leu Gly
    370                 375                 380

Arg Lys Ile Thr Leu Arg Gln Val Phe Asp Ser Leu His Met Asp Pro
385                 390                 395                 400

Tyr Asp Leu Thr Val Asp Ser Leu Asp Val His Ala Gly Arg Gln Thr
                405                 410                 415

Phe His Gly Phe Asp Lys Phe Asn Ser Lys Tyr Asn Pro Val Gly Ala
            420                 425                 430

Ser Glu Leu Arg Asp Leu Tyr Leu Lys Thr Glu Asn Tyr Leu Gly Gly

435                 440                 445

Glu Tyr Phe Ala Arg Met Val Lys Glu Val Ala Arg Glu Leu Glu Asp
    450                 455                 460

Ser Lys Tyr Gln Tyr Ser Glu Pro Arg Leu Ser Ile Tyr Gly Arg Ser
465                 470                 475                 480

Pro Lys Glu Trp Ser Ser Leu Ala Arg Trp Phe Ile Gln His Lys Val
                485                 490                 495

Tyr Ser Pro Asn Met Arg Trp Ile Ile Gln Val Pro Arg Ile Tyr Asp
            500                 505                 510

Ile Phe Arg Ser Lys Lys Leu Leu Pro Ser Phe Gly Lys Met Leu Glu
        515                 520                 525

Asn Ile Phe Leu Pro Leu Phe Gln Ala Thr Ile Asn Pro Gln Asp His
    530                 535                 540

Arg Glu Leu His Leu Phe Leu Lys Tyr Val Thr Gly Phe Asp Ser Val
545                 550                 555                 560

Asp Asp Glu Ser Lys His Ser Asp His Met Phe Ser Asp Lys Ser Pro
                565                 570                 575

Ser Pro Asp Leu Trp Thr Ser Glu Gln Asn Pro Pro Tyr Ser Tyr Tyr
            580                 585                 590

Leu Tyr Tyr Met Tyr Ala Asn Ile Met Val Leu Asn Asn Leu Arg Arg
        595                 600                 605

Glu Arg Gly Leu Ser Thr Phe Leu Phe Arg Pro His Cys Gly Glu Ala
    610                 615                 620

Gly Ser Ile Thr His Leu Val Ser Ala Phe Leu Thr Ala Asp Asn Ile
625                 630                 635                 640

Ser His Gly Leu Leu Leu Lys Lys Ser Pro Val Leu Gln Tyr Leu Tyr
                645                 650                 655

Tyr Leu Ala Gln Ile Pro Ile Ala Met Ser Pro Leu Ser Asn Asn Ser
            660                 665                 670

Leu Phe Leu Glu Tyr Ser Lys Asn Pro Leu Arg Glu Phe Leu His Lys
        675                 680                 685

Gly Leu His Val Ser Leu Ser Thr Asp Asp Pro Met Gln Phe His Tyr
    690                 695                 700

Thr Lys Glu Ala Leu Met Glu Glu Tyr Ala Ile Ala Ala Gln Val Trp
705                 710                 715                 720

Lys Leu Ser Thr Cys Asp Leu Cys Glu Ile Ala Arg Asn Ser Val Leu
                725                 730                 735

Gln Ser Gly Leu Ser His Gln Glu Lys Gln Lys Phe Leu Gly Gln Asn
            740                 745                 750

Tyr Tyr Lys Glu Gly Pro Glu Gly Asn Asp Ile Arg Lys Thr Asn Val
        755                 760                 765

Ala Gln Ile Arg Met Ala Phe Arg Tyr Glu Thr Leu Cys Asn Glu Leu
    770                 775                 780

Ser Phe Leu Ser Asp Ala Met Lys Ser Glu Glu Ile Thr Ala Leu Ala
785                 790                 795                 800

Asp

<210> 188
<211> 3915
<212> DNA
<213> Rattus norvegicus

<220>
<223> AMP deaminase isoform C

<400> 188
tgtgtgtgtg tgtgtgtaca tgcgcgcgcc cgtgtgtgtt gggaggggcg ggaaggtgat 60
tcaagggacc agttcaacac ctggagaaca ccaacgcttg ctggtcggtt tagctaccaa 120
gaccgagatg cctcgccaat tccccaagtt gaacatgtcc gatttggacg aacatgtccg 180
gctgttggca gagaaggtgt tcgctaaggt gctccgagaa gaggacagca aagatgttat 240
gtccctgttt actgtcccca aggactgtcc catcggacag aaggaagcca aggagaggga 300
gctacagaag gagttggcag agcagaagtc tgtggagaca gcaaaaagaa agaagagttt 360
caagatgatc cggtcgcagt ctatgtccct gcagatgcca acacaagact ggaaaggtcc 420
tccatctgtc agtccggcca tgtctcctac aacccctctg gtccttggag ctgcttctaa 480
gcctgggctg gcaccctatg acatgcctga gtatcagcga gccaccatca gtggagatta 540
ctgtgcaggg atcactatgg aagactatga acaggctgcc aagagcctgg ccaaggccct 600
gatgatccgg gagaagtatg cacggcttgc ctatcaccgc ttcccgcgga ccacagccca 660
gtacctggct catcaggggg aaagtgtgcc tttggaagag ggtctcccag acttccaccc 720
tcccccactg ccccaggaag acccttattg cctggacgat gcgcccccca acctgggcta 780
cctggtccgc atgcagggag gtgttctctt tgtgtatgac aatcagacga tgttggaacg 840
ccaggaaccc cacagcctgc cctaccctga cctggagacc tacatcgtgg acatgagcca 900
tattctggca ctcatcaccg atggccccac gaaaacctat tgtcaccggc gactgaactt 960
tctggaatcc aagtttagcc ttcatgagat gttaaatgaa atgtcagagt tcaaggaatt 1020
gaagagtaat ccccacagag acttttataa tgtgagaaag gtagacacac acatccatgc 1080
agcggcctgc atgaatcaga agcacctgct gcgtttcatc aagtacacat accagacaga 1140
gccggacaga actgtggccg agaagctcgg ccggaagatc accctgcggc aggtgtttga 1200
cagcctgcac atggacccct acgacctcac agtggactcg ctggatgttc atgcgggccg 1260
gcagacattt cacggcttcg acaagttcaa ctccaagtac aaccctgtgg gggccagtga 1320
gcttcgggac ctgtatttaa aaactgaaaa ctacctgggg ggagagtact ttgctcgcat 1380
ggtcaaggaa gtggcccggg aattggagga cagcaagtac cagtactcag aaccccggct 1440
gtccatctat ggtcgcagtc ctaaggaatg gtctagcctg gcccgctggt tcattcagca 1500
caaggtctac tcgcccaaca tgcgttggat catccaggtg ccgcggatct atgatatatt 1560
taggtcaaag aaactactgc caagctttgg gaagatgctg gagaacatct tcctgcccct 1620
tttccaggct actatcaacc ctcaagacca tcgggagctt cacctcttcc ttaaatacgt 1680
gacagggttt gacagcgtgg atgatgagtc caagcacagt gaccacatgt tctcagacaa 1740
gagccccagc ccagacctct ggaccagcga gcagaaccca ccctacagtt actacctgta 1800
ctacatgtat gccaacatca tggtgctgaa caacctccgc agggaacgag gcctgagcac 1860
attcttgttc cgacctcact gtggggaagc cggctccatc actcacctag tgtctgcctt 1920
cctgactgca gacaacattt cccacgggct gctcctcaag aagagtcctg ttttgcagta 1980
tctctactac cttgctcaga ttcctattgc catgtctcct ctcagcaaca acagcctgtt 2040
tctggaatat tccaagaacc cacttcggga attcctacac aagggattac acgtctctct 2100
ctctactgat gaccccatgc agttccacta caccaaggaa gcgctcatgg aggagtacgc 2160
catcgcagcc caagtgtgga agttgagtac gtgtgacctg tgtgagattg ccaggaacag 2220
cgtgctgcag agcggcctct cacatcagga gaagcagaag tttttgggtc agaattatta 2280
caaggaggga ccggagggca atgacatccg aaagacaaat gttgcacaga tccggatggc 2340
tttccgctac gagaccctgt gtaacgagct cagctttctg tccgatgcca tgaagtcaga 2400
ggagatcaca gccctggcgg actaggccag cgatccacac acactttcat cttttggttt 2460

aatgtcaggt ctgctggggc taacggtgcc cagatctcaa accggggctt ccgtctgtga 2520
cgaggacacc tctgaagaaa tctcaagctc ccagttttgg ttgcacttta tgaacatgct 2580
cacttcatta gcgtttctga gccagaggct ggctctcttc catggggctc tgggagctgg 2640
cactgtgtac acctgctcct tggttgtccg agtctcctgt gattaccaat gtttatatgg 2700
ttggggctgg gactttgcaa ggctgaatgc caagtgacac aaggcctgtc tggtgtctgc 2760
cacagtcctc ccagcagacc ctgtagagca attctctctt tggtatctgg ctggctggct 2820
gcctgaaccc ccaccaaagc cccttttata aagggctacc tagagaaaat acaaagctcc 2880
ctgctgacga ttgtgcactt ttaaaacagt ttttttccct gtgagcaccc ctccttcctg 2940
tctaggtagg aagcccaacg caccctctga ggtcatcact tctgttattt tcctcatcta 3000
ggaatgagtc atctcaaacc atgttctgtg aacccatctc atggtccact cctaggcctg 3060
gggaagatac agggcgaggt tctggggctg ttctcacttt tttgtttttt taaatgaagg 3120
cctttgaaaa gcataggacc tccgctcctt ttggctctaa tgtcctatga ttgtagctgt 3180
caccaccttc ccccaggagt cagaccaggg actgactcct caggggagtg aaatgtttca 3240
ggacatacag gactagagcc aagttcctga tccagtcaca cccggtcccc gactcccctg 3300
cttccaccta ctattttagg cttcacatat tttaattttt ttttaaagaa tcactgtaac 3360
agtgcatttt attttaaagc agatttcttc agaagctatc atggtcccag ggaccaggta 3420
gagttaagtt taatgtggac tctagcttta tcagactact catctttgtt actagtgcct 3480
agaacatagt aggtgctcaa taaatacata tggagtaatt gaattacttt ctcttttggc 3540
atctgtatgt acagaatact gaacaatgtt caaagatctt cctaactgtt cacaaggcag 3600
gaagtcctgt accatactta gatgaaccta attgcacctg gaagatgctc atgtaactaa 3660
ttacccccgt cctgtgactc tgccagcctt ccagtgttca cacagcgtat ggaaggaacc 3720
atgtcatgcc ttactaggca aataaacata ccaccctaca tagctgatta tgaaaccgaa 3780
tgaaacctgt cgttatgagg gattattttt gaaaagcctc catttgtata catttgccca 3840
aacatagcaa gtattgtaca gtttttcatt gtatcaaata cttttttaaa gactacaaat 3900
aaagtctgtt aggac 3915

<210> 189
<211> 142
<212> PRT
<213> Rattus norvegicus

<220>
<223> Globin, alpha, major

<400> 189
Met Val Leu Ser Ala Asp Asp Lys Thr Asn Ile Lys Asn Cys Trp Gly
1               5                   10                  15

Lys Ile Gly Gly His Gly Gly Glu Tyr Gly Glu Glu Ala Leu Gln Arg
            20                  25                  30

Met Phe Ala Ala Phe Pro Thr Thr Lys Thr Tyr Phe Ser His Ile Asp
        35                  40                  45

Val Ser Pro Gly Ser Ala Gln Val Lys Ala His Gly Lys Lys Val Ala
    50                  55                  60

Asp Ala Leu Ala Lys Ala Ala Asp His Val Glu Asp Leu Pro Gly Ala
65                  70                  75                  80

Leu Ser Thr Leu Ser Asp Leu His Ala His Lys Leu Arg Val Asp Pro
                85                  90                  95

Val Asn Phe Lys Phe Leu Ser His Cys Leu Leu Val Thr Leu Ala Cys
            100                 105                 110

His His Pro Gly Asp Phe Thr Pro Ala Met His Ala Ser Leu Asp Lys
        115                 120                 125

Phe Leu Ala Ser Val Ser Thr Val Leu Thr Ser Lys Tyr Arg

    130                 135                 140

<210> 190
<211> 556
<212> DNA
<213> Rattus norvegicus

<220>
<223> Globin, alpha, major

<400> 190
acattctcct tctgatagac tcaggaagca atcatggtgc tctctgcaga tgacaaaacc 60
aacatcaaga actgctgggg gaagattggt ggccatggtg gtgaatatgg cgaggaggcc 120
ctacagagga tgttcgctgc cttccccacc accaagacct acttctctca cattgatgta 180
agccccggct ctgcccaggt caaggctcac ggcaagaagg ttgctgatgc cttggccaaa 240
gctgcagacc acgtcgaaga cctgcctggt gccctgtcca ctctgagcga cctgcatgcc 300
cacaaactgc gtgtggatcc tgtcaacttc aagttcctga gccactgcct gctggtgacc 360
ttggcttgcc accaccctgg agatttcaca cccgccatgc acgcctctct ggacaaattc 420
cttgcctctg tgagcactgt gctgacctcc aagtaccgtt aagccgcctc ctgccgggct 480
tgccttctga ccaggccctt cttccctccc ttgcacctat acctcttggt ctttgaataa 540
agcctgagta ggaagc 556

<210> 191
<211> 1081
<212> DNA
<213> Rattus norvegicus

<220>
<223> Cytochrome b oxidase, mitochrondrial

<400> 191
ggcacgagaa cttcggttct ctactaggag tatgcctcat agtacaaatc ctcacaggct 60
tattcctagc aatacactac acgtctgata ccataacagc attttcatca gtcacccaca 120
tctgccgaga cgtaaactac ggctgactaa tccgatacct acacgccaac ggccctcaat 180
atttttcatc tgcctattcc tccatgtggg acgaggacta tactatggat cctacacttt 240
cctagaaacc tgaaacattg gaatcattct actatttgca gtcatagcaa ctgcattcat 300
gggctatgta ctcccatgag gacaaatatc attctgagga gctacagtaa ttacaaacct 360
attatcagct atcccttaca ttgggactac cctagtcgaa tgaatctgag gaggcttctc 420
agtagacaaa gcaaccctaa cacgcttctt cgcattccac ttcatcctcc cattcattat 480
cgccgccctt gcaattgtac atcttctttt cctccacgaa acaggatcaa ataaccccac 540
aggattaaac tccaacgcag acaaaatccc attccatcca tattatacaa ttaaagacct 600
cctaggtgta tttatattac tattattcct aataacccta gtactattct tccagaccta 660
ctaggagacc agacaattat acacccgcta acccctcaac accccacccc acatcaaacc 720
agaatgatat tttctctttg ccttacgcta ttttatgctc cattcccaac aaactaggag 780
gaggtcgtga ggccctaatc ttatcaatcc taatctttag ccttcctacc attcgctgca 840
tacttcaaaa caacgcagct taacattccg cccaatcacc caaatccttt acggaatcct 900
ggtagccaac ctccttagtc ttatacagga atcggaggcc aaccagtaga acacccattt 960
atcattattg gccaactagc ctccatcagc tacttttcaa ttatcctcat tctcatacca 1020
atctctggaa ttgttgaaga caaaatgtta aaatgaaatt aataaaaaaa gaaaaaaaaa 1080
a 1081

<210> 192
<211> 342
<212> PRT
<213> Rattus norvegicus

<220>
<223> BB1

<400> 192


<210> 193
<211> 1897
<212> DNA
<213> Rattus norvegicus

<220>
<223> BB1

<400> 193
ccacactttg cattctctgg tcaccatcct cgggacctgg gccctccatt caggcccagc 60
cctgctcctg ccacgccctg gctctggcct ggactttctc ctatctcctg ttcttccgag 120
ccctcagcct cctggcctgc ccactcccac gcccttcacc aatgccgtcc agctgctgct 180
gacgctgaag ctggtgagcc tggccagtga agtccaggac ctgcatctgg cccagaggaa 240
ggaaatggcc tcaggcttca gcaaggggcc caccctgggg ctgctgcgac gtgccctccc 300
tgatggagac actcagctac agctactgct acgtgggaat catgacaggc ccgttcttcc 360
gctaccgcac ctacctggac tggctggagc agcccttccc cggggcagtg ccagcctgcg 420
gcccctgctg cgccgcgcct ggccggcccc gctcttcggc ctgctgttcc tgctctcctc 480
tcacctcttc ccgctggagg ccgtgcgcga ggacgccttc tacgcccgcc cgctgcccgc 540
ccgcctcttc tacatgatcc ccgtcttctt cgccttccgc atgcgcttct acgtggcctg 600
gattgccgcc gagtgcggct gcattgccgc cggctttggg gcctaccccg tggccgccaa 660
agcccgggcc ggaggcggcc ccaccctcca atgcccaccc cccagcagtc cggagaaggc 720
ggcttccttg gagtatgact atgagaccat ccgcaacatc gactgctaca gcacagattt 780
ctgcgtgcgg gtgcgcgatg gcatgcggta ctggaacatg acggtgcagt ggtggctggc 840
gcagtatatc tacaagagcg cacctgcccg ttcctatgtc ctgcggacgg cctggaccat 900
gctgctgagc gcctactggc acggcctcca cccgggctac tacctgagct tcctgaccat 960
cccgctgtgc ctggctgccg agggccggct ggagtcagcc ctgcgggggc ggctgagccc 1020
agggggccag aaggcctggg actgggtgca ctggttcctg aagatgcgcg cctatgacta 1080
catgtgcatg ggcttcgtgc tgctctcctt ggccgacacc cttcggtact gggcctccat 1140
ctacttctgt atccacttcc tggccctggc agccctgggg ctggggctgg ctttaggtgg 1200
gggcagcccc agccggcgga aggcagcatc ccagcccacc agccttgccc cggagaagct 1260
ccgggaggag taagctgtca cgaccctccc tctgccagct ggtcccggga attctgtgaa 1320
ccaggctgct gtctcctccc cagaaagagt ccttaccttg gagagggtcc tggagagaat 1380
ttcctcttcc ccagctaaat accctgcctg caactgaagc agacccgggg gtgtcctccc 1440
tgccctctgc ccagaggcac ctccactcct acaaaatcaa agtattgtcc agacaagagt 1500
cactggcccc tgctccagct tctgggtatc cagagagcac tgcacttccc caaaacggaa 1560
ggggcccctg ggcagtgggt tttgggcaaa ttccctttct ttgcatccac aatgtgggtc 1620
ggagcttggg ggcaggtcct gggagtggga agcctcttcc ttgtgtcttt cgctccactt 1680
ttagctcatc gcaccaatat tgcagacttg gaaggaagca taacttccat ttcacaaagg 1740
ggaaactgag gtgcgggtgc gggcctgggg acggccgtcc catggcttcc atctgagcca 1800
cctcgggacc ccagcactcc tggcgccctc ttcttcatcg cttggcctat gacaggtcac 1860
cgtgtgtaaa tctttcccaa taaagtgttg cacaaaa                          1897


<210> 194
<211> 324
<212> PRT
<213> Homo sapiens

<220>
<223> CGI-21 protein

<400> 194
Met Phe Ser Ser Thr Ser Thr Pro Ser Ser Phe Thr Ala Phe Gln Thr

1               5                   10                  15

Thr Met Arg Ser Ser Ile Pro His Trp Arg Ile Ser Arg Met Cys Leu
            20                  25                  30

Lys Pro Thr Phe Thr Lys Gln Gln Ile Ala Asn Leu Asp Lys Gln Ala
        35                  40                  45

Lys Leu Ser Arg Ala Tyr Asp Gly Thr Thr Tyr Leu Pro Gly Ile Val
    50                  55                  60

Gly Leu Asn Asn Ile Lys Ala Asn Asp Tyr Ala Asn Ala Val Leu Gln
65                  70                  75                  80

Ala Leu Ser Asn Val Pro Pro Leu Arg Asn Tyr Phe Leu Glu Glu Asp
                85                  90                  95

Asn Tyr Lys Asn Ile Lys Arg Pro Pro Gly Asp Ile Met Phe Leu Leu
            100                 105                 110

Val Gln Arg Phe Gly Glu Leu Met Arg Lys Leu Trp Asn Pro Arg Asn
        115                 120                 125

Phe Lys Ala His Val Ser Pro His Glu Met Leu Gln Ala Val Val Leu
    130                 135                 140

Cys Ser Lys Lys Thr Phe Gln Ile Thr Lys Gln Gly Asp Gly Val Asp
145                 150                 155                 160

Phe Leu Ser Trp Phe Leu Asn Ala Leu His Ser Ala Leu Gly Gly Thr
                165                 170                 175

Lys Lys Lys Lys Lys Thr Ile Val Thr Asp Val Phe Gln Gly Ser Met
            180                 185                 190

Arg Ile Phe Thr Lys Lys Leu Pro His Pro Asp Leu Pro Ala Glu Glu
        195                 200                 205

Lys Glu Gln Leu Leu His Asn Asp Glu Tyr Gln Glu Thr Met Val Glu
    210                 215                 220

Ser Thr Phe Met Tyr Leu Thr Leu Asp Leu Pro Thr Ala Pro Leu Tyr
225                 230                 235                 240

Lys Asp Glu Lys Glu Gln Leu Ile Ile Pro Gln Val Pro Leu Phe Asn
                245                 250                 255

Ile Leu Ala Lys Phe Asn Gly Ile Thr Glu Lys Glu Tyr Lys Thr Tyr
            260                 265                 270

Lys Glu Asn Phe Leu Lys Arg Phe Gln Leu Thr Lys Leu Pro Pro Tyr
        275                 280                 285

Leu Ile Phe Cys Ile Lys Ile Phe Thr Lys Asn Asn Phe Phe Val Glu
    290                 295                 300

Lys Asn Pro Thr Ser Cys Gln Phe Pro Tyr Tyr Lys Cys Gly Ser Glu
305                 310                 315                 320

Arg Ile Leu Val

<210> 195
<211> 2166
<212> DNA
<213> Homo sapiens

<220>
<223> CGI-21 protein

<220>
<221> misc_feature
<222> 9
<223> n is a or g or c or t

<400> 195
ggtagtggna gatgtccggc cggtctaagc gggagtctcg cggttccact cgcgggaagc 60
gagagtctga gtcgcggggc agctccggtc gcgtcaaggg arcgaggatc gggagcggga 120
cctgagcggc gagctcccgg ggcagccctg tgcgcgtgaa gcggggagtt cgagccggcg 180
agcgcgcgcg aggccccggc ttctgttgtc ccgtttgtgc gggtgaagcg ggagcgmgag 240
gtcgatgagg actcggagcc tgagcgggag gtgcgagcaa agaatggccg agtggattct 300
gaggaccgga gagccgccac tgcccgtacc tggacaccat taacaggagt ktgctggact 360
ttgactttga gaaactgtgt tctatctccc tctcacacat caatgcttat gcctgtctgg 420
tgtgtggcaa gtactttcaa ggccggggtt tgaagtctca cgcctacatt cacagtgtcc 480
agtttagcca ccatgttttc ctcaacctcc acaccctcaa gttttactgc cttccagaca 540
actatgagat catcgattcc tcattggagg atatcacgta tgtgtttgaa gcccactttc 600
acaaagcagc aaattgcaaa cttggacaag caagccaaat tgtcccgggc atatgatggt 660
accacttacc tgccgggtat tgtgggactg aataacataa aggccaatga ttatgccaac 720
gctgtccttc aggctctatc taatgttcct cctctccgga actactttct ggaagaagac 780
aattataaga acatcaaacg tcctccaggg gatatcatgt tcttgttggt ccagcgtttt 840
ggagagctga tgagaaagct ctggaaccct cgaaatttca aggcacatgt gtctccccat 900
gagatgcttc aggcagttgt actttgcagt aagaagactt ttcagatcac caaacaagga 960
gatggcgttg actttctgtc ttggtttctg aatgctctgc actcagctct ggggggcaca 1020
aagaagaaaa agaagactat tgtgactgat gttttccagg ggtccatgag gatcttcact 1080
aaaaagcttc cccatcctga tctgccagca gaagaaaaag agcagttgct ccataatgac 1140
gagtaccagg agacaatggt ggagtccact tttatgtacc tgacgctgga ccttcctact 1200
gcccccctct acaaggacga gaaggagcag ctcatcattc cccaagtgcc actcttcaac 1260
atcctggcta agttcaatgg catcactgag aaggaatata agacttacaa ggagaacttt 1320
ctgaagcgct tccagcttac caagttgcct ccatatctaa tcttttgtat caagatattc 1380
actaagaaca acttctttgt tgagaagaat ccaactagtt gtcaatttcc ctattacaaa 1440
tgtggatctg agagaatact tgtctgaaga agtacacaag aataccacct atgacctcat 1500
tgccaacatc gtgcatgacg gcaagccctc cgagggctcc taccggttcc acgtgcttca 1560
tcatgggaca ggcaaatggt atgaattaca agacctccag gtgactgaca tccttcccca 1620
gatgatcaca ctgtcagagg cttacattca gatttggaag aggcgagata atgatgaaac 1680
caaccagcag ggggcttgaa ggaggcgtct agggctttgc tcccaagggc tgtggctgat 1740
gatggtaaat aagaacacag aagctgtagc tgaacacagg ctggctggtg ggcttcctag 1800
gccagcccag cttgtatggg ttctggctac accagagcac caagagccca cttgcctggg 1860
atggccccac actgtcactc agctgttctt tgatcatttt tttctagatt gatgctcctt 1920
tctcccatgc attgagctcc catctagctt cagcagggca gaacccttct ccagatgtgt 1980
gtaacttatg tctgagtatc tgggagtagt tgaagaacag ataattcctt ccaaacatca 2040
agccttggga ttcttggagc aagcagaaag ccagtaactt cgctctgtta gaggtggagg 2100
attttcctat gttcccccca tttcctgatt tgtattttta gatggattaa atagtctcct 2160
gttttt 2166

<210> 196
<211> 277
<212> PRT
<213> Rattus norvegicus

<220>

<223> Myelin proteolipid protein

<400> 196


<210> 197
<211> 3019
<212> DNA

<213> Rattus norvegicus

<220>
<223> Myelin proteolipid protein

<400> 197
agagagaaaa agtaaaggac agaagaagga gactggagag accaggatcc ttccagctga 60
acaaagtcag ccgcaaaaca gactagccag caggctgcaa ttggagccag agtgccaaag 120
acatgggttt gttagagtgc tgtgctagat gtctggtagg ggcccccttt gcttccctgg 180
tggccactgg attgtgtttc tttggagtgg cactgttctg tggatgtgga catgaagctc 240
tcactggtac agaaaagtta attgagacct atttctccaa aaactaccag gactatgagt 300
atctcattaa tgtgattcat gctttccagt atgtcatcta tggaactgcc tctttcttct 360
tcctttatgg ggccctcctg ctggccgagg gcttctacac caccggcgct gtcaggcaga 420
tctttggcga ctacaagacc accatctgcg gcaagggcct gagcgcaacg gtaacagggg 480
gccagaaggg gaggggttcc agaggccaac atcaagctca ttctttggag cgggtgtgtc 540
attgtttggg aaaatggcta ggacatcccg acaagtttgt gggcatcacc tatgccctga 600
ctgttgtatg gctcctggtg tttgcctgct ctgctgtgcc tgtgtacatt tacttcaata 660
cctggaccac ctgccagtct attgccttcc ctagcaagac ctctgccagt ataggcagtc 720
tctgcgctga tgccagaatg tatggtgttc tcccatggaa tgcttttcct ggcaaggttt 780
gtggctccaa ccttctgtcc atctgcaaaa cagccgagtt ccaaatgacc ttccacctgt 840
ttattgctgc atttgtgggt gctgcagcca cactagtttc cctgctcacc ttcatgattg 900
ctgccactta caactttgcc gtccttaaac tcatgggccg aggcaccaag ttctgatctc 960
ccatagaaat ccccctttgt ctaatagcga ggctctaacc acacagccta tagtgttgtg 1020
tctcctgtct taactctgcc tttgccactg attggccctc ttcttacttg atgagtataa 1080
caagaaagga gagtattgca gtgattaatc tctctctgtg gactctccct cttatgtacc 1140
tctttcagtc attttgctcc acagtgggct cctgctagaa atggggaata cctgagaagg 1200
tgattccccc gctgcaagtc gcagaggaat gaaagctcta attgagtttg caagcatctc 1260
ctgaaggcca ggatgtgctt ccttctcaaa gggcacttcc attgaggaaa acaaagtgga 1320
aagaaagatt ctcaggtaga aagcaggaat gtccttggtc tccttgccat cagtaggagt 1380
caaatatatt ctctttgatg cacaaaacca agaactcact cttttcttcc tagttccact 1440
gaagacagaa ggaaataagc aatagcattt gcccaaatct gcctcctgca gctaggagaa 1500
gggggtcaaa gcaaggatct ttcaccctta gaaagagagc tctgacgcca gtggcaatgg 1560
actatttaag ccctaactca gccaaccttc cttacagcat tagggagcac agtgtctatt 1620
tagacaaagt ggggcctgga ggaggggggc ctcatctgtc cttacagctc attaggaaga 1680
gaaacagagt gttgtcagga tcgactcact cccttctccc tgataacagc taccatgaca 1740
accttgtggt ttccaaggag ctgagaatag aagggaacta gcttatataa aataagactg 1800
tgacctaagg agcatcagtt gggggatgct aaaggtgtaa attgagatgc cctctggcag 1860
atgcaaaaca cttaactctt tggatagcat ctccccccac cccatcagtt gtgtcctctt 1920
ggcctctggc acaatatctt cacaatggtg ctcttttcct tgggttttat ccattcactt 1980
acagcaggtg attagaggat tttgattagt ttcagaatga ctcctgtttc atatttccca 2040
gttgtttatc tcttgtttgg agctgtacca gaaagaactg gagaatgatt ctttgagctt 2100
agttcttttt caaaagaaaa aagaaaaact ggacagtaag catcacaaaa atatttgaaa 2160
ttgtgtagtc ccatgaaatg attgggattt tccccaagta ttctaccttt tgtagaacta 2220
ggcttggtaa atttgaacct ccatttgaat aacctgtcca atattttctt ttctaataaa 2280
tgacagatga ttttgcttgc taatattatc tcagcatttt gaaagtttag gcttagcata 2340
gaggttattg tgtcttggta tatataggtc acataataga tttcagcaac tgccagctgt 2400
ttagttggtg agcttccaga gagctctgga gacatggtga ggacagacag aattggaaac 2460
ctgaagaact cccagatttc aggcttatcc tgtacttgtt aactttgggt gaaagaaaga 2520
aaagcaaaga aagaaaggaa aagcctctga tctgttttta ctgaaggaaa agttactgta 2580
gatttttttt ttttgtagat ttgtgctgtc attccccaaa gtgcttttgt catgttgaaa 2640
gagatataag gatttagaag aagatacttg agacttgttc ttgggccagt atataaggta 2700
aactagcagg atgcacagta gtgaggagag cttaaaggac ctggaagaaa ccaatcaaga 2760
taaaggaagg tgaaataatc tatatctttt atttggtttg attttaatat cataacagat 2820
aaccaaccac tccctgaaaa cctcacacgt atatatacac acacgcatgc acatacaaag 2880
agagttaatc aactgcaagt gtttccttca tttctgatat agaattttga ttttaacaac 2940
ataaaggata aactcttaga aactcatctt acaaaatgta ttttataaaa ttaaagaaaa 3000
taaaattaag aatgttctc 3019